# EUROPEAN PATENT APPLICATION

(11) **EP 2 650 311 A2**
(43) Date of publication of application: **16.10.2013**
(21) Application number: 12192982.2
(22) Date of filing: 27.11.2008
(51) Int. Cl.: C07K 16/46, C07K 16/32, C07K 16/28, C07K 16/30

(54) **Amino acid sequences directed against heterodimeric cytokines and/or their receptors and polypeptides comprising the same**

(30) Priority: 27.11.2007 US 4332 P; 04.12.2007 US 5265 P; 04.12.2007 US 5324 P; 04.12.2007 US 5331 P
(62) Divisional of application: 08853848.3
(71) Applicant: Ablynx N.V., 9052 Ghent-Zwijnaarde (BE)
(72) Inventor: Saunders, Michael John Scott, 1190 Brussels (BE); Blanchetot, Christophe, 9070 Destelbergen (BE); Rommelaere, Heidi, 9000 Gent (BE); Vercammen, Jo, 1600 Sint-Pieters-Leeuw (BE); de Haard, Johannes Joseph Wilhelmus, 4436 NA Oudelande (NL)
(74) Representative: HOFFMANN EITLE

(57) **Abstract**

The present invention relates to amino acid sequences that are directed against (as defined herein) heterodimeric cytokines and/or their receptors, as well as to compounds or constructs, and in particular proteins and polypeptides, that comprise or essentially consist of one or more such amino acid sequences (also referred to herein as "*amino acid sequences of the invention*", "*compounds of the invention*", and "*polypeptides of the invention*", respectively). The invention also relates to nucleic acids encoding such amino acid sequences and polypeptides (also referred to herein as "*nucleic acids of the invention*" or "*nucleotide sequences of the invention*"); to methods for preparing such amino acid sequences and polypeptides; to host cells expressing or capable of expressing such amino acid sequences or polypeptides; to compositions, and in particular to pharmaceutical compositions, that comprise such amino acid sequences, polypeptides, nucleic acids and/or host cells; and to uses of such amino acid sequences or polypeptides, nucleic acids, host cells and/or compositions, in particular for prophylactic, therapeutic or diagnostic purposes, such as the prophylactic, therapeutic or diagnostic purposes mentioned herein.

## Description

The present invention relates to amino acid sequences that are directed against (as defined herein) heterodimeric cytokines and/or their receptors, as well as to compounds or constructs, and in particular proteins and polypeptides, that comprise or essentially consist of one or more such amino acid sequences (also referred to herein as "*amino acid sequences of the invention*", *"compounds of the invention*", and "*polypeptides of the invention*", respectively).

The invention also relates to nucleic acids encoding such amino acid sequences and polypeptides (also referred to herein as "*nucleic acids of the invention*" or "*nucleotide sequences of the invention*"); to methods for preparing such amino acid sequences and polypeptides; to host cells expressing or capable of expressing such amino acid sequences or polypeptides; to compositions, and in particular to pharmaceutical compositions, that comprise such amino acid sequences, polypeptides, nucleic acids and/or host cells; and to uses of such amino acid sequences or polypeptides, nucleic acids, host cells and/or compositions, in particular for prophylactic, therapeutic or diagnostic purposes, such as the prophylactic, therapeutic or diagnostic purposes mentioned herein.

Other aspects, embodiments, advantages and applications of the invention will become clear from the further description herein.

Heterodimeric cytokines, their receptors and the pathways, signalling, biological mechanisms and physiological effect in which they and their receptors are involved are known from the prior art.

Some of the best-known examples of heterodimeric cytokines and their receptors are IL-12, IL-23 and IL-27, and their receptors IL-12R, IL-23R, and IL-27, respectively. As their name implies, these cytokines are heterodimeric, consisting of two different subunits, i.e. IL12p40 and IL12p35 in case of IL-12, IL12p40 and IL23p19 (also called IL-30B) in case of IL-23, and EBI3 and IL27p28 in case of IL-27. Also, the receptors for these heterodimeric cytokines consist of multiple subunits, i.e. IL12Rbeta1 and IL12Rbeta2 in case of IL-12R, IL12Rbeta1 and IL23R in case of IL-23R, and WSX1 and gp130 in case of IL-27R.

IL12 remains the prototypical heterodimeric cytokine (composed of IL12p40 and IL12p35), it was not until relatively recently that other related heterodimers exist.

In 2000, IL-23 and its subunit p19 (IL23p19) were identified on the basis of a homology search for IL-6-family members. Their studies revealed that p19 dimerizes with IL12p40 and that this cytokine, known as IL23, uses IL12Rβ1, but not IL12Rβ2, as a component of its high-affinity receptor. Functional cloning identified the other subunit of the receptor for IL23, a subunit known as IL23R (text adapted from CA Hunter 2003). It was also found that IL-23 plays an important role in proliferation of Th1 7 cells.

IL27 is another heterodimeric cytokine related to IL12 composed of EBI3 and IL27p28. Epstein-Barr virus (EBV)-induced molecule 3 (EBI3) had been identified as an IL-22p40 homologue. In 2002, the p28 subunit of IL-27 (IL-27p28) was discovered as a protein with homology to IL-12p35 and IL-6.

Recently, the existence of a further heterodimeric cytokines belonging to the IL-12 family, called IL-35, has been described (Collison et al., Nature, 22 November 2007, 566). This heterodimeric cytokine is described as contributing to regulator T-cell function and is composed of the IL12p35 and EBI-3 subunits.

Thirty-four known type I cytokine receptors have been described, and although the ligands are more difficult to identify, there are at least 27 that can be clustered into 5 distinct families (see Boulay et al 2003). One of these groupings is composed of the ligands for a series of cytokine receptors that use gp130 (glycoprotein 130) or one of several gp130-related proteins. These include the receptors for IL6 and the receptors for the heterodimeric cytokines IL-12, IL-23 and IL-27 (Hunter, supra).

The IL12 receptor is a heterodimer of IL12Rbeta1 and IL12Rbeta2.

The IL23 receptor is a heterodimer of IL12Rbeta1 and IL23R. IL12Rbeta1 is a used by both IL12 and IL-23 for signalling. Targeting this receptor will lead to a blockade of both IL-12 and IL-23 signaling.

The IL27 receptor is a hetero dimer composed of WSX1 (Identified on the basis of a homology search for gp130-like proteins) and gyp130 which is a common element of the receptor for IL-6 (which comprises IL-6Rα and gp130) and the receptor for IL27 (which comprises WSX1 and gp130), which is consistent with the close familial relationship of these cytokines.

For further information on heterodimeric cytokines and their receptors, as well as the pathways, signalling, biological mechanisms and biological effects in which they are involved, and also the diseases in which they are associated, reference is made to the following prior art: Oppmann, 2000, Immunity, 13:751-725; Gubler et al., 1991, PNAS, 88: 4143-4147; Hunter, Nature Rev., Vol.5, July 2003, 521; Watford et al., Cytokine and Growth Factor Reviews 14 (2003), 361-368; Boulay, Immunity, Vol.19, 159-163 (2003); Goriely and Goldmann, American Journal of Transplantation 2007; 7: 208-284; Langrish et al., Immunological Reviews 2004, 202, 96-105; Kaufmann et al., J. Invest. Dermatol., 123: 1037-1044, 2004; Neurath, Nature Medecine, Vol.13, January 2007, 26; Collison et al., Nature, 22 November 2007, 566; Parham et al., The Journal of Immunology, 2002, 5699; EP 433 827; EP 1 210 434; EP 790 308; EP 790 309; EP 1 175 446; EP 0 969 867; EP 1 309 692; EP 1 002 084; EP 1 587 178; EP 1 589 998; WO 04/071517; EP I 601 695; WO 05/079837; WO 06/068987; WO 07/027761; WO/2007/024846; WO 02/09748 and WO 06/069036; as well as the further prior art cited in these references and in the present specification. Some of the above references also describe antagonists of heterodimeric cytokines (such as conventional monoclonal antibodies) and mention diseases and disorders that can be prevented and/or treated by the use of such antagonists.

For the sequences of the various subunits of the heterodimeric cytokines and their receptors, reference is made to following Genbank accession numbers, which also mention additional information on as the pathways, signalling, biological mechanisms and biological effects in which they are involved, and also the diseases in which they are associated:
P19 : NM_016584
P40 : NM_002187
IL12Rbeta1 : NM_005535
IL23R NM_144701
IL12Rbeta2 NM_001559
WSX1: IL27RA: NM_004843
Gp130: NM_002184 and NM_175767
P35 (IL12A): NM_000882
P28: NM_145659
EBI3: NM_005755

From the above, it will also be clear that the aforementioned heterodimeric cytokines and their receptors have some subunits in common, with for example IL12p40 being present in both IL-12 and IL-23, and for example IL-12Rbeta1 being present in both the (cognate) receptor for IL-12 as well as the (cognate) receptor for IL-23. Also, some of the other subunits present in the heterodimeric cytokines or the receptors, although not identical, are structurally and/or functionally similar, and on the basis of these similarities can be grouped as follows:
- the p19, p35 and p28 subunits (and their present and future homologs), which will also be collectively referred to herein as the "*p19-like subunits*", it being understood that the p19-like subunit of IL-12 is p35, the p19-like subunit of IL-23 is p19, the p19-like subunit of IL-27 is p28, and the p19-like subunit of IL-35 is p35. P19-like subunits (such as the p35) are also homologous to type I cytokines, such as IL6 and oncostatin M, and for example share the "four helix bundle" that is common to type I cytokines;
- the p40 and EBI3 subunits (and their present and future homologs), which will also be collectively referred to herein as the "*p40-like subunits*", it being understood that the p40-like subunit of IL-12 is p40, the p40-like subunit of IL-23 is p40, the p40-like subunit of IL-27 is EBI3, and the p40-like subunit of IL-35 is EBI3. P40-like subunits (such as IL12p40) are structurally related to the soluble IL-6 receptor (IL-6Rα);
- the gp130 and IL-12beta-1 subunits (and their present and future homologs), which will also be collectively referred to herein as the "*gp130-like subunits*", it being understood that the gp130-like subunit of the IL-12 receptor is IL-12Rbeta-1, the gp130-like subunit *of the* IL-23 receptor is IL-12Rbeta-1, and the gp130-like subunit of the IL-27 receptor is gp130.
- the IL-12Rbeta-2, IL-23R and WSX-1 subunits (and their present and future homologs), which will also be collectively referred to herein as the "*IL-23 subunits*", it being understood that the IL-23 subunit of the IL-12 receptor is IL-12Rbeta-2, the IL-23-like subunit of the IL-23 receptor is IL-23R, and the IL-23-like subunit of the IL-27 receptor is WSX-1.

It is generally known that cytokines and their receptors are critical players in (the pathways) regulating all aspects of immune responses. This is also the case for the heterodimeric cytokines that belong to the interleukin-12 (IL12)-related family and for their receptors. For example, IL12 - the prototypical heterodimeric member of the IL-I2 family - induces interferon-□ (IFN-□) production by NK, T cells, dendritic cells (DC), and macrophages. IL-I2 also promotes the differentiation of naive CD4⁺ T cells into T helper 1 (T_{H}1) cells that produce IFN-□ and aid in cell-mediated immunity. Therefore the central role of IL12 in the generation of T_{H}1 cells (cell-mediated immune response) has long been appreciated. For example, mouse models established that IL12 is required for the development of protective innate and adaptive immune response to intracellular pathogens.

IL23 and IL27 - two of the other heterodimeric cytokines from the IL-12 family - also regulate T_{H}1-cell response, albeit with distinct functions. The ability of IL-23 to stimulate CD4⁺ T cells to produce IL-17 has a dominant role in the development and maintenance of autoimmune inflammation. By contrast, a principal function of IL-27 *in vivo* is to limit the intensity and duration of innate and adaptive immune responses.

In addition IL12p40 can be found as a monomers or homodimers which have antagonistic activities.

Recently, IL23 was shown to be responsible for the chronic inflammation observed in inflammatory bowel disease. This was confirmed by the fact that the IL23R gene was identified as being involved in inflammatory bowel disease. It has also been found that p19 knock out mice are resistant to collagen-induced arthritis and colitis, whereas comparable p35 knock out mice were found to be more susceptible to collagen-induced arthritis. Also, when p19 knock out mice were crossed with IL-10 knock out mice, the resulting offspring were resistant to colitis, whereas similar crosses of p19 knock out mice with IL-10 knock out mice resulted in offspring that was susceptible to colitis. It was further found that a monoclonal antibody against p19 inhibits the development of EAE, a preclinical animal model for multiple sclerosis, and reduces serum levels of IL-17 (which is not regulated by IL-12). Also, IL-23 rather than IL-12 appears to be the essential cytokine in CNS autoimmune inflammation. All this results suggests that IL-23/p19 may be a more attractive target for the treatment of colitis, Crohn's diseases, IBD, multiple sclerosis, rheumatoid arthritis and some of the other diseases and disorders mentioned herein that IL-12/p35 or p40 (as a compound directed against p40 will probably modulate both IL-12 and IL-23). It should also be noted that the monoclonal antibodies CNTO 1275 and ABT-874 (see below) that are currently under clinical development are both directed against p40. Thus, one specific object of the invention is to provide amino acid sequences and polypeptides that are directed against p19, and in particular amino acid sequences and polypeptides that are specific for (as defined herein) p19 compared to both p35 and p40 and/or that are specific for (as defined herein) IL-23 compared to IL-12. Examples of such amino acid sequences and polypeptides will become clear from the description herein.

As further described herein, the amino acid sequences, polypeptides and compositions of the present invention can generally be used to modulate (as defined herein) the signalling that is mediated by heterodimeric cytokines and/or their receptors, to modulate (as defined herein) the biological pathways in which heterodimeric cytokines and/or their receptors are involved, and/or to modulate (as defined herein) the biological mechanisms, responses and effects associated with heterodimeric cytokines, their receptors, such signalling and/or these pathways (all the foregoing is also collectively referred to herein as "*heterodimeric cytokine-mediated signalling*").

As such, the amino acid sequences, polypeptides and compositions of the present invention can generally be used to modulate the immune system and/or one or more specific immune responses in a subject to which one or more of the amino acid sequences, polypeptides and compositions of the present invention are administered (i.e. in therapeutically relevant amounts).

The term "heterodimeric cytokines" as used herein in its broadest sense generally includes any heterodimeric cytokine, i.e. a cytokine that comprises at least two, and more preferably only two, subunits.

In particular, the term "heterodimeric cytokine" as used herein encompasses heterodimeric cytokines that are associated with cell-mediated (T_{H}1) immunity, although the invention is its broadest sense is not limited thereto and also encompasses heterodimeric cytokines associated with humoral (T_{H}2) immunity.

According to one specific, but non-limiting aspect, the amino acid sequences and polypeptides of invention are directed against a heterodimeric cytokine that is chosen from heterodimeric cytokines that comprise a p40 subunit or p40-like subunit, such as a p40 subunit (present in for example IL-12 and IL-23) or Epstein-Barr virus (EBV)-induced molecule 3 (EBI3, present in for example IL-27 and IL-35).

According to another specific, but non-limiting aspect, the amino acid sequences and polypeptides of invention are directed against a heterodimeric cytokine that is chosen from heterodimeric cytokines that comprise a p19 subunit or a p19-like subunit, such as a p19 subunit (present in for example IL-23), a p35 subunit (present in for example IL-12 and IL-35), or a p28 subunit (present in for example IL-27) or a homolog thereof.

For example, the amino acid sequences and polypeptides of invention may be directed against a heterodimeric cytokine that will comprise at least one p19 subunit or p19-like subunit and at least one p40 subunit or p40-like subunit.

According to an even more specific, but non-limiting aspect, the amino acid sequences and polypeptides of invention are directed against a heterodimeric cytokine that is chosen from IL-12, IL-23, IL-27 and/or IL-35.

In one specific aspect, but non-limiting aspect, the Amino acid sequences and polypeptides of the invention are directed against IL-23 (i.e. against p40, p19 or both). Such amino acid sequences and polypeptides of the invention (as well as compositions comprising the same), can be used for preventing and treating disorders associated with IL-23.

In another specific aspect, but non-limiting aspect, the amino acid sequences and polypeptides of the invention are directed against IL-12 (i.e. against p40, p35 or both). Such amino acid sequences and polypeptides of the invention (as well as compositions comprising the same), may be as further described herein, and can be used for preventing and treating disorders associated with IL-12.

The amino acid sequences, polypeptides and compositions can be used to modulate (as defined herein, and for example as an agonist or an antagonist) heterodimeric cytokines and their receptors, and/or the signaling, pathways, biological mechanisms and effects in which these are involved.

The amino acid sequences and polypeptides that are antagonists of heterodimeric cytokines and their receptors (and/or of the signaling, pathways, biological mechanisms and effects in which these are involved) can also be used to reduce or inhibit the agonistic effects of heterodimeric cytokines.

More generally, the amino acid sequences (such as the p19+ sequences, p19-sequences, p40+ sequences, p40- sequences, p35 sequences, IL-27 sequences, IL-12Rb1 sequences, IL-12Rb2 sequences and IL-23 sequences described herein), polypeptides (such as the - for example multivalent, multispecific and/or biparatopic - constructs described herein that comprise at least one p19+ sequence, p19- sequence, p40+ sequence, p40- sequence, p35 sequence, IL-27 sequence, IL-12Rb1 sequence, IL-12Rb2 sequence and/or IL-23 sequence) and compositions of the present invention can be used for the prevention and treatment (as defined herein) of diseases and disorders associated with heterodimeric cytokines and their receptors (and/or with the signaling, pathways, biological mechanisms and effects in which these are involved). Generally, "diseases and disorders associated with heterodimeric cytokines and their receptors" can be defined as diseases and disorders that can be prevented and/or treated, respectively, by suitably administering to a subject in need thereof (i.e. having the disease or disorder or at least one symptom thereof and/or at risk of attracting or developing the disease or disorder) of either a polypeptide or composition *of the* invention (and in particular, of a pharmaceutically active amount thereof) and/or of a known active principle active against heterodimeric cytokines and/or their receptors or a biological pathway or mechanism in which heterodimeric cytokines and/or their receptors is involved (and in particular, of a pharmaceutically active amount thereof). Examples of such diseases and disorders associated with heterodimeric cytokines and their receptors will be clear to the skilled person based on the disclosure herein, and for example include the following diseases and disorders: inflammation and inflammatory disorders such as bowel diseases (colitis, Crohn'disease, IBD), infectious diseases, psioriasis, cancer, autoimmune diseases (such as MS), carcoidis, transplant rejection, cystic fibrosis, asthma, chronic obstructive pulmomary disease, rheumatoid arthritis, viral infection, common variable immunodeficiency, and the various diseases and disorders mentioned in the prior art cited herein. Based thereon, it will also be clear to the skilled person with heterodimeric cytokines (and/or receptors thereof) are involved in which specific diseases and disorders.

In particular, the polypeptides and compositions of the present invention can be used for the prevention and treatment of diseases and disorders associated with heterodimeric cytokines and their receptors which are characterized by excessive and/or unwanted signalling mediated by heterodimeric cytokines and/or their receptors or by the pathway(s) in which heterodimeric cytokines and/or their receptors is involved. Examples of such diseases and disorders associated with heterodimeric cytokines and their receptors will again be clear to the skilled person based on the disclosure herein. For this purpose, usually antagonists of heterodimeric cytokines and their receptors (and/or with the signaling, pathways, biological mechanisms and effects in which these are involved) will be used.

Agonists of heterodimeric cytokines and their receptors (and/or with the signaling, pathways, biological mechanisms and effects in which these are involved) can be used to stimulate or enhance one or more immune response in a human or animal, for example for the prevention and/or treatment of diseases that are characterized by a weakened immune system or that may occur as a result of having a weakened immune system. Reference is for example made to Hunter (supra), Table 1, which lists several mice knock-outs in which various heterodimeric cytokines and receptors thereof (and in particular subunits thereof) have been knock-out, and the inflammatory phenotypes associated therewith.

IL12p40 has also been shown to have an essential role in autoimmune inflammation as shown in disease model system as EAE (Experimental Allergic Encephalomyelitis) or CIA (Collagen-induced arthritis).

Thus, without being limited thereto, the amino acid sequences and polypeptides of the invention can for example be used to prevent and/or to treat all diseases and disorders that are currently being prevented or treated with active principles that can modulate heterodimeric cytokines and/or their receptors-mediated signalling, such as those mentioned in the prior art cited above (for example, the monoclonal antibody CNTO 1275 that is described in WO 02/09748 and WO 06/069036; ABT-874, a monoclonal against p40 that is being developed by Abbott; as well as the small molecule Apilimod®, Syntha Pharmaceuticals). It is also envisaged that the polypeptides of the invention can be used to prevent and/or to treat all diseases and disorders for which treatment with such active principles is currently being developed, has been proposed, or will be proposed or developed in future. In addition, it is envisaged that, because of their favourable properties as further described herein, the polypeptides of the present invention may be used for the prevention and treatment of other diseases and disorders than those for which these known active principles are being used or will be proposed or developed; and/or that the polypeptides of the present invention may provide new methods and regimens for treating the diseases and disorders described herein.

As will be clear from the further description herein, the amino acid sequences of the invention may be in a so-called "monovalent" format (i.e. comprising or essentially consisting of a single antigen binding domain or binding unit) or in a "multivalent" format (i.e. comprising or essentially consisting of two or more binding domains or binding units - which may be the same or different - that are linked to each other, optionally via one or more suitable linkers). As also further described herein, such multivalent amino acid sequences and polypeptides of the invention may for example, without limitation, be multispecific (such as bispecific or trispecific) or multiparatopic (such as biparatopic) constructs (or be both multiparatopic and multispecific, such as a biparatopic construct against the p19 subunit that contains a further binding domain for binding to a serum protein, as exemplified herein); and may for example be constructs that comprise at least two binding domains or binding units that are each directed towards a different epitope on the same subunit of a heterodimeric cytokine, constructs that comprise at least two binding domains or binding units that each have a different biological function (for example one binding domain that can block or inhibit receptor-ligand interaction, and one binding domain that does not block or inhibit receptor-ligand interaction), or constructs that comprise at least two binding domains or binding units that are each directed towards a different subunit of a heterodimeric cytokine.

Examples of such constructs will become clear from the further disclosure herein.

It will also become clear from the further description herein that such constructs can generally be provided (and in particular, purposefully designed for a specific biological action) by suitably linking (optionally via suitable linkers) or combining two or more "monovalent" amino acid sequences of the invention (or by suitably linking or combining nucleotide sequences encoding such monovalent amino acid sequences to provide a nucleic acid that encodes the desired multivalent construct, and then suitably expressing said multivalent construct). Thus, it should be clear that the invention not only makes available the monovalent and multivalent amino acid sequences and polypeptides described herein, but also provides - by making available the monovalent amino acid sequences and polypeptides described herein - the skilled person with a range of different binding domains and binding units that can be used as "building blocks" to provide a range of different multivalent, multispecific and/or multiparatopic (and in particular, biparatopic) constructs (which may have different binding affinities, avidities, specificities, potencies and/or efficacies), which through the use of suitable "building blocks" as described herein can be purposefully designed for use in different aspects of the invention (as further described herein).

Consequently, the use of the various monovalent amino acid sequences of the invention as "building blocks" for providing the proteins and polypeptides of the invention (or nucleotide sequences/nucleic acids encoding the same, which can then be expressed to provide such proteins and polypeptides) form an important aspect of the invention.

For this purpose and for the other purposes described herein, the invention in a particular aspect provides a number of different amino acid sequences that each can be used as a single binding domain or binding unit, either as such (i.e. as a monovalent amino acid sequence as further described herein) or as part of (and/or as a "building block") for, a multivalent, nmltispecific and/or multispecific construct, as further described herein.

In their monovalent form, these amino acid sequences may for example be classified as follows:
- "p19 sequences", i.e. amino acid sequences of the invention that are directed against (as defined herein) the p19 subunit (as present in for example IL-23). The p19 sequences described herein may be further subdivided into "p19+ sequences" (i.e. p19 sequences that are directed against the p19 subunt and that are capable of modulating, neutralizing, blocking and/or inhibiting the binding of a heterodimeric cytokine comprising a p19 subunit to its (cognate) receptor, and in particular capable of are capable of modulating, neutralizing, blocking and/or inhibiting the binding of IL-23 to its (cognate) receptor, for example in the alpha-screen assay of Example 19 or 22) and "p19- sequences" (i.e. p19 sequences that are directed against the p19 subunit but that (per se/as such) are (essentially) not capable of modulating, neutralizing, blocking and/or inhibiting the binding of a heterodimeric cytokine comprising a p19 subunit to its (cognate) receptor);
- "*p40 sequences*", i.e. amino acid sequences of the invention that are directed against (as defined herein) the p40 subunit (as present in for example IL-12 and IL-23). The p40 sequences described herein may be further subdivided into "p40+ sequences" (i.e. p40 sequences that are directed against the p40 subunt and that are capable of modulating, neutralizing, blocking and/or inhibiting the binding of a heterodimeric cytokine comprising a p40 subunit to its (cognate) receptor, and in particular capable of are capable of modulating, neutralizing, blocking and/or inhibiting the binding of IL-23 to its (cognate) receptor, for example in the alpha-screen assay of Example 19 or 22), and/or of modulating, neutralizing, blocking and/or inhibiting the binding of IL-12 to its (cognate) receptor) and "p40- sequences" (i.e. p40 sequences that are directed against the p40 subunit but that (per se/as such) are (essentially) not capable of modulating, neutralizing, blocking and/or inhibiting the binding of a heterodimeric cytokine comprising a p40 subunit to its (cognate) receptor);
- "*p35 sequences*", i.e. amino acid sequences of the invention that are directed against (as defined herein) the p35 subunit (as present in for example IL-12);
- "*IL-27 sequences*", i.e. amino acid sequences of the invention that are directed against (as defined herein) IL-27. The IL-27 sequences described herein may be directed against the EBI-3 subunit or against the IL-27p28 subunit;
- *"IL-12Rb1 sequences",* i.e. amino acid sequences of the invention that are directed against the Rbeta1 subunit ofIL-12R and/or of IL-23R;
- *"IL-12Rb2 sequences",* i.e. amino acid sequences of the invention that are directed against the Rbeta2 subunit of IL-12R)
- *"IL-23R sequences",* i.e. amino acid sequences of the invention that are directed against the IL-23R subunit of the IL-23 receptor.

Each of the p19+ sequences, p19- sequences, p40+ sequences, p40- sequence, p35 sequences, IL-27 sequences, IL-12Rb1 sequences, IL-12Rb2 sequences and IL-23R sequences may be as further described herein and each class of amino acid sequences of the invention forms a further aspect of the invention.

The invention also relates to the use of such p19+ sequences, p19-sequences, p40+ sequences, p40- sequences, p35 sequences, IL-27 sequences, IL-12Rb1 sequences, IL-12Rb2 sequences and IL-23R sequences (and/or of nucleotide sequences and/or nucleic acids encoding the same) as "building blocks" (i.e. as single antigen binding domains or units) in or for multivalent, multispecific and/or multiparatopic constructs, as further described herein.

In this respect, it should for example be noted that amino acid sequences of the invention that are not capable of modulating, neutralizing, blocking and/or inhibiting the binding of a heterodimeric cytokine to its cognate receptor (such as the p19- sequences or p40- sequences) may still find use as binding domains and/or binding units in multivalent, multispecific and/or multiparatopic polypeptides of the invention, for example in order to provide/improve specificity and/or to provide/improve affinity and/or avidity. Examples thereof will become clear to the skilled person from the disclosure herein.

Thus, further aspects of the invention relate to:
- the use of an amino acid sequence of the invention that comprises or essentially consists of a single binding domain or binding unit (as defined herein) in providing, constructing, and/or as part of a multivalent, multispecific and/or multiparatopic construct, protein and/or polypeptide that comprises said amino acid sequence of the invention (one or more) and at least one further binding domain or binding unit;
- the use of a nucleotide sequence and/or nucleic acid that encodes an amino acid sequence of the invention that comprises or essentially consists of a single binding domain or binding unit (as defined herein) in providing, constructing, and/or as part of a nucleotide sequence and/or nucleic acid that encodes a multivalent, multispecific and/or multiparatopic construct, protein and/or polypeptide that comprises said amino acid sequence of the invention (one or more) and at least one further binding domain or binding unit;
- the use of an amino acid sequence of the invention that comprises or essentially consists of a single binding domain or binding unit (as defined herein) in providing, constructing, and/or as part of a biparatopic construct, protein and/or polypeptide that comprises said amino acid sequence of the invention (one or more) and at least one further binding domain or binding unit;
- the use of a nucleotide sequence and/or nucleic acid that encodes an amino acid sequence of the invention that comprises or essentially consists of a single binding domain or binding unit (as defined herein) in providing, constructing, and/or as part of a nucleotide sequence and/or nucleic acid that encodes a biparatopic construct, protein and/or polypeptide that comprises said amino acid sequence of the invention (one or more) and at least one further binding domain or binding unit;
- the use of an amino acid sequence of the invention that comprises or essentially consists of a single binding domain or binding unit (as defined herein) in providing, constructing, and/or as part of a multispecific (and in particular, bispecific) construct, protein and/or polypeptide that is directed against a heterodimeric protein, polypeptide, ligand or receptor (or other heterodimeric "target"), wherein said construct, protein and/or polypeptide comprises said amino acid sequence of the invention (one or more) and at least one further binding domain or binding unit, and wherein said one or more amino acid sequences of the invention are directed against a first subunit of the heterodimeric protein, polypeptide, ligand or receptor and at least one of said further binding domains or binding units is directed against a second subunit of the heterodimeric protein, polypeptide, ligand or receptor different from the first;
- the use of a nucleotide sequence and/or nucleic acid that encodes an amino acid sequence of the invention that comprises or essentially consists of a single binding domain or binding unit (as defined herein) in providing, constructing, and/or as part of a nucleotide sequence and/or nucleic acid that encodes a multispecific (and in particular, bispecific) construct, protein and/or polypeptide that is directed against a heterodimeric protein, polypeptide, ligand or receptor (or other heterodimeric "target"), wherein said construct, protein and/or polypeptide comprises said amino acid sequence of the invention (one or more) and at least one further binding domain or binding unit, and wherein said one or more amino acid sequences of the invention are directed against a first subunit of the heterodimeric protein, polypeptide, ligand or receptor and at least one of said further binding domains or binding units is directed against a second subunit of the heterodimeric protein, polypeptide, ligand or receptor different from the first;
- the use of an amino acid sequence of the invention that comprises or essentially consists of a single binding domain or binding unit (as defined herein) in providing, constructing and/or as part of a multivalent, multispecific and/or multiparatopic construct, protein and/or polypeptide that is directed against a heterodimeric cytokine or a (cognate) receptor for a heterodimeric cytokine and that comprises said amino acid sequence of the invention and at least one further binding domain or binding unit;
- the use of a nucleotide sequence and/or nucleic acid that encodes an amino acid sequence of the invention that comprises or essentially consists of a single binding domain or binding unit (as defined herein) in providing, constructing and/or as part of a nucleotide sequence and/or nucleic acid that encodes a multivalent, multispecific and/or multiparatopic construct, protein and/or polypeptide that is directed against a heterodimeric cytokine or a (cognate) receptor for a heterodimeric cytokine and that comprises said amino acid sequence of the invention and at least one further binding domain or binding unit;
- the use of an amino acid sequence of the invention that comprises or essentially consists of a single binding domain or binding unit (as defined herein) in providing, constructing and/or as part of a biparatopic construct, protein and/or polypeptide that is directed against (a subunit of) a heterodimeric cytokine or (a subunit of) a (cognate) receptor for a heterodimeric cytokine and that comprises said amino acid sequence of the invention and at least one further binding domain or binding unit;
- the use of a nucleotide sequence and/or nucleic acid that encodes an amino acid sequence of the invention that comprises or essentially consists of a single binding domain or binding unit (as defined herein) in providing, constructing and/or as part of a nucleotide sequence and/or nucleic acid that encodes a biparatopic construct, protein and/orpolypeptide that is directed against (a subunit of) a heterodimeric cytokine or (a subunit of) a (cognate) receptor for a heterodimeric cytokine and that comprises said amino acid sequence of the invention and at least one further binding domain or binding unit;
- the use of an amino acid sequence of the invention that comprises or essentially consists of a single binding domain or binding unit (as defined herein) in providing, constructing, and/or as part of a multispecific (and in particular, bispecific) construct, protein and/or polypeptide that is directed against a heterodimeric cytokine, wherein said construct, protein and/or polypeptide comprises said amino acid sequence of the invention (one or more) and at least one further binding domain or binding unit, and wherein said one or more amino acid sequences of the invention are directed against a first subunit of the heterodimeric cytokine and at least one of said further binding domains or binding units is directed against a second subunit of the same heterodimeric cytokine (i.e. different from the first subunit);
- the use of a nucleotide sequence and/or nucleic acid that encodes an amino acid sequence of the invention that comprises or essentially consists of a single binding domain or binding unit (as defined herein) in providing, constructing, and/or as part of a nucleotide sequence and/or nucleic acid that encodes a multispecific (and in particular, bispecific) construct, protein and/or polypeptide that is directed against a heterodimeric cytokine, wherein said construct, protein and/or polypeptide comprises said amino acid sequence of the invention (one or more) and at least one further binding domain or binding unit, and wherein said one or more amino acid sequences of the invention are directed against a first subunit of the heterodimeric cytokine and at least one of said further binding domains or binding units is directed against a second subunit of the same heterodimeric cytokine (i.e. different from the first submit);
- the use of a p19+ sequence (as defined herein) in providing, constructing, and/or as part of a multivalent, multispecific and/or multiparatopic construct, protein and/or polypeptide that comprises said p19+ sequence (one or more) and one or more further binding domains or binding units;
- the use of a nucleotide sequence and/or nucleic acid that encodes a p19+ sequence (as defined herein) in providing, constructing, and/or as part of a nucleotide sequence and/or nucleic acid that encodes a multivalent, multispecific and/or multiparatopic construct, protein and/or polypeptide that comprises said p19+ sequence (one or more) and one or more further binding domains or binding units;
- the use of a p19+ sequence (as defined herein) in providing, constructing, and/or as part of a biparatopic construct, protein and/or polypeptide that is directed against p19 and/or against a heterodimeric cytokine that comprises a p19 subunit (such as IL-23) and that comprises said p19+ sequence (one or more) and at least one further binding domain or binding unit that is also directed against p19 (but to a different epitope or antigenic determinant of p19), and optionally one or more further binding domains or binding units;
- the use of a nucleotide sequence and/or nucleic acid that encodes a p19+ sequence (as defined herein) in providing, constructing, and/or as part of a nucleotide sequence and/or nucleic acid that encodes a biparatopic construct, protein and/or polypeptide that is directed against p19 and/or against a heterodimeric cytokine that comprises a p19 subunit (such as IL-23) and that comprises said p19+ sequence (one or more) and at least one further binding domain or binding unit that is also directed against p19 (but to a different epitope or antigenic determinant of p19), and optionally one or more further binding domains or binding units;
- the use of a p19+ sequence (as defined herein) in providing, constructing, and/or as part of a multispecific (and in particular, bispecific) construct, protein and/or polypeptide that is directed against a heterodimeric cytokine that comprises a p19 subunit (such as IL-23), wherein said construct, protein and/or polypeptide comprises said p19+ sequence (one or more) and one or more further binding domains or binding units, and wherein at least one of said further binding domains or binding units is directed against a second subunit of the said heterodimeric cytokine different from p29 (such as p40 in IL-23);
- the use of a nucleotide sequence and/or nucleic acid that encodes a p19+ sequence (as defined herein) in providing, constructing, and/or as part of a nucleotide sequence and/or nucleic acid that encodes a multispecific (and in particular, bispecific) construct, protein and/or polypeptide that is directed against a heterodimeric cytokine that comprises a p19 subunit (such as IL-23), wherein said construct, protein and/or polypeptide comprises said p19+ sequence (one or more) and one or more further binding domains or binding units, and wherein at least one of said further binding domains or binding units is directed against a second subunit of the said heterodimeric cytokine different from p19 (such as p40 in IL-23);
- the use of a p19- sequence (as defined herein) in providing, constructing, and/or as part of a multivalent, multispecific and/or multiparatopic construct, protein and/or polypeptide that comprises said p 19- sequence (one or more) and one or more further binding domains or binding units;
- the use of a nucleotide sequence and/or nucleic acid that encodes a p19- sequence (as defined herein) in providing, constructing, and/or as part of a nucleotide sequence and/or nucleic acid that encodes a multivalent, multispecific and/or multiparatopic construct, protein and/or polypeptide that comprises said p19- sequence (one or more) and one or more further binding domains or binding units;
- the use of a p19- sequence (as defined herein) in providing, constructing, and/or as part of a biparatopic construct, protein and/or polypeptide that is directed against p 19 and/or against a heterodimeric cytokine that comprises a p19 subunit (such as IL-23) and that comprises said p19- sequence (one or more) and at least one further binding domain or binding unit that is also directed against p19 (but to a different epitope or antigenic determinant of p19), and optionally one or more further binding domains or binding units;
- the use of a nucleotide sequence and/or nucleic acid that encodes a p19- sequence (as defined herein) in providing, constructing, and/or as part of a nucleotide sequence and/or nucleic acid that encodes a biparatopic construct, protein and/or polypeptide that is directed against p19 and/or against a heterodimeric cytokine that comprises a p19 subunit (such as IL-23) and that comprises said p19- sequence (one or more) and at least one further binding domain or binding unit that is also directed against p19 (but to a different epitope or antigenic determinant of p19), and optionally one or more further binding domains or binding units;
- the use of a p19- sequence (as defined herein) in providing, constructing, and/or as part of a multispecific (and in particular, bispecific) construct, protein and/or polypeptide that is directed against a heterodimeric cytokine that comprises a p19 subunit (such as IL-23), wherein said construct, protein and/or polypeptide comprises said p19-sequence (one or more) and one or more further binding domains or binding units, and wherein at least one of said further binding domains or binding units is directed against a second subunit of the said heterodimeric cytokine different from p19 (such as p40 in IL-23);
- the use of a nucleotide sequence and/or nucleic acid that encodes a p19- sequence (as defined herein) in providing, constructing, and/or as part of a nucleotide sequence and/or nucleic acid that encodes a multispecific (and in particular, bispecific) construct, protein and/or polypeptide that is directed against a heterodimeric cytokine that comprises a p19 subunit (such as IL-23), wherein said construct, protein and/or polypeptide comprises said p 19- sequence (one or more) and one or more further binding domains or binding units, and wherein at least one of said further binding domains or binding units is directed against a second subunit of the said heterodimeric cytokine different from p19 (such as p40 in IL-23);
- the use of a p40+ sequence (as defined herein) in providing, constructing, and/or as part of a multivalent, multispecific and/or multiparatopic construct, protein and/or polypeptide that comprises said p40+ sequence (one or more) and one or more further binding domains or binding units;
- the use of a nucleotide sequence and/or nucleic acid that encodes a p40+ sequence (as defined herein) in providing, constructing, and/or as part of a nucleotide sequence and/or nucleic acid that encodes a multivalent, multispecific and/or multiparatopic construct, protein and/or polypeptide that comprises said p40+ sequence (one or more) and one or more further binding domains or binding units;
- the use of a p40+ sequence (as defined herein) in providing, constructing, and/or as part of a biparatopic construct, protein and/or polypeptide that is directed against p40 and/or against a heterodimeric cytokine that comprises a p40 subunit (such as IL-23 or IL-12) and that comprises said p40+ sequence (one or more) and at least one further binding domain or binding unit that is also directed against p40 (but to a different epitope or antigenic determinant of p40), and optionally one or more further binding domains or binding units;
- the use of a nucleotide sequence and/or nucleic acid that encodes a p40+ sequence (as defined herein) in providing, constructing, and/or as part of a nucleotide sequence and/or nucleic acid that encodes a biparatopic construct, protein and/or polypeptide that is directed against p40 and/or against a heterodimeric cytokine that comprises a p40 subunit (such as IL-23) and that comprises said p40+ sequence (one or more) and at least one further binding domain or binding unit that is also directed against p40 (but to a different epitope or antigenic determinant of p40), and optionally one or more further binding domains or binding units;
- the use of a p40+ sequence (as defined herein) in providing, constructing, and/or as part of a multispecific (and in particular, bispecific) construct, protein and/or polypeptide that is directed against a heterodimeric cytokine that comprises a p40 subunit (such as IL-23), wherein said construct, protein and/or polypeptide comprises said p40+ sequence (one or more) and one or more further binding domains or binding units, and wherein at least one of said further binding domains or binding units is directed against a second subunit of the said heterodimeric cytokine different from p40 (such as p19 in IL-23 or p35 in IL-12);
- the use of a nucleotide sequence and/or nucleic acid that encodes a p40+ sequence (as defined herein) in providing, constructing, and/or as part of a nucleotide sequence and/or nucleic acid that encodes a multispecific (and in particular, bispecific) construct, protein and/or polypeptide that is directed against a heterodimeric cytokine that comprises a p40 subunit (such as IL-23 or IL-12), wherein said construct, protein and/or polypeptide comprises said p40+ sequence (one or more) and one or more further binding domains or binding units, and wherein at least one of said further binding domains or binding units is directed against a second subunit of the said heterodimeric cytokine different from p40 (such as p40 in IL-23 or p35 in IL-12);
- the use of a p40- sequence (as defined herein) in providing, constructing, and/or as part of a multivalent, multispecific and/or multiparatopic construct, protein and/or polypeptide that comprises said p40- sequence (one or more) and one or more further binding domains or binding units;
- the use of a nucleotide sequence and/or nucleic acid that encodes a p40- sequence (as defined herein) in providing, constructing, and/or as part of a nucleotide sequence and/or nucleic acid that encodes a multivalent, multispecific and/or multiparatopic construct, protein and/or polypeptide that comprises said p40- sequence (one or more) and one or more further binding domains or binding units;
- the use of a p40- sequence (as defined herein) in providing, constructing, and/or as part of a biparatopic construct, protein and/or polypeptide that is directed against p40 and/or against a heterodimeric cytokine that comprises a p40 subunit (such as IL-23 or IL-12) and that comprises said p40- sequence (one or more) and at least one further binding domain or binding unit that is also directed against p40 (but to a different epitope or antigenic determinant of p40), and optionally one or more further binding domains or binding units;
- the use of a nucleotide sequence and/or nucleic acid that encodes a p40- sequence (as defined herein) in providing, constructing, and/or as part of a nucleotide sequence and/or nucleic acid that encodes a biparatopic construct, protein and/or polypeptide that is directed against p40 and/or against a heterodimeric cytokine that comprises a p40 subunit (such as IL-23 or IL-12) and that comprises said p40- sequence (one or more) and at least one further binding domain or binding unit that is also directed against p40 (but to a different epitope or antigenic determinant of p40), and optionally one or more further binding domains or binding units;
- the use of a p40- sequence (as defined herein) in providing, constructing, and/or as part of a multispecific (and in particular, bispecific) construct, protein and/or polypeptide that is directed against a heterodimeric cytokine that comprises a p40 subunit (such as IL-23 or IL-12), wherein said construct, protein and/or polypeptide comprises said p40-sequence (one or more) and one or more further binding domains or binding units, and wherein at least one of said further binding domains or binding units is directed against a second subunit of the said heterodimeric cytokine different from p40 (such as p19 in IL-23 or p35 in IL-12);
- the use of a nucleotide sequence and/or nucleic acid that encodes a p40- sequence (as defined herein) in providing, constructing, and/or as part of a nucleotide sequence and/or nucleic acid that encodes a multispecific (and in particular, bispecific) construct, protein and/or polypeptide that is directed against a heterodimeric cytokine that comprises a p40 subunit (such as IL-23 or IL-12), wherein said construct, protein and/or polypeptide comprises said p40- sequence (one or more) and one or more further binding domains or binding units, and wherein at least one of said further binding domains or binding units is directed against a second subunit of the said heterodimeric cytokine different from p40 (such as p40 in IL-23 or p35 in IL-12);
- the use of a p35 sequence (as defined herein) in providing, constructing, and/or as part of a multivalent, multispecific and/or multiparatopic construct, protein and/or polypeptide that comprises said p35 sequence (one or more) and one or more further binding domains or binding units;
- the use of a nucleotide sequence and/or nucleic acid that encodes a p35 sequence (as defined herein) in providing, constructing, and/or as part of a nucleotide sequence and/or nucleic acid that encodes a multivalent, multispecific and/or multiparatopic construct, protein and/or polypeptide that comprises said p35 sequence (one or more) and one or more further binding domains or binding units;
- the use of a p35 sequence (as defined herein) in providing, constructing, and/or as part of a biparatopic construct, protein and/or polypeptide that is directed against p35 and/or against a heterodimeric cytokine that comprises a p35 subunit (such as IL-12) and that comprises said p35 sequence (one or more) and at least one further binding domain or binding unit that is also directed against p35 (but to a different epitope or antigenic determinant of p35), and optionally one or more further binding domains or binding units;
- the use of a nucleotide sequence and/or nucleic acid that encodes a p35 sequence (as defined herein) in providing, constructing, and/or as part of a nucleotide sequence and/or nucleic acid that encodes a biparatopic construct, protein and/or polypeptide that is directed against p35 and/or against a heterodimeric cytokine that comprises a p35 subunit (such as IL-12) and that comprises said p35 sequence (one or more) and at least one further binding domain or binding unit that is also directed against p35 (but to a different epitope or antigenic determinant of p35), and optionally one or more further binding domains or binding units;
- the use of a p35 sequence (as defined herein) in providing, constructing, and/or as part of a multispecific (and in particular, bispecific) construct, protein and/or polypeptide that is directed against a "heterodimeric cytokine that comprises a p35 subunit (such as IL-12), wherein said construct, protein and/or polypeptide comprises said p35 sequence (one or more) and one or more further binding domains or binding units, and wherein at least one of said further binding domains or binding units is directed against a second subunit of the said heterodimeric cytokine different from p3S (such as p40 in IL-12);
- the use of a nucleotide sequence and/or nucleic acid that encodes a p35 sequence (as defined herein) in providing, constructing, and/or as part of a nucleotide sequence and/or nucleic acid that encodes a multispecific (and in particular, bispecific) construct, protein and/or polypeptide that is directed against a heterodimeric cytokine that comprises a p35 subunit (such as IL-12), wherein said construct, protein and/or polypeptide comprises said p35 sequence (one or more) and one or more further binding domains or binding units, and wherein at least one of said further binding domains or binding units is directed against a second subunit of the said heterodimeric cytokine different from p35 (such as p40 in IL-12);
- the use of an IL-27 sequence (as defined herein) in providing, constructing, and/or as part of a multivalent, multi specific and/or multiparatopic construct, protein and/or polypeptide that comprises said IL-27 sequence (one or more) and one or more further binding domains or binding units;
- the use of a nucleotide sequence and/or nucleic acid that encodes an IL-27 sequence (as defined herein) in providing, constructing, and/or as part of a nucleotide sequence and/or nucleic acid that encodes a multivalent, multispecific and/or multiparatopic construct, protein and/or polypeptide that comprises said IL-27 sequence (one or more) and one or more further binding domains or binding units;
- the use of an IL-27 sequence (as defined herein) in providing, constructing, and/or as part of a biparatopic construct, protein and/or polypeptide that is directed against IL-27 and that comprises said IL-27 sequence (one or more) and at least one further binding domain or binding unit that is also directed against IL-27 (but to a different epitope or antigenic determinant of IL-27), and optionally one or more further binding domains or binding units;
- the use of a nucleotide sequence and/or nucleic acid that encodes a IL-27 sequence (as defined herein) in providing, constructing, and/or as part of a nucleotide sequence and/or nucleic acid that encodes a biparatopic construct, protein and/or polypeptide that is directed against IL-27 and that comprises said IL-27 sequence (one or more) and at least one further binding domain or binding unit that is also directed against IL-27 (but to a different epitope or antigenic determinant of IL-27), and optionally one or more further binding domains or binding units;
- the use of an IL-27 sequence (as defined herein) in providing, constructing, and/or as part of a multispecific (and in particular, bispecific) construct, protein and/or polypeptide that is directed against IL-27, wherein said construct, protein and/or polypeptide comprises said IL-27 sequence (one or more) and one or more further binding domains or binding units, and wherein at least one of said further binding domains or binding units is directed against a another subunit of IL-27 different from the subunit against which said IL-27 sequence is directed;
- the use of a nucleotide sequence and/or nucleic acid that encodes an IL-27 sequence (as defined herein) in providing, constructing, and/or as part of a nucleotide sequence and/or nucleic acid that encodes a multispecific (and in particular, bispecific) construct, protein and/or polypeptide that is directed against IL-27, wherein said construct, protein and/or polypeptide comprises said IL-27 sequence (one or more) and one or more further binding domains or binding units, and wherein at least one of said further binding domains or binding units is directed against another subunit of IL-27 different from the subunit against which said IL-27 sequence is directed;
- the use of an IL-12RB1 sequence (as defined herein) in providing, constructing, and/or as part of a multivalent, multispecific and/or multiparatopic construct, protein and/or polypeptide that comprises said IL-12RB1 sequence (one or more) and one or more further binding domains or binding units;
- the use of a nucleotide sequence and/or nucleic acid that encodes an IL-12RB1 sequence (as defined herein) in providing, constructing, and/or as part of a nucleotide sequence and/or nucleic acid that encodes a multivalent, multispecific and/or multiparatopic construct, protein and/or polypeptide that comprises said IL-12RB1 sequence (one or more) and one or more further binding domains or binding units;
- the use of an IL-12RB1 sequence (as defined herein) in providing, constructing, and/or as part of a biparatopic construct, protein and/or polypeptide that is directed against IL-12RB1 and/or against a receptor that comprises an IL-12Rb1 subunit (such as the cognate receptors for IL-12 and IL-23) and that comprises said IL-12RB1 sequence (one or more) and at least one further binding domain or binding unit that is also directed against IL-12RB1 (but to a different epitope or antigenic determinant of IL-12RB1), and optionally one or more further binding domains or binding units;
- the use of a nucleotide sequence and/or nucleic acid that encodes a IL-12RB1 sequence (as defined herein) in providing, constructing, and/or as part of a nucleotide sequence and/or nucleic acid that encodes a biparatopic construct, protein and/or polypeptide that is directed against IL-12RB1 and/or against a receptor that comprises an IL-12Rb1 subunit (such as the cognate receptors for IL-12 and IL-23) and that comprises said IL-12RB1 sequence (one or more) and at least one further binding domain or binding unit that is also directed against IL-12RB1 (but to a different epitope or antigenic determinant of IL-12RB1), and optionally one or more further binding domains or binding units;
- the use of an IL-12RB1 sequence (as defined herein) in providing, constructing, and/or as part of a multispecific (and in particular, bispecific) construct, protein and/or polypeptide that is directed against a heterodimeric receptor that comprises an IL-12RB1 subunit (such as the cognate receptors for 1L-12 and IL-23), wherein said construct, protein and/or polypeptide comprises said IL-12RB1 sequence (one or more) and one or more further binding domains or binding units, and wherein at least one of said further binding domains or binding units are directed against another subunit of said heterodimeric receptor different from IL-12RB1;
- the use of a nucleotide sequence and/or nucleic acid that encodes an IL-12RB1 sequence (as defined herein) in providing, constructing, and/or as part of a nucleotide sequence and/or nucleic acid that encodes a multispecific (and in particular, bispecific) construct, protein and/or polypeptide that is directed against a heterodimeric receptor that comprises an IL-12RR1 subunit (such as the cognate receptors for IL-12 and IL-23), wherein said construct, protein and/or polypeptide comprises said IL-12RB1 sequence (one or more) and one or more further binding domains or binding units, and wherein at least one of said further binding domains or binding units are directed against another subunit of said heterodimeric receptor different from IL-12RB1;
- the use of an IL-12RB2 sequence (as defined herein) in providing, constructing, and/or as part of a multivalent, multispecific and/or multiparatopic construct, protein and/or polypeptide that comprises said IL-12RB2 sequence (one or more) and one or more further binding domains or binding units;
- the use of a nucleotide sequence and/or nucleic acid that encodes an 1L-12RB2 sequence (as defined herein) in providing, constructing, and/or as part of a nucleotide sequence and/or nucleic acid that encodes a multivalent, multispecific and/or multiparatopic construct, protein and/or polypeptide that comprises said IL-12RB2 sequence (one or more) and one or more further binding domains or binding units;
- the use of an IL-12RB2 sequence (as defined herein) in providing, constructing, and/or as part of a biparatopic construct, protein and/or polypeptide that is directed against IL-12RB2 and/or against a receptor that comprises an IL-12Rb2 subunit (such as the cognate receptor for IL-12) and that comprises said IL-12RB2 sequence (one or more) and at least one further binding domain or binding unit that is also directed against IL-12RB2 (but to a different epitope or antigenic determinant of IL-12RB2), and optionally one or more further binding domains or binding units;
- the use of a nucleotide sequence and/or nucleic acid that encodes a IL-12RB2 sequence (as defined herein) in providing, constructing, and/or as part of a nucleotide sequence and/or nucleic acid that encodes a biparatopic construct, protein and/or polypeptide that is directed against IL-12RB2 and/or against a receptor that comprises an IL-12Rb2 subunit (such as the cognate receptor for IL-12) and that comprises said IL-12RB2 sequence (one or more) and at least one further binding domain or binding unit that is also directed against IL-12RB2 (but to a different epitope or antigenic determinant of IL-12RB2), and optionally one or more further binding domains or binding units;
- the use of an IL-12RB2 sequence (as defined herein) in providing, constructing, and/or as part of a multispecific (and in particular, bispecific) construct, protein and/or polypeptide that is directed against a heterodimeric receptor that comprises an IL-12RB2 subunit (such as the cognate receptor for IL-12), wherein said construct, protein and/or polypeptide comprises said IL-12RB2 sequence (one or more) and one or more further binding domains or binding units, and wherein at least one of said further binding domains or binding units are directed against another subunit of said heterodimeric receptor different from IL-12RB2;
- the use of a nucleotide sequence and/or nucleic acid that encodes an IL-12RB2 sequence (as defined herein) in providing, constructing, and/or as part of a nucleotide sequence and/or nucleic acid that encodes a multispecific (and in particular, bispecific) construct, protein and/or polypeptide that is directed against a heterodimeric receptor that comprises an IL-12RB2 subunit (such as the cognate receptor for IL-12), wherein said construct, protein and/or polypeptide comprises said IL-12RB2 sequence (one or more) and one or more further binding domains or binding units, and wherein at least one of said further binding domains or binding units are directed against another subunit of said heterodimeric receptor different from IL-12RB2;
- the use of an IL-23R sequence (as defined herein) in providing, constructing, and/or as part of a multivalent, multispecific and/or multiparatopic construct, protein and/or polypeptide that comprises said IL-23R sequence (one or more) and one or more further binding domains or binding units;
- the use of a nucleotide sequence and/or nucleic acid that encodes an IL-23R sequence (as defined herein) in providing, constructing, and/or as part of a nucleotide sequence and/or nucleic acid that encodes a multivalent, multispecific and/or multiparatopic construct, protein and/or polypeptide that comprises said IL-23R sequence (one or more) and one or more further binding domains or binding units;
- the use of an IL-23R sequence (as defined herein) in providing, constructing, and/or as part of a biparatopic construct, protein and/or polypeptide that is directed against IL-23R and/or against a receptor that comprises an IL-23R subunit (such as the cognate receptor for IL-23) and that comprises said IL-23R sequence (one or more) and at least one further binding domain or binding unit that is also directed against IL-23R (but to a different epitope or antigenic determinant of IL-23R), and optionally one or more further binding domains or binding units;
- the use of a nucleotide sequence and/or nucleic acid that encodes a IL-23R sequence (as defined herein) in providing, constructing, and/or as part of a nucleotide sequence and/or nucleic acid that encodes a biparatopic construct, protein and/or polypeptide that is directed against IL-23R and/or against a receptor that comprises an IL-23R subunit (such as the cognate receptor for IL-23) and that comprises said IL-23R sequence (one or more) and at least one further binding domain or binding unit that is also directed against IL-23R (but to a different epitope or antigenic determinant of IL-23R), and optionally one or more further binding domains or binding units;
- the use of an IL-23R sequence (as defined herein) in providing, constructing, and/or as part of a multispecific (and in particular, bispecific) construct, protein and/or polypeptide that is directed against a heterodimenc receptor that comprises an IL-23R subunit (such as the cognate receptor for IL-23), wherein said construct, protein and/or polypeptide comprises said IL-23R sequence (one or more) and one or more further binding domains or binding units, and wherein at least one of said further binding domains or binding units are directed against another subunit of said heterodimeric receptor different from IL-23R;
- the use of a nucleotide sequence and/or nucleic acid that encodes an IL-23R sequence (as defined herein) in providing, constructing, and/or as part of a nucleotide sequence and/or nucleic acid that encodes a multispecific (and in particular, bispecific) construct, protein and/or polypeptide that is directed against a heterodimeric receptor that comprises an IL-23R subunit (such as the cognate receptor for IL-23), wherein said construct, protein and/or polypeptide comprises said IL-23R sequence (one or more) and one or more further binding domains or binding units, and wherein at least one of said further binding domains or binding units are directed against another subunit of said heterodimeric receptor different from IL-23R.

Where any of the above aspects/uses comprises the use of a nucleotide sequence and/or nucleic acid that encodes a monovalent amino acid sequence in in providing, constructing, and/or as part of a nucleotide sequence and/or nucleic acid that encodes a multivalent, multispecific and/or multiparatopic constructs (such as a biparatopic construct), said aspect/use optionally further comprises the use of the a nucleotide sequence and/or nucleic acid thus obtained in preparing (e.g. by suitable expression, as further described herein) the multivalent, multispecific and/or multiparatopic construct encoded by said nucleotide sequence and/or nucleic acid.

More generally, in one aspect of the invention, there is provided a ("multispecific", as defined herein) polypeptide construct that is directed against (as defined herein) a heterodimeric protein, polypeptide, ligand or receptor (or other "target") that comprises:
- at least a first subunit;
   and
- at least a second subunit;
wherein said polypeptide construct at least comprises a first binding domain or binding unit that is directed against said first subunit and a second binding domain or binding unit that is directed against said second subunit.

In particular, in this aspect of the invention, there is provided a ("multispecific", as defined herein) polypeptide construct that is directed against (as defined herein) a first heterodimeric protein, polypeptide, ligand or receptor that comprises:
- at least a first subunit that is shared between said first heterodimeric protein, polypeptide, ligand or receptor and at least a second, different heterodimeric protein, polypeptide, ligand or receptor;
   and
- at least a second subunit that is not shared between said first heterodimeric protein, polypeptide, ligand or receptor and said second, different heterodimeric protein, polypeptide, ligand or receptor;
wherein said polypeptide construct at least comprises a first binding domain or binding unit that is directed against said first (i.e. shared) subunit and a second binding domain or binding unit that is directed against said second (i.e. not shared) subunit.

In another aspect of the invention, there is provided a polypeptide construct that is directed against (as defined herein) a heterodimeric protein, polypeptide, ligand or receptor (or other "target") that comprises:
- at least a first subunit;
   and
- at least a second subunit;
wherein said polypeptide construct is a (biparatopic - as defined herein) construct that at least comprises a first binding domain or binding unit that is directed against said first subunit and a second binding domain or binding unit different from said first binding domain or binding unit that is also directed against said first subunit, but against a different epitope, antigenic determinant or binding site on said first subunit.

In another aspect of the invention, there is provided a polypeptide construct that is directed against (as defined herein) a heterodimeric protein that is a ligand for a receptor and that comprises:
- at least a first subunit;
   and
- at least a second subunit;
wherein said polypeptide construct is a (biparatopic - as defined herein) construct that at least comprises a first binding domain or binding unit that is directed against said first subunit and a second binding domain or binding unit different from said first binding domain or binding unit that is also directed against said first subunit, but against a different epitope, antigenic determinant or binding site on said first subunit. In particular, but without limitation, in such a (biparatopic) contruct, the first binding domain or binding unit may be such that it modulates (as defined herein), blocks, inhibits and/or neutralizes the binding of said heterodimeric protein to its (cognate) receptor, and the second binding domain or binding unit may be such that it essentially does not modulate (as defined herein), block, inhibit and/or neutralize the binding of said heterodimeric protein to its (cognate) receptor (or visa versa).

In another aspect of the invention, there is provided a polypeptide construct that is directed against (as defined herein) a heterodimeric protein that is a ligand for a receptor and that comprises:
- at least a first subunit that is shared between said first heterodimeric protein, polypeptide, ligand or receptor and at least a second, different heterodimeric protein, polypeptide, ligand or receptor;
   and
- at least a second subunit that is not shared between said first heterodimeric protein, polypeptide, ligand or receptor and said second, different heterodimeric protein, polypeptide, ligand or receptor;
wherein said polypeptide construct is a (biparatopic - as defined herein) construct that at least comprises a first binding domain or binding unit that is directed against said second (i.e. not shared) subunit and a second binding domain or binding unit different from said first binding domain or binding unit that is also directed against said second (i.e. not shared) subunit, but against a different epitope, antigenic determinant or binding site on said second subunit. In particular, but without limitation, in such a (biparatopic) contruct, the first binding domain or binding unit may be such that it modulates (as defined herein), blocks, inhibits and/or neutralizes the binding of said heterodimeric protein to its (cognate) receptor, and the second binding domain or binding unit may be such that it essentially does not modulate (as defined herein), block, inhibit and/or neutralize the binding of said heterodimeric protein to its (cognate) receptor (or visa versa).

In another aspect of the invention, there is provided a polypeptide construct that is directed against (as defined herein) a heterodimeric protein, polypeptide, ligand or receptor that comprises:
- at least a first subunit;
   and
- at least a second subunit;
wherein said polypeptide construct is a (biparatopic - as defined herein) construct that at least comprises a first binding domain or binding unit that is directed against said first subunit and a second binding domain or binding unit different from said first binding domain or binding unit that is also directed against said first subunit, but against a different epitope, antigenic determinant or binding site on said first subunit.

In the above multispecific (and in particular, bispecific) and multiparatopic (and in particular biparatopic) constructs, the first and second binding domain may be as generally described herein (for example, in terms of affinity, specificity etc. for the subunit against which they are directed) for the amino acid sequences of the invention in general. Also, as described herein for the amino acid sequences of the invention, the first, second and optionally further binding domains or binding units present in said constructs are preferably such that they form or are capable of forming (optionally after suitable folding) a single antigen binding domain or antigen binding unit, and may for example be amino acid sequences that comprise an immunoglobulin fold, amino acid sequences that are comprises of four framework regions and three CDR's, and may in particular be domain antibodies, single domain antibodies, VHH's, "dAb's" or Nanobodies (all as further described herein), or suitable fragments thereof.

Suitable heterodimeric "targets" for the above multispecific (and in particular, bispecific) and multiparatopic (and in particular biparatopic) constructs will be clear to the skilled person based on the disclosure herein; as will be the advantages of the use of the above constructs against such targets.

For example, but without limitation, the heterodimeric protein, polypeptide, ligand or receptor may be a heterodimeric protein that is a ligand for a heterodimeric receptor, and may in particular be a heterodimeric cytokine (for example, IL-12, IL-23, IL-27 or IL-35). Also, for example, the heterodimeric protein, polypeptide, ligand or receptor may be a heterodimeric ligand that is a receptor for a heterodimeric ligand, and may in particular be a receptor for a heterodimeric cytokine (for example, a receptor for IL-12, IL-23, IL-27 or IL-35).

In particular, according to the invention, said heterodimeric protein, ligand or polypeptide may be a heterodimeric cytokine or a (heterodimeric) receptor for a cytokine (and in particular for a heterodimeric cytokine).

Other uses and advantages of the above constructs will become clear to the skilled person based on the disclosure herein.

Some preferred, but non-limiting constructs of the invention are:
a) a construct comprising at least one p19+ sequence (as defined herein) and at least one p 19- sequence (as defined herein);
b) a construct comprising at least one p19+ sequence (as defined herein) and at least one p40+ sequence (as defined herein);
c) a construct comprising at least one p19+ sequence (as defined herein) and at least one p40- sequence (as defined herein);
d) a construct comprising at least one p19- sequence (as defined herein) and at least one p40+ sequence (as defined herein);
e) a construct comprising at least one p35 sequence (as defined herein) and at least one p40+ sequence (as defined herein);
f) a construct comprising at least one p35 sequence (as defined herein) and at least one p40- sequence (as defined herein);
g) a construct comprising at least one p40+ sequence (as defined herein) and at least one p40- sequence (as defined herein);
h) a construct comprising at least two (the same or different) p 19- sequences (as defined herein), that is such that it capable (for example, but without limitation, through steric interaction and/or courtesy of the linker(s) present therein) of modulating, neutralizing, blocking and/or inhibiting the binding of a heterodimeric cytokine comprising a p19 subunit to its receptor, and in particular capable of are capable of modulating, neutralizing, blocking and/or inhibiting the binding of IL-23 to IL-23R (for example in the alpha-screen assay of Example 19 or 22)
i) a construct comprising at least two (the same or different) p40- sequences, that is such that it capable (for example, but without limitation, through steric interaction and/or courtesy of the linker(s) present therein) of modulating, neutralizing, blocking and/or inhibiting the binding of a heterodimeric cytokine comprising a p40 subunit to its receptor, and in particular capable of are capable of modulating, neutralizing, blocking and/or inhibiting the binding of IL-23 to IL-23R (for example in the alpha-screen assay of Example 19 or 22) and/or of modulating, neutralizing, blocking and/or inhibiting the binding of IL-12 to its receptor (for example in the alpha-screen assay of Example 19 or 22);
j) a construct comprising at least one IL-12Rb1 sequence (as defined herein) and at least one IL-12Rb2 sequence (as defined herein);
k) a construct comprising at least one II-12Rb1 sequence (as defined herein) and at least one IL-23R sequence (as defined herein);
and such construct, nucleotide sequences encoding the same, formulations and preparations comprising the same, and their preparation and various uses (all as further described herein) form further aspects of the invention,

Examples of such constructs (and/or of amino acid sequences of the invention that can be used as binding domains and/or binding units to provide such constructs), as well as possible uses and advantages of the above constructs will become clear to the skilled person based on the disclosure herein. For example, and without limitation:
- the constructs referred to under a) above: (i) will be capable of modulating, neutralizing, blocking and/or inhibiting the binding of a heterodimeric cytokine comprising a p19 subunit to its receptor, and in particular be capable of are capable of modulating, neutralizing, blocking and/or inhibiting the binding of IL-23 to IL-23R (for example in the alpha-screen assay of Example 19 or 22); and/or (ii) will generally be specific for (as defined herein) IL-23 compared to heterodimeric cytokine that do not comprise a p19 subunit (such as IL-12, IL-27 or IL-35); and/or (iii) will bind to p19 with greater avidity and specificity than the corresponding p19+ sequence (or another p 19+ sequence) per se; and/or
- the constructs referred to under b), c) and d) above (i) will be capable of modulating, neutralizing, blocking and/or inhibiting the binding of IL-23 to the (cognate) receptor for IL-23 (for example in the alpha-screen assay of Example 19 or 22); and/or (ii) will generally be specific for (as defined herein) IL-23 compared to IL-12 (and are also expected to be specific for IL-23 compared to other heterodimeric cytokines that might comprise a p19 or p40 subunit); and/or (iii) bind to IL-23 with greater avidity and specificity than the corresponding p19+ sequence (or another p19+ sequence) per se; and/or (iv) generally be preferred over similar constructs comprising only p19-sequences and p40- sequences;
- the constructs referred to under e) and f) above: (i) will be capable of modulating, neutralizing, blocking and/or inhibiting the binding of IL-12 to the (cognate) receptor for IL-12 (for example in the alpha-screen assay of Example 19 or 22) ; and/or (ii) will generally be specific for (as defined herein) IL-12 compared to IL-23 (and are also expected to be specific for IL-12 compared to other heterodimeric cytokine that might comprise a p35 or p40 subunit); and/or (iii) bind to IL-12 with greater avidity and specificity than the corresponding p35 sequence (or another p35 sequence) per se;
- the constructs referred to under g) above: (i) will be capable of modulating, neutralizing, blocking and/or inhibiting both the binding of IL-23 to the (cognate) receptor for IL-23 for example in the alpha-screen assay of Example 19 or 22)) as well as binding of IL-12 to the (cognate) receptor for IL-12 (for example in the alpha-screen assay of Example 19 or 22); (ii) will bind to p40 with greater avidity and specificity than the corresponding p40+ sequence (or another p40+ sequence) per se
- the constructs referred to under h) above: (i) will be capable of modulating, neutralizing, blocking and/or inhibiting the binding of IL-23 to the (cognate) receptor for IL-23 (for example in the alpha-screen assay of Example 19 or 22); and/or (ii) will generally be specific for (as defined herein) IL-23 compared to heterodimeric cytokine that do not comprise a p19 subunit (such as IL-12, IL-27 or IL-35); and/or (iii) bind to IL-23 with greater avidity and specificity than each of the corresponding p19-sequences (or another monomeric p19- sequence or p19+ sequence) per se;
- the constructs referred to under i) above: (i) will be capable of modulating, neutralizing, blocking and/or inhibiting both the binding of IL-23 to the (cognate) receptor for IL-23 (for example in the alpha-screen assay of Example 19 or 22) as well as binding of IL-12 to the (cognate) receptor for IL-12 (for example in the alpha-screen assay of Example 19 or 22); and (ii) bind to p40 with greater avidity and specificity than each of the corresponding p40- sequences (or another monomeric p40- sequences or p40+ sequence) per se;
- the construct referred to under j) above: (i) will be specific for the cognate receptor for IL-12 compared to the cognate receptor for IL-23; and (ii) will bind to the cognate receptor for IL-12 with greater avidity and specificity compared to a monomeric IL-12Rb2 sequence;
- the construct referred to under k) above: (i) will be specific for the cognate receptor for IL-23 compared to the cognate receptor for IL-12; and (ii) will bind to the cognate receptor for TL-23 with greater avidity and specificity compared to a monomeric IL-23R sequence;

Also, as further described herein, a construct as referred to under j) above: (i) may act as an agonist for the signalling that is mediated by the cognate receptor for IL-12 (in which case, the construct is expected to be specific for the signalling that is mediated by the cognate receptor for IL-12 compared to the signalling that is mediated by the cognate receptor for IL-23 mediated signalling, or may essentially not even be capable of acting as agonist for the signaling that is mediated by the cognate receptor for IL-23); and/or (ii) may be capable of modulating, neutralizing, blocking and/or inhibiting the binding of a IL-12 to its cognate receptor and/or may otherwise be capable of preventing, modulating, neutralizing, blocking and/or inhibiting the receptor-mediated signalling that, without the presence of said construct, would be triggered by binding of IL-12 to its cognate receptor (i.e. act as an antagonist for IL-12 and/or for the signalling that is mediated by the cognate receptor for IL-12), and as such be specific for the cognate receptor for IL-12 compared to the cognate receptor for IL-23 (and/or bind with higher avidity and/or specificity to the cognate receptor for IL-12 compared to the cognate receptor for IL-23).

Similarly, as further described herein, a construct as referred to under k) above: (i) may act as an agonist for the signalling that is mediated by the cognate receptor for IL-23 (in which case, the construct is expected to be specific for the signalling that is mediated by the cognate receptor for IL-23 compared to the signalling that is mediated by the cognate receptor for IL-12 mediated signalling, or may essentially not even be capable of acting as agonist for the signalling that is mediated by the cognate receptor for IL-12); and/or (ii) may be capable of modulating, neutralizing, blocking and/or inhibiting the binding of a IL-23 to its cognate receptor and/or may otherwise be capable of preventing, modulating, neutralizing, blocking and/or inhibiting the receptor-mediated signalling that, without the presence of said construct, would be triggered by binding of IL-23 to its cognate receptor (i.e. act as an antagonist for IL-23 and/or for the signalling that is mediated by the cognate receptor for IL-23), and as such be specific for the cognate receptor for IL-23 compared to the cognate receptor for IL-12 (and/or bind with higher avidity and/or specificity to the cognate receptor for IL-23 compared to the cognate receptor for IL-12).

Other applications and uses of the amino acid sequences and polypeptides of the invention will become clear to the skilled person from the further disclosure herein.

Generally, it is an object of the invention to provide pharmacologically active agents, as well as compositions comprising the same, that can be used in the diagnosis, prevention and/or treatment of diseases and disorders associated with heterodimeric cytokine and their receptors and of the further diseases and disorders mentioned herein; and to provide methods for the diagnosis, prevention and/or treatment of such diseases and disorders that involve the administration and/or use of such agents and compositions.

In particular, it is an object of the invention to provide such pharmacologically active agents, compositions and/or methods that have certain advantages compared to the agents, compositions and/or methods that are currently used and/or known in the art. These advantages will become clear from the further description below.

More in particular, it is an object of the invention to provide therapeutic proteins that can be used as pharmacologically active agents, as well as compositions comprising the same, for the diagnosis, prevention and/or treatment of diseases and disorders associated with heterodimeric cytokines and their receptors and of the further diseases and disorders mentioned herein; and to provide methods for the diagnosis, prevention and/or treatment of such diseases and disorders that involve the administration and/or the use of such therapeutic proteins and compositions.

Accordingly, it is a specific object of the present invention to provide amino acid sequences that are directed against (as defined herein) heterodimeric cytokine and/or their receptors, in particular against heterodimeric cytokines and/or their receptors from a warm-blooded animal, more in particular against heterodimeric cytokines and/or their receptors from a mammal, and especially against human heterodimeric cytokines and/or their receptors; and to provide proteins and polypeptides comprising or essentially consisting of at least one such amino acid sequence.

In particular, it is a specific object of the present invention to provide such amino acid sequences and such proteins and/or polypeptides that are suitable for prophylactic, therapeutic and/or diagnostic use in a warm-blooded animal, and in particular in a mammal, and more in particular in a human being.

More in particular, it is a specific object of the present invention to provide such amino acid sequences and such proteins and/or polypeptides that can be used for the prevention, treatment, alleviation and/or diagnosis of one or more diseases, disorders or conditions associated with heterodimeric cytokines and/or their receptors and/or mediated by heterodimeric cytokines and/or their receptors (such as the diseases, disorders and conditions mentioned herein) in a warm-blooded animal, in particular in a mammal, and more in particular in a human being.

It is also a specific object of the invention to provide such amino acid sequences and such proteins and/or polypeptides that can be used in the preparation of pharmaceutical or veterinary compositions for the prevention and/or treatment of one or more diseases, disorders or conditions associated with and/or mediated by heterodimeric cytokines and/or their receptors (such as the diseases, disorders and conditions mentioned herein) in a warm-blooded animal, in particular in a mammal, and more in particular in a human being.

In the invention, generally, these objects are achieved by the use of the amino acid sequences, proteins, polypeptides and compositions that are described herein.

In general, the invention provides amino acid sequences that are directed against (as defined herein) and/or can specifically bind (as defined herein) to heterodimeric cytokines and/or their receptors; as well as compounds and constructs, and in particular proteins and polypeptides, that comprise at least one such amino acid sequence. Said amino acid sequence preferably form and/or essentially consist of a single (antigen) binding domain or binding unit, and/or are capable of forming and/or of functioning as a single (antigen) binding domain or binding unit (optionally after suitable folding), either as such and/or as part of a protein or polypeptide of the invention as further described herein.

More in particular, the invention provides amino acid sequences (such as the p19+ sequences, p19- sequences, p40+ sequences, p40- sequences, p35 sequences, IL-27 sequences, IL-12Rb1 sequences, IL-12Rb2 sequences and IL-23 sequences described herein) that can bind to heterodimeric cytokines and/or their receptors with an affinity (suitably measured and/or expressed as a K_{D}-value (actual or apparent), a K_{A}-value (actual or apparent), a kₒₙ-rate and/or a k_{off}-rate, or alternatively as an IC₅₀ value, as further described herein) that is as defined herein; as well as compounds and constructs, and in particular proteins and polypeptides, that comprise at least one such amino acid sequence.

In particular, the amino acid sequences and polypeptides of the invention are preferably such that they:
- bind to heterodimeric cytokines and/or their receptors with a dissociation constant (K_{D}) of 10⁻⁵ to 10⁻¹² moles/liter or less, and preferably 10⁻⁷ to 10⁻¹² moles/liter or less and more preferably 10⁻⁸ to 10⁻¹² moles/liter (i.e. with an association constant (K_{A}) of 10⁵ to 10¹² liter/ moles or more, and preferably 10⁷ to 10¹² liter/moles or more and more preferably 10⁸ to 10¹² liter/moles);
   and/or such that they:
- bind to heterodimeric cytokines and/or their receptors with a kₒₙ-rate of between 10² M⁻¹s⁻¹ to about 10⁷ M⁻¹s⁻¹, preferably between 10³ M⁻¹s⁻¹ and 10⁷ M⁻¹s⁻¹, more preferably between 10⁴ M⁻¹s⁻¹ and 10⁷ M⁻¹s⁻¹, such as between 10⁵ M⁻¹s⁻¹ and 10⁷ M⁻¹s⁻¹;
   and/or such that they:
- bind to heterodimeric cytokines and/or their receptors with a k_{off} rate between 1 s⁻¹ (t_{1/2}=0.69 s) and 10⁻⁶ s⁻¹ (providing a near irreversible complex with a t_{1/2} of multiple days), preferably between 10⁻² s⁻¹ and 10⁻⁶ s⁻¹, more preferably between 10⁻³ s⁻¹ and 10⁻⁶ s⁻¹, such as between 10⁻⁴ s⁻¹ and 10⁻⁶ s⁻¹.

Preferably, a monovalent amino acid sequence of the invention (or a polypeptide that essentially contains only one amino acid sequence of the invention) is preferably such that it will bind to heterodimeric cytokines and/or their receptors with an affinity less than 500 nM, preferably less than 200 nM, more preferably less than 10 nM, such as less than 500 pM.

Some preferred IC50 values for binding of the ammo acid sequences or polypeptides of the invention to heterodimeric cytokines and/or their receptors will become clear from the further description and examples herein.

For binding to heterodimeric cytokines and/or their receptors, an amino acid sequence of the invention will usually contain within its amino acid sequence one or more amino acid residues or one or more stretches of amino acid residues (i.e. with each "stretch" comprising two or amino acid residues that are adjacent to each other or in close proximity to each other, i.e. in the primary or tertiary structure of the amino acid sequence) via which the amino acid sequence of the invention can bind to heterodimeric cytokines and/or their receptors, which amino acid residues or stretches of amino acid residues thus form the "site" for binding to heterodimeric cytokines and/or their receptors (also referred to herein as the "*antigen binding site*").

The amino acid sequences provided by the invention are preferably in essentially isolated form (as defined herein), or form part of a protein or polypeptide of the invention (as defined herein), which may comprise or essentially consist of one or more amino acid sequences of the invention and which may optionally further comprise one or more further amino acid sequences (all optionally linked via one or more suitable linkers). For example, and without limitation, the one or more amino acid sequences of the invention may be used as a binding unit in such a protein or polypeptide, which may optionally contain one or more further amino acid sequences that can serve as a binding unit (i.e. against one or more other targets than heterodimeric cytokines and/or their receptors), so as to provide a monovalent, multivalent or multispecific polypeptide of the invention respectively, all as described herein. Such a protein or polypeptide may also be in essentially isolated form (as defined herein).

The amino acid sequences (such as the p19+ sequences, p19- sequences, p40+ sequences, p40- sequences, p35 sequences, IL-27 sequences, IL-12Rb1 sequences, IL-12Rb2 sequences and IL-23 sequences described herein) and polypeptides (such as the for example multivalent, multispecific and/or biparatopic - constructs described herein that comprise at least one p19+ sequence, p19- sequence, p40+ sequence, p40- sequence, p35 sequence, IL-27 sequence, IL-12Rb1 sequence, IL-12Rb2 sequence and/or IL-23 sequence) of the invention as such preferably essentially consist of a single amino acid chain that is not linked via disulphide bridges to any other amino acid sequence or chain (but that may or may not contain one or more intramolecular disulphide bridges. For example, it is known that Nanobodies - as described herein - may sometimes contain a disulphide bridge between CDR3 and CDR1 or FR2). However, it should be noted that one or more amino acid sequences of the invention may be linked to each other and/or to other amino acid sequences (e.g. via disulphide bridges) to provide peptide constructs that may also be useful in the invention (for example Fab' fragments, f'(ab')₂ fragments, ScFv constructs, "diabodies" and other multispecific constructs. Reference is for example made to the review by Holliger and Hudson, Nat Biotechnol 2005 Sep;23(9): 1126-36).

Generally, when an amino acid sequence of the invention (or a compound, construct or polypeptide comprising the same) is intended for administration to a subject (for example for therapeutic and/or diagnostic purposes as described herein), it is preferably either an amino acid sequence that does not occur naturally in said subject; or, when it does occur naturally in said subject, in essentially isolated form (as defined herein).

It will also be clear to the skilled person that for pharmaceutical use, the amino acid sequences of the invention (as well as compounds, constructs and polypeptides comprising the same) are preferably directed against human heterodimeric cytokines and/or their receptors; whereas for veterinary purposes, the amino acid sequences and polypeptides of the invention are preferably directed against heterodimeric cytokines and/or their receptors from the species to be treated, or at at least cross-reactive with heterodimeric cytokines and/or their receptors from the species to be treated.

Furthermore, an amino acid sequence of the invention may optionally, and in addition to the at least one binding site for binding against heterodimeric cytokines and/or their receptors, contain one or more further binding sites for binding against other antigens, proteins or targets.

The efficacy of the Amino acid sequences and polypeptides of the invention, and of compositions comprising the same, can be tested using any suitable in vitro assay, cell-based assay, in vivo assay and/or animal model known per se, or any combination thereof, depending on the specific disease or disorder involved. Suitable assays and animal models will be clear to the skilled person, and for example include in vitro assays such as Biacore (see for example Example 12,20 or 23), Alpha-screen (see for example Example 14, Example 20 or Example22 ), FLIPR, ELISA (see for example Example 10) and competitive ELISA (see for example Example 11), cell-based assays such as proliferation of activated PBMCs (for measuring modulation of IL-12 mediated signalling,) IL17 production of activated spleen cells (for measuring modulation of IL-23 mediated signalling, see for example Aggarwal, Journal of Biological Chemistry, 278, 3, 2003, 1910-1914); and assays for measuring differentiation of TH1 and/or inhibition of TH17 cells (for for measuring modulation of IL-23 mediated signalling), and various animal models for inflammatory diseases and disorders, such as models for autoimmune inflammation such as EAE (Experimental Allergic Encephalomyelitis), CIA (Collagen-induced arthritis), IL12-induccd neopterin release, and mouse spleen IL17 production; IBD models in mice and rats such as Dextran Sulphate Salt induced Ulcerative Colitis and Dinitrofluorobenzene induced Crown's disease, as well as the assays and animal models used in the experimental part below and in the prior art cited herein. Based on the disclosure herein, and depending on the heterodimeric cytokine(s) and/or receptor(s) involved, the skilled person will generally be able to select a suitable in vitro assay, cellular assay or animal model to test the amino acid sequences and polypeptides of the invention to a heterodimeric cytokine or a receptor thereof, for their capacity to modulate heterodimeric cytokine and their receptors, and/or the signaling, pathways, biological mechanisms and effects in which these are involved; and for their therapeutic and/or prophylactic effect in respect of one or more diseases and disorders that are associate with a heterodimeric cytokine and/or a receptors thereof.

Also, according to the invention, amino acid sequences and polypeptides that are directed against heterodimeric cytokines and/or their receptors from a first species of warm-blooded animal may or may not show cross-reactivity with heterodimeric cytokines and/or their receptors from one or more other species of warm-blooded animal. For example, amino acid sequences and polypeptides directed against human heterodimeric cytokines and/or their receptors may or may not show cross reactivity with heterodimeric cytokines and/or their receptors from one or more other species of primates (such as, without limitation, monkeys from the genus *Macaca* (such as, and in particular, cynomologus monkeys (*Macaca fascicularis*) and/or rhesus monkeys (*Macaw mulatta*)) and baboon (*Papio ursinus*)) and/or with heterodimeric cytokine and/or their receptors from one or more species of animals that are often used in animal models for diseases (for example mouse, rat, rabbit, pig or dog), and in particular in animal models for diseases and disorders associated with heterodimeric cytokines and/or their receptors (such as the species and animal models mentioned herein). In this respect, it will be clear to the skilled person that such cross-reactivity, when present, may have advantages from a drug development point of view, since it allows the amino acid sequences and polypeptides against human heterodimeric cytokines and/or their receptors to be tested in such disease models.

More generally, amino acid sequences and polypeptides of the invention that are crass-reactive with heterodimeric cytokines and/or their receptors from multiple species of mammal will usually be advantageous for use in veterinary applications, since it will allow the same amino acid sequence or polypeptide to be used across multiple species. Thus, it is also encompassed within the scope of the invention that amino acid sequences and polypeptides directed against heterodimeric cytokines and/or their receptors from one species of animal (such as amino acid sequences and polypeptides against human heterodimeric cytokines and/or their receptors) can be used in the treatment of another species of animal, as long as the use of the amino acid sequences and/or polypeptides provide the desired effects in the species to be treated.

The present invention is in its broadest sense also not particularly limited to or defined by a specific antigenic determinant, epitope, part, domain, subunit or confirmation (where applicable) of heterodimeric cytokines and/or their receptors against which the amino acid sequences and polypeptides of the invention are directed. In one particular aspect of the invention, the amino acid sequences and polypeptides are (at least) directed against an interaction site (as defined herein) on the heterodimeric cytokine or the receptor.

As further described herein, a polypeptide of the invention may contain two or more amino acid sequences of the invention that are directed against their intended (cognate) cognate target (such as a heterodimeric cytokine, a receptor for the same, or a subunit of either). Generally, such polypeptides will bind to said said target with increased avidity compared to a single amino acid sequence, of the invention. Such a polypeptide may for example comprise two amino acid sequences of the invention that are directed against the same antigenic determinant, epitope, part, domain, subunit or confirmation (where applicable) of said target (which may or may not be an interaction site); or comprise at least one "first" amino acid sequence of the invention that is directed against a first same antigenic determinant, epitope, part, domain, subunit or confirmation (where applicable) of said target (which may or may not be an interaction site); and at least one "second" amino acid sequence of the invention that is directed against a second antigenic determinant, epitope, part, domain, subunit or confirmation (where applicable) different from the first (and which again may or may not be an interaction site). Preferably, in such "biparatopic" polypeptides of the invention, at least one amino acid sequence of the invention is directed against an interaction site (as defined herein), although the invention in its broadest sense is not limited thereto.

Also, when the target is part of a binding pair (for example, a receptor-ligand binding pair), the amino acid sequences and polypeptides may be such that they compete with the cognate binding partner (e.g. the ligand, receptor or other binding partner, as applicable) for binding to the target, and/or such that they (fully or partially) neutralize binding of the binding partner to the target.

It is also within the scope of the invention that, where applicable, an amino acid sequence of the invention can bind to two or more antigenic determinants, epitopes, parts, domains, subunits or confirmations of heterodimeric cytokine and/or their receptors. In such a case, the antigenic determinants, epitopes, parts, domains or subunits of heterodimeric cytokines and/or their receptors to which the amino acid sequences and/or polypeptides of the invention bind may be essentially the same (for example, if heterodimeric cytokine and/or their receptors contains repeated structural motifs or occurs in a multimeric form) or may be different (and in the latter case, the amino acid sequences and polypeptides of the invention may bind to such different antigenic determinants, epitopes, parts, domains, subunits of heterodimeric cytokines and/or their receptors with an affinity and/or specificity which may be the same or different). Also, for example, when heterodimeric cytokines and/or their receptors exists in an activated conformation and in an inactive conformation, the amino acid sequences and polypeptides of the invention may bind to either one of these confirmation, or may bind to both these confirmations (i.e. with an affinity and/or specificity which may be the same or different). Also, for example, the amino acid sequences and polypeptides of the invention may bind to a conformation of heterodimeric cytokine and/or their receptors in which it is bound to a pertinent ligand, may bind to a conformation of heterodimeric cytokines and/or their receptors in which it not bound to a pertinent ligand, or may bind to both such conformations (again with an affinity and/or specificity which may be the same or different).

It is also expected that the amino acid sequences and polypeptides of the invention will generally bind to all naturally occurring or synthetic analogs, variants, mutants, alleles, parts and fragments of heterodimeric cytokanes and/or their receptors; or at least to those analogs, variants, mutants, alleles, parts and fragments of heterodimeric cytokines and/or their receptors that contain one or more antigenic determinants or epitopes that are essentially the same as the antigenic determinant(s) or epitope(s) to which the amino acid sequences and polypeptides of the invention bind in heterodimeric cytokines and/or their receptors (e.g. in wild-type heterodimeric cytokines and/or their receptors). Again, in such a case, the amino acid sequences and polypeptides of the invention may bind to such analogs, variants, mutants, alleles, parts and fragments with an affinity and/or specificity that are the same as, or that are different from (i.e. higher than or lower than), the affinity and specificity with which the amino acid sequences of the invention bind to (wild-type) heterodimeric cytokines and/or their receptors. It is also included within the scope of the invention that the amino acid sequences and polypeptides of the invention bind to some analogs, variants, mutants, alleles, parts and fragments of heterodimeric cytokines and/or their receptors, but not to others.

The amino acid sequences, polypeptides and compositions of the present invention can generally be used to modulate (as defined herein) the signalling that is mediated by heterodimeric cytokines and/or their receptors, to modulate (as defined herein) the biological pathways in which heterodimeric cytokines and/or their receptors are involved, and/or to modulate (as defined herein) the biological mechanisms, responses and effects associated with heterodimeric cytokines, their receptors, such signalling and/or these pathways (all the foregoing is also collectively referred to herein as "*heterodimeric cytokine-mediated signalling*").

As such, the amino acid sequences, polypeptides and compositions of the present invention can generally be used to modulate the immune system and/or one or more specific immune responses in a subject to which one or more of the amino acid sequences, polypeptides and compositions of the present invention are administered (i.e. in therapeutically relevant amounts).

The term "heterodimeric cytokines" as used herein in its broadest sense generally includes any heterodimeric cytokine, i.e. a cytokine that comprises at least two, and more preferably only two, subunits.

In particular, the term "heterodimeric cytokine" as used herein encompasses heterodimeric cytokines that are associated with cell-mediated (T_{H}1) immunity, although the invention is its broadest sense is not limited thereto and also encompasses heterodimeric cytokines associated with humoral (T_{H}2) immunity.

According to one specific, but non-limiting aspect, the amino acid sequences and polypeptides of invention are directed against a heterodimeric cytokine that is chosen from heterodimeric cytokines that comprise a p40 subunit or p40-like subunit, such as a p40 subunit (present in for examples IL-12 and IL-23) or Epstein-Barr virus (EBV)-induced molecule 3 (EBI3, present in for example IL-27 and IL-35).

According to another specific, but non-limiting aspect, the amino acid sequences and polypeptides of invention are directed against a heterodimeric cytokine that is chosen from heterodimeric cytokines that comprise a p19 subunit or a p19-like subunit, such as a p 19 subunit (present in for example IL-23), a p35 subunit (present in for example IL-12 and IL-35), or a p28 subunit (present in for example IL-27) or a homolog thereof.

For example, the amino acid sequences and polypeptides of invention may be directed against a heterodimeric cytokine that will comprise at least one p19 subunit or p19-like subunit and at least one p40 subunit or p40-like subunit.

According to an even more specific, but non-limiting aspect, the amino acid sequences and polypeptides of invention are directed against a heterodimeric cytokine that is chosen from IL-12, IL-23, IL-27 and/or IL-35.

In one specific aspect, but non-limiting aspect, the amino acid sequences and polypeptides of the invention are directed against IL-23 (i.e, against p40, p19 or both). Such amino acid sequences and polypeptides of the invention (as well as compositions comprising the same) may be as further described herein and can be used for preventing and treating disorders associated with IL-23, the IL-23 receptor and/or IL-23 mediated signalling. Reference is again made to the prior art cited above. Also, as mentioned herein, the amino acid sequences and polypeptides of the invention that are directed against IL-23, and in particular those that are specific for (as defined herein) IL-23 compared to IL-12, may have advantages for therapeutic use over the amino acid sequences and polypeptides of the invention that are directed against IL-12. Also, as mentioned herein, the amino acid sequences and polypeptides of the invention that are directed against p19, and in particular those that are specific for (as defined herein) p19 compared to p35 and p40, may have advantages for therapeutic use over the amino acid sequences and polypeptides of the invention that are directed against p35 or p40.

According to another specific, but non-limiting aspect, the invention provides amino acid sequences and polypeptides that are directed against at least one subunit of a heterodimeric cytokine (as defined herein). These amino acid sequences and/or polypeptides may be as further described herein.

In another specific, but non-limiting aspect, the invention provides "bispecific" (as defined herein) polypeptides that are directed against both subunits of a heterodimeric cytokine. These polypeptides may be as further described herein.

In another specific, but non-limiting aspect, the invention provides "biparatopic" (as defined herein) polypeptides that are directed against one subunit of a heterodimeric cytokine. These polypeptides may be as further described herein.

In particular, but without limitation, the invention provides amino acid sequences and polypeptides that are directed against at least one subunit of a heterodimeric cytokine, wherein said heterodimeric cytokine is associated with cell-mediated (T_{H}1) immunity.

For example, in one specific, but non-limiting aspect, the invention provides amino acid sequences and polypeptides that are directed against at least one subunit of a heterodimeric cytokine, wherein said heterodimeric cytokine is chosen from heterodimeric cytokines that comprise a p40 subunit or p40-like subunit, such as a p40 subunit (present in for example IL-12 and IL-23) or Epstein-Barr virus (EBV)-induced molecule 3 (EBI3, present in for example IL-27 and IL-35).

In another specific, but non-limiting aspect, the invention provides amino acid sequences and polypeptides that are directed against a p40 subunit or a p40-like subunit, such as against one of the following subunits: p40 and/or EBI3, or a mutant, variant, allele or homolog of each of the foregoing.

In another specific, but non-limiting aspect, the invention provides amino acid sequences and polypeptides that are directed against at least one subunit of a heterodimeric cytokine, wherein said heterodimeric cytokine is chosen from heterodimeric cytokines that comprise a p19 subunit or p19-like subunit, such as a p19 subunit (present in for example IL-23), a p35 subunit (present in for example IL-12 and IL-35), a p28 subunit (present in for example IL-27), or a mutant, variant, allele or homolog of each of the foregoing. These amino acid sequences and/or polypeptides may be as further described herein.

In another specific, but non-limiting aspect, the invention provides amino acid sequences and polypeptides that are directed against a p 19 subunit or p19-like subunit, such as against one of the following subunits: p19, p35 and/or p28, or a mutant, variant, allele or homolog of each of the foregoing. These amino acid sequences and/or polypeptides may be as further described herein.

In another specific, but non-limiting aspect, the invention provides amino acid sequences and polypeptides that are directed against at least one subunit of one of the following heterodimeric cytokines: IL-12, IL-23, IL-27 and/or IL-35. These amino acid sequences and/or polypeptides may be as further described herein.

In another specific, but non-limiting aspect, the invention provides amino acid sequences and polypeptides that are directed against IL-12 or at least one subunit of IL-12, which amino acid sequences and/or polypeptides may be as further described herein.

In another specific, but non-limiting aspect, the invention provides amino acid sequences and polypeptides that are directed against IL-23 or at least one subunit of IL-23, which amino acid sequences and/or polypeptides may be as further described herein.

In another specific, but non-limiting aspect, the invention provides amino acid sequences and polypeptides that are directed against IL-27 or at least one subunit of IL-27, which amino acid sequences and/or polypeptides may be as further described herein.

In another specific, but non-limiting aspect, the invention provides amino acid sequences and polypeptides that are directed against IL-35 or at least one subunit of IL-35, which amino acid sequences and/or polypeptides may be as further described herein.

For example, such a polypeptide against IL-12, IL-23, IL-27 or IL-35 may comprise or essentially consist of a single amino acid sequence of the invention (such as a Nanobody) that is directed against IL-12, IL-23, IL-27 or IL-35, respectively, and in particular against an interaction site (as defined herein) on IL-12, IL-23, IL-27 or IL-35. When such a polypeptide comprises two or more amino acid sequences of the invention (optionally linked to each other via one or more suitable linkers, as described herein) that are directed against IL-12, IL-23, IL-27 or IL-35 respectively, these amino acid sequences may be directed against the same epitope, antigenic determinant, part, domain or stretch of amino acid residues on IL-12, IL-23, IL-27 or IL-35, respectively, or against different epitopes, antigenic determinants, parts, domains or stretches of amino acid residues on IL-12, IL-23, IL-27 or IL-35. For example, such a polypeptide may comprise one or more amino acid sequences of the invention that are directed against an interaction site (as defined herein, and in particular the receptor binding site) on IL-12, IL-23, IL-27 or IL-35, respectively, and one or more amino acid sequences of the invention that are directed against a site, epitope, antigenic determinant, part, domain or stretch of amino acid residues on IL-12, IL-23, IL-27 or IL-35, respectively, that is not an interaction site. Such a polypeptide may also comprise one or more amino acid sequences of the invention that are directed against an interaction site (as defined herein, and in particular the receptor binding site) on IL-12, IL-23, IL-27 or IL-35, respectively, and one or more amino acid sequences that are directed against a different interaction site (as defined herein) on IL-12, IL-23, IL-27 or IL-35. respectively. It is also possible that such a polypeptide comprises two or more amino acid sequences of the invention that are directed against the same interaction site (as defined herein, and in particular the receptor binding site) on IL-12, IL-23, IL-27 or IL-35, respectively.

For example, such a polypeptide may comprise one or more amino acid sequences of the invention (such as one or more Nanobodies) that can modulate binding of IL-12, IL-23, IL-27 or IL-35, respectively, to its receptor; and/or one or more amino acid sequences of the invention (such as one or more Nanobodies) that do not modulate (and in particular inhibit) binding of IL-12, IL-23, IL-27 or IL-35, respectively, to its receptor. For example, such a polypeptide may comprise one amino acid sequence of the invention (such as a Nanobodies) that can modulate binding of IL-12, IL-23, IL-27 or IL-35, respectively, to its receptor and one amino acid sequence of the invention (such as a Nanobody) that does not modulate binding of IL-12, IL-23, IL-27 or IL-35, respectively, to its receptor.

Examples of such polypeptides of the invention will become clear from the further description herein.

In another specific, but non-limiting aspect, the invention provides amino acid sequences and polypeptides that are directed against p19 (also referred to herein as "p19 sequences"). Such amino acid sequences and/or polypeptides may be as further described herein (for example, such amino acid sequences may be may be "p19+ sequences" or "p19-sequences"). For example, such a polypeptide may be a polypeptide that contains one or more amino acid sequences against p19, such as one or more Nanobodies against p19. It is expected that such a polypeptide of the invention will be selective for IL-23 and other heterodimeric cytokines that contain p19 compared to IL-12, IL-27 and/or IL-35..

In another specific, but non-limiting aspect, the invention provides amino acid sequences and polypeptides that are directed against p35. Such amino acid sequences and/or polypeptides may be as further described herein. For example, such a polypeptide may be a polypeptide that contains one or more amino acid sequences against p35. such as one or more Nanobodies against p35. It is expected that such a polypeptide of the invention will be selective for IL-12 and/or IL-35 and other heterodimeric cytokines that contain p40 compared to IL-12 and/or IL-27.

In another specific, but non-limiting aspect, the invention provides amino acid sequences and polypeptide that are directed against p28. Such amino acid sequences and/or polypeptides may be as further described herein. For example, such a polypeptide may be a polypeptide that contains one or more amino acid sequences against p28, such as one or more Nanobodies against p28. It is expected that such a polypeptide of the invention will be selective for IL-27 and other heterodimeric cytokines that contain p28 compared to IL-12, IL-23 and/or IL-35.

In another specific, but non-limiting aspect, the invention provides amino acid sequences and polypeptides that are directed against p40. Such amino acid sequences and/or polypeptides may be as further described herein (for example, such amino acid sequences may be may be "p40+ sequences" or "p40-sequences"). For example, such a polypeptide may be a polypeptide that contains one or more amino acid sequences against p40, such as one or more Nanobodies against p40. It is expected that such a polypeptide of the invention will be selective for IL-12 and/or IL-23 and other heterodimeric cytokines that contain p40 compared to IL-27 and/or IL-35.

In another specific, but non-limiting aspect, the invention provides amino acid sequences and polypeptides that are directed against EBI3. Such amino acid sequences and/or polypeptides may be as further described herein. For example, such a polypeptide may be a polypeptide that contains one or more amino acid sequences against EBI3, such as one or more Nanobodies against EBI3. It is expected that such a polypeptide of the invention will be selective for IL-27 and/or IL-23 and other heterodimeric cytokine that contain EBI3 compared to IL-12 and/or IL-23.

For example, such a polypeptide against p19, p35, p28, p40 or EBI3, respectively, may comprise or essentially consist of a single amino acid sequence of the invention (such as a Nanobody) that is directed against p19, p35, p28, p40 or EBI3, respectively, and in particular against an interaction site (as defined herein) on p19, p35, p28, p40 or EBI3. When such a polypeptide comprises two or more amino acid sequences of the invention (optionally linked to each other via one or more suitable linkers, as described herein) that are directed against p19, p35, p28, p40 or EBI3, respectively, these amino acid sequences may be directed against the same epitope, antigenic determinant, part, domain or stretch of amino acid residues on p19, p35, p28, p40 or EBI3 respectively, or against different epitopes, antigenic determinants, parts, domains or stretches of amino acid residues on p19, p35, p28, p40 or EBI3. For example, such a polypeptide may comprise one or more amino acid sequences of the invention that are directed against an interaction site (as defined herein, and in particular a site that is involved in binding of the heterodimeric cytokine in which said subunit is present to its receptor) on p19, p35, p28, p40 or EBI3, respectively, and one or more amino acid sequences of the invention that are directed against a site, epitope, antigenic determinant, part, domain or stretch of amino acid residues on p19, p35, p28, p40 or EBI3, respectively, that is not an interaction site. Such a polypeptide may also comprise one or more amino acid sequences of the invention that are directed against an interaction site (as defined herein, and in particular the receptor binding site) on p19, p35, p28, p40 or EBI3, respectively, and one or more amino acid sequences that are directed against a different interaction site (as defied herein) on p19, p35, p28, p40 or EBI3, respectively. It is also possible that such a polypeptide comprises two or more amino acid sequences of the invention that are directed against the same interaction site (as defined herein, and in particular a site that is involved in binding of the heterodimeric cytokine in which said subunit is present to its receptor) on p19, p35, p28, p40 or EBI3, respectively.

For example, such a polypeptide may comprise one or more amino acid sequences of the invention (such as one or more Nanobodies) that are directed against p19, p35, p28, p40 or EBI3, respectively, and that can modulate (and in particular inhibit) binding of the heterodimeric cytokines in which said subunit is present to its receptor; and/or one or more amino acid sequences of the invention (such as one or more Nanobodies) that are directed against p19, p35, p28, p40 or EBI3, respectively, but that are not capable of modulate binding of the heterodimeric cytokine in which said subunit is present to its receptor.

Again, examples of such polypeptides of the invention will become clear from the further description herein.

In another specific, but non-limiting aspect, the invention provides amino acid sequences and (in particular) polypeptides that are directed against two different subunits that occur in heterodimeric cytokines. In particular, the invention provides amino acid sequences and (in particular) polypeptides that are directed against two different subunits that occur in heterodimeric cytokines (and in particular in heterodimeric cytokines from the IL-12 family, such as in IL-12, IL-23, IL-27 7 and IL-35). For example, such an amino acid sequence or polypeptide may be directed (a) against p29 or a p19-like subunit, such as against p19, p35 or p28; and against at least one other subunit that occurs in a heterodimeric cytokine (such as in IL-12, IL-23, IL-27 and IL-35); or (b) against p40 or a p40-like subunit, such as against p40 or EBI-3 and against at least one other subunit that occurs in a heterodimeric cytokines (such as in II-12, IL-23, IL-27 and IL-35).

More in particular, the invention provides amino acid sequences and (in particular) polypeptides that are directed against (i) at least one p19 or p19-like subunit, such as against p19, p35 or p28; and (ii) at least one p40 or p40-like subunit, such as against p40 or EBI-3.

Such an amino acid sequence or polypeptide of the invention may for example also be an amino acid sequence or polypeptide of the invention that is directed towards the interface of two subunits that occur in a heterodimeric cytokine, such as towards the p19/p40 interface in IL-23, against the p35/p40 interface in IL-12, against the p28/EBI3 interface in IL-27, or against the p35/EBI3 interface in IL-35.

In a specifically preferred aspect, such a polypeptide of the invention may be a "bispecific." and in particular "biparatopic" polypeptide of the invention (as further described herein) that comprises at least one amino acid sequence of the invention (such as a Nanobody) that is directed against at least one p19 or p19-like subunit (such as against p19, p35 or p28), and at least one amino acid sequence of the invention (such as Nanobody) that is directed against at least one p40 or p40-like subunit (such as against p40 or EBI-3).

For example, the invention provides:
- amino acid sequences and (in particular) polypeptides that are directed against p19 19 and p40, which are expected to be selective for IL-23 compared to IL-12, IL-27 and IL-35. For example, such a biparatopic polypeptide may comprise at least one amino acid sequence of the invention that is directed against p19 and at least one amino acid sequence of the invention that is directed against p40;
- amino acid sequences and (in particular) polypeptides that are directed against p35 5 and p40, which are expected to be selective for IL-12 compared to IL-23 IL-27 and IL-35. For example, such a biparatopic polypeptide may comprise at least one amino acid sequence of the invention that is directed against p35 5 and at least one amino acid sequence of the invention that is directed against p40;
- amino acid sequences and (in particular) polypeptides that are directed against p35 and EBI3, which are expected to be selective for IL-35 compared to IL-12, IL-23 and IL-27. For example, such a biparatopic polypeptide may comprise at least one amino acid sequence of the invention that is directed against p35 and at least one amino acid sequence of the invention that is directed against EBI3;
- amino acid sequences and (in particular) polypeptides that are directed against p28 and EBI3, which are expected to be selective for IL-27 compared to IL-12, IL-23 and IL-35. For example, such a biparatopic polypeptide may comprise at least one amino acid sequence of the invention that is directed against p28 and at least one amino acid sequence of the invention that is directed against EBI3.

Again, all such amino acid sequences and/or polypeptides may be as further described herein, and some examples of such polypeptides of the invention will become clear from the further description herein.

For example, such a polypeptide may comprise:
- one or more amino acid sequences of the invention that are directed against an interaction site (as defined herein, and in particular a site that is involved in binding of the heterodimeric cytokine in which said subunit is present to its receptor) on the p19 or p19-1ike subunit; and one or more amino acid sequences of the invention that are directed against a site, epitope, antigenic determinant, part, domain or stretch of (amino acid residues on the p40 or p40--like subunit that is not an interaction site;
- one or more amino acid sequences of the invention that are directed against an interaction site (as defined herein, and in particular a site that is involved in binding of the heterodimeric cytokine in which said subunit is present to its receptor) on the p40 or p40-like subunit; and one or more amino acid sequences of the invention that are directed against a site, epitope, antigenic determinant, part, domain or stretch of amino acid residues on the p19 or p19-like subunit that is not an interaction site; or
- one or more amino acid sequences of the invention that are directed against an interaction site (as defined herein) on the p40 or p4(40-like subunit; and one or more amino acid sequences of the invention that are directed against an interaction site on the p19 19 or p19-like subunit;
- one or more amino acid sequences of the invention that are directed against the p 19 or p19-iike subunit and that can modulate (and in particular inhibit) binding of the heterodimeric cytokine in which said p19 or p19-like subunit is present to its receptor; and/or one or more amino acid sequences of the invention that are directed against the p40 or p40-like subunit but that are not capable of modulating binding of the heterodimeric cytokine in which said p40 or p40-like subunits is present to its receptor;
- one or more amino acid sequences of the invention that are directed against the p40 or p40-like subunit and that can modulate (and in particular inhibit) binding of the heterodimeric cytokine in which said p40 or p40-like subunit is present to its receptor; and/or one or more amino acid sequences of the invention that are directed against the p19 or p19-like subunit but that are not capable of modulating binding of the heterodimeric cytokine in which said p19 or p19-like subunit is present to its receptor;
- one or more amino acid sequences of the invention that are directed against the p19 or p19-like subunit and that can modulate (and in particular inhibit) binding of the heterodimeric cytokine in which said p19 or p19-like subunit is present to its receptor; and one or more amino acid sequences of the invention that are directed against the p40 or p40-like subunit and that can modulate (and in particular inhibit) binding of the heterodimeric cytokine in which said p40 or p40-like subunit is present to its receptor.

Again, all such amino acid sequences and/or polypeptides may be as further described herein, and some examples of such polypeptides of the invention will become clear from the further description herein.

Accordingly, in a specific, but non-limiting aspect, the invention provides amino acid sequences and polypeptides that are directed against two or more subunits of heterodimeric cytokines. For example, the invention comprises multispecific proteins and polypeptides (as described herein) that comprise at least one binding unit against a first subunit of a heterodimeric cytokine and at least one binding unit against a second subunit of a heterodimeric cytokine that is different from said first subunit. For example, the invention comprises such multispecific proteins and polypeptides which comprise at least one binding unit against a first subunit of a heterodimeric cytokine and at least one binding unit against a second subunit of a heterodimeric cytokine that is different from said first subunit, in which said first and second subunit form part of the same heterodimeric cytokine (in other words, such multispecific proteins or polypeptide are "biparatopic" with respect to said heterodimeric cytokine, in that they are capable of binding to two different epitopes on said heterodimeric cytokine. Alternatively, and without limitation, a protein or polypeptide as described herein may for example be biparatopic in respect of one of the subunits mentioned herein, i.e. comprise at least one binding unit against a first epitope on said subunit and at least one binding unit against a second epitope on said subunit). Some non-limiting examples of such multispecific proteins and polypeptide are multispecific proteins and polypeptides that are directed against p35 and p40 (which are both present in IL-12, so that such a multivalent protein or polypeptide is expected to be specific for IL-12), against p 19 and p40 (both present in IL-23), or against p28 and EBI3 (both present in IL-27).

More generally, the invention comprises such multispecific proteins and polypeptides which comprise at least one binding unit against a first subunit of a heterodimeric cytokine and at least one binding unit against a second subunit of a heterodimeric cytokine that is different from said first subunit, in which said first and second subunit are chosen from p19, p35, p28, p40 and/or EBI3; and/or mutants, variants, alleles or homologs of each of the foregoing. For example, such multispecific proteins and polypeptides may comprise at least one binding unit which is directed against a p19-Like subunit such as p19, p35 or p 28 and at least one binding unit that is directed against a p40-like subunit such as p40 or EBI3 (it should also be noted that the invention even more generally relates to any multispecific protein and polypeptide which comprises at least one binding unit that is directed against a heterodimeric cytokine or a subunit thereof- such as p19, p35, p28, p40 and/or EBI3 - and at least one further binding unit that is directed against any other (e.g. 1non-heterodimeric cytokine) desired target, antigenic determinant or epitope).

It will also be clear to the skilled person that an amino acid sequence or polypeptide as described herein may be directed against the interface between the two subunits that form a heterodimeric cytokine (usually the interface between a p19-like subunit and a p40-like subunit). Thus, such an amino acid sequence and polypeptide will often be able to (simultaneously) bind to both subunits that form the heterodimeric cytokine, so as to span the interface between said two subunits. For example, the amino acid sequences and polypeptides described herein may be directed against the p35/p40 interface of IL-12, against the p19/p40 interface of IL-23, or against the p28/EBI-3 interface of IL-28.

Thus, from the above, it will be clear to the skilled person that the amino acid sequences and polypeptides described herein may be directed against a single heterodimeric cytokine (or against a single subunit of a heterodimeric cytokine), but may also be directed against multiple heterodimeric cytokines (or against multiple subunits thereof, that either form part of the same heterodimeric cytokine or even of different heterodimeric cytokines).

In one specific, but non-limiting aspect, the amino acid sequences and polypeptides described herein are specific for (as defined herein) IL-23, compared to IL-12, IL-27 and IL-35.

In another specific, but non-limiting aspect, the amino acid sequences and polypeptides described herein are specific for (as defined herein) IL-12, compared to IL-23, IL-27 and IL-35.

In another specific, but non-limiting aspect, the amino acid sequences and polypeptides described herein are specific for (as defined herein) IL-27, compared to IL-12, IL-23 and IL-35.

In another specific, but non-limiting aspect, the amino acid sequences and polypeptides described herein are specific for (as defined herein) IL-35, compared to IL-12, IL-23 and IL-27.

In another specific, but non-limiting aspect, the amino acid sequences and polypeptides described herein are specific for (as defined herein) the p19 subunit, compared to the p35 or p28 subunits. Such amino acid sequences and polypeptides are expected to be specific for IL-23 (i.e. compared to IL-12, IL-27 and IL-35).

In another specific, but non-limiting aspect, the amino acid sequences and polypeptides described herein are specific for (as defined herein) the p28 subunit, compared to the p35 or p 19 subunits. Such amino acid sequences and polypeptides are expected to be specific for IL-27 (i.e. compared to IL-12, IL-23 and IL-35).

In another specific, but non-limiting aspect, the amino acid sequences and polypeptides described herein are specific for (as defined herein) the p35 subunit, compared to the p28 or p19 subunits. Such amino acid sequences and polypeptides are expected to be specific for IL-12 and IL-35 (i.e. compared to IL-23 and IL-27).

In another specific, but non-limiting aspect, the amino acid sequences and polypeptides described herein are specific for (as defined herein) the p40 subunit, compared to EBI-3. Such amino acid sequences and polypeptides are expected to be specific for IL-12 and IL-23 (i.e. compared to IL-27 and IL-35).

In another specific, but non-limiting aspect, the amino acid sequences and polypeptides described herein are specific for (as defined herein) the EBI-3 subunit, compared to the p40 subunit. Such amino acid sequences and polypeptides are expected to be specific for IL-27 and IL-35 (i.e. compared to IL-12 and IL-23).

In another specific, but non-limiting aspect, the amino acid sequences and polypeptides described herein are specific for both the p19 and p40 subunits (compared to other subunits), and in particular directed against (as defined herein, i.e. capable of specifically binding to) the p19 and p40 subunits, but not directed against (i.e. not capable of specifically binding to) any of the subunits p35, p28 and/or EBI3 (or, according to an even more specific aspect, not directed to any p19-like subunit other than p19 and not directed to any p40-like subunit other than p40). Such amino acid sequences (which may for example span the p19/p40 interface in IL-23 as described herein) or polypeptides (which may for example be bispecific polypeptides with at least one binding unit directed against p 19 and at least one binding unit directed against p40) are expected to be specific for IL-23 compared to IL-27, and are expected to bind with higher avidity (and preferably also selectivity) to IL-23 compared to IL-12.

In another specific, but non-limiting aspect, the amino acid sequences and polypeptides described herein are specific for both the p35 and p40 subunits (compared to other subunits), and in particular directed against (as defined herein, i.e. capable of specifically binding to) the p35 and p40 subunits, but not directed against (i.e. not capable of specifically binding to) any of the subunits p19, p28 and/or EBI3 (or, according to an even more specific aspect, not directed to any p19-like subunit other than p35 and not directed to any p40-like subunit other than p40). Such amino acid sequences (which may for example span the p35/p40 interface in IL-12 as described herein) or polypeptides (which may for example be bispecific polypeptides with at least one binding unit directed against p35 5 and at least one binding unit directed against p40) are expected to be specific for IL-12 compared to IL-27, and are expected to bind with higher avidity (and preferably also selectivity) to IL-12 compared to IL-23.

In another specific, but non-iimiting aspect, the amino acid sequences and polypeptides described herein are specific for both the p28 and EB1-3 subunits (compared to other subunits), and in particular directed against (as defined herein, i.e. capable of specifically binding to) the p28 subunit and EB1-3, but not directed against (i.e. not capable of specifically binding to) any of the subunits p19, p35 and/or p40 (or, according to an even more specific aspect, not directed to any p19-like subunit other than p28 and not directed to any p40-like subunit other than EBI-3). Such amino acid sequences (which may for example span the p28/EBI-3 interface in IL-27 as described herein) or polypeptides (which may for example be bispecific polypeptides with at least one binding unit directed against p28 and at least one binding unit directed against EBI3) are expected to be specific for IL-27 compared to IL-12 and IL-23.

The invention also provides amino acid sequences and polypeptides that are directed against receptors for heterodimeric cytokines, in particular for receptors of the heterodimeric cytokines described herein.

More in particular, the invention provides amino acid sequences and polypeptides that are directed against receptors for heterodimeric cytokines, wherein said receptors are receptors for heterodimeric cytokines that are associated with cell-mediated (T_{H}1) immunity.

In one specific, but non-limiting aspect, the invention provides amino acid sequences and polypeptides that are directed against receptors for heterodimeric cytokines, wherein said receptors are receptors for heterodimeric cytokines that contain one or more p19-like subunits, and/or that contain one or more p40-like subunits, and in particular contain one or more of the following subunits: p19, p35, p28, p40 and/or EBI3; or a mutant, variant, allele or homolog of each of the foregoing.

In another specific, but non-limiting aspect, the invention provides amino acid sequences and polypeptides that are directed against receptors for heterodimeric cytokine, wherein said receptors are receptors for heterodimeric cytokines that at least contain the p1.9 subunit.

In another specific, but non-limiting aspect, the invention provides amino acid sequences and polypeptides that are directed against receptors for heterodimeric cytokines, wherein said receptors are receptors for heterodimeric cytokines that at least contain the p35 subunit.

In another specific, but non-limiting aspect, the invention provides amino acid sequences and polypeptides that are directed against receptors for heterodimeric cytokine, wherein said receptors are receptors for heterodimeric cytokines that at least contain the p28 subunit.

In another specific, but non-limiting aspect, the invention provides amino acid sequences and polypeptides that are directed against receptors for heterodimeric cytokine, wherein said receptors are receptors for heterodimeric cytokines that at least contain the p40 subunit.

In another specific, but non-limiting aspect, the invention provides amino acid sequences and polypeptides that are directed against receptors for heterodimeric cytokines, wherein said receptors are receptors for heterodimeric cytokines that at least contain EBI_{3.}

In another specific, but non-limiting aspect, the invention provides amino acid sequences and polypeptides that are directed against a receptor for IL-12, IL-23, IL-27 and/or IL-35, respectively, and preferably against a high-affinity receptor for IL-12, IL-23, IL-27 and/or IL-35, respectively.

In another specific, but non-limiting aspect, the invention provides amino acid sequences and polypeptides that are directed against a receptor for IL-12, and preferably against a high-affinity receptor for IL-12, or against at least one subunit thereof. More preferably, such amino acid sequences and polypeptides are specific for (as defined herein) the (cognate) receptor of IL-12 compared the (cognate) receptor of IL-23R and/or the (cognate) receptor of IL-27.

In another specific, but non-limiting aspect, the invention provides amino acid sequences and polypeptides that are directed against a receptor for IL-23, and preferably against a high-affinity receptor for IL-23, or against at least one subunit thereof. More preferably, such amino acid sequences and polypeptides are specific for (as defined herein) the (cognate) receptor of IL-23 compared to the (cognate) receptor of IL-12 and/or the (cognate) receptor of IL-27.

In another specific, but non-limiting aspect, the invention provides amino acid sequences and polypeptides that are directed against a receptor for IL-27, and preferably against a high-affinity receptor for IL-27, or against. at least one subunit thereof. More preferably, such amino acid sequences and polypeptides are specific for (as defined herein) the (cognate) receptor of IL-27 compared to the (cognate) receptor of IL-12 and the (cognate) receptor of IL-23.

In another specific, but non-limiting aspect, the invention provides amino acid sequences and polypeptides that are directed against a receptor for IL-35, and preferably against a high-affinity receptor for IL-35, or against at least one subunit thereof.

The above amino acid sequences and polypeptides may all be as further described herein.

In another specific, but non-limiting aspect, the invention provides amino acid sequences and polypeptides that are directed against at least one subunit of a receptor for IL-12, II-23, IL-27 and/or IL-35, and that preferably are directed against a subunit of a high-affinity receptor for IL-12, IL-23, IL-27 and/or IL-33. Such amino acid sequences and polypeptides of the invention may for example be directed against a IL-23-like subunit of such a receptor, against a gp130-like subunit of such a receptor, or both (e.g. in the case of bispecific/biparatopic polypeptides of the invention).

Preferably, such amino acid sequences and polypeptides are directed against a IL-23-like subunit of such a receptor such as IL-12Rbeta-2, IL-23R and WSX-1 (with amino acid sequences and polypeptides against gp-130 like subunits such as the IL-12Rbeta-1 subunit or against gp130, although not excluded from the scope of the invention, being less preferred),

In another specific, but non-limiting aspect, the invention provides amino acid sequences and polypeptides that are directed against at least one subunit of a receptor for IL-12, preferably against a high-affinity receptor for IL-12. Preferably, said amino acid sequences and polypeptides are directed against the IL-12Rbeta-2 subunit. More preferably, such amino acid sequences and polypeptides are specific for (as defined herein) the IL-12Rbeta-2 subunit compared to the IL-23R subunit and the WSX-1 subunit. It is expected that such amino acid sequences and polypeptides will be specific for the (cognate) receptor of IL-12 compared to the (cognate) receptor of IL-23 and/or the (cognate) receptor of IL-27.

In another specific, but non-limiting aspect, the invention provides amino acid sequences and polypeptides that are directed against at least one subunit of a receptor for IL-23, and preferably against a high-affinity receptor for IL-23. Preferably, said amino acid sequences and polypeptides are directed against the IL-23R subunit. More preferably, such amino acid sequences and polypeptides are specific for (as defined herein) the IL-23R subunit compared to the IL-12Rbeta-2 subunit and the WSX-1 subunit. It is expected that such amino acid sequences and polypeptides will be specific for the (cognate) receptor of IL-23 compared to the (cognate) receptor of IL-12 and/or the (cognate) receptor of IL-27,

In another specific, but non-limiting aspect, the invention provides amino acid sequences and polypeptides that are directed against at least one subunit of a receptor for IL-27, and preferably against a high-affinity receptor for IL-27. Preferably, said amino acid sequences and polypeptides are directed against the WSX-1 subunit. More preferably, such amino acid sequences and polypeptides are specific for (as defined herein) the WSX-19 subunit IL-23R subunit compared to the IL-12Rbeta-2 subunit and the IL-23R subunit. It is expected that such amino acid sequences and polypeptides will be specific for the (cognate) receptor of IL-27 compared to the (cognate) receptor of IL-12 and/or the (cognate) receptor of IL-23.

The above amino acid sequences and polypeptides may all be as further described herein.

The invention also provides amino acid sequences and polypeptides that are directed against IL-12Rbeta-1. Preferably, such amino acid sequences and polypeptides are specific for (as defined herein) IL-12Rbeta-1 compared to gp130.

The invention also provides amino acid sequences and polypeptides that are directed against gp130. Preferably, such amino acid sequences and polypeptides are specific for (as defined herein) gp130 compared to IL-12Rbeta-1.

These amino acid sequences and polypeptides may all be as further described herein.

In another specific, but non-limiting aspect, the invention provides bispecific polypeptides that are directed against a first subunit of a receptor for a heterodimeric cytokine, and against a second subunit of a receptor for a heterodimeric cytokine different from said first subunit.

For example, such a bispecific polypeptide of the invention may comprise at least one amino acid sequence of the invention (such as a Nanobody) that is directed against a gp130-like subunit (such as the gp130 or IL-12beta- subunit, or variants, mutants, alleles or homologs thereof), and at least one amino acid sequence of the invention (such as a Nanobody) that is directed against an IL-23 like subunit (such as IL-12Rbeta-2, IL-23 or WSX- 1). Preferably, such a bispecific) polypeptide is such that it is directed against a gp130-like subunit and an IL-23 like subunit that form part of the same receptor. Such bispecific polypeptides may for example trigger, facilitate and/or enhance activation and/or association of the receptor (or more generally receptor-mediated signalling), for example by mimicking the effects of ligand binding; and thus act as an agonist for the receptor, its ligand and/or the relevant heterodimeric cytokine-mediated, signalling (in this respect, it should also be noted that in another aspect the invention comprises polypeptides of the invention that comprise one or more, such as two, three or four, amino acid sequences of the invention that are directed against a single cytokine receptor chain so as to induce dimerisation or oligomerization and leading to activation of the receptor).

Alternatively, such bispecific polypeptides may for example block, inhibit or reduce binding of the ligand to the receptor, or block, inhibit or reduce activation and/or association of the receptor after binding of the ligand, and/or more generally act as an agonist for the receptor, its ligand and/or the relevant heterodimeric cytokine-mediated signalling

For example, the invention provides:
- amino acid sequences and (in particular) polypeptides that are directed against IL12Rbeta1 and IL12Rbeta2, which are expected to be (and preferably are) selective for the IL-12 receptor compared to the IL-23 receptor and the IL-27 receptor. For example, such a biparatopic polypeptide may comprise at least one amino acid sequence of the invention that is directed against IL12Rbeta1 and at least one amino acid sequence of the invention that is directed against IL12Rbeta2;
- amino acid sequences and (in particular) polypeptides that are directed against IL12Rbeta1 and IL23R, which are expected to be (and preferably are) selective for the IL-23 receptor compared to the IL-12 receptor and the IL-27 receptor. For example, such a biparatopic polypeptide may comprise at least one amino acid sequence of the invention that is directed against IL12Rbeta1 and at least one amino acid sequence of the invention that is directed against IL23R;
- amino acid sequences and (in particular) polypeptides that are directed against WSX-1 and gp130, which are expected to be (and preferably are) selective for the IL-27 receptor compared to the IL-12 receptor and the IL-23 receptor. For example, such a biparatopic polypeptide may comprise at least one amino acid sequence of the invention that is directed against WSX-1 and at least one amino acid sequence of the invention that is directed against gap130.

Again, such amino acid sequences and polypeptides may all be as further described herein.

In another non-limiting aspect of the invention, a polypeptide of the invention may be a bispecific polypeptide that comprises at least one amino acid sequence of the invention that is directed against a heterodimeric cytokine (or against at least one subunit thereof), and at least one amino acid sequence of the invention that is directed against a receptor for a heterodimeric cytokine (or against at least one subunit thereof). In particular, in this aspect of the invention, a polypeptide of the invention may comprise at least amino acid sequence of the invention that is directed against a heterodimeric cytokine (or at least one subunit thereof). and at least one amino acid sequence of the invention that is directed against a receptor for said heterodimeric cytokine (or at least one subunit thereof), i.e. against the cognate receptor for said heterodimeric cytokine.

It is expected that such bispecific polypeptides may act as agonists of heterodimeric cytokine, their receptors, and heterodimeric cytokine-mediated signalling, i.e. by promoting or facilitating binding of the heterodimeric cytokine to its receptor, and/or by stabilizing the ligand/receptor complex upon binding of the heterodimeric cytokine to its receptor. For this purposes, such bispecific polypeptides preferably comprise amino acid sequences that do not neutralize binding of the heterodimeric cytokine to the receptor.

Depending on the amino acid sequences that are chosen to form the construct, it is further expected that such bispecific polypeptides may also be designed to act as an antagonists, i.e. link the cytokine to the receptor without activating it, act as a dominant negative regulator since the receptor is then occupied and inactive).

Bispecific polypeptides as described herein can also be linked to a Fc portion as described in Applicant's copending application entitled "Immunoglobulin constructs", which has the same filing date as this application, December 4, 2007.

Also, a bispecific polypeptide that is directed against a heterodimeric cytokine and against a receptor that is not the cognate receptor for said heterodimeric cytokine, may also be used to modulate the signalling that is mediated by the cytokine against which it is directed and (in particular) by the receptor against which it is directed. For example, a bispecific anti-IL12p35 and anti-IL23R polypeptide of the invention could link IL12 to the IL23 receptor and trigger a IL23 signal.

For example, the above bispecific polypeptides may comprise:
- at least one amino acid sequence of the invention that is directed against IL-12 (or at least one subunit thereof, and preferably the p35 subunit), and at least one amino acid sequence of the invention that that is directed against the receptor for IL-12 (or at least one subunit thereof, and preferably the IL-I2Rbeta-2 subunit).
- at least one amino acid sequence of the invention that that is directed against IL-23 (or at least one subunit thereof, and preferably the p19 subunit), and at least one amino acid sequence of the invention that that is directed against the receptor for IL-23 (or at least one subunit thereof, and preferably the IL-23 subunit); or
- at least one amino acid sequence of the invention that that is directed against IL-27 (or at least one subunit thereof, and preferably the p28 subunit), and at least one amino acid sequence of the invention that that is directed against the receptor for IL-27 (or at least one subunit thereof and preferably the WSX- subunit).

Again, such amino acid sequences and polypeptides may all be as further described herein.

When an amino acid sequence or polypeptide of the invention is directed against a heterodimeric cytokine, it may modulate (as defined herein) heterodimeric cytokine-mediated signalling (as defined herein) in several different ways. For example, and although the invention in its broadest sense is not limited to any specific explanation, hypothesis or mechanism, it may be that such an amino acid sequence or polypeptide, upon binding to the heterodimeric cytokine (or to at least one subunit thereof):
- prevents, reduces or inhibits (in part or in full) binding of said heterodimeric cytokine to its receptor (or to at least one subunit thereof);
- prevents, reduces, inhibits the association (i.e. the heterodimerization) of the heterodimeric cytokine (e.g. of its subunits);
- destabilizes the heterodimeric cytokine or otherwise influences the conformation of the heterodimeric cytokine or prevents or reduces the ability of the heterodimeric cytokine to change its confirmation, in particular so as to fully or partially reduce its ability to bind to its receptor (or to at least one subunit thereof) or, upon binding to its receptor, to trigger receptor-mediated signalling;
- still allows the heterodimeric cytokine to bind to its receptor (or to at least one subunit thereof), but upon such binding prevents, reduces, inhibits (in part or in full) the activation and/or dimerization of the receptor (i.e. where the receptor associates upon ligand binding, as is for example the case for the IL-23 receptor, see Parham et al., supra) :
or otherwise prevents, reduces or inhibits the signaling that is caused by binding of the heterodimeric cytokine to its receptor

Thus, according to one non-limiting aspect, an amino acid sequence or polypeptide of the invention that is directed against a heterodimeric cytokine (and that may further be as described herein) is such that, upon binding to the heterodimeric cytokine, it prevents, reduces or inhibits binding of said heterodimeric cytokine to its receptor or to at least one subunit thereof (i.e. compared to the binding of the heterodimeric cytokine to the same receptor without the presence of the amino acid sequence or polypeptide, and by at least 1%, such as by at least 5%, for example by at least 10%, at least 30%, at least 50%, at least 70% and up to 90% or more, as determined by a suitable assay, such as one of the assays mentioned herein and/or used in the Experimental Part).

According to another non-limiting aspect, an amino acid sequence or polypeptide of the invention that is directed against a heterodimeric cytokine (and that may further be as described herein) is such that, upon binding to the "heterodimeric cytokine and following binding of the heterodimeric cytokine to its receptor (or to at least one subunit of the receptor), it prevents, reduces or inhibit activation and/or association of its receptor (i.e. compared to the association of the receptor mediated by the heterodimeric cytokine without the presence of the amino acid sequence or polypeptide, and by at least 1%, such as by at least 5%, for example by at least 10%, at least 30%, at least 50%, at least 70% and up to 90% or more, as determined by a suitable assay, such as one of the assays mentioned herein and/or used in the Experimental Part).

According to another non-limiting aspect, an amino acid sequence or polypeptide of the invention that is directed against a heterodimeric cytokine (and that may further be as described herein) is such that, upon binding to the heterodimeric cytokine, it prevents, reduces or inhibits the signalling of the receptor that is triggered by the heterodimeric cytokine-mediated association of the receptor (i.e. compared to the signalling following heterodimeric cytokine-mediated association of the receptor without the presence of the amino acid sequence or polypeptide, and by at least 1%, such as by at least 5%, for example by at least 10%, at least 30%, at least 50%, at least 70% and up to 90% or more, as determined by a suitable assay, such as one of the assays mentioned herein and/or used in the Experimental Part).

Generally, according to a preferred aspect, an amino acid sequence or polypeptide of the invention that is directed against a heterodimeric cytokine (and that may further be as described herein) is such that, upon binding to the heterodimeric cytokine, it prevents, reduces or inhibits the heterodimeric cytokine-mediated signalling (as defined herein) associated with said heterodimeric cytokine and/or with its receptor (i.e. compared to the heterodimeric cytokine-mediated signalling mediated by the heterodimeric cytokines without the presence of the amino acid sequence or polypeptide, and by at least 1%, such as by at least 5%, for example by at least 10%, at least 30%, at least 50%, at least 70% and up to 90% or more, as determined by a suitable assay, such as one of the assays mentioned herein and/or used in the Experimental Part).

Again, such amino acid sequences and polypeptides may all be as further described herein.

When an amino acid sequence (such as the p19+ sequences, p19- sequences, p40+ sequences, p40- sequences, p35 sequences, IL-27 sequences, IL-12Rb1 sequences, IL-12Rb2 sequences and IL-23 sequences described herein) or polypeptide (such as the - for example multivalent, multispecific and/or biparatopic - constructs described herein that comprise at least one p19+ sequence, p19- sequence, p40+ sequence, p40- sequence, p35 sequence, IL-27 sequence, IL-12Rb1 sequence, IL-12Rb2 sequence and/or IL-23 sequence) of the invention is directed against a receptor for a heterodimeric cytokine, it may modulate (as defined herein) heterodimeric cytokine-mediated signaling (as defined herein) in several different ways. For example, and although the invention in its broadest sense is not limited to any specific explanation, hypothesis or mechanism, it may be that such an amino acid sequence or polypeptide, upon binding to the receptor (or to at least one subunit thereof):
- prevents, reduces or inhibits (in part or in full) binding of the ligand (i.e. of the heterodimeric cytokine that is the ligand of the receptor) to the receptor (or to at least one subunit thereof);
- still allows the ligand to bind to the receptor, but prevents, reduces or inhibits the signalling that would normally be triggered by binding of the ligand to the receptor (for example, and without limitation, influencing the conformation of the receptor or by reducing the ability of the receptor to change its confirmation)
- prevents, reduces, inhibits the activation and/orassociation (e.g. dimerization) of the receptor (e.g. of its subunits), and in particular the association of the receptor that is triggered by binding of the ligand (i.e. of the heterodimeric cytokine that is the ligand of the receptor) to the receptor (or to at least one subunit thereof), as is for example the case for the the IL-23 receptor, see Parham et al., supra;
- still allows ligand-mediated association (e.g. dimerization) of the receptor, but prevents, reduces or inhibits the signalling that would normally be triggered by such association (for example, and without limitation, influencing, the confirmation of the associated receptor or by reducing the ability of the associated receptor to change its confirmation)
or otherwise prevents, reduces or inhibits the signalling that is caused by binding of the heterodimeric cytokine to its receptor or by ligand-mediated association of the receptor.

Thus, according to one non-limiting aspect, an amino acid sequence or polypeptide of the invention that is directed against a receptor for a heterodimeric cytokine (and that may further be as described herein) is such that, upon binding to the receptor (e.g. to at least one subunit thereof), it prevents, reduces or inhibits binding of its ligand to said receptor or to at least one subunit thereof (i.e. compared to the binding of the ligand to said receptor without the presence of the amino acid sequence or polypeptide, and by at least 1%, such as by at least 5%, for example by at least 10%, at least 30%, at least 50%, at least 70% and up to 90% or more, as determined by a suitable assay, such as one of the assays mentioned herein and/or used in the Experimental Part).

According to one non-limiting aspect, an amino acid sequence or polypeptide of the invention that is directed against a receptor for a heterodimeric cytokine (and that may further be as described herein) is such that, upon binding to the receptor (e.g. to at least one subunit thereof), allows the ligand to bind to the receptor, but prevents, reduces or inhibits the signalling that is (or normally would be) triggered by binding of the ligand to the receptor or to at least one subunit thereof (i.e. compared to the signalling upon binding of the ligand to said receptor without the presence of the amino acid sequence or polypeptide, and by at least 1%, such as by at least 5%, for example by at least 10%, at least 30%, at least 50%, at least 70% and up to 90% or more, as determined by a suitable assay, such as one of the assays mentioned herein and/or used in the Experimental Part).

According to another non-limiting aspect, an amino acid sequence or polypeptide of the invention that is directed against a receptor for a "heterodimeric cytokine (and that may further be as described herein) is such that, upon binding to the receptor (or to at least one subunit thereof), it prevents, reduces or inhibits activation and/or association of the receptor, and in particular ligand-mediated association of the receptor (i.e. compared to the ligand-mediated association of the receptor without the presence of the amino acid sequence or polypeptide, and by at least 1%, such as by at least 5%, for example by at least 10%, at least 30%, at least 50%, at least 70% and up to 90% or more, as determined by a suitable assay, such as one of the assays mentioned herein and/or used in the Experimental Part).

According to another non-limiting aspect, an amino acid sequence or polypeptide of the invention that is directed against a receptor for a heterodimeric cytokine (and that may further be as described herein) is such that, upon binding to the receptor, it prevents, reduces or inhibits the signaling that is triggered by ligand-mediated association of the receptor (i.e. compared to the signalling following binding of the ligand to the receptor without the presence of the amino acid sequence or polypeptide, and by at least 1%, such as by at least 5%, for example by at least 10%, at least 30%, at least 50%, at least 70% and up to 90% or more, as determined by a suitable assay, such as one of the assays mentioned herein and/or used in the Experimental Part).

Generally, according to a preferred aspect, an amino acid sequence or polypeptide of the invention that is directed against a receptor for heterodimeric cytokine (and that may further be as described herein) is such that, upon binding to the receptor, it prevents, reduces or inhibits heterodimeric cytokine-mediated signalling (as defined herein) associated with said receptor and/or with its ligand (i.e, compared to the heterodimeric cytokine-mediated signalling without the presence of the amino acid sequence or polypeptide, and by at least 1%, such as by at least 5%, for example by at least 10%, at least 30%, at least 50%, at least 70% and up to 90% or more, as determined by a suitable assay, such as one of the assays mentioned herein and/or used in the Experimental Part).

Again, such amino acid sequences and polypeptides may all be as further described herein.

It will be clear to the skilled person that the above amino acid sequences and polypeptides of the invention will generally act as antagonists of heterodimeric cytokine-mediated signalling (by which is generally meant herein the signaling associated with the heterodimeric cytokine and/or with the receptor for the heterodimeric cytokine, and in particular the signalling that is caused by binding of a heterodimeric cytokine to its receptor, as well as the biological mechanisms and effects that are triggered by such signalling).

However, the invention also relates to amino acid sequences and polypeptides of the invention that act as agonists of heterodimeric cytokine-mediated signalling. For example, such agonists may be amino acid sequences or polypeptides of the invention that can bind to a receptor for a heterodimeric cytokine (such as the receptor for IL-12, IL-23, IL-27 or IL-35) or to at least one subunit thereof so as to trigger receptor mediated signalling. It is also expected that the some of the above-described bispecific polypeptides that comprise at least one amino acid sequence of the invention that is directed against a heterodimeric cytokine (or against at least one subunit thereof) and at least one amino acid sequence of the invention that is directed against a receptor for said heterodimeric cytokine (or against at least one subunit thereof) may act as agonists for heterodimeric cytokine-mediated signalling, as further described herein.. For this purpose, such bispecific polypeptides preferably comprise amino acid sequences that do not neutralize binding of the heterodimeric cytokine to the receptor.

It is also within the scope of the invention to use parts, fragments, analogs, mutants, variants, alleles and/or derivatives of the amino acid sequences and polypeptides of the invention, and/or to use proteins or polypeptides comprising or essentially consisting of one or more of such parts, fragments, analogs, mutants, variants, alleles and/or derivatives, as long as these are suitable for the uses envisaged herein. Such parts, fragments, analogs, mutants, variants, alleles and/or derivatives will usually contain (at least part of) a functional antigen-binding site for binding against heterodimeric cytokines and/or their receptors; and more preferably will be capable of specific binding to heterodimeric cytokines and/or their receptors, and even more preferably capable of binding to heterodimeric cytokines and/or their receptors with an affinity (suitably measured and/or expressed as a K_{D}-value (actual or apparent), a K_{A}-value (actual or apparent), a kₒₙ-rate and/or a k_{off}-rate, or alternatively as an IC₅₀ value, as further described herein) that is as defined herein. Some non-limiting examples of such parts, fragments, analogs, mutants, variants, alleles, derivatives, proteins and/or polypeptides will become clear from the further description herein. Additional fragments or polypeptides of the invention may also be provided by suitably combining (i.e. by linking or genetic fusion) one or more (smaller) parts or fragments as described herein.

In one specific, but non-limiting aspect of the invention, which will be further described herein, such analogs, mutants, variants, alleles, derivatives have an increased half-life in serum (as further described herein) compared to the amino acid sequence from which they have been derived. For example, an amino acid sequence of the invention may be linked (chemically or otherwise) to one or more groups or moieties that extend the half-life (such as PEG), so as to provide a derivative of an amino acid sequence of the invention with increased half-life.In one specific, but non-limiting aspect, the amino acid sequences of the invention (such as the p19+ sequences, p19- sequences, p40+ sequences, p40- sequences, p35 sequences, IL-27 sequences, IL-12Rb1 sequences, IL-12Rb2 sequences and IL-23 sequences described herein) may be amino acid sequences that comprise an immunoglobulin fold or may be amino acid sequences that, under suitable conditions (such as physiological conditions) are capable of forming an immunoglobulin fold (i.e. by folding). Reference is inter alia made to the review by Halaby et al., J. (1999) Protein Eng. 12, 563-71. Preferably, when properly folded so as to form an immunoglobulin fold, such an amino acid sequence is capable of specific binding (as defined herein) to heterodimeric cytokines and/or their receptors; and more preferably capable of binding to heterodimeric cytokines and/or their receptors with an affinity (suitably measured and/or expressed as a K_{D}-value (actual or apparent), a K_{A}-value (actual or apparent), a kₒₙ-rate and/or a k_{off}-rate, or alternatively as an IC₅₀ value, as further described herein) that is as defined herein. Also, parts, fragments, analogs, mutants, variants, alleles and/or derivatives of such amino acid sequences are preferably such that they comprise an immunoglobulin fold or are capable for forming, under suitable conditions, an immunoglobulin fold.

In particular, but without limitation, the amino acid sequences of the invention (such as the p19+ sequences, p19- sequences, p40+ sequences, p40- sequences, p35 sequences, IL-27 sequences, IL-12Rb1 sequences, IL-12Rb2 sequences and IL-23 sequences described herein) may be amino acid sequences that essentially consist of 4 framework regions (FR1 to FR4 respectively) and 3 complementarity determining regions (CDR1 to CDR3 respectively); or any suitable fragment of such an amino acid sequence (which will then usually contain at least some of the amino acid residues that form at least one of the CDR's, as further described herein).

The amino acid sequences of the invention (such as the p19+ sequences, p19-sequences, p40+ sequences, p40- sequences, p35 sequences, IL-27 sequences, IL-12Rb1 sequences, IL-12Rb2 sequences and IL-23 sequences described herein) may in particular be an immunoglobulin sequence or a suitable fragment thereof, and more in particular be an immunoglobulin variable domain sequence or a suitable fragment thereof, such as light chain variable domain sequence (e.g. a V_{L}-sequence) or a suitable fragment thereof; or a heavy chain variable domain sequence (e.g. a V_{H}-sequence) or a suitable fragment thereof. When the amino acid sequence of the invention is a heavy chain variable domain sequence, it may be a heavy chain variable domain sequence that is derived from a conventional four-chain antibody (such as, without limitation, a V_{H} sequence that is derived from a human antibody) or be a so-called V_{HH}-sequence (as defined herein) that is derived from a so-called "heavy chain antibody" (as defined herein).

However, it should be noted that the invention is not limited as to the origin of the amino acid sequence of the invention (or of the nucleotide sequence of the invention used to express it), nor as to the way that the amino acid sequence or nucleotide sequence of the invention is (or has been) generated or obtained. Thus, the amino acid sequences of the invention may be naturally occurring amino acid sequences (from any suitable species) or synthetic or semi-synthetic amino acid sequences. In a specific but non-limiting aspect of the invention, the amino acid sequence is a naturally occurring immunoglobulin sequence (from any suitable species) or a synthetic or semi-synthetic immunoglobulin sequence, including but not limited to "humanized" (as defined herein) immunoglobulin sequences (such as partially or fully humanized mouse or rabbit immunoglobulin sequences, and in particular partially or fully humanized V_{HH} sequences or Nanobodies), "camelized" (as defined herein) immunoglobulin sequences, as well as immunoglobulin sequences that have been obtained by techniques such as affinity maturation (for example, starting from synthetic, random or naturally occurring immunoglobulin sequences), CDR grafting, veneering, combining fragments derived from different immunoglobulin sequences, PCR assembly using overlapping primers, and similar techniques for engineering immunoglobulin sequences well known to the skilled person; or any suitable combination of any of the foregoing. Reference is for example made to the standard handbooks, as well as to the further description and prior art mentioned herein.

Similarly, the nucleotide sequences of the invention may be naturally occurring nucleotide sequences or synthetic or semi-synthetic sequences, and may for example be sequences that are isolated by PCR from a suitable naturally occurring template (e.g. DNA or RNA isolated from, a cell), nucleotide sequences that have been isolated from a library (and in particular, an expression library), nucleotide sequences that have been prepared by introducing mutations into a naturally occurring nucleotide sequence (using any suitable technique known per se, such as mismatch PCR), nucleotide sequence that have been prepared by PCR using overlapping primers, or nucleotide sequences that have been prepared using techniques for DNA synthesis known per se.

The amino acid sequences of the invention (such as the p19+ sequences, p19-sequences, p40+ sequences, p40- sequences, p35 sequences, IL-27 sequences, IL-12Rb1 sequences, IL-12Rb2 sequences and IL-23 sequences described herein) may in particular be a domain antibody (or an amino acid sequence that is suitable for use as a domain antibody), a single domain antibody (or an amino acid sequence that is suitable for use as a single domain antibody), a "dAb" (or an amino acid sequence that is suitable for use as a dAb) or a Nanobody™ (as defined herein, and including but not limited to a V_{HH} sequence); other single variable domains, or any suitable fragment of any one thereof. For a general description of (single) domain antibodies, reference is also made to the prior art cited above, as well as to EP 0 368 684. For the term "dAb's", reference is for example made to Ward et al. (Nature 1989 Oct 12; 341 (6242): 544-6), to Holt et al., Trends Biotechnol., 2003, 21(11):484-490; as well as to for example WO 06/030220, WO 06/003388 and other published patent applications of Domantis Ltd. It should also be noted that, although less preferred in the context of the present invention because they are not of mammalian origin, single domain antibodies or single variable domains can be derived from certain species of shark (for example, the so-called "IgNAR domains", see for example WO 05/18629).

In particular, the amino acid sequence of the invention may be a Nanobody™ (as defined herein) or a suitable fragment thereof. *[Note: Nonobody*™*, Nanobodies*™ *and Nanoclone*™ *are registered trademarks of Ablynx N. V.]* Such Nanobodies directed against heterodimeric cytokines and/or their receptors will also be referred to herein as *"Nanobodies of the invention".*

For a general description of Nanobodies, reference is made to the further description below, as well as to the prior art cited herein. In this respect, it should however be noted that this description and the prior art mainly described Nanobodies of the so-called "V_{H}3 class" (i.e. Nanobodies with a high degree of sequence homology to human germline sequences of the V_{H}³ class such as DP-47, DP-51 or DP-29), which Nanobodies form a preferred aspect of this invention. It should however be noted that the invention in its broadest sense generally covers any type of Nanobody directed against heterodimeric cytokines and/or their receptors, and for example also covers the Nanobodies belonging to the so-called "V_{H}4 class" (i.e. Nanobodies with a high degree of sequence homology to human germline sequences of the V_{H}4 class such as DP-78), as for example described in the US provisional application 60/792,279 by Ablynx N.V. entitled *"DP-78-like Nanobodies"* filed on April 14, 2006 (see also PCT/EP2007/003259 and WO 07/118670).

Generally, Nanobodies (in particular V_{HH} sequences and partially humanized Nanobodies) can in particular be characterized by the presence of one or more *"Hallmark residues"* (as described herein) in one or more of the framework sequences (again as further described herein).

Thus, generally, a Nanobody™ can be defined as an amino acid sequence with the (general) structure

FR1- CDR1 - FR2 - CDR2 - FR3 - CDR3 - FR4

in which FR1 to FR4 refer to framework regions 1 to 4, respectively, and in which CDR1 to CDR3 refer to the complementarity determining regions I to 3, respectively, and in which one or more of the Hallmark residues are as further defined herein.

In particular, a Nanobody can be an amino acid sequence with the (general) structure

FR1 - CDR1 - FR2 - CDR2 - FR3 - CDR3 - FR4

in which FR1 to FR4 refer to framework regions 1 to 4, respectively, and in which CDR1 to CDR3 refer to the complementarity determining regions 1 to 3, respectively, and in which the framework sequences are as further defined herein.

More in particular, a Nanobody™ can be an amino acid sequence with the (general) structure

FR1 - CDR1 - FR2 - CDR2 - FR3 - CDR3 - FR4

in which FR1 to FR4 refer to framework regions 1 to 4, respectively, and in which CDR1 to CDR3 refer to the complementarity determining regions 1 to 3, respectively, and in which:
i) preferably one or more of the amino acid residues at positions 11, 37, 44, 45, 47, 83, 84, 103, 104 and 108 according to the Kabat numbering are chosen from the Hallmark residues mentioned in Table A-4 below;
   and in which:
ii) said amino acid sequence has at least 80% amino acid identity with at least one of the amino acid sequences of SEQ ID NO's: 1 to 22, in which for the purposes of determining the degree of amino acid identity, the amino acid residues that form the CDR sequences (indicated with X in the sequences of SEQ ID NO's: 1 to 22) are disregarded.

In these Nanobodies, the CDR sequences are generally as further defined herein.

Thus, the invention also relates to such Nanobodies that can bind to (as defined herein) and/or are directed against heterodimeric cytokines and/or their receptors, to suitable fragments thereof, as well as to polypeptides that comprise or essentially consist of one or more of such Nanobodies and/or suitable fragments.
- Some (other) examples of suitable framework sequences are:
- For framework 1: the framework 1 sequences of FR1 Sequences Group 1; FR1 Sequences Group 8; FR1 Sequences Group 15; FR1 Sequences Group 22; FR1 Sequences Group 29; FR1 Sequences Group 36; FR1 Sequences Group 43; FR1 Sequences Group 50 and/or FR1 Sequences Group 57 (see Table A-1 below), or amino acid sequences that have no more than 5, such as 4, 3, 2, or only 1 amino acid difference (as defined herein) with one or more of said framework I sequences (in which case Optional Condition I. Optional Condition II and/or Optional Condition IV (as defined herein) may apply);
- For framework 2: the framework 2 sequences of FR2 Sequences Group 3; FR2 Sequences Group 10; FR2 Sequences Group 17; FR2 Sequences Group 24: FR2 Sequences Group 31; FR2 Sequences Group 38; FR2 Sequences Group 45; FR2 Sequences Group 52 and/or FR2 Sequences Group 59 (see Table A-1 below), or amino acid sequences that have no more than 5, such as 4, 3, 2, or only 1 amino acid difference (as defined herein) with one or more of said framework I sequences (in which case Optional Condition I. Optional Condition II and/or Optional Condition IV (as defined herein) may apply);
- For framework 3: the framework 3 sequences of FR3 Sequences Group 5; FR3 Sequences Group 12; FR3 Sequences Group 19; FR3 Sequences Group 26; FR3 Sequences Group 33; FR3 Sequences Group 40; FR3 Sequences Group 47; FR3 Sequences Group 54 and/or FR3 Sequences Group 61 (see Table A-1 below), or amino acid sequences that have no more than 5, such as 4, 3, 2, or only 1 amino acid difference (as defined herein) with one or more of said framework I sequences(in which case Optional Condition I. Optional Condition II and/or Optional Condition IV (as defined herein) may apply);
- For framework 4: the framework 4 sequences of FR4 Sequences Group 7; FR4 Sequences Group 14; FR4 Sequences Group 21; FR4 Sequences Group 28; FR4 Sequences Group 35; FR4 Sequences Group 42; FR4 Sequences Group 49; FR4 Sequences Group 56 and/or FR4 Sequences Group 63 (see Table A-1 below), or amino acid sequences that have no more than 5, such as 4, 3, 2, or only 1 amino acid difference (as defined herein) with one or more of said framework I sequences (in which case Optional Condition I. Optional Condition II and/or Optional Condition IV (as defined herein) may apply);

In the further description herein, reference will be made to certain groups of amino acid sequences (i.e. framework sequences and CDR sequences). These groups of amino acid sequences (63 in total) are defined in Table A-1 below:

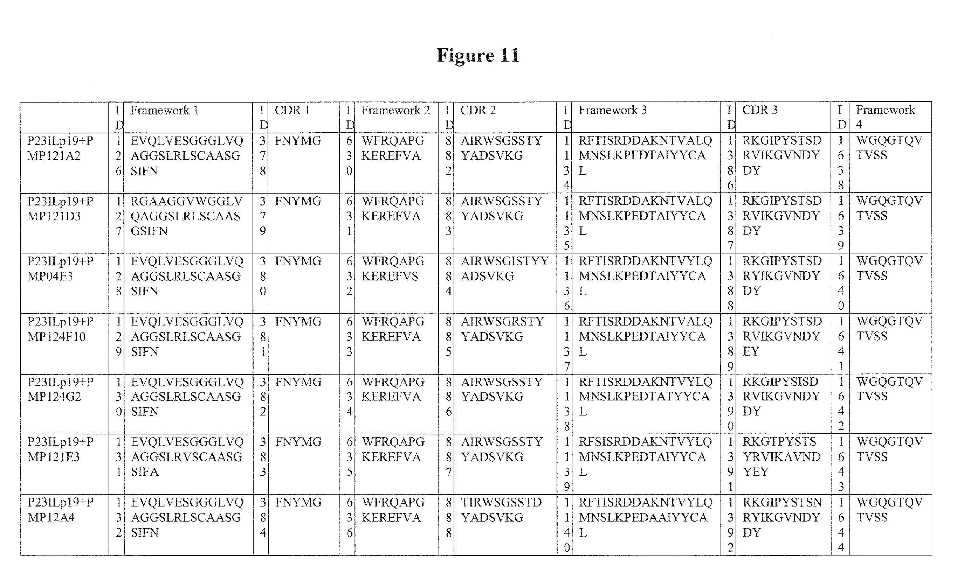

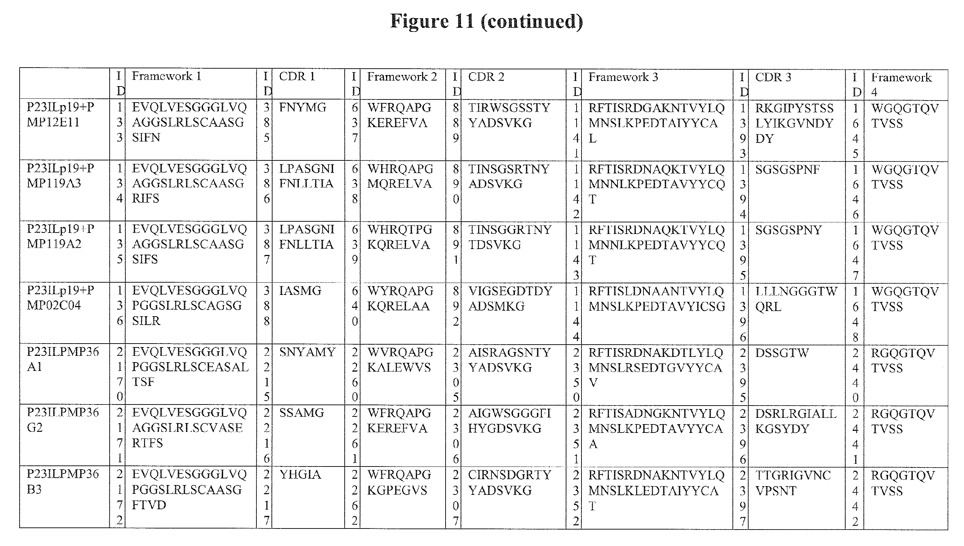

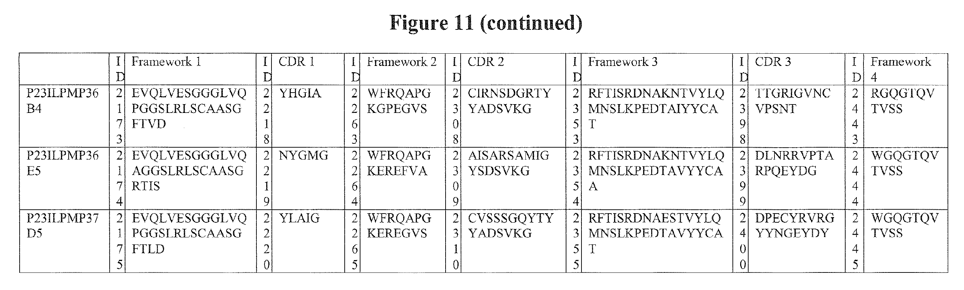

**Table A-1: Framework sequences and CDR sequences referred to in this specification. The SEQ ID NO's refer to the SEQ ID NO's given in the sequence listing and in Figure 11 to 19, respectively.**

| **Framework sequences and CDR sequences derived from p19+ Nanobodies (see also** **Figure 11****)** |
|---|
| FR1 Sequences Group 1: SEQ ID NO's: 126 to 136 and 2170 to 2175 |
| CDR1 Sequences Group 2 : SEQ ID NO's: 378 to 388 and 2215 to 2220 |
| FR2 Sequences Group 3: SEQ ID NO's: 630 to 640 and 2260 to 2265 |
| CDR2 Sequences Group 4: SEQ ID NO's: 882 to 892 and 2305 to 2310 |
| FR3 Sequences Group 5: SEQ ID NO's: 1134 to 1144 and 2350 to 2355 |
| CDR3 Sequences Group 6: SEQ ID NO's: 1386 to 1396 and 2395 to 2400 |
| FR4 Sequences Group 7: SEQ ID NO's: 1638 to 1648 and 2440 to 2445 |

| **Framework sequences and CDR sequences derived from p19- Nanobodies (see also** **Figure 12****)** |
|---|
| FR1 Sequences Group 8: SEQ ID NO's: 137 to 175 and 2187 to 2188 |
| CDR1 Sequences Group 9: SEQ ID NO's: 389 to 427 and 2232 to 2233 |
| FR2 Sequences Group 10: SEQ ID NO's: 641 to 679 and 2277 to 2278 |
| CDR2 Sequences Group 11: SEQ ID NO's: 893 to 931 and 2322 to 2323 |
| FR3 Sequences Group 12: SEQ ID NO's: 1145 to 1183 and 2367 to 2368 |
| CDR3 Sequences Group 13: SEQ ID NO's: 1397 to 1435 and 2412 to 2413 |
| FR4 Sequences Group 14: SEQ ID NO's: 1649 to 1687 and 2457 to 2458 |

| **Framework sequences and CDR sequences derived from p40- Nanobodies (see also** **Figure 13****)** |
|---|
| FR1 Sequences Group 15: SEQ ID NO's: 176 to 181 and 191; 194 ; 204; 207; 208; 210 to 216; 219; 222; 225; 227; 228; 252; 238; 240; 242; 243; 259 ; 260; 264; 269 and 2189 to 2194 |
| CDR1 Sequences Group 16: SEQ ID NO's: 428 to 433 and 443; 446; 456; 459; 460; 462 to 468; 471; 474; 477; 479; 480; 484; 490; 492; 494; 495; 511; 512; 516; 521 and 2234 to 2239 |
| FR2 Sequences Group 17: SEQ ID NO's: 680 to 685 and 695; 698; 708; 711; 712; 714 to 720; 723; 726; 729; 731; 732; 736; 742; 744; 746; 747; 763; 764; 768; 773 and 2279 to 2284 |
| CDR2 Sequences Group 18: SEQ ID NO's: 932 to 937 and 947; 950; 960; 963; 964; 966 to 972; 975; 978; 981; 983; 984; 988; 994; 996; 998; 999; 1015; 1016; 1020; 1025 and 2324 to 2329 |
| FR3 Sequences Group 19: SEQ ID NO's: 1184 to 1189 and 1199; 1202; 1212; 1215; 1216; 1218 to 1224; 1227; 1230; 1233; 1235; 1236; 1240; 1246; 1248; 1250; 1251; 1267; 1268; 1272; 1277 and 2369 to 2374 |
| CDR3 Sequences Group 20: SEQ ID NO's: 1436 to 1441 and 1451; 1454; 1464; 1467; 1468; 1470 to 1476; 1479; 1482; 1485; 1487; 1488; 1492; 1498; 1500; 1502; 1503; 1519; 1520; 1524; 1529 and 2414 to 2419 |
| FR4 Sequences Group 21: SEQ ID NO's: 1688 to 1693 and 1703; 1706; 1716; 1719; 1720; 1722 to 1728; 1731; 1734; 1737; 1739; 1740; 1744; 1750; 1752; 1754; 1755; 1771; 1772; 1776; 1781 and 2459 to 2464 |

| **Framework sequences and CDR sequences derived from p40+ Nanobodies (see also** **Figure 13****)** |
|---|
| FR1 Sequences Group 22: SEQ ID NO's: 178; 182; 184; 186; 188; 189; 190; 192; 193; 195; 198 to 201; 203; 205; 206; 209; 217; 218; 220; 221; 223; 224; 226; 229; 230; 231; 233 to 237; 239; 241; 266 and 2195 to 2213 |
| CDR1 Sequences Group 23: SEQ ID NO's: 430; 434; 436; 438; 440; 441; 442; 444; 443; 447; 450 to 453; 455; 457; 458; 461; 469; 470; 472; 473; 475; 476; 478; 481; 482; 483; 485 to 489; 491; 493; 518 and 2240 to 2258 |
| FR2 Sequences Group 24: SEQ ID NO's: 682; 686; 688; 690; 692; 693; 694; 696; 697; 699; 702 to 705; 707; 709; 710; 713; 721; 722; 724; 725; 727; 728; 730; 733; 734; 735; 737 to 741; 743; 745; 770 and 2285 to 2303 |
| CDR2 Sequences Group 25: SEQ ID NO's: 934; 938; 940; 942; 944; 945; 946; 948; 949; 951; 954 to 957; 959; 961; 962; 965; 973; 974; 976; 977; 979; 980; 982; 985; 986; 987; 989 to 993; 995; 997; 1022 and 2330 to 2348 |
| FR3 Sequences Group 26: SEQ ID NO's: 1186; 1190; 1192; 1194; 1196; 1197; 1198; 1200; 1201; 1203; 1206 to 2209; 1231; 1213; 1214; 1217; 1225; 1226; 1228; 1229; 1231; 1232; 1234; 1237; 1238; 1239; 1241 to 1245; 1247; 1249; 1274 and 2375 to 2393 |
| CDR3 Sequences Group 27: SEQ ID NO's: 1438; 1442; 1444; 1446; 1448; 1449; 1450; 1452; 1453; 1455; 1458 to 1461; 1463; 1465; 1466; 1469; 1477; 1478; 1480; 1481; 1483; 1484; 1486; 1489; 1490; 1491; 1493 to 1497; 1499; 1501; 1526 and 2420 to 2438 |
| FR4 Sequences Group 28: (SEQ ID NO's: 1690; 1694; 1696; 1698; 1700; 1701; 1702; 1704; 1705; 1707; 1710 to 1713; 1715; 1717; 1718; 1721; 1729; 1730; 1732; 1733; 1735; 1736; 1738; 1741; 1742; 1743; 1745 to 1749; 1751: 1753; 1778 and 2465 to 2483 |

| **Framework sequences and CDR sequences derived from p35 Nanobodies (see also** **Figure 15****)** |
|---|
| FR1 Sequences Group 29: SEQ ID NO's: 183; 185; 187; 196; 197; 202; 244 to 258; 261 to 263; 265; 267; 268; 270 to 273 and 2214 |
| CDR1 Sequences Group 30: SEQ ID NO's: 435; 437; 439; 448; 449; 454; 496 to 510; 513 to 515; 517; 519; 520; 522 to 525 and 2259 |
| FR2 Sequences Group 31: SEQ ID NO's: 687; 689; 691; 700; 701; 706; 748 to 762; 765 to 767; 769; 771; 772; 774 to 777 and 2304 |
| CDR2 Sequences Group 32: SEQ ID NO's: 939; 941; 943; 952; 953; 958; 1000 to 1014; 1017 to 1019; 1021; 1023; 1024; 1026 to 1029 and 2349 |
| FR3 Sequences Group 33: SEQ ID NO's: 1191; 1193; 1195; 1204; 1205; 1210; 1252 to 1266; 1269 to 1271; 1273; 1275; 1276; 1278 to 1281 and 2394 |
| CDR3 Sequences Group 34: SEQ ID NO's: 1443; 1445; 1447; 1456; 1457; 1462; 1504 to 1518; 1521 to 1523; 1525; 1527; 1528; 1530 to 1533 and 2439 |
| FR4 Sequences Group 35: SEQ ID NO's: 1695; 1697; 1699; 1708; 1709; 1714; 1756 to 1770; 1773 to 1775; 1777; 1779; 1780; 1782 to 1785 and 2484 |

| **FR sequences and CDR sequences derived from Nanobodies against IL-27 (see also** **Figure 16****)** |
|---|
| FR1 Sequences Group 36: SEQ ID NO's: 274 to 312 |
| CDR1 Sequences Group 37: SEQ ID NO's: 526 to 564 |
| FR2 Sequences Group 38: SEQ ID NO's: 778 to 816 |
| CDR2 Sequences Group 39: SEQ ID NO's: 1030 to 1068 |
| FR3 Sequences Group 40: SEQ ID NO's: 1282 to 1320 |
| CDR3 Sequences Group 41: SEQ ID NO's: 1534 to 1572 |
| FR4 Sequences Group 42: SEQ ID NO's: 1786 to 1824 |

| **FR sequences and CDR sequences derived from Nanobodies against IL-12Rb1 (see also** **Figure 17****)** |
|---|
| FR1 Sequences Group 43: SEQ ID NO's: 313 to 339 |
| CDR1 Sequences Group 44: SEQ ID NO's: 565 to 591 |
| FR2 Sequences Group 45: SEQ ID NO's: 817 to 843 |
| CDR2 Sequences Group 46: SEQ ID NO's: 1069 to 1095 |
| FR3 Sequences Group 47: SEQ ID NO's: 1321 to 1347 |
| CDR3 Sequences Group 48: SEQ ID NO's: 1573 to 1599 |
| FR4 Sequences Group 49: SEQ ID NO's: 1825 to 1851 |

| **FR sequences and CDR sequences derived from Nanobodies against IL-12Rb2 (see also** **Figure 18****)** |
|---|
| FR1 Sequences Group 50: SEQ ID NO's: 340 to 360 |
| CDR1 Sequences Group 51: SEQ ID NO's: 592 to 612 |
| FR2 Sequences Group 52: SEQ ID NO's; 844 to 864 |
| CDR2 Sequences Group 53: SEQ ID NO's: 1096 to 1116 |
| FR3 Sequences Group 54: SEQ ID NO's: 1348 to 1368 |
| CDR3 Sequences Group 55: SEQ ID NO's: 1600 to 1620 |
| FR4 Sequences Group 56: SEQ ID NO's: 1852 to 1872 |

| **FR sequences and CDR sequences derived from Nanobodies against IL-23R (see also** **Figure 19****)** |
|---|
| FR1 Sequences Group 57: SEQ ID NO's: 361 to 377 |
| CDR1 Sequences Group 58: SEQ ID NO's: 613 to 629 |
| FR2 Sequences Group 59: SEQ ID NO's: 865 to 881 |
| CDR2 Sequences Group 60: SEQ ID NO's: 1117 to 1133 |
| FR3 Sequences Group 61: SEQ ID NO's: 1369 to 1385 |
| CDR3 Sequences Group 62: SEQ ID NO's: 1621 to 1637 |
| FR4 Sequences Group 63: SEQ ID NO's: 1873 to 1889 |

Also, in the description herein, reference will be made to certain groups of amino acid sequences that form complete single antigen-binding domains (in this particular case, Nanobody sequences). These groups of sequences are defined in Table A-2 below:

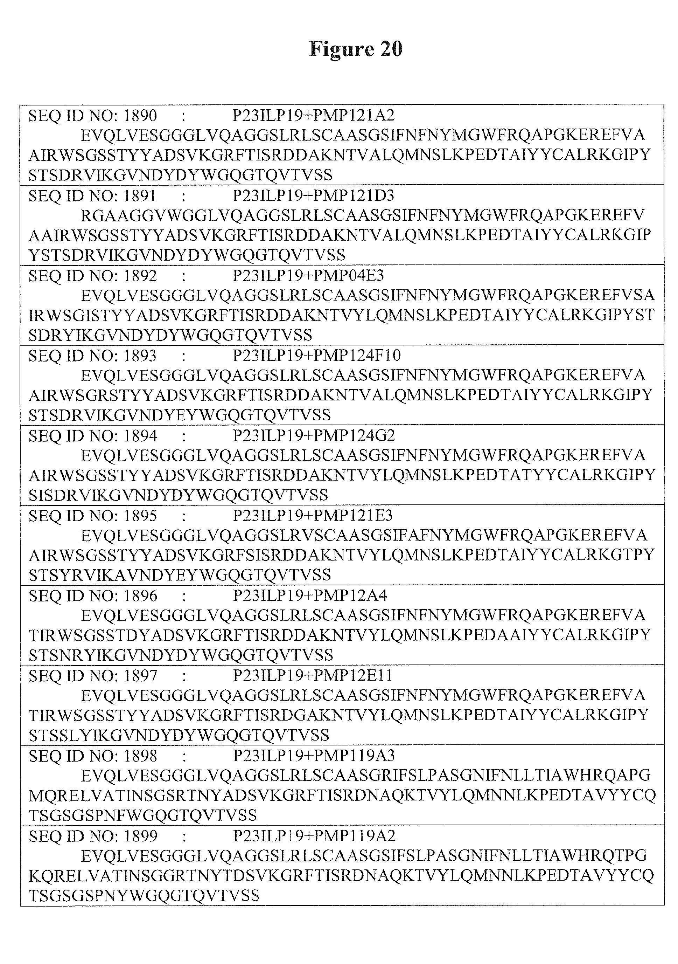

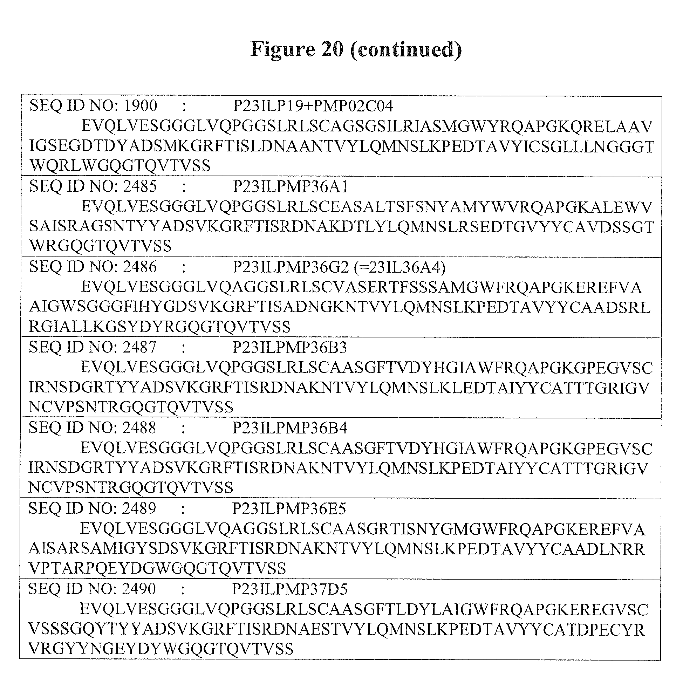

**Table A-2: Non-limiting examples of P19+ sequences, p19-sequences, P40-sequences, P40+ sequences, P35 sequences, IL-27 sequences, IL-12Rb1 sequences, IL-12Rb2 sequences and IL-23R sequences of the invention. The SEQ ID NO's refer to the SEQ ID NO's given in the sequence listing and in Figures 20 to 27, respectively.**

| **Group name** | **SEQ ID NO's** |
|---|---|
| "*P19+ sequences"* (see also Figure 20) | SEQ ID NO's: 1890; 1891; 1892; 1893; 1894; 1895; 1896; 1897; 1898; 1899; 1900; 2485; 2486; 2487; 2488; 2489 and/or 2490 |
| "*P19-sequences"* (see also Figure 21) | SEQ ID NO's: 1901; 1902; 1903; 1904; 1905; 1906; 1907; 1908; 1909; 1910; 1911; 1912; 1913; 1914; 1915; 1916; 1917; 1918; 1919; 1920; 1921; 1922; 1923; 1924; 1925; 1926; 1927; 1928; 1929; 1930; 1931; 1932; 1933; 1934; 1935; 1936; 1937; 1938; 1939; 2502 and/or 2503 |
| *"P40-sequences"* (see also Figure 22) | SEQ ID NO's: 1940; 1941; 1942; 1943; 1944; 1945; 1955; 1958; 1968; 1971; 1972; 1974; 1975; 1976; 1977; 1978; 1979; 1980; 1983; 1986; 1989; 1991; 1992; 1996; 2002; 2004; 2006; 2007; 2023; 2024; 2028; 2433; 2504; 2505; 2506; 2507; 2508 and/or 2509 |
| *"P40+sequences"* (see also Figure 23) | SEQ ID NO's: 1942; 1946; 1948; 1950; 1952; 1953; 1954; 1956; 1957; 1959; 1962; 1963; 1964; 1965; 1967; 1969; 1970; 1973; 1981; 1982; 1984; 1985; 1987; 1988; 1990; 1993; 1994; 1995; 1997; 1998; 1999; 2000; 2001; 2003; 2005; 2030; 2510; 2511; 2512; 2513; 2514; 2515; 2516; 2317; 2518; 2519; 2520; 2521; 2522; 2523; 2524; 2525; 2526; 2527 and/or 2528 |
| *"P35 sequences"* (see also Figure 24) | SEQ ID NO's: 1947; 1949; 1951; 1960; 1961; 1966; 2008; 2009; 2010; 2011; 2012; 2013; 2014; 2015; 2016; 2017; 2018; 2019; 2020; 2021; 2022; 2025; 2026; 2027; 2029; 2031; 2032; 2034; 2035; 2036; 2037 and/or 2529 |
| *"IL-27 sequences "*(see also Figure 26) | SEQ ID NO's: 2038; 2039; 2040; 2041; 2042; 2043; 2044; 2045; 2046; 2047; 2048; 2049; 2050; 2051; 2052, 2053; 2054; 2055; 2056; 2057; 2058; 2059; 2060, 2061; 2062; 2063; 2064; 2065; 2066; 2067; 2068; 2069; 2070; 2071; 2072; 2073; 2074; 2075 and/or 2076 |
| *"IL-12Rb1 sequences"* (see also Figure 27) | SEQ ID NO's: 2077; 2078; 2079; 2080; 2081; 2082; 2083; 2084; 2085; 2086; 2087; 2088; 2089; 2090; 2091; 2092; 2093; 2094; 2095; 2096; 2097; 2098; 2099; 2100; 2101; 2102 and/or 2103 |
| *"IL-12Rb2 sequences"* (see also Figure 28) | SEQ ID NO's: 2104; 2105; 2106; 2107; 2108; 2109; 2110; 2111; 2112; 2113; 2114; 2115; 2116; 2117; 2118; 2119; 2120; 2121; 2122; 2123 and/or 2124 |
| *"IL-23R sequences"* (see also Figure 29) | SEQ ID NO's: 2125; 2126; 2127; 2128; 2129; 2130; 2131; 2132; 2133; 2134; 2135; 2136; 2137; 2138; 2139; 2140 and/or 2141 |

In particular, the invention in some specific aspects provides:
- amino acid sequences that are directed against (as defined herein) p19 and that have at least 80%, preferably at least 85%, such as 90% or 95% or more sequence identity with at least one of the p19+ sequences listed in Table A-2 above. These amino acid sequences are preferably such that they neutralize binding of IL-23 to its receptor;
- amino acid sequences that cross-block (as defined herein) the binding of at least one of the p19+ sequences listed in Table A-2 above to p19 and/or that compete with at least one of the p19+ sequences listed in Table A-2 abovefor binding to p19.
- amino acid sequences that are directed against (as defined herein) p19 and that have at least 80%, preferably at least 85%, such as 90% or 95% or more sequence identity with at least one of the p19- sequences listed in Table A-2 above. These amino acid sequences are preferably such that they essentially do not block or neutralize binding of IL-23 to its receptor;
- amino acid sequences that cross-block (as defined herein) the binding of at least one of the p19- sequences listed in Table A-2 above to p19 and/or that compete with at least one of the p19- sequences listed in Table A-2 above for binding to p19.
- amino acid sequences that are directed against (as defined herein) p40 and that have at least 80%, preferably at least 85%, such as 90% or 95% or more sequence identity with at least one of the p40+ sequences listed in Table A-2 above. These amino acid sequences are preferably such that they neutralize binding of IL-23 and/or IL-12 to its receptor;
- amino acid sequences that cross-block (as defined herein) the binding of at least one of the p40+ sequences listed in Table A-2 above to p40 and/or that compete with at least one of the p40+ sequences listed in Table A-2 above for binding to p40.
- amino acid sequences that are directed against (as defined herein) p40 and that have at least 80%, preferably at least 85%, such as 90% or 95% or more sequence identity with at least one of the p40- sequences listed in Table A-2 above. These amino acid sequences are preferably such that they essentially do not block or neutralize binding of IL-23 or of IL-12 to its receptor;
- amino acid sequences that cross-block (as defined herein) the binding of at least one of the p40- sequences listed in Table A-2 above to p40 and/or that compete with at least one of the p40- sequences listed in Table A-2 above for binding to p40.
- amino acid sequences that are directed against (as defined herein) p35 and that have at least 80%, preferably at least 85%, such as 90% or 95% or more sequence identity with at least one of the p35 sequences listed in Table A-2 above.
- amino acid sequences that cross-block (as defined herein) the binding of at least one of the p35 sequences listed in Table A-2 above to p35 and/or that compete with at least one of the p35 sequences listed in Table A-2 above for binding to p35.
- amino acid sequences that are directed against (as defined herein) IL-12 and that have at least 80%, preferably at least 85%, such as 90% or 95% or more sequence identity with at least one of the p35 sequences, p40+ sequences and/or p40- sequences listed in Table A-2 above;
- amino acid sequences that cross-block (as defined herein) the binding of at least one of the p35 sequences, p40+ sequences and/or p40- sequences listed in Table A-2 above to IL-12 and/or that compete with at least one of the p35 sequences, p40+ sequences and/or p40- sequences listed in Table A-2 above for binding to IL-12.
- amino acid sequences that are directed against (as defined herein) IL-23 and that have at least 80%, preferably at least 85%, such as 90% or 95% or more sequence identity with at least one of the p19+ sequences, p19- sequences, p40+ sequences and/or p40-sequences listed in Table A-2 above;
- amino acid sequences that cross-block (as defined herein) the binding of at least one of the p19+ sequences, p19- sequences, p40+ sequences and/or p40- sequences listed in Table A-2 above to IL-23 and/or that compete with at least one of the p19+ sequences, p19- sequences, p40+ sequences and/or p40- sequences listed in Table A-2 above for binding to IL-23.
- amino acid sequences that are directed against (as defined herein) IL-27 and that have at least 80%, preferably at least 85%, such as 90% or 95% or more sequence identity with at least one of the IL-27 sequences listed in Table A-2 above;
- amino acid sequences that cross-block (as defined herein) the binding of at least one of the IL-27 sequences listed in Table A-2 above to IL-27 and/or that compete with at least one of the IL-27 sequences listed in Table A-2 above for binding to IL-27.
- amino acid sequences that are directed against (as defined herein) IL-12Rb1 and that have at least 80%, preferably at least 85%, such as 90% or 95% or more sequence identity with at least one of the IL-12Rb1 sequences listed in Table A-2 above;
- amino acid sequences that cross-block (as defined herein) the binding of at least one of the IL-12Rb1 sequences listed in Table A-2 aboveto IL-12Rb1 and/or that compete with at least one of the IL-12Rb1 sequences listed in Table A-2 above for binding to IL-12Rb1.
- amino acid sequences that are directed against (as defined herein) the (cognate) receptor for IL-12 and/or the (cognate) receptor for IL-23 and that have at least 80%, preferably at least 85%, such as 90% or 95% or more sequence identity with at least one of the IL-12Rb1 sequences listed in Table A-2 above;
- amino acid sequences that cross-block (as defined herein) the binding of at least one of the IL-12Rb1 sequences listed in Table A-2 above to the (cognate) receptor for IL-12 and/or the (cognate) receptor for IL-23 and/or that compete with at least one of the IL-12Rb1 sequences listed in Table A-2 above for binding to the (cognate) receptor for IL-12 and/or the (cognate) receptor for IL-23.
- amino acid sequences that are directed against (as defined herein) IL-12Rb2 and that have at least 80%, preferably at least 85%, such as 90% or 95% or more sequence identity with at least one of the IL-12Rb2 sequences listed in Table A-2 above;
- amino acid sequences that cross-block (as defined herein) the binding of at least one of the IL-12Rb2 sequences listed in Table A-2 above and/or that compete with at least one of the IL-12Rb2 sequences listed in Table A-2 above for binding to IL-12Rb2;
- amino acid sequences that are directed against (as defined herein) the (cognate) receptor for IL-12 and that have at least 80%, preferably at least 85%, such as 90% or 95% or more sequence identity with at least one of the IL-12Rb2 sequences listed in Table A-2 above;
- amino acid sequences that cross-block (as defined herein) the binding of at least one of the IL-12Rb2 sequences listed in Table A-2 above to the (cognate) receptor for IL-12 and/or that compete with at least one of the IL-12Rb2 sequences listed in Table A-2 above for binding to the (cognate) receptor for IL-12.
- amino acid sequences that are directed against (as defined herein) IL-23R and that have at least 80%, preferably at least 85%, such as 90% or 95% or more sequence identity with at least one of the IL-23R sequences listed in Table A-2;
- amino acid sequences that cross-block (as defined herein) the binding of at least one of the IL-23R sequences listed in Table A-2 to IL-23R and/or that compete with at least one of the IL-23R sequences listed in Table A-2;
- amino acid sequences that are directed against (as defined herein) the (cognate) receptor for IL-23 and that have at least 80%, preferably at least 85%, such as 90% or 95% or more sequence identity with at least one of the IL-23R sequences listed in Table A-2 above;
- amino acid sequences that cross-block (as defined herein) the binding of at least one of the IL-23R sequences listed in Table A-2 above to the (cognate) receptor for IL-23 and/or that compete with at least one of the IL-33R sequences listed in Table A-2 above for binding to the (cognate) receptor for IL-23.
which amino acid sequences may be as further described herein (and may for example be Nanobodies); as well as polypeptides of the invention that comprise one or more of such amino acid sequences (which may be as further described herein, and may for example be bispecific) and/or biparatopic polypeptides as described herein), and nucleic acid sequences that encode such amino acid sequences and polypeptides.

Accordingly, some particularly preferred Nanobodies of the invention are Nanobodies which can bind (as further defined herein) to and/or are directed against to heterodimeric cytokines and/or their receptors and which:
i) have at least 80% amino acid identity with at least one of the amino acid sequences of SEQ ID NO's: 1890 to 2141, 2485 to 2529 and/or 2559 to 2614, in which for the purposes of determining the degree of amino acid identity, the amino acid residues that form the CDR sequences are disregarded. In this respect, reference is also made to the various groups of Framework 1 sequences, Framework 2 sequences, Framework 3 sequences and Framework 4 sequences mentioned Table A-1 (see also Figure 11 to 19) (with respect to the amino acid residues at positions 1 to 4 and 27 to 30 of the framework 1 sequences, reference is also made to the comments made below. Thus, for determining the degree of amino acid identity, these residues are preferably disregarded);
   and in which:
ii) preferably one or more of the amino acid residues at positions 11, 37, 44, 45, 47, 83, 84, 103, 104 and 108 according to the Kabat numbering are chosen from the Hallmark residues mentioned in Table A-4 below.

In these Nanobodies, the CDR1 sequences are generally as further defined herein.

Again, such Nanobodies may be derived in any suitable manner and from any suitable source, and may for example be naturally occurring V_{HH} sequences (i.e. from a suitable species of Camelid) or synthetic or semi-synthetic amino acid sequences, including but not limited to "humanized" (as defined herein) Nanobodies, "camelized" (as defined herein) immunoglobulin sequences (and in particular camelized heavy chain variable domain sequences), as well as Nanobodies that have been obtained by techniques such as affinity maturation (for example, starting from synthetic, random or naturally occurring immunoglobulin sequences), CDR grafting, veneering, combining fragments derived from different immunoglobulin sequences, PCR assembly using overlapping primers, and similar techniques for engineering immunoglobulin sequences well known to the skilled person; or any suitable combination of any of the foregoing as further described herein. Also, when a Nanobody comprises a V_{HH} sequence, said Nanobody may be suitably humanized, as further described herein, so as to provide one or more further (partially or fully) humanized Nanobodies of the invention. Similarly, when a Nanobody comprises a synthetic or semi-synthetic sequence (such as a partially humanized sequence), said Nanobody may optionally be further suitably humanized, again as described herein, again so as to provide one or more further (partially or fully) humanized Nanobodies of the invention.

In particular, humanized Nanobodies may be amino acid sequences that are as generally defined for Nanobodies in the previous paragraphs, but in which at least one amino acid residue is present (and in particular, in at least one of the framework residues) that is and/or that corresponds to a humanizing substitution (as defined herein). Some preferred, but non-limiting humanizing substitutions (and suitable combinations thereof) will become clear to the skilled person based on the disclosure herein. In addition, or alternatively, other potentially useful humanizing substitutions can be ascertained by comparing the sequence of the framework regions of a naturally occurring V_{HH} sequence with the corresponding framework sequence of one or more closely related human V_{H} sequences, after which one or more of the potentially useful humanizing substitutions (or combinations thereof) thus determined can be introduced into said V_{HH} sequence (in any manner known per se, as further described herein) and the resulting humanized V_{HH} sequences can be tested for affinity for the target, for stability, for ease and level of expression, and/or for other desired properties. In this way, by means of a limited degree of trial and error, other suitable humanizing substitutions (or suitable combinations thereof) can be determined by the skilled person based on the disclosure herein. Also, based on the foregoing, (the framework regions of) a Nanobody may be partially humanized or fully humanized.

Some particularly preferred humanized Nanobodies of the invention are humanized variants of the Nanobodies of SEQ ID NO's: 1890 to 2141, 2485 to 2490 and/or 2502 to 2529, which may for example, be humanized variants of Nanobodies that are directed against p19 (for example, humanized variants of Nanobodies that are p19+ sequences or p19-sequences, for example a humanized variant of one of the Nanobodies shown in Figures 20 and 21, respectively), against p40 (for example humanized variants of Nanobodies that are p40- sequences or p40+ sequences, for example a humanized variant of one of the Nanobodies shown in Figures 22 and 23, respectively), against p35 (for example a humanized variant of one of the Nanobodies shown in Figure 24), against IL-27 (for example of one of the Nanobodies shown in Figure 26), against IL-12Rb1 (for example a humanized variant of one of the Nanobodies shown in Figure 27), against IL-12Rb2 (for example a humanized variant of one of the Nanobodies shown in Figure 28), or against IL-23R (for example a humanized variant of one of the Nanobodies shown in Figure 29). Examples of such humanized Nanobodies are given in SEQ ID NO's: 2559 to 2614 (see also Figure 31), and the skilled person will be able to find other suitable humanized variants based on the disclosure herein, optionally after some limited trial-and-error.

Thus, some other preferred Nanobodies of the invention are Nanobodies which can bind (as further defined herein) to "heterodimeric cytokines and/or their receptors and which:
i) are a humanized variant of one of the amino acid sequences of SEQ ID NO's: 1890 to 2141, 2485 to 2490 and/or 2502 to 2529; and/or
ii) have at least 80% amino acid identity with at least one of the amino acid sequences of SEQ ID NO's: 1890 to 2141, 2485 to 2490 and/or 2502 to 2529, in which for the purposes of determining the degree of amino acid identity, the amino acid residues that form the CDR sequences are disregarded;
   and in which:
   i) preferably one or more of the amino acid residues at positions 11, 37, 44, 45, 47, 83, 84, 103, 104 and 108 according to the Kabat numbering are chosen from the Hallmark residues mentioned in Table A-4 below.

Another aspect of the invention relates to nanobodies that are directed against p19 from mouse. Some non-limiting examples of such nanobodies are given in SEQ ID NO's: 2491-2501.

According to another specific aspect of the invention, the invention provides a number of stretches of amino acid residues (i.e. small peptides) that are particularly suited for binding to heterodimeric cytokines and/or their receptors (i.e. to p19, p40, p35, IL-12, IL-23, IL-27, IL-12Rb1, IL-12Rb2, IL-23R, the cognate receptor for IL-12 or the cognate receptor for IL-23, respectively, as further described herein). These stretches of amino acid residues may be present in, and/or may be corporated into, an amino acid sequence of the invention, in particular in such a way that they form (part of) the antigen binding site of an amino acid sequence of the invention. As these stretches of amino acid residues were first generated as CDR sequences of heavy chain antibodies or V_{HH} sequences that were raised against heterodimeric cytokines and/or their receptors (or may be based on and/or derived from such CDR sequences, as further described herein), they will also generally be referred to herein as *"CDR sequences"* (i.e. as CDR1 sequences, CDR2 sequences and CDR3 sequences, respectively). It should however be noted that the invention in its broadest sense is not limited to a specific structural role or function that these stretches of amino acid residues may have in an amino acid sequence of the invention, as long as these stretches of amino acid residues allow the amino acid sequence of the invention to bind to heterodimeric cytokines and/or their receptors. Thus, generally, the invention in its broadest sense comprises any amino acid sequence that is capable of binding to heterodimeric cytokines and/or their receptors and that comprises one or more CDR sequences as described herein, and in particular a suitable combination of two or more such CDR sequences, that are suitably linked to each other via one or more further amino acid sequences, such that the entire amino acid sequence forms a binding domain and/or binding unit that is capable of binding to heterodimeric cytokines and/or their receptors. It should however also be noted that the presence of only one such CDR sequence in an amino acid sequence of the invention may by itself already be sufficient to provide an amino acid sequence of the invention that is capable of binding to heterodimeric cytokines and/or their receptors; reference is for example again made to the so-called "Expedite fragments" described in WO 03/050531.

Thus, in another specific, but non-limiting aspect, the amino acid sequence of the invention may be an amino acid sequence that comprises at least one amino acid sequence that is chosen from the group consisting of the CDR1 sequences, CDR2 sequences and CDR3 sequences that are described herein from or any suitable combination thereof. Particularly suitable combinations wll become clear to the skilled person based on the disclosure herein. In particular, an amino acid sequence of the invention may be an amino acid sequence that comprises at least one antigen binding site, wherein said antigen binding site comprises at least one amino acid sequence that is chosen from the group consisting of the CDR1 sequences, CDR2 sequences and CDR3 sequences that are described herein or any suitable combination thereof, such as the combinations that are described herein.

Generally, in this aspect of the invention, the amino acid sequence of the invention may be any amino acid sequence that comprises at least one stretch of amino acid residues, in which said stretch of amino acid residues has an amino acid sequence that corresponds to the sequence of at least one of the CDR sequences described herein. Such an amino acid sequence may or may not comprise an immunoglobulin fold. For example, and without limitation, such an amino acid sequence may be a suitable fragment of an immunoglobulin sequence that comprises at least one such CDR sequence, but that is not large enough to form a (complete) immunoglobulin fold (reference is for example again made to the "Expedite fragments" described in WO 03/050531). Alternatively, such an amino acid sequence may be a suitable "protein scaffold" that comprises least one stretch of amino acid residues that corresponds to such a CDR sequence (i.e. as part of its antigen binding site). Suitable scaffolds for presenting amino acid sequences will be clear to the skilled person, and for example comprise, without limitation, to binding scaffolds based on or derived from immunoglobulins (i.e. other than the immunoglobulin sequences already described herein), protein scaffolds derived from protein A domains (such as Affibodies™), tendamistat, fibronectin, lipocalin, CTLA-4, T-cell receptors, designed ankyrin repeats, avimers and PDZ domains (Binz et al., Nat. Biotech 2005, Vol 23:1257), and binding moieties based on DNA or RNA including but not limited to DNA or RNA aptamers (Ulrich et al., Comb Chem High Throughput Screen 2006 9(8):619-32).

Again, any amino acid sequence of the invention that comprises one or more of these CDR sequences is preferably such that it can specifically bind (as defined herein) to heterodimeric cytokines and/or their receptors, and more in particular such that it can bind to heterodimeric cytokines and/or their receptors with an affinity (suitably measured and/or expressed as a K_{D}-value (actual or apparent), a K_{A}-value (actual or apparent), a kₒₙ-rate and/or a k_{off}-rate, or alternatively as an IC₅₀ value, as further described herein), that is as defined herein.

In particular, the amino acid sequences of the invention may be amino acid sequences that are directed against p19 (which may be "p19+ sequences" or "p19- sequences", both as defined herein); amino acid sequences that are directed against p40 (which may be "p40+ sequences" or "p40- sequences", both as defined herein); amino acid sequences that are directed against p35; amino acid sequences that are directed against IL-23 (which may be amino acid sequences that are directed against p19 or against p40); amino acid sequences that are directed against IL-12(which may be amino acid sequences that are directed against p35 or against p40); amino acid sequences that are directed against IL-23 (which may be amino acid sequences that are directed against p 19 or against p40); amino acid sequences that are directed against IL-27; amino acid sequences that are directed against IL-12Rb1;amino acid sequences that are directed against IL-12Rb2; amino acid sequences that are directed against IL-23R; amino acid sequences that are directed against the cognate receptor of IL-12 (which may be amino acid sequences that are directed against IL-12Rb1 or IL-12Rb2); and/or amino acid sequences that are directed against the cognate receptor of IL-23 (which may be amino acid sequences that are directed against IL-12Rb1 or IL-23R). These amino acid sequences may be as further described herein and form further aspects of the invention (as do Nucleotide sequences/nucleic acids encoding the same, polypeptides comprising the same and the use of these amino acid sequences in such constructs, methods for preparing the same and uses of the same, all as further described herein).

### A) "p19+ sequences".

One specific, but non-limiting aspect relates to "p19+ sequences", which generally are defined herein as amino acid sequences of the invention that are directed against (as defined herein) the p19 subunit (as present in for example IL-23), and that are capable of modulating, neutralizing, blocking and/or inhibiting the binding of a heterodimeric cytokine comprising a p19 subunit to its receptor, and in particular capable of are capable of modulating, neutralizing, blocking and/or inhibiting the binding of IL-23 to IL-23R (for example in the alpha-screen assay of Example 19 or 22).

P19+ sequences may generally be as further described herein (for example, in terms of affinity, specificity etc. for p19) for amino acid sequences of the invention in general. Also, as described herein for the amino acid sequences of the invention, the p19+ sequences are preferably such that they form or are capable of forming (optionally after suitable folding) a single antigen binding domain or antigen binding unit, and may for example be amino acid sequences that comprise an immunoglobulin fold, amino acid sequences that are comprised of four framework regions and three CDR's, and may in particular be domain antibodies, single domain antibodies, VHH's, "dAb's" or Nanobodies (all as further described herein), or suitable fragments thereof.

In one particular aspect, a p19+ sequence may comprise one or more stretches of amino acid residues chosen from the group consisting of:
a) the amino acid sequences from the "CDR1 Sequences Group 2" (as defined and listed in Table A-1; see also Figure 11);
b) amino acid sequences that have at least 80% amino acid identity with at least one of the amino acid sequences from the "CDR1 Sequences Group 2";
c) amino acid sequences that have 3, 2, or 1 amino acid difference with at least one of the amino acid sequences from the "CDR1 Sequences Group 2";
d) the amino acid sequences from the "CDR2 Sequences Group 4" (as defined and listed in Table A-1; see also Figure 11);
e) amino acid sequences that have at least 80% amino acid identity with at least one of the amino acid sequences from the "CDR2 Sequences Group 4";
f) amino acid sequences that have 3, 2, or 1 amino acid difference with at least one of the amino acid sequences from the "CDR2 Sequences Group 4";
g) the amino acid sequences from the "CDR3 Sequences Group 6" (as defined and listed in Table A-1; see also Figure 11);
h) amino acid sequences that have at least 80% amino acid identity with at least one of the amino acid sequences from the "CDR3 Sequences Group 6";
i) amino acid sequences that have 3, 2, or 1 amino acid difference with at least one of the amino acid sequences from the "CDR3 Sequences Group 6"; or any suitable combination thereof

Optionally, when an amino acid sequence of the invention contains one or more amino acid sequences according to b) and/or c), Optional Condition I, Optional Condition II and/or Optional Condition III (all as defined herein) may apply to said amino acid sequence. (i.e. compared to the original amino acid sequence according to a)). Also, optionally, when an amino acid sequence of the invention contains one or more amino acid sequences according to e) and/or f), Optional Condition I, Optional Condition II and/or Optional Condition III (all as defined herein) may apply to said amino acid sequence (i.e. compared to the original amino acid sequence according to d)). Also, optionally, when an amino acid sequence of the invention contains one or more amino acid sequences according to h) and/or i), Optional Condition I, Optional Condition II and/or Optional Condition III (all as defined herein) may apply to said amino acid sequence (i.e. compared to the original amino acid sequence according to g)).

In this specific aspect, the amino acid sequence preferably comprises one or more stretches of amino acid residues chosen from the group consisting of:
a) the amino acid sequences from the "CDR1 Sequences Group 2";
b) the amino acid sequences from the "CDR2 Sequences Group 4", and
c) the amino acid sequences from the "CDR3 Sequences Group 6';
or any suitable combination thereof.

Also, preferably, in such an amino acid sequence, at least one of said stretches of amino acid residues forms part of the antigen binding site for binding against p19.

In a more specific, but again non-limiting aspect, a p19+ sequence may comprise two or more stretches of amino acid residues chosen from the group consisting of:
a) the amino acid sequences from the "CDR1 Sequences Group 2";
b) amino acid sequences that have at least 80% amino acid identity with at least one of the amino acid sequences from the "CDR1 Sequences Group 2";
c) amino acid sequences that have 3, 2, or 1 amino acid difference with at least one of the amino acid sequences from the "CDR1 Sequences Group 2";
d) the amino acid sequences from the "CDR2 Sequences Group 4";
e) amino acid sequences that have at least 80% amino acid identity with at least one of the amino acid sequences from the "CDR2 Sequences Group 4";
f) amino acid sequences that have 3, 2, or 1 amino acid difference with at least one of the amino acid sequences from the "CDR2 Sequences Group 4";
g) the amino acid sequences from the "CDR3 Sequences Group 6";
h) amino acid sequences that have at least 80% amino acid identity with at least one of the amino acid sequences from the "CDR3 Sequences Group 6'';
i) amino acid sequences that have 3, 2, or 1 amino acid difference with at least one of the amino acid sequences from the "CDR3 Sequences Group 6";
such that (i) when the first stretch of amino acid residues corresponds to one of the amino acid sequences according to a), b) or c), the second stretch of amino acid residues corresponds to one of the amino acid sequences according to d), e), f), g), h) or i); (ii) when the first stretch of amino acid residues corresponds to one of the amino acid sequences according to d), e) or f), the second stretch of amino acid residues corresponds to one of the amino acid sequences according to a), b), c), g), h) or i); or (iii) when the first stretch of amino acid residues corresponds to one of the amino acid sequences according to g), h) or i), the second stretch of amino acid residues corresponds to one of the amino acid sequences according to a), b), c), d), e) or f).

In this specific aspect, the amino acid sequence preferably comprises two or more stretches of amino acid residues chosen from the group consisting of:
a) the amino acid sequences from the "CDR1 Sequences Group 2";
b) the amino acid sequences from the "CDR2 Sequences Group 4"; and
c) the amino acid sequences from the '"CDR3 Sequences Group 6";
such that, (i) when the first stretch of amino acid residues corresponds to one of the amino acid sequences from the "CDR1 Sequences Group 2", the second stretch of amino acid residues corresponds to one of the amino acid sequences from the "CDR2 Sequences Group 4" or from the "CDR3 Sequences Group 6"; (ii) when the first stretch of amino acid residues corresponds to one of the amino acid sequences from the "CDR2 Sequences Group 4", the second stretch of amino acid residues corresponds to one of the amino acid sequences from the "CDR1 Sequences Group 2" or from the "CDR3 Sequences Group 6"; or (iii) when the first stretch of amino acid residues corresponds to one of the amino acid sequences from the "CDR3 Sequences Group 6", the second stretch of amino acid residues corresponds to one of the amino acid sequences from the "CDR1 Sequences Group 2" or from the "CDR2 Sequences Group 4".

Also, in such an amino acid sequence, the at least two stretches of amino acid residues again preferably form part of the antigen binding site for binding against p19.

In an even more specific, but non-limiting aspect, a p19+ sequence may comprise three or more stretches of amino acid residues, in which the first stretch of amino acid residues is chosen from the group consisting of:
a) the amino acid sequences from the "CDR1 Sequences Group 2";
b) amino acid sequences that have at least 80% amino acid identity with at least one of the amino acid sequences from the "CDR1 Sequences Group 2";
c) amino acid sequences that have 3, 2, or 1 amino acid difference with at least one of the amino acid sequences from the "CDR1 Sequences Group 2";
   the second stretch of amino acid residues is chosen from the group consisting of:
d) the amino acid sequences from the "CDR2 Sequences Group 4'';
e) amino acid sequences that have at least 80% amino acid identity with at least one of the amino acid sequences from the "CDR2 Sequences Group 4";
f) amino acid sequences that have 3, 2, or 1 amino acid difference with at least one of the amino acid sequences from the "CDR2 Sequences Group 4";
   and the third stretch of amino acid residues is chosen from the group consisting of:
g) the amino acid sequences from the "CDR3 Sequences Group 6";
h) amino acid sequences that have at least 80% amino acid identity with at least one of the amino acid sequences from the "CDR3 Sequences Group 6'';
i) amino acid sequences that have 3, 2, or 1 amino acid difference with at least one of the amino acid sequences from the "CDR3 Sequences Group 6".

Preferably, in this specifc aspect, the first stretch of amino acid residues is chosen from the group consisting of the amino acid sequences from the "CDR1 Sequences Group 2"; the second stretch of amino acid residues is chosen from the group consisting of the amino acid sequences from the "CDR2 Sequences Group 4"; and the third stretch of amino acid residues is chosen from the group consisting of the amino acid sequences from the "CDR3 Sequences Group 6".

Again, preferably, in such an amino acid sequence, the at least three stretches of amino acid residues forms part of the antigen binding site for binding against p19.

Preferred combinations of such stretches of amino acid sequences will become clear from the further disclosure herein.

Preferably, in such amino acid sequences the CDR sequences have at least 70% amino acid identity, preferably at least 80% amino acid identity, more preferably at least 90% amino acid identity, such as 95% amino acid identity or more or even essentially 100% amino acid identity with the CDR sequences of at least one of the p19+ sequences listed in Table A-2 and Figure 20. This degree of amino acid identity can for example be determined by determining the degree of amino acid identity (in a manner described herein) between said amino acid sequence and one or more of the sequences of SEQ ID NO's: 1890; 1891; 1892; 1893; 1894; 1895; 1896; 1897; 1898; 1899; 1900; 2485; 2486; 2487; 2488; 2489 and/or 2490 (see Table A-2 and Figure 20), in which the amino acid residues that form the framework regions are disregarded. Also, such amino acid sequences of the invention can be as further described herein.

Also, such amino acid sequences are preferably such that they can specifically bind (as defined herein) to the p 19 subunit; and more in particular bind to the p 19 subunit with an affinity (suitably measured and/or expressed as a K_{D-Value} (actual or apparent), a K_{A}-value (actual or apparent), a kₒₙ-rate and/or a k_{off}-rate, or alternatively as an IC₅₀ value (all as further) described herein) that is as defined herein.

When the amino acid sequence of the invention essentially consists of 4 framework regions (FR1 to FR4, respectively) and 3 complementarity determining regions (CDR1 to CDR3, respectively), the amino acid sequence of the invention is preferably such that:
- CDR1 is chosen from the group consisting of:
   a) the amino acid sequences from the "CDR1 Sequences Group 2";
   b) amino acid sequences that have at least 80% amino acid identity with at least one of the amino acid sequences from the "CDR1 Sequences Group 2";
   c) amino acid sequences that have 3, 2, or 1 amino acid difference with at least one of the amino acid sequences from the "CDR1 Sequences Group 2";
   and/or
- CDR2 is chosen from the group consisting of:
   d) the amino acid sequences from the "CDR2 Sequences Group 4";
   e) amino acid sequences that have at least 80% amino acid identity with at least one of the amino acid sequences from the "CDR2 Sequences Group 4";
   f) amino acid sequences that have 3, 2, or 1 amino acid difference with at least one of the amino acid sequences from the "CDR2 Sequences Group 4";
   and/or
- CDR3 is chosen from the group consisting of:
   g) the amino acid sequences from the "CDR3 Sequences Group 6";
   h) amino acid sequences that have at least 80% amino acid identity with at least one of the amino acid sequences from the "CDR3 Sequences Group 6";
   i) amino acid sequences that have 3,2, or 1 amino acid difference with at least one of the amino acid sequences from the "CDR3 Sequences Group 6".

In particular, such an amino acid sequence of the invention may be such that CDR1 is chosen from the group consisting of the amino acid sequences from the "CDR1 Sequences Group 2"; and/or CDR2 is chosen from the group consisting of the amino acid sequences from the "CDR2 Sequences Group 4"; and/or CDR3 is chosen from the group consisting of the amino acid sequences from the "CDR3 Sequences Group 6",

In particular, when the amino acid sequence of the invention essentially consists of 4 framework regions (FR1 to FR4, respectively) and 3 complementarity determining regions (CDR1 to CDR3, respectively), the amino acid sequence of the invention is preferably such that:
- CDR1 is chosen from the group consisting of:
   a) the amino acid sequences from the "CDR1 Sequences Group 2";
   b) amino acid sequences that have at least 80% amino acid identity with at least one of the amino acid sequences from the "CDR1 Sequences Group 2";
   c) amino acid sequences that have 3, 2, or 1 amino acid difference with at least one of the amino acid sequences from the "CDR1 Sequences Group 2";
   and
- CDR2 is chosen from the group consisting of:
   d) the amino acid sequences from the "CDR2 Sequences Group 4";
   e) amino acid sequences that have at least 80% amino acid identity with at least one of the amino acid sequences from the "CDR2 Sequences Group 4";
   f) amino acid sequences that have 3, 2, or I amino acid difference with at least one of the amino acid sequences from the "CDR2 Sequences Group 4";
   and
- CDR3 is chosen from the group consisting of:
   g) the amino acid sequences from the "CDR3 Sequences Group 6";
   h) amino acid sequences that have at least 80% amino acid identity with at least one of the amino acid sequences from the "CDR3 Sequences Group 6";
   i) amino acid sequences that have 3, 2, or 1 amino acid difference with at least one of the amino acid sequences from the "CDR3 Sequences Group 6"; or any suitable fragment of such an amino acid sequence

In particular, such an amino acid sequence of the invention may be such that CDR1 is chosen from the group consisting of the amino acid sequences from the "CDR1 Sequences Group 2"; and CDR2 is chosen from the group consisting of the amino acid sequences from the "CDR2 Sequences Group 4"; and CDR3 is chosen from the group consisting of the amino acid sequences from the "CDR3 Sequences Group 6".

Again, preferred combinations of CDR sequences will become clear from the further description herein.

Also, such amino acid sequences are preferably such that they can specifically bind (as defined herein) to the p19 subunit; and more in particular bind to the p19 subunit with an affinity (suitably measured and/or expressed as a K_{D}-value (actual or apparent), a K_{A}-value (actual or apparent), a kₒₙ-rate and/or a k_{off}-rate, or alternatively as an IC₅₀ value (all as further) described herein) that is as defined herein.

In one preferred, but non-limiting aspect, the invention relates to an amino acid sequence that essentially consists of 4 framework regions (FR1 to FR4, respectively) and 3 complementarity determining regions (CDR1 to CDR3, respectively), in which the CDR sequences of said amino acid sequence have at least 70% amino acid identity, preferably at least 80% amino acid identity, more preferably at least 90% amino acid identity, such as 95% amino acid identity or more or even essentially 100% amino acid identity with the CDR sequences of at least one of the amino acid sequences of SEQ ID NO's: 1890; 1891; 1892; 1893; 1894; 1895; 1896; 1897; 1898; 1899; 1944; 2485; 2486; 2487; 2488; 2489 and/or 2490 (see Table A-2 and Figure 20). This degree of amino acid identity can for example be determined by determining the degree of amino acid identity (in a manner described herein) between said amino acid sequence and one or more of the sequences of SEQ ID NO's: 1890; 1891; 1892; 1893; 1894; 1895; 1896; 1897; 1898; 1899; 1900; 2485; 2486; 2487; 2488; 2489 and/or 2490 (see Table A-2 and Figure 20), in which the amino acid residues that form the framework regions are disregarded. Such amino acid sequences of the invention can be as further described herein.

Some preferred, but non-limiting examples of p19+ sequences are the amino acid sequences of SEQ ID NO's: 1890; 1391; 1892; 1893; 1894; 1895; 1896; 1897; 1898; 1899; 1900; 2485; 2486; 2487; 2488; 2489 and/or 2490 (see Table A-2 and Figure 20). Thus, according to another preferred, but non-limiting aspect of the invention, a p19+ sequence is an amino acid sequence that is directed against (as defined herein) the p19 subunit (as present in for example IL-23) and that are capable of modulating, neutralizing, blocking and/or inhibiting the binding of a heterodimeric cytokine comprising a p19 subunit to its receptor, and in particular capable of are capable of modulating, neutralizing, blocking and/or inhibiting the binding of IL-23 to IL-23R (for example in the alpha-screen assay of Example 19 or 22), and that either
a) has at least 70% amino acid identity, preferably at least 80% amino acid identity, more preferably at least 90% amino acid identity, such as 95% amino acid identity or more or even essentially 100% amino acid identity with at least one of the amino acid sequences of SEQ ID NO's: 1890; 1891; 1892; 1893; 1894; 1895; 1896; 1897; 1898; 1899; 1900; 2485; 2486; 2487; 2488; 2489 and/or 2490 (see Table A-2 and Figure 20);
   and/or that
b) has no more than 20, preferably no more than 10, such as 9, 8, 7, 6, 5, 4, 3, 2 or only one amino acid difference with at least one of the amino acid sequences of SEQ ID NO's: 1890; 1891; 1892; 1893; 1894; 1895; 1896; 1897; 1898; 1899; 1900; 2485; 2486; 2487; 2488; 2489 and/or 2490 (see Table A-2 and Figure 20). Preferably, such an amino acid sequence has no more than a total of 5 (such as 4, 3. 2 or only one) such amino acid differences in the CDR's and/or no more than a total of 5 (such as 4, 3.2 or only 1) such amino acid differences in the framework sequences;
   and/or that
c) is either (i) capable of cross-blocking (as defined herein) the interaction of at least one of the amino acid sequences of SEQ ID NO's: 1890; 1891; 1892; 1893; 1894; 1895; 1896; 1897; 1898; 1899; 1900; 2485; 2486; 2487; 2488; 2489 and/or 2490 with the p19 subunit and/or (ii) being able to compete with (i.e. is a competitor for) the binding of at least one of the amino acid sequences of SEQ ID NO's: 1890; 1891; 1892; 1893; 1894; 1895; 1896; 1897; 1898; 1899; 1900;2485;2486; 2487; 2488; 2489 and/or 2490 (see Table A-2 and Figure 20) to the p19 subunit.

In another preferred, but non-limiting aspect, a p19+ sequence is chosen from one of the amino acid sequences of SEQ ID NO's: 1890; 1891; 1892; 1893; 1894; 1895; 1896; 1897; 1898; 1899; 1900; 2485; 2486; 2487; 2488; 2489 and/or 2490 (see Table A-2 and Figure 20).

### B) "p19-sequences".

One specific, but non-limiting aspect relates to "p19- sequences", which generally are defined herein as amino acid sequences of the invention that are directed against (as defined herein) the p19 subunit (as present in for example IL-23), but that (essentially) are not capable of neutralizing or inhibiting the binding of a heterodimeric cytokine comprising a p19 subunit to its receptor (for example, in a suitable alpha-screen assay as exemplified in Examples 19 and 22 for IL-23 and its cognate receptor and for IL-12 and its cognate receptor).

P19- sequences may generally be as further described herein (for example, in terms of affinity, specificity etc. for p19) for amino acid sequences of the invention in general. Also, as described herein for the amino acid sequences of the invention, the p19- sequences are preferably such that they form or are capable of forming (optionally after suitable folding) a single antigen binding domain or antigen binding unit, and may for example be amino acid sequences that comprise an immunoglobulin fold, amino acid sequences that are comprised of four framework regions and three CDR's, and may in particular be domain antibodies, single domain antibodies, VHH's, "dAb's" or Nanobodies (all as further described herein), or suitable fragments thereof.

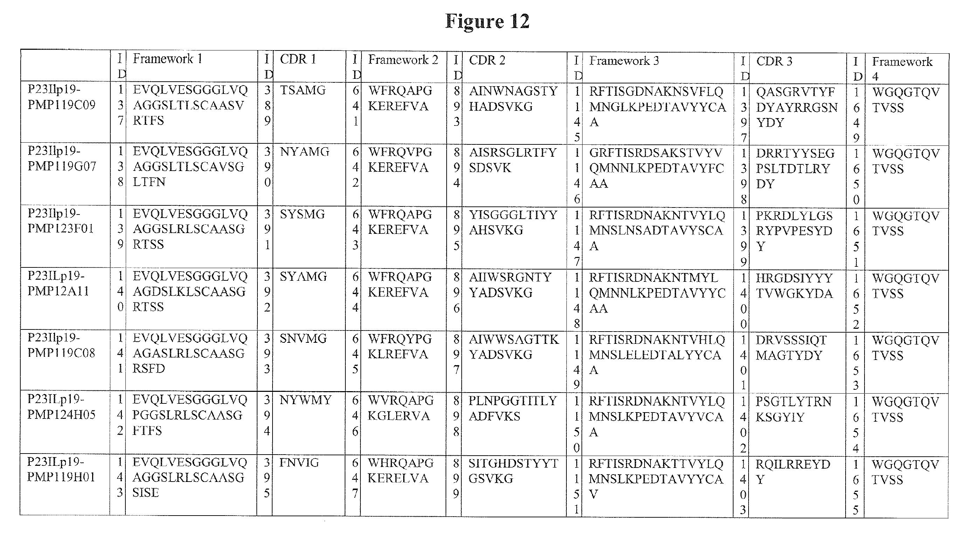

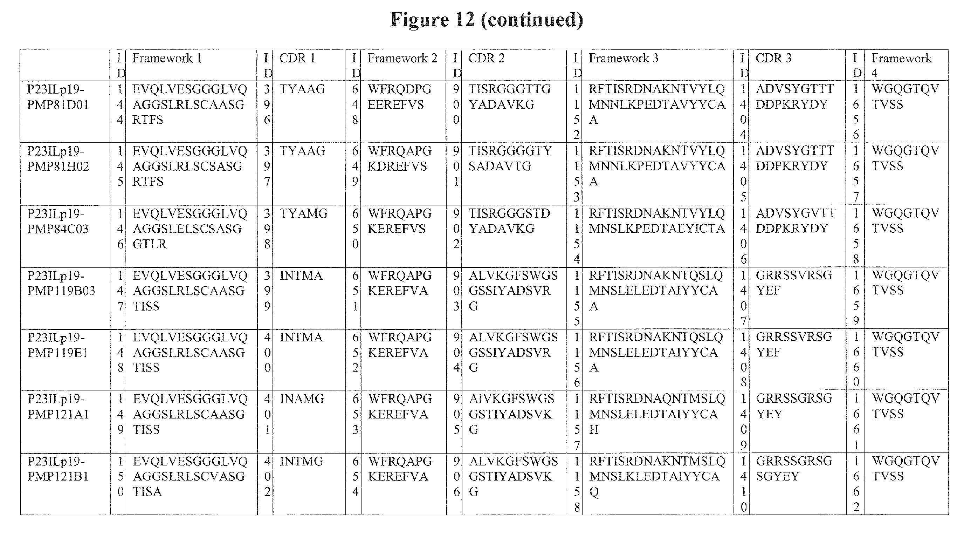

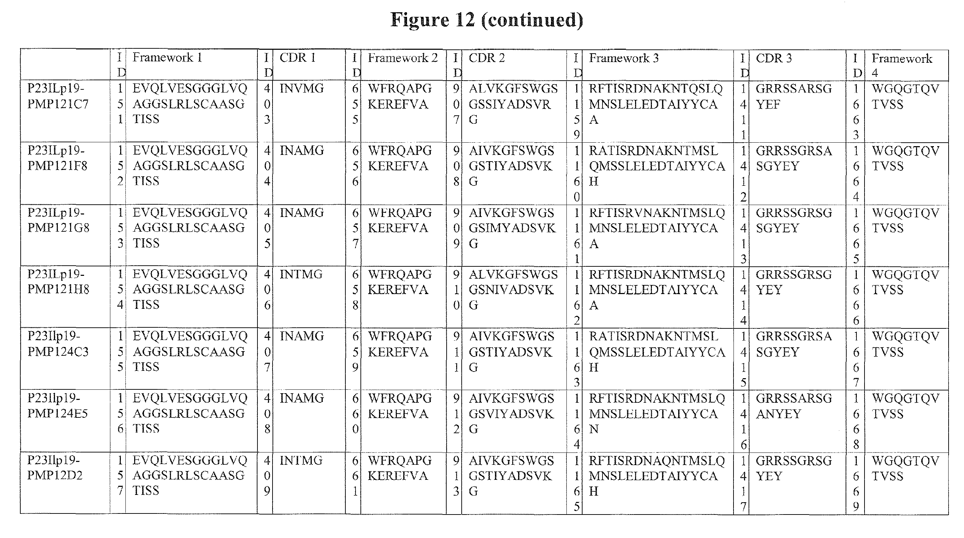

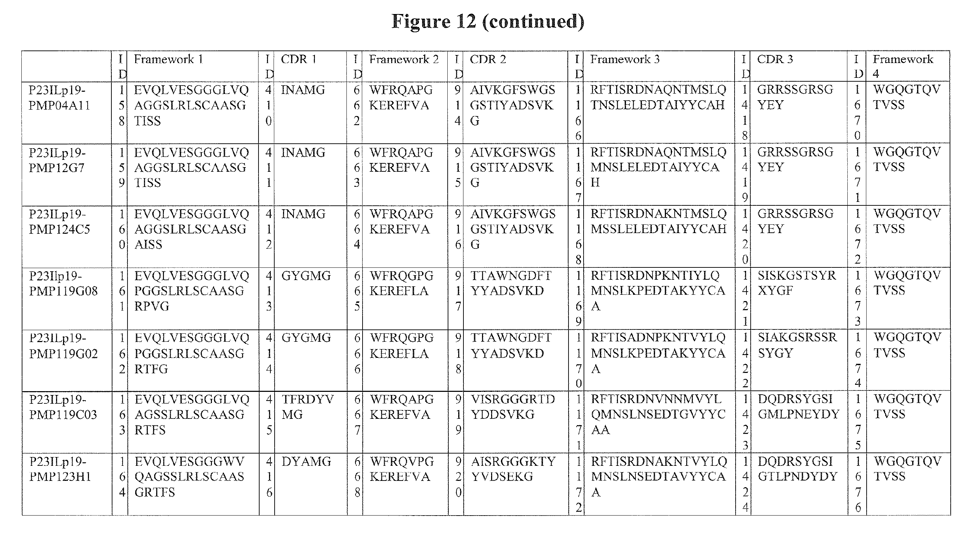

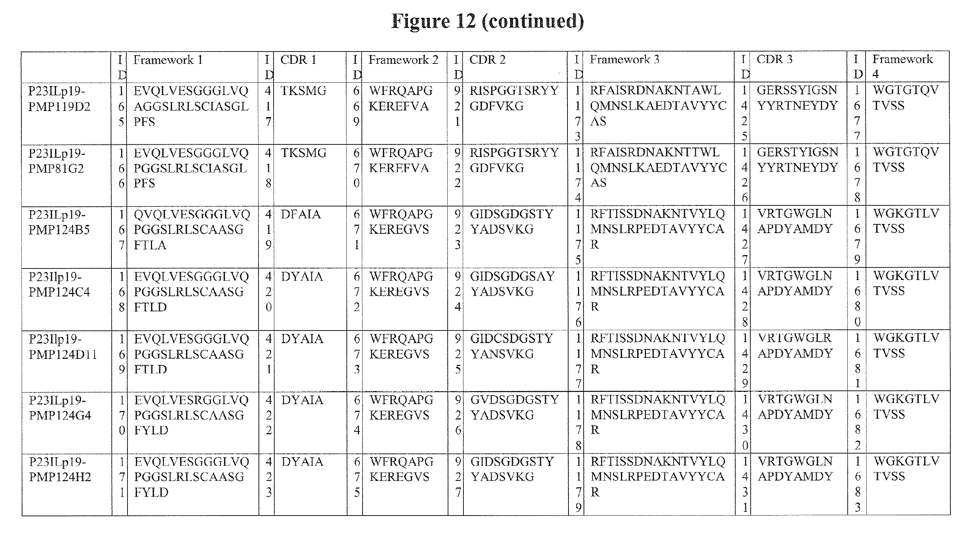

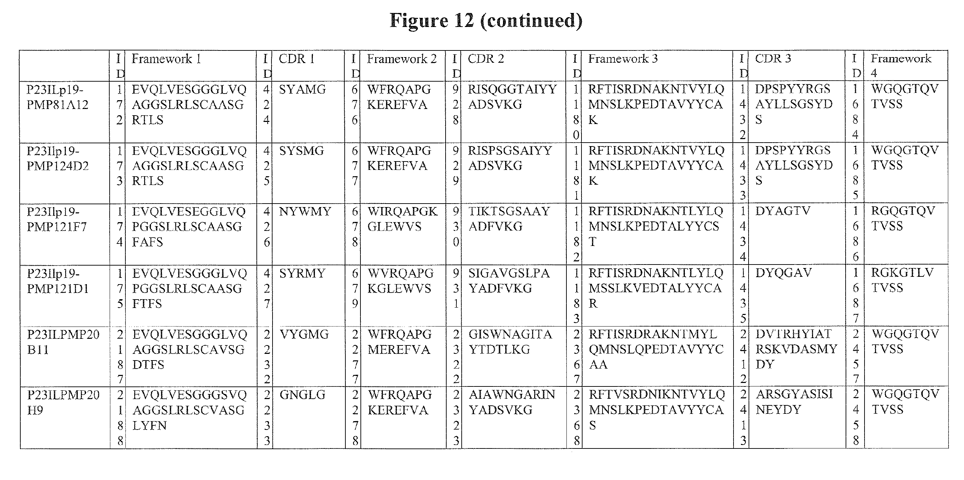

In one particular aspect, a p 19- sequence may comprise one or more stretches of amino acid residues chosen from the group consisting of:
a) the amino acid sequences from the "CDR1 Sequences Group 9" (as defined and listed in Table A-1; see also Figure 12);
b) amino acid sequences that have at least 80% amino acid identity with at least one of the amino acid sequences from the "CDR1 Sequences Group 9";
c) amino acid sequences that have 3, 2, or 1 amino acid difference with at least one of the amino acid sequences from the "CDR1 Sequences Group 9";
d) the amino acid sequences from the "CDR2 Sequences Group 11" (as defined and listed in Table A-1; see also Figure 12);
e) amino acid sequences that have at least 80% amino acid identity with at least one of the amino acid sequences from the "CDR2 Sequences Group 11";
f) amino acid sequences that have 3, 2, or 1 amino acid difference with at least one of the amino acid sequences from the "CDR2 Sequences Group 11";
g) the amino acid sequences from the "CDR3 Sequences Group 13" (as defined and listed in Table A-1; see also Figure 12);
h) amino acid sequences that have at least 80% amino acid identity with at least one of the amino acid sequences from the "CDR3 Sequences Group 13";
i) amino acid sequences that have 3, 2, or 1 amino acid difference with at least one of the amino acid sequences from the "CDR3 Sequences Group 13";
   or any suitable combination thereof.

Optionally, when an amino acid sequence of the invention contains one or more amino acid sequences according to b) and/or c), Optional Condition I, Optional Condition II and/or Optional Condition III (all as defined herein) may apply to said amino acid sequence (i.e. compared to the original amino acid sequence according to a)). Also, optionally, when an amino acid sequence of the invention contains one or more amino acid sequences according to e) and/or f), Optional Condition I, Optional Condition II and/or Optional Condition III (all as defined herein) may apply to said amino acid sequence (i.e. compared to the original amino acid sequence according to d)). Also, optionally, when an amino acid sequence of the invention contains one or more amino acid sequences according to h) and/or i), Optional Condition I, Optional Condition II and/or Optional Condition III (all as defined herein) may apply to said amino acid sequence (i.e. compared to the original amino acid sequence according to g)).

In this specific aspect, the amino acid sequence preferably comprises one or more stretches of amino acid residues chosen from the group consisting of:
a) the amino acid sequences from the "CDR1 Sequences Group 9";
b) the amino acid sequences from the "CDR2, Sequences Group 11"; and
c) the amino acid sequences from the "CDR3 Sequences Group 13";
or any suitable combination thereof.

Also, preferably, in such an amino acid sequence, at least one of said stretches of amino acid residues forms part of the antigen binding site for binding against p19.

In a more specific, but again non-limiting aspect, a p19- sequence may comprise two or more stretches of amino acid residues chosen from the group consisting of:
a) the amino acid sequences from the "CDR1 Sequences Group 9'';
b) amino acid sequences that have at least 80% amino acid identity with at least one of the amino acid sequences from the "CDR1 Sequences Group 9'';
c) amino acid sequences that have 3, 2, or 1 amino acid difference with at least one of the amino acid sequences from the "CDR1 Sequences Group 9";
d) the amino acid sequences from the "CDR2 Sequences Group 11";
e) amino acid sequences that have at least 80% amino acid identity with at least one of the amino acid sequences from the "CDR2 Sequences Group 11";
f) amino acid sequences that have 3, 2, or 1 amino acid difference with at least one of the amino acid sequences from the "CDR2 Sequences Group 11";
g) the amino acid sequences from the "CDR3 Sequences Group 13";
h) amino acid sequences that have at least 80% amino acid identity with at least one of the amino acid sequences from the "CDR3 Sequences Group 13";
i) amino acid sequences that have 3, 2, or 1 amino acid difference with at least one of the amino acid sequences from the "CDR3 Sequences Group 13";
such that (i) when the first stretch of amino acid residues corresponds to one of the amino acid sequences according to a), b) or c), the second stretch of amino acid residues corresponds to one of the amino acid sequences according to d), e), f), g), h) or i); (ii) when the first stretch of amino acid residues corresponds to one of the amino acid sequences according to d), e) or f), the second stretch of amino acid residues corresponds to one of the amino acid sequences according to a), b), c), g), h) or i); or (iii) when the first stretch of amino acid residues corresponds to one of the amino acid sequences according to g), h) or i), the second stretch of amino acid residues corresponds to one of the amino acid sequences according to a), b), c), d), e) or f).

In this specific aspect, the amino acid sequence preferably comprises two or more stretches of amino acid residues chosen from the group consisting of:
a) the amino acid sequences from the "CDR1 Sequences Group 9";
b) the amino acid sequences from the "CDR2 Sequences Group 11"; and
c) the amino acid sequences from the "CDR3 Sequences Group 13";
such that, (i) when the first stretch of amino acid residues corresponds to one of the amino acid sequences from the "CDR1 Sequences Group 9", the second stretch of amino acid residues corresponds to one of the amino acid sequences from the "CDR2 Sequences Group 11'' or from the "CDR3 Sequences Group 13"; (ii) when the first stretch of amino acid residues corresponds to one of the amino acid sequences from the "CDR2 Sequences Group 11", the second stretch of amino acid residues corresponds to one of the amino acid sequences from the "CDR1 Sequences Group 9" or from the "CDR3 Sequences Group 13''; or (iii) when the first stretch of amino acid residues corresponds to one of the amino acid sequences from the "CDR3 Sequences Group 13", the second stretch of amino acid residues corresponds to one of the amino acid sequences from the "CDR1 Sequences Group 9" or from the "CDR2 Sequences Group 11".

Also, in such an amino acid sequence, the at least two stretches of amino acid residues again preferably form part of the antigen binding site for binding against p19.

In an even more specific, but non-limiting aspect, a p19- sequence may comprise three or more stretches of amino acid residues, in which the first stretch of amino acid residues is chosen from the group consisting of:
a) the amino acid sequences from the "CDR1 Sequences Group 9";
b) amino acid sequences that have at least 80% amino acid identity with at least one of the amino acid sequences from the "CDR1 Sequences Group 9";
c) amino acid sequences that have 3, 2, or 1 amino acid difference with at least one of the amino acid sequences from the "CDR1 Sequences Group 9";
   the second stretch of amino acid residues is chosen from the group consisting of:
d) the amino acid sequences from the "CDR2 Sequences Group 11";
e) amino acid sequences that have at least 80% amino acid identity with at least one of the amino acid sequences from the "CDR2 Sequences Group 11";
f) amino acid sequences that have 3, 2, or 1 amino acid difference with at least one of the amino acid sequences from the "CDR2 Sequences Group 11";
   and the third stretch of amino acid residues is chosen from the group consisting of:
g) the amino acid sequences from the "CDR3 Sequences Group 13";
h) amino acid sequences that have at least 80% amino acid identity with at least one of the amino acid sequences from the "CDR3 Sequences Group 13";
i) amino acid sequences that have 3, 2, or 1 amino acid difference with at least one of the amino acid sequences from the "CDR3 Sequences Group 13".

Preferably, in this specifc aspect, the first stretch of amino acid residues is chosen from the group consisting of the amino acid sequences from the "CDR1 Sequences Group 9"; the second stretch of amino acid residues is chosen from the group consisting of the amino acid sequences from the "CDR2 Sequences Group 11"; and the third stretch of amino acid residues is chosen from the group consisting of the amino acid sequences from the "CDR3 Sequences Group 13".

Again, preferably, in such an amino acid sequence, the at least three stretches of amino acid residues forms part of the antigen binding site for binding against p19.

Preferred combinations of such stretches of amino acid sequences will become clear from the further disclosure herein.

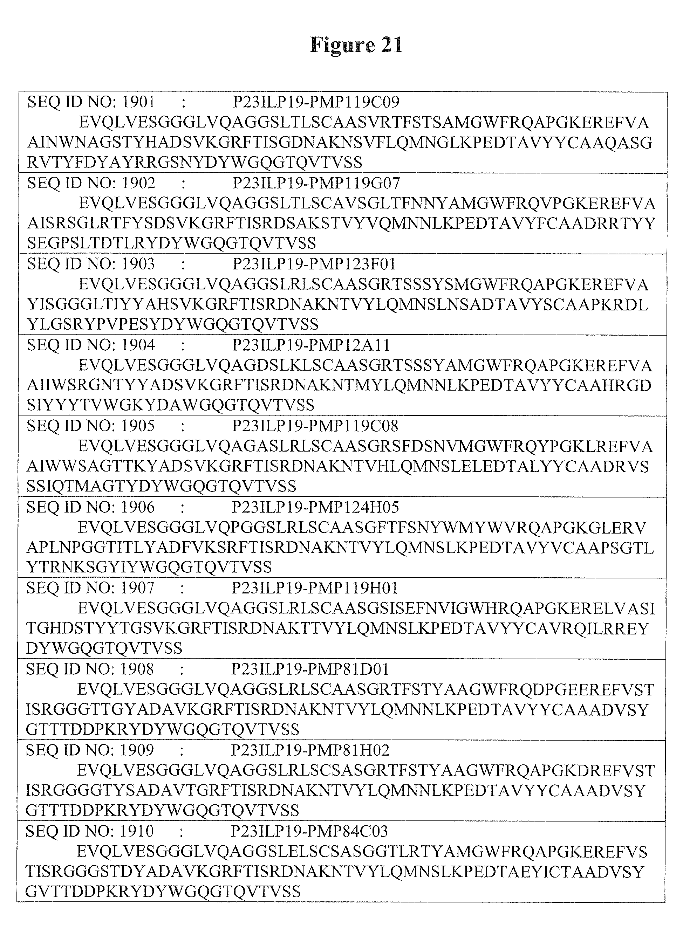

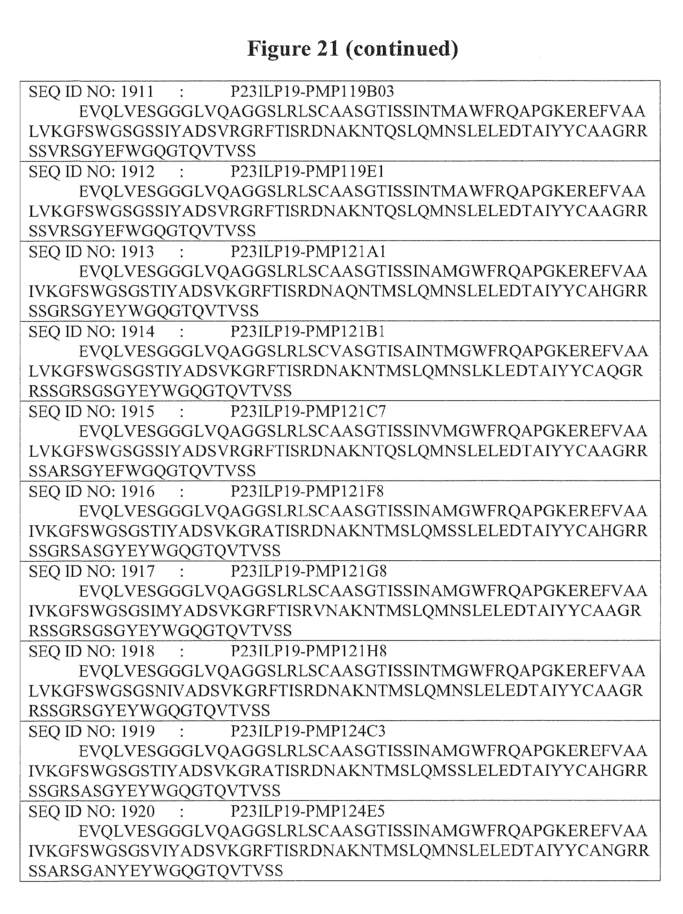

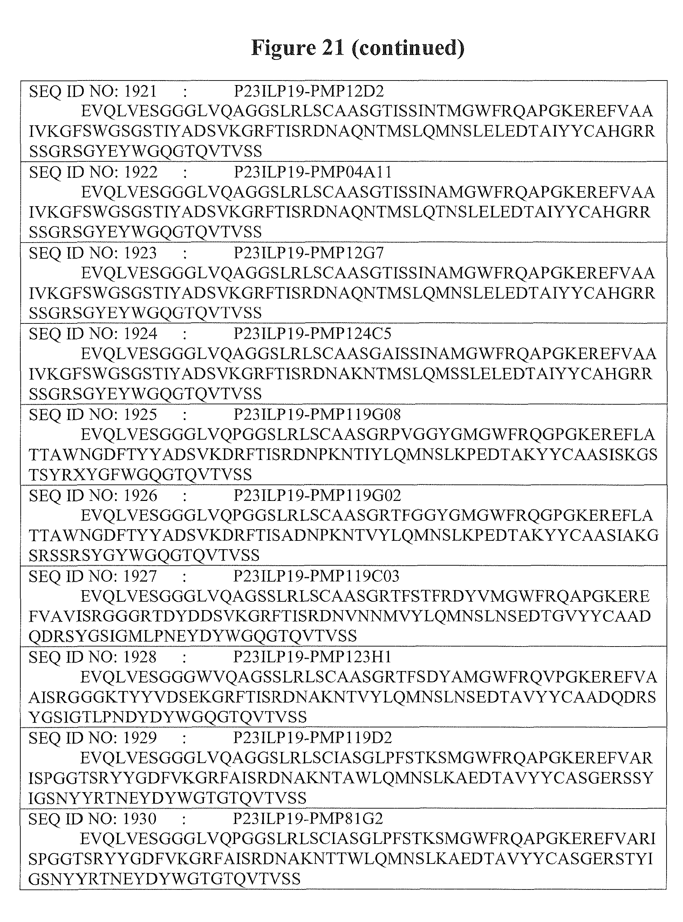

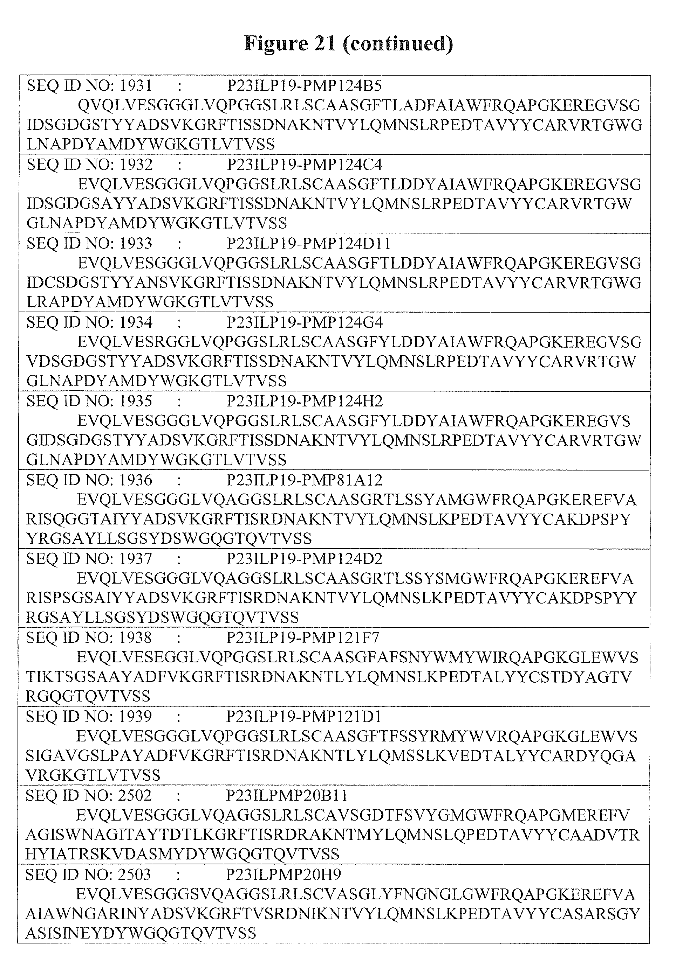

Preferably, in such amino acid sequences the CDR sequences have at least 70% amino acid identity, preferably at least 80% amino acid identity, more preferably at least 90% amino acid identity, such as 95% amino acid identity or more or even essentially 100% amino acid identity with the CDR sequences of at least one of the p19- sequences listed in Table A-2 and Figure 21. This degree of amino acid identity can for example be determined by determining the degree of amino acid identity (in a manner described herein) between said amino acid sequence and one or more of the sequences of SEQ ID NO's: 1901; 1902; 1903; 1904; 1905; 1906; 1907; 1908; 1909; 1910; 1911; 1912; 1913; 1914; 1915; 1916; 1917; 1918; 1919; 1920; 1921; 1922; 1923; 1924; 1925; 1926; 1927; 1928; 1929; 1930; 1931; 1932; 1933; 1934; 1935; 1936; 1937; 1938; 1939; 2502 and/or 2503 (see Table A-2 and Figure 21), in which the amino acid residues that form the framework regions are disregarded. Also, such amino acid sequences of the invention can be as further described herein.

Also, such amino acid sequences are preferably such that they can specifically bind (as defined herein) to the p19 subunit; and more in particular bind to the p19 subunit with an affinity (suitably measured and/or expressed as a K_{D}-value (actual or apparent), a K-A-value (actual or apparent), a kₒₙ-rate and/or a k_{off}-rate, or alternatively as an IC₅₀ value (all as further) described herein) that is as defined herein.

When the amino acid sequence of the invention essentially consists of 4 framework regions (FR1 to FR4, respectively) and 3 complementarity determining regions (CDR1 to CDR3, respectively), the amino acid sequence of the invention is preferably such that:
- CDR1 is chosen from the group consisting of:
   a) the amino acid sequences from the "CDR1 Sequences Group 9";
   b) amino acid sequences that have at least 80% amino acid identity with at least one of the amino acid sequences from the "CDR1 Sequences Group 9";
   c) amino acid sequences that have 3, 2, or 1 amino acid difference with at least one of the amino acid sequences from the "CDR1 Sequences Group 9";
   and/or
- CDR2 is chosen from the group consisting of:
   d) the amino acid sequences from the "CDR2 Sequences Group 11";
   e) amino acid sequences that have at least 80% amino acid identity with at least one of the amino acid sequences from the "CDR2 Sequences Group 11";
   f) amino acid sequences that have 3, 2, or 1 amino acid difference with at least one of the amino acid sequences from the "'CDR2 Sequences Group 11";
   and/or
- CDR3 is chosen from the group consisting of:
   g) the amino acid sequences from the "CDR3 Sequences Group 13";
   h) amino acid sequences that have at least 80% amino acid identity with at least one of the amino acid sequences from the "CDR3 Sequences Group 13";
   i) amino acid sequences that have 3, 2, or 1 amino acid difference with at least one of the amino acid sequences from the "CDR3 Sequences Group 13".

In particular, such an amino acid sequence of the invention may be such that CDR1 is chosen from the group consisting of the amino acid sequences from the "CDR1 Sequences Group 9"; and/or CDR2 is chosen from the group consisting of the amino acid sequences from the "CDR12 Sequences Group 11"; and/or CDR13 is chosen from the group consisting of the amino acid sequences from the "CDR3 Sequences Group 13".

In particular, when the amino acid sequence of the invention essentially consists of 4 framework regions (FR1 to FR4, respectively) and 3 complementarity determining regions (CDR1 to CDR3, respectively), the (amino acid sequence of the invention is preferably such that:
- CDR1 is chosen from the group consisting of:
   a) the amino acid sequences from the "CDR1 Sequences Group 9";
   b) amino acid sequences that have at least 80% amino acid identity with at least one of the amino acid sequences from the "CDR1 Sequences Group 9";
   c) amino acid sequences that have 3, 2, or 1 amino acid difference with at least one of the Amino acid sequences from the "CDR1 Sequences Group 9";
   and
- CDR2 is chosen from the group consisting of:
   d) the amino acid sequences from the "CDR2 Sequences Group 11";
   e) amino acid sequences that have at least 80% amino acid identity with at least one of the amino acid sequences from the "CDR2 Sequences Group 11";
   f) amino acid sequences that have 3, 2, or 1 amino acid difference with at least one of the amino acid sequences from the "CDR2 Sequences Group 11";
   and
- CDR3 is chosen from the group consisting of:
   g) the amino acid sequences from the "CDR3 Sequences Group 13";
   h) amino acid sequences that have at least 80% amino acid identity with at least one of the amino acid sequences from the "CDR3 Sequences Group 13";
   i) amino acid sequences that have 3, 2, or 1 amino acid difference with at least one of the amino acid sequences from the "CDR3 Sequences Group 13"; or any suitable fragment of such an amino acid sequence

In particular, such an amino acid sequence of the invention may be such that CDR1 is chosen from the group consisting of the amino acid sequences from the "CDR1 Sequences Group 9"; and CDR2 is chosen from the group consisting of the amino acid sequences from the "CDR2 Sequences Group 11"; and CDR3 is chosen from the group consisting of the amino acid sequences from the "CDR3 Sequences Group 13".

Again, preferred combinations of CDR sequences will become clear from the further description herein.

Also, such amino acid sequences are preferably such that they can specifically bind (as defined herein) to the p19 subunit; and more in particular bind to the p19 subunit with an affinity (suitably measured and/or expressed as a K_{D}-value (actual or apparent), a K_{A}-value (actual or apparent), a kₒₙ-rate and/or a k_{off}-rate, or alternatively as an IC₅₀ value (all as further) described herein) that is as defined herein.

In one preferred, but non-limiting aspect, the invention relates to an amino acid sequence that essentially consists of 4 Framework regions (FR1 to FR4, respectively) and 3 complementarity determining regions (CDR1 to CDR3, respectively), in which the CDR sequences of said amino acid sequence have at least 70% amino acid identity, preferably at least 80% amino acid identity, more preferably at least 90% amino acid identity, such as 95% amino acid identity or more or even essentially 100% amino acid identity with the CDR sequences of at least one of the amino acid sequences of SEQ ID NO's: 1901; 1902; 1903 ; 1904; 1905; 1906; 1907; 1908; 1909; 1910; 1911; 1912; 1913; 1914; 1915; 1916; 1917; 1918; 1919; 1920; 1921; 1922; 1923; 1924; 1925; 1926; 1927; 1928; 1929; 1930; 1931; 1932; 1933; 1934; 1935; 1936; 1937; 1938; 1939; 2502 and/or 2503 (see Table A-2 and Figure 21). This degree of amino acid identity can for example be determined by determining the degree of amino acid identity (in a manner described herein) between said amino acid sequence and one or more of the sequences of SEQ ID NO's: 1901; 1902; 1903; 1904; 1905; 1906; 1907; 1908; 1909; 1910; 1911; 1912; 1913; 1914; 1915; 1916; 1917; 1918; 1919; 1920; 1921; 1922; 1923; 1924; 1925; 1926; 1927; 1928; 1929; 1930; 1931; 1932; 1933; 1934; 1935; 1936; 1937; 1938; 1939; 2502 and/or 2503 (see Table A-2 and Figure 21), in which the amino acid residues that form the framework regions are disregarded. Such amino acid sequences of the invention can be as further described herein.

Some preferred, but non-limiting examples of p19- sequence are the amino acid sequences of SEQ ID NO's: 1901; 1902; 1903; 1904; 1905; 1906; 1907; 1908; 1909; 1910; 1911; 1912; 1913; 1914; 1915; 1916; 1917; 1918; 1919; 1920; 1921; 1922; 1923; 1924; 1925; 1926; 1927; 1928; 1929; 1930; 1931; 1932; 1933; 1934; 1935; 1936; 1937; 1938; 1939; 2502 and/or 2503 (see Table A-2 and Figure 21). Thus, according to another preferred, but non-limiting aspect of the invention, a p 19- sequence is an amino acid sequence that is directed against (as defined herein) the p19 subunit (as present in for example IL-23) but that (essentially) are not capable of neutralizing or inhibiting the binding of a heterodimeric cytokine comprising a p19 subunit to its receptor (for example, in a suitable alpha-screen assay as exemplified in Examples 19 and 22 for IL-23 and its cognate receptor and for IL-12 and its cognate receptor), and that either:
a) has at least 70% amino acid identity, preferably at least 80% amino acid identity, more preferably at least 90% amino acid identity, such as 95% amino acid identity or more or even essentially 100% amino acid identity with at least one of the amino acid sequences of SEQ ID NO's: 1901; 1902; 1903; 1904; 1905; 1906; 1907; 1908; 1909; 1910; 1911; 1912; 1913; 1914; 1915; 1916; 1917; 1918; 1919; 1920; 1921; 1922; 1923; 1924; 1925; 1926; 1927; 1928; 1929; 1930; 1931; 1932; 1933; 1934; 1935; 1936; 1937; 1938; 1939; 2502 and/or 2503 (see Table A-2 and Figure 21);
   and/or that
b) has no more than 20, preferably no more than 10, such as 9, 8, 7, 6, 5, 4, 3, 2 or only one amino acid difference with at least one of the amino acid sequences of SEQ ID NO's: 1901; 1902; 1903; 1904; 1905; 1906; 1907; 1908; 1909; 1910; 1911; 1912; 1913; 1914; 1915; 1916; 1917; 1918; 1919; 1920; 1921; 1922; 1923; 1924; 1925; 1926; 1927; 1928; 1929; 1930; 1931; 1932; 1933; 1934; 1935; 1936; 1937; 1938; 1939; 2502 and/or 2503 (see Table A-2 and Figure 21). Preferably, such an amino acid sequence has no more than a total of 5 (such as 4, 3. 2 or only one) such amino acid differences in the CDR's and/or no more than a total of 5 (such as 4, 3. 2 or only 1) such Amino acid differences in the framework sequences;
   and/or that
c) is either (i) capable of cross-blocking (as defined herein) the interaction of at least one of the amino acid sequences of SEQ ID NO's: 1901; 1902; 1903; 1904; 1905; 1906; 1907; 1908; 1909; 1910; 1911; 1912; 1913; 1914; 1915; 1916; 1917; 1918; 1919; 1920; 1921; 1922; 1923; 1924; 1925; 1926; 1927; 1928; 1929; 1930; 1931; 1932; 1933; 1934; 1935; 1936; 1937; 1938; 1939; 2502 and/or 2503 (see Table A-2 and Figure 20) with the p19 subunit and/or (ii) being able to compete with (i.e. is a competitor for) the binding of at least one of the amino acid sequences of SEQ ID NO's: 1901; 1902; 1903; 1904; 1905; 1906; 1907; 1908; 1909; 1910; 1911; 1912; 1913; 1914; 1915; 1916; 1917; 1918; 1919; 1920; 1921; 1922; 1923; 1924; 1925; 1926; 1927; 1928; 1929; 1930; 1931; 1932; 1933; 1934; 1935; 1936; 1937; 1938; 1939; 2502 and/or 2503 (see Table A-2 and Figure 21) to the p19 subunit.

In another preferred, but non-limiting aspect, a p19 sequence is chosen from one of the amino acid sequences of SEQ ID NO's: 1901; 1902; 1903; 1904; 1905; 1906; 1907; 1908; 1909; 1910; 1911; 1912; 1913; 1914; 1915; 1916; 1917; 1918; 1919; 1920; 1921; 1922; 1923; 1924; 1925; 1926; 1927; 1928; 1929; 1930; 1931; 1932; 1933; 1934; 1935; 1936; 1937; 1938; 1939; 2502 and/or 2503 (see Table A-2 and Figure 21).

### C) P40- sequences.

One specific, but non-limiting aspect relates to "p40- sequences", which generally are defined herein as amino acid sequences of the invention that are directed against (as defined herein) the p40 subunit (as present in for example IL-23 and IL-12), but that (essentially) are not capable of neutralizing or inhibiting the binding of a heterodimeric cytokine comprising a p40 subunit to its receptor (for example, in a suitable alpha-screen assay as exemplified in Examples 19 and 22 for IL-23 and its cognate receptor and for IL-12 and its cognate receptor).

P40- sequences may generally be as further described herein (for example, in terms of affinity, specificity etc. for p40) for amino acid sequences of the invention in general. Also, as described herein for the amino acid sequences of the invention, the p40- sequences are preferably such that they form or are capable of forming (optionally after suitable folding) a single antigen binding domain or antigen binding unit, and may for example be amino acid sequences that comprise an immunoglobulin fold, amino acid sequences that are comprised of four framework regions and three CDR's, and may in particular be domain antibodies, single domain antibodies, VHH's, "dAb's" or Nanobodies (all as further described herein), or suitable fragments thereof.

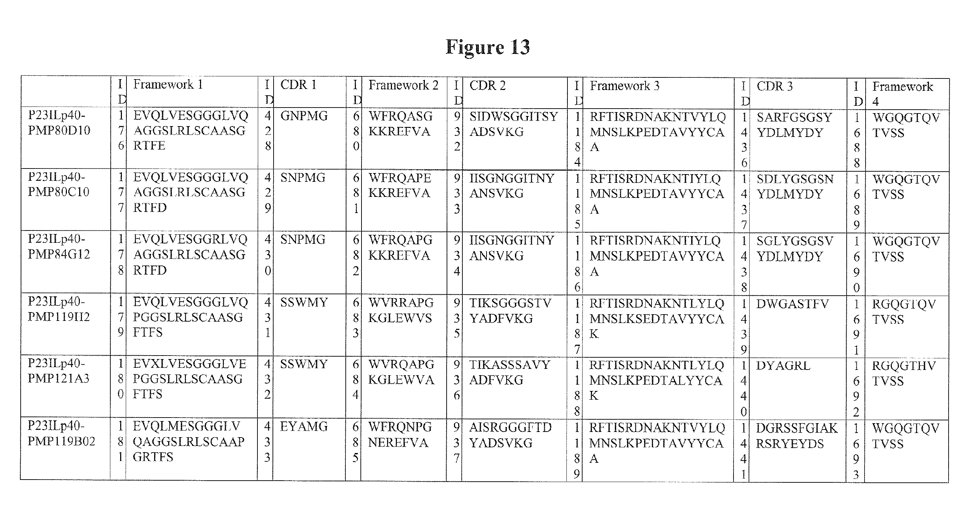

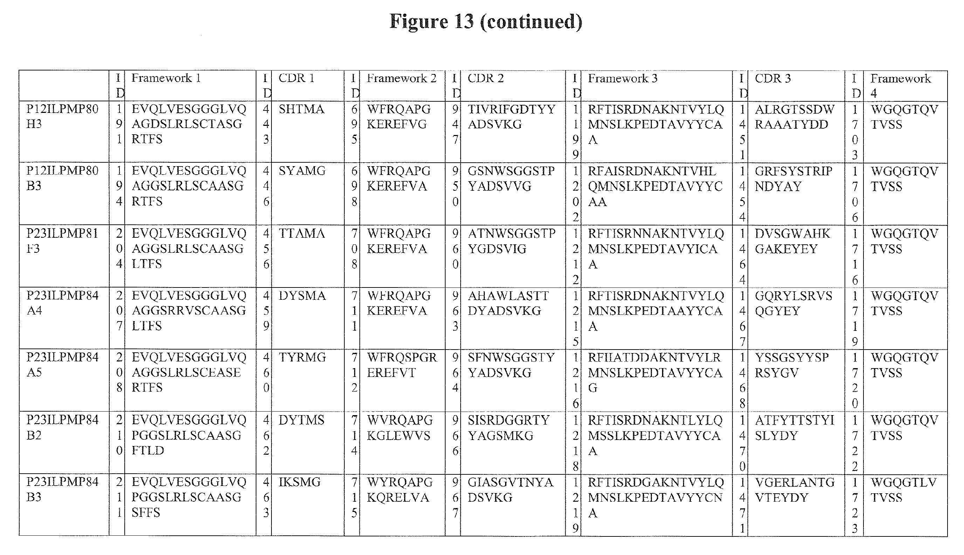

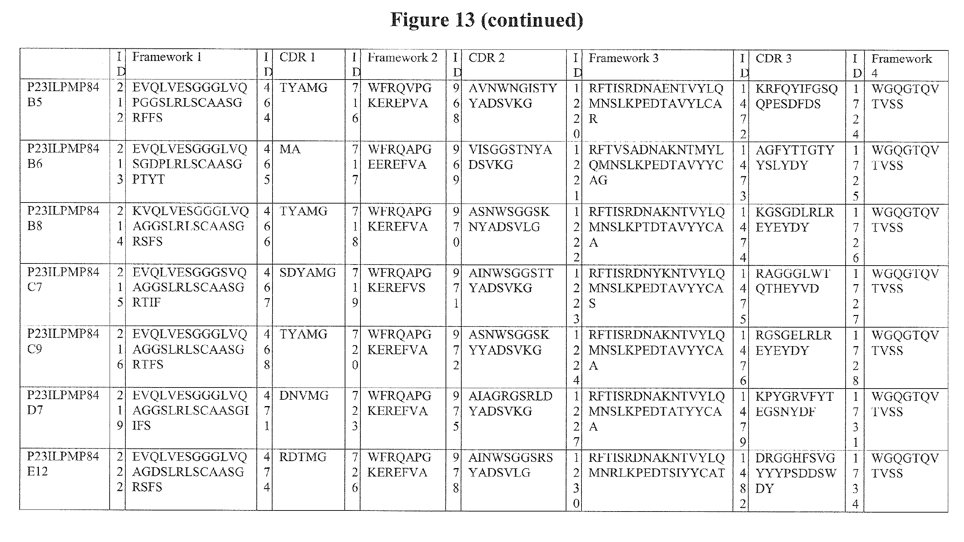

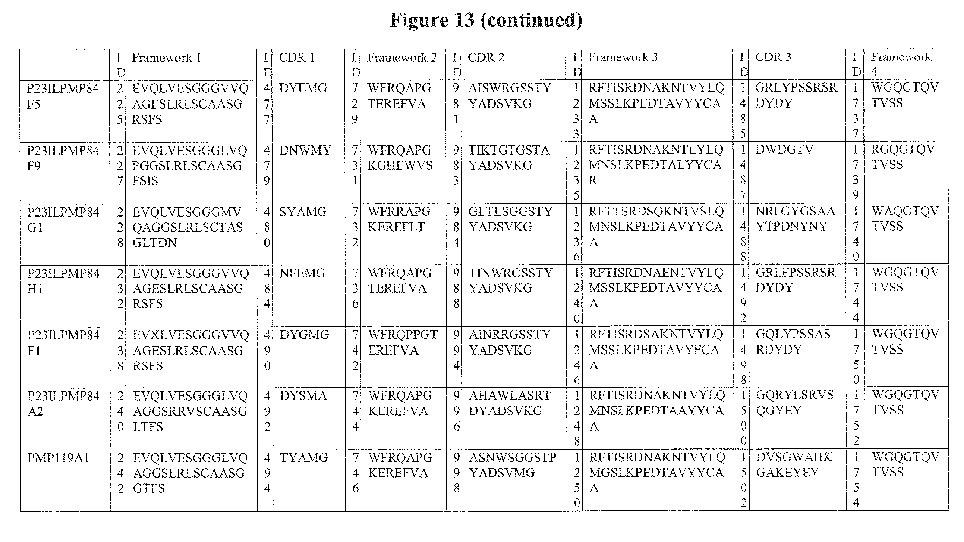

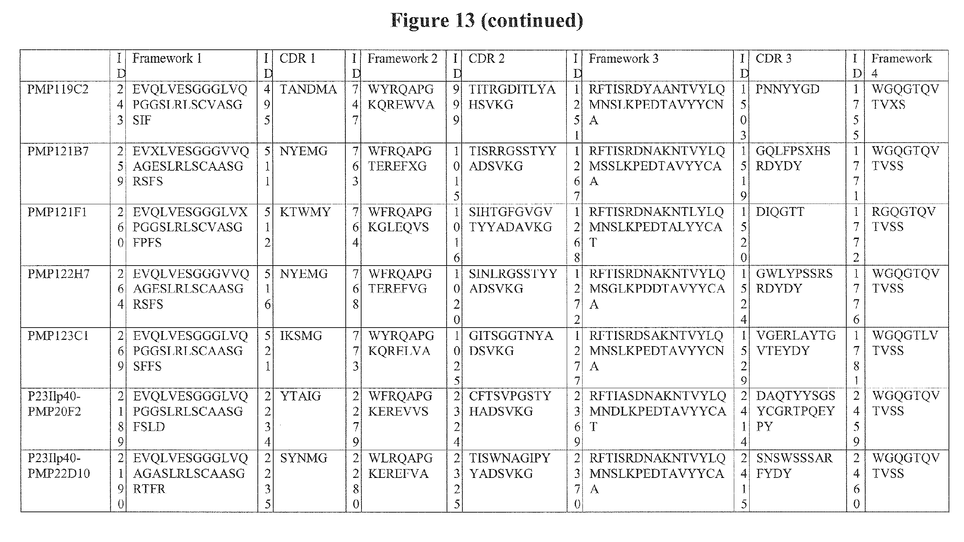

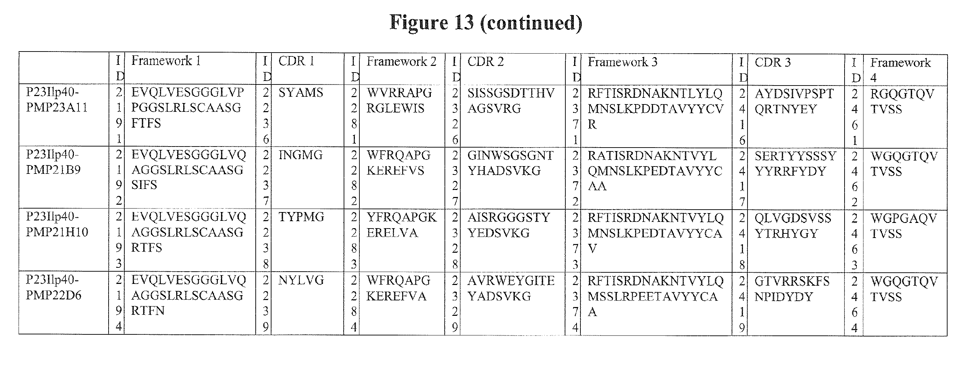

In one particular aspect, a p40- sequence may comprise one or more stretches of amino acid residues chosen from the group consisting of:
a) the amino acid sequences from the "CDR1 Sequences Group 16" (as defined and listed in Table A-1; see also Figure 13);
b) amino acid sequences that have at least 80% amino acid identity with at least one of the amino acid sequences from the "CDR1 Sequences Group 16";
c) amino acid sequences that have 3, 2, or 1 amino acid difference with at least one of the amino acid sequences from the "CDR1 Sequences Group 16";
d) the amino acid sequences from the "CDR2 Sequences Group 18" (as defined and listed in Table A-1; see also Figure 13);
e) amino acid sequences that have at least 80% amino acid identity with at least one of the amino acid sequences from the "CDR2 Sequences Group 18";
f) amino acid sequences that have 3, 2, or 1 amino acid difference with at least one of the amino acid sequences from the "CDR2 Sequences Group 18";
g) the amino acid sequences from the "CDR3 Sequences Group 20" (as defined and listed in Table A-1; see also Figure 13);
h) amino acid sequences that have at least 80% amino acid identity with at least one of the amino acid sequences from the "CDR3 Sequences Group 20";
i) amino acid sequences that have 3, 2, or 1 amino acid difference with at least one of the amino acid sequences from the "CDR3 Sequences Group 20";
or any suitable combination thereof.

Optionally, when an amino acid sequence of the invention contains one or more amino acid sequences according to b) and/or c), Optional Condition I, Optional Condition II and/or Optional Condition III (all as defined herein) may apply to said amino acid sequence (i.e. compared to the original amino acid sequence according to a)). Also, optionally, when an amino acid sequence of the invention contains one or more amino acid sequences according to e) and/or f), Optional Condition I, Optional Condition II and/or Optional Condition III (all as defined herein) may apply to said amino acid sequence (i.e. compared to the original amino acid sequence according to d)). Also, optionally, when an amino acid sequence of the invention contains one or more amino acid sequences according to h) and/or i), Optional Condition I, Optional Condition II and/or Optional Condition III (all as defined herein) may apply to said amino acid sequence (i.e. compared to the original amino acid sequence according to g)).

In this specific aspect, the amino acid sequence preferably comprises one or more stretches of amino acid residues chosen from the group consisting of:
a) the amino acid sequences from the "CDR1 Sequences Group 16";
b) the amino acid sequences from the "CDR2 Sequences Group 18"; and
c) the amino acid sequences from the "CDR3 Sequences Group 20";
or any suitable combination thereof.

Also, preferably, in such an amino acid sequence, at least one of said stretches of amino acid residues forms part of the antigen binding site for binding against p40.

In a more specific, but again non-limiting aspect, a p40- sequence may comprise two or more stretches of amino acid residues chosen from the group consisting of:
a) the amino acid sequences from the "CDR1 Sequences Group 16";
b) amino acid sequences that have at least 80% amino acid identity with at least one of the amino acid sequences from the "CDR1 Sequences Group 16";
c) amino acid sequences that have 3, 2, or 1 amino acid difference with at least one of the amino acid sequences from the "CDR1 Sequences Group 16";
d) the amino acid sequences from the "CDR2 Sequences Group 18";
e) amino acid sequences that have at least 80% amino acid identity with at least one of the amino acid sequences from the "CDR2 Sequences Group 18";
f) amino acid sequences that have 3, 2, or 1 amino acid difference with at least one of the amino acid sequences from the "CDR2 Sequences Group 18";
g) the amino acid sequences from the "CDR3 Sequences Group 20";
h) amino acid sequences that have at least 80% amino acid identity with at least one of the amino acid sequences from the "CDR3 Sequences Group 20";
i) amino acid sequences that have 3, 2, or 1 amino acid difference with at least one of the amino acid sequences from the "CDR3 Sequences Group 20";
such that (i) when the first stretch of amino acid residues corresponds to one of the amino acid sequences according to a), b) or c), the second stretch of amino acid residues corresponds to one of the amino acid sequences according to d), e), f), g), h) or i); (ii) when the first stretch of amino acid residues corresponds to one of the amino acid sequences according to d), e) or f), the second stretch of amino acid residues corresponds to one of the amino acid sequences according to a), b), c), g), h) or i); or (iii) when the first stretch of amino acid residues corresponds to one of the amino acid sequences according to g), h) or i), the second stretch of amino acid residues corresponds to one of the amino acid sequences according to a), b), c), d), e) or f).

In this specific aspect, the amino acid sequence preferably comprises two or more stretches of amino acid residues chosen from the group consisting of:
a) the amino acid sequences from the "CDR1 Sequences Group 16";
b) the amino acid sequences from the "CDR2 Sequences Group 18"; and
c) the amino acid sequences from the "CDR3 Sequences Group 20";
such that, (i) when the first stretch of amino acid residues corresponds to one of the amino acid sequences from the "CDR1 Sequences Group 16", the second stretch of amino acid residues corresponds to one of the amino acid sequences from the "CDR2 Sequences Group 18" or from the "CDR3 Sequences Group 20"; (ii) when the first stretch of amino acid residues corresponds to one of the amino acid sequences from the "CDR2 Sequences Group 18", the second stretch of amino acid residues corresponds to one of the amino acid sequences from the "CDR1 Sequences Group 16" or from the "CDR3 Sequences Group 20"; or (iii) when the first stretch of amino acid residues corresponds to one of the amino acid sequences from the "CDR3 Sequences Group 20", the second stretch of amino acid residues corresponds to one of the amino acid sequences from the "CDR1 Sequences Group 16" or from the "CDR2 Sequences Group 18",

Also, in such an amino acid sequence, the at least two stretches of amino acid residues again preferably form part of the antigen binding site for binding against p40.

In an even more specific, but non-limiting aspect, a p40- sequence may comprise three or more stretches of amino acid residues, in which the first stretch of amino acid residues is chosen from the group consisting of:
a) the amino acid sequences from the "CDR1 Sequences Group 16";
b) amino acid sequences that have at least 80% amino acid identity with at least one of the amino acid sequences from the "CDR1 Sequences Group 16";
c) amino acid sequences that have 3, 2, or 1 amino acid difference with at least one of the amino acid sequences from the "CDR1 Sequences Group 16";
   the second stretch of amino acid residues is chosen from the group consisting of:
d) the amino acid sequences from the "CDR2 Sequences Group 18";
e) amino acid sequences that have at least 80% amino acid identity with at least one of the amino acid sequences from the "GDR2 Sequences Group 18";
f) amino acid sequences that have 3, 2, or 1 amino acid difference with at least one of the amino acid sequences from the "CDR2 Sequences Group 18";
   and the third stretch of amino acid residues is chosen from the group consisting of:
g) the amino acid sequences from the "CDR3 Sequences Group 20";
h) amino acid sequences that have at least 80% amino acid identity with at least one of the amino acid sequences from the "CDR3 Sequences Group 20";
i) amino acid sequences that have 3, 2, or 1 amino acid difference with at least one of the amino acid sequences from the "CDR3 Sequences Group 20".

Preferably, in this specifc aspect, the first stretch of amino acid residues is chosen from the group consisting of the amino acid sequences from the "CDR1 Sequences Group 16"; the second stretch of amino acid residues is chosen from the group consisting of the amino acid sequences from the "CDR2 Sequences Group 18"; and the third stretch of amino acid residues is chosen from the group consisting of the amino acid sequences from the "CDR3 Sequences Group 20".

Again, preferably, in such an amino acid sequence, the at least three stretches of amino acid residues forms part of the antigen binding site for binding against p40.

Preferred combinations of such stretches of amino acid sequences will become clear from the further disclosure herein.

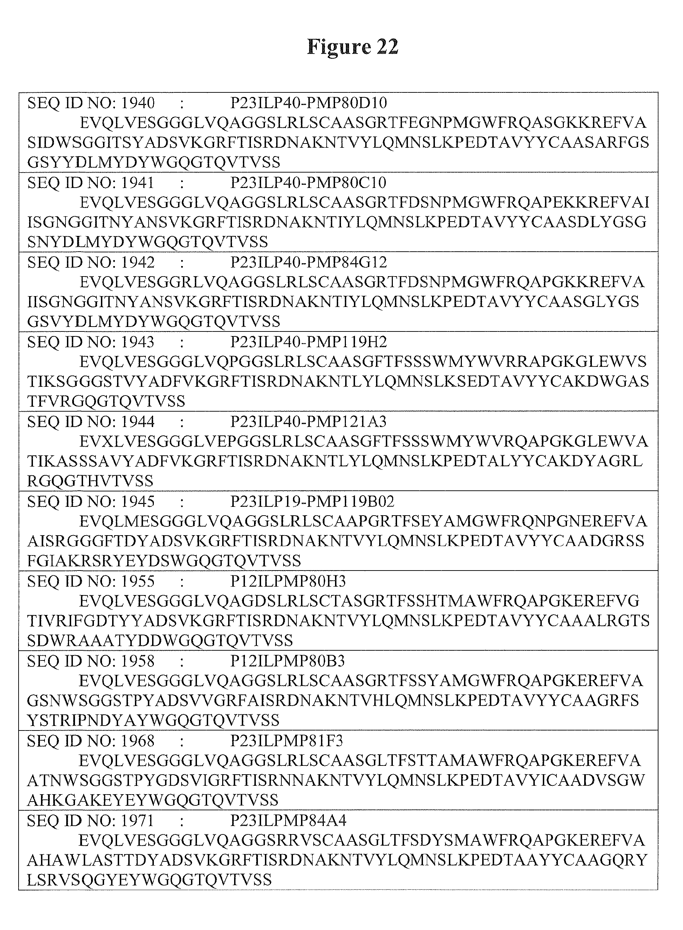

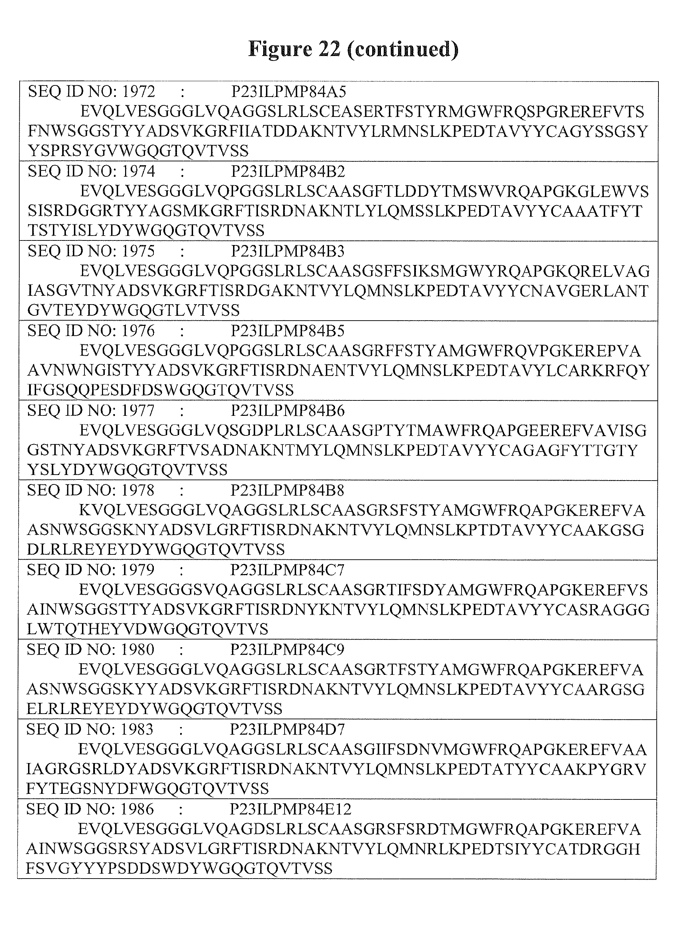

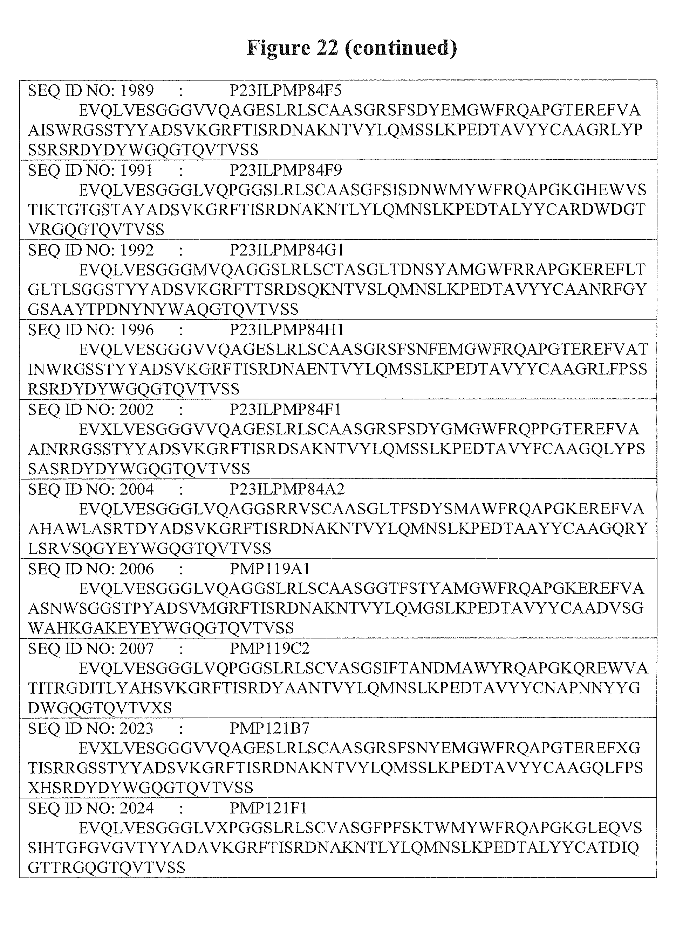

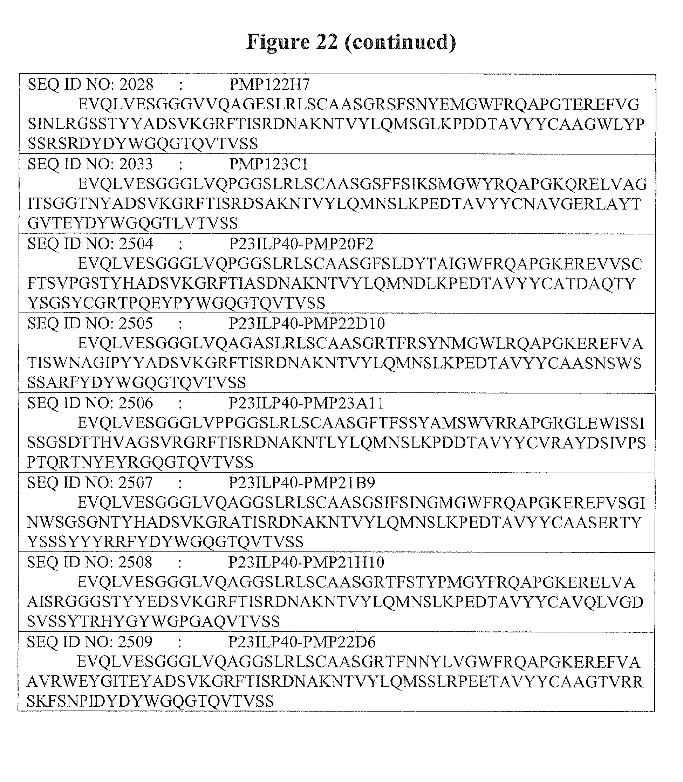

Preferably, in such amino acid sequences the CDR sequences have at least 70% amino acid identity, preferably at least 80% amino acid identity, more preferably at least 90% amino acid identity, such as 95% amino acid identity or more or even essentially 100% amino acid identity with the CDR sequences of at least one of the p40- sequences listed in Table A-2 and Figure 22. This degree of amino acid identity can for example be determined by determining the degree of amino acid identity (in a manner described herein) between said amino acid sequence and one or more of the sequences of SEQ ID NO's: 1940; 1941; 1942; 1943; 1944; 1945; 1955; 1958; 1968; 1971; 1972; 1974; 1975; 1976; 1977; 1978; 1979; 1980; 1983; 1986; 1989; 1991; 1992; 1996; 2002; 2004; 2006; 2007; 2023; 2024; 2028; 2033; 2504; 2505; 2506; 2507; 2508 and/or 2509 (see Table A-2 and Figure 22), in which the amino acid residues that form the framework regions are disregarded. Also, such amino acid sequences of the invention can be as further described herein.

Also, such amino acid sequences are preferably such that they can specifically bind (as defined herein) to the p40 subunit; and more in particular bind to the p40 subunit with an affinity (suitably measured and/or expressed as a K_{D}-value (actual or apparent), a K_{A}-value (actual or apparent), a kₒₙ-rate and/or a k_{off}-rate, or alternatively as an IC₅₀ value (all as further) described herein) that is as defined herein.

When the amino acid sequence of the invention essentially consists of 4 framework regions (FR1 to FR4, respectively) and 3 complementarity determining regions (CDR1 to CDR3, respectively), the amino acid sequence of the invention is preferably such that:
- CDR1 is chosen from the group consisting of:
   a) the amino acid sequences from the "CDR1 Sequences Group 16";
   b) amino acid sequences that have at least 80% amino acid identity with at least one of the amino acid sequences from the "CDR1 Sequences Group 16";
   c) amino acid sequences that have 3, 2, or 1 amino acid difference with at least one of the amino acid sequences from the "CDR1 Sequences Group 16";
   and/or
- CDR2 is chosen from the group consisting of:
   d) the amino acid sequences from the "CDR2 Sequences Group 18";
   e) amino acid sequences that have at least 80% amino acid identity with at least one of the amino acid sequences from the "CDR2 Sequences Group 18";
   f) amino acid sequences that have 3, 2, or 1 amino acid difference with at least one of the amino acid sequences from the "CDR2 Sequences Group 18";
   and/or
- CDR3 is chosen from the group consisting of:
   g) the amino acid sequences from the "CDR3 Sequences Group 20";
   h) amino acid sequences that have at least 80% amino acid identity with at least one of the amino acid sequences from the "CDR3 Sequences Group 20";
   i) amino acid sequences that have 3, 2, or 1 amino acid difference with at least one of the amino acid sequences from the "CDR3 Sequences Group 20".

In particular, such an amino acid sequence of the invention may be such that CDR1 is chosen from the group consisting of the amino acid sequences from the "CDR1 Sequences Group 1 G"; andl'or CDR2 is chosen from the group consisting of the amino acid sequences from the "CDR2 Sequences Group 18"; and/or CDR3 is chosen from the group consisting of the amino acid sequences from the "CDR3 Sequences Group 20".

In particular, when the amino acid sequence of the invention essentially consists of 4 framework regions (FR1 to FR4, respectively) and 3 complementarity determining regions (CDR1 to CDR3, respectively), the amino acid sequence of the invention is preferably such that:
- CDR1 is chosen from the group consisting of:
   a) the amino acid sequences from the "CDR1 Sequences Group 16";
   b) amino acid sequences that have at least 80% amino acid identity with at least one of the amino acid sequences from the "CDR1 Sequences Group 16";
   c) amino acid sequences that have 3, 2, or 1 amino acid difference with at least one of the amino acid sequences from the "CDR1 Sequences Group 16";
   and
- CDR2 is chosen from the group consisting of:
   d) the amino acid sequences from the "CDR2 Sequences Group 18";
   e) amino acid sequences that have at least 80% amino acid identity with at least one of the amino acid sequences from the "CDR2 Sequences Group 18";
   f) amino acid sequences that have 3, 2, or 1 amino acid difference with at least one of the amino acid sequences from the "CDR2 Sequences Group 18";
   and
- CDR3 is chosen from the group consisting of:
   g) the amino acid sequences from the "CDR3 Sequences Group 20";
   h) amino acid sequences that have at least 80% amino acid identity with at least one of the amino acid sequences from the "CDR3 Sequences Group 20";
   i) amino acid sequences that have 3, 2, or 1 amino acid difference with at least one of the amino acid sequences from the "CDR3 Sequences Group 20"; or any suitable fragment of such an amino acid sequence

In particular, such an amino acid sequence of the invention may be such that CDR1 is chosen from the group consisting of the amino acid sequences from the "CDR1 Sequences Group 16"; and CDR2 is chosen from the group consisting of the amino acid sequences from the "CDR2 Sequences Group 18"; and CDR3 is chosen from the group consisting of the amino acid sequences from the "CDR3 Sequences Group 20".

Again, preferred combinations of CDR sequences will become clear from the further description herein.

Also, such amino acid sequences are preferably such that they can specifically bind (as defined herein) to the p40 subunit; and more in particular bind to the p40 subunit with an affinity (suitably measured and/or expressed as a K_{D}-value (actual or apparent), a K_{A}-value (actual or apparent), a kₒₙ-rate and/or a k_{off}-rate, or alternatively as an IC₅₀ value (all as further) described herein) that is as defined herein.

In one preferred, but non-limiting aspect, the invention relates to an amino acid sequence that essentially consists of 4 framework regions (FR1 to FR4, respectively) and 3 complementarity determining regions (CDR1 to CDR3, respectively), in which the CDR sequences of said amino acid sequence have at least 70% amino acid identity, preferably at least 80% amino acid identity, more preferably at least 90% amino acid identity, such as 95% amino acid identity or more or even essentially 100% amino acid identity with the CDR sequences of at least one of the amino acid sequences of SEQ ID NO's: 1940; 1941; 1942; 1943; 1944; 1945; 1955; 1958; 1968; 1971; 1972; 1974; 1975; 1976; 1977; 1978, 1979; 1980_{;} 1983; 1986; 1989; 1991; 1992; 1996;2002;2004;2006;2007;2023;2024;2028; 2033; 2504; 2505; 2506; 2507; 2508 and/or 2509 (see Table A-2 and Figure 22). This degree of amino acid identity can for example be determined by determining the degree of amino acid identity (in a manner described herein) between said amino acid sequence and one or more of the sequences of SEQ ID NO's: 1940; 1941; 1942; 1943; 1944; 1945; 1955; 1958; 1968; 1971; 1972; 1974; 1975; 1976; 1977; 1978; 1979; 1980_{;} 1983; 1986; 1989; 1991; 1992; 1996;2002;2004;2006;2007;2023;2024;2028;2033;2504;2505;2506;2507;2508 and/or 2509 (see Table A-2 and Figure 22), in which the amino acid residues that form the framework regions are disregarded. Such amino acid sequences of the invention can be as further described herein.

Some preferred, but non-limiting examples of p40- sequence are the amino acid sequences of SEQ ID NO's: 1940; 1941; 1942; 1943; 1944; 1945; 1955; 1958; 1968; 1971; 1972; 1974; 1975; 1976; 1977; 1978; 1979; 1980; 1983; 1986; 1989; 1991; 1992; 1996; 2002;2004;2006;2007;2023;2024;2028;2033;2504; 2505; 2506;2507;2508 and/or 2509 (see Table A-2 and Figure 22). Thus, according to another preferred, but non-limiting aspect of the invention, a p40- sequence is an amino acid sequence that is directed against (as defined herein) the p40 subunit (as present in for example IL-23 and IL-12) but that (essentially) are not capable of neutralizing or inhibiting the binding of a heterodimeric cytokine comprising a p40 subunit to its receptor (for example, in a suitable alpha-screen assay as exemplified in Examples 19 and 22 for IL-23 and its cognate receptor and for IL-12 and its cognate receptor), and that either:
a) has at least 70% amino acid identity, preferably at least 80% amino acid identity, more preferably at least 90% amino acid identity, such as 95% amino acid identity or more or even essentially 100% amino acid identity with at least one of the amino acid sequences of SEQ ID NO's: 1940; 1941; 1942; 1943; 1944; 1945; 1955; 1958; 1968; 1971; 1972; 1974; 1975; 1976; 1977; 1978; 1979; 1980; 1983; 1986; 1989; 1991; 1992; 1996; 2002; 2004;2006;2007;2023;2024;2028;2033;2504;2505;2506;2507;2508 and/or 2509 (see Table A-2 and Figure 22);
   and/or that
b) has no more than 20, preferably no more than 10, such as 9, 8, 7, 6, 5, 4, 3, 2 or only one amino acid difference with at least one of the amino acid sequences of SEQ ID NO's: 1940; 1941; 1942; 1943; 1944; 1945; 1955; 1958; 1968; 1971; 1972; 1974; 1975; 1976; 1977; 1978; 1979; 1980; 1983; 1986; 1989; 1991; 1992; 1996; 2002; 2004; 2006; 2007; 2023; 2024; 2028; 2033; 2504; 2505; 2506; 2507; 2508 and/or 2509 (see Table A-2 and Figure 22). Preferably, such an amino acid sequence has no more than a total of 5 (such as 4, 3. 2 or only one) such amino acid differences in the CDR's and/or no more than a total of 5 (such as 4, 3. 2 or only 1) such amino acid differences in the framework sequences;
   and/or that
c) is either (i) capable of cross-blocking (as defined herein) the interaction of at least one of the amino acid sequences of SEQ ID NO's: 1940; 1941; 1942; 1943; 1944; 1945; 1955; 1958; 1968; 1971; 1972; 1974; 1975; 1976; 1977; 1978; 1979; 1980; 1983; 1986; 1989; 1991; 1992; 1996; 2002; 2004; 2006; 2007; 2023; 2024; 2028; 2033; 2504; 2505; 2506; 2507; 2508 and/or 2509 (see Table A-2 and Figure 20) with the p40 subunit and/or (ii) being able to compete with (i.e. is a competitor for) the binding of at least one of the amino acid sequences of SEQ ID NO's: 1940; 1941; 1942; 1943; 1944; 1945; 1955; 1958; 1968; 1971; 1972; 1974; 1975; 1976; 1977; 1978; 1979; 1980; 1983; 1986; 1989; 1991; 1992; 1996;2002;2004;2006;2007;2023;2024;2028;2033;2504; 2505; 2506; 2507; 2508 and/or 2509 (see Table A-2 and Figure 22) to the p40 subunit.

In another preferred, but non-limiting aspect, a p40 sequence is chosen from one of the amino acid sequences of SEQ ID NO's: 1940; 1941; 1942; 1943; 1944; 1945; 1955; 1958; 1968; 1971; 1972; 1974; 1975; 1976; 1977; 1978; 1979; 1980; 1983; 1986; 1989; 1991; 1992; 1996;2002;2004;2006;2007;2023;2024;2028;2033;2504;2505;2506; 2507; 2508 and/or 2509 (see Table A-2 and Figure 22).

### D) P40+ sequences.

One specific, but non-limiting aspect relates to "p40+ sequences", which generally are defined herein as amino acid sequences of the invention that are directed against (as defined herein) the p40 subunit (as present in for example IL-23 and IL-12), and that are capable of modulating, neutralizing, blocking and/or inhibiting the binding of a heterodimeric cytokine, comprising a p40 subunit to its receptor, and in particular capable of modulating, neutralizing, blocking and/or inhibiting the binding of IL-L3 to its (cognate) receptor (in particular, in the alpha-screen assay of described in Example 19 or 22); and/or the binding of IL-12 to its (cognate) receptor (in particular, in the alpha-screen assay of described in Example 19).

P40+ sequences may generally be as further described herein (for example, in terms of affinity, specificity etc. for p40) for amino acid sequences of the invention in general. Also, as described herein for the amino acid sequences of the invention, the p40+ sequences are preferably such that they form or are capable of forming (optionally after suitable folding) a single antigen binding domain or antigen binding unit, and may for example be amino acid sequences that comprise an immunoglobulin fold, amino acid sequences that are comprised of four framework regions and three CDR's, and may in particular be domain antibodies, single domain antibodies, VHH's, "dAb's" or Nanobodies (all as further described herein), or suitable fragments thereof.

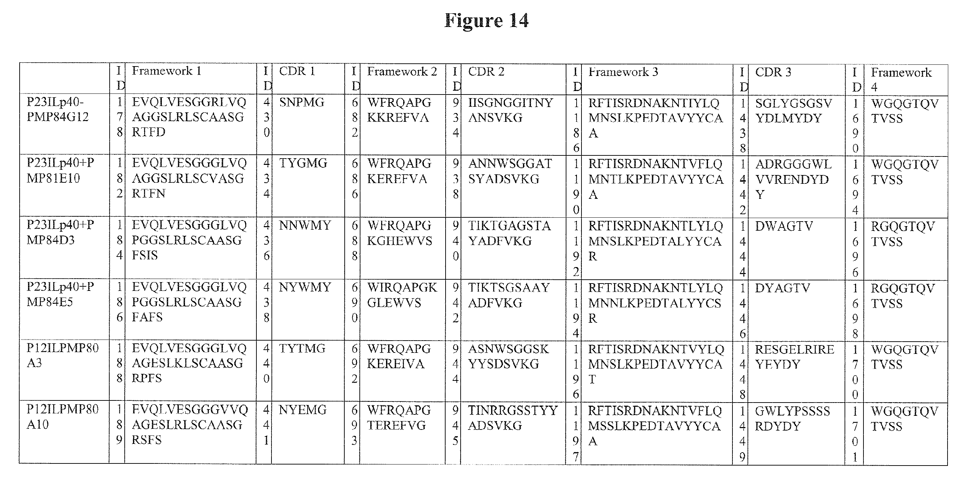

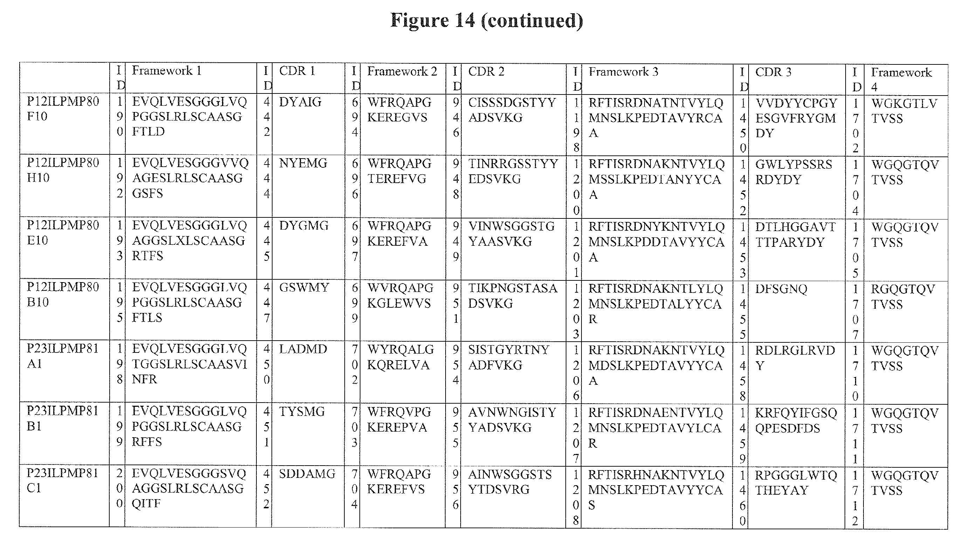

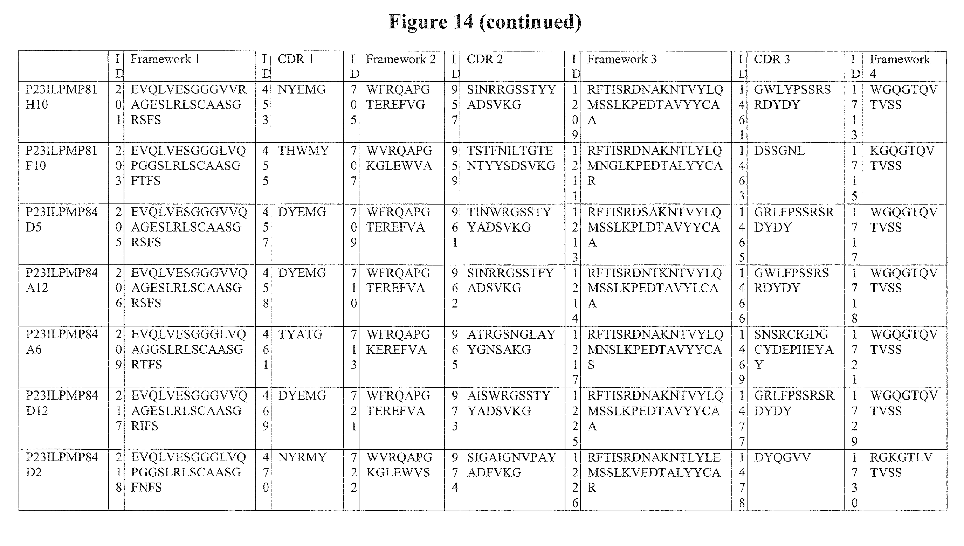

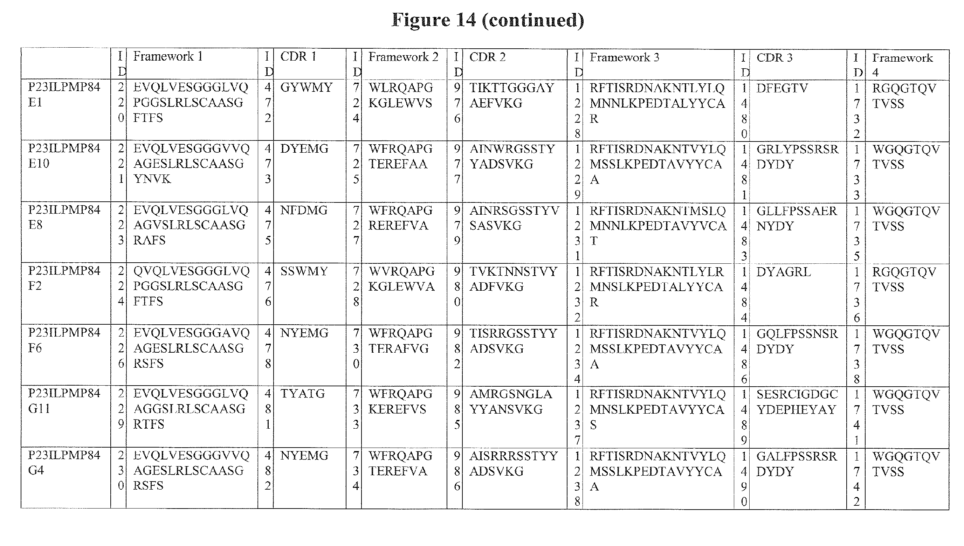

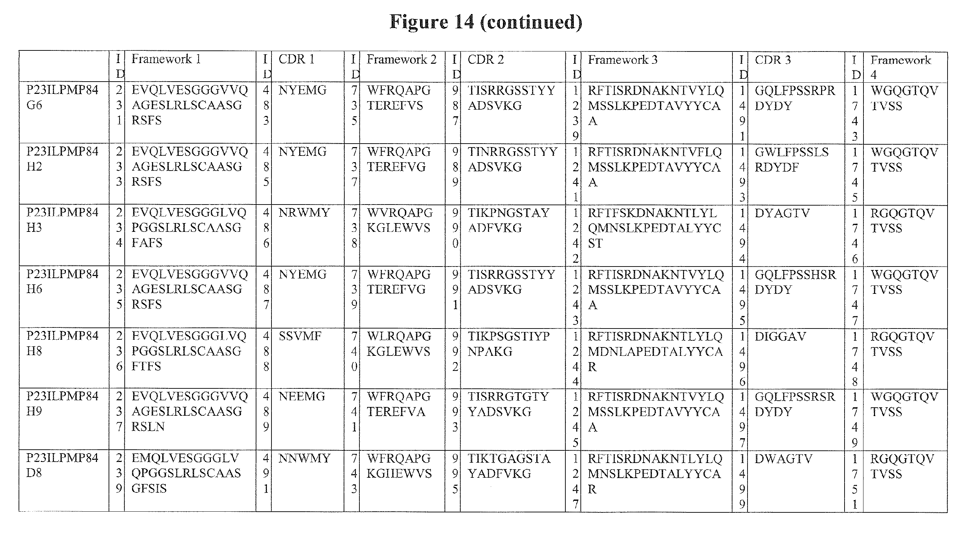

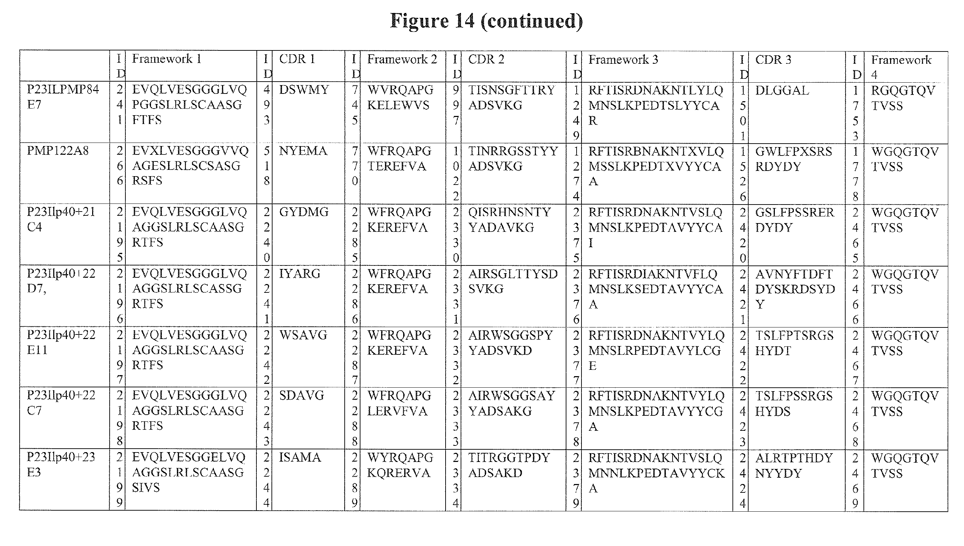

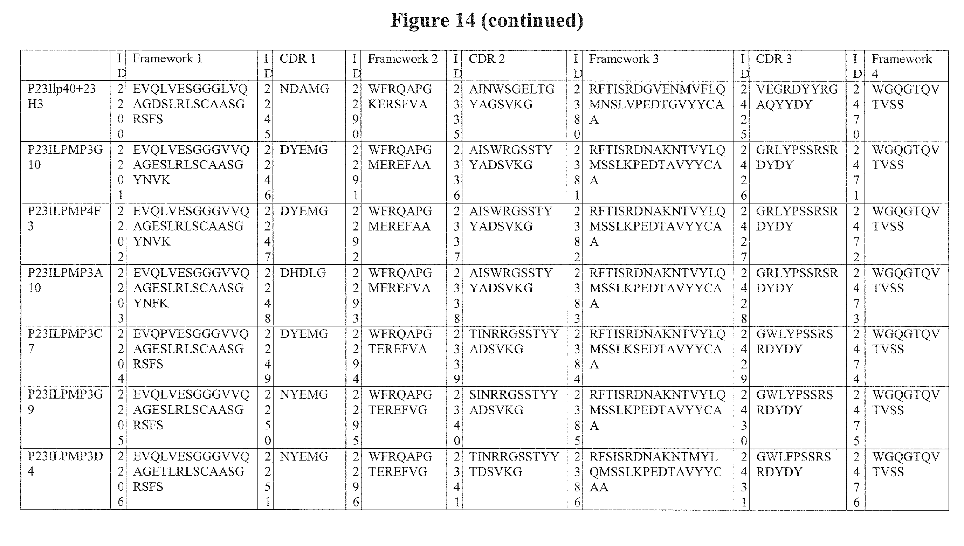

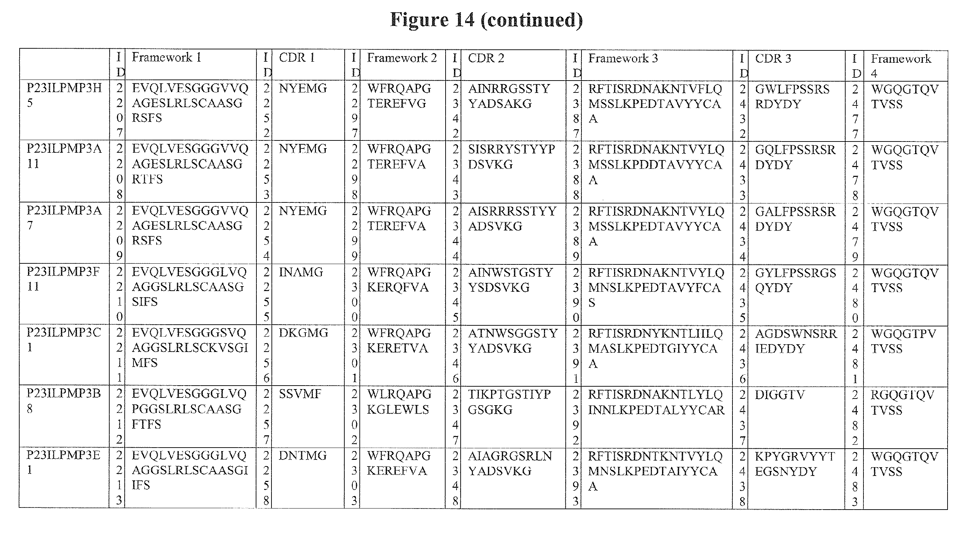

In one particular aspect, a p40+ sequence may comprise one or more stretches of amino acid residues chosen from the group consisting of:
a) the amino acid sequences from the "CDR1 Sequences Group 23" (as defined and listed in Table A-1; see also Figure 14);
b) amino acid sequences that have at least 80% amino acid identity with at least one of the amino acid sequences from the "CDR1 Sequences Group 23";
c) amino acid sequences that have 3, 2, or 1 amino acid difference with at least one of the amino acid sequences from the "CDR1 Sequences Group 23";
d) the amino acid sequences from the "CDR2 Sequences Group 25" (as defined and listed in Table A-1; see also Figure 14);
e) amino acid sequences that have at least 80% amino acid identity with at least one of the amino acid sequences from the "CDR2 Sequences Group 25";
f) amino acid sequences that have 3, 2, or 1 amino acid difference with at least one of the amino acid sequences from the "CDR2 Sequences Group 25";
g) the amino acid sequences from the "CDR3 Sequences Group 27" (as defined and listed in Table A-1; see also Figure 14);
h) amino acid sequences that have at least 80% amino acid identity with at least one of the amino acid sequences from the "CDR3 Sequences Group 27";
i) amino acid sequences that have 3, 2, or 1 amino acid difference with at least one of the amino acid sequences from the "CDR3 Sequences Group 27";
or any suitable combination thereof.

Optionally, when an amino acid sequence of the invention contains one or more amino acid sequences according to b) and/or c), Optional Condition I, Optional Condition II and/or Optional Condition III (all as defined herein) may apply to said amino acid sequence (i.e. compared to the original amino acid sequence according to a)). Also, optionally, when an amino acid sequence of the invention contains one or more amino acid sequences according to e) and/or f), Optional Condition I, Optional Condition II and/or Optional Condition III (all as defined herein) may apply to said amino acid sequence (i.e. compared to the original amino acid sequence according to d)). Also, optionally, when an amino acid sequence of the invention contains one or more amino acid sequences according to h) and/or i), Optional Condition I, Optional Condition II and/or Optional Condition III (all as defined herein) may apply to said amino acid sequence (i.e. compared to the original amino acid sequence according to g)).

In this specific aspect, the amino acid sequence preferably comprises one or more stretches of amino acid residues chosen from the group consisting of:
a) the amino acid sequences from the "CDR1 Sequences Group 23";
b) the amino acid sequences from the "CDR2 Sequences Group 25"; and
c) the amino acid sequences from the "CDR3 Sequences Group 27";
or any suitable combination thereof.

Also, preferably, in such an amino acid sequence, at least one of said stretches of amino acid residues forms part of the antigen binding site for binding against p40.

In a more specific, but again non-limiting aspect, a p40+ sequence may comprise two or more stretches of amino acid residues chosen from the group consisting of:
a) the amino acid sequences from the "CDR1 Sequences Group 23";
b) amino acid sequences that have at least 80% amino acid identity with at least one of the amino acid sequences from the "CDR1 Sequences Group 23";
c) amino acid sequences that have 3, 2, or 1 amino acid difference with at least one of the amino acid sequences from the "CDR1 Sequences Group 23";
d) the amino acid sequences from the "CDR2 Sequences Group 25";
e) amino acid sequences that have at least 80% amino acid identity with at least one of the amino acid sequences from the "CDR2 Sequences Group 25";
f) amino acid sequences that have 3, 2, or 1 amino acid difference with at least one of the amino acid sequences from the "CDR2 Sequences Group 25";
g) the amino acid sequences from the "CDR3 Sequences Group 27";
h) amino acid sequences that have at least 80% amino acid identity with at least one of the amino acid sequences from the "CDR3 Sequences Group 27";
i) amino acid sequences that have 3, 2, or 1 amino acid difference with at least one of the amino acid sequences from the "CDR3 Sequences Group 27";
such that (i) when the first stretch of amino acid residues corresponds to one of the amino acid sequences according to a), b) or c), the second stretch of amino acid residues corresponds to one of the amino acid sequences according to d), e), f), g), h) or i); (ii) when the first stretch of amino acid residues corresponds to one of the amino acid sequences according to d), e) or f), the second stretch of amino acid residues corresponds to one of the amino acid sequences according to a), b), c), g), h) or i); or (iii) when the first stretch of amino acid residues corresponds to one of the amino acid sequences according to g), h) or i), the second stretch of amino acid residues corresponds to one of the amino acid sequences according to a), b), c), d), e) or f).

In this specific aspect, the amino acid sequence preferably comprises two or more stretches of amino acid residues chosen from the group consisting of:
a) the amino acid sequences from the "CDR1 Sequences Group 23";
b) the amino acid sequences from the "CDR2 Sequences Group 25"; and
c) the amino acid sequences from the "CDR3 Sequences Group 27";
such that, (i) when the first stretch of amino acid residues corresponds to one of the amino acid sequences from the "CDR1 Sequences Group 23", the second stretch of amino acid residues corresponds to one of the amino acid sequences from the "CDR2 Sequences Group 25" or from the "CDR3 Sequences Group 27"; (ii) when the first stretch of amino acid residues corresponds to one of the amino acid sequences from the "CDR2 Sequences Group 25", the second stretch of amino acid residues corresponds to one of the amino acid sequences from the "CDR1 Sequences Group 23" or from the "CDR3 Sequences Group 27"; or (iii) when the first stretch of amino acid residues corresponds to one of the amino acid sequences from the "CDR3 Sequences Group 27", the second stretch of amino acid residues corresponds to one of the amino acid sequences from the "CDR1 Sequences Group 23" or from the "CDR2 Sequences Group 25".

Also, in such an amino acid sequence, the at least two stretches of amino acid residues again preferably form part of the antigen binding site for binding against p40.

In an even more specific, but non-limiting aspect, a p40+ sequence may comprise three or more stretches of amino acid residues, in which the first stretch of amino acid residues is chosen from the group consisting of:
a) the amino acid sequences from the "CDR1 Sequences Group 23";
b) amino acid sequences that have at least 80% amino acid identity with at least one of the amino acid sequences from the "CDR1 Sequences Group 23";
c) amino acid sequences that have 3, 2, or 1 amino acid difference with at least one of the amino acid sequences from the "CDR1 Sequences Group 23";
   the second stretch of amino acid residues is chosen from the group consisting of:
d) the amino acid sequences from the "CDR2 Sequences Group 25";
e) amino acid sequences that have at least 80% amino acid identity with at least one of the amino acid sequences from the "CDR2 Sequences Group 25";
f) amino acid sequences that have 3, 2, or 1 amino acid difference with at least one of the amino acid sequences from the "CDR2 Sequences Group 25";
   and the third stretch of amino acid residues is chosen from the group consisting of:
g) the amino acid sequences from the "CDR3 Sequences Group 27";
h) amino acid sequences that have at least 80% amino acid identity with at least one of the amino acid sequences from the "CDR3 Sequences Group 27";
i) amino acid sequences that have 3, 2, or 1 amino acid difference with at least one of the amino acid sequences from the "CDR3 Sequences Group 27".

Preferably, in this specifc aspect, the first stretch of amino acid residues is chosen from the group consisting of the amino acid sequences from the "CDR1 Sequences Group 23"; the second stretch of amino acid residues is chosen from the group consisting of the amino acid sequences from the "CDR2 Sequences Group 25"; and the third stretch of amino acid residues is chosen from the group consisting of the amino acid sequences from the "CDR3 Sequences Group 27".

Again, preferably, in such an amino acid sequence, the at least three stretches of amino acid residues forms part of the antigen binding site for binding against p40.

Preferred combinations of such stretches of amino acid sequences will become clear from the further disclosure herein.

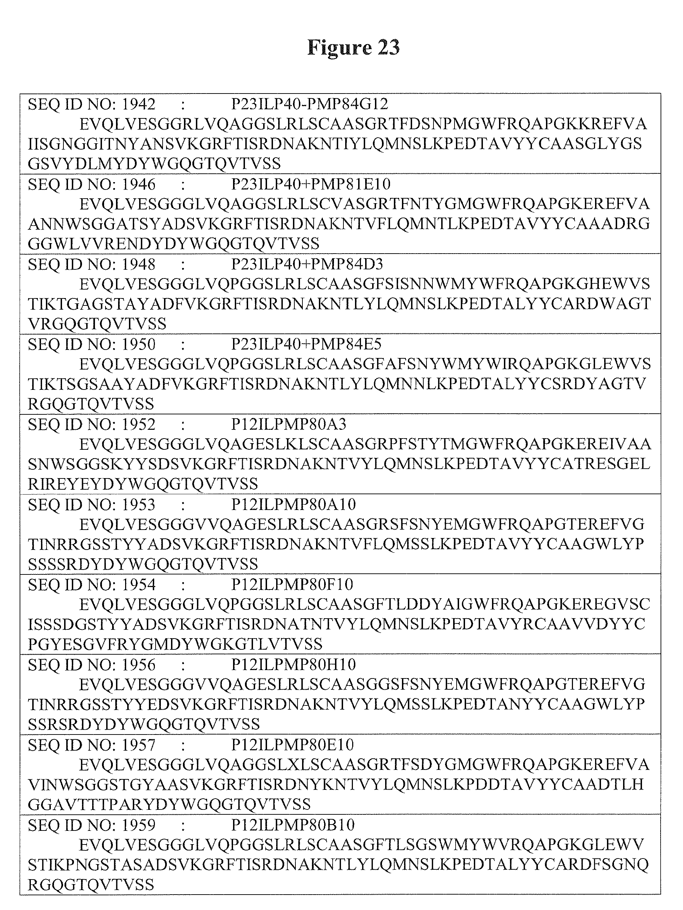

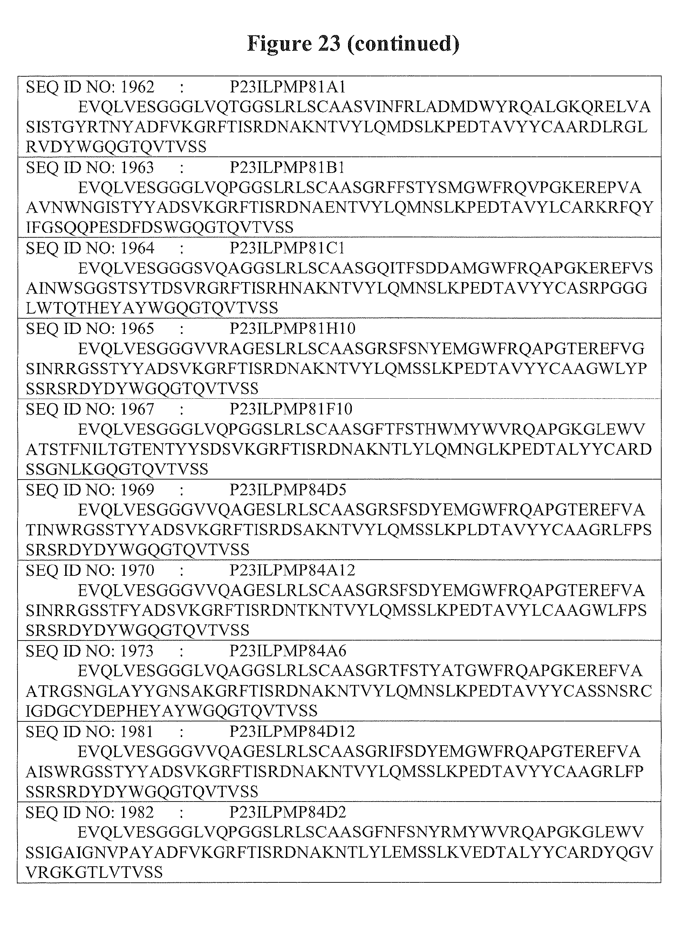

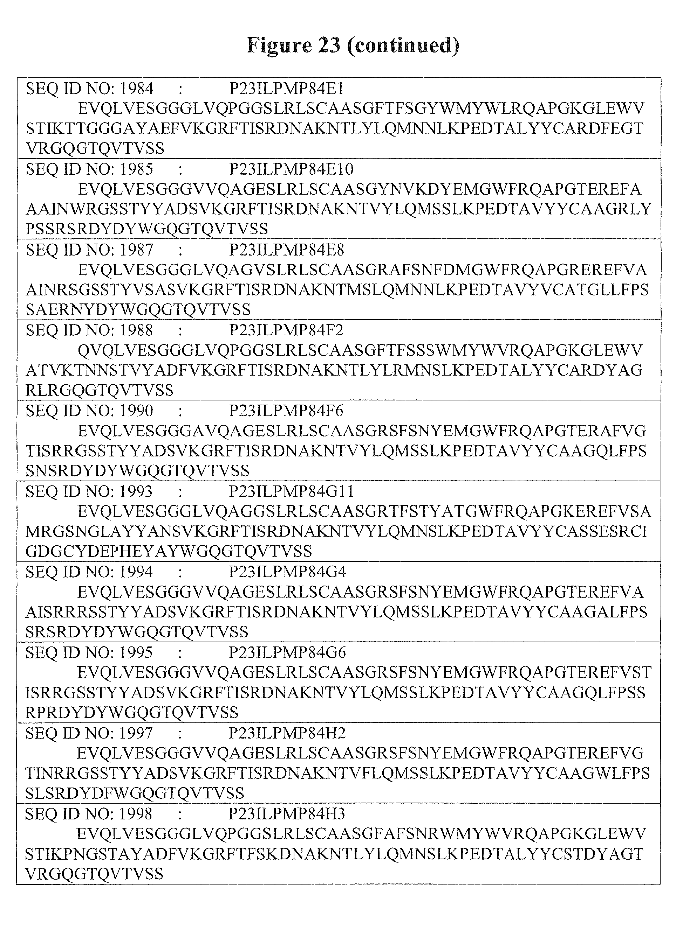

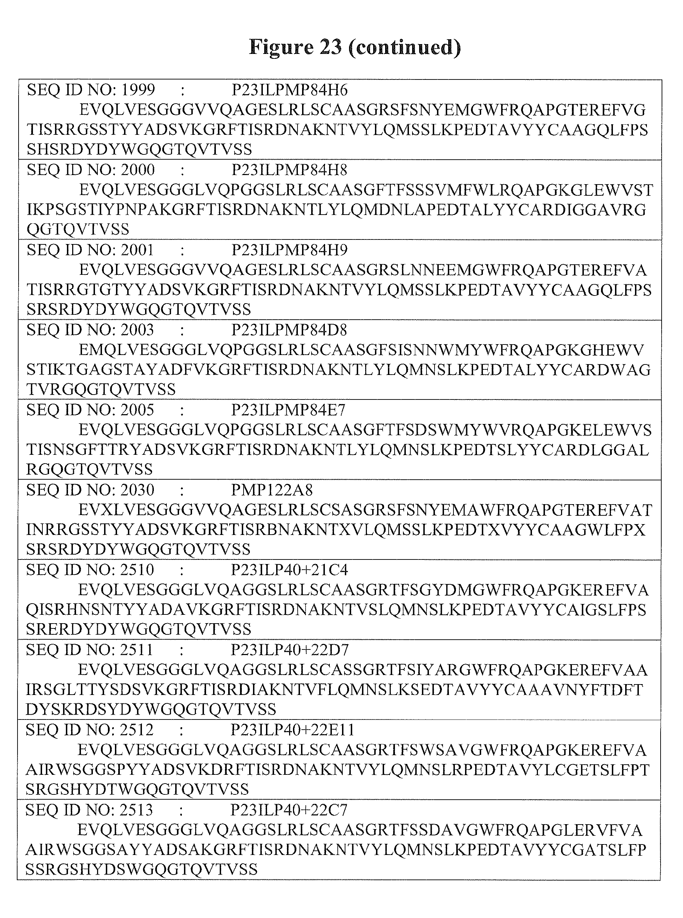

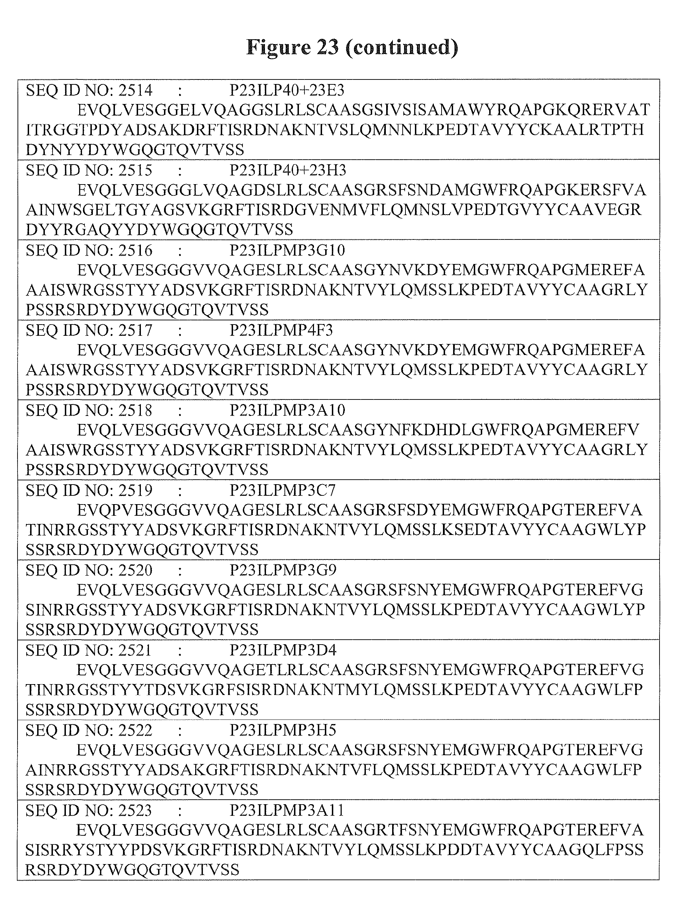

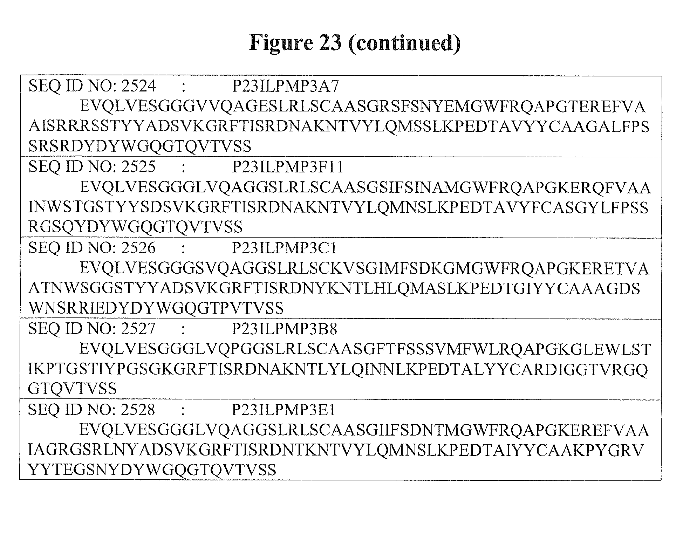

Preferably, in such amino acid sequences the CDR sequences have at least 70% amino acid identity, preferably at least 80% amino acid identity, more preferably at least 90% amino acid identity, such as 95% amino acid identity or more or even essentially 100% amino acid identity with the CDR sequences of at least one of the p40+ sequences listed in Table A-2 and Figure 23. This degree of amino acid identity can for example be determined by determining the degree of amino acid identity (in a manner described herein) between said amino acid sequence and one or more of the sequences of SEQ ID NO's: 1942; 1946; 1948; 1950; 1952; 1953; 1954; 1956; 1957; 1959; 1962; 1963; 1964; 1965; 1967; 1969; 1970; 1973; 1981; 1982; 1984; 1985; 1987; 1988; 1990; 1993; 1994; 1995; 1997; 1998; 1999; 2000;2001;2003;2005;2030;2510;2511;2512;2513;2514;2515;2516;2517;2518; 2519; 2520; 2521; 2522; 2523; 2524; 2525; 2526; 2527 and/or 2528 (see Table A-2 and Figure 23), in which the amino acid residues that form the framework regions are disregarded. Also, such amino acid sequences of the invention can be as further described herein.

Also, such amino acid sequences are preferably such that they can specifically bind (as defined herein) to the p40 subunit; and more in particular bind to the p40 subunit with an affinity (suitably measured and/or expressed as a K_{D}-value (actual or apparent), a K_{A}-value (actual or apparent), a kₒₙ-rate and/or a k_{off}-rate, or alternatively as an IC₅₀ value (all as further) described herein) that is as defined herein.

When the amino acid sequence of the invention essentially consists of 4 framework regions (FR1 to FR4, respectively) and 3 complementarity determining regions (CDR1 to CDR3, respectively), the amino acid sequence of the invention is preferably such that:
- CDR1 is chosen from the group consisting of:
   a) the amino acid sequences from the "CDR1 Sequences Group 23";
   b) amino acid sequences that have at least 80% amino acid identity with at least one of the amino acid sequences from the "CDR1 Sequences Group 23";
   c) amino acid sequences that have 3, 2, or 1 amino acid difference with at least one of the amino acid sequences from the "CDR1 Sequences Group 23";
   and/or
- CDR2 is chosen from the group consisting of:
   d) the amino acid sequences from the "CDR2 Sequences Group 25";
   e) amino acid sequences that have at least 80% amino acid identity with at least one of the amino acid sequences from the "CDR2 Sequences Group 25";
   f) amino acid sequences that have 3, 2, or 1 amino acid difference with at least one of the amino acid sequences from the "CDR2 Sequences Group 25";
   and/or
- CDR3 is chosen from the group consisting of:
   g) the amino acid sequences from the "CDR3 Sequences Group 27";
   h) amino acid sequences that have at least 80% amino acid identity with at least one of the amino acid sequences from the "CDR3 Sequences Group 27";
   i) amino acid sequences that have 3, 2, or 1 amino acid difference with at least one of the amino acid sequences from the "CDR3 Sequences Group 27".

In particular, such an amino acid sequence of the invention may be such that CDR1 is chosen from the group consisting of the amino acid sequences from the "CDR1 Sequences Group 23"; and/or CDR2 is chosen from the group consisting of the amino acid sequences from the "CDR2 Sequences Group 25"; and/or CDR3 is chosen from the group consisting of the amino acid sequences from the "CDR3 Sequences Group 27".

In particular, when the amino acid sequence of the invention essentially consists of 4 framework regions (FR1 to FR4, respectively) and 3 complementarity determining regions (CDR1 to CDR3, respectively), the amino acid sequence of the invention is preferably such that:
- CDR1 is chosen from the group consisting of:
   a) the amino acid sequences from the "CDR1 Sequences Group 23";
   b) amino acid sequences that have at least 80% amino acid identity with at least one of the amino acid sequences from the "CDR1 Sequences Group 23";
   c) amino acid sequences that have 3, 2, or 1 amino acid difference with at least one of the amino acid sequences from the "CDR1 Sequences Group 23";
   and
- CDR2 is chosen from the group consisting of:
   d) the amino acid sequences from the "CDR2 Sequences Group 25";
   e) amino acid sequences that have at least 80% amino acid identity with at least one of the amino acid sequences from the "CDR2 Sequences Group 25";
   f) amino acid sequences that have 3, 2, or 1 amino acid difference with at least one of the amino acid sequences from the "CDR2 Sequences Group 25";
   and
- CDR3 is chosen from the group consisting of:
   g) the amino acid sequences from the "CDR3 Sequences Group 27";
   h) amino acid sequences that have at least 80% amino acid identity with at least one of the amino acid sequences from the "CDR3 Sequences Group 27";
   i) amino acid sequences that have 3, 2, or 1 amino acid difference with at least one of the amino acid sequences from the "CDR3 Sequences Group 27"; or any suitable fragment of such an amino acid sequence

In particular, such an amino acid sequence of the invention may be such that CDR1 is chosen from the group consisting of the amino acid sequences from the "CDR1 Sequences Group 23"; and CDR2 is chosen from the group consisting of the amino acid sequences from the "CDR2 Sequences Group 25"; and CDR3 is chosen from the group consisting of the amino acid sequences from the "CDR3 Sequences Group 27".

Again, preferred combinations of CDR sequences will become clear from the further description herein.

Also, such amino acid sequences are preferably such that they can specifically bind (as defined herein) to the p40 subunit; and more in particular bind to the p40 subunit with an affinity (suitably measured and/or expressed as a K_{D}-value (actual or apparent), a K_{A}-value (actual or apparent), a kₒₙ-rate and/or a k_{off}-rate, or alternatively as an IC₅₀ value (all as further) described herein) that is as defined herein.

In one preferred, but non-limiting aspect, the invention relates to an amino acid sequence that essentially consists of 4 framework regions (FR1 to FR4, respectively) and 3 complementarity determining regions (CDR1 to CDR3 respectively), in which the CDR sequences of said amino acid sequence have at least 70% amino acid identity, preferably at least 80% amino acid identity, more preferably at least 90% amino acid identity, such as 95% amino acid identity or more or even essentially 100% amino acid identity with the CDR sequences of at least one of the amino acid sequences of SEQ ID NO's: 1942; 1946; 1948; 1950; 1952; 1953; 1954; 1956; 1957; 1959; 1962; 1963; 1964; 1965; 1967; 1969; 1970; 1973; 1981; 1982; 1984; 1985; 1987; 1988; 1990; 1993; 1994; 1995; 1997; 1998; 1999; 2000; 2001; 2003; 2005; 2030; 2510; 2511; 2512; 2513; 2514; 2515; 2516; 2517; 2518; 2519; 2520; 2521; 2522; 2523; 2524; 2525; 2526; 2527 and/or 2528 (see Table A-2 and Figure 23). This degree of amino acid identity can for example be determined by determining the degree of amino acid identity (in a manner described herein) between said amino acid sequence and one or more of the sequences of SEQ ID NO's: 1942; 1946; 1948; 1950; 1952; 1953; 1954; 1956; 1957; 1959; 1962; 1963; 1964; 1965; 1967; 1969; 1970; 1973; 1981; 1982; 1984; 1985; 1987; 1988; 1990; 1993; 1994; 1995; 1997; 1998; 1999; 2000; 2001; 2003;2005;2030;2510;2511;2512;2513;2514;2515;2516;2517;2518;2519;2520; 2521; 2522; 2523; 2524; 2525; 2526; 2527 and/or 2528 (see Table A-2 and Figure 23), in which the amino acid residues that form the framework regions are disregarded. Such amino acid sequences of the invention can be as further described herein.

Some preferred, but non-limiting examples of p40+ sequences are the amino acid sequences of SEQ ID NO's: 1942; 1946; 1948; 1950; 1952; 1953; 1954; 1956; 1957; 1959; 1962; 1963; 1964; 1965; 1967; 1969; 1970; 1973; 1981; 1982; 1984; 1985; 1987; 1988; 1990; 1993; 1994; 1995; 1997; 1998; 1999; 2000; 2001; 2003; 2005; 2030; 2510; 2511; 2512;2513;2514;2515;2516;2517;2518;2519; 2520;2521;2522;2523;2524;2525; 2526; 2527 and/or 2528 (see Table A-2 and Figure 23). Thus, according to another preferred, but non-limiting aspect of the invention, a p40+ sequence is an amino acid sequence that is directed against (as defined herein) the p40 subunit (as present in for example IL-23 and Il-12) and that are capable of modulating, neutralizing, blocking and/or inhibiting the binding of a heterodimeric cytokine comprising a p40 subunit to its receptor, and in particular capable of modulating, neutralizing, blocking and/or inhibiting the binding of IL-23 to its (cognate) receptor (in particular, in the alpha-screen assay of described in Example 19 or 22); and/or the binding of IL-12 to its (cognate) receptor (in particular, in the alpha-screen assay of described in Example 19), and that either
a) has at least 70% amino acid identity, preferably at least 80% amino acid identity, more preferably at least 90% amino acid identity, such as 95% amino acid identity or more or even essentially 100% amino acid identity with at least one of the amino acid sequences of SEQ ID NO's: 1942; 1946; 1948; 1950; 1952; 1953; 1954; 1956; 1957; 1959; 1962; 1963; 1964; 1965; 1967; 1969; 1970; 1973; 1981; 1982; 1984; 1985; 1987; 1988; 1990; 1993; 1994; 1995; 1997; 1998: 1999; 2000; 2001; 2003; 2005; 2030; 2510; 2511; 2512; 2513;2514;2515;2516;2517;2518;2519;2520;2321;2522;2523;2524;2525;2526; 2527 and/or 2528 (see Table A-2 and Figure 23);
   and/or that
b) has no more than 20, preferably no more than 10, such as 9, 8, 7, 6, 5, 4, 3, 2 or only one amino acid difference with at least one of the amino acid sequences of SEQ ID NO's: 1942; 1946; 1948; 1950; 1952; 1953; 1954; 1956; 1957; 1959; 1962; 1963; 1964; 1965; 1967; 1969; 1970; 1973; 1981; 1982; 1984; 1985; 1987; 1988; 1990; 1993; 1994; 1995; 1997; 1998; 1999; 2000; 2001; 2003; 2005; 2030; 2510; 2511; 2512; 2513; 2514;2515;2516;2517;2518;2519;2520;2521;2522;2523;2524;2525;2526;2527 and/or 2528 (see Table A-2 and Figure 23). Preferably, such an amino acid sequence has no more than a total of 5 (such as 4, 3, 2 or only one) such amino acid differences in the CDR's and/or no more than a total of 5 (such as 4, 3, 2 or only 1) such amino acid differences in the framework sequences;
   and/or that
c) is either (i) capable of cross-blocking (as defined herein) the interaction of at least one of the amino acid sequences of SEQ ID NO's: 1942; 1946; 1948; 1950; 1952; 1953; 1954; 1956; 1957; 1959; 1962; 1963; 1964; 1965; 1967; 1969; 1970; 1973; 1981; 1982; 1984; 1985; 1987; 1988;1990; 1993; 1994; 1995; 1997; 1998; *1999;* 2000; 2001; 2003; 2005; 2030; 2510; 2511; 2512; 2513; 2514; 2515; 2516; 2517; 2518; 2519; 2520; 2521; 2522; 2523; 2524; 2525; 2526; 2527 and/or 2528 with the p40 subunit and/or (ii) being able to compete with (i.e. is a competitor for) the binding of at least one of the amino acid sequences of SEQ ID NO's: 1942; 1946; 1948; 1950; 1952; 1953; 1954; 1956; 1957; 1959; 1962; 1963; 1964; 1965; 1967; 1969; 1970; 1973; 1981; 1982; 1984; 1985; 1987; 1988; 1990; 1993; 1994; 1995; 1997; 1998; 1999; 2000; 2001; 2003; 2005; 2030; 2510; 2511; 2512; 2513; 2514; 2515; 2516; 2517; 2518; 2519; 2520; 2521; 2522; 2523; 2524; 2525; 2526; 2527 and/or 2528 (see Table A-2 and Figure 23) to the p40 subunit.

In another preferred, but non-limiting aspect, a p40+ sequence is chosen from one of the amino acid sequences of SEQ ID NO's: 1942; 1946; 1948; 1950; 1952; 1953; 1954; 1956; 1957; 1959; 1962; 1963; 1964; 1965; 1967; 1969; 1970; 1973; 1981; 1982; 1984; 1985; 1987; 1988; 1990; 1993; 1994; 1995; 1997; 1998; 1999; 2000; 2001; 2003; 2005; 2030; 2510; 2511; 2512; 2513; 2514; 2515; 2516; 2517; 2518; 2519; 2520; 2521; 2522; 2523; 2524; 2525; 2526; 2527 and/or 2528 (see Table A-2 and Figure 23).

### E) P35 sequences.

One specific, but non-limiting aspect relates to "p35 sequences", which generally are defined herein as amino acid sequences of the invention that are directed against (as defined herein) the p35 subunit (as present in for example IL-12).

P35 sequences may generally be as further described herein for amino acid sequences of the invention, i.e. in terms of affinity, specificity etc. for p35. Also, as described herein for the amino acid sequences of the invention, the p35 sequences are preferably such that they form or are capable of forming (optionally after suitable folding) a single antigen binding domain or antigen binding unit, and may for example be amino acid sequences that comprise an immunoglobulin fold, amino acid sequences that are comprised of four framework regions and three CDR's, and may in particular be domain antibodies, single domain antibodies, VHH's, "dAb's" or Nanobodies (all as further described herein), or suitable fragments thereof.

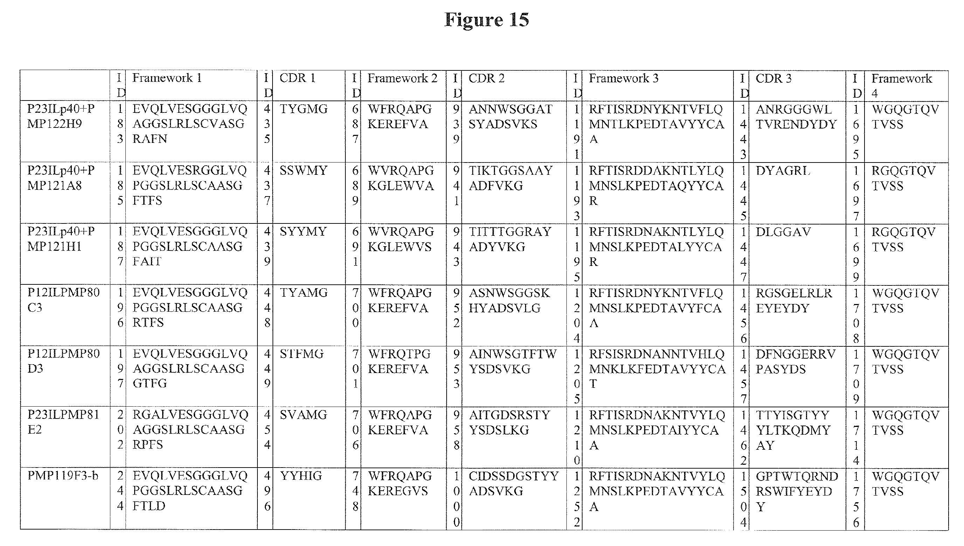

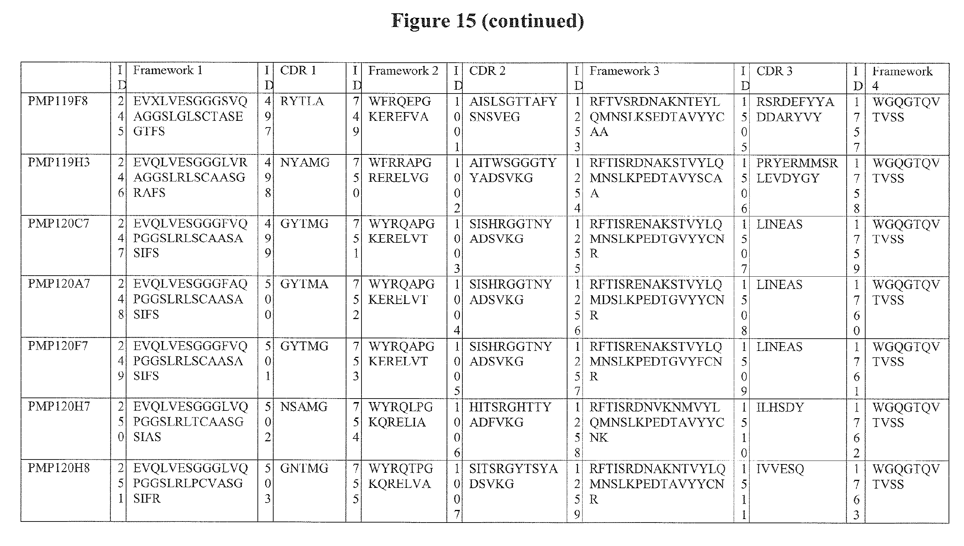

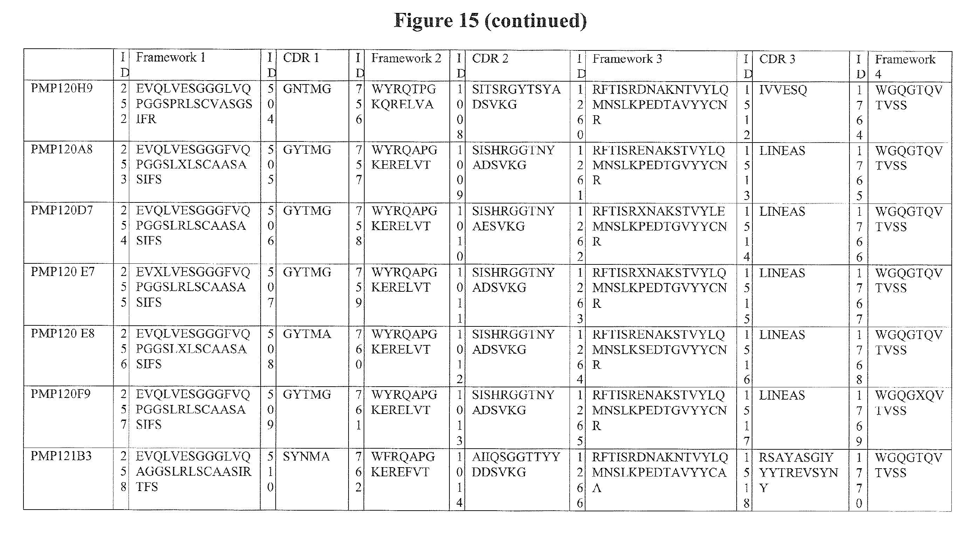

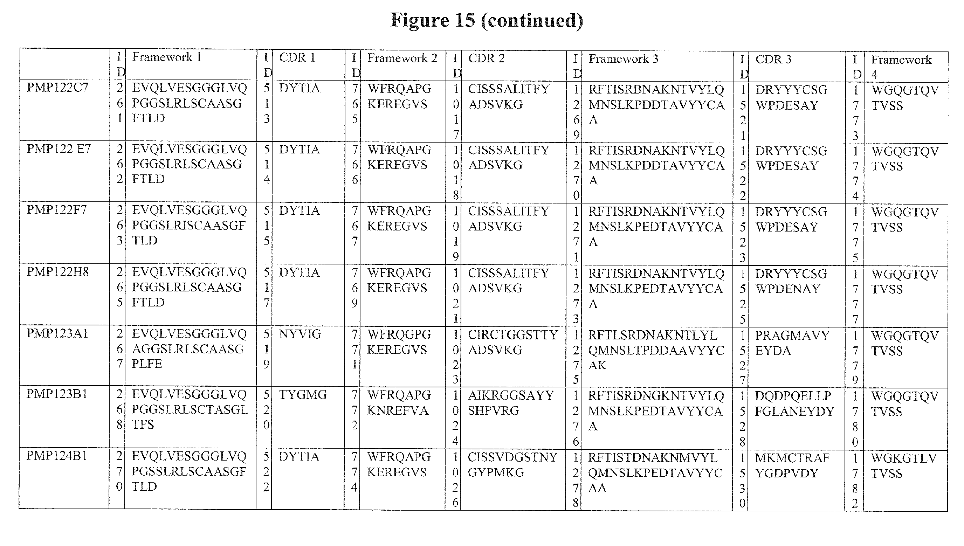

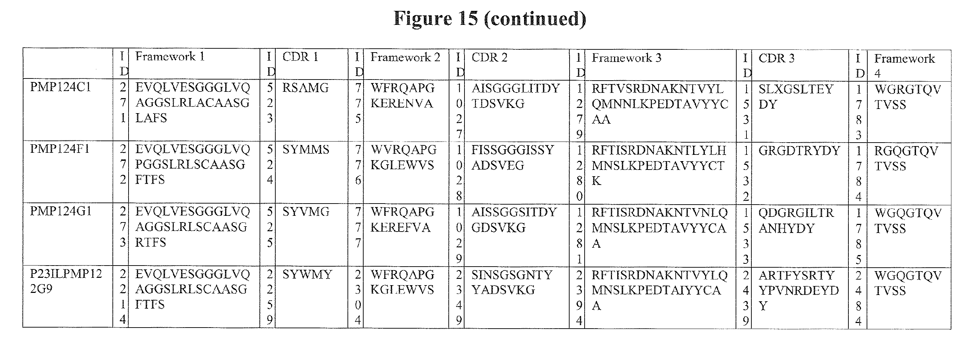

In one particular aspect, a p35 sequence may comprise one or more stretches of Amino acid residues chosen from the group consisting of:
a) the amino acid sequences from the "CDR1 Sequences Group 30" (as defined and listed in Table A-1; see also Figure 15);
b) amino acid sequences that have at least 80% amino acid identity with at least one of the amino acid sequences from the "CDR1 Sequences Group 30";
c) amino acid sequences that have 3, 2, or I amino acid difference with at least one of the amino acid sequences from the "CDR1 Sequences Group 30";
d) the amino acid sequences from the "CDR2 Sequences Group 32" (as defined and listed in Table A-1; see also Figure 15);
e) amino acid sequences that have at least 80% amino acid identity with at least one of the amino acid sequences from the "CDR2 Sequences Group 32";
f) amino acid sequences that have 3, 2, or 1 amino acid difference with at least one of the amino acid sequences from the "CDR2 Sequences Group 32";
g) the amino acid sequences from the "CDR3 Sequences Group 34" (as defined and listed in Table A-1; see also Figure 15);
h) amino acid sequences that have at least 80% amino acid identity with at least one of the amino acid sequences from the "CDR3 Sequences Group 34";
i) amino acid sequences that have 3, 2, or 1 amino acid difference with at least one of the amino acid sequences from the "CDR3 Sequences Group 34";
or any suitable combination thereof.

Optionally, when an amino acid sequence of the invention contains one or more amino acid sequences according to b) and/or c), Optional Condition I, Optional Condition II and/or Optional Condition III (all as defined herein) may apply to said amino acid sequence (i.e. compared to the original amino acid sequence according to a)). Also, optionally, when an amino acid sequence of the invention contains one or more amino acid sequences according to e) and/or f), Optional Condition I, Optional Condition II and/or Optional Condition III (all as defined herein) may apply to said amino acid sequence (i.e. compared to the original amino acid sequence according to d)). Also, optionally, when an amino acid sequence of the invention contains one or more amino acid sequences according to h) and/or i), Optional Condition I, Optional Condition II and/or Optional Condition III (all as defined herein) may apply to said amino acid sequence (i.e. compared to the original amino acid sequence according to g)).

In this specifc aspect, the amino acid sequence preferably comprises one or more stretches of amino acid residues chosen from the group consisting of:
a) the amino acid sequences from the "CDR1 Sequences Group 30";
b) the amino acid sequences from the "CDR2 Sequences Group 32"; and
c) the amino acid sequences from the "CDR3 Sequences Group 34";
or any suitable combination thereof.

Also, preferably, in such an amino acid sequence, at least one of said stretches of amino acid residues forms part of the antigen binding site for binding against p35.

In a more specific, but again non-limiting aspect, a p35 sequence may comprise two or more stretches of amino acid residues chosen from the group consisting of:
a) the amino acid sequences from the "CDR1 Sequences Group 30";
b) amino acid sequences that have at least 80% amino acid identity with at least one of the amino acid sequences from the "CDR1 Sequences Group 30";
c) amino acid sequences that have 3, 2, or 1 amino acid difference with at least one of the amino acid sequences from the "CDR1 Sequences Group 30";
d) the amino acid sequences from the "CDR2 Sequences Group 32";
e) amino acid sequences that have at least 80% amino acid identity with at least one of the amino acid sequences from the "CDR2 Sequences Group 32";
f) amino acid sequences that have 3, 2, or 1 amino acid difference with at least one of the amino acid sequences from the "CDR2 Sequences Group 32";
g) the amino acid sequences from the "CDR3 Sequences Group 34";
h) amino acid sequences that have at least 80% amino acid identity with at least one of the amino acid sequences from the "CDR3 Sequences Group 34";
i) amino acid sequences that have 3, 2, or 1 amino acid difference with at least one of the amino acid sequences from the "CDR3 Sequences Group 34";
such that (i) when the first stretch of amino acid residues corresponds to one of the amino acid sequences according to a), b) or c), the second stretch of amino acid residues corresponds to one of the amino acid sequences according to d), e), f), g), h) or i); (ii) when the first stretch of amino acid residues corresponds to one of the amino acid sequences according to d), e) or f), the second stretch of amino acid residues corresponds to one of the amino acid sequences according to a), b), c), g), h) or i); or (iii) when the first stretch of amino acid residues corresponds to one of the amino acid sequences according to g), h) or i), the second stretch of amino acid residues corresponds to one of the amino acid sequences according to a), b), c), d), e) or f).

In this specific aspect, the amino acid sequence preferably comprises two or more stretches of amino acid residues chosen from the group consisting of:
a) the amino acid sequences from the "CDR1 Sequences Group 30";
b) the amino acid sequences from the "CDR2 Sequences Group 32"; and
c) the amino acid sequences from the "CDR3 Sequences Group 34";
such that, (i) when the first stretch of amino acid residues corresponds to one of the amino acid sequences from the "CDR1 Sequences Group 30", the second stretch of amino acid residues corresponds to one of the amino acid sequences from the "CDR2 Sequences Group 32" or from the "CDR3 Sequences Group 34"; (ii) when the first stretch of amino acid residues corresponds to one of the amino acid sequences from the "CDR2 Sequences Group 32", the second stretch of amino acid residues corresponds to one of the amino acid sequences from the "CDR1 Sequences Group 30" or from the "CDR3 Sequences Group 34"; or (iii)) when the first stretch of amino acid residues corresponds to one of the amino acid sequences from the "CDR3 Sequences Group 34", the second stretch of amino acid residues corresponds to one of the amino acid sequences from the "CDR1 Sequences Group 30" or from the "CDR2 Sequences Group 32".

Also, in such an amino acid sequence, the at least two stretches of amino acid residues again preferably form part of the antigen binding site for binding against p35.

In an even more specific, but non-limiting aspect, a p35 sequence may comprise three or more stretches of amino acid residues, in which the first stretch of amino acid residues is chosen from the group consisting of:
a) the amino acid sequences from the "CDR1 Sequences Group 30";
b) amino acid sequences that have at least 80% amino acid identity with at least one of the amino acid sequences from the "CDR1 Sequences Group 30";
c) amino acid sequences that have 3, 2, or 1 amino acid difference with at least one of the amino acid sequences from the "CDR1 Sequences Group 30";
   the second stretch of amino acid residues is chosen from the group consisting of:
d) the amino acid sequences from the "CDR2 Sequences Group 32";
e) amino acid sequences that have at least 80% amino acid identity with at least one of the amino acid sequences from the "CDR2 Sequences Group 32";
f) amino acid sequences that have 3, 2, or 1 amino acid difference with at least one of the amino acid sequences from the "CDR2 Sequences Group 32";
   and the third stretch of amino acid residues is chosen from the group consisting of:
g) the amino acid sequences from the "CDR3 Sequences Group 34";
h) amino acid sequences that have at least 80% amino acid identity with at least one of the amino acid sequences from the "CDR3 Sequences Group 34";
i) amino acid sequences that have 3, 2, or 1 amino acid difference with at least one of the amino acid sequences from the "CDR3 Sequences Group 34",

Preferably, in this specifc aspect, the first stretch of amino acid residues is chosen from the group consisting of the amino acid sequences from the "CDR1 Sequences Group 30"; the second stretch of amino acid residues is chosen from the group consisting of the amino acid sequences from the "CDR2 Sequences Group 32"; and the third stretch of amino acid residues is chosen from the group consisting of the amino acid sequences from the "CDR3 Sequences Group 34".

Again, preferably, in such an amino acid sequence, the at least three stretches of amino acid residues forms part of the antigen binding site for binding against p35.

Preferred combinations of such stretches of amino acid sequences will become clear from the further disclosure herein.

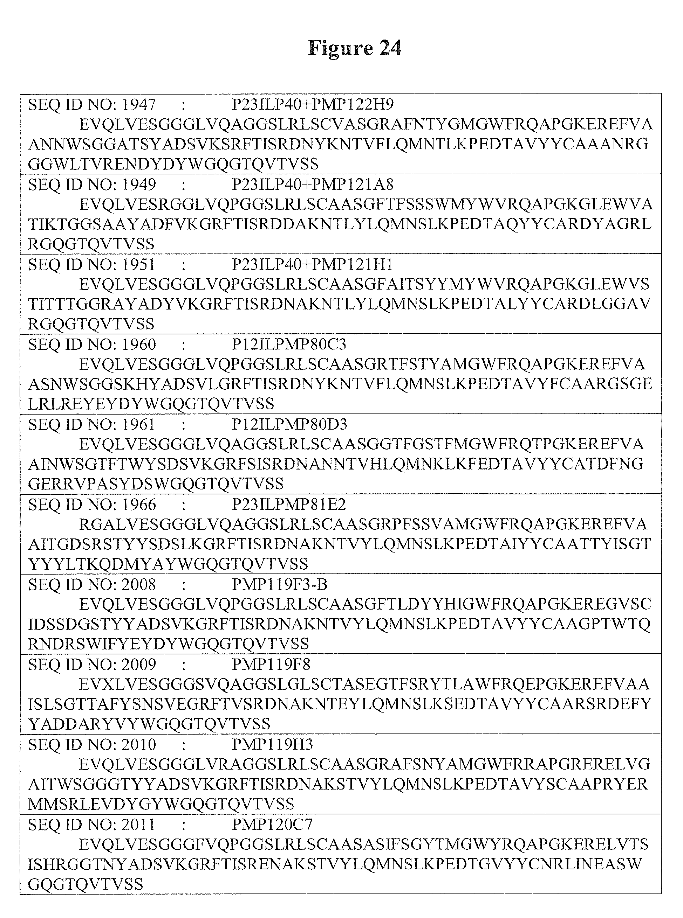

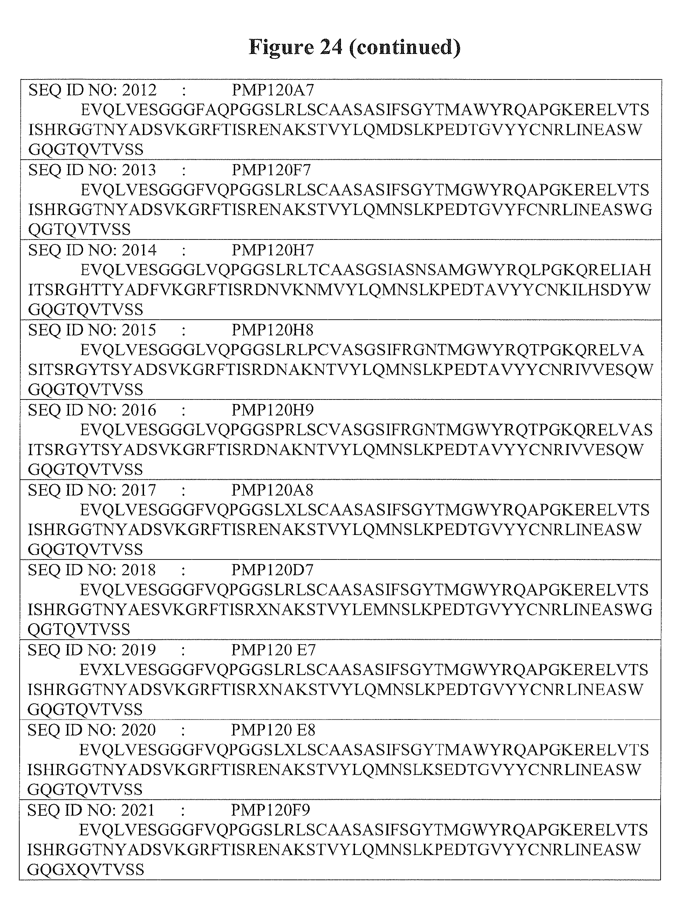

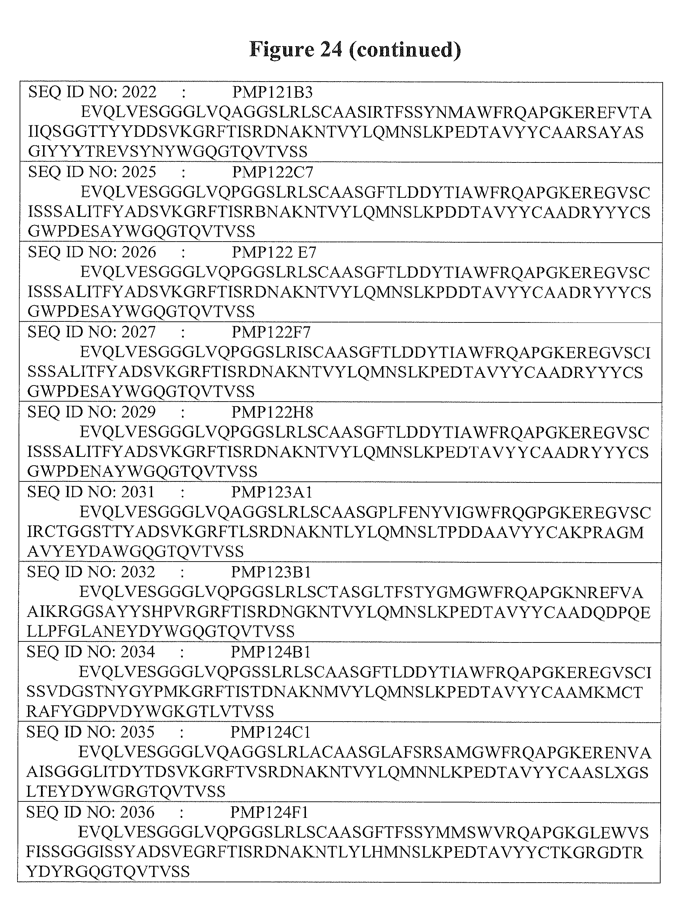

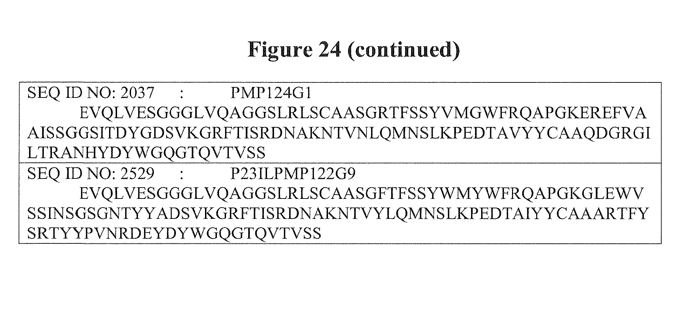

Preferably, in such amino acid sequences the CDR sequences have at least 70% amino acid identity, preferably at least 80% amino acid identity, more preferably at least 90% amino acid identity, such as 95% amino acid identity or more or even essentially 100% amino acid identity with the CDR sequences of at least one of the p35 sequences listed in Table A-2 and Figure 24. This degree of amino acid identity can for example be determined by determining the degree of amino acid identity (in a manner described herein) between said amino acid sequence and one or more of the sequences of SEQ ID NO's: 1947; 1949; 1951; 1960; 1961; 1966; 2008; 2009; 2010; 2011; 2012; 2013; 2014; 2015; 2016; 2017; 2018; 2019; 2020; 2021; 2022; 2025; 2026; 2027; 2029; 2031; 2032; 2034; 2035; 2036; 2037 and/or 2529 (see Table A-2 and Figure 24), in which the amino acid residues that form the framework regions are disregarded. Also, such amino acid sequences of the invention can be as further described herein.

Also, such amino acid sequences are preferably such that they can specifically bind (as defined herein) to the p35 subunit; and more in particular bind to the p35 subunit with an affinity (suitably measured and/or expressed as a K_{D}-value (actual or apparent), a K_{A}-value (actual or apparent), a kₒₙ-rate and/or a k_{off}-rate, or alternatively as an IC₅₀ value (all as further) described herein) that is as defined herein.

When the amino acid sequence of the invention essentially consists of 4 framework regions (FR1 to FR4, respectively) and 3 complementarity determining regions (CDR1 to CDR3, respectively), the amino acid sequence of the invention is preferably such that:
- CDR1 is chosen from the group consisting of:
   a) the amino acid sequences from the "CDR1 Sequences Group 30";
   b) amino acid sequences that have at least 80% amino acid identity with at least one of the amino acid sequences from the "CDR1 Sequences Group 30";
   c) amino acid sequences that have 3, 2, or 1 amino acid difference with at least one of the amino acid sequences from the "CDR1 Sequences Group 30";
   and/or
- CDR2 is chosen from the group consisting of:
   d) the amino acid sequences from the "CDR2 Sequences Group 32";
   e) amino acid sequences that have at least 80% amino acid identity with at least one of the amino acid sequences from the "CDR2 Sequences Group 32";
   f) amino acid sequences that have 3, 2, or 1 amino acid difference with at least one of the amino acid sequences from the "CDR2 Sequences Group 32";
   and/or
- CDR3 is chosen from the group consisting of:
   g) the amino acid sequences from the "CDR3 Sequences Group 34";
   h) amino acid sequences that have at least 80% amino acid identity with at least one of the amino acid sequences from the "CDR3 Sequences Group 34";
   i) amino acid sequences that have 3, 2, or 1 amino acid difference with at least one of the amino acid sequences from the "CDR3 Sequences Group 34".

In particular, such an amino acid sequence of the invention may be such that CDR1 is chosen from the group consisting of the amino acid sequences from the "CDR1 Sequences Group 30"; and/or CDR2 is chosen from the group consisting of the amino acid sequences from the "CDR2 Sequences Group 32"; and/or CDR3 is chosen from the group consisting of the amino acid sequences from the "CDR3 Sequences Group 34".

In particular, when the amino acid sequence of the invention essentially consists of) 4 framework regions (FR1 to FR4, respectively) and 3 complementarity determining regions (CDR1 to CDR3, respectively), the amino acid sequence of the invention is preferably such that:
- CDR1 is chosen from the group consisting of:
   a) the amino acid sequences from the "CDR1 Sequences Group 30";
   b) amino acid sequences that have at least 80% amino acid identity with at least one of the amino acid sequences from the "CDR1 Sequences Group 30";
   c) amino acid sequences that have 3, 2, or I amino acid difference with at least one of the amino acid sequences from the "CDR1 Sequences Group 30";
   and
- CDR2 is chosen from the group consisting of:
   d) the amino acid sequences from the "CDR2 Sequences Group 32";
   e) amino acid sequences that have at least 80% amino acid identity with at least one of the amino acid sequences from the "CDR2 Sequences Group 32";
   f) amino acid sequences that have 3, 2, or 1 amino acid difference with at least one of the amino acid sequences from the "CDR2 Sequences Group 32";
   and
- CDR3 is chosen from the group consisting of:
   g) the amino acid sequences from the "CDR3 Sequences Group 34";
   h) amino acid sequences that have at least 80% amino acid identity with at least one of the amino acid sequences from the "CDR3 Sequences Group 34";
   i) amino acid sequences that have 3, 2, or 1 amino acid difference with at least one of the amino acid sequences from the "CDR3 Sequences Group 34"; or any suitable fragment of such an amino acid sequence

In particular, such an amino acid sequence of the invention may be such that CDR1 is chosen from the group consisting of the amino acid sequences from the "CDR1 Sequences Group 30"; and CDR2 is chosen from the group consisting of the amino acid sequences from the "CDR2 Sequences Group 32"; and CDR3 is chosen from the group consisting of the amino acid sequences from the "CDR3 Sequences Group 34".

Again, preferred combinations of CDR sequences will become clear from the further description herein,

Also, such amino acid sequences are preferably such that they can specifically bind (as defined herein) to the p35 subunit; and more in particular bind to the p35 subunit with an affinity (suitably measured and/or expressed as a K_{D}-value (actual or apparent), a K_{A}-value (actual or apparent), a kₒₙ-rate and/or a k_{off}-rate, or alternatively as an IC₅₀ value (all as further) described herein) that is as defined herein.

In one preferred, but non-limiting aspect, the invention relates to an amino acid sequence that essentially consists of 4 framework regions (FR1 to FR4, respectively) and 3 complementarity determining regions (CDR1 to CDR3, respectively), in which the CDR sequences of said amino acid sequence have at least 70% amino acid identity, preferably at least 80% amino acid identity, more preferably at least 90% amino acid identity, such as 95% amino acid identity or more or even essentially 100% amino acid identity with the CDR sequences of at least one of the amino acid sequences of SEQ ID NO's: 1947; 1949; 1951; 1960; 1961; 1966; 2008; 2009; 2010; 2011; 2012; 2013; 2014; 2015; 2016; 2017; 2018; 2019; 2020; 2021; 2022; 2025; 2026; 2027; 2029; 2031; 2032; 2034; 2035; 2036; 2037 and/or 2529 (see Table A-2 and Figure 24). This degree of amino acid identity can for example be determined by determining the degree of amino acid identity (in a manner described herein) between said amino acid sequence and one or more of the sequences of SEQ ID NO's: 1947; 1949; 1951; 1960; 1961; 1966; 2008; 2009; 2010; 2011; 2012; 2013; 2014; 2015; 2016; 2017; 2018; 2019; 2020; 2021; 2022; 2025; 2026; 2027; 2029; 2031; 2032; 2034; 2035; 2036; 2037 and/or 2529 (see Table A-2 and Figure 24), in which the amino acid residues that form the framework regions are disregarded. Such amino acid sequences of the invention can be as further described herein.

Some preferred, but non-limiting examples of p35 sequences are the amino acid sequences of SEQ ID NQ's: 1947; 1949; 1951; 1960; 1961; 1966; 2008; 2009; 2010; 2011; 2012; 2013; 2014; 2015; 2016; 2017; 2018; 2019; 2020; 2021; 2022; 2025; 2026; 2027; 2029; 2031; 2032; 2034; 2035; 2036; 2037 and/or 2529 (see Table A-2 and Figure 24). Thus, according to another preferred, but non-limiting aspect of the invention, a p35 sequence is an amino acid sequence that is directed against (as defined herein) the p35 subunit (as present in for example IL-12) and that either:
a) has at least 70% amino acid identity, preferably at least 80% amino acid identity, more preferably at least 90% amino acid identity, such as 95% amino acid identity or more or even essentially 100% amino acid identity with at least one of the amino acid sequences of SEQ ID NO's: 1947; 1949; 1951; 1960; 1961; 1966; 2008; 2009; 2010; 2011; 2012; 2013; 2014; 2015; 2016; 2017; 2018; 2019; 2020; 2021; 2022; 2025; 2026; 2027; 2029; 2031; 2032; 2034; 2035; 2036; 2037 and/or 2529 (see Table A-2 and Figure 24); and/or that
b) has no more than 20, preferably no more than 10, such as 9, 8, 7, 6, 5, 4, 3, 2 or only one amino acid difference with at least one of the amino acid sequences of SEQ ID NO's: 1947; 1949; 1951; 1960; 1961; 1966; 2008; 2009; 2010; 2011; 2012; 2013; 2014; 2015; 2016; 2017; 2018; 2019; 2020; 2021; 2022; 2025; 2026; 2027; 2029; 2031; 2032; 2034; 2035; 2036; 2037 and/or 2529 (see Table A-2 and Figure 24). Preferably, such an amino acid sequence has no more than a total of 5 (such as 4, 3, 2 or only one) such amino acid differences in the CDR's and/or no more than a total of 5 (such as 4, 3. 2 or only 1) such amino acid differences in the framework sequences;
   and/or that
c) is either (i) capable of cross-blocking (as defined herein) the interaction of at least one of the amino acid sequences of SEQ ID NO's: 1947; 1949; 1951; 1960; 1961; 1966; 2008; 2009; 2010; 2011; 2012; 2013; 2014; 2015; 2016; 2017; 2018; 2019; 2020; 2021; 2022; 2025; 2026; 2027; 2029; 2031; 2032; 2034; 2035; 2036; 2037 and/or 2529 with the p35 subunit and/or (ii) being able to compete with (i.e. is a competitor for) the binding of at least one of the amino acid sequences of SEQ ID NO's: 1947; 1949; 1951; 1960; 1961; 1966; 2008; 2009; 2010; 2011; 2012; 2013; 2014; 2015; 2016; 2017; 2018; 2019; 2020; 2021; 2022; 2025; 2026; 2027; 2029; 2031; 2032; 2034; 2035; 2036; 2037 and/or 2529 (see Table A-2 and Figure 24) to the p35 subunit.

In another preferred, but non-limiting aspect, a p35 5 sequence is chosen from one of the amino acid sequences of SEQ ID NO's: 1947; 1949; 1951; 1960; 1961; 1966; 2008; 2009; 2010; 2011; 2012; 2013; 2014; 2015; 2016; 2017; 2018; 2019; 2020; 2021; 2022; 2025; 2026; 2027; 2029; 2031; 2032; 2034; 2035; 2036; 2037 and/or 2529 (see Table A-2 and Figure 24).

### F) IL-27 sequences.

One specific, but non-limiting aspect relates to "IL-27 sequences", which generally are defined herein as amino acid sequences of the invention that are directed against (as defined herein) IL-27 (either against the EB13 subunit or the p28 subunit).

IL-27 sequences may generally be as further described herein (for example, in terms of affinity, specificity etc, for IL-27 or one of its subunits) for amino acid sequences of the invention in general. Also, as described herein for the amino acid sequences of the invention, the IL-27 sequences are preferably such that they form or are capable of forming (optionally after suitable folding) a single antigen binding domain or antigen binding unit, and may for example be amino acid sequences that comprise an immunoglobulin fold, amino acid sequences that are comprised of four framework regions and three CDR's, and may in particular be domain antibodies, single domain antibodies, VHH's, "dAb's" or Nanobodies (all as further described herein), or suitable fragments thereof.

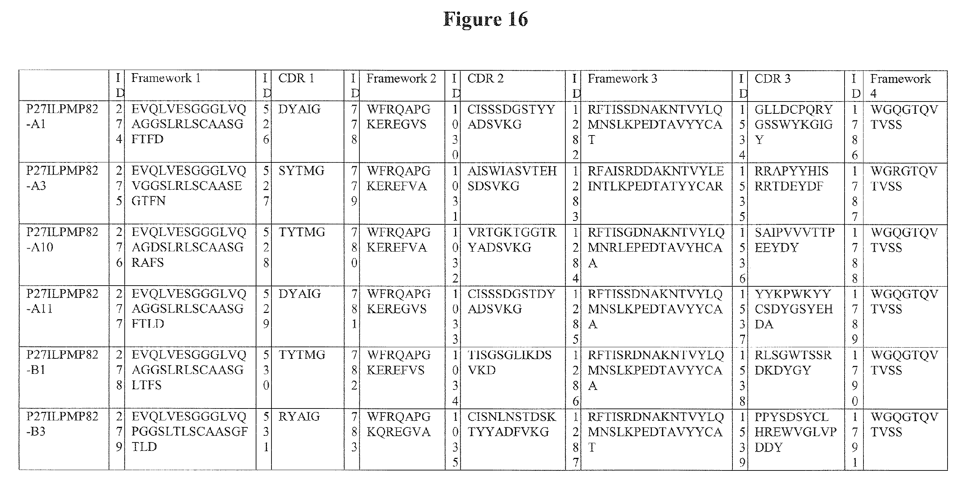

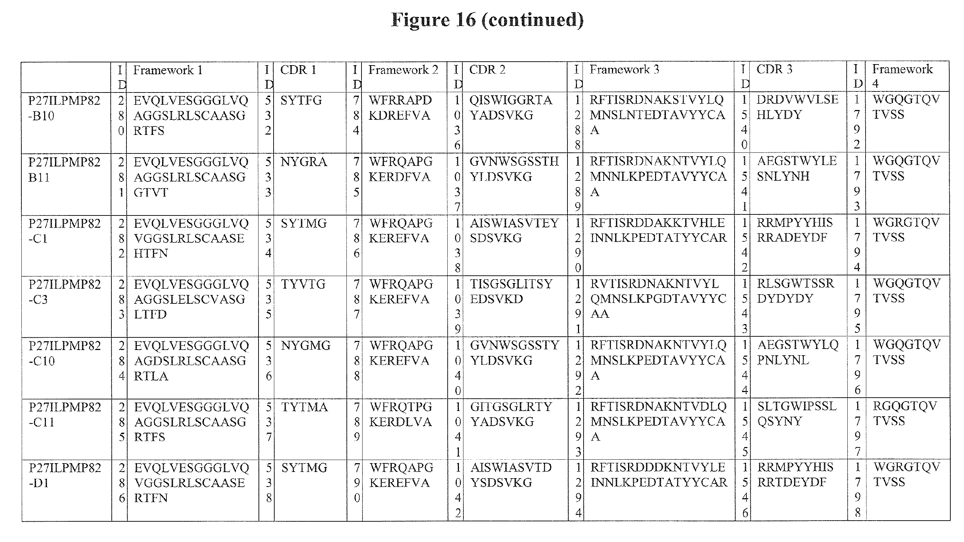

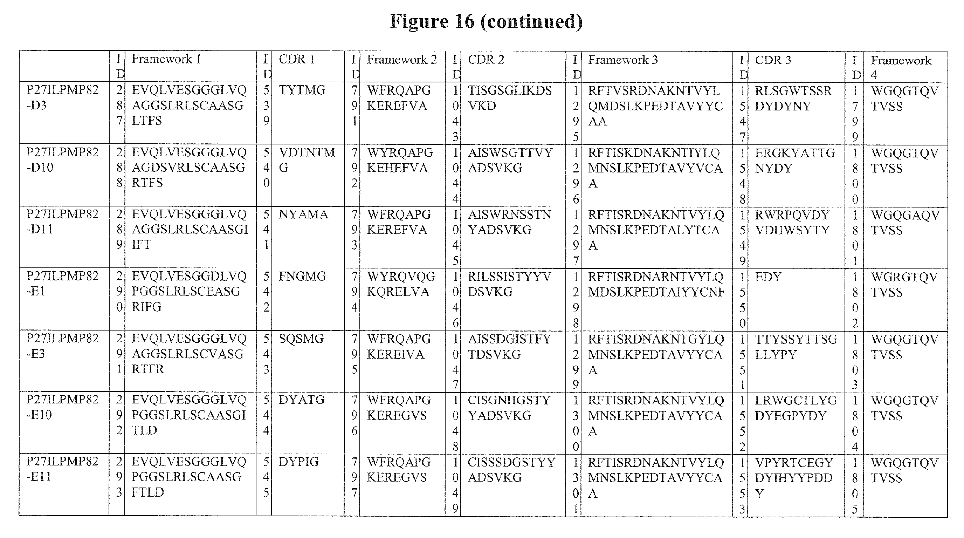

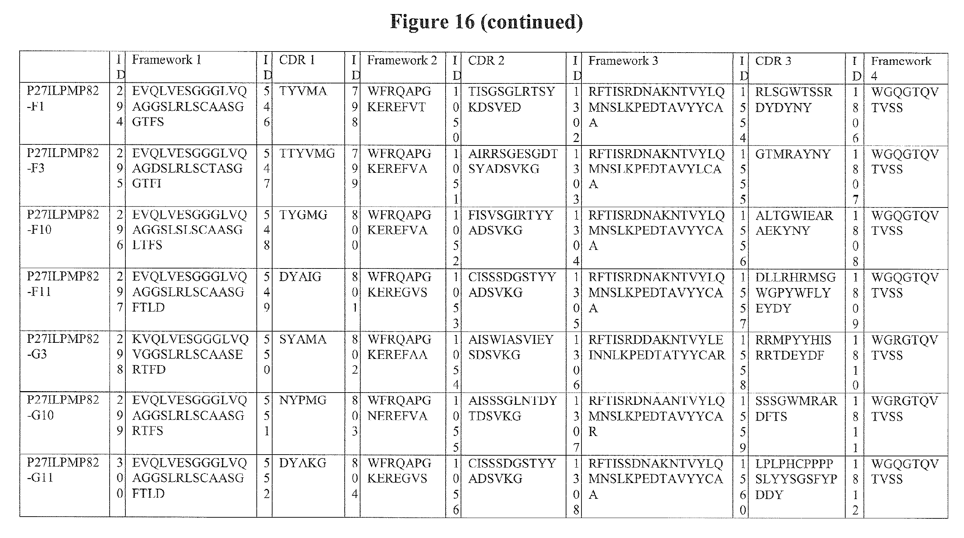

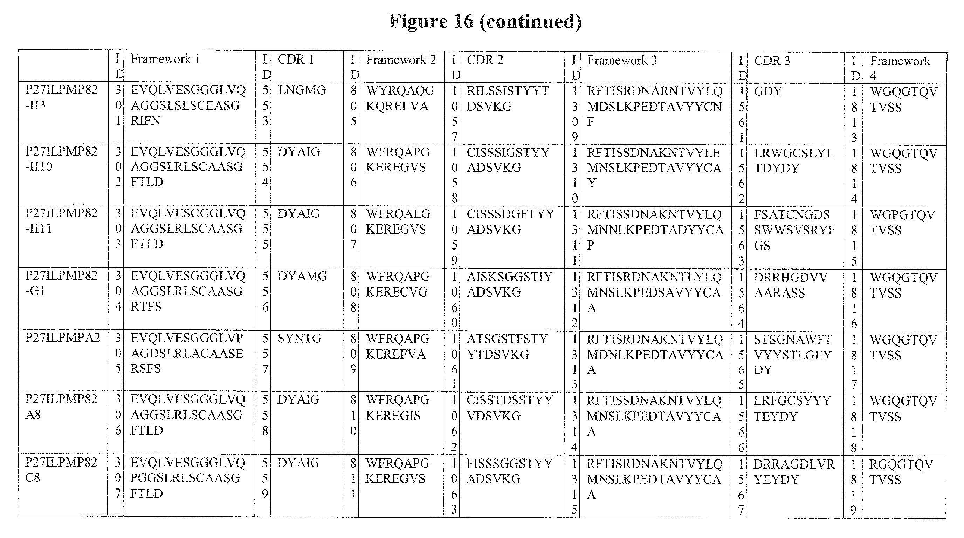

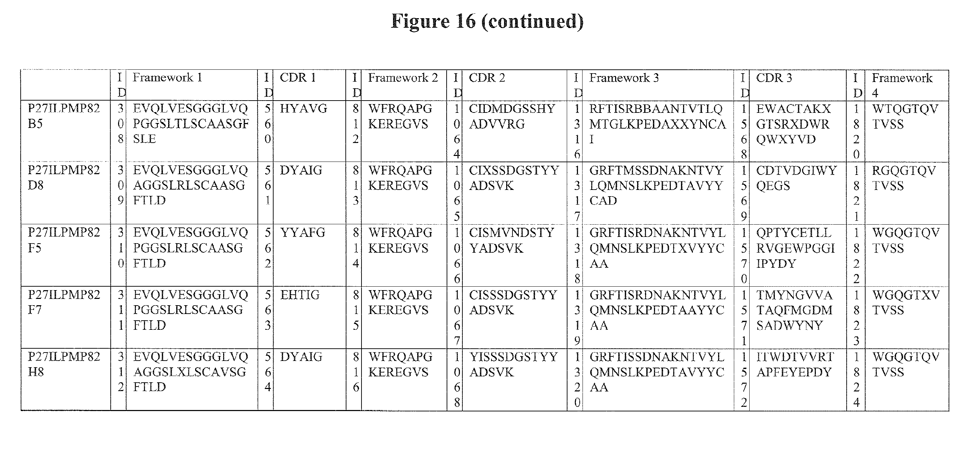

In one particular aspect, an IL-27 sequence may comprise one or more stretches of amino acid residues chosen from the group consisting of:
a) the amino acid sequences from the "CDR1 Sequences Group 37" (as defined and listed in Table A-1; see also Figure 16);
b) amino acid sequences that have at least 80% amino acid identity with at least one of the amino acid sequences from the "CDR1 Sequences Group 37";
c) amino acid sequences that have 3, 2, or 1 amino acid difference with at least one of the amino acid sequences from the "CDR1 Sequences Group 37";
d) the amino acid sequences from the "CDR2 Sequences Group 39" (as defined and listed in Table A-1; see also Figure 16);
e) amino acid sequences that have at least 80% amino acid identity with at least one of the amino acid sequences from the "CDR2 Sequences Group 39";
f) amino acid sequences that have 3, 2, or 1 amino acid difference with at least one of the amino acid sequences from the "CDR2 Sequences Group 39";
g) the amino acid sequences from the "CDR3 Sequences Group 41" (as defined and listed in Table A-1; see also Figure 16);
h) amino acid sequences that have at least 80% amino acid identity with at least one of the amino acid sequences from the "CDR3 Sequences Group 41";
i) amino acid sequences that have 3, 2, or 1 amino acid difference with at least one of the amino acid sequences from the "CDR3 Sequences Group 41";
or any suitable combination thereof.

Optionally, when an amino acid sequence of the invention contains one or more amino acid sequences according to b) and/or c), Optional Condition 1, Optional Condition II and/or Optional Condition III (all as defined herein) may apply to said amino acid sequence (i.e. compared to the original amino acid sequence according to a)). Also, optionally, when an amino acid sequence of the invention contains one or more amino acid sequences according to e) and/or f), Optional Condition I, Optional Condition II and/or Optional Condition HI (all as defined herein) may apply to said amino acid sequence (i.e. compared to the original amino acid sequence according to d)). Also, optionally, when an amino acid sequence of the invention contains one or more amino acid sequences according to h) and/or i), Optional Condition I, Optional Condition II and/or Optional Condition III (all as defined herein) may apply to said amino acid sequence (i.e, compared to the original amino acid sequence according to g)).

In this specific aspect, the amino acid sequence preferably comprises one or more stretches of amino acid residues chosen from the group consisting of:
a) the amino acid sequences from the "CDR1 Sequences Group 37";
b) the amino acid sequences from the "CDR2 Sequences Group 39"; and
c) the amino acid sequences from the "CDR3 Sequences Group 41";
or any suitable combination thereof.

Also, preferably, in such an amino acid sequence, at least one of said stretches of amino acid residues forms part of the antigen binding site for binding against IL-27.

In a more specific, but again non-limiting aspect, an IL-27 sequence may comprise two or more stretches of amino acid residues chosen from the group consisting of:
a) the amino acid sequences from the "CDR1 Sequences Group 37";
b) amino acid sequences that have at least 80% amino acid identity with at least one of the amino acid sequences from the "CDR1 Sequences Group 37";
c) amino acid sequences that have 3, 2, or 1 amino acid difference with at least one of the amino acid sequences from the "CDR1 Sequences Group 37";
d) the amino acid sequences from the "CDR2 Sequences Group 39";
e) amino acid sequences that have at least 80% amino acid identity with at least one of the amino acid sequences from the "CDR2 Sequences Group 39";
f) amino acid sequences that have 3, 2, or 1 amino acid difference with at least one of the amino acid sequences from the "CDR2 Sequences Group 39";
g) the amino acid sequences from the "CDR3 Sequences Group 41";
h) amino acid sequences that have at least 80% amino acid identity with at least one of the amino acid sequences from the "CDR3 Sequences Group 41";
i) amino acid sequences that have 3,2, or 1 amino acid difference with at least one of the amino acid sequences from the "CDR3 Sequences Group 41 ";
such that (i) when the first stretch of amino acid residues corresponds to one of the amino acid sequences according to a), b) or c), the second stretch of amino acid residues corresponds to one of the amino acid sequences according to d), e), f), g), h) or i); (ii) when the first stretch of amino acid residues corresponds to one of the amino acid sequences according to d), e) or f), the second stretch of amino acid residues corresponds to one of the amino acid sequences according to a), b), c), g), h) or i); or (iii) when the first stretch of amino acid residues corresponds to one of the amino acid sequences according to g), h) or i). the second stretch of amino acid residues corresponds to one of the amino acid sequences according to a), b), c), d), e) or f).

In this specific aspect, the amino acid sequence preferably comprises two or more stretches of amino acid residues chosen from the group consisting of:
a) the amino acid sequences from the "CDR1 Sequences Group 37";
b) the amino acid sequences from the "CDR2 Sequences Group 39"; and
c) the amino acid sequences from the "CDR3 Sequences Group 41";
such that, (i) when the first stretch of amino acid residues corresponds to one of the amino acid sequences from the "CDR1 Sequences Group 37", the second stretch of amino acid residues corresponds to one of the amino acid sequences from the "CDR2 Sequences Group 39" or from the "CDR3 Sequences Group 41 "; (ii) when the first stretch of amino acid residues corresponds to one of the amino acid sequences from the "CDR2 Sequences Group 39", the second stretch of amino acid residues corresponds to one of the amino acid sequences from the "CDR1 Sequences Group 37" or from the "CDR3 Sequences Group 41"; or (iii) when the first stretch of amino acid residues corresponds to one of the amino acid sequences from the "CDR3 Sequences Group 41", the second stretch of amino acid residues corresponds to one of the amino acid sequences from the "CDR1 Sequences Group 37" or from the "CDR2 Sequences Group 39".

Also, in such an amino acid sequence, the at least two stretches of amino acid residues again preferably form part of the antigen binding site for binding against IL-27.

In an even more specific, but non-limiting aspect, an IL-27 sequence may comprise three or more stretches of amino acid residues, in which the first stretch of amino acid residues is chosen from the group consisting of:
a) the amino acid sequences from the "CDR1 Sequences Group 37";
b) amino acid sequences that have at least 80% amino acid identity with at least one of the amino acid sequences from the "CDR1 Sequences Group 37";
c) amino acid sequences that have 3, 2, or 1 amino acid difference with at least one of the amino acid sequences from the "CDR1 Sequences Group 37";
   the second stretch of amino acid residues is chosen from the group consisting of:
d) the amino acid sequences from the "CDR2 Sequences Group 39";
e) amino acid sequences that have at least 80% amino acid identity with at least one of the amino acid sequences from the "CDR2 Sequences Group 39";
f) amino acid sequences that have 3, 2, or 1 amino acid difference with at least one of the amino acid sequences from the "CDR2 Sequences Group 39";
   and the third stretch of amino acid residues is chosen from the group consisting of:
g) the amino acid sequences from the "CDR3 Sequences Group 41";
h) amino acid sequences that have at least 80% amino acid identity with at least one of the amino acid sequences from the "CDR3 Sequences Group 41";
i) amino acid sequences that have 3, 2, or 1 amino acid difference with at least one of the amino acid sequences from the "CDR3 Sequences Group 41".

Preferably, in this specifc aspect, the first stretch of amino acid residues is chosen from the group consisting of the amino acid sequences from the "CDR1 Sequences Group 37"; the second stretch of amino acid residues is chosen from the group consisting of the amino acid sequences from the "CDR2 Sequences Group 39"; and the third stretch of amino acid residues is chosen from the group consisting of the amino acid sequences from the "CDR3 Sequences Group 41".

Again, preferably, in such an amino acid sequence, the at least three stretches of amino acid residues forms part of the antigen binding site for binding against IL-27.

Preferred combinations of such stretches of amino acid sequences will become clear from the further disclosure herein.

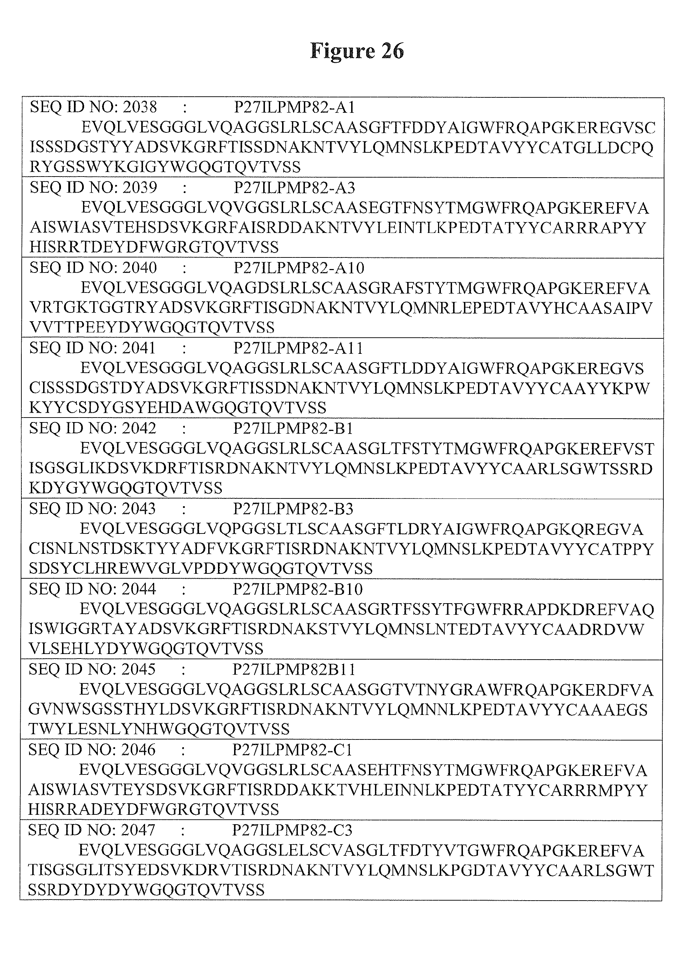

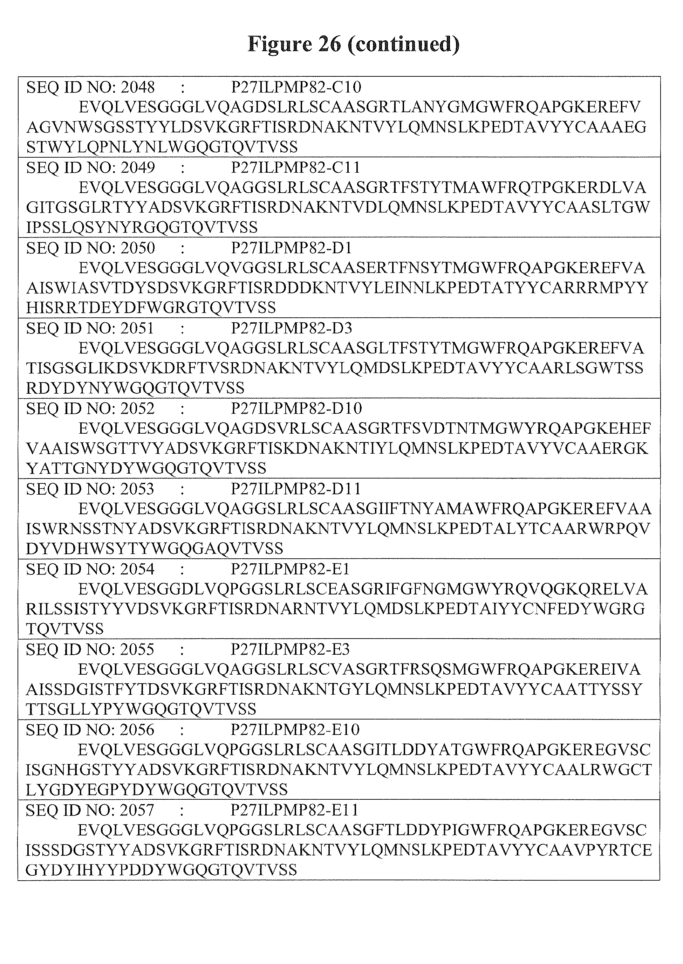

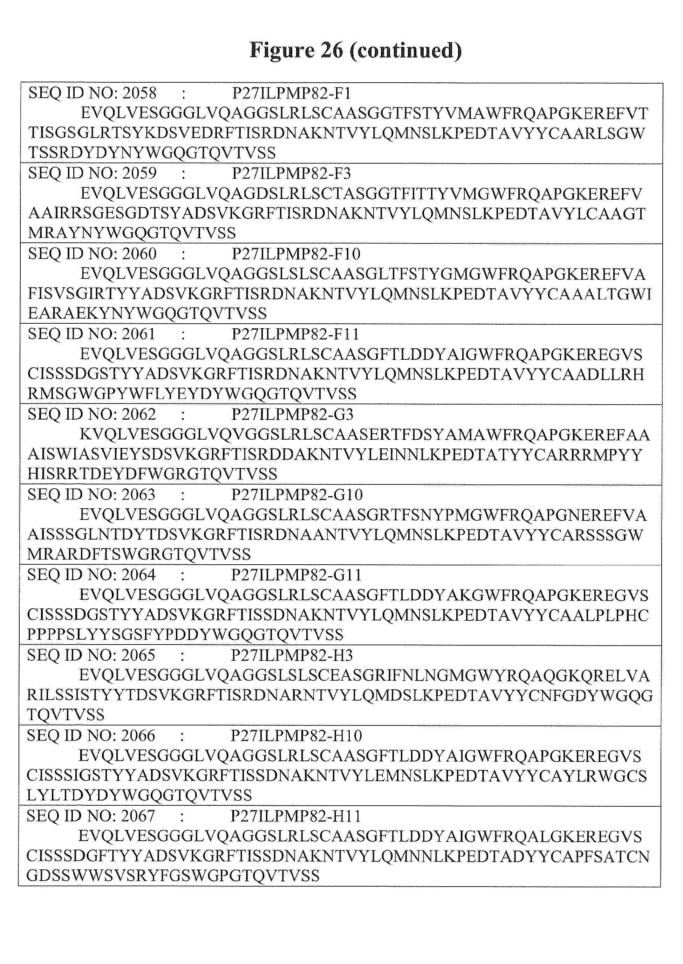

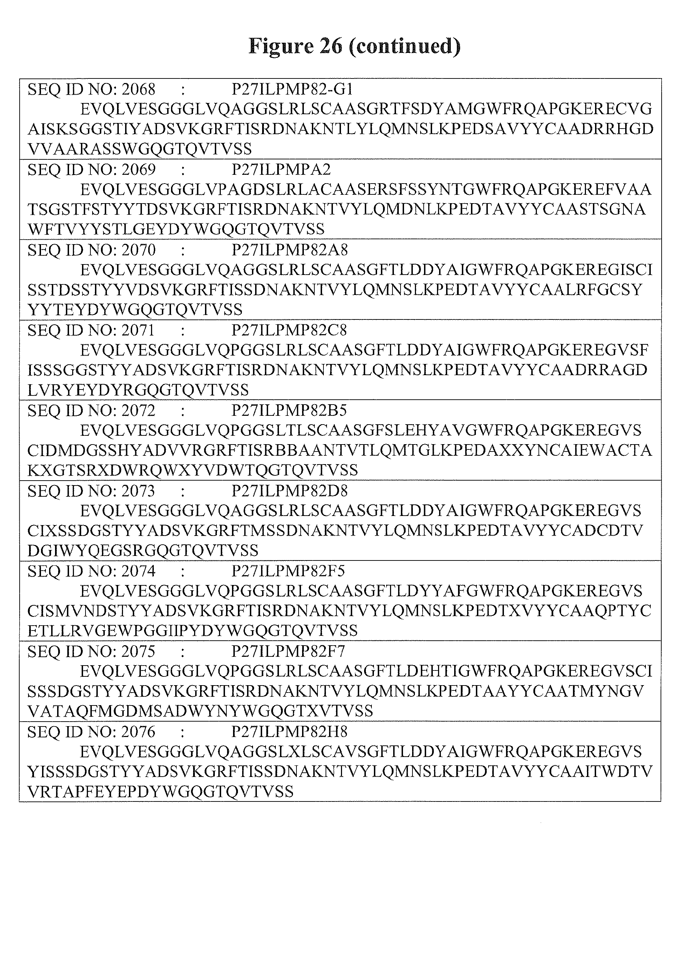

Preferably, in such amino acid sequences the CDR sequences have at least 70% amino acid identity, preferably at least 80% amino acid identity, more preferably at least 90% amino acid identity, such as 95% amino acid identity or more or even essentially 100% amino acid identity with the CDR sequences of at least one of the IL-27 sequences listed in Table A-2 and Figure 26. This degree of amino acid identity can for example be determined by determining the degree of amino acid identity (in a manner described herein) between said amino acid sequence and one or more of the sequences of SEQ ID NO's: 2038; 2039; 2040; 2041; 2042; 2043; 2044; 2045; 2046; 2047; 2048; 2049; 2050; 2051; 2052; 2053; 2054; 2055; 2056; 2057; 2058; 2059; 2060; 2061; 2062; 2063; 2064; 2065; 2066; 2067; 2068; 2069; 2070; 2071; 2072; 2073; 2074; 2075 and/or 2076 (see Table A-2 and Figure 26), in which the amino acid residues that form the framework regions are disregarded. Also, such amino acid sequences of the invention can be as further described herein.

Also, such amino acid sequences are preferably such that they can specifically bind (as defined herein) to IL-27 (i.e. to the EBI3 subunit or the p28 subunit); and more in particular bind to IL-27 with an affinity (suitably measured and/or expressed as a Two-value (actual or apparent), a K_{A}-value (actual or apparent), a kₒₙ-rate and/or a k_{off}-rate, or alternatively as an IC₅₀ value (all as further) described herein) that is as defined herein.

When the amino acid sequence of the invention essentially consists of 4 framework regions (FR1 to FR4, respectively) and 3 complementarity determining regions (CDR1 to CDR3, respectively), the amino acid sequence of the invention is preferably such that:
- CDR1 is chosen from the group consisting of:
   a) the amino acid sequences from the "CDR1 Sequences Group 37";
   b) amino acid sequences that have at least 80% amino acid identity with at least one of the amino acid sequences from the "CDR1 Sequences Group 37";
   c) amino acid sequences that have 3, 2, or 1 amino acid difference with at least one of the amino acid sequences from the "CDR1 Sequences Group 37";
   and/or
- CDR2 is chosen from the group consisting of:
   d) the amino acid sequences from the "CDR2 Sequences Group 39";
   e) amino acid sequences that have at least 80% amino acid identity with at least one of the amino acid sequences from the "CDR2 Sequences Group 39";
   f) amino acid sequences that have 3, 2, or 1 amino acid difference with at least one of the amino acid sequences from the "CDR2 Sequences Group 39";
   and/or
- CDR3 is chosen from the group consisting of:
   g) the amino acid sequences from the "CDR3 Sequences Group 41";
   h) amino acid sequences that have at least 80% amino acid identity with at least one of the amino acid sequences from the "CDR3 Sequences Group 41";
   i) amino acid sequences that have 3,2, or 1 amino acid difference with at least one of the amino acid sequences from the "CDR3 Sequences Group 41".

In particular, such an amino acid sequence of the invention may be such that CDR1 is chosen from the group consisting of the amino acid sequences from the "CDR1 Sequences Group 37"; and/or CDR2 is chosen from the group consisting of the amino acid sequences from the "CDR2 Sequences Group 39"; and/or CDR3 is chosen from the group consisting of the amino acid sequences from the "CDR3 Sequences Group 41".

In particular, when the amino acid sequence of the invention essentially consists of 4 framework regions (FR1 to FR4, respectively) and 3 complementarity determining regions (CDR1 to CDR3, respectively), the amino acid sequence of the invention is preferably such that:
- CDR1 is chosen from the group consisting of:
   a) the amino acid sequences from the "CDR1 Sequences Group 37";
   b) amino acid sequences that have at least 80% amino acid identity with at least one of the amino acid sequences from the "CDR1 Sequences Group 37'';
   c) amino acid sequences that have 3, 2, or 1 amino acid difference with at least one of the amino acid sequences from the "CDR1 Sequences Group 37";
   and
- CDR2 is chosen from the group consisting of:
   d) the amino acid sequences from the "CDR2 Sequences Group 39";
   e) amino acid sequences that have at least 80% amino acid identity with at least one of the amino acid sequences from the "CDR2 Sequences Group 39";
   f) amino acid sequences that have 3, 2, or 1 amino acid difference with at least one of the amino acid sequences from the "CDR2 Sequences Group 39";
   and
- CDR3 is chosen from the group consisting of:
   g) the amino acid sequences from the "CDR3 Sequences Group 41";
   h) amino acid sequences that have at least 80% amino acid identity with at least one of the amino acid sequences from the "CDR3 Sequences Group 41";
   i) amino acid sequences that have 3,2, or 1 amino acid difference with at least one of the amino acid sequences from the "CDR3 Sequences Group 41"; or any suitable fragment of such an amino acid sequence

In particular, such an amino acid sequence of the invention may be such that CDR1 is chosen from the group consisting of the amino acid sequences from the "CDR1 Sequences Group 37"; and CDR2 is chosen from the group consisting of the amino acid sequences from the "CDR2 Sequences Group 39"; and CDR3 is chosen from the group consisting of the amino acid sequences from the "CDR3 Sequences Group 41 ".

Again, preferred combinations of CDR sequences will become clear from the further description herein.

Also, such amino acid sequences are preferably such that they can specifically bind (as defined herein) to IL-27; and more in particular bind to IL-27 with an affinity (suitably measured and/or expressed as a K_{D}-value (actual or apparent), a K_{A}-value (actual or apparent), a kₒₙ-rate and/or a k_{off}-rate, or alternatively as an IC₅₀ value (all as further) described herein) that is as defined herein.

In one preferred, but non-limiting aspect, the invention relates to an amino acid sequence that essentially consists of 4 framework regions (FR1 to FR4, respectively) and 3 complementarity determining regions (CDR1 to CDR3, respectively), in which the CDR sequences of said amino acid sequence have at least 70% amino acid identity, preferably at least 80% amino acid identity, more preferably at least 90% amino acid identity, such as 95% amino acid identity or more or even essentially 100% amino acid identity with the CDR sequences of at least one of the amino acid sequences of SEQ ID NO's: 2038; 2039; 2040; 2041; 2042; 2043; 2044; 2045; 2046; 2047; 2048; 2049; 2050; 2051; 2052; 2053; 2054; 2055; 2056; 2057; 2058; 2059; 2060; 2061; 2062; 2063; 2064; 2065; 2066; 2067; 2068; 2069; 2070; 2071; 2072; 2073; 2074; 2075 and/or 2076 (see Table A-2 and Figure 26). This degree of amino acid identity can for example be determined by determining the degree of amino acid identity (in a manner described herein) between said amino acid sequence and one or more of the sequences of SEQ ID NO's: 2038; 2039; 2040; 2041; 2042; 2043; 2044; 2045; 2046; 2047; 2048; 2049; 2050; 2051; 2052; 2053; 2054; 2055; 2056; 2057; 2058; 2059; 2060; 2061; 2062; 2063; 2064, 2065; 2066; 2067; 2068; 2069; 2070; 2071; 2072; 2073; 2074; 2075 and/or 2076 (see Table A-2 and Figure 26), in which the amino acid residues that form the framework regions are disregarded. Such amino acid sequences of the invention can be as further described herein.

Some preferred, but non-limiting examples of IL-27 sequences are the amino acid sequences of SEQ ID NO's: 2038; 2039; 2040; 2041; 2042; 2043; 2044; 2045; 2046; 2047; 2048; 2049; 2050; 2051; 2052; 2053; 2054; 2055; 2056; 2057; 2058; 2059; 2060; 2061; 2062; 2063; 2064; 2065; 2066; 2067; 2068; 2069; 2070; 2071; 2072; 2073; 2074; 2075 and/or 2076 (see Table A-2 and Figure 26). Thus, according to another preferred, but non-limiting aspect of the invention, an IL-27 sequence is an amino acid sequence that is directed against (as defined herein) IL-27 and that either:
a) has at least 70% amino acid identity, preferably at least 80% amino acid identity, more preferably at least 90% amino acid identity, such as 95% amino acid identity or more or even essentially 100% amino acid identity with at least one of the amino acid sequences of SEQ ID NO's: 2038; 2039; 2040; 2041; 2042; 2043; 2044; 2045; 2046; 2047; 2048; 2049; 2050; 2051; 2052; 2053; 2054; 2055; 2056; 2057; 2058; 2059; 2060; 2061; 2062; 2063; 2064; 2065; 2066; 2067; 2068; 2069; 2070; 2071; 2072; 2073; 2074; 2075 and/or 2076 (see Table A-2 and Figure 26);
   and/or that
b) has no more than 20, preferably no more than 10, such as 9, 8, 7, 6, 5, 4, 3, 2 or only one amino acid difference with at least one of the amino acid sequences of SEQ ID NO's: 2038; 2039; 2040; 2041; 2042; 2043; 2044; 2045; 2046; 2047; 2048; 2049; 2050; 2051; 2052; 2053; 2054; 2055; 2056; 2057; 2058; 2059; 2060; 2061; 2062; 2063; 2064; 2065; 2066; 2067; 2068; 2069; 2070; 2071; 2072; 2073; 2074; 2075 and/or 2076 (see Table A-2 and Figure 26). Preferably, such an amino acid sequence has no more than a total of 5 (such as 4, 3. 2 or only one) such amino acid differences in the CDR's and/or no more than a total of 5 (such as 4, 3. 2 or only 1) such amino acid differences in the framework sequences;
   and/or that
c) is either (i) capable of cross-blocking (as defined herein) the interaction of at least one of the amino acid sequences of SEQ ID NO's: 2038; 2039; 2040; 2041; 2042; 2043; 2044; 2045; 2046; 2047; 2048; 2049; 2050; 2051; 2052; 2053; 2054; 2055; 2056; 2057; 2058; 2059; 2060; 2061; 2062; 2063; 2064; 2065; 2066; 2067; 2068; 2069; 2070; 2071; 2072; 2073; 2074; 2075 and/or 2076 with IL-27 and/or (ii) being able to compete with (i.e. is a competitor for) the binding of at least one of the amino acid sequences of SEQ ID NO's: 2038; 2039; 2040; 2041; 2042; 2043, 2044; 2045; 2046; 2047; 2048; 2049; 2050; 2051; 2052; 2053; 2054; 2055; 2056; 2057; 2058; 2059; 2060; 2061; 2062; 2063; 2064; 2065; 2066; 2067; 2068; 2069; 2070; 2071; 2072; 2073; 2074; 2075 and/or 2076 (see Table A-2 and Figure 26) to IL-27.

In another preferred, but non-limiting aspect, an IL-27 sequence is chosen from one of the amino acid sequences of SEQ ID NO's: 2038; 2039; 2040; 2041; 2042; 2043; 2044; 2045; 2046; 2047; 2048; 2049; 2050; 2051; 2052; 2053; 2054; 2055; 2056; 2057; 2058; 2059; 2060; 2061; 2062; 2063; 2064; 2065; 2066; 2067; 2068; 2069; 2070; 2071; 2072; 2073; 2074; 2075 and/or 2076 (see Table A-2 and Figure 26).

### G) IL-12Rb1 sequences.

One specific, but non-limiting aspect relates to "IL-12Rb1 sequences'', which generally are defined herein as amino acid sequences of the invention that are directed against (as defined herein) the IL-12Rb1 subunit, as present in both the receptor for IL-12 as well as the receptor for IL-23 (and thereby, against both the receptor for IL-12 as well as IL-23).

IL-12Rb1 sequences may generally be as further described herein (for example, in terms of affinity, specificity etc. for IL-12Rb1) for amino acid sequences of the invention in general. Also, as described herein for the amino acid sequences of the invention, the IL-12Rb1 sequences are preferably such that they form or are capable of forming (optionally after suitable folding) a single antigen binding domain or antigen binding unit, and may for example be amino acid sequences that comprise an immunoglobulin fold, amino acid sequences that are comprised of four framework regions and three CDR's, and may in particular be domain antibodies, single domain antibodies, VHH's, "dAb's" or Nanobodies (all as further described herein), or suitable fragments thereof.

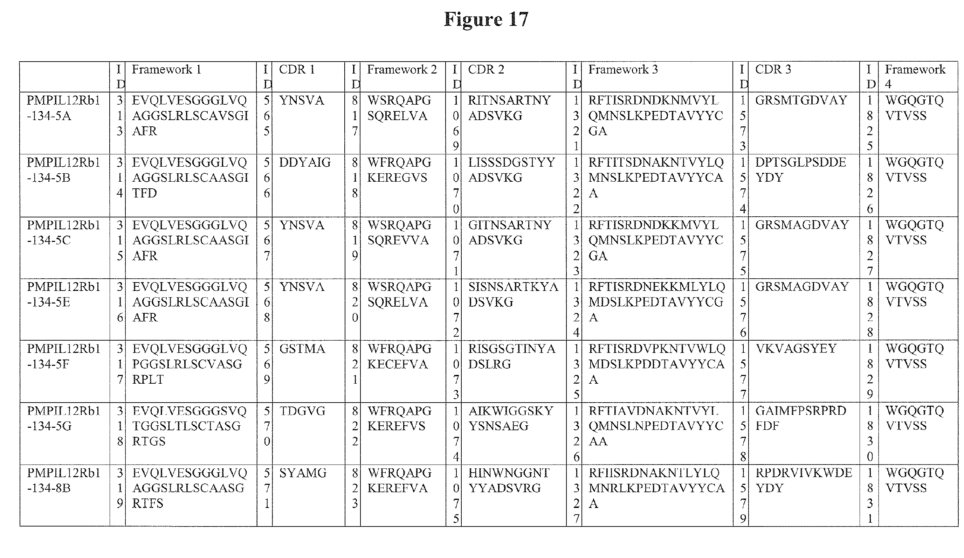

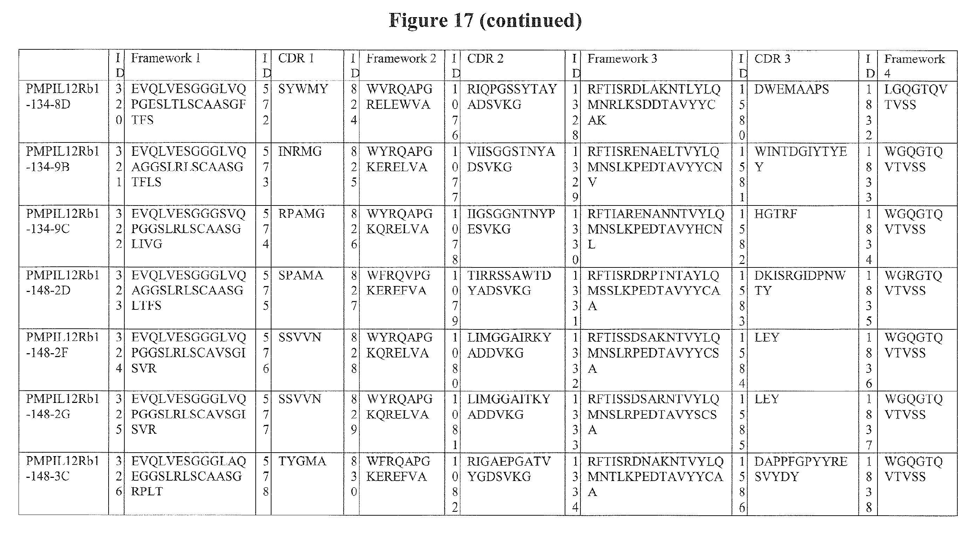

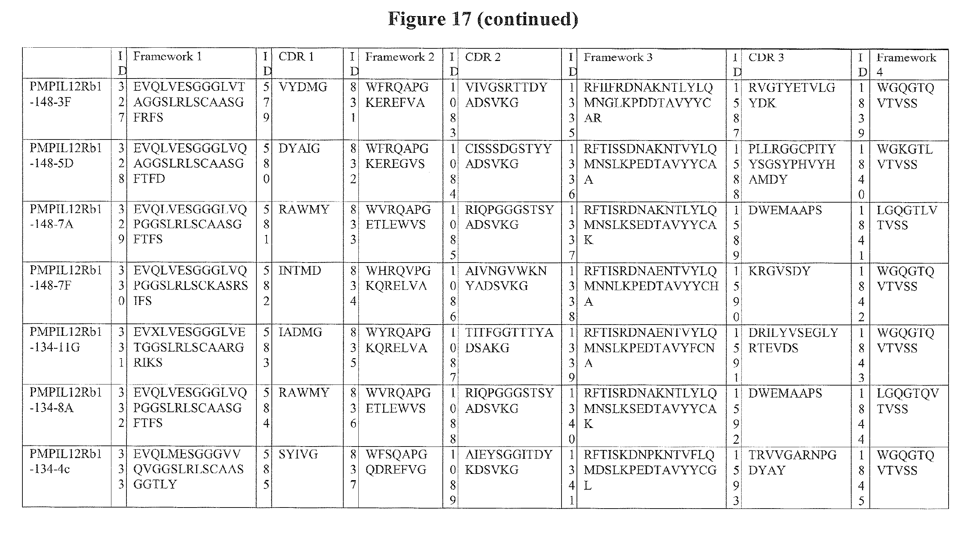

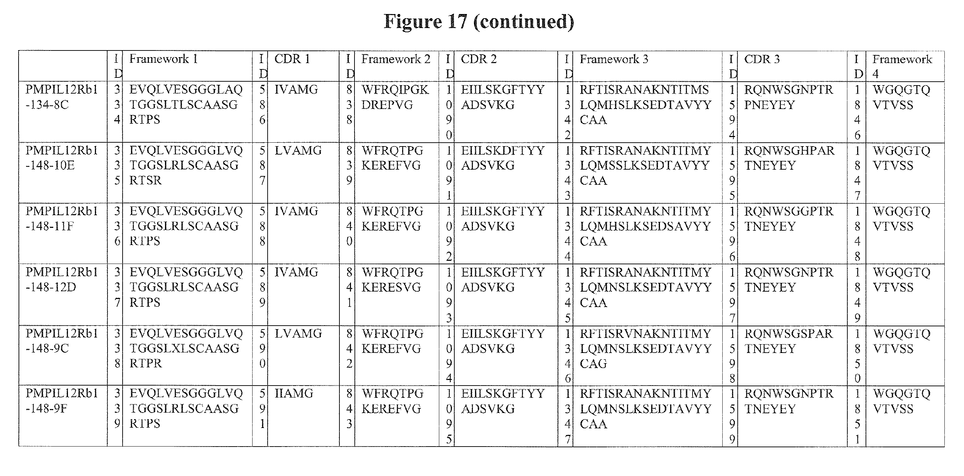

In one particular aspect, an IL-12Rb1 sequence may comprise one or more stretches of amino acid residues chosen from the group consisting of:
a) the amino acid sequences from the "CDR1 Sequences Group 44" (as defined and listed in Table A-1; see also Figure 17);
b) amino acid sequences that have at least 80% amino acid identity with at least one of the amino acid sequences from the "CDR1 Sequences Group 44";
c) amino acid sequences that have 3, 2, or 1 amino acid difference with at least one of the amino acid sequences from the "CDR1 Sequences Group 44";
d) the amino acid sequences from the "CDR2 Sequences Group 46" (as defined and listed in Table A-1; see also Figure 17);
e) amino acid sequences that have at least 80% amino acid identity with at least one of the amino acid sequences from the "CDR2 Sequences Group 46";
f) amino acid sequences that have 3, 2, or 1 amino acid difference with at least one of the amino acid sequences from the "CDR2 Sequences Group 46";
g) the amino acid sequences from the "CDR3 Sequences Group 48" (as defined and listed in Table A-1; see also Figure 17);
h) amino acid sequences that have at least 80% amino acid identity with at least one of the amino acid sequences from the "CDR3 Sequences Group 48",
i) amino acid sequences that have 3, 2, or 1 amino acid difference with at least one of the amino acid sequences from the "CDR3 Sequences Group 48";
or any suitable combination thereof.

Optionally, when an amino acid sequence of the invention contains one or more amino acid sequences according to b) and/or c), Optional Condition I, Optional Condition II and/or Optional Condition III (all as defined herein) may apply to said amino acid sequence (i.e. compared to the original amino acid sequence according to a)). Also, optionally, when an amino acid sequence of the invention contains one or more amino acid sequences according to e) and/or f), Optional Condition I, Optional Condition II and/or Optional Condition III (all as defined herein) may apply to said amino acid sequence (i.e. compared to the original amino acid sequence according to d)). Also, optionally, when an amino acid sequence of the invention contains one or more amino acid sequences according to h) and/or i), Optional Condition I, Optional Condition II and/or Optional Condition III (all as defined herein) may apply to said amino acid sequence (i.e. compared to the original amino acid sequence according to g)).

In this specific aspect, the amino acid sequence preferably comprises one or more stretches of amino acid residues chosen from the group consisting of:
a) the amino acid sequences from the "CDR1 Sequences Group 44";
b) the amino acid sequences from the "CDR2 Sequences Group 46"; and
c) the amino acid sequences from the "CDR3 Sequences Group 48";
or any suitable combination thereof.

Also, preferably, in such an amino acid sequence, at least one of said stretches of amino acid residues forms part of the antigen binding site for binding against IL-12Rb1.

In a more specific, but again non-limiting aspect, an IL-12Rb1 sequence may comprise two or more stretches of amino acid residues chosen from the group consisting of:
a) the amino acid sequences from the "CDR1 Sequences Group 44";
b) amino acid sequences that have at least 80% amino acid identity with al least one of the amino acid sequences from the "CDR1 Sequences Group 44";
c) amino acid sequences that have 3, 2, or 1 amino acid difference with at least one of the amino acid sequences from the "CDR1 Sequences Group 44";
d) the amino acid sequences from the "CDR2 Sequences Group 46";
e) amino acid sequences that have at least 80% amino acid identity with at least one of the amino acid sequences from the "CDR2 Sequences Group 46";
f) amino acid sequences that have 3, 2, or 1 amino acid difference with at least one of the amino acid sequences from the "CDR2 Sequences Group 46'';
g) the amino acid sequences from the "CDR3 Sequences Group 48";
h) amino acid sequences that have at least 80% amino acid identity with at least one of the amino acid sequences from the "CDR3 Sequences Group 48";
i) amino acid sequences that have 3, 2, or 1 amino acid difference with at least one of the amino acid sequences from the "CDR3 Sequences Group 48";
such that (i) when the first stretch of amino acid residues corresponds to one of the amino acid sequences according to a), b) or c), the second stretch of amino acid residues corresponds to one of the amino acid sequences according to d), e), f), g), h) or i); (ii) when the first stretch of amino acid residues corresponds to one of the amino acid sequences according to d), e) or f), the second stretch of amino acid residues corresponds to one of the amino acid sequences according to a), b), c), g), h) or i); or (iii) when the first stretch of amino acid residues corresponds to one of the amino acid sequences according to g), h) or i), the second stretch of amino acid residues corresponds to one of the amino acid sequences according to a), b), c), d), e) or f).

In this specific aspect, the amino acid sequence preferably comprises two or more stretches of amino acid residues chosen from the group consisting of:
a) the amino acid sequences from the "CDR1 Sequences Group 44";
b) the amino acid sequences from the "CDR2 Sequences Group 46"; and
c) the amino acid sequences from the "CDR3 Sequences Group 48";
such that, (i) when the first stretch of amino acid residues corresponds to one of the amino acid sequences from the "CDR1 Sequences Group 44", the second stretch of amino acid residues corresponds to one of the amino acid sequences from the "CDR2 Sequences Group 46" or from the "CDR3 Sequences Group 48"; (ii) when the first stretch of amino acid residues corresponds to one of the amino acid sequences from the "CDR2 Sequences Group 46", the second stretch of amino acid residues corresponds to one of the amino acid sequences from the "CDR1 Sequences Group 44" or from the "CDR3 Sequences Group 48''; or (iii) when the first stretch of amino acid residues corresponds to one of the amino acid sequences from the "CDR3 Sequences Group 48", the second stretch of amino acid residues corresponds to one of the amino acid sequences from the "CDR1 Sequences Group 44" or from the "CDR2 Sequences Group 46".

Also, in such an amino acid sequence, the at least two stretches of amino acid residues again preferably form part of the antigen binding site for binding against IL-12Rb1.

In an even more specific, but non-limiting aspect, an IL-12Rb1 sequence may comprise three or more stretches of amino acid residues, in which the first stretch of amino acid residues is chosen from the group consisting of:
a) the amino acid sequences from the "CDR1 Sequences Group 44";
b) amino acid sequences that have at least 80% amino acid identity with at least one of the amino acid sequences from the "CDR1 Sequences Group 44";
c) amino acid sequences that have 3, 2, or 1 amino acid difference with at least one of the amino acid sequences from the "CDR1 Sequences Group 44";
   the second stretch of amino acid residues is chosen from the group consisting of:
d) the amino acid sequences from the "CDR2 Sequences Group 46'';
e) amino acid sequences that have at least 80% amino acid identity with at least one of the amino acid sequences from the "CDR2 Sequences Group 46";
f) amino acid sequences that have 3, 2, or 1 amino acid difference with at least one of the amino acid sequences from the "CDR2 Sequences Group 46";
   and the third stretch of amino acid residues is chosen from the group consisting of:
g) the amino acid sequences from the "CDR3 Sequences Group 48";
h) amino acid sequences that have at least 80% amino acid identity with at least one of the amino acid sequences from the "CDR3 Sequences Group 48'';
i) amino acid sequences that have 3, 2, or 1 amino acid difference with at least one of the amino acid sequences from the "CDR3 Sequences Group 48".

Preferably, in this specifc aspect, the first stretch of amino acid residues is chosen from the group consisting of the amino acid sequences from the "CDR1 Sequences Group 44"; the second stretch of amino acid residues is chosen from the group consisting of the amino acid sequences from the "CDR2 Sequences Group 46"; and the third stretch of amino acid residues is chosen from the group consisting of the amino acid sequences from the "CDR3 Sequences Group 48".

Again, preferably, in such an amino acid sequence, the at least three stretches of amino acid residues forms part of the antigen binding site for binding against IL-12Rb1.

Preferred combinations of such stretches of amino acid sequences will become clear from the further disclosure herein.

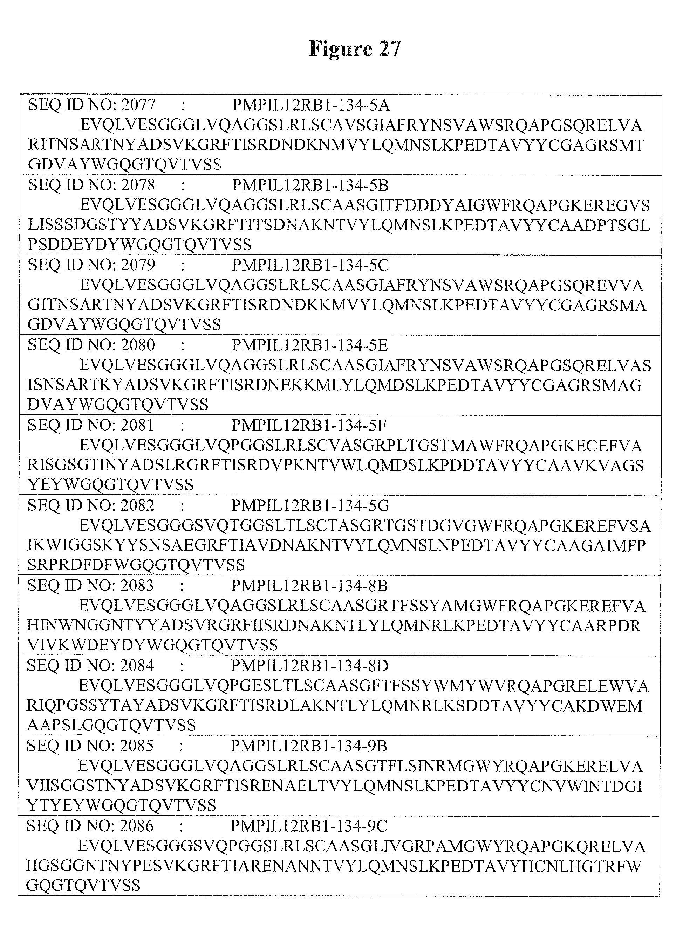

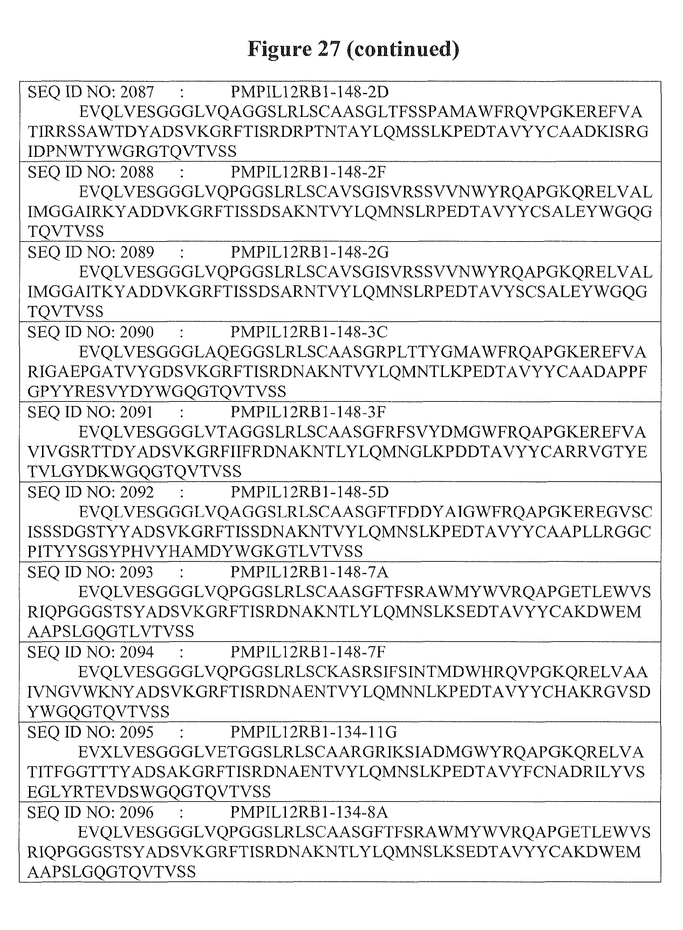

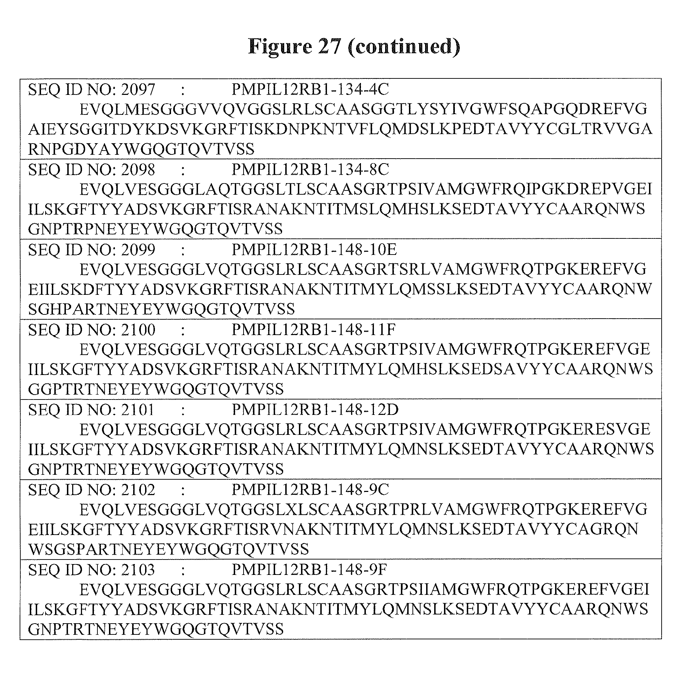

Preferably, in such amino acid sequences the CDR sequences have at least 70% amino acid identity, preferably at least 80% amino acid identity, more preferably at least 90% amino acid identity, such as 95% amino acid identity or more or even essentially 100% amino acid identity with the CDR sequences of at least one of the IL-12Rb1 sequences listed in Table A-2 and Figure 27. This degree of amino acid identity can for example be determined by determining the degree of amino acid identity (in a manner described herein) between said amino acid sequence and one or more of the sequences of SEQ ID NO's: 2077; 2078; 2079; 2080; 2081; 2082; 2083; 2084; 2085; 2086; 2087; 2088; 2089; 2090; 2091; 2092; 2093; 2094; 2095; 2096; 2097; 2098; 2099; 2100; 2101; 2102 and/or 2103 (see Table A-2 and Figure 27), in which the amino acid residues that form the framework regions are disregarded. Also, such amino acid sequences of the invention can be as further described herein.

Also, such amino acid sequences are preferably such that they can specifically bind (as defined herein) to the IL-12Rb1 subunit (i.e. as present in the receptor for IL-12 and/or in the receptor for IL-23); and more in particular bind to the IL-12Rb1 subunit with an affinity (suitably measured and/or expressed as a K_{D}-value (actual or apparent), a K_{A}-value (actual or apparent), a kₒₙ-rate and/or a k_{off}-rate, or alternatively as an IC₅₀ value (all as further) described herein) that is as defined herein.

When the amino acid sequence of the invention essentially consists of 4 framework regions (FR1 to FR4, respectively) and 3 complementarity determining regions (CDR1 to CDR3, respectively), the amino acid sequence of the invention is preferably such that:
- CbR1 is chosen from the group consisting of:
   a) the amino acid sequences from the "CDR1 Sequences Group 44";
   b) amino acid sequences that have at least 80% amino acid identity with at least one of the amino acid sequences from the "CDR1 Sequences Group 44";
   c) amino acid sequences that have 3, 2, or 1 amino acid difference with at least one of the amino acid sequences from the "CDR1 Sequences Group 44";
   and/or
- CDR2 is chosen from the group consisting of:
   d) the amino acid sequences from the "CDR2 Sequences Group 46";
   e) amino acid sequences that have at least 80% amino acid identity with at least one of the amino acid sequences from the "CDR2 Sequences Group 46";
   f) amino acid sequences that have 3, 2, or 1 amino acid difference with at least one of the amino acid sequences from the "CDR2 Sequences Group 46";
   and/or
- CDR3 is chosen from the group consisting of:
   g) the amino acid sequences from the "CDR3 Sequences Group 48";
   h) amino acid sequences that have at least 80% amino acid identity with at least one of the amino acid sequences from the "CDR3 Sequences Group 48";
   i) amino acid sequences that have 3, 2, or 1 amino acid difference with at least one of the amino acid sequences from the "CDR3 Sequences Group 48".

In particular, such an amino acid sequence of the invention may be such that CDR1 is chosen from the group consisting of the amino acid sequences from the "CDR1 Sequences Group 44"; and/or CDR2 is chosen from the group consisting of the amino acid sequences from the "CDR2 Sequences Group 46"; and/or CDR3 is chosen from the group consisting of the amino acid sequences from the "CDR3 Sequences Group 48".

In particular, when the amino acid sequence of the invention essentially consists of 4 framework, regions (FR1 to FR4, respectively) and 3 complementarity determining regions (CDR1 to CDR3, respectively), the amino acid sequence of the invention is preferably such that:
- CDR1 is chosen from the group consisting of:
   a) the amino acid sequences from the "CDR1 Sequences Group 44";
   b) amino acid sequences that have at least 80% amino acid identity with at least one of the amino acid sequences from the "CDR1 Sequences Group 44";
   c) amino acid sequences that have 3, 2, or 1 amino acid difference with at least one of the amino acid sequences from the "CDR1 Sequences Group 44'';
   and
- CDR2 is chosen from the group consisting of:
   d) the amino acid sequences from the "CDR2 Sequences Group 46";
   e) amino acid sequences that have at least 80% amino acid identity with at least one of the amino acid sequences from the "CDR2 Sequences Group 46'';
   f) amino acid sequences that have 3, 2, or 1 amino acid difference with at least one of the amino acid sequences from the "CDR2 Sequences Group 46'';
   and
- CDR3 is chosen from the group consisting of:
   g) the amino acid sequences from the "CDR3 Sequences Group 48";
   h) amino acid sequences that have at least 80% amino acid identity with at least one of the amino acid sequences from the "CDR3 Sequences Group 48'';
   i) amino acid sequences that have 3, 2, or 1 amino acid difference with at least one of the amino acid sequences from the "CDR3 Sequences Group 48''; or any suitable fragment of such an amino acid sequence

In particular, such an amino acid sequence of the invention may be such that CDR1 is chosen from the group consisting of the amino acid sequences from the "CDR1 Sequences Group 44"; and CDR2 is chosen from the group consisting of the amino acid sequences from the "CDR2 Sequences Group 46"; and CDR3 is chosen from the group consisting of the amino acid sequences from the "CDR3 Sequences Group 48",

Again, preferred combinations of CDR sequences will become clear from the further description herein.

Also, such amino acid sequences are preferably such that they can specifically bind (as defined herein) to IL-12Rb1; and more in particular bind to IL-12Rb1 with an affinity (suitably measured and/or expressed as a K_{D}-value (actual or apparent), a K_{A}-value (actual or apparent), a kₒₙ-rate and/or a k_{off}-rate, or alternatively as an IC₅₀ value (all as further) described herein) that is as defined herein.

In one preferred, but non-limiting aspect, the invention relates to an amino acid sequence that essentially consists of 4 framework regions (FR1 to FR4, respectively) and 3 complementarity determining regions (CDR1 to CDR3, respectively), in which the CDR sequences of said amino acid sequence have at least 70% amino acid identity, preferably at least 80% amino acid identity, more preferably at least 90% amino acid identity, such as 95% amino acid identity or more or even essentially 100% amino acid identity with the CDR sequences of at least one of the amino acid sequences of SEQ ID NO's: 2077; 2078; 2079; 2080; 2081; 2082; 2083; 2084; 2085; 2086; 2087; 2088; 2089; 2090; 2091; 2092; 2093; 2094; 2095; 2096; 2097; 2098; 2099; 2100; 2101; 2102 and/or 2103 (see Table A-2 and Figure 27). This degree of amino acid identity can for example be determined by determining the degree of amino acid identity (in a manner described herein) between said amino acid sequence and one or more of the sequences of SEQ ID NO's: 2077; 2078; 2079; 2080; 2081; 2082; 2083; 2084; 2085; 2086; 2087; 2088; 2089; 2090; 2091; 2092; 2093; 2094; 2095; 2096; 2097; 2098; 2099; 2100; 2101; 2102 and/or 2103 (see Table A-2 and Figure 27), in which the amino acid residues that form the framework regions are disregarded. Such amino acid sequences of the invention can be as further described herein.

Some preferred, but non-limiting examples of IL-12Rb1 sequences are the amino acid sequences of SEQ ID NO's: 2077; 2078; 2079; 2080; 2081; 2082; 2083; 2084; 2085; 2086; 2087; 2088; 2089; 2090; 2091; 2092; 2093; 2094; 2095; 2096; 2097; 2098; 2099; 2100; 2101; 2102 and/or 2103 (see Table A-2 and Figure 27). Thus, according to another preferred, but non-limiting aspect of the invention, an IL-12Rb1 sequence is an amino acid sequence that is directed against (as defined herein) IL-12Rb1 and that either:
a) has at least 70% amino acid identity, preferably at least 80% amino acid identity, more preferably at least 90% amino acid identity, such as 95% amino acid identity or more or even essentially 100% amino acid identity with at least one of the amino acid sequences of SEQ ID NO's: 2077; 2078; 2079; 2080; 2081; 2082; 2083; 2084; 2085; 2086; 2087; 2088; 2089; 2090; 2091; 2092; 2093; 2094; 2095; 2096; 2097; 2098; 2099; 2100; 2101; 2102 and/or 2103 (see Table A-2 and Figure 27);
   and/or that
b) has no more than 20, preferably no more than 10, such as 9, 8, 7, 6, 5, 4, 3, 2 or only one amino acid difference with at least one of the amino acid sequences of SEQ ID NO's: 2077; 2078; 2079; 2080; 2081; 2082; 2083; 2084; 2085; 2086; 2087; 2088; 2089; 2090; 2091; 2092; 2093; 2094; 2095; 2096; 2097; 2098; 2099; 2100; 2100; 2101; 2102 and/or 2103 (see Table A-2 and Figure 27). Preferably, such an amino acid sequence has no more than a total of 5 (such as 4, 3. 2 or only one) such amino acid differences in the CDR's and/or no more than a total of 5 (such, as 4, 3. 2 or only 1) such amino acid differences in the framework sequences;
   and/or that
c) is either (i) capable of cross-blocking (as defined herein) the interaction of at least one of the amino acid sequences of SEQ ID NO' s: 2077; 2078; 2079; 2080; 2081; 2082; 2083; 2084; 2085; 2086; 2087; 2088; 2089; 2090; 2091; 2092; 2093; 2094; 2095; 2096; 2097; 2098; 2099; 2100; 2101; 2102 and/or 2103 with the IL-12Rb1 subunit and/or (ii) being able to compete with (i.e. is a competitor for) the binding of at least one of the amino acid sequences of SEQ ID NO's: 2077; 2078; 2079; 2080; 2081; 2082; 2083; 2084; 2085; 2086; 2087; 2088; 2089; 2090; 2091; 2092; 2093; 2094; 2095; 2096; 2097; 2098; 2099; 2100; 2101; 2102 and/or 2103 (see Table A-2 and Figure 27) to IL-12Rb1.

In another preferred, but non-limiting aspect, an IL-12Rb1 sequence is chosen from one of the amino acid sequences of SEQ ID NO's: 2077; 2078; 2079; 2080; 2081; 2082; 2083; 2084; 2085; 2086; 2087; 2088; 2089; 2090; 2091; 2092; 2093; 2094; 2095; 2096; 2097; 2098; 2099; 2100; 2101; 2102 and/or 2103 (see Table A-2 and Figure 27),

### H) IL-12Rb2 sequences.

One specific, but non-limiting aspect relates to "IL-12Rb2 sequences'', which generally are defined herein as amino acid sequences of the invention that are directed against (as defined herein) the IL-12Rb2 subunit, for example as present in the receptor for IL-12 (and thereby against the receptor for IL-12).

IL-12Rb2 sequences may generally be as further described herein (for example, in terms of affinity, specificity etc. for IL-12Rb2) for amino acid sequences of the invention in general. Also, as described herein for the amino acid sequences of the invention, the IL-12Rb2 sequences are preferably such that they form or are capable of forming (optionally after suitable folding) a single antigen binding domain or antigen binding unit, and may for example be amino acid sequences that comprise an immunoglobulin fold, amino acid sequences that are comprised of four framework regions and three CDR's, and may in particular be domain antibodies, single domain antibodies, VHH's, "dAb's" or Nanobodies (all as further described herein), or suitable fragments thereof.

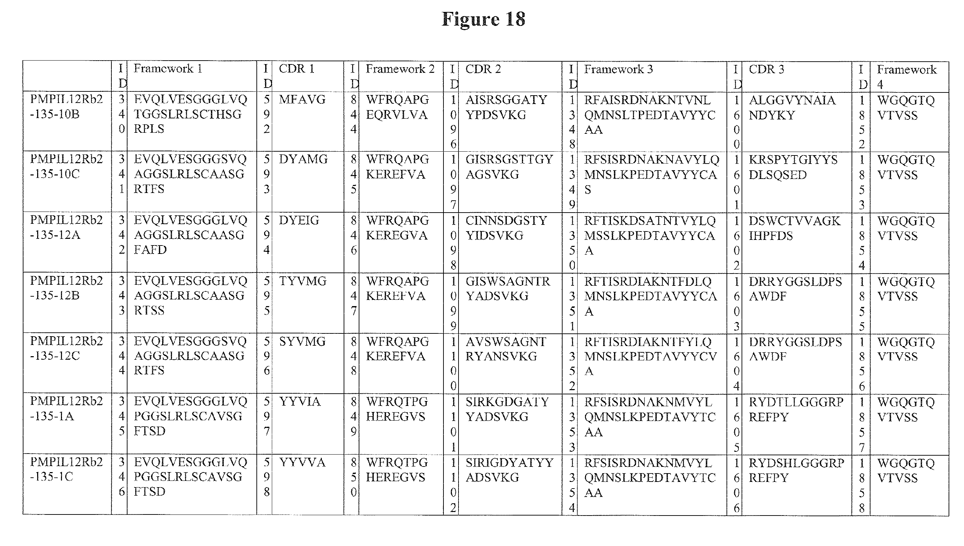

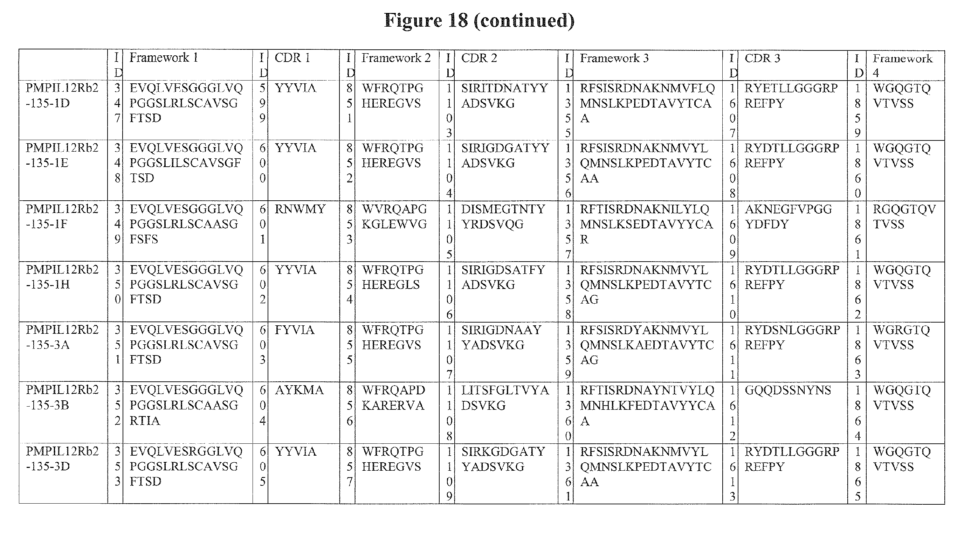

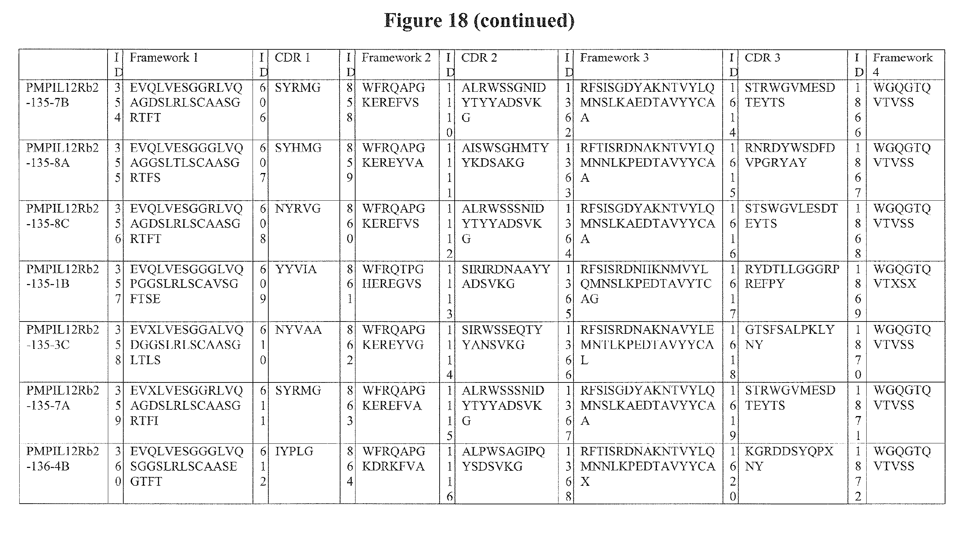

In one particular aspect, an IL-12Rb2 sequence may comprise one or more stretches of amino acid residues chosen from the group consisting of:
a) the amino acid sequences from the "CDR1 Sequences Group 51" (as defined and listed in Table A-1; see also Figure 18);
b) amino acid sequences that have at least 80% amino acid identity with at least one of the amino acid sequences from the "CDR1 Sequences Group 51";
c) amino acid sequences that have 3. 2, or 1 amino acid difference with at least one of the amino acid sequences from the "CDR1 Sequences Group 51";
d) the amino acid sequences from the "CDR2 Sequences Group 53" (as defined and listed in Table A-1; see also Figure 18);
e) amino acid sequences that have at least 80% amino acid identity with at least one of the amino acid sequences from the "CDR2 Sequences Group 53";
f) amino acid sequences that have 3, 2, or 1 amino acid difference with at least one of the amino acid sequences from the "CDR2 Sequences Group 53";
g) the amino acid sequences from the "CDR3 Sequences Group 55" (as defined and listed in Table A-1; see also Figure 18);
h) amino acid sequences that have at least 80% amino acid identity with at least one of the amino acid sequences from the "CDR3 Sequences Group 55";
i) amino acid sequences that have 3, 2, or 1 amino acid difference with at least one of the amino acid sequences from the "CDR3 Sequences Group 55";
or any suitable combination thereof.

Optionally, when an amino acid sequence of the invention contains one or more amino acid sequences according to b) and/or c), Optional Condition I, Optional Condition II. and/or Optional Condition III (all as defined herein) may apply to said amino acid sequence (i.e. compared to the original amino acid sequence according to a)), Also, optionally, when an amino acid sequence of the invention contains one or more amino acid sequences according to e) and/or f), Optional Condition I, Optional Condition II and/or Optional Condition III (all as defined herein) may apply to said amino acid sequence (i.e. compared to the original amino acid sequence according to d)). Also, optionally, when an amino acid sequence of the invention contains one or more amino acid sequences according to h) and/or i), Optional Condition I, Optional Condition II and/or Optional Condition III (all as defined herein) may apply to said amino acid sequence (i.e. compared to the original amino acid sequence according to g)).

In this specific aspect, the amino acid sequence preferably comprises one or more stretches of amino acid residues chosen from the group consisting of:
a) the amino acid sequences from the "CDR1 Sequences Group 51";
b) the amino acid sequences from the "CDR2 Sequences Group 53"; and
c) the amino acid sequences from the "CDR3 Sequences Group 55";
or any suitable combination thereof.

Also, preferably, in such an amino acid sequence, at least one of said stretches of amino acid residues forms part of the antigen binding site for binding against IL-12Rb2.

In a more specific, but again non-limiting aspect, an IL-12Rb2 sequence may comprise two or more stretches of amino acid residues chosen from the group consisting of:
a) the amino acid sequences from the "CDR1 Sequences Group 51";
b) amino acid sequences that have at least 80% amino acid identity with at least one of the amino acid sequences from the "CDR1 Sequences Group 51";
c) amino acid sequences that have 3, 2, or 1 amino acid difference with at least one of the amino acid sequences from the "CDR1 Sequences Group 51";
d) the amino acid sequences from the "CDR2 Sequences Group 53";
e) amino acid sequences that have at least 80% amino acid identity with at least one of the amino acid sequences from the "CDR2 Sequences Group 53";
f) amino acid sequences that have 3, 2, or 1 amino acid difference with at least one of the amino acid sequences from the "CDR2 Sequences Group 53'';
g) the amino acid sequences from the "CDR3 Sequences Group 55";
h) amino acid sequences that have at least 80% amino acid identity with at least one of the amino acid sequences from the "CDR3 Sequences Group 55";
i) amino acid sequences that have 3, 2, or 1 amino acid difference with at least one of the amino acid sequences from the "CDR3 Sequences Group 55";
such that (i) when the first stretch of amino acid residues corresponds to one of the amino acid sequences according to a), b) or c), the second stretch of amino acid residues corresponds to one of the amino acid sequences according to d), e), f), g), h) or i); (ii) when the first stretch of amino acid residues corresponds to one of the amino acid sequences according to d), e) or f), the second stretch of amino acid residues corresponds to one of the amino acid sequences according to a), b), c), g), h) or i); or (iii) when the first stretch of amino acid residues corresponds to one of the amino acid sequences according to g), h) or i), the second stretch of amino acid residues corresponds to one of the amino acid sequences according to a), b), c), d), e) or f).

In this specific aspect, the amino acid sequence preferably comprises two or more stretches of amino acid residues chosen from the group consisting of:
a) the amino acid sequences from the "CDR1 Sequences Group 51'';
b) the amino acid sequences from the "CDR2 Sequences Group 53"; and
c) the amino acid sequences from the "CDR3 Sequences Group 55";
such that, (i) when the first stretch of amino acid residues corresponds to one of the amino acid sequences from the "CDR1 Sequences Group 51", the second stretch of amino acid residues corresponds to one of the amino acid sequences from the "CDR2 Sequences Group 53" or from the "CDR3 Sequences Group 55"; (ii) when the first stretch of amino acid residues corresponds to one of the amino acid sequences from the "CDR2 Sequences Group 53", the second stretch of amino acid residues corresponds to one of the amino acid sequences from the "CDR1 Sequences Group 51" or from the "CDR3 Sequences Group 55"; or (iii) when the first stretch of amino acid residues corresponds to one of the amino acid sequences from the "CDR3 Sequences Group 55", the second stretch of amino acid residues corresponds to one of the amino acid sequences from the "CDR1 Sequences Group 51" or from the "CDR2 Sequences Group 53".

Also, in such an amino acid sequence, the at least two stretches of amino acid residues again preferably form part of the antigen binding site for binding against IL-12Rb2.

In an even more specific, but non-limiting aspect, an IL-12Rb2 sequence may comprise three or more stretches of amino acid residues, in which the first stretch of amino acid residues is chosen from the group consisting of:
a) the amino acid sequences from the "CDR1 Sequences Group 51";
b) amino acid sequences that have at least 80% amino acid identity with at least one of the amino acid sequences from the "CDR1 Sequences Group 51";
c) amino acid sequences that have 3, 2, or 1 amino acid difference with at least one of the amino acid sequences from the "CDR1 Sequences Group 51";
   the second stretch of amino acid residues is chosen from the group consisting of:
d) the amino acid sequences from the "CDR2 Sequences Group 53'';
e) amino acid sequences that have at least 80% amino acid identity with at least one of the amino acid sequences from the "CDR2 Sequences Group 53'';
f) amino acid sequences that have 3, 2, or 1 amino acid difference with at least one of the amino acid sequences from the "CDR2 Sequences Group 53'';
   and the third stretch of amino acid residues is chosen from the group consisting of:
g) the amino acid sequences from the "CDR3 Sequences Group 55";
h) amino acid sequences that have at least 80% amino acid identity with at least one of the amino acid sequences from the "CDR3 Sequences Group 55";
i) amino acid sequences that have 3, 2, or 1 amino acid difference with at least one of the amino acid sequences from the "CDR3 Sequences Group 55",

Preferably, in this specifc aspect, the first stretch of amino acid residues is chosen from the group consisting of the amino acid sequences from the "CDR1 Sequences Group 51"; the second stretch of amino acid residues is chosen from the group consisting of the amino acid sequences from the "CDR2 Sequences Group 53''; and the third stretch of amino acid residues is chosen from the group consisting of the amino acid sequences from the "CDR3 Sequences Group 55".

Again, preferably, in such an amino acid sequence, the at least three stretches of amino acid residues forms part of the antigen binding site for binding against IL-12Rb2.

Preferred combinations of such stretches of amino acid sequences will become clear from the further disclosure herein.

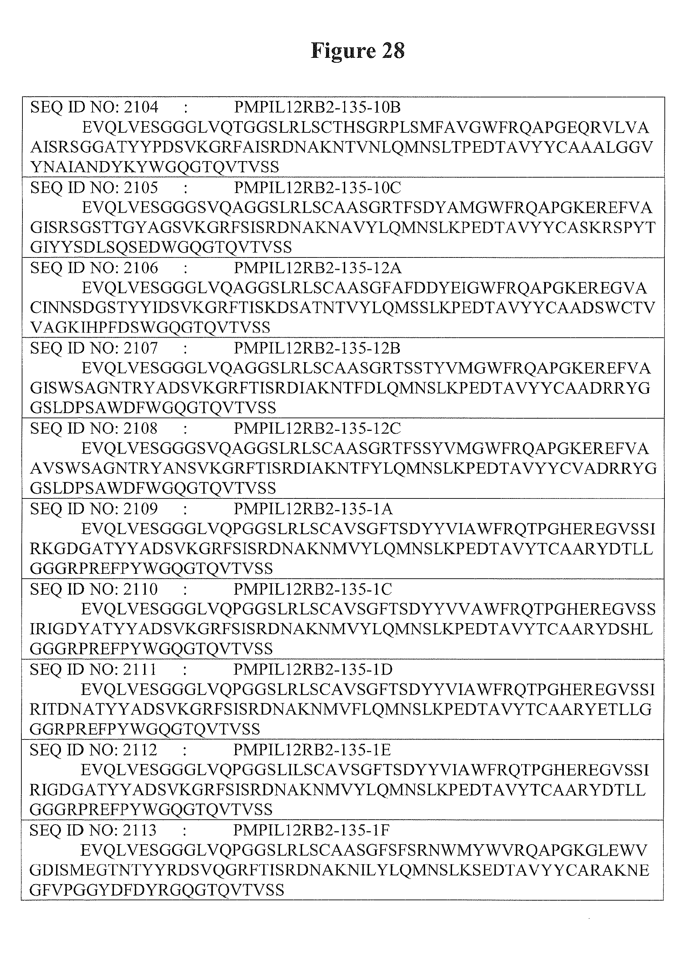

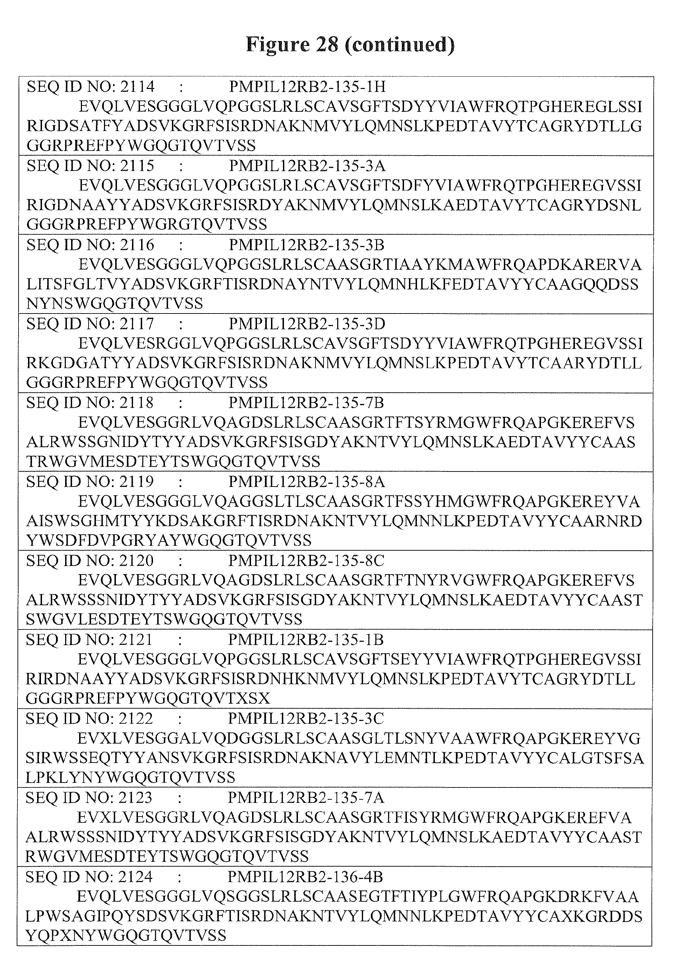

Preferably, in such amino acid sequences the CDR sequences have at least 70% amino acid identity, preferably at least 80% amino acid identity, more preferably at least 90% amino acid identity, such as 95% amino acid identity or more or even essentially 100% amino acid identity with the CDR sequences of at least one of the IL-12Rb2 sequences listed in Table A-2 and Figure 28. This degree of amino acid identity can for example be determined by determining the degree of amino acid identity (in a manner described herein) between said amino acid sequence and one or more of the sequences of SEQ ID NO's: 2104; 2105; 2106; 2107; 2108; 2109; 2110; 2111; 2112; 2113; 2114; 2115; 2116 ;2117; 2118; 2119; 2120; 2121; 2122; 2123 and/or 2124 (see Table A-2 and Figure 28), in which the amino acid residues that form the framework regions are disregarded. Also, such amino acid sequences of the invention can be as further described herein.

Also, such amino acid sequences are preferably such that they can specifically bind (as defined herein) to the IL-12Rb2 subunit (i.e. as present in the receptor for IL-12); and more in particular bind to the IL-12Rb2 subunit with an affinity (suitably measured and/or expressed as a K_{D}-value (actual or apparent), a K_{A}-value (actual or apparent), a kₒₙ-rate and/or a k_{off}-rate, or alternatively as an IC₅₀ value (all as further) described herein) that is as defined herein.

When the amino acid sequence of the invention essentially consists of 4 framework regions (FR1 to FR4, respectively) and 3 complementarity determining regions (CDR1 to CDR3, respectively), the amino acid sequence of the invention is preferably such that:
- CDR1 is chosen from the group consisting of:
   a) the amino acid sequences from the "CDR1 Sequences Group 51";
   b) amino acid sequences that have at least 80% amino acid identity with at least one of the amino acid sequences from the "CDR1 Sequences Group 51";
   c) amino acid sequences that have 3, 2, or 1 amino acid difference with at least one of the amino acid sequences from the "CDR1 Sequences Group 51";
   and/or
- CDR2 is chosen from the group consisting of:
   d) the amino acid sequences from the "CDR2 Sequences Group 53";
   e) amino acid sequences that have at least 80% amino acid identity with at least one of the amino acid sequences from the "CDR2 Sequences Group 53";
   f) amino acid sequences that have 3, 2, or 1 amino acid difference with at least one of the amino acid sequences from the "CDR2 Sequences Group 53";
   and/or
- CDR3 is chosen from the group consisting of:
   g) the amino acid sequences from the "CDR3 Sequences Group 55";
   h) amino acid sequences that have at least 80% amino acid identity with at least one of the amino acid sequences from the "CDR3 Sequences Group 55";
   i) amino acid sequences that have 3, 2, or 1 amino acid difference with at least one of the amino acid sequences from the "CDR3 Sequences Group 55".

In particular, such an amino acid sequence of the invention may be such that CDR1 is chosen from the group consisting of the amino acid sequences from the "CDR1 Sequences Group 51"; and/or CDR2 is chosen from the group consisting of the amino acid sequences from the "CDR2 Sequences Group 53''; and/or CDR3 is chosen from the group consisting of the amino acid sequences from the "CDR3 Sequences Group 55".

In particular, when the amino acid sequence of the invention essentially consists of 4 framework regions (FR1 to FR4, respectively) and 3 complementarity determining regions (CDR1 to CDR3, respectively), the amino acid sequence of the invention is preferably such that:
- CDR1 is chosen from the group consisting of:
   a) the amino acid sequences from the "CDR1 Sequences Group 51";
   b) amino acid sequences that have at least 80% amino acid identity with at least one of the amino acid sequences from the "CDR1 Sequences Group 51";
   c) amino acid sequences that have 3, 2, or 1 amino acid difference with at least one of the amino acid sequences from the "CDR1 Sequences Group 51";
   and
- CDR2 is chosen from the group consisting of:
   d) the amino acid sequences from the "CDR2 Sequences Group 53";
   e) amino acid sequences that have at least 80% amino acid identity with at least one of the amino acid sequences from the "CDR2 Sequences Group 53";
   f) amino acid sequences that have 3. 2, or 1 amino acid difference with at least one of the amino acid sequences from the "CDR2 Sequences Group 53";
   and
- CDR3 is chosen from the group consisting of:
   g) the amino acid sequences from the "CDR3 Sequences Group 55";
   h) amino acid sequences that have at least 80% amino acid identity with at least one of the amino acid sequences from the "CDR3 Sequences Group 55";
   i) amino acid sequences that have 3,2, or 1 amino acid difference with at least one of the amino acid sequences from the "CDR3 Sequences Group 55"; or any suitable fragment of such an amino acid sequence

In particular, such an amino acid sequence of the invention may be such that CDR1 is chosen from the group consisting of the amino acid sequences from the "CDR1 Sequences Group 51"; and CDR2 is chosen from the group consisting of the amino acid sequences from the "CDR2 Sequences Group 53"; and CDR3 is chosen from the group consisting of the amino acid sequences from the "CDR3 Sequences Group 55".

Again, preferred combinations of CDR sequences will become clear from the further description herein.

Also, such amino acid sequences are preferably such that they can specifically bind (as defined herein) to IL-12Rb2; and more in particular bind to IL-12Rb2 with an affinity (suitably measured and/or expressed as a K_{D}-value (actual or apparent), a K_{A}-value (actual or apparent), a kₒₙ-rate and/or a k_{off}-rate, or alternatively as an IC₅₀ value (all as further) described herein) that is as defined herein.

In one preferred, but non-limiting aspect, the invention relates to an amino acid sequence that essentially consists of 4 framework regions (FR1 to FR4, respectively) and 3 complementarity determining regions (CDR1 to CDR3, respectively), in which the CDR sequences of said amino acid sequence have at least 70% amino acid identity, preferably at least 80% amino acid identity, more preferably at least 90% amino acid identity, such as 95% amino acid identity or more or even essentially 100% amino acid identity with the CDR sequences of at least one of the amino acid sequences of SEQ ID NO's: 2104; 2105; 2106; 2107; 2108; 2109; 2110; 2111; 2112; 2113; 2114; 2115; 2116; 2117; 2118; 2119; 2120; 2121; 2122; 2123 and/or 2124 (see Table A-2 and Figure 28). This degree of amino acid identity can for example be determined by determining the degree of amino acid identity (in a manner described herein) between said amino acid sequence and one or more of the sequences of SEQ ID NO's: 2104; 2105; 2106; 2107; 2108; 2109; 2110; 2111; 2112; 2113; 2114; 2115; 2116; 2117; 2118; 2119; 2120; 2121; 2122; 2123 and/or 2124 (see Table A-2 and Figure 28), in which the amino acid residues that form the framework regions are disregarded. Such amino acid sequences of the invention can be as further described herein.

Some preferred, but non-limiting examples of IL-12Rb2 sequences are the amino acid sequences of SEQ ID NO's: 2104; 2105; 2106; 2107; 2108; 2109; 2110; 2111; 2112; 2113; 2114; 2115; 2116; 2117; 2118; 2119; 2120; 2121; 2122; 2123 and/or 2124 (see Table A-2 and Figure 28). Thus, according to another preferred, but non-limiting aspect of the invention, an IL-12Rb2 sequence is an amino acid sequence that is directed against (as defined herein) IL-12Rb2 and that either:
a) has at least 70% amino acid identity, preferably at least 80% amino acid identity, more preferably at least 90% amino acid identity, such as 95% amino acid identity or more or even essentially 100% amino acid identity with at least one of the amino acid sequences of SEQ ID NO's: 2104; 2105; 2106; 2107; 2108; 2109; 2110; 2111; 2112; 2113; 2114; 2115; 2116; 2117; 2118; 2119; 2120; 2121; 2122; 2123 and/or 2124 (see Table A-2 and Figure 28);
   and/or that
b) has no more than 20, preferably no more than 10, such as 9, 8, 7, 6, 5, 4, 3, 2 or only one amino acid difference with at least one of the amino acid sequences of SEQ ID NO's: 2104; 2105; 2106; 2107; 2108; 2109; 2110; 2111; 2112; 2113; 2114; 2115; 2116; 2117; 2118; 2119; 2120; 2121; 2122; 2123 and/or 2124 (see Table A-2 and Figure 28). Preferably, such an amino acid sequence has no more than a total of 5 (such as 4, 3, 2 or only one) such amino acid differences in the CDR's and/or no more than a total of 5 (such as 4, 3. 2 or only 1) such amino acid differences in the framework sequences;
   and/or that
c) is either (i) capable of cross-blocking (as defined herein) the interaction of at least one of the amino acid sequences of SEQ ID NO's: 2104; 2105; 2106; 2107; 2108; 2109; 2110; 2111; 2112; 2113; 2114; 211.5; 2116; 2117; 2118; 2119; 2120; 2121; 2122; 2123 and/or 2124 with the IL-12Rb2 subunit and/or (ii) being able to complete with (i.e. is a competitor for) the binding of at least one of the amino acid sequences of SEQ ID NO's: 2104; 2105; 2106; 2107; 2108; 2109; 2110; 2111; 2112; 2113; 2114; 2115; 2116; 2117; 2118; 2119; 2120; 2121; 2122; 2123 and/or 2124 (see Table A-2 and Figure 28) to IL-12Rb2.

In another preferred, but non-limiting aspect, an IL-12Rb2 sequence is chosen from one of the amino acid sequences of SEQ ID NO's: 2104; 2105; 2106; 2107; 2108; 2109; 2110; 2111; 2112; 2113; 2114; 2115; 2116; 2117; 2118; 2119; 2120; 2121; 2122; 2123 and/or 2124 (see Table A-2 and Figure 28).

### I) IL-23R sequences.

One specific, but non-limiting aspect relates to "IL-23R sequences'', which generally are defined herein as amino acid sequences of the invention that are directed against (as defined herein) the IL-23R subunit, for example as present in the (cognate) receptor for IL-23 (and thereby against the receptor for IL-23).

IL-23R sequences may generally be as further described herein (for example, in terms of affinity, specificity etc. for IL-23R) for amino acid sequences of the invention in general. Also, as described herein for the amino acid sequences of the invention, the IL-23R sequences are preferably such that they form or are capable of forming (optionally after suitable folding) a single antigen binding domain or antigen binding unit, and may for example be amino acid sequences that comprise an immunoglobulin fold, amino acid sequences that are comprised of four framework regions and three CDR's, and may in particular be domain antibodies, single domain antibodies, VHH's, "dAb's" or Nanobodies (all as further described herein), or suitable fragments thereof.

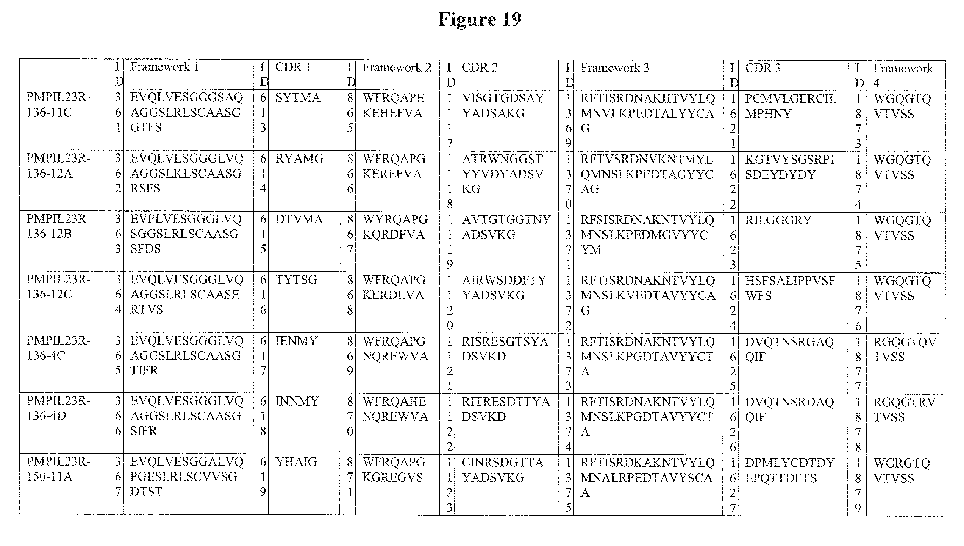

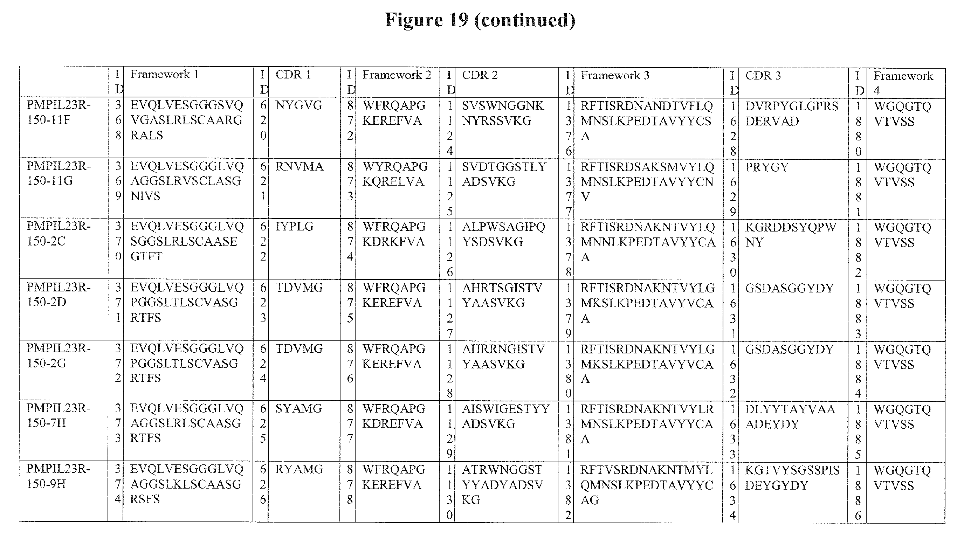

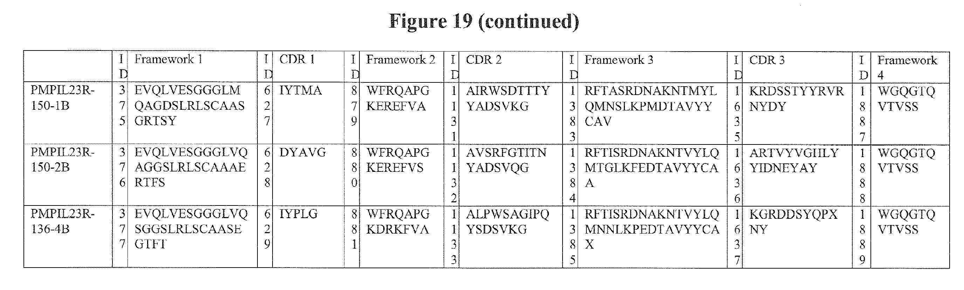

In one particular aspect, an IL-23R sequence may comprise one or more stretches of amino acid residues chosen from the group consisting of:
a) the amino acid sequences from the "CDR1 Sequences Group 58" (as defined and listed in Table A-1; see also Figure 19);
b) amino acid sequences that have at least 80% amino acid identity with at least one of the amino acid sequences from the "CDR1 Sequences Group 58'';
c) amino acid sequences that have 3, 2, or 1 amino acid difference with at least one of the amino acid sequences from the "CDR1 Sequences Group 58";
d) the amino acid sequences from the "CDR2 Sequences Group 60" (as defined and listed in Table A-1; see also Figure 19);
e) amino acid sequences that have at least 80% amino acid identity with at least one of the amino acid sequences from the "CDR2 Sequences Group 60";
f) amino acid sequences that have 3, 2, or 1 amino acid difference with at least one of the amino acid sequences from the "CDR2 Sequences Group 60";
g) the amino acid sequences from the "CDR3 Sequences Group 62" (as defined and listed in Table A-1; see also Figure 19);
h) amino acid sequences that have at least 80% amino acid identity with at least one of the amino acid sequences from the "CDR3 Sequences Group 62";
i) amino acid sequences that have 3, 2, or 1 amino acid difference with at least one of the amino acid sequences from the "CDR3 Sequences Group 62";
or any suitable combination thereof.

Optionally, when an amino acid sequence of the invention contains one or more amino acid sequences according to b) and/or c), Optional Condition I, Optional Condition II and/or Optional Condition III (all as defined herein) may apply to said amino acid sequence (i.e. compared to the original amino acid sequence according to a)). Also, optionally, when an amino acid sequence of the invention contains one or more amino acid sequences according to e) and/or f), Optional Condition I, Optional Condition II and/or Optional Condition III (all as defined herein) may apply to said amino acid sequence (i.e. compared to the original amino acid sequence according to d)). Also, optionally, when an amino acid sequence of the invention contains one or more amino acid sequences according to h) and/or i), Optional Condition I, Optional Condition II and/or Optional Condition III (all as defined herein) may apply to said amino acid sequence (i.e. compared to the original amino acid sequence according to g)).

In this specific aspect, the amino acid sequence preferably comprises one or more stretches of amino acid residues chosen from the group consisting of:
a) the amino acid sequences from the "CDR1 Sequences Group 58";
b) the amino acid sequences from the "CDR2 Sequences Group 60"; and
c) the amino acid sequences from the "CDR3 Sequences Group 62'';
or any suitable combination thereof.

Also, preferably, in such an amino acid sequence, at least one of said stretches of amino acid residues forms part of the antigen binding site for binding against IL-23R.

In a more specific, but again non-limiting aspect, an IL-23R sequence may comprise two or more stretches of amino acid residues chosen from the group consisting of:
a) the amino acid sequences from the "CDR1 Sequences Group 58";
b) amino acid sequences that have at least 80% amino acid identity with at least one of the amino acid sequences from the "CDR1 Sequences Group 58";
c) amino acid sequences that have 3, 2, or 1 amino acid difference with at least one of the amino acid sequences from the "CDR1 Sequences Group 58";
d) the amino acid sequences from the "CDR2 Sequences Group 60";
e) amino acid sequences that have at least 80% amino acid identity with at least one of the amino acid sequences from the "CDR2 Sequences Group 60";
f) amino acid sequences that have 3, 2, or 1 amino acid difference with at least one of the amino acid sequences from the "CDR2 Sequences Group 60";
g) the amino acid sequences from the "CDR3 Sequences Group 62",
h) amino acid sequences that have at least 80% amino acid identity with at least one of the amino acid sequences from the "CDR3 Sequences Group 62";
i) amino acid sequences that have 3, 2, or 1 amino acid difference with at least one of the amino acid sequences from the "CDR3 Sequences Group 62";
such that (i) when the first stretch of amino acid residues corresponds to one of the amino acid sequences according to a), b) or c), the second stretch of amino acid residues corresponds to one of the amino acid sequences according to d), e), f), g), h) or i); (ii) when the first stretch of amino acid residues corresponds to one of the amino acid sequences according to d), e) or f), the second stretch of amino acid residues corresponds to one of the amino acid sequences according to a), b), c), g), h) or i); or (iii) when the first stretch of amino acid residues corresponds to one of the amino acid sequences according to g), h) or i), the second stretch of amino acid residues corresponds to one of the amino acid sequences according to a), b), c), d), e) or f).

In this specific aspect, the amino acid sequence preferably comprises two or more stretches of amino acid residues chosen from the group consisting of:
a) the amino acid sequences from the "CDR1 Sequences Group 58";
b) the amino acid sequences from the "CDR2 Sequences Group 60"; and
c) the amino acid sequences from the "CDR3 Sequences Group 62";
such that, (i) when the first stretch of amino acid residues corresponds to one of the amino acid sequences from the "CDR1 Sequences Group 58", the second stretch of amino acid residues corresponds to one of the amino acid sequences from the "CDR2 Sequences Group 60" or from the "CDR3 Sequences Group 62"; (ii) when the first stretch of amino acid residues corresponds to one of the amino acid sequences from the "CDR2 Sequences Group 60", the second stretch of amino acid residues corresponds to one of the amino acid sequences from the "CDR1 Sequences Group 58" or from the "CDR3 Sequences Group 62"; or (iii) when the first stretch of amino acid residues corresponds to one of the amino acid sequences from the "CDR3 Sequences Group 62", the second stretch of amino acid residues corresponds to one of the amino acid sequences from the "CDR1 Sequences Group 58" or from the "CDR2 Sequences Group 60".

Also, in such an amino acid sequence, the at least two stretches of amino acid residues again preferably form part of the antigen binding site for binding against IL-23R.

In an even more specific, but non-limiting aspect, an IL-23R sequence may comprise three or more stretches of amino acid residues, in which the first stretch of amino acid residues is chosen from the group consisting of:
a) the amino acid sequences from the "CDR1 Sequences Group 58";
b) amino acid sequences that have at least 80% amino acid identity with at least one of the amino acid sequences from the "CDR1 Sequences Group 58";
c) amino acid sequences that have 3, 2, or 1 amino acid difference with at least one of the amino acid sequences from the "CDR1 Sequences Group 58";
   the second stretch of amino acid residues is chosen from the group consisting of:
d) the amino acid sequences from the "CDR2 Sequences Group 60";
e) amino acid sequences that have at least 80% amino acid identity with at least one of the amino acid sequences from the "CDR2 Sequences Group 60";
f) amino acid sequences that have 3, 2, or 1 amino acid difference with at least one of the amino acid sequences from the "CDR2 Sequences Group 60";
   and the third stretch of amino acid residues is chosen from the group consisting of:
g) the amino acid sequences from the "CDR3 Sequences Group 62";
h) amino acid sequences that have at least 80% amino acid identity with at least one of the amino acid sequences from the "CDR3 Sequences Group 62";
i) amino acid sequences that have 3, 2, or 1 amino acid difference with at least one of the amino acid sequences from the "CDR3 Sequences Group 62".

Preferably, in this specifc aspect, the first stretch of amino acid residues is chosen from the group consisting of the amino acid sequences from the "CDR1 Sequences Group 58"; the second stretch of amino acid residues is chosen from the group consisting of the amino acid sequences from the "CDR2 Sequences Group 60"; and the third stretch of amino acid residues is chosen from the group consisting of the amino acid sequences from the "CDR3 Sequences Group 62".

Again, preferably, in such an amino acid sequence, the at least three stretches of amino acid residues forms part of the antigen binding site for binding against IL-23R.

Preferred combinations of such stretches of amino acid sequences will become clear from the further disclosure herein.

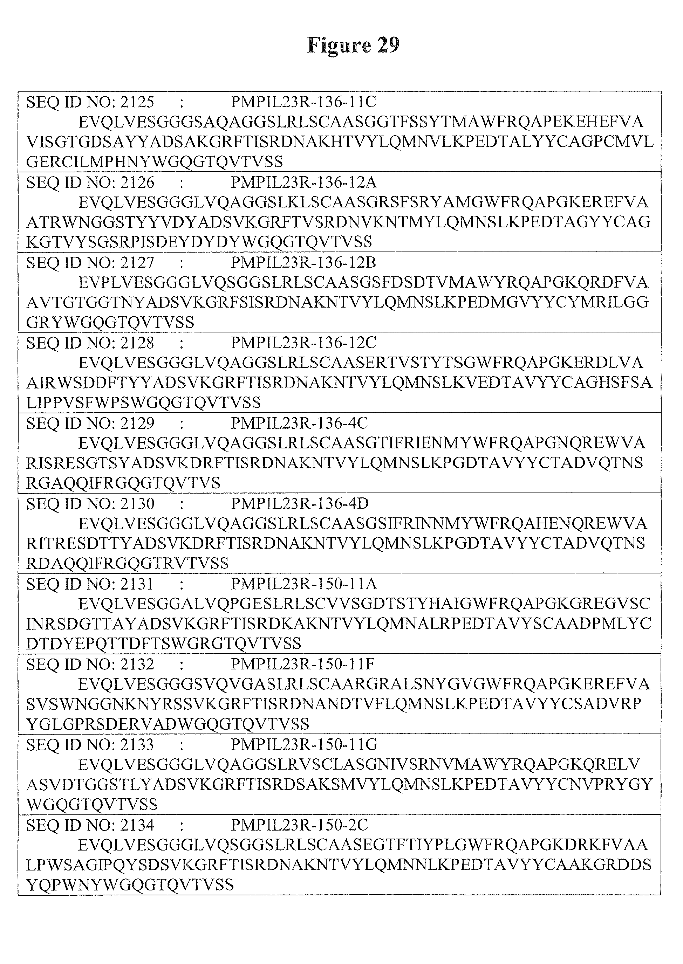

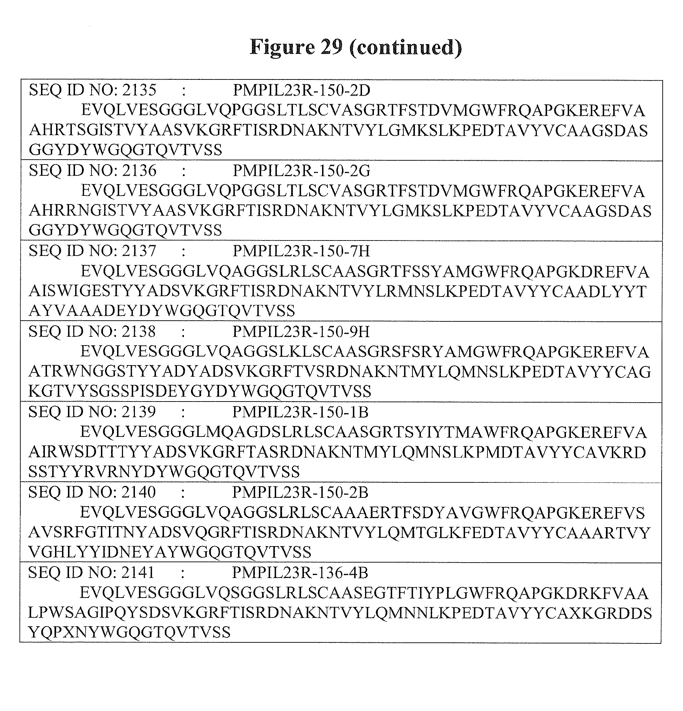

Preferably, in such amino acid sequences the CDR sequences have at least 70% amino acid identity, preferably at least 80% amino acid identity, more preferably at least 90% amino acid identity, such as 95% amino acid identity or more or even essentially 100% amino acid identity with the CDR sequences of at least one of the IL-23R sequences listed in Table A-2 and Figure 29. This degree of amino acid identity can for example be determined by determining the degree of amino acid identity (in a manner described herein) between said amino acid sequence and one or more of the sequences of SEQ ID NO's: 2125; 2126; 2127; 2128; 2129; 2130; 2131; 2132; 2133; 2134; 2135; 2136; 2137; 2138; 2139; 2140 and/or 2141 (see Table A-2 and Figure 29), in which the amino acid residues that form the framework regions are disregarded. Also, such amino acid sequences of the invention can be as further described herein.

Also, such amino acid sequences are preferably such that they can specifically bind (as defined herein) to the IL-23R subunit (i.e. as present in the receptor for IL-23): and more in particular bind to the IL-23R subunit with an affinity (suitably measured and/or expressed as a K_{D}-value (actual or apparent), a K_{A}-value (actual or apparent), a kₒₙ-rate and/or a k_{off}-rate, or alternatively as an IC₅₀ value (all as further) described herein) that is as defined herein.

When the amino acid sequence of the invention essentially consists of 4 framework regions (FR1 to FR4, respectively) and 3 complementarity determining regions (CDR1 to CDR3, respectively), the amino acid sequence of the invention is preferably such that:
- CDR1 is chosen from the group consisting of:
   a) the amino acid sequences from the "CDR1 Sequences Group 58";
   b) amino acid sequences that have at least 80% amino acid identity with at least one of the amino acid sequences from the "CDR1 Sequences Group 58";
   c) amino acid sequences that have 3, 2, or 1 amino acid difference with at least one of the amino acid sequences from the "CDR1 Sequences Group 58";
   and/or
- CDR2 is chosen from the group consisting of:
   d) the amino acid sequences from the "CDR2 Sequences Group 60";
   e) amino acid sequences that have at least 80% amino acid identity with at least one of the amino acid sequences from the "CDR2 Sequences Group 60";
   f) amino acid sequences that have 3, 2, or 1 amino acid difference with at least one of the amino acid sequences from the "CDR2 Sequences Group 60";
   and/or
- CDR3 is chosen from the group consisting of:
   g) the amino acid sequences from the "CDR3 Sequences Group 62";
   h) amino acid sequences that have at least 80% amino acid identity with at least one of the amino acid sequences from the "CDR3 Sequences Group 62";
   i) amino acid sequences that have 3, 2, or 1 amino acid difference with at least one of the amino acid sequences from the "CDR3 Sequences Group 62".

In particular, such an amino acid sequence of the invention may be such that CDR1 is chosen from the group consisting of the amino acid sequences from the "CDR1 Sequences Group 58"; and/or CDR2 is chosen from the group consisting of the amino acid sequences from the "CDR2 Sequences Group 60"; and/or CDR3 is chosen from the group consisting of the amino acid sequences from the "CDR3 Sequences Group 62".

In particular, when the amino acid sequence of the invention essentially consists of 4 framework regions (FR1 to FR.4, respectively) and 3 complementarity determining regions (CDR1 to CDR3, respectively), the amino acid sequence of the invention is preferably such that:
- CDR1 is chosen from the group consisting of:
   a) the amino acid sequences from the "CDR1 Sequences Group 58";
   b) amino acid sequences that have at least 80% amino acid identity with at least one of the amino acid sequences from the "CDR1 Sequences Group 58";
   c) amino acid sequences that have 3, 2, or 1 amino acid difference with at least one of the amino acid sequences from the "CDR1 Sequences Group 58";
   and
- CDR2 is chosen from the group consisting of:
   d) the amino acid sequences from the "CDR2 Sequences Group 60";
   e) amino acid sequences that have at least 80% amino acid identity with at least one of the amino acid sequences from the "CDR2 Sequences Group 60";
   f) amino acid sequences that have 3, 2, or 1 amino acid difference with at least one of the amino acid sequences from the "CDR2 Sequences Group 60'';
   and
- CDR3 is chosen from the group consisting of:
   g) the amino acid sequences from the "CDR3 Sequences Group 62";
   h) amino acid sequences that have at least 80% amino acid identity with at least one of the amino acid sequences from the "CDR3 Sequences Group 62";
   i) amino acid sequences that have 3, 2, or 1 amino acid difference with at least one of the amino acid sequences from the "CDR3 Sequences Group 62"; or any suitable fragment of such an amino acid sequence

In particular, such an amino acid sequence of the invention may be such that CDR1 is chosen from the group consisting of the amino acid sequences from the "CDR1 Sequences Group 58"; and CDR2 is chosen from the group consisting of the amino acid sequences from the "`CDR2 Sequences Group 60"; and CDR3 is chosen from the group consisting of the amino acid sequences from the "CDR3 Sequences Group 62".

Again, preferred combinations of CDR sequences will become clear from the further description herein.

Also, such amino acid sequences are preferably such that they can specifically bind (as defined herein) to IL-23R; and more in particular bind to IL-23R with an affinity (suitably measured and/or expressed as a K_{D}-value (actual or apparent), a K_{A}-value (actual or apparent), a kₒₙ-rate and/or a k_{off}-rate, or alternatively as an IC₅₀ value (all as further) described herein) that is as defined herein.

In one preferred, but non-limiting aspect, the invention relates to an amino acid sequence that essentially consists of 4 framework regions (FR1 to FR4, respectively) and 3 complementarity determining regions (CDR1 to CDR3, respectively), in which the CDR sequences of said amino acid sequence have at least 70% amino acid identity, preferably at least 80% amino acid identity, more preferably at least 90% amino acid identity, such as 95% amino acid identity or more or even essentially 100% amino acid identity with the CDR sequences of at least one of the amino acid sequences of SEQ ID NO's: 2125; 2126; 2127; 2128; 2129; 2130; 2131; 2132; 2133; 2134; 2135; 2136; 2137; 2138; 2139; 2140 and/or 2141 (see Table A-2 and Figure 29). This degree of amino acid identity can for example be determined by determining the degree of amino acid identity (in a manner described herein) between said amino acid sequence and one or more of the sequences of SEQ ID NO's: 2125; 2126; 2127; 2128; 2129; 2130; 2131; 2132; 2133; 2134; 2135; 2136; 2137; 2138; 2139; 2140 and/or 2141 (see Table A-2 and Figure 29), in which the amino acid residues that form the framework regions are disregarded. Such amino acid sequences of the invention can be as further described herein.

Some preferred, but non-limiting examples of IL-23R sequences are the amino acid sequences of SEQ ID NO's: 2125; 2126; 2127; 2128; 2129; 2130; 2131; 2132; 2133; 2134; 2135; 2136; 2137; 2138; 2139; 2140 and/or 2141 (see Table A-2 and Figure 29). Thus, according to another preferred, but non-limiting aspect of the invention, an IL-23R sequence is an amino acid sequence that is directed against (as defined herein) IL-23R, and that either:
a) has at least 70% amino acid identity, preferably at least 80% amino acid identity, more preferably at least 90% amino acid identity, such as 95% amino acid identity or more or even essentially 100% amino acid identity with at least one of the amino acid sequences of SEQ ID NO's: 2125; 2126; 2127; 2128; 2129; 2130; 2131; 2132; 2133; 2134; 2135; 2136; 2137; 2138; 2139; 2140 and/or 2141 (see Table A-2 and Figure 29);
   and/or that
b) has no more than 20, preferably no more than 10, such as 9, 8, 7, 6, 5,4, 3, 2 or only one amino acid difference with at least one of the amino acid sequences of SEQ ID NO's:2125; 2126; 2127; 2128; 2129; 2130; 2131; 2132; 2133; 2134; 2135; 2136; 2137; 2138; 2139; 2140 and/or 2141 (see Table A-2 and Figure 29). Preferably, such an amino acid sequence has no more than a total of 5 (such as 4, 3, 2 or only one) such amino acid differences in the CDR's and/or no more than a total of 5 (such as 4, 3. 2 or only 1) such amino acid differences in the framework sequences;
   and/or that
c) is either (i) capable of cross-blocking (as defined herein) the interaction of at least one of the amino acid sequences of SEQ ID NO's: 2125; 2126; 2127; 2128; 2129; 2130; 2131; 2132; 2133; 2134; 2135; 2136; 2137; 2138; 2139; 2140 and/or 2141 with the IL-23R subunit and/or (ii) being able to compete with (i.e. is a competitor for) the binding of at least one of the amino acid sequences of SEQ ID NO's: 2125; 2126; 2127; 2128; 2129; 2130; 2131; 2132; 2133; 2134; 2135; 2136; 2137; 2138; 2139; 2140 and/or 2141 (see Table A-2 and Figure 29) to IL-23R.

In another preferred, but non-limiting aspect, an IL-23R sequence is chosen from one of the amino acid sequences of SEQ ID NO's: 2125; 2126; 2127; 2128; 2129; 2130; 2131; 2132; 2133; 2134; 2135; 2136; 2137; 2138; 2139; 2140 and/or 2141 (see Table A-2 and Figure 29).

In some other non-limiting aspects, the invention relates to:
- an amino acid sequence, protein or polypeptide that is directed against a heterodimeric cytokine that comprises at least one p 19 subunit, which amino acid sequence, protein or polypeptide comprises or essentially consists of at least one p19+ sequence (as defined herein);
- an amino acid sequence, protein or polypeptide that is directed against a heterodimeric cytokine that comprises at least one p19 subunit, which amino acid sequence, protein or polypeptide comprises or essentially consists of at least one p19- sequence (as defined herein);
- an amino acid sequence, protein or polypeptide that is directed against a heterodimeric cytokine that comprises at least one p40 subunit, which amino acid sequence, protein or polypeptide comprises or essentially consists of at least one p40- sequence (as defined herein);
- an amino acid sequence, protein or polypeptide that is directed against a heterodimeric cytokine that comprises at least one p40 subunit, which amino acid sequence, protein or polypeptide comprises or essentially consists of at least one p40+ sequence (as defined herein);
- an amino acid sequence, protein or polypeptide that is directed against a heterodimeric cytokine that comprises at least one p35 subunit, which amino acid sequence, protein or polypeptide comprises or essentially consists of at least one p35 sequence (as defined herein);
- an amino acid sequence, protein or polypeptide that is directed against IL-23, which amino acid sequence, protein or polypeptide comprises or essentially consists of at least one p19+ sequence (as defined herein);
- an amino acid sequence, protein or polypeptide that is directed against IL-23, which amino acid sequence, protein or polypeptide comprises or essentially consists of at least one p19- sequence (as defined herein);
- an amino acid sequence, protein or polypeptide that is directed against IL-23, which amino acid sequence, protein or polypeptide comprises or essentially consists of at least one p40- sequence (as defined herein);
- an amino acid sequence, protein or polypeptide that is directed against IL-23, which amino acid sequence, protein or polypeptide comprises or essentially consists of at least one p40+ sequence (as defined herein);
- an amino acid sequence, protein or polypeptide that is directed against IL-12, which amino acid sequence, protein or polypeptide comprises or essentially consists of at least one p35 sequence (as defined herein);
- an amino acid sequence, protein or polypeptide that is directed against IL-12, which amino acid sequence, protein or polypeptide comprises or essentially consists of at least one p40- sequence (as defined herein);
- an amino acid sequence, protein or polypeptide that is directed against IL-12, which amino acid sequence, protein or polypeptide comprises or essentially consists of at least one p40+ sequence (as defined herein);
- an amino acid sequence, protein or polypeptide that is directed against IL-12 and IL-23, and that is preferably specific for (as defined herein) IL-12 and/or IL-23 compared to IL-27 and/or IL-35 , which amino acid sequence, protein or polypeptide comprises or essentially consists of at least one p40- sequence (as defined herein);
- an amino acid sequence, protein or polypeptide that is directed against IL-12 and IL-23, and that is preferably specific for (as defined herein) IL- 12 and/or IL-23 compared to IL-27 and/or IL-35 , which amino acid sequence, protein or polypeptide comprises or essentially consists of at least one p40+ sequence (as defined herein);
- an amino acid sequence, protein or polypeptide that is directed against IL-27, and that is preferably specific for (as defined herein) IL-27 compared to IL-12 and/or 1L-23, which amino acid sequence, protein or polypeptide comprises or essentially consists of at least one IL-27 sequence (as defined herein);
- an amino acid sequence, protein or polypeptide that is directed against a receptor for a heterodimeric cytokine that comprises at least one IL-12Rb1 subunit, which amino acid sequence, protein or polypeptide comprises or essentially consists of at least one 1L-12Rb1 sequence (as defined herein);
- an amino acid sequence, protein or polypeptide that is directed against a receptor for a heterodimeric cytokine that comprises at least one IL-12Rb2 subunit, which amino acid sequence, protein or polypeptide comprises or essentially consists of at least one IL-12Rb2 sequence (as defined herein);
- an amino acid sequence, protein or polypeptide that is directed against a receptor for a heterodimeric cytokine that comprises at least one IL-23R subunit, which amino acid sequence, protein or polypeptide comprises or essentially consists of at least one IL-23R sequence (as defined herein);
- an amino acid sequence, protein or polypeptide that is directed against the (cognate) receptor for IL-23, which amino acid sequence, protein or polypeptide comprises or essentially consists of at least one IL-12Rb1 sequence (as defined herein):
- an amino acid sequence, protein or polypeptide that is directed against the (cognate) receptor for IL-23, and that is preferably specific for (as defined herein) the (cognate) receptor for IL-23 compared to the (cognate) receptor for IL-12, which amino acid sequence, protein or polypeptide comprises or essentially consists of at least one IL-23R sequence (as defined herein);
- an amino acid sequence, protein or polypeptide that is directed against the (cognate) receptor for IL-12, which amino acid sequence, protein or polypeptide comprises or essentially consists of at least one IL-12Rb1 sequence (as defined herein);
- an amino acid sequence, protein or polypeptide that is directed against the (cognate) receptor for IL-12, and that is preferably specific for (as defined herein) the (cognate) receptor for IL-12 compared to the (cognate) receptor for IL-23, which amino acid sequence, protein or polypeptide comprises or essentially consists of at least one IL-12Rb2 sequence (as defined herein);
- an amino acid sequence, protein or polypeptide that is directed against the (cognate) receptor for IL-12 as well as the cognate receptor for IL-23, and that is preferably specific for (as defined herein) the (cognate) receptor for IL-12 and/or the cognate receptor for IL-27 and/or the (cognate) receptor for Il-35, which amino acid sequence, protein or polypeptide comprises or essentially consists of at least one IL-12Rb1 sequence (as defined herein),

Again, such amino acid sequences, proteins or polypeptides can be as further described herein. The invention also relates to nucleotide sequences/nucleic acids encoding the same, to preparations and formulations comprising the same, to methods for producing the same and to uses of the same, all as further described herein.

In the amino acid sequence of the invention (such as the p19+ sequences, p19-sequences, p40+ sequences, p40- sequences, p35 sequences, IL-27 sequences, IL-12Rb1 sequences, IL-12Rb2 sequences and IL-23 sequences described, herein), the framework sequences may be any suitable framework sequences, and examples of suitable framework sequences will be clear to the skilled person, for example on the basis the standard handbooks and the further disclosure and prior art mentioned herein.

The framework sequences are preferably (a suitable combination of) immunoglobulin framework sequences or framework sequences that have been derived from immunoglobulin framework sequences (for example, by humanization or camelization). For example, the framework sequences may be framework sequences derived from a light chain variable domain (e.g. a V_{L}-sequence) and/or from a heavy chain variable domain (e.g. a V_{H}-sequence), In one particularly preferred aspect, the framework sequences are either framework sequences that have been derived from a V_{HH}-sequence (in which said framework sequences may optionally have been partially or fully humanized) or are conventional V_{H} sequences that have been camelized (as defined herein),

The framework sequences are preferably such that the amino acid sequence of the invention is a domain antibody (or an amino acid sequence that is suitable for use as a domain antibody); is a single domain antibody (or an amino acid sequence that is suitable for use as a single domain antibody); is a "dAb" (or an amino acid sequence that is suitable for use as a dAb); or is a Nanobody™ (including but not limited to V_{HH} sequence). Again, suitable framework sequences will be clear to the skilled person, for example, on the basis the standard handbooks and the further disclosure and prior art mentioned herein.

In particular, the framework sequences present in the amino acid sequences of the invention may contain one or more of Hallmark residues (as defined herein), such that the amino acid sequence of the invention is a Nanobody™. Some preferred, but non-limiting examples of (suitable combinations of) such framework sequences will become clear from the further disclosure herein.

Again, as generally described herein for the amino acid sequences of the invention, it is also possible to use suitable fragments (or combinations of fragments) of any of the foregoing, such as fragments that contain one or more CDR sequences, suitably flanked by and/or linked via one or more framework sequences (for example, in the same order as these CDR's and framework sequences may occur in the full-sized immunoglobulin sequence from which the fragment has been derived). Such fragments may also again be such that they comprise or can form an immunoglobulin fold, or alternatively be such that they do not comprise or cannot form an immunoglobulin fold.

In one specific aspect, such a fragment comprises a single CDR sequence as described herein (and in particular a CDR3 sequence), that is flanked on each side by (part of) a framework sequence (and in particular, part of the framework sequence(s) that, in the immunoglobulin sequence from which the fragment is derived, are adjacent to said CDR sequence. For example, a CDR3 sequence may be preceded by (part of) a FR3 sequence and followed by (part of) a FR4 sequence). Such a fragment may also contain a disulphide bridge, and in particular a disulphide bridge that links the two framework regions that precede and follow the CDR sequence, respectively (for the purpose of forming such a disulphide bridge, cysteine residues that naturally occur in said framework regions may be used, or alternatively cysteine residues may be synthetically added to or introduced into said framework regions). For a further description of these "Expedite fragments", reference is again made to WO 03/05031, as well as to the US provisional application of Ablynx N.V. entitled *"Peptides capable of binding to serum proteins"* of Ablynx N.V. (inventors: Revets, Hilde Adi Pierrette; Kolkman, Joost Alexander; and Hoogenboom, Hendricus Renerus Jacobus Mattheus) filed on December 5, 2006 (see also PCT/EP2007/063348).

In another aspect, the invention relates to a compound or construct, and in particular a protein or polypeptide (also referred to herein as a *"compound of the invention"* or *"polypeptide of the invention"*, respectively) that comprises or essentially consists of one or more amino acid sequences of the invention (or suitable fragments thereof), and optionally further comprises one or more other groups, residues, moieties or binding units. As will become clear to the skilled person from the further disclosure herein, such further groups, residues, moieties, binding units or amino acid sequences may or may not provide further functionality to the amino acid sequence of the invention (and/or to the compound or construct in which it is present) and may or may not modify the properties of the amino acid sequence of the invention.

For example, such further groups, residues, moieties or binding units may be one or more additional amino acid sequences, such that the compound or construct is a (fusion) protein or (fusion) polypeptide. In a preferred but non-limiting aspect, said one or more other groups, residues, moieties or binding units are immunoglobulin sequences. Even more preferably, said one or more other groups, residues, moieties or binding units are chosen from the group consisting of domain antibodies, amino acid sequences that are suitable for use as a domain antibody, single domain antibodies, amino acid sequences that are suitable for use as a single domain antibody, "dAb"'s, amino acid sequences that are suitable for use as a dAb, or Nanobodies.

Alternatively, such groups, residues, moieties or binding units may for example be chemical groups, residues, moieties, which may or may not by themselves be biologically and/or pharmacologically active. For example, and without limitation, such groups may be linked to the one or more amino acid sequences of the invention so as to provide a "derivative" of an amino acid sequence or polypeptide of the invention, as further described herein.

Also within the scope of the present invention are compounds or constructs, that comprises or essentially consists of one or more derivatives as described herein, and optionally further comprises one or more other groups, residues, moieties or binding units, optionally linked via one or more linkers. Preferably, said one or more other groups, residues, moieties or binding units are amino acid sequences.

In the compounds or constructs described above, the one or more amino acid sequences of the invention and the one or more groups, residues, moieties or binding units may be linked directly to each other and/or via one or more suitable linkers or spacers. For example, when the one or more groups, residues, moieties or binding units are amino acid sequences, the linkers may also be amino acid sequences, so that the resulting compound or construct is a fusion (protein) or fusion (polypeptide),

The compounds or polypeptides of the invention can generally be prepared by a method which comprises at least one step of suitably linking the one or more amino acid sequences of the invention to the one or more further groups, residues, moieties or bending units, optionally via the one or more suitable linkers, so as to provide the compound or polypeptide of the invention. Polypeptides of the invention can also be prepared by a method which generally comprises at least the steps of providing a nucleic acid that encodes a polypeptide of the invention, expressing said nucleic acid in a suitable manner, and recovering the expressed polypeptide of the invention. Such methods can be performed in a manner known per se, which will be clear to the skilled person, for example on the basis of the methods and techniques further described herein.

The process of designing/selecting and/or preparing a compound or polypeptide of the invention, starting from an amino acid sequence of the invention, is also referred to herein as *"formatting"* said amino acid sequence of the invention; and an amino acid of the invention that is made part of a compound or polypeptide of the invention is said to be "*formatted"* or to be *"in the format of*" said compound or polypeptide of the invention. Examples of ways in which an amino acid sequence of the invention can be formatted and examples of such formats will be clear to the skilled person based on the disclosure herein; and such formatted amino acid sequences form a further aspect of the invention.

In one specific aspect of the invention, a compound of the invention or a polypeptide of the invention may have an increased half-life, compared to the corresponding amino acid sequence of the invention. Some preferred, but non-limiting examples of such compounds and polypeptides will become clear to the skilled person based on the further disclosure herein, and for example comprise amino acid sequences or polypeptides of the invention that have been chemically modified to increase the half-life thereof (for example, by means of pegylation); amino acid sequences of the invention that comprise at least one additional binding site for binding to a serum protein (such as serum albumin; see for example EP 0 368 684 B1, page 4); or polypeptides of the invention that comprise at least one amino acid sequence of the invention that is linked to at least one moiety (and in particular at least one amino acid sequence) that increases the half-life of the amino acid sequence of the invention. Examples of polypeptides of the invention that comprise such half-life extending moieties or amino acid sequences will become clear to the skilled person based on the further disclosure herein; and for example include, without limitation, polypeptides in which the one or more amino acid sequences of the invention are suitable linked to one or more serum proteins or fragments thereof (such as (human) serum albumin or suitable fragment thereof) or to one or more binding units that can bind to serum proteins (such as, for example, domain antibodies, amino acid sequences that are suitable for use as a domain antibody, single domain antibodies, amino acid sequences that are suitable for use as a single domain antibody, "dAb"'s, amino acid sequences that are suitable for use as a dAb, or Nanobodies that can bind to serum proteins such as serum albumin (such as human serum albumin), serum immunoglobulins such as IgG, or transferrine; reference is made to the further description and references mentioned herein); polypeptide in which an amino acid sequence of the invention is linked to an Fc portion (such as a human Fc) or a suitable part or fragment thereof; or polypeptide in which the one or more amino acid sequences of the invention are suitable linked to one or more small proteins or peptides that can bind to serum proteins (such as, without limitation, the proteins and peptides described in WO 91/01743, WO 01/45746, WO 02/076489 and to the US provisional application of Ablynx N.V. entitled *"Peptides capable of binding to serum proteins"* of Ablynx N.V. filed on December 5, 2006 (see also PCT/EP2007/063348 and WO 08/(068280) as well as the US provisional applications 61/050,385 and 61/045,690 of Ablynx N.V. both entitled "Improved peptides capable of binding to serum proteins"

Generally, the compounds or polypeptides of the invention with increased half-life preferably have a half-life that is at least 1.5 times, preferably at least 2 times, such as at least 5 times, for example at least 10 times or more than 20 times, greater than the half-life of the corresponding amino acid sequence of the invention per se. For example, the compounds or polypeptides of the invention with increased half-life may have a half-life that is increased with more than 1 hours, preferably more than 2 hours, more preferably more than 6 hours, such as more than 12 hours, or even more than 24, 48 or 72 hours, compared to the corresponding amino acid sequence of the invention per se.

In a preferred, but non-limiting aspect of the invention, such compounds or polypeptide of the invention have a serum half-life that is increased with more than 1 hours, preferably more than 2 hours, more preferably more than 6 hours, such as more than 12 hours, or even more than 24, 48 or 72 hours, compared to the corresponding amino acid sequence of the invention per se.

In another preferred, but non-limiting aspect of the invention, such compounds or polypeptide of the invention exhibit a serum half-life in human of at least about 12 hours, preferably at least 24 hours, more preferably at least 48 hours, even more preferably at least 72 hours or more. For example, compounds or polypeptides of the invention may have a half-life of at least 5 days (such as about 5 to 10 days), preferably at least 9 days (such as about 9 to 14 days), more preferably at least about 10 days (such as about 10 to 15 days), or at least about 11 days (such as about 11 to 16 days), more preferably at least about 12 days (such as about 12 to 18 days or more), or more than 14 days (such as about 14 to 19 days).

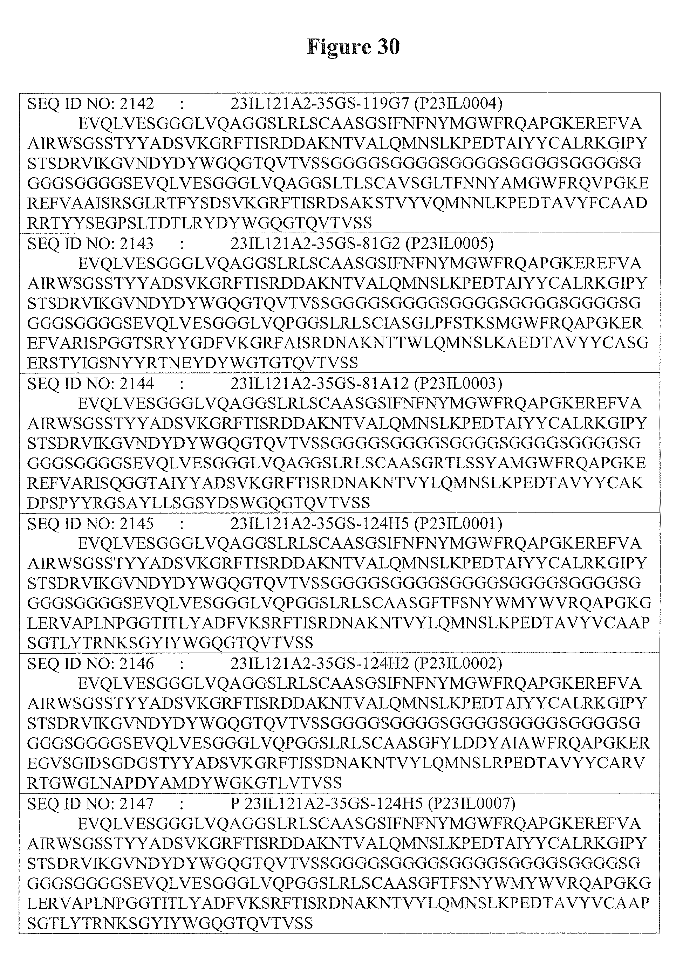

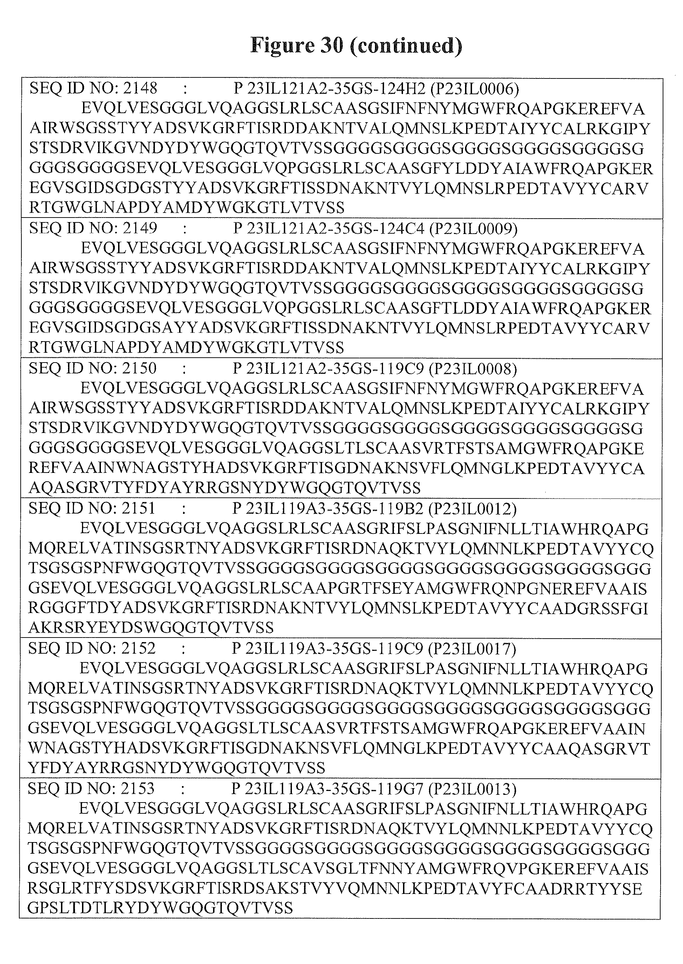

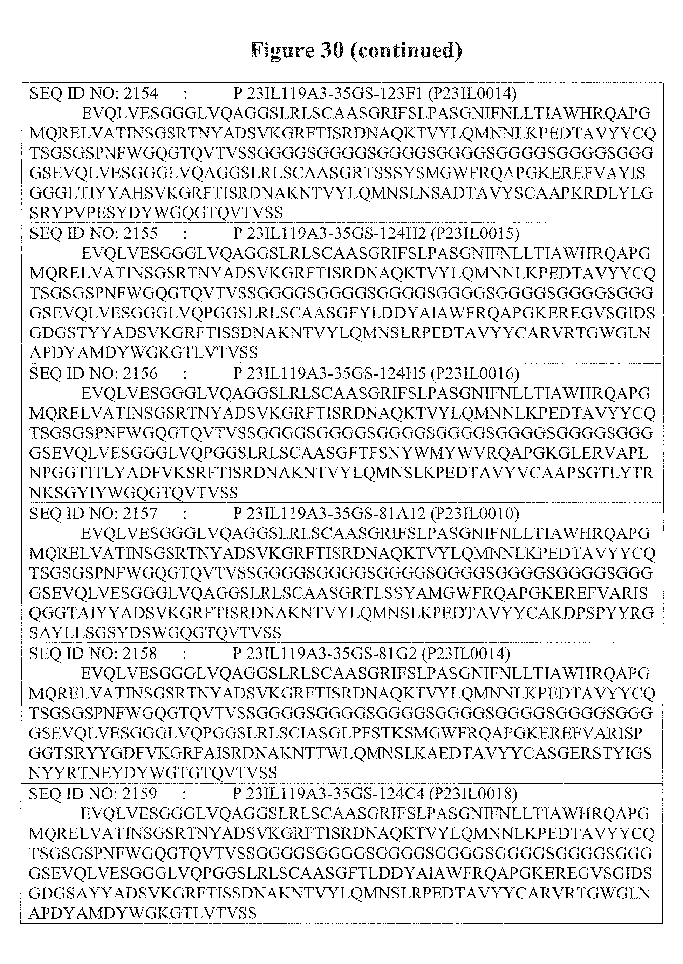

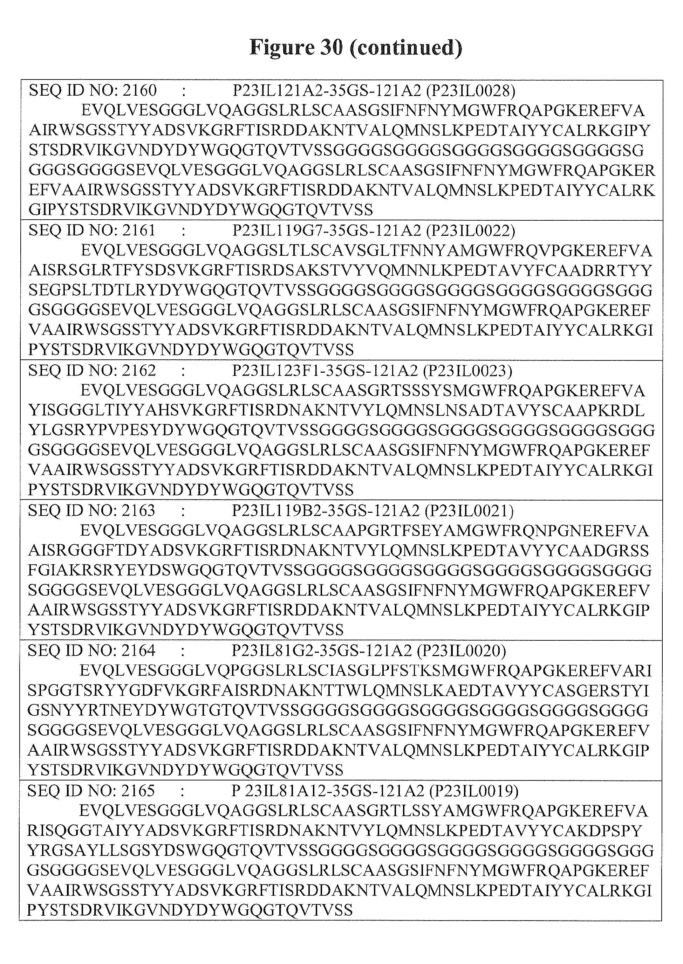

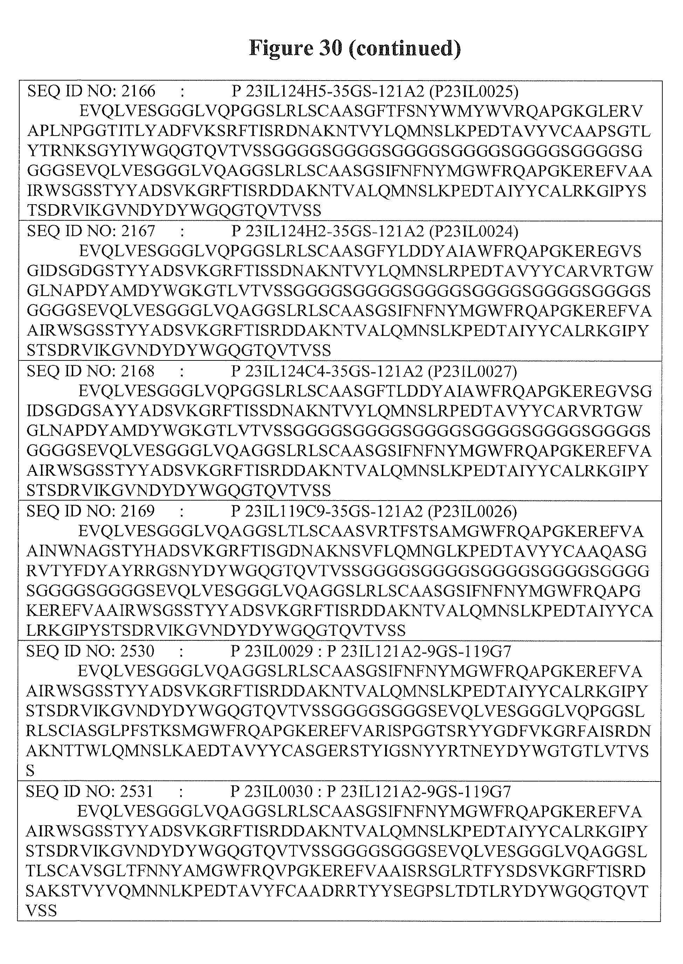

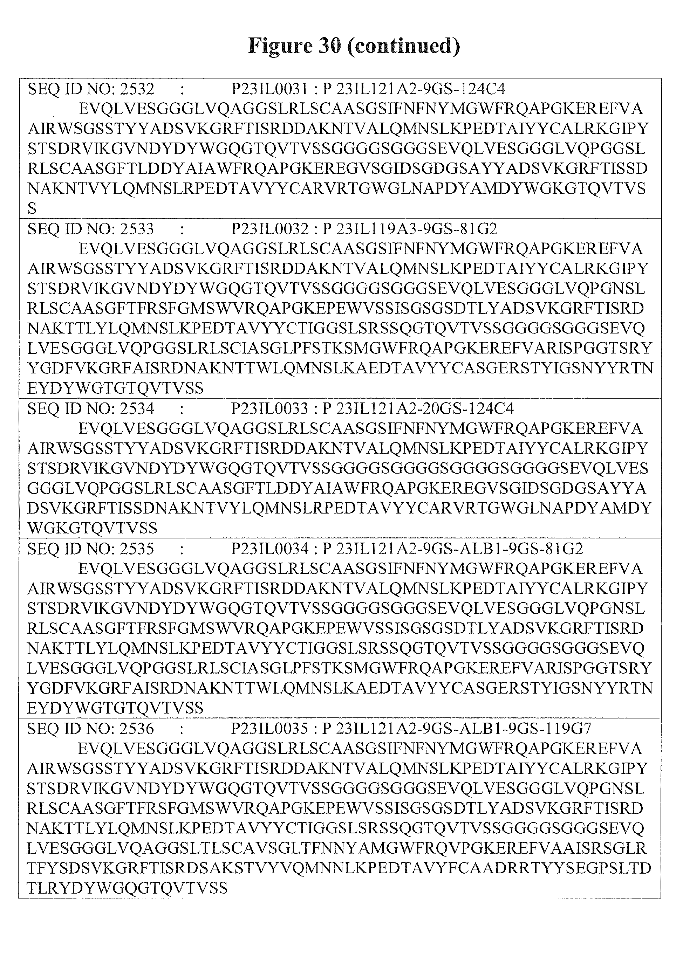

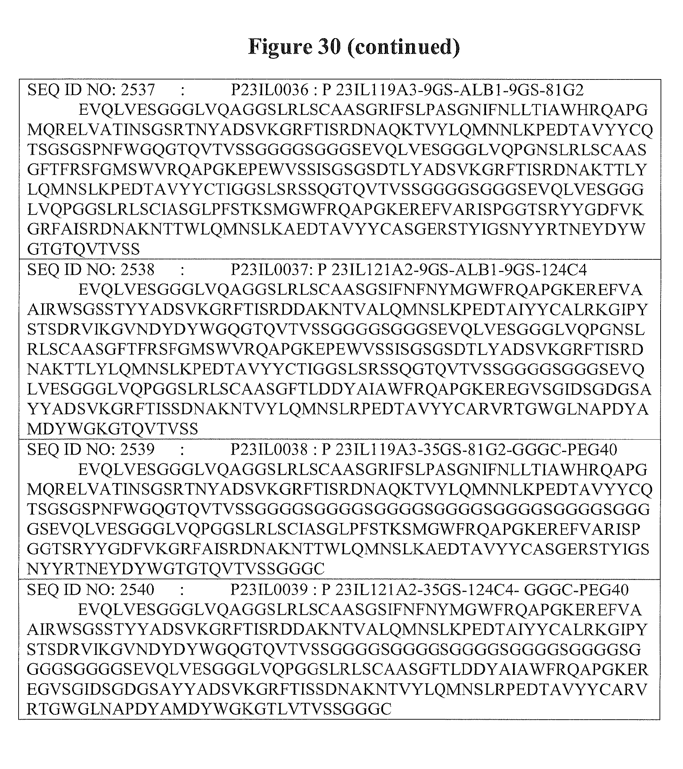

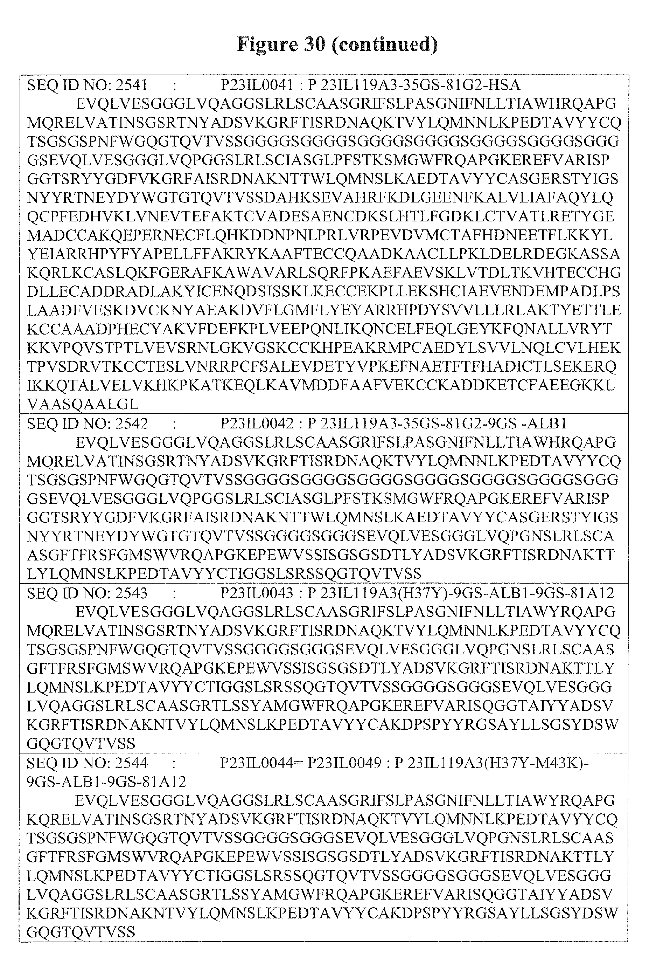

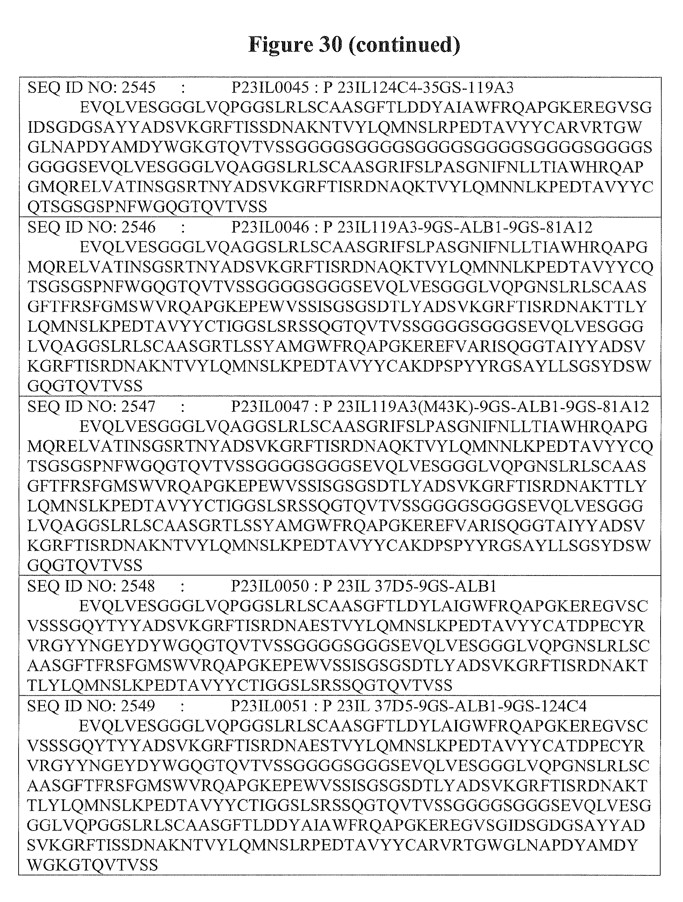

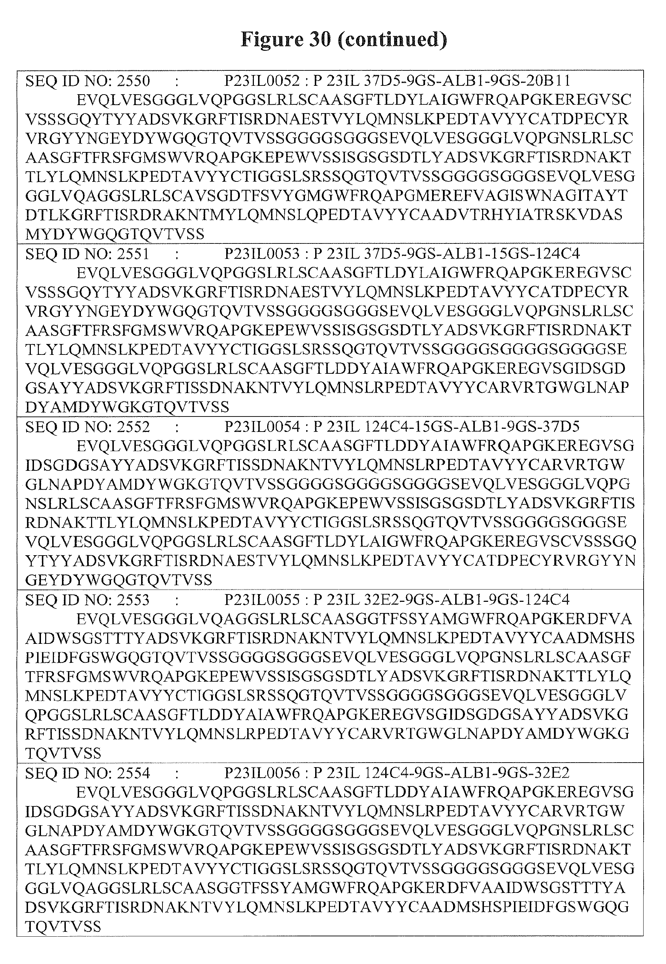

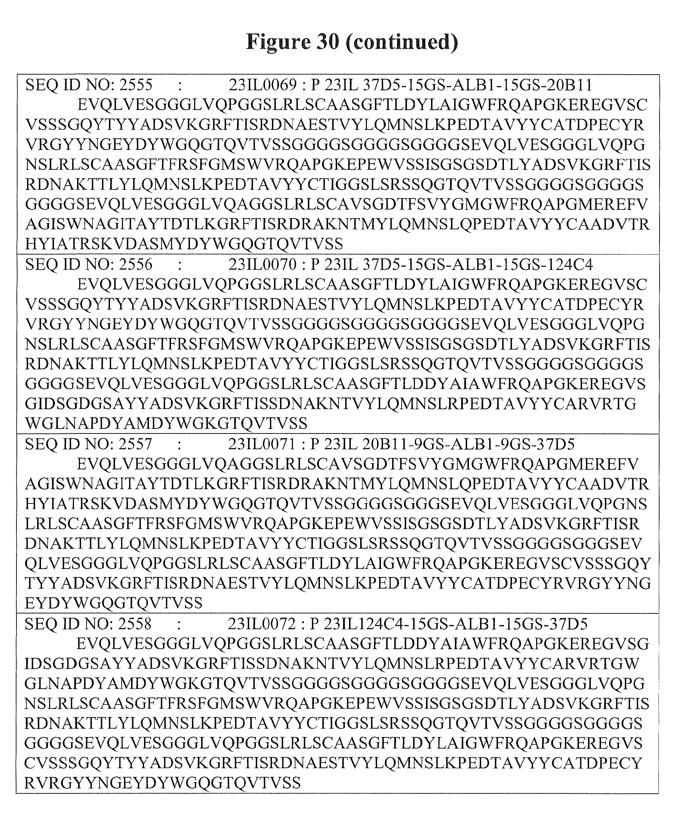

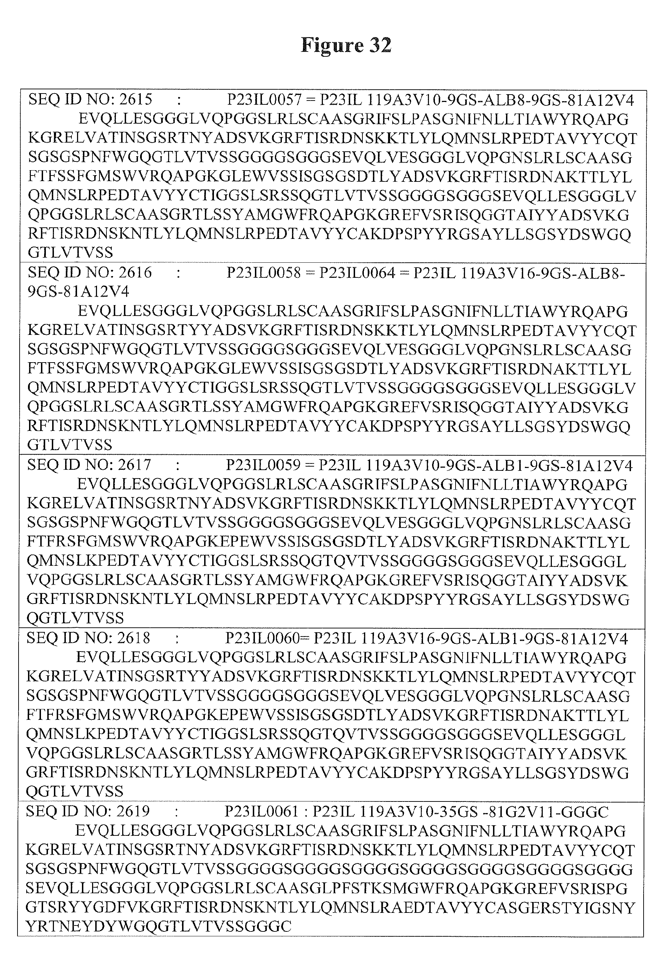

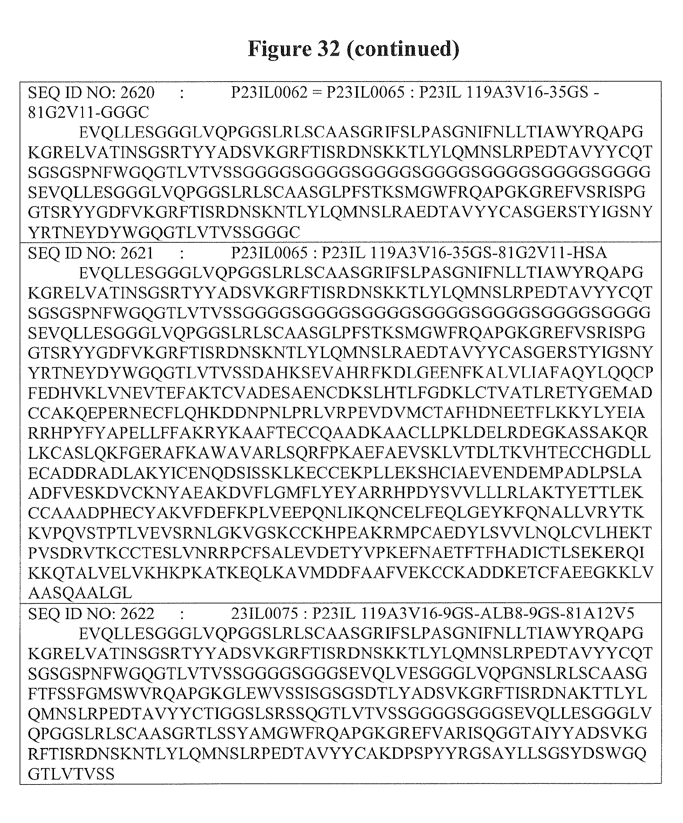

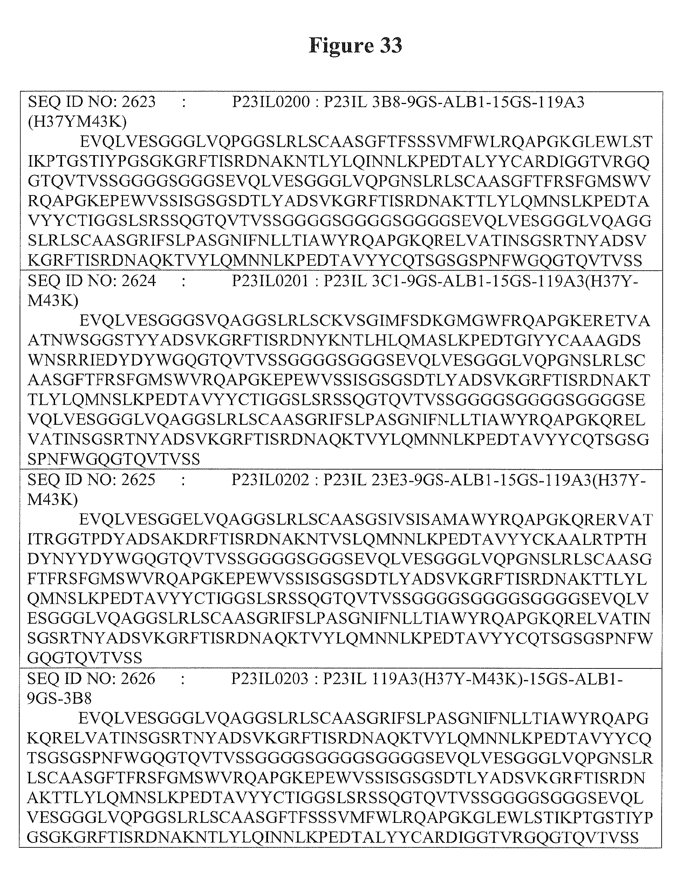

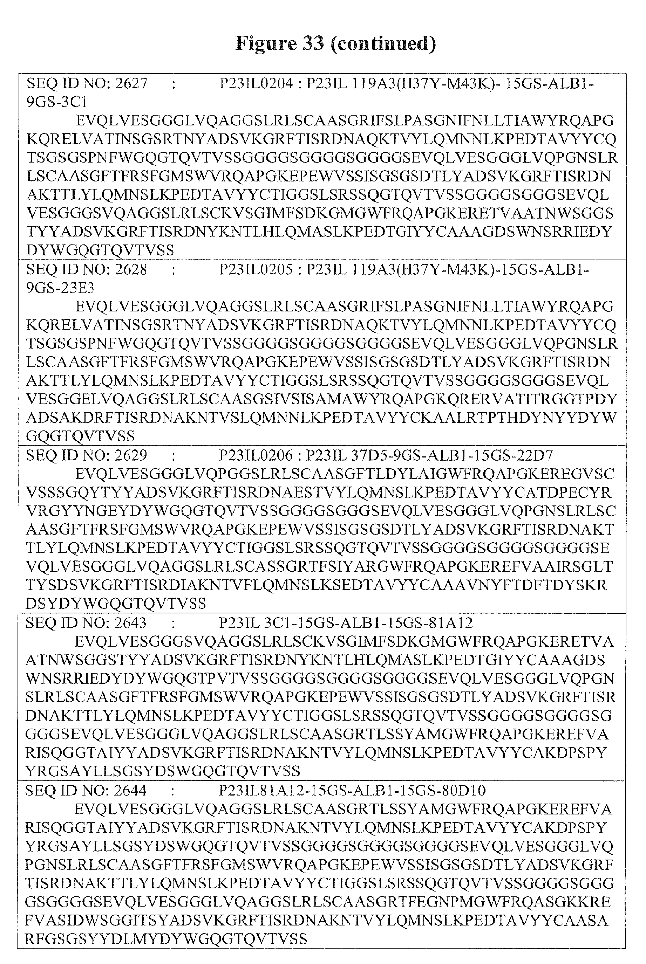

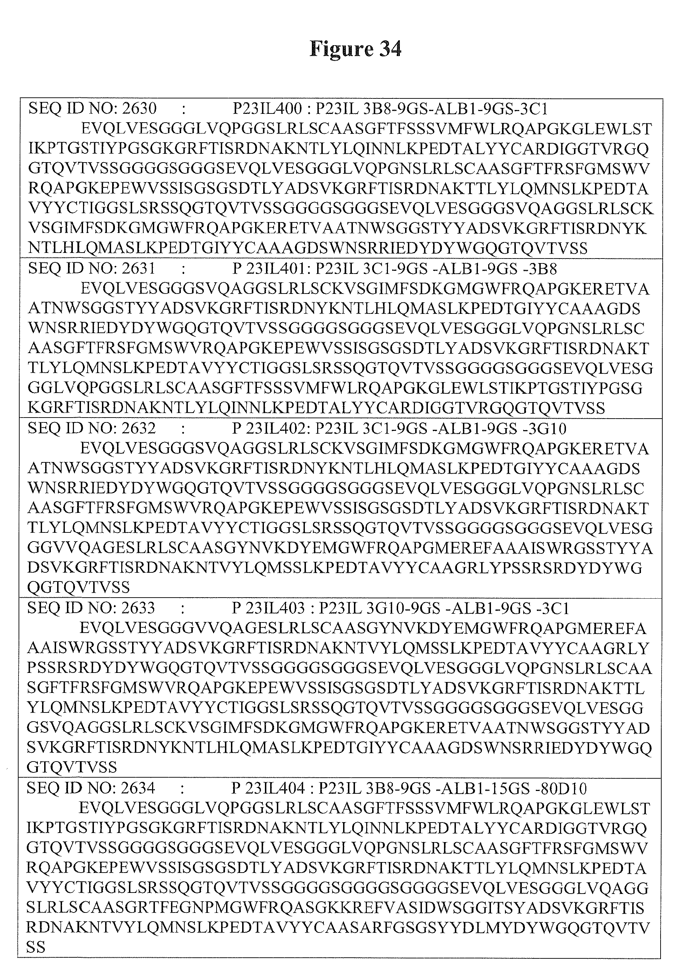

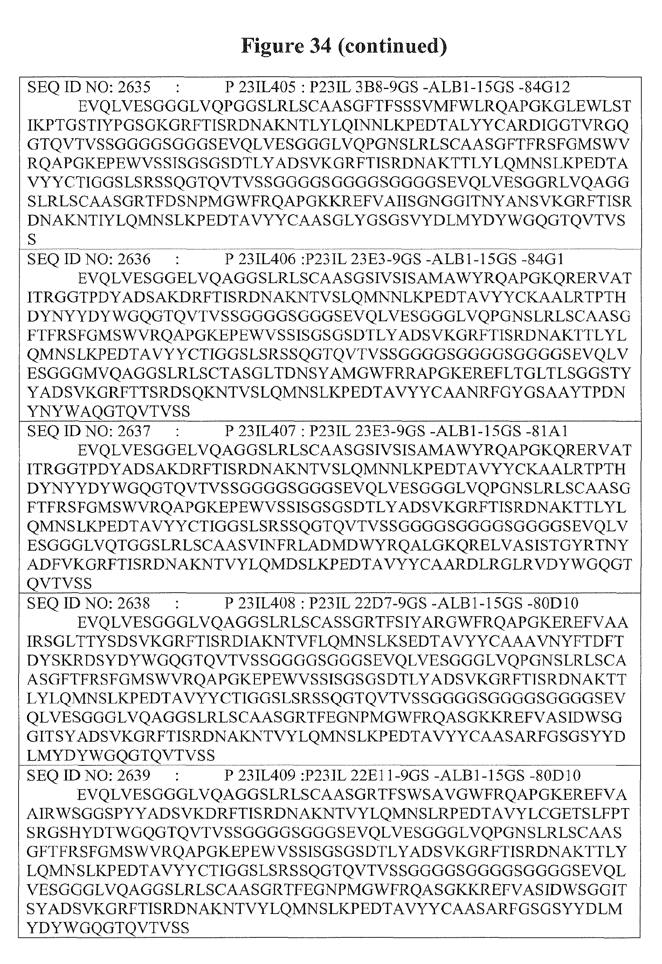

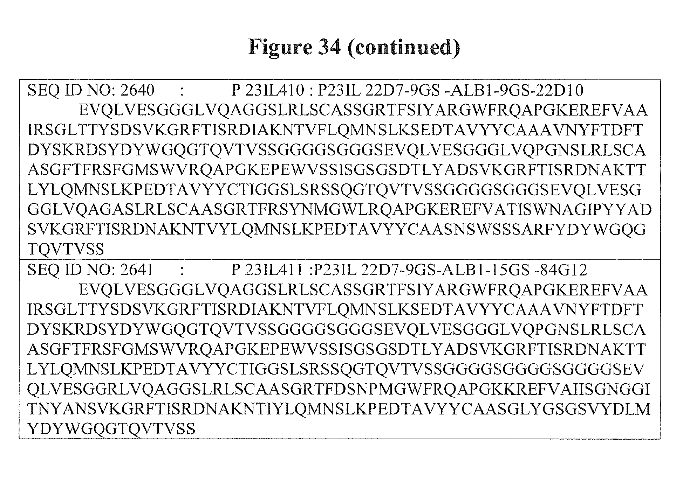

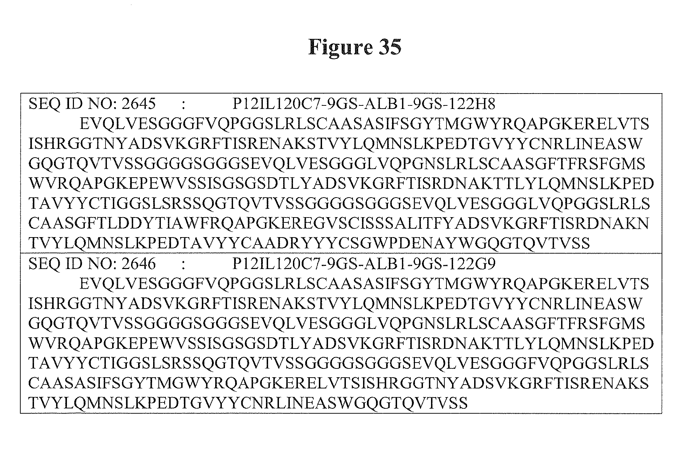

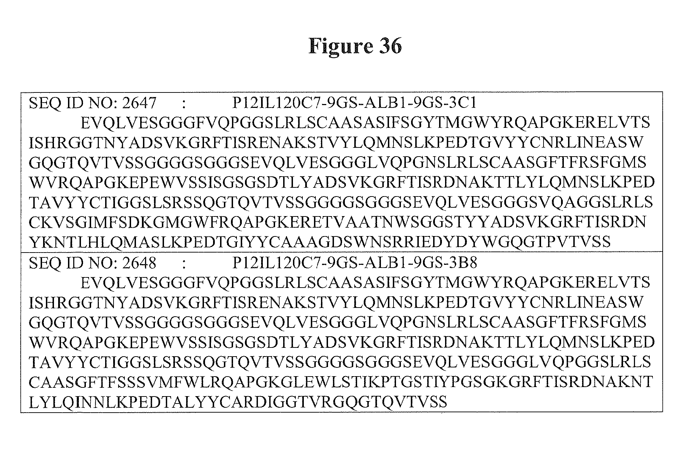

Some preferred, but non-limiting examples of polypeptides of the invention are:
- the polypeptides of SEQ ID NO: 2142; SEQ ID NO: 2143; SEQ ID NO: 2144; SEQ ID NO: 2145; SEQ ID NO: 2146; SEQ ID NO: 2147; SEQ ID NO: 2148; SEQ ID NO: 2149; SEQ ID NO: 2150; SEQ ID NO: 2151; SEQ ID NO: 2152; SEQ ID NO: 2153; SEQ ID NO: 2154; SEQ ID NO: 2155; SEQ ID NO: 2156; SEQ ID NO: 2157; SEQ ID NO: 2158; SEQ ID NO: 2159; SEQ ID NO: 2160; SEQ ID NO: 2161; SEQ ID NO: 2162; SEQ ID NO: 2163; SEQ ID NO: 2164; SEQ ID NO: 2165; SEQ ID NO: 2166; SEQ ID NO: 2167; SEQ ID NO: 2168; SEQ ID NO: 2.169; SEQ ID NO: 2530; SEQ ID NO: 2531; SEQ ID NO: 2532; SEQ ID NO: 2533; SEQ ID NO: 2534; SEQ ID NO: 2535; SEQ ID NO: 2536; SEQ ID NO: 2537; SEQ ID NO: 2538; SEQ ID NO: 2539; SEQ ID NO: 2540; SEQ ID NO: 2341; SEQ ID NO: 2542; SEQ ID NO: 2543; SEQ ID NO: 2544; SEQ ID NO: 2545; SEQ ID NO: 2546; SEQ ID NO: 2547; SEQ ID NO: 2548; SEQ ID NO: 2549; SEQ ID NO: 2550; SEQ ID NO: 2551; SEQ ID NO: 2552; SEQ ID NO: 2553; SEQ ID NO: 2554; SEQ ID NO: 2555; SEQ ID NO: 2556; SEQ ID NO: 2557 and/or SEQ ID NO: 2558 (see also Figure 30); which are some non-limiting examples of multivalent, multispecific and/or biparatopic polypeptides of the invention that are directed against p19 (i.e. comprising at least one p19+ sequence and/or at least one p19- sequence). These polypeptides are directed against (as defined herein) and (expected to be) specific for (as defined herein) a heterodimeric cytokine comprising a p19 subunit (compared to other heterodimerie cytokines that do not comprise a p19 subunit). For example, these polypeptides are expected to be specific for (as defined herein) IL-23 compared to IL-12 (and also IL-27 and/or IL-35);
- the polypeptides of SEQ ID NO: 2515; SEQ ID NO: 2616; SEQ ID NO: 2617; SEQ ID NO: 2618; SEQ ID NO: 2619; SEQ ID NO: 2620; SEQ ID NO: 262.1 and/or SEQ ID NO: 2622 (see also Figure 32); which are some non-limiting examples of multivalent, multispecific and/or biparatopic polypeptide of the invention that are directed against p19 that comprise at least one humanized p19+ sequence and/or at least one humanized p19- sequence). These polypeptide are directed against (as defined herein) and (expected to be) specific for (as defined herein) a "heterodimeric cytokine comprising a p19 subunit (compared to other heterodimeric cytokines that do not comprise a p19 subunit). For example, these polypeptides are expected to be specific, for (as defined herein) IL-23 compared to IL-12 (and also IL-27 and/or IL-35);
- the polypeptides of SEQ ID NO: 2623; SEQ ID NO: 2624; SEQ ID NO: 2625; SEQ ID NO: 2626; SEQ ID NO: 2627; SEQ ID NO: 2628; SEQ ID NO: 2629; SEQ ID NO: 2643 and/or SEQ ID NO: 2644 (see also Figure 33); which are some non-limiting examples of multispecific "p19-p40" polypeptides of the invention that comprise at least one amino acid sequence of the invention that is directed against p19 (i.e. at least one p19+ sequence and/or at least one p19- sequence) and at least one amino acid sequence of the invention that is directed against p40 (i.e. at least one p40+ sequence and/or at least one p40- sequence). These polypeptides are expected to be specific for (as defined herein) IL-23 compared to IL-12 (and also IL-27 and/or IL-35);
- the polypeptides of SEQ ID NO: 2630; SEQ ID NO: 2631; SEQ ID NO: 2632; SEQ ID NO: 2633; SEQ ID NO: 2634; SEQ ID NO: 2635; SEQ ID NO: 2636; SEQ ID NO: 2637; SEQ ID NO: 2638; SEQ ID NO: 2639; SEQ ID NO: 2640 and/or SEQ ID NO: 2641 (see also Figure 34); which are some non-limiting examples of multivalent, multispecific and/or biparatopic polypeptides of the invention that are directed against p40 (i.e. comprising at least one p40+ sequence and/or at least one p40- sequence). These polypeptides are directed against (as defined herein) and (expected to be) specific for (as defined herein) a heterodimeric cytokine comprising a p40 subunit (compared to other heterodimeric cytokines that do not comprise a p40 subunit). For example, these polypeptides are expected to be specific for (as defined herein) IL-23 and/or IL-12 compared to IL-27 and/or IL-35;
- the polypeptides of SEQ ID NO: 2645 and/or SEQ ID NO: 2646 (see also Figure 35), which are some non-limiting examples of multivalent, multispecific and/or biparatopic polypeptides of the invention that are directed against p35. These polypeptides are directed against (as defined herein) and (expected to be) specific for (as defined herein) a heterodimeric cytokine comprising a p35 subunit (compared to other heterodimeric cytokines that do not comprise a p35 subunit). For example, these polypeptides are expected to be specific for (as defined herein) IL-12 compared to IL-23 (and also IL-27 and/or IL-35);
- the polypeptides of SEQ ID NO: 2647 and/or SEQ ID NO: 2648 (see also Figure 36), which are some non-limiting examples of multispecific "p35-p40" polypeptides of tolypepe invention that comprise at least one amino acid sequence of the invention that is directed against p35 and at least one amino acid sequence of the invention that is directed against p40 (i.e, at least one p40+ sequence and/or at least one p40- sequence). These polypeptides are expected to be specific for (as defined herein) IL-12 compared to IL-23 (and also IL-27 and/or IL-35).

Other examples of polypeptides suitable for use in the invention, of amino acid sequences of the invention (or nucleotide sequences/nucleic acids comprising the same) that can be used in such polypeptides (such as the p19+ sequences, p19- sequences, p40+ sequences, p40- sequences, p35 sequences, IL-27 sequences, IL-12Rb1 sequences, IL-12Rb2 sequences and IL-23 sequences described herein), and how polypeptides of the invention can be constructed and produced using such amino acid sequences of the invention will be clear to the skilled person based on the disclosure herein.

Thus, some further aspects of the invention relate to:
- the polypeptide (construct) of SEQ ID NO: 2142; or a polypeptide (construct) that has at least 70%, preferably at least 80%, more preferably at least 85%, such as at least 90%, for example more than 95%, amino acid identity (as defined herein) with the polypeptide of SEQ ID NO: 2142;
- the polypeptide (construct) of SEQ ID NO: 2143; or a polypeptide (construct) that has at least 70%, preferably at least 80%, more preferably at least 85%, such as at least 90%, for example more than 95%, amino acid identity (as defined herein) with the polypeptide of SEQ ID NO: 2143;
- the polypeptide (construct) of SEQ ID NO: 2144; or a polypeptide (construct) that has at least 70%, preferably at least 80%, more preferably at least 85%, such as at least 90%, for example more than 95%, amino acid identity (as defined herein) with the polypeptide of SEQ ID NO: 2144;
- the polypeptide (construct) of SEQ ID NO: 2145; or a polypeptide (construct) that has at least 70%, preferably at least 80%, more preferably at least 85%, such as at least 90%, for example more than 95%, amino acid identity (as defined herein) with the polypeptide of SEQ ID NO: 2145;
- the polypeptide (construct) of SEQ ID NO: 2146; or a polypeptide (construct) that has at least 70%, preferably at least 80%, more preferably at least 85%, such as at least 90%, for example more than 95%, amino acid identity (as defined herein) with the polypeptide of SEQ ID NO: 2146;
- the polypeptide (construct) of SEQ ID NO: 2147; or a polypeptide (construct) that has at least 70%, preferably at least 80%, more preferably at least 85%, such as at least 90%, for example more than 95%, amino acid identity (as defined herein) with the polypeptide of SEQ ID NO: 2147;
- the polypeptide (construct) of SEQ ID NO: 2148; or a polypeptide (construct) that has at least 70%, preferably at least 80%, more preferably at least 85%, such as at least 90%, for example more than 95%, amino acid identity (as defined herein) with the polypeptide of SEQ ID NO: 2148;
- the polypeptide (construct) of SEQ ID NO: 2149; or a polypeptide (construct) that has at least 70%, preferably at least 80%, more preferably at least 85%, such as at least 90%, for example more than 95%, amino acid identity (as defined herein) with the polypeptide of SEQ ID NO: 2149;
- the polypeptide (construct) of SEQ ID NO: 2150; or a polypeptide (construct) that has at least 70%, preferably at least 80%, more preferably at least 85%, such as at least 90%, for example more than 95%, amino acid identity (as defined herein) with the polypeptide of SEQ ID NO: 2150;
- the polypeptide (construct) of SEQ ID NO: 2151; or a polypeptide (construct) that has at least 70%, preferably at least 80%, more preferably at least 85%, such as at least 90%, for example more than 95%, amino acid identity (as defined herein) with the polypeptide of SEQ ID NO: 2151;
- the polypeptide (construct) of SEQ ID NO: 2152; or a polypeptide (construct) that has at least 70%, preferably at least 80%, more preferably at least 85%, such as at least 90%, for example more than 95%, amino acid identity (as defined herein) with the polypeptide of SEQ ID NO: 2152;
- the polypeptide (construct) of SEQ ID NO: 2153; or a polypeptide (construct) that has at least 70%, preferably at least 80%, more preferably at least 85%, such as at least 90%, for example more than 95%, amino acid identity (as defined herein) with the polypeptide of SEQ ID NO: 2153;
- the polypeptide (construct) of SEQ ID NO: 2154; or a polypeptide (construct) that has at least 70%, preferably at least 80%, more preferably at least 85%, such as at least 90%, for example more than 95%, amino acid identity (as defined herein) with the polypeptide of SEQ ID NO: 2154;
- the polypeptide (construct) of SEQ ID NO: 2155; or a polypeptide (construct) that has at least 70%, preferably at least 80%, more preferably at least 85%, such as at least 90%, for example more than 95%, amino acid identity (as defined herein) with the polypeptide of SEQ ID NO: 2155;
- the polypeptide (construct) of SEQ ID NO: 2156; or a polypeptide (construct) that has at least 70%, preferably at least 80%, more preferably at least 85%, such as at least 90%, for example more than 95%, amino acid identity (as defined herein) with the polypeptide of SEQ ID NO: 2156;
- the polypeptide (construct) of SEQ ID NO: 2157; or a polypeptide (construct) that has at least 70%, preferably at least 80%, more preferably at least 85%, such as at least 90%, for example more than 95%, amino acid identity (as defined herein) with the polypeptide of SEQ ID NO:2157;
- the polypeptide (construct) of SEQ ID NO: 2158; or a polypeptide (construct) that has at least 70%, preferably at least 80%, more preferably at least 85%, such as at least 90%, for example more than 95%, amino acid identity (as defined herein) with the polypeptide of SEQ ID NO: 2158;
- the polypeptide (construct) of SEQ ID NO: 2159; or a polypeptide (construct) that has at least 70%, preferably at least 80%, more preferably at least 85%, such as at least 90%, for example more than 95%, amino acid identity (as defined herein) with the polypeptide of SEQ ID NO: 2159;
- the polypeptide (construct) of SEQ ID NO: 2160; or a polypeptide (construct) that has at least 70%, preferably at least 80%, more preferably at least 85%, such as at least 90%, for example more than 95%, amino acid identity (as defined herein) with the polypeptide of SEQ ID NO: 2160;
- the polypeptide (construct) of SEQ ID NO: 2161; or a polypeptide (construct) that has at least 70%, preferably at least 80%, more preferably at least 85%, such as at least 90%, for example more than 95%, amino acid identity (as defined herein) with the polypeptide of SEQ ID NO: 2161;
- the polypeptide (construct) of SEQ ID NO: 2162; or a polypeptide (construct) that has at least 70%, preferably at least 80%, more preferably at least 85%, such as at least 90%, for example more than 95%, amino acid identity (as defined herein) with the polypeptide of SEQ ID NO: 2162;
- the polypeptide (construct) of SEQ ID NO: 2163; or a polypeptide (construct) that has at least 70%, preferably at least 80%, more preferably at least 85%, such as at least 90%, for example more than 95%, amino acid identity (as defined herein) with the polypeptide of SEQ ID NO: 2163;
- the polypeptide (construct) of SEQ ID NO:2164; or a polypeptide (construct) that has at least 70%, preferably at least 80%, more preferably at least 85%, such as at least 90%, for example more than 95%, amino acid identity (as defmed herein) with the polypeptide of SEQ ID NO: 2164;
- the polypeptide (construct) of SEQ ID NO: 2165; or a polypeptide (construct) that has at least 70%, preferably at least 80%, more preferably at least 85%, such as at least 90%, for example more than 95%, amino acid identity (as defined herein) with the polypeptide of SEQ ID NO: 2165;
- the polypeptide (construct) of SEQ ID NO: 2166; or a polypeptide (construct) that has at least 70%, preferably at least 80%, more preferably at least 85%, such as at least 90%, for example more than 95%, amino acid identity (as defined herein) with the polypeptide of SEQ ID NO: 2166;
- the polypeptide (construct) of SEQ ID NO: 2167; or a polypeptide (construct) that has at least 70%, preferably at least 80%, more preferably at least 85%, such as at least 90%, for example more than 95%, amino acid identity (as defined herein) with the polypeptide of SEQ ID NO: 2167;
- the polypeptide (construct) of SEQ ID NO: 2168; or a polypeptide (construct) that has at least 70%, preferably at least 80%, more preferably at least 85%, such as at least 90%, for example more than 95%, amino acid identity (as defined herein) with the polypeptide of SEQ ID NO: 2168;
- the polypeptide (construct) of SEQ ID NO: 2169; or a polypeptide (construct) that has at least 70%, preferably at least 80%, more preferably at least 85%, such as at least 90%, for example more than 95%, amino acid identity (as defined herein) with the polypeptide of SEQ ID NO: 2169;
- the polypeptide (construct) of SEQ ID NO: 2530; or a polypeptide (construct) that has at least 70%, preferably at least 80%, more preferably at least 85%, such as at least 90%, for example more than 95%, amino acid identity (as defined herein) with the polypeptide of SEQ ID NO: 2530;
- the polypeptide (construct) of SEQ ID NO: 2531; or a polypeptide (construct) that has at least 70%, preferably at least 80%, more preferably at least 85%, such as at least 90%, for example more than 95%, amino acid identity (as defined herein) with the polypeptide of SEQ ID NO: 2531;
- the polypeptide (construct) of SEQ ID NO: 2532; or a polypeptide (construct) that has at least 70%, preferably at least 80%, more preferably at least 85%, such as at least 90%, for example more than 95%, amino acid identity (as defined herein) with the polypeptide of SEQ ID NO: 2532;
- the polypeptide (construct) of SEQ ID NO: 2533; or a polypeptide (construct) that has at least 70%, preferably at least 80%, more preferably at least 85%, such as at least 90%, for example more than 95%, amino acid identity (as defined herein) with the polypeptide of SEQ ID NO: 2533;
- the polypeptide (construct) of SEQ ID NO: 2534; or a polypeptide (construct) that has at least 70%, preferably at least 80%, more preferably at least 85%, such as at least 90%, for example more than 95%, amino acid identity (as defined herein) with the polypeptide of SEQ ID NO: 2534;
- the polypeptide (construct) of SEQ ID NO: 2535; or a polypeptide (construct) that has at least 70%, preferably at least 80%, more preferably at least 85%, such as at least 90%, for example more than 95%, amino acid identity (as defined herein) with the polypeptide of SEQ ID NO: 2535;
- the polypeptide (construct) of SEQ ID NO: 2536; or a polypeptide (construct) that has at least 70%, preferably at least 80%, more preferably at least 85%, such as at least 90%, for example more than 95%, amino acid identity (as defined herein) with the polypeptide of SEQ ID NO: 2536;
- the polypeptide (construct) of SEQ ID NO: 2537; or a polypeptide (construct) that has at least 70%, preferably at least 80%, more preferably at least 85%, such as at least 90%, for example more than 95%, amino acid identity (as defined herein) with the polypeptide of SEQ ID NO: 2537;
- the polypeptide (construct) of SEQ ID NO: 2538; or a polypeptide (construct) that has at least 70%, preferably at least 80%, more preferably at least 85%, such as at least 90%, for example more than 95%, amino acid identity (as defined herein) with the polypeptide of SEQ ID NO: 2538;
   the polypeptide (construct) of SEQ ID NO: 2539; or a polypeptide (construct) that has at least 70%, preferably at least 80%, more preferably at least 85%, such as at least 90%, for example more than 95%, amino acid identity (as defined herein) with the polypeptide of SEQ ID NO: 2539;
- the polypeptide (construct) of SEQ ID NO: 2540; or a polypeptide (construct) that has at least 70%, preferably at least 80%, more preferably at least 85%, such as at least 90%, for example more than 95%, amino acid identity (as defined herein) with the polypeptide of SEQ ID NO: 2540;
- the polypeptide (construct) of SEQ ID NO: 2541; or a polypeptide (construct) that has at least 70%, preferably at least 80%, more preferably at least 85%, such as at least 90%, for example more than 95%, amino acid identity (as defined herein) with the polypeptide of SEQ ID NO: 2541;
- the polypeptide (construct) of SEQ ID NO: 2542; or a polypeptide (construct) that has at least 70%, preferably at least 80%, more preferably at least 85%, such as at least 90%, for example more than 95%, amino acid identity (as defined herein) with the polypeptide of SEQ ID NO: 2542;
- the polypeptide (construct) of SEQ ID NO: 2543; or a polypeptide (construct) that has at least 70%, preferably at least 80%, more preferably at least 85%, such as at least 90%, for example more than 95%, amino acid identity (as defined herein) with the polypeptide of SEQ ID NO: 2543;
- the polypeptide (construct) of SEQ ID NO: 2544; or a polypeptide (construct) that has at least 70%, preferably at least 80%, more preferably at least 85%, such as at least 90%, for example more than 95%, amino acid identity (as defined herein) with the polypeptide of SEQ ID NO: 2544;
- the polypeptide (construct) of SEQ ID NO: 2545; or a polypeptide (construct) that has at least 70%, preferably at least 80%, more preferably at least 85%, such as at least 90%, for example more than 95%, amino acid identity (as defined herein) with the polypeptide of SEQ ID NO: 2545;
- the polypeptide (construct) of SEQ ID NO: 2546; or a polypeptide (construct) that has at least 70%, preferably at least 80%, more preferably at least 85%, such as at least 90%, for example more than 95%, amino acid identity (as defined herein) with the polypeptide of SEQ ID NO: 2546;
- the polypeptide (construct) of SEQ ID NO: 2547; or a polypeptide (construct) that has at least 70%, preferably at least 80%, more preferably at least 85%, such as at least 90%, for example more than 95%, amino acid identity (as defined herein) with the polypeptide of SEQ ID NO: 2547;
- the polypeptide (construct) of SEQ ID NO: 2548; or a polypeptide (construct) that has at least 70%, preferably at least 80%, more preferably at least 85%, such as at least 90%, for example more than 95%, amino acid identity (as defined herein) with the polypeptide of SEQ ID NO: 2548;
- the polypeptide (construct) of SEQ ID NO: 2549; or a polypeptide (construct) that has at least 70%, preferably at least 80%, more preferably at least 85%, such as at least 90%, for example more than 95%, amino acid identity (as defined herein) with the polypeptide of SEQ ID NO: 2549;
- the polypeptide (construct) of SEQ ID NO: 2550; or a polypeptide (construct) that has at least 70%, preferably at least 80%, more preferably at least 85%, such as at least 90%, for example more than 95%, amino acid identity (as defined herein) with the polypeptide of SEQ ID NO: 2550;
- the polypeptide (construct) of SEQ ID NO: 2551; or a polypeptide (construct) that has at least 70%, preferably at least 80%, more preferably at least 85%, such as at least 90%, for example more than 95%, amino acid identity (as defined herein) with the polypeptide of SEQ ID NO: 2551;
- the polypeptide (construct) of SEQ ID NO: 2552; or a polypeptide (construct) that has at least 70%, preferably at least 80%, more preferably at least 85%, such as at least 90%, for example more than 95%, amino acid identity (as defined herein) with the polypeptide of SEQ ID NO: 2552;
- the polypeptide (construct) of SEQ ID NO: 2553; or a polypeptide (construct) that has at least 70%, preferably at least 80%, more preferably at least 85%, such as at least 90%, for example more than 95%, amino acid identity (as defined herein) with the polypeptide of SEQ ID NO: 2553;
- the polypeptide (construct) of SEQ ID NO: 2554; or a polypeptide (construct) that has at least 70%, preferably at least 80%, more preferably at least 85%, such as at least 90%, for example more than 95%, amino acid identity (as defined herein) with the polypeptide of SEQ ID NO: 2554;
- the polypeptide (construct) of SEQ ID NO: 2555; or a polypeptide (construct) that has at least 70%, preferably at least 80%, more preferably at least 85%, such as at least 90%, for example more than 95%, amino acid identity (as defined herein) with the polypeptide of SEQ ID NO: 2555;
- the polypeptide (construct) of SEQ ID NO: 2556; or a polypeptide (construct) that has at least 70%, preferably at least 80%, more preferably at least 85%, such as at least 90%, for example more than 95%, amino acid identity (as defined herein) with the polypeptide of SEQ ID NO: 2556;
- the polypeptide (construct) of SEQ ID NO: 2557; or a polypeptide (construct) that has at least 70%, preferably at least 80%, more preferably at least 85%, such as at least 90%, for example more than 95%, amino acid identity (as defined herein) with the polypeptide of SEQ ID NO: 2557;
- the polypeptide (construct) of SEQ ID NO: 2558; or a polypeptide (construct) that has at least 70%, preferably at least 80%, more preferably at least 85%, such as at least 90%, for example more than 95%, amino acid identity (as defined herein) with the polypeptide of SEQ ID NO: 2558;
- the polypeptide (construct) of SEQ ID NO: 2615; or a polypeptide (construct) that has at least 70%, preferably at least 80%, more preferably at least 85%, such as at least 90%, for example more than 95%, amino acid identity (as defined herein) with the polypeptide of SEQ ID NO: 2615;
- the polypeptide (construct) of SEQ ID NO: 2616; or a polypeptide (construct) that has at least 70%, preferably at least 80%, more preferably at least 85%, such as at least 90%, for example more than 95%, amino acid identity (as defined herein) with the polypeptide of SEQ ID NO: 2616;
- the polypeptide (construct) of SEQ ID NO: 2617; or a polypeptide (construct) that has at least 70%, preferably at least 80%, more preferably at least 85%, such as at least 90%, for example more than 95%, amino acid identity (as defined herein) with the polypeptide of SEQ ID NO: 2617;
- the polypeptide (construct) of SEQ ID NO: 2618; or a polypeptide (construct) that has at least 70%, preferably at least 80%, more preferably at least 85%, such as at least 90%, for example more than 95%, amino acid identity (as defined herein) with the polypeptide of SEQ ID NO: 2618;
- the polypeptide (construct) of SEQ ID NO: 2619; or a polypeptide (construct) that has at least 70%, preferably at least 80%, more preferably at least 85%, such as at least 90%, for example more than 95%, amino acid identity (as defined herein) with the polypeptide of SEQ ID NO: 2619;
- the polypeptide (construct) of SEQ ID NO: 2620; or a polypeptide (construct) that has at least 70%, preferably at least 80%, more preferably at least 85%, such as at least 90%, for example more than 95%, amino acid identity (as defined herein) with the polypeptide of SEQ ID NO: 2620;
- the polypeptide (construct) of SEQ ID NO: 2621; or a polypeptide (construct) that has at least 70%, preferably at least 80%, more preferably at least 85%, such as at least 90%, for example more than 95%, amino acid identity (as defined herein) with the polypeptide of SEQ ID NO: 2621;
- the polypeptide (construct) of SEQ ID NO: 2622, or a polypeptide (construct) that has at least 70%, preferably at least 80%, more preferably at least 85%, such as at least 90%, for example more than 95%, amino acid identity (as defined herein) with the polypeptide of SEQ ID NO: 2622;
as well as to nucleotide sequences or nucleotide sequences encoding the same. These polypeptide constructs are preferably further such that they are directed against p19 and/or IL-23 (and more preferably also specific for p19 and/or IL-23), and even more preferably capable of modulating, blocking, neutralizing or inhibiting the binding of IL-23 to its cognate receptor (for example, in the alpha-screen assay described in Example 19 or 22).

Yet further aspects of the invention relate to:
- the polypeptide (construct) of SEQ ID NO: 2623; or a polypeptide (construct) that has at least 70%, preferably at least 80%, more preferably at least 85%, such as at least 90%, for example more than 95%, amino acid identity (as defined herein) with the polypeptide of SEQ ID NO: 2623;
- the polypeptide (construct) of SEQ ID NO: 2624; or a polypeptide (construct) that has at least 70%, preferably at least 80%, more preferably at least 85%, such. as at least 90%, for example more than 95%, amino acid identity (as defined herein) with the polypeptide af SEQ ID NO: 2624:
- the polypeptide (construct) of SEQ ID NO: 2625; or a polypeptide (construct) that has at least 70%, preferably at least 80%, more preferably at least 85%, such as at least 90%, for example more than 95%, amino acid identity (as defined herein) with the polypeptide of SEQ ID NO: 2625;
- the polypeptide (construct) of SEQ ID NO: 2626; or a polypeptide (construct) that has at least 70%, preferably at least 80%, more preferably at least 85%, such as at least 90%, for example more than 95%, amino acid identity (as defined herein) with the polypeptide of SEQ ID NO: 2626;
- the polypeptide (construct) of SEQ ID NO: 2627; or a polypeptide (construct) that has at least 70%, preferably at least 80%, more preferably at least 85%, such as at least 90%, for example more than 95%, amino acid identity (as defined herein) with the polypeptide of SEQ ID NO: 2627;
- the polypeptide (construct) of (SEQ ID NO: 2628; or a polypeptide (construct) that has at least 70%, preferably at least 80%, more preferably at least 85%, such as at least 90%, for example more than 95%, amino acid identity (as defined herein) with the polypeptide of SEQ ID NO: 2628;
- the polypeptide (construct) of SEQ ID NO: 2629; or a polypeptide (construct) that has at least 70%, preferably at least 80%, more preferably at least 85%, such as at least 90%, for example more than 95%, amino acid identity (as defined herein) with the polypeptide of SEQ ID NO: 2629;
- the polypeptide (construct) of SEQ ID NO: 2643; or a polypeptide (construct) that has at least 70%, preferably at least 80%, more preferably at least 85%, such as at least 90%, for example more than 95%, amino acid identity (as defined herein) with the polypeptide of SEQ ID NO: 2643;
- the polypeptide (construct) of SEQ ID NO: 2644; or a polypeptide (construct) that has at least 70%, preferably at least 80%. more preferably at least 85%, such as at least 90%, for example more than 95%, amino acid identity (as defined herein) with the polypeptide of SEQ ID NO: 2644;
as well as to nucleotide sequences or nucleotide sequences encoding the same. These polypeptide constructs are preferably further such that they are directed against p19 and/or IL-23 (and more preferably also specific for p19 and/or IL-23), and even more preferably capable of modulating, blocking, neutralizing or inhibiting the binding of IL-23 to its cognate receptor (for example, in the alpha-screen assay described in Example 19 or 22).

Yet further aspects of the invention relate to:
- the polypeptide (construct) of SEQ ID NO: 2630; or a polypeptide (construct) that has at least 70%, preferably at least 80%, more preferably at least 85%, such as at least 90%, for example more than 95%, amino acid identity (as defined herein) with the polypeptide of SEQ ID NO: 2630;
- the polypeptide (construct) of SEQ ID NO: 2631; or a polypeptide (construct) that has at least 70%, preferably at least 80%, more preferably at least 85%, such as at least 90%, for example more than 95%, amino acid identity (as defined herein) with the polypeptide of SEQ ID NO: 2631;
- the polypeptide (construct) of SEQ ID NO: 2632; or a polypeptide (construct) that has at least 70%, preferably at least 80%, more preferably at least 85%, such as at least 90%, for example more than 95%, amino acid identity (as defined herein) with the polypeptide of SEQ ID NO: 2632;
- the polypeptide (construct) of SEQ ID NO: 2633; or a polypeptide (construct) that has at least 70%, preferably at least 80%, more preferably at least 85%, such as at least 90%, for example more than 95%, amino acid identity (as defined herein) with the polypeptide of SEQ ID NO: 2633;
- the polypeptide (construct) of SEQ ID NO: 2634; or a polypeptide (construct) that has at least 70%, preferably at least 80%, more preferably at least 85%, such as at least 90%, for example more than 95%, amino acid identity (as defined herein) with the polypeptide of SEQ ID NO: 2634;
- the polypeptide (construct) of SEQ ID NO: 2635; or a polypeptide (construct) that has at least 70%, preferably at least 80%, more preferably at: least 85%, such as at least 90%, for example more than 95%, amino acid identity (as defined herein) with the polypeptide of SEQ ID NO: 2635;
- the polypeptide (construct) of SEQ ID NO: 2636; or a polypeptide (construct) that has at least 70%, preferably at least 80%, more preferably at least 85%, such as at least 90%, for example more than 95%, amino acid identity (as defined herein) with the polypeptide of SEQ ID NO: 2636;
- the polypeptide (construct) of SEQ ID NO: 2637; or a. polypeptide (construct) that has at least 70%, preferably at least 80%, more preferably at least 85%, such as at least 90%, for example more than 95%, amino acid identity (as defined herein) with the polypeptide of SEQ ID NO: 2637;
- the polypeptide (construct) of SEQ ID NO: 2638; or a polypeptide (construct) that has at least 70%, preferably at least 80%, more preferably at least 85%, such as at least 90%, for example more than 95%, amino acid identity (as defined herein) with the polypeptide of SEQ ID NO: 2638;
- the polypeptide (construct) of SEQ ID NO: 2639; or a polypeptide (construct) that has at least 70%, preferably at least 80%, more preferably at least 85%, such as at least 90%, for example more than 95%, amino acid identity (as defined herein) with the polypeptide of SEQ ID NO: 2639;
- the polypeptide (construct) of (SEQ ID NO: 2640; or a polypeptide (construct) that has at least 70%, preferably at least 80%, more preferably at least 85%, such as at least 90%, for example more than 95%, amino acid identity (as defined herein) with the polypeptide of SEQ ID NO: 2640;
- the polypeptide (construct) of SEQ ID NO: 2641; or a polypeptide (construct) that has at least 70%, preferably at least 80%, more preferably at least 85%, such as at least 90%, for example more than 95%, amino acid identity (as defined herein) with the polypeptide of SEQ ID NO: 2641;
as well as to nucleotide sequences or nucleotide sequences encoding the same. These polypeptide constructs are preferably further such that they are directed against p40, IL-12 and/or IL-23 (and more preferably also specific for p40, IL-12 and/or IL-23 compared to IL-27 and/or IL-35), and even more preferably capable of modulating, blocking, neutralizing or inhibiting the binding of IL-23 to its cognate receptor and/or of modulating, blocking, neutralizing or inhibiting the binding of IL-12 to its cognate receptor (for example, in the alpha-screen assay described in Example 19 or 22).

Yet further aspects of the invention relate to
- the polypeptide (construct) of SEQ ID NO: 2645; or a polypeptide (construct) that has at least 70%, preferably at least 80%, more preferably at least 85%, such as at least 90%, for example more than 95%, amino acid identity (as defined herein) with the polypeptide of SEQ ID NO: 2645;
- the polypeptide (construct) of SEQ ID NO: 2646; or a polypeptide (construct) that has at least 70%, preferably at least 80%, more preferably at least 85%, such as at least 90%, for example more than 95%, amino acid identity (as defined herein) with the polypeptide of SEQ ID NO: 2646;
as well as to nucleotide sequences or nucleotide sequences encoding the same. These polypeptide constructs are preferably further such that they are directed against p35 and/or IL-12 (and more preferably also specific for p35 and/or IL-12), and even more preferably capable of modulating, blocking, neutralizing or inhibiting the binding of IL-12 to its cognate receptor.

Yet other aspects of the invention relate to
- the polypeptide (construct) of SEQ ID NO: 2647; or a polypeptide (construct) that has at least 70%, preferably at least 80%, more preferably at least 85%, such as at least 90%, for example more than 95%, amino acid identity (as defined herein) with the polypeptide of SEQ ID NO: 2647;
- the polypeptide (construct) of SEQ ID NO: 2648; or a polypeptide (construct) that has at least 70%, preferably at least 80%, more preferably at least 85%, such as at least 90%, for example more than 95%, amino acid identity (as defined herein) with the polypeptide of SEQ ID NO: 2648;
as well as to nucleotide sequences or nucleotide sequences encoding the same. These polypeptide constructs are preferably further such that they are directed against p35 and/or IL-12 (and more preferably also specific for p35 and/or IL-12), and even more preferably capable of modulating, blocking, neutralizing or inhibiting the binding of IL-12 to its cognate receptor (for example, in the alpha-screen assay described in Example 19 or 22).

In another aspect, the invention relates to a nucleic acid that encodes an amino acid sequence of the invention (such as a (single) domain antibody and/or Nanobody of the invention) or a polypeptide of the invention (or a suitable fragment thereof). Such a nucleic acid will also be referred to herein as *a "nucleic acid of the invention"* and may for example be in the form of a genetic construct, as further described herein.

In another aspect, the invention relates to a host or host cell that expresses (or that under suitable circumstances is capable of expressing) an amino acid sequence of the invention (such as a (single) domain antibody and/or Nanobody of the invention) and/or a polypeptide of the invention; and/or that contains a nucleic acid of the invention. Some preferred but non-limiting examples of such hosts or host cells will become clear from the further description herein.

The invention further relates to a product or composition containing or comprising at least one amino acid sequence of the invention, at least one polypeptide of the invention (or a suitable fragment thereof) and/or at least one nucleic acid of the invention, and optionally one or more further components of such compositions known per se, i.e, depending on the intended use of the composition. Such a product or composition may for example be a pharmaceutical composition (as described herein), a veterinary composition or a product or composition for diagnostic use (as also described herein). Some preferred but non-limiting examples of such products or compositions will become clear from the further description herein.

The invention also relates to the use of an amino acid sequence, Nanobody or polypeptide of the invention, or of a composition comprising the same. in (methods or compositions for) modulating (as defined herein) a heterodimeric cytokine, a receptor for a heterodimeric cytokine and/or heterodimeric cytokine-mediated signalling (as defined herein), either in vitro (e.g. in an in vitro or cellular assay) or in vivo (e.g. in an a single cell or in a multicellular organism, and in particular in a mammal, and more in particular in a human being, such as in a human being that is at risk of or suffers from a disease or disorder associated with heterodimeric cytokines and their receptors).

The invention also relates to methods for modulating (as defined herein) a heterodimeric cytokine, a receptor for a heterodimeric cytokine and/or heterodimeric cytokine-mediated signalling (as defined herein), either in vitro (e.g. in an in vitro or cellular assay) or in vivo (e.g. in an a single cell or multicellular organism, and in particular in a mammal, and more in particular in a human being, such as in a human being that is at risk of or suffers from a disease or disorder associated with heterodimeric cytokines and their receptors), which method comprises at least the step of contacting a heterodimeric cytokines and/or a receptor of a heterodimeric cytokines with at least one amino acid sequence, Nanobody or polypeptide of the invention, or with a composition comprising the same, in a manner and in an amount suitable to modulate the heterodimeric cytokine, the receptor and/or heterodimeric cytokine-mediated signalling.

The invention also relates to the use of an one amino acid sequence, Nanobody or polypeptide of the invention in the preparation of a composition (such as, without limitation, a pharmaceutical composition or preparation as further described herein) for modulating (as defined herein) a heterodimeric cytokine, a receptor for a heterodimeric cytokine and/or heterodimeric cytokine-mediated signalling (as defined herein), either in vitro (e.g. in an in vitro or cellular assay) or in vivo (e.g. in an a single cell or multicellular organism, and in particular in a mammal, and more in particular in a human being, such as in a human being that is at risk of or suffers from a disease or disorder associated with heterodimeric cytokines and their receptors).

The invention further relates to methods for preparing or generating the amino acid sequences, compounds, constructs, polypeptides, nucleic acids, host cells, products and compositions described herein. Some preferred but non-limiting examples of such methods will become clear from the further description herein.

Generally, these methods may comprise the steps of:
a) providing a set, collection or library of amino acid sequences; and
b) screening said set, collection or library of amino acid sequences for amino acid sequences that can bind to and/or have affinity for heterodimeric cytokines and/or their receptors;
   and
c) isolating the amino acid sequence(s) that can bind to and/or have affinity for heterodimeric cytokines and/or their receptors.

In such a method, the set, collection or library of amino acid sequences may be any suitable set, collection or library of amino acid sequences. For example, the set, collection or library of amino acid sequences may be a set, collection or library of immunoglobulin sequences (as described herein), such as a naïve set, collection or library of immunoglobulin sequences; a synthetic or semi-synthetic set, collection or library of immunoglobulin sequences; and/or a set, collection or library of immunoglobulin sequences that have been subjected to affinity maturation.

Also, in such a method, the set, collection or library of amino acid sequences may be a set, collection or library of heavy chain variable domains (such as V_{H} domains or V_{HH} domains) or of light chain variable domains. For example, the set, collection or library of amino acid sequences may be a set, collection or library of domain antibodies or single domain antibodies, or may be a set, collection or library of amino acid sequences that are capable of functioning as a domain antibody or single domain antibody.

In a preferred aspect of this method, the set, collection or library of amino acid sequences may be an immune set, collection or library of immunoglobulin sequences, for example derived from a mammal that has been suitably immunized with heterodimeric cytokines and/or their receptors or with a suitable antigenic determinant based thereon or derived therefrom, such as an antigenic part, fragment, region, domain, loop or other epitope thereof. In one particular aspect, said antigenic determinant may be an extracellular part, region, domain, loop or other extracellular epitope(s).

In the above methods, the set, collection or library of amino acid sequences may be displayed on a phage, phagemid, ribosome or suitable micro-organism (such as yeast), such as to facilitate screening. Suitable methods, techniques and host organisms for displaying and screening (a set, collection or library of) amino acid sequences will be clear to the person skilled in the art, for example on the basis of the further disclosure herein. Reference is also made to the review by Hoogenboom in Nature Biotechnology, 23, 9,1105-1116 (2005).

In another aspect, the method for generating amino acid sequences comprises at least the steps of:
a) providing a collection or sample of cells expressing amino acid sequences;
b) screening said collection or sample of cells for cells that express an amino acid sequence that can bind to and/or have affinity for heterodimeric cytokines and/or their receptors;
   and
c) either (i) isolating said amino acid sequence; or (ii) isolating from said cell a nucleic acid sequence that encodes said amino acid sequence, followed by expressing said amino acid sequence.

For example, when the desired amino acid sequence is an immunoglobulin sequence, the collection or sample of cells may for example be a collection or sample of B-cells. Also, in this method, the sample of cells may be derived from a mammal that has been suitably immunized with heterodimeric cytokines and/or their receptors or with a suitable antigenic determinant based thereon or derived therefrom, such as an antigenic part, fragment, region, domain, loop or other epitope thereof. In one particular aspect, said antigenic determinant may be an extracellular part, region, domain., loop or other extracellular epitope(s).

The above method may be performed in any suitable manner, as will be clear to the skilled person. Reference is for example made to EP 0 542 810, WO 05/19824, WO 04/051268 and WO 04/106377. The screening of step b) is preferably performed using a flow cytometry technique such as FAC S. For this, reference is for example made to Lieby et al., Blood, Vol. 97, No. 12, 3820 (2001).

In another aspect, the method for generating an amino acid sequence directed against heterodimeric cytokines and/or their receptors may comprise at least the steps of:
a) providing a set, collection or library of nucleic acid sequences encoding amino acid sequences;
b) screening said set, collection or library of nucleic acid sequences for nucleic acid sequences that encode an amino acid sequence that can bind to and/or has affinity for heterodimeric cytokines and/or their receptors:
   and
c) isolating said nucleic acid sequence, followed by expressing said amino acid sequence.

In such a method, the set, collection or library of nucleic acid sequences encoding amino acid sequences may for example be a set, collection or library of nucleic acid sequences encoding a naïve set, collection or library of immunoglobulin sequences; a set, collection or library of nucleic acid sequences encoding a synthetic or semi-synthetic set, collection or library of immunoglobulin sequences; and/or a set, collection or library of nucleic acid sequences encoding a set, collection or library of immunoglobulin sequences that have been subjected to affinity maturation.

Also, in such a method, the set, collection or library of nucleic acid sequences may encode a set, collection or library of heavy chain variable domains (such as V_{H} domains or V_{HH} domains) or of light chain variable domains. For example, the set, collection or library of nucleic acid sequences may encode a set, collection or library of domain antibodies or single domain antibodies, or a set, collection or library of amino acid sequences that are capable of functioning as a domain antibody or single domain antibody.

In a preferred aspect of this method, the set, collection or library of amino acid sequences may be an immune set, collection or library of nucleic acid sequences, for example derived from a mammal that has been suitably immunized with heterodimeric cytokines and/or their receptors or with a suitable antigenic determinant based thereon or derived therefrom, such as an antigenic part, fragment, region, domain, loop or other epitope thereof. In one particular aspect, said antigenic determinant may be an extracellular part, region, domain, loop or other extracellular epitope(s).

The set, collection or library of nucleic acid sequences may for example encode an immune set, collection or library of heavy chain variable domains or of light chain variable domains. In one specific aspect, the set, collection or library of nucleotide sequences may encode a set, collection or library of V_{HH} sequences.

In the above methods, the set, collection or library of nucleotide sequences may be displayed on a phage, phagemid, ribosome or suitable micro-organism (such as yeast), such as to facilitate screening. Suitable methods, techniques and host organisms for displaying and screening (a set, collection or library of) nucleotide sequences encoding amino acid sequences will be clear to the person skilled in the art, for example on the basis of the further disclosure herein. Reference is also made to the review by Hoogenboom in Nature Biotechnology, 23, 9, 1105-1116 (2005).

The invention also relates to amino acid sequences that are obtained by the above methods, or alternatively by a method that comprises the one of the above methods and in addition at least the steps of determining the nucleotide sequence or amino acid sequence of said immunoglobulin sequence; and of expressing or synthesizing said amino acid sequence in a manner known per se, such as by expression in a suitable host cell or host organism or by chemical synthesis.

Also, following the steps above, one or more amino acid sequences of the invention may be suitably humanized (or alternatively camelized); and/or the amino acid sequence(s) thus obtained may be linked to each other or to one or more other suitable amino acid sequences (optionally via one or more suitable linkers) so as to provide a polypeptide of the invention. Also, a nucleic acid sequence encoding an amino acid sequence of the invention may be suitably humanized (or alternatively camelized) and suitably expressed; and/or one or more nucleic acid sequences encoding an amino acid sequence of the invention may be linked to each other or to one or more nucleic acid sequences that encode other suitable amino acid sequences (optionally via nucleotide sequences that encode one or more suitable linkers), after which the nucleotide sequence thus obtained may be suitably expressed so as to provide a polypeptide of the invention.

The invention further relates to applications and uses of the amino acid sequences, polypeptides, nucleic acids, host cells, products and compositions described herein, as well as to methods for the prevention and/or treatment for diseases and disorders associated with heterodimeric cytokines and/or their receptors. Some preferred but non-limiting applications and uses will become clear from the further description herein.

Some specific, preferred, but non-limiting aspects of the invention relate to:
1. A protein or polypeptide, comprising at least one amino acid sequence that is directed against the p19 subunit and at least one amino acid sequence that is directed against the p40 subunit, optionally linked via a suitable linker, and optionally comprising one or more further amino acid sequences, binding domains and/or binding units.
2. A protein or polypeptide according to aspect 1, in which the amino acid sequence that is directed against the p19 subunit is a p19+ sequence (i.e. an amino acid sequence that is capable of modulating, neutralizing, blocking and/or inhibiting the binding of a heterodimeric cytokines comprising a p19 subunit to its receptor), and in which the amino acid sequence that is directed against the p40 subunit is a p40+ sequence (i.e. an amino acid sequence that is capable of modulating, neutralizing, blocking and/or inhibiting the binding of a heterodimeric cytokines comprising a p40 subunit to its receptor).
3. A protein or polypeptide according to aspect 1, in which the amino acid sequence that is directed against the p19 subunit is a p19+ sequence (i.e. an amino acid sequence that is capable of modulating, neutralizing, blocking and/or inhibiting the binding of a heterodimeric cytokine comprising a p19 subunit to its receptor), and in which the amino acid sequence that is directed against the p40 subunit is a p40- sequence (i.e. an amino acid sequence that is essentially not capable of modulating, neutralizing, blocking and/or inhibiting the binding of a heterodimeric cytokines comprising a p40 subunit to its receptor).
4. A protein or polypeptide according to aspect 1, in which the amino acid sequence that is directed against the p19 subunit is a p19+ sequence (i.e. an amino acid sequence that is essentially not capable of modulating, neutralizing, blocking and/or inhibiting the binding of a heterodimeric cytokine comprising a p19 subunit to its receptor), and in which the amino acid sequence that is directed against the p40 subunit is a p40+ sequence (i.e. an amino acid sequence that is capable of modulating, neutralizing, blocking and/or inhibiting the binding of a heterodimeric cytokines comprising a p40 subunit to its receptor).
5. A protein or polypeptide, comprising at least one amino acid sequence that is directed against the p35 subunit and at least one amino acid sequence that is directed against the p40 subunit, optionally linked via a suitable linker, and optionally comprising one or more further amino acid sequences, binding domains and/or binding units.
6. A protein or polypeptide, comprising at least one amino acid sequence that is directed against a first epitope or antigenic determinant on the p19 subunit and at least one further amino acid sequence that is directed against a second epitope or antigenic determinant on the p19 subunit different from the first, optionally linked via a suitable linker, and optionally comprising one or more further amino acid sequences, binding domains and/or binding units.
7. A protein or polypeptide according to aspect 6, in which the first amino acid sequence is a p19+ sequence (i.e. an amino acid sequence that is essentially not capable of modulating, neutralizing, blocking and/or inhibiting the binding of a heterodimeric cytokine comprising a p19 subunit to its receptor), and in which the second amino acid sequence is a p19- sequence (i.e. an amino acid sequence that is essentially not capable of modulating, neutralizing, blocking and/or inhibiting the binding of a heterodimeric cytokine comprising a p19 subunit to its receptor).
8. A protein or polypeptide, comprising at least one amino acid sequence that is directed against a first epitope or antigenic determinant on the p40 subunit and at least one further amino acid sequence that is directed against a second epitope or antigenic determinant on the p40 subunit different from the first, optionally linked via a suitable linker, and optionally comprising one or more further amino acid sequences, binding domains and/or binding units.
9. A protein or polypeptide, in which the first amino acid sequence is a p40+ sequence (i.e. an amino acid sequence that is essentially not capable of modulating, neutralizing, blocking and/or inhibiting the binding of a heterodimeric cytokine comprising a p40 subunit to its receptor), and in which the second amino acid sequence is a p40- sequence (i.e. an amino acid sequence that is essentially not capable of modulating, neutralizing, blocking and/or inhibiting the binding of a heterodimeric cytokine comprising a p40 subunit to its receptor).
10. A protein or polypeptide according to any of aspects 1 to 9, in which each amino acid sequence that is comprised within said protein or polypeptide and that is directed against a subunit forms and/or essentially consist of a single (antigen) binding domain or binding unit, and/or is capable of forming and/or of functioning as a single (antigen) binding domain or binding unit (optionally after suitable folding).
11. A protein or polypeptide according to any of aspects 1 to 10, in which each amino acid sequence that is comprised within said protein or polypeptide and that is directed against a subunit comprises an immunoglobulin fold or is capable of, under suitable conditions, forming an immunoglobulin fold.
12. A protein or polypeptide according to any of aspects 1 to 11, in which each amino acid sequence that is comprised within said protein or polypeptide and that is directed against a subunit essentially consist of 4 framework regions (FR1 to FR4 respectively) and 3 complementarity determining regions.
13. A protein or polypeptide according to any of aspects 1 to 12, in which each amino acid sequence that is comprised within said protein or polypeptide and that is directed against a subunit is a domain antibody (or an amino acid sequence that is suitable for use as a domain antibody), a single domain antibody (or an amino acid sequence that is suitable for use as a single domain antibody), a "dAb" (or an amino acid sequence that is suitable for use as a dAb) or a Nanobody™ (including but not limited to a VHH sequence) or another single variable domain, or any suitable fragment of any one thereof.
14. A protein or polypeptide that is directed against a heterodimeric protein, polypeptide, ligand. or receptor that comprises:
   - at least a first subunit;
   - and
   - at least a second subunit;
   wherein said protein or polypeptide at least comprises a first binding domain or binding unit that is directed against said first subunit;and a second binding domain or binding unit that is directed against said second subunit.
15. A protein or polypeptide that is directed against a first heterodimeric protein, polypeptide, ligand or receptor that comprises:
   - at least a first subunit that is shared between said first heterodimeric protein, polypeptide, ligand or receptor and at least a second, different heterodimeric protein, polypeptide, ligand or receptor;
      and
   - at least a second subunit that is not shared between said first heterodimeric protein, polypeptide, ligand or receptor and said second, different heterodimeric protein, polypeptide, ligand or receptor;
   wherein said protein or polypeptide at least comprises a first binding domain or binding unit that is directed against said first (i.e. shared) subunit and a second binding domain or binding unit that is directed against said second (i.e. not shared) subunit.
16. A protein or polypeptide that is directed against a first heterodimeric protein, polypeptide, ligand or receptor that comprises:
   - at least a first subunit;
      and
   - at least a second subunit;
   wherein said protein or polypeptide at least comprises a first binding domain or binding unit that is directed against said first subunit and a second binding domain or binding unit different from said first binding domain or binding unit that is also directed against said first subunit, but against a different epitope, antigenic determinant or binding site on said first subunit.
17. A protein or polypeptide according to aspect 15 or 16, which is directed against a ligand for a receptor, and which comprises at least one binding domain or binding unit that is capable of modulating, neutralizing, blocking and/or inhibiting the binding of the ligand to its (cognate) receptor and at least one binding domain or binding unit that is essentially not capable of modulating, neutralizing, blocking and/or inhibiting the binding of the ligand to its (cognate) receptor.
18. A protein or polypeptide according to aspect 15, which is directed against a ligand for a receptor, in which both the first binding domain or binding unit as well as the second binding domain or binding unit are capable of modulating, neutralizing, blocking and/or inhibiting the binding of the ligand to its (cognate) receptor.
19. A protein or polypeptide according to any of aspects 14 to 18, in which each binding domain or binding unit comprises an immunoglobulin fold or is capable of, under suitable conditions, forming an immunoglobulin fold.
20. A protein or polypeptide according to any of aspects 14 to 19, in which each binding domain or binding unit essentially consist of 4 framework regions (FR1 to FR4 respectively) and 3 complementarity determining regions.
21. A protein or polypeptide according to any of aspects 14 to 20, in which each binding domain or binding unit is a domain antibody (or an amino acid sequence that is suitable for use as a domain antibody), a single domain antibody (or an amino acid sequence that is suitable for use as a single domain antibody), a "dAb" (or an amino acid sequence that is suitable for use as a dAb) or a Nanobody™ (including but not limited to a VHH sequence) or another single variable domain, or any suitable fragment of any one thereof.
22. A nucleotide sequence or nucleic acid encoding a protein or polypeptide according to any of aspects 1 to 21.
23. A composition comprising at least one protein or polypeptide according to any of aspects 1 to 21 ar a nucleotide sequence or nucleic acid according to aspect 22.
24. A pharmaceutical composition comprising at least one protein or polypeptide according to any of aspects 1 to 21 and at least one pharmaceutically acceptable carrier, diluent or excipient and/or adjuvant.
25. The use of (a nucleotide sequence and/or nucleic acid that encodes) a p19+ sequence in providing, constructing, and/or as part of (a nucleotide sequence and/or nucleic acid that encodes) a multivalent, multispecific and/or multiparatopic construct, protein and/or polypeptide that comprises said p19+ sequence (one or more) and one or more further binding domains or binding units.
26. The use of (a nucleotide sequence and/or nucleic acid that encodes) a p19- sequence in providing, constructing, and/or as part of (a nucleotide sequence and/or nucleic acid that encodes) a multivalent, multispecific and/or multiparatopic construct, protein and/or polypeptide that comprises said p19- sequence (one or more) and one or more further binding domains or binding units.
27. The use of (a nucleotide sequence and/or nucleic acid that encodes) a p40- sequence in providing, constructing, and/or as part of (a nucleotide sequence and/or nucleic acid that encodes) a multivalent, multispecific and/or multiparatopic construct, protein and/or polypeptide that comprises said p40- sequence (one or more) and one or more further binding domains or binding units.
28. The use of (a nucleotide sequence and/or nucleic acid that encodes) a p40+ sequence in providing, constructing, and/or as part of (a nucleotide sequence and/or nucleic acid that encodes) a multivalent, multispecific and/or multiparatopic construct, protein and/or polypeptide that comprises said p40+ sequence (one or more) and one or more further binding domains or binding units.
29. The use of (a nucleotide sequence and/or nucleic acid that encodes) a p35 sequence in providing, constructing, and/or as part of (a nucleotide sequence and/or nucleic acid that encodes) a multivalent, multispecific and/or multiparatopic construct, protein and/or polypeptide that comprises said p35 sequence (one or more) and one or more further binding domains or binding units.
30. The use according to any of aspects 25 to 29, in which the multivalent, multispecific and/or multiparatopic construct, protein and/or polypeptide is directed against a heterodimeric cytokine.
31. The use according to any of aspects 25 to 30, in which the construct, protein and/or polypeptide is a biparatopic construct, protein and/or polypeptide that is directed against one subunit of the heterodimeric cytokine.
32. The use according to any of aspects 30 and 31, in which the construct, protein and/or polypeptide is a multispecific construct, protein and/or polypeptide comprising at least one binding domain or binding unit that is directed against a first subunit of said heterodimeric cytokine and at least one binding domain or binding unit that is directed against a second subunit of said heterodimeric cytokine.
33. The use according to any of aspects 30 to 32, in which the construct, protein and/or polypeptide comprises at least one least one binding domain or binding unit that is capable of modulating, neutralizing, blocking and/or inhibiting the binding of the heterodimeric cytokine to its (cognate) receptor.
34. The use of (a nucleotide sequence and/or nucleic acid that encodes) an amino acid sequence that comprises or essentially consists of a single binding domain or binding unit in providing, constructing, and/or as part of (a nucleotide sequence and/or nucleic acid that encodes) a multispecific construct, protein and/or polypeptide that is directed against a heterodimeric protein, polypeptide, ligand or receptor, wherein said construct, protein and/or polypeptide comprises said amino acid sequence (one or more) and at least one further binding domain or binding unit, and wherein said one or more amino acid sequences are directed against a first subunit of the heterodimeric protein, polypeptide, ligand or receptor and at least one of said further binding domains or binding units is directed against a second subunit of the heterodimeric protein, polypeptide, ligand or receptor different from the first subunit.
35. The use according to aspect 34, in which the construct, protein and/or polypeptide is a directed against a heterodimeric cytokine or against a heterodimeric receptor for a heterodimeric cytokine.
36. The use of (a nucleotide sequence and/or nucleic acid that encodes) an amino acid sequence that comprises or essentially consists of a single binding domain or binding unit in providing, constructing, and/or as part of (a nucleotide sequence and/or nucleic acid that encodes) a biparatopic construct, protein and/or polypeptide that is directed against a heterodimeric protein, polypeptide, ligand or receptor, wherein said construct, protein and/or polypeptide comprises said amino acid sequence (one or more) and at least one further binding domain or binding unit, and wherein said one or more amino acid sequences are directed against a first subunit of the heterodimeric protein, polypeptide, ligand or receptor and at least one of said further binding domains or binding units is also directed against said first subunit, but to a different epitope or antigenic determinant on sais subunit.
37. The use according to aspect 36, in which the construct, protein and/or polypeptide is directed against a ligand for a receptor and comprises at least one binding domain or binding unit that is capable of modulating, neutralizing, blocking and/or inhibiting the binding of the ligand to its (cognate) receptor and at least one binding domain or binding unit that is essentially not capable of modulating, neutralizing, blocking and/or inhibiting the binding of the ligand to its (cognate) receptor.

Also, all the amino acid sequences of the invention (such as (single) domain antibodies and/or Nanobodies), constructs, polypeptides and proteins described herein (in all their various and/or preferred aspects), preferably have IC50 values as follows:
- when the amino acid sequence of the invention, protein or polypeptide is a monovalent amino acid sequence (as described herein) that is directed against p19 and that is a p19+ sequence, an IC50 value in the assay described in Example 25 (using human IL-23 at 19pM) of less than 100 nM, more preferably less than 50nM, even more preferably less than 10nM, such as less than 1 nM (for example, in the picomolar range);
- when the amino acid sequence of the invention, protein or polypeptide is a monovalent amino acid sequence (as described herein) that is directed against p40 and that is a p40+ sequence, an IC50 value in the assay described in Example 25 (using human IL-23 at 19pM) and/or in the assay described in Example 27 (using human IL-12 at IpM) of less than 100 nM, more preferably less than 50nM, even more preferably less than 10nM, such as less than 1 nM (for example, in the picomolar range);
- when the amino acid sequence of the invention, protein or polypeptide is a multivalent, multispecific and/or biparatopic construct that is directed against IL-23, and IC50 value in the assay described in Example 25 (using human IL-23 at 19pM) of less than 10 nM, more preferably less than 1nM, even more preferably less than 500pM, such as less than 100 pM (for example, in the 1-50 picomolar range);
- when the amino acid sequence of the invention, protein or polypeptide is a multivalent, multispecific and/or biparatopic construct that is directed against IL-12, and IC50 value in the assay described in Example 27 (using human IL-12 at 1 pM) of less than 10 nM, more preferably less than 1nM, even more preferably less than 500pM, such as less than 100 pM (for example, in the 1-50 picomolar range).

Other aspects, embodiments, advantages and applications of the invention will also become clear from the further description herein, in which the invention will be described and discussed in more detail with reference to the Nanobodies of the invention and polypeptides of the invention comprising the same, which form some of the preferred aspects of the invention.

As will become clear from the further description herein, Nanobodies generally offer certain advantages (outlined herein) compared to "dAb's" or similar (single) domain antibodies or immunoglobulin sequences, which advantages are also provided by the Nanobodies of the invention. However, it will be clear to the skilled person that the more general aspects of the teaching below can also be applied (either directly or analogously) to other amino acid sequences of the invention.

### Detailed description of the invention

In the present description, examples and claims:
a) Unless indicated or defined otherwise, all terms used have their usual meaning in the art, which will be clear to the skilled person. Reference is for example made to the standard handbooks, such as Sambrook et al, "Molecular Cloning: A Laboratory Manual" (2nd. Ed.), Vols, 1-3, Cold Spring Harbor Laboratory Press (1989); F. Ausubel et al, eds., "Current protocols in molecular biology", Green Publishing and Wiley Interscience, New York (1987); Lewin, "Genes II", John Wiley & Sons, New York, N.Y., (1985); Old et al., "Principles of Gene Manipulation: An Introduction to Genetic Engineering", 2nd edition, University of California Press, Berkeley, CA (1981); Roitt et al., "Immunology" (6th. Ed.), Mosby/Elsevier, Edinburgh (2001); Roitt et al., Roitt's Essential Immunology, 10th Ed. Blackwell Publishing, UK (2001); and Janeway et al., "Immunobiology", (6th Ed.), Garland Science Publishing/Churchill Livingstone, New York (2005), as well as to the general background art cited herein;
b) Unless indicated otherwise, the term "immunoglobulin sequence" - whether used herein to refer to a heavy chain antibody or to a conventional 4-chain antibody - is used as a general term to include both the full-size antibody, the individual chains thereof, as well as all parts, domains or fragments thereof (including but not limited to antigen-binding domains or fragment such as V_{HH} domains or V_{H}/V_{L} domains, respectively). In addition, the term "sequence" as used herein (for example in terms like "immunoglobulin sequence", "antibody sequence", "variable domain sequence", "V_{HH} sequence" or "protein sequence"), should generally be understood to include both the relevant amino acid sequence as well as nucleic acid sequences or nucleotide sequences encoding the same, unless the context requires a more limited interpretation;
c) Unless indicated otherwise, all methods, steps, techniques and manipulations that are not specifically described in detail can be performed and have been performed in a manner known per se, as will be clear to the skilled person. Reference is for example again made to the standard handbooks and the general background art mentioned herein and to the further references cited therein; as well as to for example the following reviews Presta, Adv. Drug Deliv. Rev. 2006, 58 (5-6): 640-56; Levin and Weiss, Mol. Biosyst. 2006, 2(1); 49-57; Irving et al., J. Immunol. Methods, 2001, 248(1-2), 31-45; Schmitz et al., Placenta, 2000,21 Suppl. A, S106-12, Gonzales et al.. Tumour Biol., 2005, 26(1), 31-43, which describe techniques for protein engineering, such as affinity maturation and other techniques for improving the specificity and other desired properties of proteins such as immunoglobulins.
d) Amino acid residues will be indicated according to the standard three-letter or one-letter amino acid code, as mentioned in Table A-3;

**Table A-3: one-letter and three-letter amino acid code**

| | | | | |
|---|---|---|---|---|
| Nonpolar, uncharged (at pH 6.0 -7.0)⁽³⁾ | | Alanine | Ala | A |
| | | Valine | Val | V |
| | | Leucine | Leu | L |
| | | Isoleucine | Ile | I |
| | | Phenylalanine | Phe | F |
| | | Methionine⁽¹⁾ | Met | M |
| | | Tryptophan | Trp | W |
| | | Proline | Pro | P |
| Polar, uncharged (at pH 6.0-7.0) | | Glycine⁽²⁾ | Gly | G |
| | | Serine | Ser | S |
| | | Threonine | Thr | T |
| | | Cysteine | Cys | C |
| | | Asparagine | Asn | N |
| | | Glutamine | Gln | Q |
| | | Tyrosine | Tyr | Y |
| Polar, charged (at pH 6.0-7.0) | | Lysine | Lys | K |
| | | Arginine | Arg | R |
| | | Histidine⁽⁴⁾ | His | H |
| | | Aspartate | Asp | D |
| | | Glutamate | Glu | E |
| | Notes: | | | |
| ⁽¹⁾ | Sometimes also considered to be a polar uncharged amino acid. | | | |
| ⁽²⁾ | Sometimes also considered to be a nonpolar uncharged amino acid. | | | |
| ⁽³⁾ | As will be clear to the skilled person, the fact that an amino acid residue is referred to in this Table as being either charged or uncharged at pH 6.0 to 7.0 does not reflect in any way on the charge said amino acid residue may have at a pH lower than 6.0 and/or at a pH higher than 7.0; the amino acid residues mentioned in the Table can be either charged and/or uncharged at such a higher or lower pH, as will be clear to the skilled person. | | | |
| ⁽⁴⁾ | As is known in the art, the charge of a His residue is greatly dependant upon even small shifts in pH, but a His residu can generally be considered essentially uncharged at a pH of about 6.5. | | | |

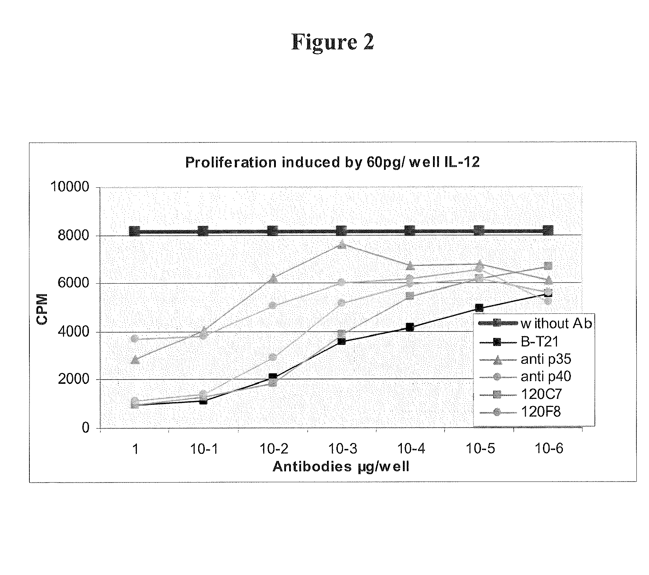

e) For the purposes of comparing two or more nucleotide sequences, the percentage of *"sequence identity"* between a first nucleotide sequence and a second nucleotide sequence may be calculated by dividing [*the number of nucleotides in the first nucleotide sequence that are identical to the nucleotides at the corresponding positions in the second nucleotide sequence]* by *[the total number of nucleotides in the first nucleotide sequence*] and multiplying by [*100%*], in which each deletion, insertion, substitution or addition of a nucleotide in the second nucleotide sequence - compared to the first nucleotide sequence - is considered as a difference at a single nucleotide (position).
Alternatively, the degree of sequence identity between two or more nucleotide sequences may be calculated using a known computer algorithm for sequence alignment such as NCBI Blast v2.0, using standard settings. Some other techniques, computer algorithms and settings for determining the degree of sequence identity are for example described in WO 04/037999, EP 0 967 284, EP 1 085 089, WO 00/55318, WO 00/78972, WO 98/49185 and GB 2 357 768-A.
Usually, for the purpose of determining the percentage of "sequence identity" between two nucleotide sequences in accordance with the calculation method outlined hereinabove, the nucleotide sequence with the greatest number of nucleotides will be taken as the "first"' nucleotide sequence, and the other nucleotide sequence will be taken as the "second" nucleotide sequence;
f) For the purposes of comparing two or more amino acid sequences, the percentage of "*sequence identity*" between a first amino acid sequence and a second amino acid sequence (also referred to herein as "*amino acid identity*") may be calculated by dividing [*the number of amino acid residues in the first amino acid sequence that are identical to the amino acid residues at the corresponding positions in the second amino acid sequence*] by [*the total number of amino acid residues in the first amino acid sequence* and multiplying by [*100*%], in which each deletion, insertion, substitution or addition of an amino acid residue in the second amino acid sequence - compared to the first amino acid sequence - is considered as a difference at a single amino acid residue (position), i.e. as an "amino acid difference" as defined herein.
Alternatively, the degree of sequence identity between two amino acid sequences may be calculated using a known computer algorithm, such as those mentioned above for determining the degree of sequence identity for nucleotide sequences, again using standard settings.
Usually, for the purpose of determining the percentage of "sequence *identity"* between two amino acid sequences in accordance with the calculation method outlined hereinabove, the amino acid sequence with the greatest number of amino acid residues will be taken as the "first" amino acid sequence, and the other amino acid sequence will be taken as the "second" amino acid sequence.
Also, in determining the degree of sequence identity between two amino acid sequences, the skilled person may take into account so-called "conservative" amino acid substitutions, which can generally be described as amino acid substitutions in which an amino acid residue is replaced with another amino acid residue of similar chemical structure and which has little or essentially no influence on the function, activity or other biological properties of the polypeptide. Such conservative amino acid substitutions are well known in the art, for example from WO 04/037999, GB-A-4 357 768, WO 98/49185, WO 00/46383 and WO 01/09300; and (preferred) types and/or combinations of such substitutions may be selected on the basis of the pertinent teachings from WO 04/037999 as well as WO 98/49185 and from the further reference cited therein.
Such conservative substitutions preferably are substitutions in which one amino acid within the following groups (a) -- (e) is substituted by another amino acid residue within the same group: (a) small aliphatic, nonpolar or slightly polar residues: Ala, Ser, Thr, Pro and Gly; (b) polar, negatively charged residues and their (uncharged) amides: Asp, Asn, Glu and Gln; (c) polar, positively charged residues: His, Arg and Lys; (d) large aliphatic, nonpolar residues: Met, Leu, Ile, Val and Cys; and (e) aromatic residues: Phe, Tyr and Trp.
Particularly preferred conservative substitutions are as follows; Ala into Gly or into Ser; Arg into Lys; Asn into Gln or into His; Asp into Glu; Cys into Ser; Gln into Asn; Glu into Asp; Gly into Ala or into Pro; His into Asn or into Gln; Ile into Leu or into Val; Leu into Ile or into Val; Lys into Arg, into Gln or into Glu; Met into Leu, into Tyr or into Ile; Phe into Met, into Leu or into Tyr; Ser into Thr; Thr into Ser; Trp into Tyr; Tyr into Trp; and/or Phe into Val, into Ile or into Leu.
Any amino acid substitutions applied to the polypeptides described herein may also be based on the analysis of the frequencies of amino acid variations between homologous proteins of different species developed by Schulz et al., Principles of Protein Structure, Springer-Verlag, 1978, on the analyses of structure forming potentials developed by Chou and Fasman, Biochemistry 13: 211, 1974 and Adv. Enzymol., 47: 45-149, 1978, and on the analysis of hydrophobicity patterns in proteins developed by Eisenberg et al., Proc. Nad. Acad Sci. USA 81: 140-144, 1984; Kyte & Doolittle; J Molec. Biol. 157: 105-132, 198 1, and Goldman et al., Ann. Rev. Biophys. Chem. 15: 321-353, 1986, all incorporated herein in their entirety by reference. Information on the primary, secondary and tertiary structure of Nanobodies is given in the description herein and in the general background art cited above. Also, for this purpose, the crystal structure of a V_{HH} domain from a Ilama is for example given by Desmyter et al., Nature Structural Biology, Vol. 3, 9, 803 (1996); Spinelli et al., Natural Structural Biology (1996); 3, 752 - 757; and Decanniere et al., Structure, Vol. 7, 4, 361 (1999). Further information about some of the amino acid residues that in conventional V_{H} domains form the V_{H}/V_{L} interface and potential camelizing substitutions on these position can be found in the prior art cited above.
g) Amino acid sequences and nucleic acid sequences are said to be *"exactely the same"* if they have 100% sequence identity (as defined herein) over their entire length;
h) When comparing two amino acid sequences, the term "*amino acid difference*" refers to an insertion, deletion or substitution of a single amino acid residue on a position of the first sequence, compared to the second sequence; it being understood that two amino acid sequences can contain one, two or more such amino acid differences;
i) When a nucleotide sequence or amino acid sequence is said to "comprise" another nucleotide sequence or amino acid sequence, respectively, or to "essentially consist of" another nucleotide sequence or amino acid sequence, this may mean that the latter nucleotide sequence or amino acid sequence has been incorporated into the firstmentioned nucleotide sequence or amino acid sequence, respectively, but more usually this generally means that the firstmentioned nucleotide sequence or amino acid sequence comprises within its sequence a stretch of nucleotides or amino acid residues, respectively, that has the same nucleotide sequence or amino acid sequence, respectively, as the latter sequence, irrespective of how the firstmentioned sequence has actually been generated or obtained (which may for example be by any suitable method described herein). By means of a non-limiting example, when a Nanobody of the invention is said to comprise a CDR sequence, this may mean that said CDR sequence has been incorporated into the Nanobody of the invention, but more usually this generally means that the Nanobody of the invention contains within its sequence a stretch of amino acid residues with the same amino acid sequence as said CDR sequence, irrespective of how said Nanobody of the invention has been generated or obtained. It should also be noted that when the latter amino acid sequence has a specific biological or structural function, it preferably has essentially the same, a similar or an equivalent biological or structural function in the firstmentioned amino acid sequence (in other words, the firstmentioned amino acid sequence is preferably such that the latter sequence is capable of performing essentially the same, a similar or an equivalent biological or structural function). For example, when a Nanobody of the invention is said to comprise a CDR sequence or framework sequence, respectively, the CDR sequence and framework are preferably capable, in said Nanobody, of functioning as a CDR sequence or framework sequence, respectively. Also, when a nucleotide sequence is said to comprise another nucleotide sequence, the firstmentioned nucleotide sequence is preferably such that, when it is expressed into an expression product (e.g. a polypeptide), the amino acid sequence encoded by the latter nucleotide sequence forms part of said expression product (in other words, that the latter nucleotide sequence is in the same reading frame as the firstmentioned, larger nucleotide sequence).
j) A nucleic acid sequence or amino acid sequence is considered to be "*(in) essentially isolated* (*form*)" *-* for example, compared to its native biological source and/or the reaction medium or cultivation medium from which it has been obtained - when it has been separated from at least one other component with which it is usually associated in said source or medium, such as another nucleic acid, another protein/polypeptide, another biological component or macromolecule or at least one contaminant, impurity or minor component. In particular, a nucleic acid sequence or amino acid sequence is considered "essentially isolated" when it has been purified at least 2-fold, in particular at least 10-fold, more in particular at least 100-fold, and up to 1000-fold or more. A nucleic acid sequence or amino acid sequence that is "in essentially isolated form" is preferably essentially homogeneous, as determined using a suitable technique, such as a suitable chromatographical technique, such as polyacrylamide-gel electrophoresis;
k) The term "*domain*" as used herein generally refers to a globular region of an amino acid sequence (such as an antibody chain, and in particular to a globular region of a heavy chain antibody), or to a polypeptide that essentially consists of such a globular region. Usually, such a domain will comprise peptide loops (for example 3 or 4 peptide loops) stabilized, for example, as a sheet or by disulfide bonds. The term "*binding domain*" refers to such a domain that is directed against an antigenic determinant (as defined herein);
l) The term "*antigenic determinant*" refers to the epitope on the antigen recognized by the antigen-binding molecule (such as a Nanobody or a polypeptide of the invention) and more in particular by the antigen-binding site of said molecule. The terms "*antigenic determinant*" and "*epitope*" may also be used interchangeably herein.
m) An amino acid sequence (such as a Nanobody, an antibody, a polypeptide of the invention, or generally an antigen binding protein or polypeptide or a fragment thereof) that can (specifically) bind to, that has affinity for and/or that has specificity for a specific antigenic determinant, epitope, antigen or protein (or for at least one part, fragment or epitope thereof) is said to be "*against*", "*directed against*" *or* "*directed to* " said antigenic determinant, epitope, antigen or protein.
n) The term *"specificity"* refers to the number of different types of antigens or antigenic determinants to which a particular antigen-binding molecule or antigen-binding protein (such as a Nanobody or a polypeptide of the invention) molecule can bind. The specificity of an antigen-binding protein can be determined based on affinity and/or avidity. The affinity, represented by the equilibrium constant for the dissociation of an antigen with an antigen-binding protein (K_{D}), is a measure for the binding strength between an antigenic determinant and an antigen-binding site on the antigen-binding protein: the lesser the value of the K_{D}, the stronger the binding strength between an antigenic determinant and the antigen-binding molecule (alternatively, the affinity can also be expressed as the affinity constant (K_{A}), which is 1/K_{D}). As will be clear to the skilled person (for example on the basis of the further disclosure herein), affinity can be determined in a manner known per se, depending on the specific antigen of interest. Avidity is the measure of the strength of binding between an antigen-binding molecule (such as a Nanobody or polypeptide of the invention) and the pertinent antigen. Avidity is related to both the affinity between an antigenic determinant and its antigen binding site on the antigen-binding molecule and the number of pertinent binding sites present on the antigen-binding molecule. Typically, antigen-binding proteins (such as the amino acid sequences, Nanobodies and/or polypeptides of the invention) will bind to their antigen with a dissociation constant (K_{D}) of 10⁻⁵ to 10⁻¹² moles/liter or less, and preferably 10⁻⁷ to 10⁻¹² moles/liter or less and more preferably 10⁻⁸ to 10⁻¹² moles/liter (i.e. with an association constant (K_{A}) of 10⁵ to 10¹² liter/ moles or more, and preferably 10⁷ to 10¹² liter/moles or more and more preferably 10⁸ to 10¹² liter/moles). Any K_{D} value greater than 10⁻⁴ mol/liter (or any K_{A} value lower than 10⁴ M⁻¹) liters/mol is generally considered to indicate non-specific binding. Preferably, a monovalent immunoglobulin sequence of the invention will bind to the desired antigen with an affinity less than 500 nM, preferably less than 200 nM, more preferably less than 10 nM, such as less than 500 pM. Specific binding of an antigen-binding protein to an antigen or antigenic determinant can be determined in any suitable manner known per se, including, for example, Scatchard analysis and/or competitive binding assays, such as radioimmunoassays (RIA), enzyme immunoassays (EIA) and sandwich competition assays, and the different variants thereof known per se in the art; as well as the other techniques mentioned herein.
The dissociation constant may be the actual or apparent dissociation constant, as will be clear to the skilled person. Methods for determining the dissociation constant will be clear to the skilled person, and for example include the techniques mentioned herein. In this respect, it will also be clear that it may not be possible to measure dissociation constants of more then 10⁻⁴ moles/liter or 10⁻³ moles/liter (e,g, of 10⁻² moles/liter). Optionally, as will also be clear to the skilled person, the (actual or apparent) dissociation constant may be calculated on the basis of the (actual or apparent) association constant (K_{A}), by means of the relationship [K_{D} = 1/K_{A}].
The affinity denotes the strength or stability of a molecular interaction. The affinity is commonly given as by the K_{D}, or dissociation constant, which has units of mol/liter (or M). The affinity can also be expressed as an association constant, K_{A}, which equals 1/K_{D} and has units of (mol/liter)⁻¹ (or M⁻¹). In the present specification, the stability of the interaction between two molecules (such as an amino acid sequence, Nanobody or polypeptide of the invention and its intended target) will mainly be expressed in terms of the K_{D} value of their interaction; it being clear to the skilled person that in view of the relation K_{A} =1/K_{D}, specifying the strength of molecular interaction by its K_{D} value can also be used to calculate the corresponding K_{A} value. The KD-value characterizes the strength of a molecular interaction also in a thermodynamic sense as it is related to the free energy (DG) of binding by the well known relation DG=RT.ln(K_{D}) (equivalently DG=-RT.ln(K_{A})), where R equals the gas constant, T equals the absolute temperature and in denotes the natural logarithm.
The K_{D} for biological interactions which are considered meaningful (e.g. specific) are typically in the range of 10⁻¹⁰M (0.1 nM) to 10⁻⁵M (10000 nM). The stronger an interaction is, the lower is its K_{D}.
The K_{D} can also be expressed as the ratio of the dissociation rate constant of a complex, denoted as k_{off}, to the rate of its association, denoted kₒₙ (so that K_{D} =k_{off}/kₒₙ and K_{A} = kₒₙ/k_{off}). The off-rate ko_{ff} has units s⁻¹ (where s is the SI unit notation of second). The on-rate kₒₙ has units M⁻¹ s⁻¹. The on-rate may vary between 10² M⁻¹s⁻¹ to about 10⁷ M⁻¹ s⁻¹, approaching the diffusion-limited association rate constant for bimolecular interactions. The off-rate is related to the half-life of a given molecular interaction by the relation t_{1/2}=ln(2)/k_{off}. The off-rate may vary between 1⁶ s⁻¹ (near irreversible complex with a t_{1/2} of multiple days) to 1 s⁻¹ (t_{1/2}=0.69 s).
The affinity of a molecular interaction between two molecules can be measured via different techniques known per se, such as the well known surface plasmon resonance (SPR) biosensor technique (see for example Ober et al., Intern. Immunology, 13, 1551-1559, 2001) where one molecule is immobilized on the biosensor chip and the other molecule is passed over the immobilized molecule under flow conditions yielding kₒₙ, k_{off} measurements and hence K_{D} (or K_{A}) values. This can for example be performed using the well-known Biacore instruments (see for example Example 12 or 20),
It will also be clear to the skilled person that the measured K_{D} may correspond to the apparent K_{D} if the measuring process somehow influences the intrinsic binding affinity of the implied molecules for example by artefacts related to the coating on the biosensor of one molecule. Also, an apparent K_{D} may be measured if one molecule contains more than one recognition sites for the other molecule. In such situation the measured affinity may be affected by the avidity of the interaction by the two molecules.
Another approach that may be used to assess affinity is the 2-step ELISA (Enzyme-Linked Immunosorbent Assay) procedure of Friguet et al. (J. Immunol. Methods, 77, 305-19, 1985). This method establishes a solution phase binding equilibrium measurement and avoids possible artefacts relating to adsorption of one of the molecules on a support such as plastic.
However, the accurate measurement of K_{D} may be quite labor-intensive and as consequence, often apparent K_{D} values are determined to assess the binding strength of two molecules. It should be noted that as long all measurements are made in a consistent way (e.g. keeping the assay conditions unchanged) apparent K_{D} measurements can be used as an approximation of the true K_{D} and hence in the present document K_{D} and apparent K_{D} should be treated with equal importance or relevance.
Finally, it should be noted that in many situations the experienced scientist may judge it to be convenient to determine the binding affinity relative to some reference molecule. For example, to assess the binding strength between molecules A and B, one may e.g. use a reference molecule C that is known to bind to B and that is suitably labelled with a fluorophore or chromophore group or other chemical moiety, such as biotin for easy detection in an ELISA or FACS (Fluorescent activated cell sorting) or other format (the fluorophore for fluorescence detection, the chromophore for light absorption detection, the biotin for streptavidin-mediated ELISA detection). Typically, the reference molecule C is kept at a fixed concentration and the concentration of A is varied for a given concentration or amount of B. As a result an IC₅₀ value is obtained corresponding to the concentration of A at which the signal measured for C in absence of A is halved. Provided K_{D ref}, the K_{D} of the reference molecule, is known, as well as the total concentration c_{ref} of the reference molecule, the apparent K_{D} for the interaction A-B can be obtained from following formula: K_{D} =IC₅₀/(1+_{Cref}/ K_{D ref}). Note that if _{Cref} <<K_{D ref}, K_{D} ≈ IC₅₀. Provided the measurement of the IC₅₀ is performed in a consistent way (e.g. keeping c_{ref} fixed) for the binders that are compared, the strength or stability of a molecular interaction can be assessed by the IC₅₀ and this measurement is judged as equivalent to K_{D} or to apparent K_{D} throughout this text.
o) The half-life of an Amino acid sequence, compound or polypeptide of the invention can generally be defined as the time taken for the serum concentration of the amino acid sequence, compound or polypeptide to be reduced by 50%, in vivo, for example due to degradation of the sequence or compound and/or clearance or sequestration of the sequence or compound by natural mechanisms. The in vivo half-life of an amino acid sequence, compound or polypeptide of the invention can be determined in any manner known per se, such as by pharmacokinetic analysis. Suitable techniques will be clear to the person skilled in the art, and may for example generally involve the steps of suitably administering to a warm-blooded animal (i.e. to a human or to another suitable mammal, such as a mouse, rabbit, rat, pig, dog or a primate, for example monkeys from the genus *Macaca* (such as, and in particular, cynomologus monkeys (*Macaca fascicular is*) and/or rhesus monkeys (*Macaca mulatta*)) and baboon (*Papio ursinus*)) a suitable dose of the amino acid sequence, compound or polypeptide of the invention; collecting blood samples or other samples from said animal; determining the level or concentration of the amino acid sequence, compound or polypeptide of the invention in said blood sample; and calculating, from (a plot of) the data thus obtained, the time until the level or concentration of the amino acid sequence, compound or polypeptide of the invention has been reduced by 50% compared to the initial level upon dosing. Reference is for example made to the Experimental Part below, as well as to Dennis et al., J. Biol. Chem 277:35035-42 (2002), and to the standard handbooks, such as Kenneth, A et al: Chemical Stability of Pharmaceuticals: A Handbook for Pharmacists and Peters et al, Pharmacokinete analysis: A Practical Approach (1996), Reference is also made to "Pharmacokmetics", M Gibaldi & D Perron, published by Marcel Dekker, 2nd Rev. edition (1982).
As will also be clear to the skilled person (see for example pages 6 and 7 of WO 04/003019 and in the further references cited therein), the half-life can be expressed using parameters such as the t1/2-alpha, t1/2-beta and the area under the curve (AUC). In the present specification, an "increase in half-life" refers to an increase in any one of these parameters, such as any two of these parameters, or essentially all three these parameters. As used herein "increase in half-life" or "increased half-life" in particular refers to an increase in the t1/2-beta, either with or without an increase in the t1/2-alpha and/or the AUC or both.
For example, the half-life of an amino acid sequence or polypeptide of the invention may be determined by means of a pharmacokinetic study, performed in a rodent or non-human primate model, as follows. Groups of animals (n=2-10) are given an intravenous bolus injection of 1mg/kg or 10 mg/kg 2D3-17D12 fusion protein. Plasma samples are obtained via a vein at different timepoints after dosing (eg. 1, 2,4, 6, 8, 12, 24,48,144, 192, 240, 288 and 336 h after dosing) and analyzed for the presence of the 2D3-17D12 fusion protein by ELISA. Plasma concentration versus time are fitted to a two-compartment elimination model. The pharmacokinetic parameters of clearance, V1, steady state volume (Vss), T½, AUC, and AUC corrected for actual dose administered (AUC/dose) are averaged for each treatment group. Differences between groups are determined by analysis of variance.
p) In the context of the present invention, "modulating" or "to modulate" generally means either reducing or inhibiting the activity of, or alternatively increasing the activity of, a target or antigen, as measured using a suitable in vitro, cellular or in vivo assay. In particular, "modulating" or "to modulate" may mean either reducing or inhibiting the activity of, or alternatively increasing a (relevant or intended) biological activity of, a target or antigen, as measured using a suitable in vitro, cellular or in vivo assay (which will usually depend on the target or antigen involved), by at least 1%, preferably at least 5%, such as at least 10% or at least 25%, for example by at least 50%, at least 60%, at least 70%, at least 80%, or 90% or more, compared to activity of the target or antigen in the same assay under the same conditions but without the presence of the construct of the invention.
As will be clear to the skilled person, "modulating" may also involve effecting a change (which may either be an increase or a decrease) in affinity, avidity, specificity and/or selectivity of a target or antigen for one or more of its ligands, binding partners, partners for association into a homomultimeric or heteromultimeric form, or substrates; and/or effecting a change (which may either be an increase or a decrease) in the sensitivity of the target or antigen for one or more conditions in the medium or surroundings in which the target or antigen is present (such as pH, ion strength, the presence of co-factors, etc.), compared to the same conditions but without the presence of the construct of the invention. As will be clear to the skilled person, this may again be determined in any suitable manner and/or using any suitable assay known per se, depending on the target or antigen involved.
"Modulating" may also mean effecting a change (i.e. an activity as an agonist, as an antagonist or as a reverse agonist, respectively, depending on the target or antigen and the desired biological or physiological effect) with respect to one or more biological or physiological mechanisms, effects, responses, functions, pathways or activities in which the target or antigen (or in which its substrate(s), ligand(s) or pathway(s) are involved, such as its signalling pathway or metabolic pathway and their associated biological or physiological effects) is involved. Again, as will be clear to the skilled person, such an action as an agonist or an antagonist may be determined in any suitable manner and/or using any suitable (in vitro and usually cellular or in assay) assay known per se, depending on the target or antigen involved. In particular, an action as an agonist or antagonist may be such that an intended biological or physiological activity is increased or decreased, respectively, by at least 1 %, preferably at least 5%, such as at least 10% or at least 25%, for example by at least 50%, at least 60%, at least 70%, at least 80%, or 90% or more, compared to the biological or physiological activity in the same assay under the same conditions but without the presence of the construct of the invention.
Modulating may for example also involve allosteric modulation of the target or antigen; and/or reducing or inhibiting the binding of the target or antigen to one of its substrates or ligands and/or competing with a natural ligand, substrate for binding to the target or antigen. Modulating may also involve activating the target or antigen or the mechanism or pathway in which it is involved. Modulating may for example also involve effecting a change in respect of the folding or confirmation of the target or antigen, or in respect of the ability of the target or antigen to fold, to change its confirmation (for example, upon binding of a ligand), to associate with other (sub)units, or to disassociate. Modulating may for example also involve effecting a change in the ability of the target or antigen to transport other compounds or to serve as a channel for other compounds (such as ions).
Modulating may be reversible or irreversible, but for pharmaceutical and pharmacological purposes will usually be in a reversible manner.
q) In respect of a target or antigen, the term "interaction site" on the target or antigen means a site, epitope, antigenic determinant, part, domain or stretch of amino acid residues on the target or antigen that is a site for binding to a ligand, receptor or other binding partner, a catalytic site, a cleavage site, a site for allosteric interaction, a site involved in multimerisation (such as homomerization or heterodimerization) of the target or antigen; or any other site, epitope, antigenic determinant, part, domain or stretch of amino acid residues on the target or antigen that is involved in a biological action or mechanism of the target or antigen. More generally, an "interaction site" can be any site, epitope, antigenic determinant, part, domain or stretch of amino acid residues on the target or antigen to which an amino acid sequence or polypeptide of the invention can bind such that the target or antigen (and/or any pathway, interaction, signalling, biological mechanism or biological effect in which the target or antigen is involved) is modulated (as defined herein).
r) An amino acid sequence or polypeptide is said to be "*specific for*" a first target or antigen compared to a second target or antigen when is binds to the first antigen with an affinity (as described above, and suitably expressed as a K_{D} value, K_{A} value, K_{off} rate and/or Kₒₙ rate) that is at least 10 times, such as at least 100 times, and preferably at least 1000 times, and up to 10.000 times or more better than the affinity with which said amino acid sequence or polypeptide binds to the second target or polypeptide. For example, the first antigen may bind to the target or antigen with a K_{D} value that is at least 10 times less, such as at least 100 times less, and preferably at least 1000 times less, such as 10.000 times less or even less than that, than the K_{D} with which said amino acid sequence or polypeptide binds to the second target or polypeptide. Preferably, when an amino acid sequence or polypeptide is "specific for" a first target or antigen compared to a second target or antigen, it is directed against (as defined herein) said first target or antigen, but not directed against said second target or antigen.
s) The terms "cross-block", "cross-blocked" and "cross-blocking", are used interchangeably herein to mean the ability of an amino acid sequence or other binding agents (such as a polypeptide of the invention) to interfere with the binding of other amino acid sequences or binding agents of the invention to a given target. The extend to which an amino acid sequence or other binding agents of the invention is able to interfere with the binding of another to [target], and therefore whether it can be said to cross-block according to the invention, can be determined using competition binding assays. One particularly suitable quantitative assay uses a Biacore machine which can measure the extent of interactions using surface plasmon resonance technology. Another suitable quantitative cross-blocking assay uses an ELISA-based approach to measure competition between amino acid sequence or another binding agents in terms of their binding to the target.
The following generally describes a suitable Biacore assay for determining whether an amino acid sequence or other binding agent cross-blocks or is capable of cross-blocking according to the invention. It will be appreciated that the assay can be used with any of the amino acid sequence or other binding agents described herein. The Biacore machine (for example the Biacore 3000) is operated in line with the manufacturer's recommendations. Thus in one cross-blocking assay, the target protein is coupled to a CM5 Biacore chip using standard amine coupling chemistry to generate a surface that is coated with the target. Typically 200- 800 resonance units of the target would be coupled to the chip (an amount that gives easily measurable levels of binding but that is readily saturable by the concentrations of test reagent being used), Two test amino acid sequences (termed A* and B*) to be assessed for their ability to cross-block each other are mixed at a one to one molar ratio of binding sites in a suitable buffer to create the test mixture. When calculating the concentrations on a binding site basis the molecular weight of an amino acid sequence is assumed to be the total molecular weight of the amino acid sequence divided by the number of target binding sites on that amino acid sequence. The concentration of each amino acid sequence in the test mix should be high enough to readily saturate the binding sites for that amino acid sequence on the target molecules captured on the Biacore chip. The amino acid sequences in the mixture are at the same molar concentration (on a binding basis) and that concentration would typically be between 1.00 and 1.5 micromolar (on a binding site basis). Separate solutions containing A* alone and B* alone are also prepared. A* and B* in these solutions should be in the same buffer and at the same concentration as in the test mix. The test mixture is passed over the target-coated Biacore chip and the total amount of binding recorded. The chip is then treated in such a way as to remove the bound amino acid sequences without damaging the chip-bound target. Typically this is done by treating the chip with 30 mM HCl for 60 seconds. The solution of A* alone is then passed over the target-coated surface and the amount of binding recorded. The chip is again treated to remove all of the bound amino acid sequences without damaging the chip-bound target. The solution of B* alone is then passed over the target-coated surface and the amount of binding recorded. The maximum theoretical binding of the mixture of A* and B* is next calculated, and is the sum of the binding of each amino acid sequence when passed over the target surface alone. If the actual recorded binding of the mixture is less than this theoretical maximum then the two amino acid sequences are cross-blocking each other. Thus, in general, a cross-blocking amino acid sequence or other binding agent according to the invention is one which will bind to the target in the above Biacore cross-blocking assay such that during the assay and in the presence of a second amino acid sequence or other binding agent of the invention the recorded binding is between 80% and 0.1% (e.g. 80% to 4%) of the maximum theoretical binding, specifically between 75% and 0.1% (e.g. 75% to 4%) of the maximum theoretical binding, and more specifically between 70% and 0.1% (e.g. 70% to 4%) of maximum theoretical binding (as just defined above) of the two amino acid sequences or binding agents in combination. The Biacore assay described above is a primary assay used to determine if amino acid sequences or other binding agents cross-block each other according to the invention. On rare occasions particular amino acid sequences or other binding agents may not bind to target coupled via amine chemistry to a CM5 Biacore chip (this usually occurs when the relevant binding site on target is masked or destroyed by the coupling to the chip). In such cases cross-blocking can be determined using a tagged version of the target, for example a N-terminal His-tagged version. In this particular format, an anti-His amino acid sequence would be coupled to the Biacore chip and then the His-tagged target would be passed over the surface of the chip and captured by the anti-His amino acid sequence. The cross blocking analysis would be carried out essentially as described above, except that after each chip regeneration cycle, new His-tagged target would be loaded back onto the anti-His amino acid sequence coated surface. In addition to the example given using N-terminal His-tagged [target], C-terminal His-tagged target could alternatively be used. Furthermore, various other tags and tag binding protein combinations that are known in the art could be used for such a cross-blocking analysis (e.g. HA tag with anti-HA antibodies; FLAG tag with anti-FLAG antibodies; biotin tag with streptavidin).
The following generally describes an ELISA assay for determining whether an amino acid sequence or other binding agent directed against a target cross-blocks or is capable of cross-blocking as defined herein. It will be appreciated that the assay can be used with any of the amino acid sequences (or other binding agents such as polypeptides of the invention) described herein. The general principal of the assay is to have an amino acid sequence or binding agent that is directed against the target coated onto the wells of an ELISA plate. An excess amount of a second, potentially cross-blocking, anti-target amino acid sequence is added in solution (i.e. not bound to the ELISA plate). A limited amount of the target is then added to the wells. The coated amino acid sequence and the amino acid sequence in solution compete for binding of the limited number of target molecules. The plate is washed to remove excess target that has not been bound by the coated amino acid sequence and to also remove the second, solution phase amino acid sequence as well as any complexes formed between the second, solution phase amino acid sequence and target. The amount of bound target is then measured using a reagent that is appropriate to detect the target. An amino acid sequence in solution that is able to cross-block the coated amino acid sequence will be able to cause a decrease in the number of target molecules that the coated amino acid sequence can bind relative to the number of target molecules that the coated amino acid sequence can bind in the absence of the second, solution phase, amino acid sequence. In the instance where the first amino acid sequence, e.g, an Ab-X, is chosen to be the immobilized amino acid sequence, it is coated onto the wells of the ELISA plate, after which the plates are blocked with a suitable blocking solution to minimize non-specific binding of reagents that are subsequently added. An excess amount of the second amino acid sequence, i.e. Ab-Y, is then added to the ELISA plate such that the moles of Ab-Y [target] binding sites per well are at least 10 fold higher than the moles of Ab-X [target] binding sites that were used, per well, during the coating of the ELISA plate. [target] is then added such that the moles of [target] added per well are at least 25-fold lower than the moles of Ab-X [target] binding sites that were used for coating each well. Following a suitable incubation period the ELISA plate is washed and a reagent for detecting the target is added to measure the amount of target specifically bound by the coated anti-[target] amino acid sequence (in this case Ab-X). The background signal for the assay is defined as the signal obtained in wells with the coated amino acid sequence (in this case Ab-X), second solution phase amino acid sequence (in this case Ab-Y), [target] buffer only (i.e. no target) and target detection reagents. The positive control signal for the assay is defined as the signal obtained in wells with the coated amino acid sequence (in this case Ab-X), second solution phase amino acid sequence buffer only (i.e. no second solution phase amino acid sequence), target and target detection reagents. The ELISA assay may be run in such a manner so as to have the positive control signal be at least 6 times the background signal. To avoid any artefacts (e.g. significantly different affinities between Ab-X and Ab-Y for [target]) resulting from the choice of which amino acid sequence to use as the coating amino acid sequence and which to use as the second (competitor) amino acid sequence, the cross-blocking assay may to be run in two formats: 1) format 1 is where Ab-X is the amino acid sequence that is coated onto the ELISA plate and Ab-Y is the competitor amino acid sequence that is in solution and 2) format 2 is where Ab-Y is the amino acid sequence that is coated onto the ELISA plate and Ab-X is the competitor amino acid sequence that is in solution. Ab-X and Ab-Y are defined as cross-blocking if, either in format 1 or in format 2, the solution phase anti-target amino acid sequence is able to cause a reduction of between 60% and 100%, specifically between 70% and 100%, and more specifically between 80% and 100%, of the target detection signal {i.e. the amount of target bound by the coated amino acid sequence) as compared to the target detection signal obtained in the absence of the solution phase anti- target amino acid sequence (i.e. the positive control wells).
t) An amino acid sequence is said to be "*cross-reactive*" for two different antigens or antigenic determinants (such as serum albumin from two different species of mammal, such as human serum albumin and cyno serum albumin) if it is specific for (as defined herein) both these different antigens or antigenic determinants.
u) By binding that. is "*essentially independent of the pH*" is generally meant herein that the association constant (K_{A}) of the amino acid sequence with respect to the serum protein (such as serum albumin) at the pH value(s) that occur in a cell of an animal or human body (as further described herein) is at least 5%, such as at least 10%, preferably at least 25%, more preferably at least 50%, even more preferably at least 60%, such as even more preferably at least 70%, such as at least 80% or 90% or more (or even more than 100%, such as more than 110%, more than 120% or even 130% or more, or even more than 150%, or even more than 200%) of the association constant (K_{A}) of the amino acid sequence with respect to the same serum protein at the pH value(s) that occur outside said cell. Alternatively, by binding that is "*essentially independent of the pH*" is generally meant herein that the k_{off} rate (measured by Biacore) of the amino acid sequence with respect to the serum protein (such as serum albumin) at the pH value(s) that occur in a cell of an animal or human body (as e.g. further described herein, e.g. pH around 5.5, e.g. 5.3 to 5.7) is at least 5%, such as at least 10%, preferably at least 25%, more preferably at least 50%, even more preferably at least 60%, such as even more preferably at least 70%, such as at least 80% or 90% or more (or even more than 100%, such as more than 110%, more than 120% or even 130% or more, or even more than 150%, or even more than 200%) of the k_{off} rate of the amino acid sequence with respect to the same serum protein at the pH value(s) that occur outside said cell, e.g. pH 7.2 to 7.4. By "*the pH value(s) that occur in a cell of an animal or human body"* is meant the pH value(s) that may occur inside a cell, and in particular inside a cell that is involved in the recycling of the serum protein. In particular, by "*the pH value(s) that occur in a cell of an animal or human body*" is meant the pH value(s) that may occur inside a (sub)cellular compartment or vesicle that is involved in recycling of the serum protein (e.g. as a result of pinocytosis, endocytosis, transcytosis, exocytosis and phagocytosis or a similar mechanism of uptake or internalization into said cell), such as an endosome, lysosome or pinosome.
v) As further described herein, the total number of amino acid residues in a Nanobody can be in the region of 110-120, is preferably 112-115, and is most preferably 113. It should however be noted that parts, fragments, analogs or derivatives (as further described herein) of a Nanobody are not particularly limited as to their length and/or size, as long as such parts, fragments, analogs, or derivatives meet the further requirements outlined herein and are also preferably suitable for the purposes described herein;
w) The amino acid residues of a Nanobody are numbered according to the general numbering for V_{H} domains given by Kabat et al. ("Sequence of proteins of immunological interest", US Public Health Services, NIH Bethesda, MD, Publication No. 91), as applied to V_{HH} domains from Camelids in the article of Riechmann and Muyldermans, J. Immunol. Methods 2000 Jun 23; 240 (1-2): 185-195 (see for example Figure 2 of this publication); or referred to herein. According to this numbering, FR1 of a Nanobody comprises the amino acid residues at positions 1-30, CDR1 of a Nanobody comprises the amino acid residues at positions 31-35, FR2 of a Nanobody comprises the amino acids at positions 36-49, CDR2 of a Nanobody comprises the amino acid residues at positions 50-65, FR3 of a Nanobody comprises the amino acid residues at positions 66-94, CDR3 of a Nanobody comprises the amino acid residues at positions 95-102, and FR4 of a Nanobody comprises the amino acid residues at positions 103-113. [In this respect, it should be noted that - as is well known in the art for V_{H} domains and for V_{HH} domains - the total number of amino acid residues in each of the CDR's may vary and may not correspond to the total number of amino acid residues indicated by the Kabat numbering (that is, one or more positions according to the Kabat numbering may not be occupied in the actual sequence, or the actual sequence may contain more amino acid residues than the number allowed for by the Kabat numbering). This means that, generally, the numbering according to Kabat may or may not correspond to the actual numbering of the amino acid residues in the actual sequence. Generally, however, it can be said that, according to the numbering of Kabat and irrespective of the number of amino acid residues in the CDR's, position 1 according to the Kabat numbering corresponds to the start of FR1 and vice versa, position 36 according to the Kabat numbering corresponds to the start of FR2 and vice versa, position 66 according to the Kabat numbering corresponds to the start of FR3 and vice versa, and position 103 according to the Kabat numbering corresponds to the start of FR4 and vice versa.].
Alternative methods for numbering the amino acid residues of V_{H} domains, which methods can also be applied in an analogous manner to V_{HH} domains from Camelids and to Nanobodies, are the method described by Chothia et al. (Nature 342, 877-883 (1989)), the so-called "AbM definition" and the so-called "contact definition". However, in the present description, claims and Figures, the numbering according to Kabat as applied to V_{HH} domains by Riechmann and Muyldermans will be followed, unless indicated otherwise; and
x) In respect of any amino acid sequence described herein that is a CDR sequence (such as any of the CDR sequences from the CDR1 Sequences Groups 2, 9, 16, 23, 30, 37, 44, 51 and/or 58 (see Table A-1); from the CDR2 Sequences Groups 4, 11, 18, 25, 32, 39, 46, 53, and/or 60 (see Table A-1); and/or from the CDR3 Sequences Groups 6,13, 20, 27, 34, 41, 48, 54 and/or 62 (see Table A-1)), *"Optional Condition I"* means that when said amino acid sequence contains an amino acid substitution, such an amino acid substitution is preferably, and compared to the original amino acid sequence without said substitution, a conservative amino acid substitution (as defined herein): *"Optional Condition II"* means that said preferably only contains amino acid substitutions, and no amino acid deletions or insertions, compared to the original amino acid sequence without said substitution; *"Optional Condition III"* means that said amino acid sequence may be an amino acid sequence that is derived from the corresponding amino acid sequence by means of affinity maturation using one or more techniques of affinity maturation known per se.
y) In respect of any amino acid sequence described herein that is a framework sequence, *"Optional Condition I"* means that when said amino acid sequence contains an amino acid substitution, such an amino acid substitution is preferably, and compared to the original amino acid sequence without said substitution, a conservative amino acid substitution (as defined herein): *"Optional Condition II"* means that said preferably only contains amino acid substitutions, and no amino acid deletions or insertions, compared to the original amino acid sequence without said substitution; and *"Optional Condition IV"* means that where such an amino acid sequence contains any amino acid differences, these amino acid differences are preferably not present at one of the Hallmark Residues (although the presence of an amino acid difference at a position of a Hallmark Residue is not excluded, provided the favourable properties of a VHH or nanobody as described herein are essentially maintained or not affected to an extent that would make the resulting amino acid sequence no longer suitable for use as a single antigen binding domain or unit (for example, as a nanobody);
z) The Figures, Sequence Listing and the Experimental Part/Examples are only given to further illustrate the invention and should not be interpreted or construed as limiting the scope of the invention and/or of the appended claims in any way, unless explicitly indicated otherwise herein. Also, generally, the amino acid sequences and polypeptides of the invention that are explicitly referred to in the Experimental Part are preferered examples of amino acid sequences and polypeptides of the invention. Further preferences from within these amino acid sequences and polypeptides will become clear from the data presented in the Experimental Part,

For a general description of heavy chain antibodies and the variable domains thereof, reference is inter alia made to the prior art cited herein, to the review article by Muyldermans in Reviews in Molecular Biotechnology 74(2001), 277-302; as well as to the following patent applications, which are mentioned as general background art: WO 94/04678, WO 95/04079 and WO 96/34103 of the Vrije Universiteit Brussel; WO 94/25591, WO 99/37681, WO 00/40968, WO 00/43507, WO 00/65057, WO 01/40310, WO 01/44301, EP 1134231 and WO 02/48193 of Unilever; WO 97/49805, WO 01/21817, WO 03/035694, WO 03/054016 and WO 03/055527 of the Vlaams Instituut voor Biotechnologie (VIB); WO 03/050531 of Algonomics N.V. and Ablynx N.V.; WO 01/90190 by the National Research Council of Canada; WO 03/025020 (= EP 1 433 793) by the Institute of Antibodies; as well as WO 04/041867, WO 04/041862, WO 04/041865, WO 04/041863, WO 04/062551, WO 05/044858, WO 06/40153, WO 06/079372, WO 06/122786, WO 06/122787 and WO 06/122825, by Ablynx N.V. and the further published patent applications by Ablynx N.V. Reference is also made to the further prior art mentioned in these applications, and in particular to the list of references mentioned on pages 41-43 of the International application WO 06/040153, which list and references are incorporated herein by reference.

In accordance with the terminology used in the art (see the above references), the variable domains present in naturally occurring heavy chain antibodies will also be referred to as *"V_{HH} domains",* in order to distinguish them from the heavy chain variable domains that are present in conventional 4-chain antibodies (which will be referred to hereinbelow as "*V_{H} domains*") and from the light chain variable domains that are present in conventional 4-chain antibodies (which will be referred to hereinbelow as *"V_{L} domains*").

As mentioned in the prior art referred to above, V_{HH} domains have a number of unique structural characteristics and functional properties which make isolated V_{HH} domains (as well as Nanobodies based thereon, which share these structural characteristics and functional properties with the naturally occurring V_{HH} domains) and proteins containing the same highly advantageous for use as functional antigen-binding domains or proteins. In particular, and without being limited thereto, V_{HH} domains (which have been "designed" by nature to functionally bind to an antigen without the presence of, and without any interaction with, a light chain variable domain) and Nanobodies can function as a single, relatively small, functional antigen-binding structural unit, domain or protein. This distinguishes the V_{HH} domains from the V_{H} and V_{L} domains of conventional 4-chain antibodies, which by themselves are generally not suited for practical application as single antigen-binding proteins or domains, but need to be combined in some form or another to provide a functional antigen-binding unit (as in for example conventional antibody fragments such as Fab fragments; in ScFv's fragments, which consist of a V_{H} domain covalently linked to a V_{L} domain).

Because of these unique properties, the use of V_{HH} domains and Nanobodies as single antigen-binding proteins or as antigen-binding domains (i.e. as part of a larger protein or polypeptide) offers a number of significant advantages over the use of conventional V_{H} and V_{L} domains, scFv's or conventional antibody fragments (such as Fab- or F(ab')₂-fragments):
- only a single domain is required to bind an antigen with high affinity and with high selectivity, so that there is no need to have two separate domains present, nor to assure that these two domains are present in the right spacial conformation and configuration (i.e. through the use of especially designed linkers, as with scFv's);
- V_{HH} domains and Nanobodies can be expressed from a single gene and require no post-translational folding or modifications;
- V_{HH} domains and Nanobodies can easily be engineered into multivalent and multispecific formats (as further discussed herein);
- V_{HH} domains and Nanobodies are highly soluble and do not have a tendency to aggregate (as with the mouse-derived "dAb's" described by Ward et al., Nature, Vol. 341, 1989, p. 544);
- V_{HH} domains and Nanobodies are highly stable to heat, pH, proteases and other denaturing agents or conditions (see for example Ewert et al, supra);
- V_{HH} domains and Nanobodies are easy and relatively cheap to prepare, even on a scale required for production. For example, V_{HH} domains, Nanobodies and proteins/polypeptides containing the same can be produced using microbial fermentation (e.g. as further described below) and do not require the use of mammalian expression systems, as with for example conventional antibody fragments;
- V_{HH} domains and Nanobodies are relatively small (approximately 15 kDa, or 10 times smaller than a conventional IgG) compared to conventional 4-chain antibodies and antigen-binding fragments thereof, and therefore show high(er) penetration into tissues (including but not limited to solid tumors and other dense tissues) than such conventional 4-chain antibodies and antigen-binding fragments thereof;
- V_{HH} domains and Nanobodies can show so-called cavity-binding properties (inter alia due to their extended CDR3 loop, compared to conventional V_{H} domains) and can therefore also access targets and epitopes not accessable to conventional 4-chain antibodies and antigen-binding fragments thereof. For example, it has been shown that V_{HH} domains and Nanobodies can inhibit enzymes (see for example WO 97/49805; Transue et al., Proteins 1998 Sep 1; 32(4): 515-22; Lauwereys et al., EMBO J. 1998 Jul 1;17(13):3512-20).

In a specific and preferred aspect, the invention provides Nanobodies against heterodimeric cytokines and/or their receptors, and in particular Nanobodies against heterodimeric cytokine and/or their receptors from a warm-blooded animal, and more in particular Nanobodies against heterodimeric cytokines and/or their receptors from a mammal, and especially Nanobodies against human heterodimeric cytokines and/or their receptors; as well as proteins and/or polypeptides comprising at least one such Nanobody.

In particular, the invention provides Nanobodies against heterodimeric cytokine and/or their receptors, and proteins and/or polypeptides comprising the same, that have improved therapeutic and/or pharmacological properties and/or other advantageous properties (such as, for example, improved ease of preparation and/or reduced costs of goods), compared to conventional antibodies against heterodimeric cytokines and/or their receptors or fragments thereof, compared to constructs that could be based on such conventional antibodies or antibody fragments (such as Fab' fragments, F(ab')₂ fragments, ScFv constructs, "diabodies" and other multispecific constructs (see for example the review by Holliger and Hudson, Nat Biotechnol. 2005 Sep;23(9):1126-36)), and also compared to the so-called "dAb's" or similar (single) domain antibodies that may be derived from variable domains of conventional antibodies. These improved and advantageous properties will become clear from the further description herein, and for example include, without limitation, one or more of:
- increased affinity and/or avidity for heterodimeric cytokines and/or their receptors, either in a monovalent format, in a multivalent format (for example in a bivalent format) and/or in a multispecific format (for example one of the multispecific formats described hereinbelow);
- better suitability for formatting in a multivalent format (for example in a bivalent format);
- better suitability for formatting in a multispecific format (for example one of the multispecific formats described hereinbelow);
- improved suitability or susceptibility for "humanizing" substitutions (as defined herein);
- less immunogenicity, either in a monovalent format, in a multivalent format (for example in a bivalent format) and/or in a multispecific format (for example one of the multispecific formats described hereinbelow);
- increased stability, either in a monovalent format, in a multivalent format (for example in a bivalent format) and/or in a multispecific format (for example one of the multispecific formats described hereinbelow);
- increased specificity towards heterodimeric cytokines and/or their receptors, either in a monovalent format, in a multivalent format (for example in a bivalent format) and/or in a multispecific format (for example one of the multispecific formats described hereinbelow);
- decreased or where desired increased cross-reactivity with heterodimeric cytokines and/or their receptors from different species;
   and/or
- one or more other improved properties desirable for pharmaceutical use (including prophylactic use and/or therapeutic use) and/or for diagnostic use (including but not limited to use for imaging purposes), either in a monovalent format, in a multivalent format (for example in a bivalent format) and/or in a multispecific format (for example one of the multispecific formats described hereinbelow).

As generally described herein for the amino acid sequences of the invention, the Nanobodies of the invention are preferably in essentially isolated form (as defined herein), or form part of a protein or polypeptide of the invention (as defined herein), which may comprise or essentially consist of one or more Nanobodies of the invention and which may optionally further comprise one or more further amino acid sequences (all optionally linked via one or more suitable linkers). For example, and without limitation, the one or more amino acid sequences of the invention may be used as a binding unit in such a protein or polypeptide, which may optionally contain one or more further amino acid sequences that can serve as a binding unit (i.e. against one or more other targets than heterodimeric cytokine and/or their receptors), so as to provide a monovalent, multivalent or multispecific polypeptide of the invention, respectively, all as described herein. In particular, such a protein or polypeptide may comprise or essentially consist of one or more Nanobodies of the invention and optionally one or more (other) Nanobodies (i.e. directed against other targets than heterodimeric cytokines and/or their receptors), all optionally linked via one or more suitable linkers, so as to provide a monovalent, multivalent or multispecific Nanobody construct, respectively, as further described herein. Such proteins or polypeptides may also be in essentially isolated form (as defined herein).

In a Nanobody of the invention, the binding site for binding against heterodimeric cytokines and/or their receptors is preferably formed by the CDR sequences. Optionally, a Nanobody of the invention may also, and in addition to the at least one binding site for binding against heterodimeric cytokines and/or their receptors, contain one or more further binding sites for binding against other antigens, proteins or targets. For methods and positions for introducing such second binding sites, reference is for example made to Keck and Huston, Biophysical Journal, 71, October 1996, 2002-2011; EP 0 640 130; WO 06/07260.

As generally described herein for the amino acid sequences of the invention, when a Nanobody of the invention (or a polypeptide of the invention comprising the same) is intended for administration to a subject (for example for therapeutic and/or diagnostic purposes as described herein), it is preferably directed against human heterodimeric cytokines and/or their receptors; whereas for veterinary purposes, it is preferably directed against heterodimeric cytokines and/or their receptors from the species to be treated. Also, as with the amino acid sequences of the invention, a Nanobody of the invention may or may not be cross-reactive (i.e. directed against heterodimeric cytokine and/or their receptors from two or more species of mammal, such as against human heterodimeric cytokines and/or their receptors and heterodimeric cytokines and/or their receptors from at least one of the species of mammal mentioned herein).

Also, again as generally described herein for the amino acid sequences of the invention, the Nanobodies of the invention may generally be directed against any antigenic determinant, epitope, part, domain, subunit or confirmation (where applicable) of heterodimeric cytokines and/or their receptors, such as an interaction site (as defined herein) or a site, antigenic determinant, epitope, part, domain that is not an interaction site.

As already described herein, the amino acid sequence and structure of a Nanobody can be considered - without however being limited thereto - to be comprised of four framework regions or "FR's" (or sometimes also referred to as "FW's"), which are referred to in the art and herein as "Framework region 1" or "FR1"; as "Framework region 2" or "FR2"; as "Framework region 3" or "FR3"; and as "Framework region 4" or "FR4", respectively; which framework regions are interrupted by three complementary determining regions or "CDR's", which are referred to in the art as "Complementarity Determining Region 1" or "CDR1"; as "Complementarity Determining Region 2" or "CDR2"; and as "Complementarity Determining Region 3" or "CDR3", respectively. Some preferred framework sequences and CDR's (and combinations thereof) that are present in the Nanobodies of the invention are as described herein. Other suitable CDR sequences can be obtained by the methods described herein.

According to a non-limiting but preferred aspect of the invention, (the CDR sequences present in) the Nanobodies of the invention are such that:
- the Nanobodies can bind to heterodimeric cytokines and/or their receptors with a dissociation constant (K_{D}) of 10⁻⁵ to 10⁻¹² moles/liter or less, and preferably 10⁻⁷ to 10⁻¹² moles/liter or less and more preferably 10⁻⁸ to 10⁻¹² moles/liter (i.e. with an association constant (K_{A}) of 10⁵ to 10¹² liter/ moles or more, and preferably 10⁷ to 10¹² liter/moles or more and more preferably 10⁸ to 10¹² liter/moles);
   and/or such that:
- the Nanobodies can bind to heterodimeric cytokines and/or their receptors with a kₒₙ-rate of between 10² M⁻¹s⁻¹ to about 10⁷ M⁻¹s⁻¹, preferably between 10³ M⁻¹s⁻¹ and 10⁷ M⁻¹s⁻¹, more preferably between 10⁴ M⁻¹s⁻¹ and 10⁷ M⁻¹s⁻¹, such as between 10⁵ M⁻¹s⁻¹ and 10⁷ M⁻¹s⁻¹;
   and/or such that they:
- the Nanobodies can bind to heterodimeric cytokines and/or their receptors with a k_{off} rate between 1s⁻¹ (t_{1/2}=0.69 s) and 10⁻⁶ s⁻¹ (providing a near irreversible, complex with a t_{1/2} of multiple days), preferably between 10⁻² s⁻¹ and 10⁻⁶ s⁻¹, more preferably between 10⁻³ s⁻¹ and 10⁻⁶ s⁻¹, such as between 10⁻⁴ s⁻¹ and 10⁻⁶ s⁻¹,

Preferably, (the CDR sequences present in) the Nanobodies of the invention are such that: a monovalent Nanobody of the invention (or a polypeptide that contains only one Nanobody of the invention) is preferably such that it will bind to heterodimeric cytokines and/or their receptors with an affinity less than 500 nM, preferably less than 200 nM, more preferably less than 10 nM, such as less than 500 pM.

The affinity of the Nanobody of the invention against heterodimeric cytokines and/or their receptors can be determined in a manner known per se, for example using the general techniques for measuring K_{D}. K_{A}, k_{off} or kₒₙ mentioned herein, as well as some of the specific assays described herein.

Some preferred IC50 values for binding of the Nanobodies of the invention (and of polypeptides comprising the same) to heterodimeric cytokines and/or their receptors will become clear from the further description and examples herein.

In a preferred but non-limiting aspect, the invention relates to a (single) domain antibody and/or a Nanobody (as defined herein) which is a p19+ sequence (as defined herein), which consists of 4 framework regions (FR1 to FR4 respectively) and 3 complementarity determining regions (CDR1 to CDR3 respectively), in which:
- CDR1 is chosen from the group consisting of:
   a) the amino acid sequences from the CDR1 Sequences Group 2 (see Table A-1 and Figure 11);
   b) amino acid sequences that have at least 80% amino acid identity with at least one of the amino acid sequences from the CDR1 Sequences Group 2;
   c) amino acid sequences that have 3, 2, or 1 amino acid difference with at least one of the amino acid sequences from the CDR1 Sequences Group 2;
   and/or
- CDR2 is chosen from the group consisting of:
   d) the amino acid sequences from the CDR2 Sequences Group 4;
   e) amino acid sequences that have at least 80% amino acid identity with at least one of the amino acid sequences from the CDR2 Sequences Group 4;
   f) amino acid sequences that have 3, 2, or 1 amino acid difference with at least one of the amino acid sequences from the CDR2 Sequences Group 4;
   and/or
- CDR3 is chosen from the group consisting of:
   g) the amino acid sequences from the CDR3 Sequences Group 6;
   h) amino acid sequences that have at least 80% amino acid identity with at least one of the amino acid sequences from the CDR3 Sequences Group 6;
   i) amino acid sequences that have 3, 2, or 1 amino acid difference with at least one of the amino acid sequences from the CDR3 Sequences Group 6;
or any suitable fragment of such an amino acid sequence.

In particular, according to this preferred but non-limiting aspect, the invention relates to a (single) domain antibody and/or Nanobody (as defined herein) which is a p19+sequence, which consists of 4 framework regions (FR1 to FR4 respectively) and 3 complementarity determining regions (CDR1 to CDR3 respectively), in which:
- CDR1 is chosen from the group consisting of:
   a) the amino acid sequences from the CDR1 Sequences Group 2;
   b) amino acid sequences that have at least 80% amino acid identity with at least one of the amino acid sequences from the CDR1 Sequences Group 2;
   c) amino acid sequences that have 3, 2, or 1 amino acid difference with at least one of the amino acid sequences from the CDR1 Sequences Group 2;
   and
- CDR2 is chosen from the group consisting of:
   d) the amino acid sequences from the CDR2 Sequences Group 4;
   e) amino acid sequences that have at least 80% amino acid identity with at least one of the amino acid sequences from the CDR2 Sequences Group 4;
   f) amino acid sequences that have 3, 2, or 1 amino acid difference with at least one of the amino acid sequences from the CDR2 Sequences Group 4;
   and
- CDR3 is chosen from the group consisting of:
   g) the amino acid sequences from the CDR3 Sequences Group 6;
   h) amino acid sequences that have at least 80% amino acid identity with at least one of the amino acid sequences from the CDR3 Sequences Group 6;
   i) amino acid sequences that have 3, 2, or 1 amino acid difference with at least one of the amino acid sequences from the CDR3 Sequences Group 6;
or any suitable fragment of such an amino acid sequence.

In another preferred but non-limiting aspect, the invention relates to a (single) domain antibody and/or a Nanobody (as defined herein) which is a p19- sequence (as defined herein), which consists of 4 framework regions (FR1 to FR4 respectively) and 3 complementarity determining regions (CDR1 to CDR3 respectively), in which:
- CDR1 is chosen from the group consisting of:
   a) the amino acid sequences from the CDR1 Sequences Group 9 (see Table A-1 and Figure 12);
   b) amino acid sequences that have at least 80% amino acid identity with at least one of the amino acid sequences from the CDR1 Sequences Group 9;
   c) amino acid sequences that have 3, 2, or 1 amino acid difference with at least one of the amino acid sequences from the CDR1 Sequences Group 9;
   and/or
- CDR2 is chosen from the group consisting of:
   d) the amino acid sequences from the CDR2 Sequences Group 11;
   e) amino acid sequences that have at least 80% amino acid identity with at least one of the amino acid sequences from the CDR2 Sequences Group 11;
   f) amino acid sequences that have 3, 2, or 1 amino acid difference with at least one of the amino acid sequences from the CDR2 Sequences Group 11;
   and/or
- CDR3 is chosen from the group consisting of:
   g) the amino acid sequences from the CDR3 Sequences Group 13;
   h) amino acid sequences that have at least 80% amino acid identity with at least one of the amino acid sequences from the CDR3 Sequences Group 13;
   i) amino acid sequences that have 3, 2, or 1 amino acid difference with at least one of the amino acid sequences from the CDR3 Sequences Group 13;
or any suitable fragment of such an amino acid sequence.

In particular, according to this preferred but non-limiting aspect, the invention relates to a (single) domain antibody and/or a Nanobody (as defined herein) which is a p19-sequence, which consists of 4 framework regions (FR1 to FR4 respectively) and 3 complementarity determining regions (CDR1 to CDR3 respectively), in which:
- CDR1 is chosen from the group consisting of:
   a) the amino acid sequences from the CDR1 Sequences Group 9;
   b) amino acid sequences that have at least 80% amino acid identity with at least one of the amino acid sequences from the CDR1 Sequences Group 9;
   c) amino acid sequences that have 3, 2, or 1 amino acid difference with at least one of the amino acid sequences from the CDR1 Sequences Group 9;
   and
- CDR2 is chosen from the group consisting of:
   d) the amino acid sequences from the CDR2 Sequences Group 11;
   e) amino acid sequences that have at least 80% amino acid identity with at least one of the amino acid sequences from the CDR2 Sequences Group 11;
   f) amino acid sequences that have 3, 2, or 1 amino acid difference with at least one of the amino acid sequences from the CDR2 Sequences Group 11;
   and
- CDR3 is chosen from the group consisting of:
   g) the amino acid sequences from the CDR3 Sequences Group 13;
   h) amino acid sequences that have at least 80% amino acid identity with at least one of the amino acid sequences from the CDR3 Sequences Group 13;
   i) amino acid sequences that have 3, 2, or 1 amino acid difference with at least one of the amino acid sequences from the CDR3 Sequences Group 13;
or any suitable fragment of such an amino acid sequence.

In another preferred but non-limiting aspect, the invention relates to a (single) domain antibody and/or a Nanobody (as defined herein) which is a p40- sequence (as defined herein), which consists of 4 framework regions (FR1 to FR4 respectively) and 3 complementarity determining regions (CDR1 to CDR3 respectively), in which:
- CDR1 is chosen from the group consisting of:
   a) the amino acid sequences from the CDR1 Sequences Group 16 (see Table A-1 and Figure 13);
   b) amino acid sequences that have at least 80% amino acid identity with at least one of the amino acid sequences from the CDR1 Sequences Group 16;
   c) amino acid sequences that have 3, 2, or 1 amino acid difference with at least one of the amino acid sequences from the CDR1 Sequences Group 16;
   and/or
- CDR2 is chosen from the group consisting of:
   d) the amino acid sequences from the CDR2 Sequences Group 18;
   e) amino acid sequences that have at least 80% amino acid identity with at least one of the amino acid sequences from the CDR2 Sequences Group 18;
   f) amino acid sequences that have 3, 2, or 1 amino acid difference with at least one of the amino acid sequences from the CDR2 Sequences Group 18;
   and/or
- CDR3 is chosen from the group consisting of:
   g) the amino acid sequences from the CDR3 Sequences Group 20;
   h) amino acid sequences that have at least 80% amino acid identity with at least one of the amino acid sequences from the CDR3 Sequences Group 20;
   i) amino acid sequences that have 3, 2, or 1 amino acid difference with at least one of the amino acid sequences from the CDR3 Sequences Group 20;
or any suitable fragment of such an amino acid sequence.

In particular, according to this preferred but non-limiting aspect, the invention relates to a (single) domain antibody and/or a Nanobody (as defined herein) which is a p40-sequences, which consists of 4 framework regions (FR1 to FR4 respectively) and 3 complementarity determining regions (CDR1 to CDR3 respectively), in which:
- CDR1 is chosen from the group consisting of:
   a) the amino acid sequences from the CDR1 Sequences Group 16;
   b) amino acid sequences that have at least 80% amino acid identity with at least one of the amino acid sequences from the CDR1 Sequences Group 16;
   c) amino acid sequences that have 3, 2, or 1 amino acid difference with at least one of the amino acid sequences from the CDR1 Sequences Group 16;
   and
- CDR2 is chosen from the group consisting of:
   d) the amino acid sequences from the CDR2 Sequences Group 18;
   e) amino acid sequences that have at least 80% amino acid identity with at least one of the amino acid sequences from the CDR2 Sequences Group 18;
   f) amino acid sequences that have 3, 2, or 1 amino acid difference with at least one of the amino acid sequences from the CDR2 Sequences Group 18;
   and
- CDR3 is chosen from the group consisting of:
   g) the amino acid sequences from the CDR3 Sequences Group 20;
   h) amino acid sequences that have at least 80% amino acid identity with at least one of the amino acid sequences from the CDR3 Sequences Group 20;
   i) amino acid sequences that have 3, 2, or 1 amino acid difference with at least one of the amino acid sequences from the CDR3 Sequences Group 20;
or any suitable fragment of such an amino acid sequence.

In another preferred but non-limiting aspect, the invention relates to a (single) domain antibody and/or a Nanobody (as defined herein) which is a p40+ sequence (as defined herein), which consists of 4 framework regions (FR1 to FR4 respectively) and 3 complementarity determining regions (CDR1 to CDR3 respectively), in which:
- CDR1 is chosen from the group consisting of:
   a) the amino acid sequences from the CDR1 Sequences Group 23 (see Table A-1 and Figure 14);
   b) amino acid sequences that have at least 80% amino acid identity with at least one of the amino acid sequences from the CDR1 Sequences Group 23;
   c) amino acid sequences that have 3, 2, or 1 amino acid difference with at least one of the amino acid sequences from the CDR1 Sequences Group 23;
   and/or
- CDR2 is chosen from the group consisting of:
   d) the amino acid sequences from the CDR2 Sequences Group 25;
   e) amino acid sequences that have at least 80% amino acid identity with at least one of the amino acid sequences from the CDR2 Sequences Group 25;
   f) amino acid sequences that have 3, 2, or I amino acid difference with at least one of the amino acid sequences from the CDR2 Sequences Group 25;
   and/or
- CDR3 is chosen from the group consisting of:
   g) the amino acid sequences from the CDR3 Sequences Group 27;
   h) amino acid sequences that have at least 80% amino acid identity with at least one of the amino acid sequences from the CDR3 Sequences Group 27;
   i) amino acid sequences that have 3, 2, or 1 amino acid difference with at least one of the amino acid sequences from the CDR3 Sequences Group 27;
or any suitable fragment of such an amino acid sequence.

In particular, according to this preferred but non-limiting aspect, the invention relates to a (single) domain antibody and/or a Nanobody (as defined herein) which is a p40+ sequence, which consists of 4 framework regions (FR1 to FR4 respectively) and 3 complementarity determining regions (CDR1 to CDR3 respectively), in which:
- CDR1 is chosen from the group consisting of:
   a) the amino acid sequences from the CDR1 Sequences Group 23;
   b) amino acid sequences that have at least 80% amino acid identity with at least one of the amino acid sequences from the CDR1 Sequences Group 23;
   c) amino acid sequences that have 3; 2, or 1 amino acid difference with at least one of the amino acid sequences from the CDR1 Sequences Group 23;
   and
- CDR2 is chosen from the group consisting of:
   d) the amino acid sequences from the CDR2 Sequences Group 25;
   e) amino acid sequences that have at least 80% amino acid identity with at least one of the amino acid sequences from the CDR2 Sequences Group 25;
   f) amino acid sequences that have 3, 2, or 1 amino acid difference with at least one of the amino acid sequences from the CDR2 Sequences Group 25;
   and
- CDR3 is chosen from the group consisting of:
   g) the amino acid sequences from the CDR3 Sequences Group 27;
   h) amino acid sequences that have at least 80% amino acid identity with at least one of the amino acid sequences from the CDR3 Sequences Group 27;
   i) amino acid sequences that have 3, 2, or 1 amino acid difference with at least one of the amino acid sequences from the CDR3 Sequences Group 27;
or any suitable fragment of such an amino acid sequence.

In another preferred but non-limiting aspect, the invention relates to a (single) domain antibody and/or a Nanobody (as defined herein) which is a p35 sequence (as defined herein), which consists of 4 framework regions (FR1 to FR4 respectively) and 3 complementarity determining regions (CDR1 to CDR3 respectively), in which:
- CDR1 is chosen from the group consisting of:
   a) the amino acid sequences from the CDR1 Sequences Group 30 (see Table A-1 and Figure 15);
   b) amino acid sequences that have at least 80% amino acid identity with at least one of the amino acid sequences from the CDR1 Sequences Group 30;
   c) amino acid sequences that have 3, 2, or 1 amino acid difference with at least one of the amino acid sequences from the CDR1 Sequences Group 30;
   and/or
- CDR2 is chosen from the group consisting of:
   d) the amino acid sequences from the CDR2 Sequences Group 32;
   e) amino acid sequences that have at least 80% amino acid identity with at least one of the amino acid sequences from the CDR2 Sequences Group 32;
   f) amino acid sequences that have 3, 2, or 1 amino acid difference with at least one of the amino acid sequences from the CDR2 Sequences Group 32;
   and/or
- CDR3 is chosen from the group consisting of:
   g) the amino acid sequences from the CDR3 Sequences Group 34;
   h) amino acid sequences that have at least 80% amino acid identity with at least one of the amino acid sequences from the CDR3 Sequences Group 34;
   i) amino acid sequences that have 3, 2, or 1 amino acid difference with at least one of the amino acid sequences from the CDR3 Sequences Group 34;
or any suitable fragment of such an amino acid sequence.

In particular, according to this preferred but non-limiting aspects, the invention relates to a (single) domain antibody and/or Nanobody (as defined herein) which is a p35 sequence, which consists of 4 framework regions (FR1 to FR4 respectively) and 3 complementarity determining regions (CDR1 to CDR3 respectively). in which:
- CDR1 is chosen from the group consisting of:
   a) the amino acid sequences from the CDR1 Sequences Group 30;
   b) amino acid sequences that have at least 80% amino acid identity with at least one of the amino acid sequences from the CDR1 Sequences Group 30;
   c) amino acid sequences that have 3, 2, or 1 amino acid difference with at least one of the amino acid sequences from the CDR1 Sequences Group 30;
   and
- CDR2 is chosen from the group consisting of:
   d) the amino acid sequences from the CDR2 Sequences Group 32;
   e) amino acid sequences that have at least 80% amino acid identity with at least one of the amino acid sequences from the CDR2 Sequences Group 32;
   f) amino acid sequences that have 3, 2, or 1 amino acid difference with at least one of the amino acid sequences from the CDR2 Sequences Group 32;
   and
- CDR3 is chosen from the group consisting of:
   g) the amino acid sequences from the CDR3 Sequences Group 34;
   h) amino acid sequences that have at least 80% amino acid identity with at least one of the amino acid sequences from the CDR3 Sequences Group 34;
   i) amino acid sequences that have 3, 2, or 1 amino acid difference with at least one of the amino acid sequences from the CDR3 Sequences Group 34;
or any suitable fragment of such an amino acid sequence.

In another preferred but non-limiting aspect, the invention relates to a (single) domain antibody and/or Nanobody (as defined herein) which is an IL-27 sequence (as defined herein), which consists of 4 framework regions (FR1 to FR4 respectively) and 3 complementarity determining regions (CDR1 to CDR3 respectively), in which:
- CDR1 is chosen from the group consisting of:
   a) the amino acid sequences from the CDR1 Sequences Group 37 (see Table A-1 and Figure 16);
   b) amino acid sequences that have at least 80% amino acid identity with at least one of the amino acid sequences from the CDR1 Sequences Group 37;
   c) amino acid sequences that have 3, 2, or 1 amino acid difference with at least one of the amino acid sequences from the CDR1 Sequences Group 37;
   and/or
- CDR2 is chosen from the group consisting of:
   d) the amino acid sequences from the CDR2 Sequences Group 39;
   e) amino acid sequences that have at least 80% amino acid identity with at least one of the amino acid sequences from the CDR2 Sequences Group 39;
   f) amino acid sequences that have 3, 2, or 1 amino acid difference with at least one of the amino acid sequences from the CDR2 Sequences Group 39;
   and/or
- CDR3 is chosen from the group consisting of:
   g) the amino acid sequences from the CDR3 Sequences Group 41;
   h) amino acid sequences that have at least 80% amino acid identity with at least one of the amino acid sequences from the CDR3 Sequences Group 41;
   i) amino acid sequences that have 3, 2, or 1 amino acid difference with at least one of the amino acid sequences from the CDR3 Sequences Group 41;
or any suitable fragment of such an amino acid sequence.

In particular, according to this preferred but non-limiting aspect, the invention relates to a (single) domain antibody and/or a Nanobody (as defined herein) which is an IL-27 sequence, which consists of 4 framework regions (FR1 to FR4 respectively) and 3 complementarity determining regions (CDR1 to CDR3 respectively), in which:
- CDR1 is chosen from the group consisting of:
   a) the amino acid sequences from the CDR1 Sequences Group 37;
   b) amino acid sequences that have at least 80% amino acid identity with at least one of the amino acid sequences from the CDR1 Sequences Group 37;
   c) amino acid sequences that have 3, 2, or 1 amino acid difference with at least one of the amino acid sequences from the CDR1 Sequences Group 37;
   and
- CDR2 is chosen from the group consisting of:
   d) the amino acid sequences from the CDR2 Sequences Group 39;
   e) amino acid sequences that have at least 80% amino acid identity with at least one of the amino acid sequences from the CDR2 Sequences Group 39;
   f) amino acid sequences that have 3, 2, or I amino acid difference with at least one of the amino acid sequences from the CDR2 Sequences Group 39;
   and
- CDR3 is chosen from the group consisting of:
   g) the amino acid sequences from the CDR3 Sequences Group 41:
   h) amino acid sequences that have at least 80% amino acid identity with at least one of the amino acid sequences from the CDR3 Sequences Group 41;
   i) amino acid sequences that have 3, 2, or 1 amino acid difference with at least one of the amino acid sequences from the CDR3 Sequences Group 41;
or any suitable fragment of such an amino acid sequence.

In another preferred but non-limiting aspect, the invention relates to a (single) domain antibody and/or a Nanobody (as defined herein) which is an IL-12Rb1 sequence (as defined herein), which consists of 4 framework regions (FR1 to FR4 respectively) and 3 complementarity determining regions (CDR1 to CDR3 respectively), in which:
- CDR1 is chosen from the group consisting of:
   a) the amino acid sequences from the CDR1 Sequences Group 44 (see Table A-1 and Figure 17);
   b) amino acid sequences that have at least 80% amino acid identity with at least one of the amino acid sequences from the CDR1 Sequences Group 44;
   c) amino acid sequences that have 3, 2, or 1 amino acid difference with at least one of the amino acid sequences from the CDR1 Sequences Group 44;
   and/or
- CDR2 is chosen from the group consisting of:
   d) the amino acid sequences from the CDR2 Sequences Group 46;
   e) amino acid sequences that have at least 80% amino acid identity with at least one of the amino acid sequences from the CDR2 Sequences Group 46;
   f) amino acid sequences that have 3, 2, or 1 amino acid difference with at least one of the amino acid sequences from the CDR2 Sequences Group 46;
   and/or
- CDR3 is chosen from the group consisting of:
   g) the amino acid sequences from the CDR3 Sequences Group 48;
   h) amino acid sequences that have at least 80% amino acid identity with at least one of the amino acid sequences from the CDR3 Sequences Group 48;
   i) amino acid sequences that have 3, 2, or 1 amino acid difference with at least one of the amino acid sequences from the CDR3 Sequences Group 48;
or any suitable fragment of such an amino acid sequence.

In particular, according to this preferred but non-limiting aspect, the invention relates to a (single) domain antibody and/or a Nanobody (as defined herein) which is an IL-12Rb1 sequence, which consists of 4 framework regions (FR1 to FR4 respectively) and 3 complementarity determining regions (CDR1 to CDR3 respectively), in which:
- CDR1 is chosen from the group consisting of:
   a) the amino acid sequences from the CDR1 Sequences Group 44;
   b) amino acid sequences that have at least 80% amino acid identity with at least one of the amino acid sequences from the CDR1 Sequences Group 44;
   c) amino acid sequences that have 3, 2, or 1 amino acid difference with at least one of the amino acid sequences from the CDR1 Sequences Group 44;
   and
- CDR2 is chosen from the group consisting of:
   d) the amino acid sequences from the CDR2 Sequences Group 46;
   e) amino acid sequences that have at least 80% amino acid identity with at least one of the amino acid sequences from the CDR2 Sequences Group 46;
   f) amino acid sequences that have 3, 2, or 1 amino acid difference with at least one of the amino acid sequences from the CDR2 Sequences Group 46;
   and
- CDR3 is chosen from the group consisting of:
   g) the amino acid sequences from the CDR3 Sequences Group 48;
   h) amino acid sequences that have at least 80% amino acid identity with at least once of the amino acid sequences from the CDR3 Sequences Group 48;
   i) amino acid sequences that have 3, 2, or 1 amino acid difference with at least one of the amino acid sequences from the CDR3 Sequences Group 48;
or any suitable fragment of such an amino acid sequence.

In another preferred but non-limiting aspect, the invention relates to a (single) domain antibody and/or a Nanobody (as defined herein) which is an IL-12Rb2 sequence (as defined herein), which consists of 4 framework regions (FR1 to FR4 respectively) and 3 complementarity determining regions (CDR1 to CDR3 respectively), in which:
- CDR1 is chosen from the group consisting of:
   a) the amino acid sequences from the CDR1 Sequences Group 51 (see Table A-1 and Figure 18);
   b) amino acid sequences that have at least 80% amino acid identity with at least one of the amino acid sequences from the CDR1 Sequences Group 51;
   c) amino acid sequences that have 3, 2, or 1 amino acid difference with at least one of the amino acid sequences from the CDR1 Sequences Group 51;
   and/or
- CDR2 is chosen from the group consisting of:
   d) the amino acid sequences from the CDR2 Sequences Group 53;
   e) amino acid sequences that have at least 80% amino acid identity with at least one of the amino acid sequences from the CDR2 Sequences Group 53;
   f) amino acid sequences that have 3, 2, or 1 amino acid difference with at least one of the amino acid sequences from the CDR2 Sequences Group 53;
   and/or
- CDR3 is chosen from the group consisting of:
   g) the amino acid sequences from the CDR3 Sequences Group 55;
   h) amino acid sequences that have at least 80% amino acid identity with at least one of the amino acid sequences from the CDR3 Sequences Group 55;
   i) amino acid sequences that have 3, 2, or 1 amino acid difference with at least one of the amino acid sequences from the CDR3 Sequences Group 55;
or any suitable fragment of such an amino acid sequence.

In particular, according to this preferred but non-limiting aspect, the invention relates to a (single) domain antibody and/or a Nanobody (as defined herein) which is an IL-12Rb2 sequence, which consists of 4 framework regions (FR1 to FR4 respectively) and 3 complementarity determining regions (CDR1 to CDR3 respectively), in which:
- CDR1 is chosen from the group consisting of:
   a) the amino acid sequences from the CDR1 Sequences Group 51;
   b) amino acid sequences that have at least 80% amino acid identity with at least one of the amino acid sequences from the CDR1 Sequences Group 51;
   c) amino acid sequences that have 3, 2, or 1 amino acid difference with at least one of the amino acid sequences from the CDR1 Sequences Group 51;
   and
- CDR2 is chosen from the group consisting of:
   d) the amino acid sequences from the CDR2 Sequences Group 53;
   e) amino acid sequences that have at least 80% amino acid identity witch at least one of the amino acid sequences from the CDR2 Sequences Group 53;
   f) amino acid sequences that have 3, 2, or 1 amino acid difference with at least one of the amino acid sequences from the CDR2 Sequences Group 53;
   and
- CDR3 is chosen from the group consisting of:
   g) the amino acid sequences from the CDR3 Sequences Group 55;
   h) amino acid sequences that have at least 80% amino acid identity with at least one of the amino acid sequences from the CDR3 Sequences Group 55;
   i) amino acid sequences that have 3, 2, or I amino acid difference with at least one of the amino acid sequences from the CDR3 Sequences Group 55;
or any suitable fragment of such an amino acid sequence.

In another preferred but non-limiting aspect, the invention relates to a (single) domain antibody and/or a Nanobody (as defined herein) which is an IL-23R sequence (as defined herein), which consists of 4 framework regions (FR1 to FR4 respectively) and 3 complementarity determining regions (CDR1 to CDR3 respectively), in which:
- CDR1 is chosen from the group consisting of:
   a) the amino acid sequences from the CDR1 Sequences Group 58 (see Table A-1 and Figure 18);
   b) amino acid sequences that have at least 80% amino acid identity with at least one of the amino acid sequences from the CDR1 Sequences Group 58;
   c) amino acid sequences that have 3, 2, or 1 amino acid difference with at least one of the amino acid sequences from the CDR1 Sequences Group 58;
   and/or
- CDR2 is chosen from the group consisting of:
   d) the amino acid sequences from the CDR2 Sequences Group 60;
   e) amino acid sequences that have at least 80% amino acid identity with at least one of the amino acid sequences from the CDR2 Sequences Group 60;
   f) amino acid sequences that have 3, 2, or 1 amino acid difference with at least one of the amino acid sequences from the CDR2 Sequences Group 60;
   and/or
- CDR3 is chosen from the group consisting of:
   g) the amino acid sequences from the CDR3 Sequences Group 62;
   h) amino acid sequences that have at least 80% amino acid identity with at least one of the amino acid sequences from the CDR3 Sequences Group 62;
   i) amino acid sequences that have 3, 2, or 1 amino acid difference with at least one of the amino acid sequences from the CDR3 Sequences Group 62;
or any suitable fragment of such an amino acid sequence.

In particular, according to this preferred but non-limiting aspect, the invention relates to a (single) domain antibody and/or a Nanobody (as defined herein) which is an IL-23R sequence, which consists of 4 framework regions (FR1 to FR4 respectively) and 3 complementarity determining regions (CDR1 to CDR3 respectively), in which:
- CDR1 is chosen from the group consisting of:
   a) the amino acid sequences from the CDR1 Sequences Group 58;
   b) amino acid sequences that have at least 80% amino acid identity with at least one of the amino acid sequences from the CDR1 Sequences Group 58;
   c) amino acid sequences that have 3, 2, or 1 amino acid difference with at least one of the amino acid sequences from the CDR1 Sequences Group 58;
   and
- CDR2 is chosen from the group consisting of:
   d) the amino acid sequences from the CDR2 Sequences Group 60;
   e) amino acid sequences that have at least 80% amino acid identity with at least one of the amino acid sequences from the CDR2 Sequences Group 60;
   f) amino acid sequences that have 3, 2, or 1 amino acid difference with at least one of the amino acid sequences from the CDR2 Sequences Group 60;
   and
- CDR3 is chosen from the group consisting of:
   g) the amino acid sequences from the CDR3 Sequences Group 62;
   h) amino acid sequences that have at least 80% amino acid identity with at least one of the amino acid sequences from the CDR3 Sequences Group 62;
   i) amino acid sequences that have 3, 2, or 1 amino acid difference with at least one of the amino acid sequences from the CDR3 Sequences Group 62;
or any suitable fragment of such an amino acid sequence.

Generally, Nanobodies with the above CDR sequences may be as further described herein, and preferably have framework sequences that are also as further described herein, Thus, for example and as mentioned herein, such Nanobodies may be naturally occurring Nanobodies (from any suitable species), naturally occurring V_{HH} sequences (i.e. from a suitable species of Camelid) or synthetic or semi-synthetic amino acid sequences or Nanobodies, including but not limited to partially humanized Nanobodies or V_{HH} sequences, fully humanized Nanobodies or V_{HH} sequences, camelized heavy chain variable domain sequences, as well as Nanobodies that have been obtained by the techniques mentioned herein.

Also, the above p19+, p19-, p40+, p40-, anti p35, anti- IL-27, anti IL-12Rb1, anti IL-12Rb2 and anti IL-23R Nanobodies, respectively, preferably as described herein in terms of degree of sequence identity with, the number of amino acid differences with, and/or of the ability to cross-block and/or compete, with the p19+ sequences, p19- sequences, p40-sequences, p40+ sequences, p35 sequences, IL-27 sequences, IL-12Rb1 sequences, IL-12Rb2 sequences or IL-23R sequences, respectively, that are mentioned in Table A-2. Preferably, the p19+, p19-, p40+, p40-, anti p35, anti- IL-27, anti IL-12Rb1, anti IL-12Rb2 and anti IL-23R Nanobodies are chosen from the corresponding sequences mentioned in Table A-2.

Thus, in one specific, but non-limiting aspect, the invention relates to a humanized Nanobody, which consists of 4 framework regions (FR1 to FR4 respectively) and 3 complementarity determining regions (CDR1 to CDR3 respectively), in which CDR1 to CDR3 are as defined herein and in which said humanized Nanobody comprises at least one humanizing substitution (as defined herein), and in particular at least one humanizing substitution in at least one of its framework sequences (as defined herein).

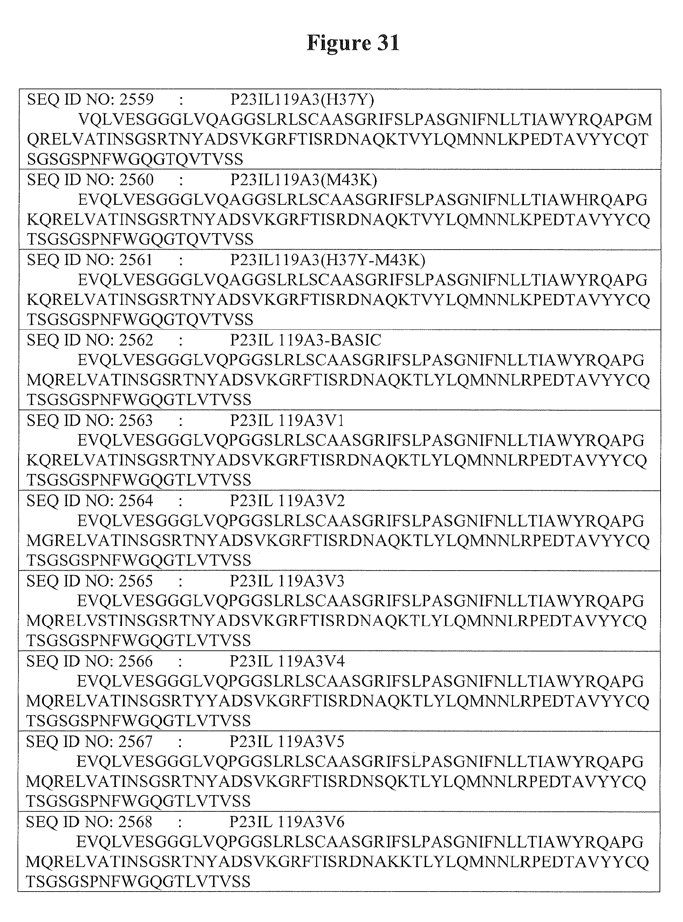

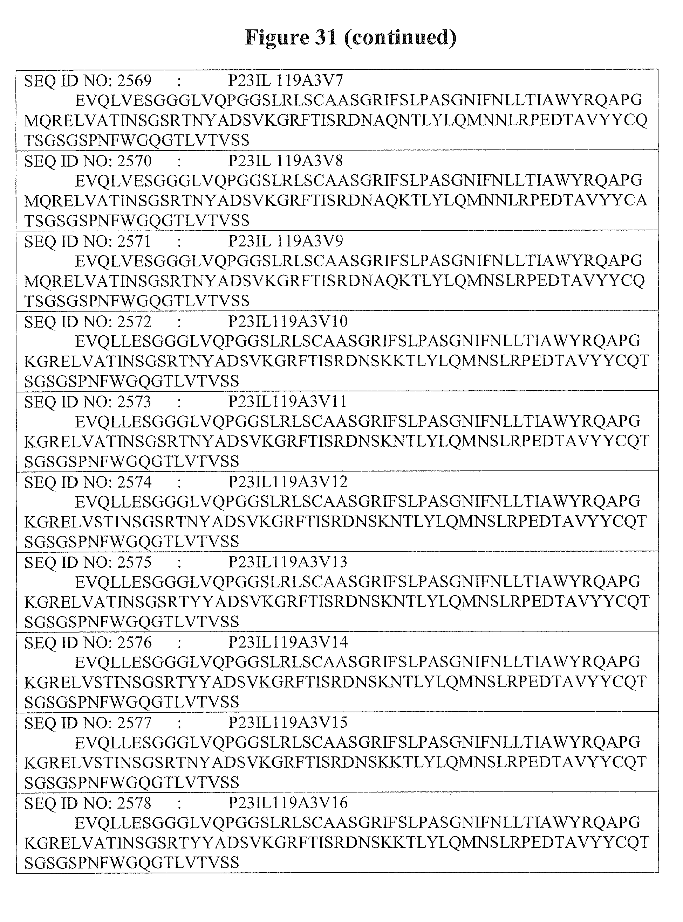

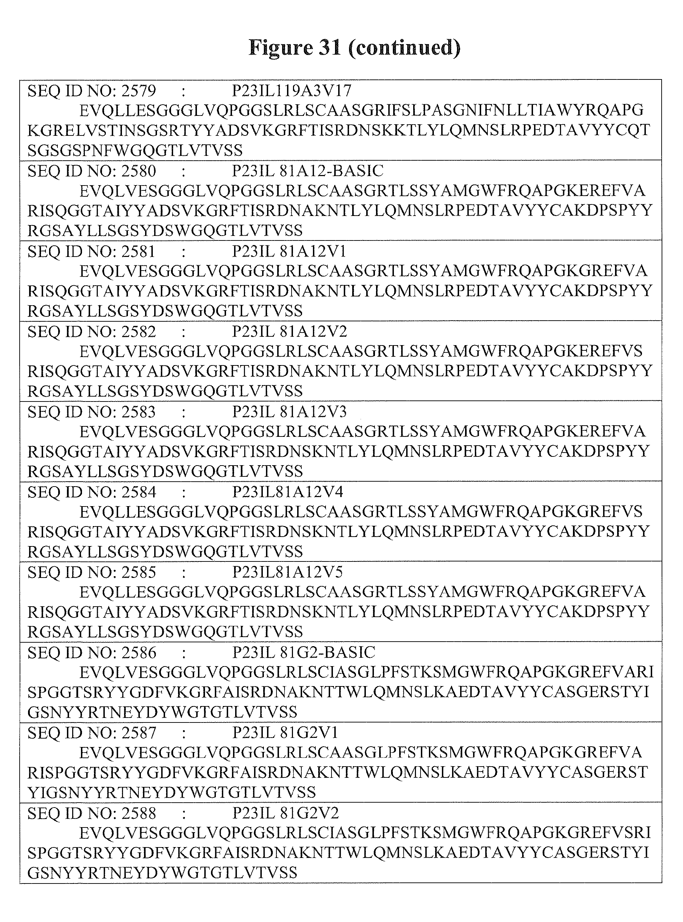

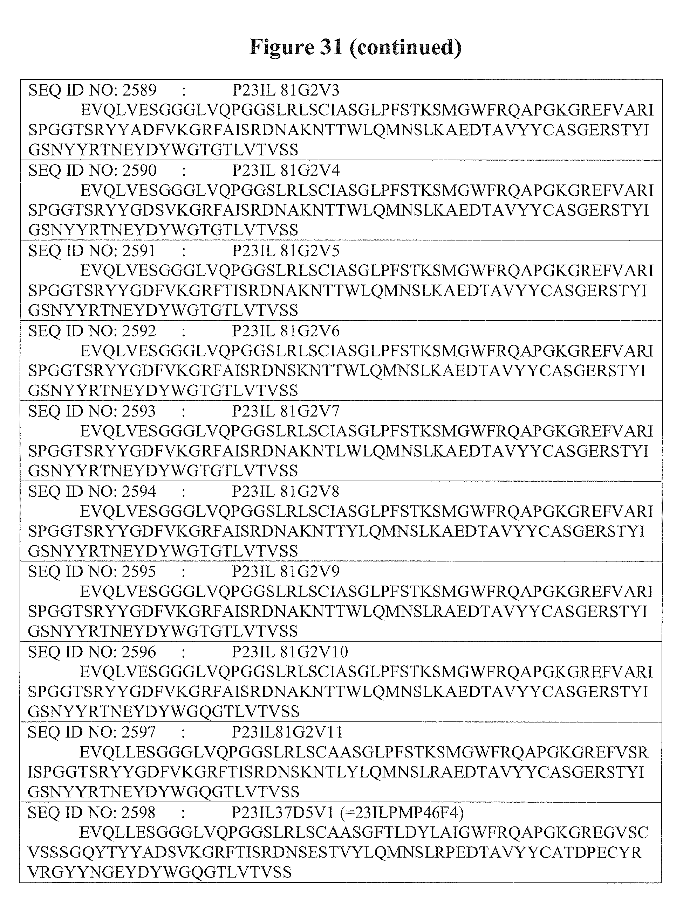

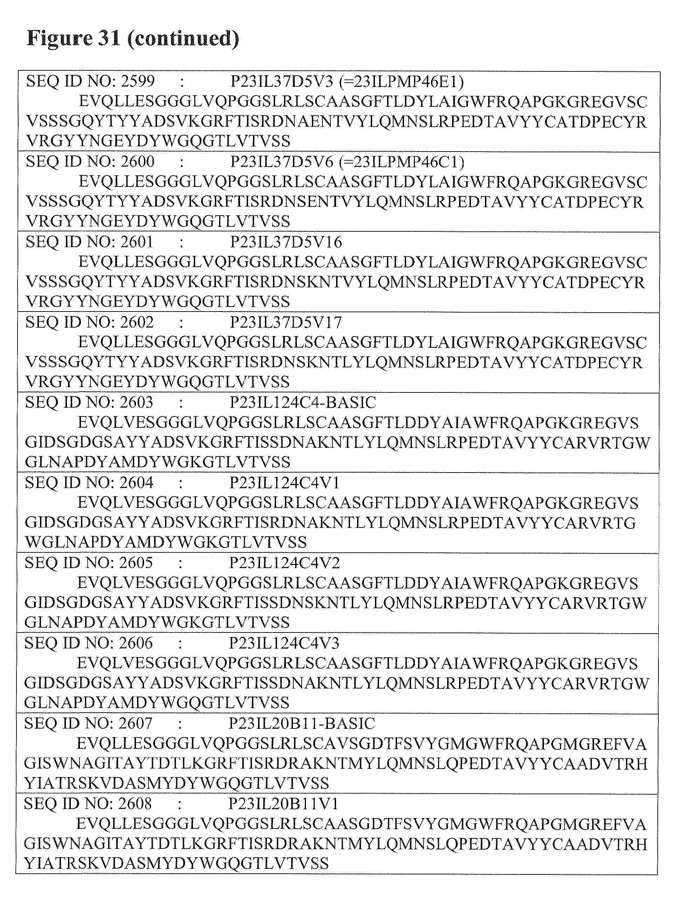

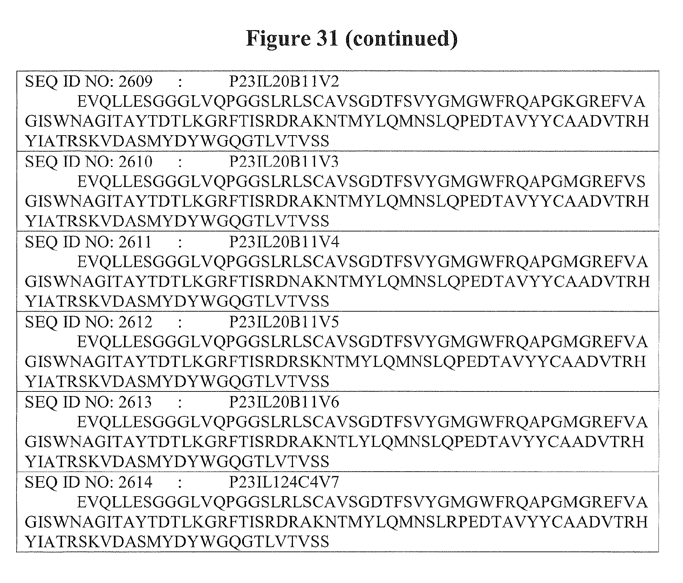

Another preferred, but non-limiting aspect of the invention relates to humanized variants of the above p19+, p19-. p40+, p40-, anti p35, anti- IL-27, anti IL-12Rb1, anti IL-12Rb2 and anti IL-23R Nanobodies, that comprise, compared to the corresponding native V_{HH} sequence (i.e. as mentioned in Table A-2), at least one humanizing substitution (as defined herein), and in particular at least one humanizing substitution in at least one of its framework sequences (as defined herein). Examples of such humanized Nanobodies are given in SEQ ID NO's: 2559 to 2614 (see also Figure 31), and the skilled person will be able to find other suitable humanized variants based on the disclosure herein, optionally after some limited trial-and-error.

The polypeptides of the invention comprise or essentially consist of at least one Nanobody of the invention. Some preferred, but non-limiting examples of polypeptides of the invention are given in SEQ ID NO's: 2142 to 2169 and 2530 to 2558 (see also Figure 30), as well as SEQ ID NO:2615 to 2622 (see Figure 32), 2623 to 2629, 2643 and 2644 (see Figure 33), SEQ ID NO: 2630 to 2641 (see Figure 34), SEQ ID NO: 2645 and 2646 (see Figure 35) and SEQ ID NO: 2647 and 2648 (see Figure 36). The invention also relates to polypeptides that have at least 70%, such as at least 80%, for example at least 90% sequence identity with at least one of these polypeptides.

For example, as further described in the Experimental Part, in the splenocyte assay described by Aggarwal (see Example 15), the Monovalent Nb 121A2 gave an IC50 of about 1.5nM, whereas the following biparatopic constructs gave the following IC 50 values: 121A2-35GS-81A2: IC50= ∼60pM; 121A2-35GS-81G2: IC50= ∼90pM; 121A2-35GS-119G7): IC50=30-60pM; 121A2-35GS-124H2: IC50= ∼90pM; and 121A2-35GS-124H IC50= ∼180pM.

It will be clear to the skilled person that the Nanobodies that are mentioned herein as "preferred" (or "more preferred", "even more preferred", etc.) are also preferred (or more preferred, or even more preferred, etc.) for use in the polypeptides described herein. Thus, polypeptides that comprise or essentially consist of one or more "preferred" Nanobodies of the invention will generally be preferred, and polypeptides that comprise or essentially consist of one or more "more preferred" Nanobodies of the invention will generally be more preferred, etc..

Generally, proteins or polypeptides that comprise or essentially consist of a single Nanobody (such as a single Nanobody of the invention) will be referred to herein as "monovalent" proteins or polypeptides or as "monovalent constructs". Proteins and polypeptides that comprise or essentially consist of two or more Nanobodies (such as at least two Nanobodies of the invention or at least one Nanobody of the invention and at least one other Nanobody) will be referred to herein as "multivalent" proteins or polypeptides or as "multivalent constructs", and these may provide certain advantages compared to the corresponding monovalent Nanobodies of the invention. Some non-limiting examples of such multivalent constructs will become clear from the further description herein.

According to one specific, but non-limiting aspect, a polypeptide of the invention comprises or essentially consists of at least two Nanabodies of the invention, such as two or three Nanobodies of the invention. As further described herein, such multivalent constructs can provide certain advantages compared to a protein or polypeptide comprising or essentially consisting of a single Nanobody of the invention, such as a much improved avidity for heterodimeric cytokines and/or their receptors. Such multivalent constructs will be clear to the skilled person based on the disclosure herein.

According to another specific, but non-limiting aspect, a polypeptide of the invention comprises or essentially consists of at least one Nanobody of the invention and at least one other binding unit (i.e. directed against another epitope, antigen, target, protein or polypeptide), which is preferably also a Nanobody. Such proteins or polypeptides are also referred to herein as "multispecific" proteins or polypeptides or as 'multispecific constructs", and these may provide certain advantages compared to the corresponding monovalent Nanobodies of the invention (as will become clear from the further discussion herein of some preferred, but-nonlimiting multispecific constructs). Such multispecific constructs will be clear to the skilled person based on the disclosure herein, and may in particular be biparatopic constructs (as mentioned herein). Some preferred, but non-limiting examples of such multispecific Nanobody constructs will be clear to the skilled person based on the disclosure herein.

According to yet another specific, but non-limiting aspect, a polypeptide of the invention comprises or essentially consists of at least one Nanobody of the invention, optionally one or more further Nanobodies, and at least one other amino acid sequence (such as a protein or polypeptide) that confers at least one desired property to the Nanobody of the invention and/or to the resulting fusion protein. Again, such fusion proteins may provide certain advantages compared to the corresponding monovalent Nanobodies of the invention. Some non-limiting examples of such amino acid sequences and of such fusion constructs will become clear from the further description herein.

It is also possible to combine two or more of the above aspects, for example to provide a trivalent bispecific construct comprising two Nanobodies of the invention and one other Nanobody, and optionally, one or more other amino acid sequences. Further non-limiting examples of such constructs, as well as some constructs that are particularly preferred within the context of the present invention, will become clear from the further description herein.

In the above constructs, the one or more Nanobodies and/or other amino acid sequences may be directly linked to each other and/or suitably linked to each other via one or more linker sequences. Some suitable but non-limiting examples of such linkers will become clear from the further description herein.

In one specific aspect of the invention, a Nanobody of the invention or a compound, construct or polypeptide of the invention comprising at least one Nanobody of the invention may have an increased half-life, compared to the corresponding amino acid sequence of the invention. Some preferred, but non-limiting examples of such Nanobodies, compounds and polypeptides will become clear to the skilled person based on the further disclosure herein, and for example comprise Nanobodies sequences or polypeptides of the invention that have been chemically modified to increase the half-life thereof (for example, by means of pegylation); amino acid sequences of the invention that comprise at least one additional binding site for binding to a serum protein (such as serum albumin; or polypeptides of the invention that comprise at least one Nanobody of the invention that is linked to at least one moiety (and in particular at least one amino acid sequence) that increases the half-life of the Nanobody of the invention. Examples of polypeptides of the invention that comprise such half-life extending moieties or amino acid sequences will become clear to the skilled person based on the further disclosure herein; and for example include, without limitation, polypeptides in which the one or more Nanobodies of the invention are suitable linked to one or more serum proteins or fragments thereof (such as serum albumin or suitable fragments thereof) or to one or more binding units that can bind to serum proteins (such as, for example, Nanobodies or (single) domain antibodies that can bind to serum proteins such as serum albumin, serum immunoglobulin such as IgG, or transferrine); polypeptides in which a Nanobody of the invention is linked to an Fc portion (such as a human Fc) or a suitable part or fragment thereof; or polypeptides in which the one or more Nanobodies of the invention are suitable linked to one or more small proteins or peptides that can bind to serum proteins (such as, without limitation, the proteins and peptides described in WO 91/01743, WO 01/45746, WO 02/076489 and to the US provisional application of Ablynx N.V. entitled *"Peptides capable of binding to serum proteins"* of Ablynx N.V. filed on December 5, 2006 (see also WO 068280) as well as the US provisional applications 61/050,385 and 61/045,690 of Ablynx N.V. both entitled "Improved peptides capable of binding to serum proteins".

Again, as will be clear to the skilled person, such Nanobodies, compounds, constructs or polypeptides may contain one or more additional groups, residues, moieties or binding units, such as one or more further amino acid sequences and in particular one or more additional Nanobodies (i.e. not directed against heterodimeric cytokines and/or their receptors), so as to provide a tri- of multispecific Nanobody construct.

Generally, the Nanobodies of the invention (or compounds, constructs or polypeptides comprising the same) with increased half-life preferably have a half-life that is at least 1.5 times, preferably at least 2 times, such as at least 5 times, for example at least 10 times or more than 20 times, greater than the half-life of the corresponding amino acid sequence of the invention per se. For example, the Nanobodies, compounds, constructs or polypeptides of the invention with increased half-life may have a half-life that is increased with more than 1 hours, preferably more than 2 hours, more preferably more than 6 hours, such as more than 12 hours, or even more than 24, 48 or 72 hours, compared to the corresponding amino acid sequence of the invention per se.

In a preferred, but non-limiting aspect of the invention, such Nanobodies, compound, constructs or polypeptides of the invention exhibit a serum half-life in human of at least about 12 hours, preferably at least 24 hours, more preferably at least 48 hours, even more preferably at least 72 hours or more. For example, compounds or polypeptides of the invention may have a half-life of at least 5 days (such as about 5 to 10 days), preferably at least 9 days (such as about 9 to 14 days), more preferably at least about 10 days (such as about 10 to 15 days), or at least about 11 days (such as about 11 to 16 days), more preferably at least about 12 days (such as about 12 to 18 days or more), or more than 14 days (such as about 14 to 19 days).

In another one aspect of the invention, a polypeptide of the invention comprises one or more (such as two or preferably one) Nanobodies of the invention linked (optionally via one or more suitable linker sequences) to one or more (such as two and preferably one) amino acid sequences that allow the resulting polypeptide of the invention to cross the blood brain barrier. In particular, said one or more amino acid sequences that allow the resulting polypeptides of the invention to cross the blood brain barrier may be one or more (such as two and preferably one) Nanobodies, such as the Nanobodies described in WO 02/057445, of which FC44 (SEQ ID NO: 189 of WO 06/040153) and FC5 (SEQ ID NO: 190 of WO 06/040154) are preferred examples.

In particular, polypeptides comprising one or more Nanobodies of the invention are preferably such that they:
- bind to heterodimeric cytokines and/or their receptors with a dissociation constant (K_{D}) of 10⁻⁵ to 10⁻¹² moles/liter or less, and preferably 10⁻⁷ to 10⁻¹² moles/liter or less and more preferably 10⁻⁸ to 10⁻¹² moles/liter (i.e. with an association constant (K_{A}) of 10⁵ to 10¹² liter/ moles or more, and preferably 10⁷ to 10¹² liter/moles or more and more preferably 10⁸ to 10¹² liter/moles);
   and/or such that they:
- bind to heterodimeric cytokines and/or their receptors with a kₒₙ-rate of between 10² M⁻¹s⁻¹ to about 10⁷ M⁻¹s⁻¹, preferably between 10³ M⁻¹s⁻¹ and 10⁷ M⁻¹s⁻¹, more preferably between 10⁴ M⁻¹s⁻¹ and 10⁷ M⁻¹s⁻¹, such as between 10⁵ M⁻¹s⁻¹ and 10⁷ M⁻¹s⁻¹;
   and/or such that they:
- bind to heterodimeric cytokines and/or their receptors with a k_{off} rate between 1s⁻¹ (t_{1/2}=0.69 s) and 10⁻⁶ s⁻¹ (providing a near irreversible complex with a t_{1/2} of multiple days), preferably between 10⁻² s⁻¹ and 10⁻⁶ s⁻¹, more preferably between 10⁻³ s⁻¹ and 10⁻⁶ s⁻¹, such as between 10⁻⁴ s⁻¹ and 10⁻⁶ s⁻¹.

Preferably, a polypeptide that contains only one amino acid sequence of the invention is preferably such that it will bind to "heterodimeric cytokines and/or their receptors with an affinity less than 500 nM, preferably less than 200 nM, more preferably less than 10 nM, such as less than 500 pM. In this respect, it will be clear to the skilled person that a polypeptide that contains two or more Nanobodies of the invention may bind to heterodimeric cytokines and/or their receptors with an increased avidity, compared to a polypeptide that contains only one amino acid sequence of the invention.

Some preferred IC₅₀ values for binding of the amino acid sequences or polypeptides of the invention to heterodimeric cytokines and/or their receptors will become clear from the further description and examples herein.

Another aspect of this invention relates to a nucleic acid that encodes a Nanobody of the invention or a polypeptide of the invention comprising the same. Again, as generally described herein for the nucleic acids of the invention, such a nucleic acid may be in the form of a genetic construct, as defined herein.

In another aspect, the invention relates to host or host cell that expresses or that is capable of expressing an amino acid sequence (such as a (single) domain antibody and/or Nanobody) of the invention and/or a polypeptide of the invention comprising the same; and/or that contains a nucleic acid of the invention. Some preferred but non-limiting examples of such hosts or host cells will become clear from the further description herein.

Another aspect of the invention relates to a product or composition containing or comprising at least one Amino acid sequence (such as a (single) domain antibody and/or Nanobody) of the invention, at least one polypeptide of the invention and/or at least one nucleic acid of the invention, and optionally one or more further components of such compositions known per se, i.e. depending on the intended use of the composition. Such a product or composition may for example be a pharmaceutical composition (as described herein), a veterinary composition or a product or composition for diagnostic use (as also described herein). Some preferred but non-limiting examples of such products or compositions will become clear from the further description herein.

The invention further relates to methods for preparing or generating the amino acid sequences, compounds, constructs, polypeptides, nucleic acids, host cells, products and compositions described herein. Some preferred but non-limiting examples of such methods will become clear from the further description herein.

The invention further relates to applications and uses of the amino acid sequences, compounds, constructs, polypeptides, nucleic acids, host cells, products and compositions described herein, as well as to methods for the prevention and/or treatment for diseases and disorders associated with heterodimeric cytokines and/or their receptors. Some preferred but non-limiting applications and uses will become clear from the further description herein.

Other aspects, embodiments, advantages and applications of the invention will also become clear from the further description hereinbelow.

Generally, it should be noted that the term Nanobody as used herein in its broadest sense is not limited to a specific biological source or to a specific method of preparation. For example, as will be discussed in more detail below, the Nanobodies of the invention can generally be obtained: (1) by isolating the V_{HH} domain of a naturally occurring heavy chain antibody; (2) by expression of a nucleotide sequence encoding a naturally occurring V_{HH} domain; (3) by "humanization" (as described herein) of a naturally occurring V_{HH} domain or by expression of a nucleic acid encoding a such humanized V_{HH} domain; (4) by "camelization" (as described herein) of a naturally occurring V_{H} domain from any animal species, and in particular a from species of mammal, such as from a human being, or by expression of a nucleic acid encoding such a camelized V_{H} domain; (5) by "camelisation" of a "domain antibody" or "Dab" as described by Ward et al (supra), or by expression of a nucleic acid encoding such a camelized V_{H} domain; (6) by using synthetic or semi-synthetic, techniques for preparing proteins, polypeptides or other amino acid sequences known per se; (7) by preparing a nucleic acid encoding a Nanobody using techniques for nucleic acid synthesis known per se, followed by expression of the nucleic acid thus obtained; and/or (8) by any combination of one or more of the foregoing. Suitable methods and techniques for performing the foregoing will be clear to the skilled, person based on the disclosure herein and for example include the methods and techniques described in more detail herein.

One preferred class of Nanobodies corresponds to the V_{HH} domains of naturally occurring heavy chain antibodies directed against "heterodimeric cytokines and/or their receptors. As further described herein, such V_{HH} sequences can generally be generated or obtained by suitably immunizing a species of Camelid with heterodimeric cytokines and/or their receptors (i.e. so as to raise an immune response and/or heavy chain antibodies directed against heterodimeric cytokines and/or their receptors), by obtaining a suitable biological sample from said Camelid (such as a blood sample, serum sample or sample of B-cells), and by generating V_{HH} sequences directed against heterodimeric cytokines and/or their receptors, starting from said sample, using any suitable technique known per se. Such techniques will be clear to the skilled person and/or are further described herein.

Alternatively, such naturally occurring V_{HH} domains against heterodimeric cytokines and/or their receptors, can be obtained from naïve libraries of Camelid V_{HH} sequences, for example by screening such a library using heterodimeric cytokines and/or their receptors, or at least one part, fragment, antigenic determinant or epitope thereof using one or more screening techniques known per se. Such libraries and techniques are for example described in WO 99/37681, WO 01/90190, WO 03/025020 and WO 03/035694. Alternatively, improved synthetic or semi-synthetic libraries derived from naïve V_{HH} libraries may be used, such as V_{HH} libraries obtained from naive V_{HH} libraries by techniques such as random mutagenesis and/or CDR shuffling, as for example described in WO 00/43507.

Thus, in another aspect, the invention relates to a method for generating Nanobodies, that are directed against heterodimeric cytokines and/or their receptors. In one aspect, said method at least comprises the steps of:
a) providing a set, collection or library of Nanobody sequences; and
b) screening said set, collection or library of Nanobody sequences for Nanobody sequences that can bind to and/or have affinity for heterodimeric cytokines and/or their receptors;
   and
c) isolating the amino acid sequeuce(s) that can bind to and/or have affinity for heterodimeric cytokines and/or their receptors.

In such a method, the set, collection or library of Nanobody sequences may be a naïve set, collection or library of Nanobody sequences; a synthetic or semi-synthetic set, collection or library of Nanobody sequences; and/or a set, collection or library of Nanobody sequences that have been subjected to affinity maturation.

In a preferred aspect of this method, the set, collection or library of Nanobody sequences may be an immune set, collection or library of Nanobody sequences, and in particular an immune set, collection or library of V_{HH} sequences, that have been derived from a species of Camelid that has been suitably immunized with heterodimeric cytokines and/or their receptors or with a suitable antigenic determinant based thereon or derived therefrom, such as an antigenic part, fragment, region, domain, loop or other epitope thereof. In one particular aspect, said antigenic determinant may be an extracellular part, region, domain, loop or other extracellular epitope(s).

In the above methods, the set, collection or library of Nanobody or V_{HH} sequences, may be displayed on a phage, phagemid, ribosome or suitable micro-organism (such as yeast), such as to facilitate screening. Suitable methods, techniques and host organisms for displaying and screening (a set, collection or library of) Nanobody sequences will be clear to the person skilled in the art, for example on the basis of the further disclosure herein. Reference is also made to WO 03/054016 and to the review by Hoogenboom in Nature Biotechnology, 23, 9, 1105-1116 (2005).

In another aspect, the method for generating Nanobody sequences comprises at least the steps of:
a) providing a collection or sample of cells derived from a species of Camelid that express immunoglobulin sequences;
b) screening said collection or sample of cells for (i) cells that express an immunoglobulin sequence that can bind to and/or have affinity for heterodimeric cytokines and/or their receptors; and (ii) cells that express heavy chain antibodies, in which substeps (i) and (ii) can be performed essentially as a single screening step or in any suitable order as two separate screening steps, so as to provide at least one cell that expresses a heavy chain antibody that can bind to and/or has affinity for heterodimeric cytokines and/or their receptors;
   and
c) either (i) isolating from said cell the V_{HH} sequence present in said heavy chain antibody; or (ii) isolating from said cell a nucleic acid sequence that encodes the V_{HH} sequence present in said heavy chain antibody, followed by expressing said V_{HH} domain.

In the method according to this aspect, the collection or sample of cells may for example be a collection or sample of B-cells. Also, in this method, the sample of cells may be derived from a Camelid that has been suitably immunized with heterodimeric cytokines and/or their receptors or a suitable antigenic determinant based thereon or derived therefrom, such as an antigenic part, fragment, region, domain, loop or other epitope thereof. In one particular aspect, said antigenic determinant may be an extracellular part, region, domain, loop or other extracellular epitope(s).

The above method may be performed in any suitable manner, as will be clear to the skilled person. Reference is for example made to EP 0 542 810, WO 05/19824, WO 04/051268 and WO 04/106377. The screening of step b) is preferably performed using a flow cytometry technique such as FACS. For this, reference is for example made to Lieby et al., Blood, Vol. 97, No. 12, 3820. Particular reference is made to the so-called "Nanoclone™" technique described in International application WO 06/079372 by Ablynx N.V.

In another aspect, the method for generating an amino acid sequence directed against heterodimeric cytokines and/or their receptors may comprise at least the steps of:
a) providing a set, collection or library of nucleic acid sequences encoding heavy chain antibodies or Nanobody sequences;
b) screening said set, collection or library of nucleic acid sequences for nucleic acid sequences that encode a heavy chain antibody or a Nanobody sequence that can bind to and/or has affinity for "heterodimeric cytokines and/or their receptors;
   and
c) isolating said nucleic acid sequence, followed by expressing the V_{HH} sequence present in said heavy chain antibody or by expressing said Nanobody sequence, respectively.

In such a method, the set, collection or library of nucleic acid sequences encoding heavy chain antibodies or Nanobody sequences may for example be a set, collection or library of nucleic acid sequences encoding a naïve set, collection or library of heavy chain antibodies or V_{HH} sequences; a set, collection or library of nucleic acid sequences encoding a synthetic or semi-synthetic set, collection or library ofNanobody sequences; and/or a set, collection or library of nucleic acid sequences encoding a set, collection or library of Nanobody sequences that have been subjected to affinity maturation.

In a preferred aspect of this method, the set, collection or library of nucleic acid sequences may be an immune set, collection or library of nucleic acid sequences encoding heavy chain antibodies or V_{HH} sequences derived from a Camelid that has been suitably immunized with heterodimeric cytokines and/or their receptors or with a suitable antigenic determinant based thereon or derived therefrom, such as an antigenic part, fragment, region, domain, loop or other epitope thereof. In one particular aspect, said antigenic determinant may be an extracellular part, region, domain, loop or other extracellular epitope(s).

In the above methods, the set, collection or library of nucleotide sequences may be displayed on a phage, phagemid, ribosome or suitable micro-organism (such as yeast), such as to facilitate screening. Suitable methods, techniques and host organisms for displaying and screening (a set, collection or library of) nucleotide sequences encoding amino acid sequences will be clear to the person skilled in the art, for example on the basis of the further disclosure herein. Reference is also made to WO 03/054016 and to the review by Hoogenboom in Nature Biotechnology, 23, 9, 1105-1116 (2005).

As will be clear to the skilled person, the screening step of the methods described herein can also be performed as a selection step. Accordingly the term "screening" as used in the present description can comprise selection, screening or any suitable combination of selection and/or screening techniques. Also, when a set, collection or library of sequences is used, it may contain any suitable number of sequences, such as 1, 2, 3 or about 5, 10, 50, 100, 500, 1000, 5000, 10⁴, 10⁵, 10⁶, 10⁷ 10⁸ or more sequences.

Also, one or more or all of the sequences in the above set, collection or library of amino acid sequences may be obtained or defined by rational, or semi-empirical approaches such as computer modelling techniques or biostatics or datamining techniques.

Furthermore, such a set, collection or library can comprise one, two or more sequences that are variants from one another (e.g. with designed point mutations or with randomized positions), compromise multiple sequences derived from a diverse set of naturally diversified sequences (e.g. an immune library)), or any other source of diverse sequences (as described for example in Hoogenboom et al, Nat Biotechnol 23:1105, 2005 and Binz et al, Nat Biotechnol 2005, 23:1247). Such set, collection or library of sequences can be displayed on the surface of a phage particle, a ribosome, a bacterium, a yeast cell, a mammalian cell, and linked to the nucleotide sequence encoding the amino acid sequence within these carriers. This makes such set, collection or library amenable to selection procedures to isolate the desired anaino acid sequences of the invention. More generally, when a sequence is displayed on a suitable host or host cell, it is also possible (and customary) to first isolate from said host or host cell a nucleotide sequence that encodes the desired sequence, and then to obtain the desired sequence by suitably expressing said nucleotide sequence is a suitable host organism. Again, this can be performed in any suitable manner known per se, as will be clear to the skilled person.

Yet another technique for obtaining V_{HH} sequences or Nanobody sequences directed against heterodimeric cytokines and/or their receptors involves suitably immunizing a transgenic mammal that is capable of expressing heavy chain antibodies (i.e. so as to raise an immune response and/or heavy chain antibodies directed against heterodimeric cytokines and/or their receptors), obtaining a suitable biological sample from said transgenic mammal that contains (nucleic acid sequences encoding) said V_{HH} sequences or Nanobody sequences (such as a blood sample, serum sample or sample of B-cells), and then generating V_{HH} sequences directed against heterodimeric cytokines and/or their receptors, starting from said sample, using any suitable technique known per se (such as any of the methods described herein or a hybridoma technique). For example, for this purpose, the heavy chain antibody-expressing mice and the further methods and techniques described in WO 02/085945, WO 04/049794 and WO 06/008548 and Janssens et al., Proc. Natl. Acad. Sci .USA. 2006 Oct 10;103(41):15130-3 can be used. For example, such heavy chain antibody expressing mice can express heavy chain antibodies with any suitable (single) variable domain, such as (single) variable domains from natural sources (e.g. human (single) variable domains, Camelid (single) variable domains or shark (single) variable domains), as well as for example synthetic or semi-synthetic (single) variable domains.

The invention also relates to the V_{HH} sequences or Nanobody sequences that are obtained by the above methods, or alternatively by a method that comprises the one of the above methods and in addition at least the steps of determining the nucleotide sequence or ammo acid sequence of said V_{HH} sequence or Nanobody sequence; and of expressing or synthesizing said V_{HH} sequence or Nanobody sequence in a manner known per se, such as by expression in a suitable host cell or host organism or by chemical synthesis.

As mentioned herein, a particularly preferred class ofNanobodies of the invention comprises Nanobodies with an amino acid sequence that corresponds to the amino acid sequence of a naturally occurring V_{HH} domain, but that has been "humanized", i.e. by replacing one or more amino acid residues in the amino acid sequence of said naturally occurring V_{HH} sequence (and in particular in the framework sequences) by one or more of the amino acid residues that occur at the corresponding position(s) in a V_{H} domain from a conventional 4-chain antibody from a human being (e.g. indicated above). This can be performed in a manner known per se, which will be clear to the skilled person, for example on the basis of the further description herein and the prior art on humanization referred to herein. Again, it should be noted that such humanized Nanobodies of the invention can be obtained in any suitable manner known per se (i.e. as indicated under points (1) - (8) above) and thus are not strictly limited to polypeptides that have been obtained using a polypeptide that comprises a naturally occurring V_{HH} domain as a starting material.

Another particularly preferred class of Nanobodies of the invention comprises Nanobodies with an amino acid sequence that corresponds to the amino acid sequence of a naturally occurring V_{H} domain, but that has been "camelized", i.e. by replacing one or more amino acid residues in the amino acid sequence of a naturally occurring V_{H} domain from a conventional 4-chain antibody by one or more of the amino acid residues that occur at the corresponding position(s) in a V_{HH} domain of a heavy chain antibody. This can be performed in a manner known per se, which will be clear to the skilled person, for example on the basis of the further description herein. Such "camelizing" substitutions are preferably inserted at amino acid positions that form and/or are present at the V_{H}-V_{L} interface, and/or at the so-called Camelidae Hallmark residues, as defined herein (see for example WO 94/04678 and Davies and Riechmann (1994 and 1996), supra). Preferably, the V_{H} sequence that is used as a starting material or starting point for generating or designing the camelized Nanobody is preferably a V_{H} sequence from a mammal, more preferably the V_{H} sequence of a human being, such as a V_{H}3 sequence. However, it should be noted that such camelized Nanobodies of the invention can be obtained in any suitable manner known per se (i.e. as indicated under points (1) - (8) above) and thus are not strictly limited to polypeptides that have been obtained using a polypeptide that comprises a naturally occurring V_{H} domain as a starting material.

For example, again as further described herein, both "humanization" and "camelization" can be performed by providing a nucleotide sequence that encodes a naturally occurring V_{HH} domain or V_{H} domain, respectively, and then changing, in a manner known per se, one or more codons in said nucleotide sequence in such a way that the new nucleotide sequence encodes a "humanized" or "camelized" Nanobody of the invention, respectively. This nucleic acid can then be expressed in a manner known per se, so as to provide the desired Nanobody of the invention. Alternatively, based on the amino acid sequence of a naturally occurring V_{HH} domain or V_{H} domain, respectively, the amino acid sequence of the desired humanized or camelized Nanobody of the invention, respectively, can be designed and then synthesized *de novo* using techniques for peptide synthesis known per se. Also, based on the amino acid sequence or nucleotide sequence of a naturally occurring V_{HH} domain or V_{H} domain, respectively, a nucleotide sequence encoding the desired humanized or camelized Nanobody of the invention, respectively, can be designed and then synthesized *de novo* using techniques for nucleic acid synthesis known per se, after which the nucleic acid thus obtained can be expressed in a manner known per se, so as to provide the desired Nanobody of the invention.

Other suitable methods and techniques for obtaining the Nanobodies of the invention and/or nucleic acids encoding the same, starting from naturally occurring V_{H} sequences or preferably V_{HH} sequences, will be clear from the skilled person, and may for example comprise combining one or more parts of one or more naturally occurring V_{H} sequences (such as one or more FR sequences and/or CDR sequences), one or more parts of one or more naturally occurring V_{HH} sequences (such as one or more FR sequences or CDR sequences), and/or one or more synthetic or semi-synthetic sequences, in a suitable manner, so as to provide a Nanobody of the invention or a nucleotide sequence or nucleic acid encoding the same (which may then be suitably expressed). Nucleotide sequences encoding framework sequences of V_{HH} sequences or Nanobodies will be clear to the skilled person based on the disclosure herein and/or the further prior art cited herein (and/or may alternatively be obtained by PCR starting from the nucleotide sequences obtained using the methods, described herein) and may be suitably combined with nucleotide sequences that encode the desired CDR's (for example, by PCR assembly using overlapping primers), so as to provide a nucleic acid encoding a Nanobody of the invention.

As mentioned herein, Nanobodies may in particular be characterized by the presence of one or more *"Hallmark residues"* (as described herein) in one or more of the Framework sequences.

Thus, according to one preferred, but non-limiting aspect of the invention, a Nanobody in its broadest sense can be generally defined as a polypeptide comprising:
a) an amino acid sequence that is comprised of four framework regions/sequences interrupted by three complementarity determining regions/sequences, in which the amino acid residue at position 108 according to the Kabat numbering is Q;
   and/or:
b) an amino acid sequence that is comprised of four framework regions/sequences interrupted by three complementarity determining regions/sequences, in which the amino acid residue at position 45 according to the Kabat numbering is a charged amino acid (as defined herein) or a cysteine residue, and position 44 is preferably an E; and/or:
c) an amino acid sequence that is comprised of four framework regions/sequences interrupted by three complementarity determining regions/sequences, in which the amino acid residue at position 103 according to the Kabat numbering is chosen from the group consisting of P, R and S, and is in particular chosen from the group consisting of R and S.

Thus, in a first preferred, but non-limiting aspect, a Nanobody of the invention may have the structure

FR1 - CDR1 - FR2 - CDR2 - FR2 - CDR3 - FR4

in which FR1 to FR4 refer to framework regions 1 to 4, respectively, and in which CDR1 to CDR3 refer to the complementarity determining regions 1 to 3, respectively, and in which
a) the amino acid residue at position 108 according to the Kabat numbering is Q;
   and/or in which:
b) the amino acid residue at position 45 according to the Kabat numbering is a charged amino acid or a cysteine and the amino acid residue at position 44 according to the Kabat numbering is preferably E;
   and/or in which:
c) the amino acid residue at position 103 according to the Kabat numbering is chosen from the group consisting of P, R and S, and is in particular chosen from the group consisting of R and S;
   and in which:
d) CDR1, CDR2 and CDR3 are as defined herein, and are preferably as defined according to one of the preferred aspects herein, and are more preferably as defined according to
one of the more preferred aspects herein.

In particular, a Nanobody in its broadest sense can be generally defined as a polypeptide comprising:
a) an amino acid sequence that is comprised of four framework regions/sequences interrupted by three complementarity determining regions/sequences, in which the amino acid residue at position 108 according to the Kabat numbering is Q;
   and/or:
b) an amino acid sequence that is comprised of four framework regions/sequences interrupted by three complementarity determining regions/sequences, in which the amino acid residue at position 44 according to the Kabat numbering is E and in which the amino acid residue at position 45 according to the Kabat numbering is an R; and/or
c) an amino acid sequence that is comprised of four framework regions/sequences interrupted by three complementarity determining regions/sequences, in which the amino acid residue at position 103 according to the Kabat numbering is chosen from the group consisting of P, R and S, and is in particular chosen from the group consisting of R and S.

Thus, according to a preferred, but non-limiting aspect, a Nanobody of the invention may have the structure

FR1 - CDR1 - FR2 - CDR2 - FR3 - CDR3 - FR4

in which FR1 to FR4 refer to framework regions 1 to 4, respectively, and in which CDR1 to CDR3 refer to the complementarity determining regions 1 to 3, respectively, and in which
a) the amino acid residue at position 108 according to the Kabat numbering is Q;
   and/or in which:
b) the amino acid residue at position 44 according to the Kabat numbering is E and in which the amino acid residue at position 45 according to the Kabat numbering is an R; and/or in which:
c) the amino acid residue at position 103 according to the Kabat numbering is chosen from the group consisting of P, R and S, and is in particular chosen from the group consisting of R and S;
   and in which:
d) CDR1, CDR2 and CDR3 are as defined herein, and are preferably as defined according to one of the preferred aspects herein, and are more preferably as defined according to
one of the more preferred aspects herein.

In particular, a Nanobody against heterodimeric cytokines and/or their receptors according to the invention may have the structure:

FR1 - CDR1 - FR2 - CDR2 - FR3 - CDR3 - FR4

in which FR1 to FR4 refer to framework regions 1 to 4, respectively, and in which CDR1 to CDR3 refer to the complementarity determining regions 1 to 3, respectively, and in which
a) the amino acid residue at position 108 according to the Kabat numbering is Q;
   and/or in which:
b) the amino acid residue at position 44 according to the Kabat numbering is E and in which the amino acid residue at position 45 according to the Kabat numbering is an R;
   and/or in which:
c) the amino acid residue at position 103 according to the Kabat numbering is chosen from the group consisting of P, R and S, and is in particular chosen from the group consisting of R and S;
   and in which:
d) CDR1, CDR2 and CDR3 are as defined herein, and are preferably as defined according to one of the preferred aspects herein, and are more preferably as defined according to one of the more preferred aspects herein.

In particular, according to one preferred, but non-limiting aspect of the invention, a Nanobody can generally be defined as a polypeptide comprising an amino acid sequence that is comprised of four framework regions/sequences interrupted by three complementarity determining regions/sequences, in which;
a-1) the amino acid residue at position 44 according to the Kabat numbering is chosen from the group consisting of A, G, E, D, G, Q, R, S, L; and is preferably chosen from the group consisting of G, E or Q; and
a-2) the amino acid residue at position 45 according to the Kabat numbering is chosen from the group consisting of L, R or C; and is preferably chosen from the group consisting of L or R; and
a-3) the amino acid residue at position 103 according to the Kabat numbering is chosen from the group consisting of W, R or S; and is preferably W or R, and is most preferably W;
a-4) the amino acid residue at position 108 according to the Kabat numbering is Q;
   or in which:
b-1) the amino acid residue at position 44 according to the Kabat numbering is chosen from the group consisting of E and Q; and
b-2) the amino acid residue at position 45 according to the Kabat numbering is R; and
b-3) the amino acid residue at position 103 according to the Kabat numbering is chosen from the group consisting of W, R and S; and is preferably W;
b-4) the amino acid residue at position 108 according to the Kabat numbering is chosen from the group consisting of Q and L; and is preferably Q;
   or in which:
c-1) the amino acid residue at position 44 according to the Kabat numbering is chosen from the group consisting of A, G, E, D, Q, R, S and L; and is preferably chosen from the group consisting of G, E and Q; and
c-2) the amino acid residue at position 45 according to the Kabat numbering is chosen from the group consisting of L, R and C; and is preferably chosen from the group consisting of L and R; and
c-3) the amino acid residue at position 103 according to the Kabat numbering is chosen from the group consisting of P, R and S; and is in particular chosen from the group consisting of R and S; and
c-4) the amino acid residue at position 108 according to the Kabat numbering is chosen from the group consisting of Q and L; is preferably Q;
   and in which
d) CDR1, CDR2 and CDR3 are as defined herein, and are preferably as defined according to one of the preferred aspects herein, and are more preferably as defined according to one of the more preferred aspects herein.

Thus, in another preferred, but non-limiting aspect, a Nanobody of the invention may have the structure

FR1 - CDR1 - FR2 - CDR2 - FR3 - CDR3 - FR4

in which FR1 to FR4 refer to framework regions 1 to 4, respectively, and in which CDR1 to CDR3 refer to the complementarity determining regions 1 to 3, respectively, and in which:
a-1) the amino acid residue at position 44 according to the Kabat numbering is chosen from the group consisting of A, G, E, D, G, Q, R, S, L; and is preferably chosen from the group consisting of G, E or Q;
   and in which:
a-2) the amino acid residue at position 45 according to the Kabat numbering is chosen from the group consisting of L, R or C; and is preferably chosen from the group consisting of L or R;
   and in which:
a-3) the amino acid residue at position 103 according to the Kabat numbering is chosen from the group consisting of W, R or S; and is preferably W or R, and is most preferably W;
   and in which
a-4) the amino acid residue at position 108 according to the Kabat numbering is Q;
   and in which:
d) CDR1, CDR2 and CDR3 are as defined herein, and are preferably as defined according to one of the preferred aspects herein, and are more preferably as defined according to one of the more preferred aspects herein.

In another preferred, but non-limiting aspect, a Nanobody of the invention may have the structure

FR1 - CDR1 - FR2 - CDR2 - FR3 - CDR3 - FR4

in which FR1 to FR4 refer to framework regions 1 to 4, respectively, and in which CDR1 to CDR3 refer to the complementarity determining regions 1 to 3, respectively, and in which:
b-1) the amino acid residue at position 44 according to the Kabat numbering is chosen from the group consisting of E and Q;
   and in which:
b-2) the amino acid residue at position 45 according to the Kabat numbering is R;
   and in which:
b-3) the amino acid residue at position 103 according to the Kabat numbering is chosen from the group consisting of W, R and S; and is preferably W;
   and in which:
b-4) the amino acid residue at position 108 according to the Kabat numbering is chosen from the group consisting of Q and L; and is preferably Q;
   and in which:
d) CDR1, CDR2 and CDR3 are as defined herein, and are preferably as defined according to one of the preferred aspects herein, and are more preferably as defined according to one of the more preferred aspects herein.

In another preferred, but non-limiting aspect, a Nanobody of the invention may have the structure

FR1 - CDR1 - FR2 - CDR2 - FR3 - CDR3 - FR4

in which FR1 to FR4 refer to framework regions 1 to 4, respectively, and in which CDR1 to CDR3 refer to the complementarity determining regions I to 3, respectively, and in which:
c-1) the amino acid residue at position 44 according to the Kabat numbering is chosen from the group consisting of A, G, E, D, Q, R, S and L; and is preferably chosen from the group consisting of G, E and Q;
   and in which:
c-2) the amino acid residue at position 45 according to the Kabat numbering is chosen from the group consisting of L, R and C; and is preferably chosen from the group consisting of L and R;
   and in which:
c-3) the amino acid residue at position 103 according to the Kabat numbering is chosen from the group consisting of P, R and S; and is in particular chosen from the group consisting of R and S;
   and in which:
c-4) the amino acid residue at position 108 according to the Kabat numbering is chosen from the group consisting of Q and L; is preferably Q;
   and in which:
d) CDR1, CDR2 and CDR3 are as defined herein, and are preferably as defined according to one of the preferred aspects herein, and are more preferably as defined according to one of the more preferred aspects herein.

Two particularly preferred, but non-limiting groups of the Nanobodies of the invention are those according to a) above; according to (a-1) to (A-5) above; according to b) above; according to (b-1) to (b-4) above; according to (c) above; and/or according to (c-1) to (c-4) above, in which either:
i) the amino acid residues at positions 44-47 according to the Kabat numbering form the sequence GLEW (or a GLEW-like sequence as described herein) and the amino acid residue at position 108 is Q;
   or in which:
ii) the amino acid residues at positions 43-46 according to the Kabat numbering form the sequence KERE or KQRE (or a KERE-like sequence as described) and the amino acid residue at position 108 is Q or L, and is preferably Q.

Thus, in another preferred, but non-limiting aspect, a Nanobody of the invention may have the structure

FR1 - CDR1 - FR2 - CDR2 - FR3 - CDR3 - FR4

in which FR1 to FR4 refer to framework regions 1 to 4, respectively, and in which CDR1 to CDR3 refer to the complementarity determining regions 1 to 3, respectively, and in which:
i) the amino acid residues at positions 44-47 according to the Kabat numbering form the sequence GLEW (or a GLEW-like sequence as defined herein) and the amino acid residue at position 108 is Q;
   and in which:
ii) CDR1, CDR2 and CDR3 are as defined herein, and are preferably as defined according to one of the preferred aspects herein, and are more preferably as defined according to one of the more preferred aspects herein.

In another preferred, but non-limiting aspect, a Nanobody of the invention may have the structure

FR1 - CDR1 - FR2 - CDR2 - FR3 - CDR3 - FR4

in which FR1 to FR4 refer to framework regions 1 to 4, respectively, and in which CDR1 to CDR3 refer to the complementarity determining regions 1 to 3, respectively, and in which:
i) the amino acid residues at positions 43-46 according to the Kabat numbering form the sequence KERE or KQRE (or a KERE-like sequence) and the amino acid residue at position 108 is Q or L, and is preferably Q;
   and in which:
ii) CDR1, CDR2 and CDR3 are as defined herein, and are preferably as defined according to one of the preferred aspects herein, and are more preferably as defined according to one of the more preferred aspects herein.

In the Nanobodies of the invention in which the amino acid residues at positions 43-46 according to the Kabat numbering form the sequence KERE or KQRE, the amino acid residue at position 37 is most preferably F. In the Nanobodies of the invention in which the amino acid residues at positions 44-47 according to the Kabat numbering form the sequence GLEW, the amino acid residue at position 37 is chosen from the group consisting of Y, H, I, L, V or F, and is most preferably V.

Thus, without being limited hereto in any way, on the basis of the amino acid residues present on the positions mentioned above, the Nanobodies of the invention can generally be classified on the basis of the following three groups:
i) The "*GLEW-group*": Nanobodies with the amino acid sequence GLEW at positions 44-47 according to the Kabat numbering and Q at position 108 according to the Kabat numbering. As further described herein, Nanobodies within this group usually have a V at position 37, and can have a W, P, R or S at position 103, and preferably have a W at position 103. The GLEW group also comprises some GLEW-like sequences such as those mentioned in Table A-4 below. More generally, and without limitation, Nanobodies belonging to the GLEW-group can be defined as Nanobodies with a G at position 44 and/or with a W at position 47, in which position 46 is usually E and in which preferably position 45 is not a charged amino acid residue and not cysteine;
ii) The "*KERE-group*": Nanobodies with the amino acid sequenee KERE or KQRE (or another KERE-like sequence) at positions 43-46 according to the Kabat numbering and Q or L at position 108 according to the Kabat numbering. As further described herein, Nanobodies within this group usually have a F at position 37, an L or F at position 47; and can have a W, P, R or S at position 103, and preferably have a W at position 103. More generally, and without limitation, Nanobodies belonging to the KERE-group can be defined as Nanobodies with a K, Q or R at position 44 (usually K) in which position 45 is a charged amino acid residue or cysteine, and position 47 is as further defined herein;
iii) The "*103 P, R, S-group*": Nanobodies with a P, R or S at position 103. These Nanobodies can have either the amino acid sequence GLEW at positions 44-47 according to the Kabat numbering or the amino acid sequence KERE or KQRE at positions 43-46 according to the Kabat numbering, the latter most preferably in combination with an F at position 37 and an L or an F at position 47 (as defined for the KERE-group); and can have Q or L at position 108 according to the Kabat numbering, and preferably have Q.

Also, where appropriate, Nanobodies may belong to (i.e. have characteristics of) two or more of these classes. For example, one specifically preferred group of Nanobodies has GLEW or a GLEW-like sequence at positions 44-47; P,R or S (and in particular R) at position 103; and Q at position 108 (which may be humanized to L).

More generally, it should be noted that the definitions referred to above describe and apply to Nanobodies in the form of a native (i.e. non-humanized) V_{HH} sequence, and that humanized variants of these Nanobodies may contain other amino acid residues than those indicated above (i.e. one or more humanizing substitutions as defined herein). For example, and without limitation, in some humanized Nanobodies of the GLEW-group or the 103 P, R, S-group, Q at position 108 may be humanized to 108L. As already mentioned herein, other humanizing substitutions (and suitable combinations thereof) will become clear to the skilled person based on the disclosure herein. in addition, or alternatively, other potentially useful humanizing substitutions can be ascertained by comparing the sequence of the framework regions of a naturally occurring V_{HH} sequence with the corresponding framework sequence of one or more closely related human V_{H} sequences, after which one or more of the potentially useful humanizing substitutions (or combinations thereof) thus determined can be introduced into said V_{HH} sequence (in any manner known per se, as further described herein) and the resulting humanized V_{HH} sequences can be tested for affinity for the target, for stability, for ease and level of expression, and/or for other desired properties. In this way, by means of a limited degree of trial and error, other suitable humanizing substitutions (or suitable combinations thereof) can be determined by the skilled person based on the disclosure herein. Also, based on the foregoing, (the framework regions of) a Nanobody may be partially humanized or fully humanized.

Thus, in another preferred, but non-limiting aspect, a Nanobody of the invention may be a Nanobody belonging to the GLEW-group (as defined herein), and in which CDR1, CDR2 and CDR3 are as defined herein, and are preferably as defined according to one of the preferred aspects herein, and are more preferably as defined according to one of the more preferred aspects herein.

In another preferred, but non-limiting aspect, a Nanobody of the invention may be a Nanobody belonging to the KERE-group (as defined herein), and CDR1, CDR2 and CDR3 are as defined herein, and are preferably as defined according to one of the preferred aspects herein, and are more preferably as defined according to one of the more preferred aspects herein.

Thus, in another preferred, but non-limiting aspect, a Nanobody of the invention may be a Nanobody belonging to the 103 P, R, S-group (as defined herein), and in which CDR1, CDR2 and CDR3 are as defined herein, and are preferably as defined according to one of the preferred aspects herein, and are more preferably as defined according to one of the more preferred aspects herein.

Also, more generally and in addition to the 108Q, 43E/44R and 103 P,R,S residues mentioned above, the Nanobodies of the invention can contain, at one or more positions that in a conventional V_{H} domain would form (part of) the V_{H}/V_{L} interface, one or more amino acid residues that are more highly charged than the amino acid residues that naturally occur at the same position(s) in the corresponding naturally occurring V_{H} sequence, and in particular one or more charged amino acid residues (as mentioned in Table A-3). Such substitutions include, but are not limited to, the GLEW-like sequences mentioned in Table A-4 below; as well as the substitutions that are described in the International Application WO 00/29004 for so-called "microbodies", e.g. so as to obtain a Nanobody with Q at position 108 in combination with KLEW at positions 44-47. Other possible substitutions at these positions will be clear to the skilled person based upon the disclosure herein.

In one aspect of the Nanobodies of the invention, the amino acid residue at position 83 is chosen from the group consisting of L, M, S, V and W; and is preferably L.

Also, in one aspect of the Nanobodies of the invention, the amino acid residue at position 83 is chosen from the group consisting of R, K, N, E, G, I, T and Q; and is most preferably either K or E (for Nanobodies corresponding to naturally occurring V_{HH} domains) or R (for "humanized" Nanobodies, as described herein). The amino acid residue at position 84 is chosen from the group consisting of P, A, R, S, D T, and V in one aspect, and is most preferably P (for Nanobodies corresponding to naturally occurring V_{HH} domains) or R (for "humanized" Nanobodies, as described herein).

Furthermore, in one aspect of the Nanobodies of the invention, the amino acid residue at position 104 is chosen from the group consisting of G and D; and is most preferably G.

Collectively, the amino acid residues at positions 11, 37,44,45,47, 83, 84, 103,104 and 108, which in the Nanobodies are as mentioned above, will also be referred to herein as the "Hallmark Residues". The Hallmark Residues and the amino acid residues at the corresponding positions of the most closely related human V_{H} domain, V_{H}3, are summarized in Table A-4.

Some especially preferred but non-limiting combinations of these Hallmark Residues as occur in naturally occurring V_{HH} domains are mentioned in Table A-5. For comparison, the corresponding amino acid residues of the human V_{H}3 called DP-47 have been indicated in italics.

**Table A-4: Hallmark Residues in Nanobodies**

| Position | | Human V_{H}3 | Hallmark Residues |
|---|---|---|---|
| 11 | | L, V; predominantly L | L, M, S, V,W; preferably L |
| 37 | | V, I, F; usually V | F⁽¹⁾, Y, H, I, L or V, preferably F⁽¹⁾ or Y |
| 44⁽⁸⁾ | | G | G⁽²⁾, E⁽³⁾, A,D,Q,R,S,L; preferably G⁽²⁾, E⁽³⁾ or Q; most preferably G⁽²⁾ or E⁽³⁾ |
| 45⁽⁸⁾ | | L | L⁽²⁾, R⁽³⁾, C, I, L, P, Q, V; preferably L⁽²⁾ or R⁽³⁾ |
| 47⁽⁸⁾ | | W,Y | W⁽²⁾, L⁽¹⁾ or F⁽¹⁾, A, G, I, M, R, S, V or Y; preferably W⁽²⁾, L⁽¹⁾, F⁽¹⁾ or R |
| 83 | | R or K; usually R | R, K⁽⁵⁾, N, E⁽⁵⁾, G, I, M, Q or T; preferably K or R; most preferably K |
| 84 | | A, T, D; predominantly A | P⁽⁵⁾, A, L, R, S, T, D, V; preferably P |
| 103 | | w | W⁽⁴⁾, P⁽⁶⁾, R⁽⁶⁾, S; preferably W |
| 104 | | G | G or D; preferably G |
| 108 | | L, M or T; predominantly L | Q, L⁽⁷⁾ or R; preferably Q or L⁽⁷⁾ |
| | Notes: | | |
| (1) | In particular, but not exclusively, in combination with KERE or KQRE at positions 43-46. | | |
| (2) | Usually as GLEW at positions 44-47. | | |
| (3) | Usually as KERE or KQRE at positions 43-46, e.g. as KEREL, KEREF, KQREL, KQREF or KEREG at positions 43-47. Alternatively, also sequences such as TERE (for example TEREL), KECE (for example KECEL or KECER), RERE (for example REREG), QERE (for example QEREG), KGRE (for example KGREG), KDRE (for example KDREV) are possible. Some other possible, but less preferred sequences include for example DECKL and NVCEL. | | |
| (4) | With both GLEW at positions 44-47 and KERE or KQRE at positions 43-46, | | |
| (5) | Often as KP or EP at positions 83-84 of naturally occurring V_{HH} domains. | | |
| (6) | In particular, but not exclusively, in combination with GLEW at positions 44-47. | | |
| | (7) With the proviso that when positions 44-47 are GLEW, position 108 is always Q in (non-humanized) V_{HH} sequences that also contain a W at 103. | | |
| (8) | The GLEW group also contains GLEW-like sequences at positions 44-47, such as for example GVEW, EPEW, GLER, DQEW, DLEW, GIEW, ELEW, GPEW, EWLP, GPER, GLER and ELEW. | | |

**Table A-5: Some preferred but non-limiting combinations of Hallmark Residues in naturally occurring Nanobodies.**

| For humanization of these combinations, reference is made to the specification. | | | | | | | | | | |
|---|---|---|---|---|---|---|---|---|---|---|
| | **11** | **37** | **44** | **45** | **47** | **83** | **84** | **103** | **104** | **108** |
| *DP-47 (human)* | *M* | *V* | *G* | *L* | *W* | *R* | *A* | *W* | *G* | *L* |
| | | | | | | | | | | |
| "KERE" group | L | F | E | R | L | K | P | W | G | Q |
| | L | F | E | R | F | E | P | W | G | Q |
| | L | F | E | R | F | K | P | W | G | Q |
| | L | Y | Q | R | L | K | P | W | G | Q |
| | L | F | L | R | V | K | P | Q | G | Q |
| | L | F | Q | R | L | K | P | W | G | Q |
| | L | F | E | R | F | K | P | W | G | Q |
| | | | | | | | | | | |
| "GLEW" group | L | V | G | L | W | K | S | W | G | Q |
| | M | V | G | L | W | K | P | R | G | Q |

In the Nanobodies, each amino acid residue at any other position than the Hallmark Residues can be any amino acid residue that naturally occurs at the corresponding position (according to the Kabat numbering) of a naturally occurring V_{HH} domain.

Such amino acid residues will be clear to the skilled person. Tables A-6 to A-9 mention some non-limiting residues that can be present at each position (according to the Kabat numbering) of the FR1, FR2, FR3 and FR4 of naturally occurring V_{HH} domains. For each position, the amino acid residue that most frequently occurs at each position of a naturally occurring V_{HH} domain (and which is the most preferred amino acid residue for said position in a Nanobody) is indicated in bold; and other preferred amino acid residues for each position have been underlined (note: the number of amino acid residues that are found at positions 26-30 of naturally occurring V_{HH} domains supports the hypothesis underlying the numbering by Chothia (supra) that the residues at these positions already form part of CDR1.)

In Tables A-6 - A-9, some of the non-limiting residues that can be present at each position of a human V_{H}3 domain have also been mentioned. Again, for each position, the amino acid residue that most frequently occurs at each position of a naturally occurring human V_{H}3 domain is indicated in bold; and other preferred amino acid residues have been underlined.

For reference only, Tables A-6-A-9 also contain data on the V_{HH} entropy ("*V_{HH} Ent.*") and V_{HH} variability ("*V_{HH} Var*.") at each amino acid position for a Representative sample of 1118 V_{HH} sequences (data kindly provided by David Lutje Hulsing and Prof. Theo Verrips of Utrecht University). The values for the V_{HH} entropy and the V_{HH} variability provide a measure for the variability and degree of conservation of amino acid residues between the 1118 V_{HH} sequences analyzed: low values (i.e. <1, such as < 0.5) indicate that an amino acid residue is highly conserved between the V_{HH} sequences (i.e. little variability). For example, the G at position 8 and the G at position 9 have values for the V_{HH} entropy of 0.1 and 0 respectively, indicating that these residues are highly conserved and have little variability (and in case of position 9 is G in all 1118 sequences analysed), whereas for residues that form part of the CDR's generally values of 1.5 or more are found (data not shown). Note that (1) the amino acid residues listed in the second column of Tables A-6-A-9 are based on a bigger sample than the 1118 V_{HH} sequences that were analysed for determining the V_{HH} entropy and V_{HH} variability referred to in the last two columns; and (2) the data represented below support the hypothesis that the amino acid residues at positions 27-30 and maybe even also at positions 93 and 94 already form part of the CDR's (although the invention is not limited to any specific hypothesis or explanation, and as mentioned above, herein the numbering according to Kabat is used). For a general explanation of sequence entropy, sequence variability and the methodology for determining the same, see Oliveira et al., PROTEINS: Structure, Function and Genetics, 52: 544-552 (2003).

**Table A-6: Non-limiting examples of amino acid residues in FR1 (for the footnotes, see the footnotes to Table A-4)**

| **Pos.** | **Amino acid residue(s):** | | V_{HH} Ent. | V_{HH} Var. |
|---|---|---|---|---|
| | *Human V_{H}3* | *Camelid V_{HH}'s* | | |
| 1 | E, Q | Q,A,E | - | - |
| 2 | V | V | 0.2 | 1 |
| 3 | Q | Q, K | 0.3 | 2 |
| 4 | L | L | 0.1 | 1 |
| 5 | V, L | Q,E,L,V | 0.8 | 3 |
| 6 | E | E,D,Q,A | 0.8 | 4 |
| 7 | S,T | S, F | 0.3 | 2 |
| 8 | G, R | G | 0.1 | I |
| 9 | G | G | 0 | 1 |
| 10 | G,V | G,D,R | 0.3 | 2 |
| 11 | **Hallmark residue:** L,M,S,V,W; preferably L | | 0.8 | 2 |
| 12 | V, I | V, A | 0.2 | 2 |
| 13 | Q, K, R | Q, E, K, P, R | 0.4 | 4 |
| 14 | P | A, Q, A, G, P, S, T, V | 1 | 5 |
| 15 | G | G | 0 | 1 |
| 16 | G, R | G, A, E, D | 0.4 | 3 |
| 17 | S | S, F | 0.5 | 2 |
| 18 | L | L, V | 0.1 | 1 |
| 19 | R,K | R, K, L, N, S, T | 0.6 | 4 |
| 20 | L | L,F,I,V | 0.5 | 4 |
| 21 | s | S, A, F, T | 0.2 | 3 |
| 22 | C | C | 0 | 1 |
| 23 | A, T | A, D, E, P, S, T, V | 1.3 | 5 |
| 24 | A | A, I, L, S, T, V | 1 | 6 |
| 25 | **S** | **S**, A, F, P, T | 0.5 | 5 |
| 26 | **G** | **G**, A, D, E, R, S, T, V | 0.7 | 7 |
| 27 | **F** | S, F, R, L, P, G, N, | 2.3 | 13 |
| 28 | **T** | N, T, E, D, S, I, R, A, G, R, F, Y | 1.7 | 11 |
| 29 | **F**, V | F,L, D, S, I, G, V, A | 1.9 | 11 |
| 30 | **S**, D, G | N, S, E, G, A, D, M, T | 1.8 | 11 |

**Table A-7: Non-limiting examples of amino acid residues in FR2 (for the footnotes, see the footnotes to Table A-4)**

| **Pos.** | **Amino acid residue(s):** | | V_{HH} Ent. | V_{HH} Var. |
|---|---|---|---|---|
| | *Human V_{H}3* | *Camelid V_{HH}'s* | | |
| 36 | **W** | **W** | 0.1 | 1 |
| 37 | **Hallmark residue:** F⁽¹⁾, H, I, L, Y or V, preferably F⁽¹⁾ or Y | | 1.1 | 6 |
| 38 | **R** | **R** | 0.2 | 1 |
| 39 | **Q** | **Q**,H,P,R | 0.3 | 2 |
| 40 | **A** | **A**, F, G, L, P, T, V | 0.9 | 7 |
| 41 | **P**, S, T | **P**, A, L, S | 0.4 | 3 |
| 42 | **G** | **G**, E | 0.2 | 2 |
| 43 | **K** | **K**, D, E, N, Q, R, T, V | 0.7 | 6 |
| 44 | **Hallmark residue:** G⁽²⁾, E⁽³⁾, A, D, Q, R, S, L; preferably G⁽²⁾, E⁽³⁾ or Q; most preferably G⁽²⁾ or E⁽³⁾ | | 1.3 | 5 |
| 45 | **Hallmark residue**: L⁽²⁾, R⁽³⁾, C, I, L, P, Q, V; preferably L⁽²⁾ or R⁽³⁾ | | 0.6 | 4 |
| 46 | **E**, V | **E**, D, K, Q, V | 0.4 | 2 |
| 47 | **Hallmark residue:** W⁽²⁾,L⁽¹⁾ or F⁽¹⁾, A,G,I,M,R,S,V or Y; preferably **W**⁽²⁾, L⁽¹⁾, F⁽¹⁾ or R | | 1.9 | 9 |
| 48 | **V** | **V**, I, L | 0.4 | 3 |
| 49 | **S**, A, G | **A**, S, G, T, V | 0.8 | 3 |

**Table A-8: Non-limitting examples of amino acid residues in FR3 (for the footnotes, see the footnotes to Table A-4)**

| **Pos.** | **Amino acid residue(s):** | | **V**_{HH} **Ent.** | **V**_{HH} **Vat.** |
|---|---|---|---|---|
| | *Human V_{H}3* | *Camelid V_{HH}'_{S}* | | |
| 66 | **R** | **R** | 0.1 | 1 |
| 67 | **F** | **F**, L, V | 0.1 | 1 |
| 68 | **T** | **T**, A, N, S | 0.5 | 4 |
| 69 | **I** | **I**, L, M, V | 0.4 | 4 |
| 70 | **S** | **S**, A, F, T | 0.3 | 4 |
| 71 | **R** | **R**, G, H, I, L, K, Q, S, T, W | 1.2 | 8 |
| 72 | **D**, E | **D**, E, G, N, V | 0.5 | 4 |
| 73 | **N**, D, G | **N,** A, D, F, I, K, L, R, S, T, V, Y | 1.2 | 9 |
| 74 | **A**, S | **A**, D, G, N, P, S, T, V | 1 | 7 |
| 75 | **K** | **K**, A, E, K, L, N, Q, R | 0.9 | 6 |
| 76 | **N**, S | **N**, D, K, R, S, T, Y | 0.9 | 6 |
| 77 | S, T, I | **T**, A, E, I, M, P, S | 0.8 | 5 |
| 78 | **L**, A | **V**, L, A, F, G, I, M | 1.2 | 5 |
| 79 | **Y**, H | **Y**, A, D, F, H, N, S, T | 1 | 7 |
| 80 | **L** | **L**, F, V | 0.1 | 1 |
| 81 | **Q** | **Q**, E, I, L_{,} R, T | 0.6 | 5 |
| 82 | **M** | **M**, I, L, V | 0.2 | 2 |
| 82a | **N**, G | **N**, D, G, H, S, T | 0.8 | 4 |
| 82b | **S** | **S**, N, D, G, R, T | 1 | 6 |
| 82c | **L** | **L**,P,V | 0.1 | 2 |
| 83 | **Hallmark residue:** R, K⁽⁵⁾, N, E⁽⁵⁾, G, I, M, Q or T: preferably K or R; most preferably K | | 0.9 | 7 |
| 84 | **Hallmark residue:** P⁽⁵⁾, A, D, L, R, S, T, V; preferably P | | 0.7 | 6 |
| 85 | **E**, G | **E**, D, G, Q | 0.5 | 3 |
| 86 | **D** | **D** | 0 | 1 |
| 87 | **T**, M | **T**, A, S | 0.2 | 3 |

**Table A-8: Non-limiting examples of amino acid residues in FR3 (continued)**

| **Pos.** | **Amino acid residue(s):** | | V_{HH} Ent. | V_{HH} Var. |
|---|---|---|---|---|
| | *Human V_{H}3* | *Camelid V_{HH}'s.* | | |
| 88 | **A** | **A**, G, S | 0.3 | 2 |
| 89 | **V**, L | **V**, A, D, I, L, M, N, R, T | 1.4 | 6 |
| 90 | **Y** | **Y**, F | 0 | 1 |
| 91 | **Y**, H | **Y**, D, F, H, L, S, T, V | 0.6 | 4 |
| 92 | **C** | **C** | 0 | 1 |
| 93 | **A**, K, T | **A**, N, G, H, K, N, R, S, T, V, Y | 1.4 | 10 |
| 94 | **K**, R, T | **A**, V, C, F, G, I, K, L, R, S or T | 1.6 | 9 |

**Table A-9: Non-limiting examples of amino acid residues in FR4 (for the footnotes, see the footnotes to Table A-4)**

| **Pos.** | **Amino acid residue(s):** | | V_{HH} Ent. | V_{HH} Var. |
|---|---|---|---|---|
| | *Human V_{H}3* | *Camelid V_{HH}'s* | | |
| 103 | **Hallmark residue:** W⁽⁴⁾, p⁽⁶⁾, P⁽⁶⁾, S; preferably W | | 0.4 | 2 |
| 104 | **Hallmark residue:** G or D; preferably G | | 0.1 | I |
| 105 | **Q**, R | **Q**, E, K, P, R | 0.6 | 4 |
| 106 | **G** | **G** | 0.1 | 1 |
| 107 | **T** | **T**, A, I | 0.3 | 2 |
| 108 | **Hallmark residue:** Q, L⁽⁷⁾ or R; preferably Q or L⁽⁷⁾ | | 0.4 | 3 |
| 109 | **V** | **V** | 0.1 | 1 |
| 110 | **T** | **T**, I, A | 0.2 | 1 |
| 111 | **V** | **V**, A, I | 0.3 | 2 |
| 112 | **S** | **S**. F | 0.3 | 1 |
| 113 | **S** | **S**, A, L, P, T | 0.4 | 3 |

Thus, in another preferred, but not limiting aspect, a Nanobody of the invention can be defined as an amino acid sequence with the (general) structure

FR1 - CDR1 - FR2 - CDR2 - FR3 - CDR3 - FR4

in which FR1 to FR4 refer to framework regions 1 to 4, respectively, and in which CDR1 to CDR3 refer to the complementarity determining regions 1 to 3, respectively, and in which:
i) one or more of the amino acid residues at positions 11, 37, 44, 45, 47, 83, 84, 103, 104 and 108 according to the Kabat numbering are chosen from the Hallmark residues mentioned in Table A-4;
   and in which:
ii) CDR1, CDR2 and CDR3 are as defined herein, and are preferably as defined according to one of the preferred aspects herein, and are more preferably as defined according to one of the more preferred aspects herein.

The above Nanobodies may for example be V_{HH} sequences or may be humanized Nanobodies. When the above Nanobody sequences are V_{HH} sequences, they may be suitably humanized, as further described herein. When the Nanobodies. are partially hunmnized Nanobodies, they may optionally be further suitably humanized, again as described herein.

In particular, a Nanobody of the invention can be an amino acid sequence with the (general) structure

FR1 - CDR1 - FR2 - CDR2 - FR3 - CDR3 - FR4

in which FR1 to FR4 refer to framework regions 1 to 4, respectively, and in which CDR1 to CDR3 refer to the complementarity determining regions 1 to 3, respectively, and in which:
i) (preferably) one or more of the amino acid residues at positions 11, 37, 44, 45, 47, 83, 84, 103, 104 and 108 according to the Kabat numbering are chosen from the Hallmark residues mentioned in Table A-4 (it being understood that V_{HH} sequences will contain one or more Hallmark residues; and that partially humanized Nanobodies will usually, and preferably, [still] contain one or more Hallmark residues [although it is also within the scope of the invention to provide - where suitable in accordance with the invention - partially humanized Nanobodies in which all Hallmark residues, but not one or more of the other amino acid residues, have been humanized]; and that in fully humanized Nanobodies, where suitable in accordance with the invention, all amino acid residues at the positions of the Hallmark residues will be amino acid residues that occur in a human V_{H}3 sequence. As will be clear to the skilled person based on the disclosure herein that such V_{HH} sequences, such partially humanized Nanobodies with at least one Hallmark residue, such partially humanized Nanobodies without Hallmark residues and such fully humanized Nanobodies all form aspects of this invention); and in which:
ii) said amino acid sequence has at least 80% amino acid identity with at least one of the amino acid sequences of SEQ ID NO's: 1 to 22, in which for the purposes of determining the degree of amino acid identity, the amino acid residues that form the CDR sequences (indicated with X in the sequences of SEQ ID NO's: 1 to 22) are disregarded;
   and in which:
iii) CDR1, CDR2 and CDR3 are as defined herein, and are preferably as defined according to one of the preferred aspects herein, and are more preferably as defined according to one of the more preferred aspects herein.

The above Nanobodies may for example be V_{HH} sequences or may be humanized Nanobodies. When the above Nanobody sequences are V_{HH} sequences, they may be suitably humanized, as further described herein. When the Nanobodies are partially humanized Nanobodies, they may optionally be further suitably humanized, again as described herein.

**Table A-10: Representative amino acid sequences for Nanobodies of the KERE, GLEW and P,R,S 103 group.**

| The CDR's are indicated with XXXX | | |
|---|---|---|
| KERE sequence no.1 | SEQ ID NO:1 | EVQLVESGGGLVQPGGSLRLSCAASGIPFSXXXXXWFRQAPGKQRDSVAXXXXXRFTISRDNAKN TVYLQMNSLKPEDTAVYRCYFXXXXXWGQGTQVTVSS |
| KERE sequence no. 2 | SEQ ID NO:2 | QVKLEESGGGLVQAGGSLRLSCVGSGRTFSXXXXXWFRLAPGKEREFVAXXXXXRFTISRDTASNR GYLHMNNLTPEDTAVYYCAAXXXXXWGQGTQVTVSS |
| KERE sequence no. 3 | SEQ ID NO:3 | AVQLVDSGGGLVQAGDSLKLSCALTGGAFTXXXXXWFRQTPGREREFVAXXXXXRFTISRDNAKN MVYLRMNSLIPEDAAVYSCAAXXXXXWGQGTLVTVSS |
| KERE sequence no. 4 | SEQ ID NO:4 | QVQLVESGGGLVEAGGSLRLSCTASESPFRXXXXXWFRQTSGQEREFVAXXXXXRFTISRDDAKNT[?] VWLHGSTLKPEDTAVYYCAAXXXXXWGQGTQVTVSS |
| KERE sequence no. 5 | SEQ ID NO:5 | AVQLVESGGGLVQGGGSLRLACAASERIFDXXXXXWYRQGPGNERELVAXXXXXRFTISMDYTKQ TVYLHMNSLRPEDTGLYYCKIXXXXXWGQGTQVTVSS |
| KERE sequence no. 6 | SEQ ID NO:6 | DVKFVESGGGLVQAGGSLRLSCVASGFNFDXXXXXWFRQAPGKEREEVAXXXXXRFTISSEKDKN SVYLQMNSLKPEDTALYICAGXXXXXWGRGTQVTVSS |
| KERE sequence no. 7 | SEQ ID NO:7 | QVRLAESGGGLVQSGGSLRLSCVASGSTYTXXXXXWYRQYPGKQRALVAXXXXXRFTIARDSTKDT FCLQMNNLKPEDTAVYYCYAXXXXXWGQGTQVTVSS |
| KERE sequence no. 8 | SEQ ID NO:8 | EVQLVESGGGLVQAGGSLRLSCAASGFTSDXXXXXWFRQAPGKPREGVSXXXXXRFTISTDNAKN TVHLLMNRVNAEDTALYYCAVXXXXXWGRGTRVTVSS |
| KERE sequence no. 9 | SEQ ID NO:9 | QVQLVESGGGLVQPGGSLRLSCQASGDISTXXXXXWYRQVPGKLREFVAXXXXXRFTISGDNAKR AIYLQMNNLKPDDTAVYYCNRXXXXXWGQGTQVTVSP |
| KERE sequence no. 10 | SEQ ID NO:10 | QVPVVESGGGLVQAGDSLRLFCAVPSFTSTXXXXXWFRQAPGKEREFVAXXXXXRFTISRNATKNT LTLRMDSLKPEDTAVYYCAAXXXXXWGQGTQVTVSS |
| KERE sequence no. 11 | SEQ ID NO:11 | EVQLVESGGGLVQAGDSLRLFCTVSGGTASXXXXXWFRQAPGEKREFVAXXXXXRFTIARENAGN MVYLQMNNLKPDDTALYTCAAXXXXXWGRGTQVTVSS |
| KERE sequence no. 12 | SEQ ID NO:12 | AVQLVESGGDSVQPGDSQTLSCAASGRTNSXXXXXWFRQAPGKERVFLAXXXXXRFTISRDSAKN MMYLQMNNLKPQDTAVYYCAAXXXXXWGQGTQVTVSS |
| KERE sequence no. 13 | SEQ ID NO:13 | AVQLVESGGGLVQAGGSLRLSCVVSGLTSSXXXXXWFRQTPWQERDFVAXXXXXRFTISRDNYKD TVLLEMNFLKPEDTAIYYCAAXXXXXWGQGTQVTVSS |
| KERE sequence no. 14 | SEQ ID NO:14 | AVQLVESGGGLVQAGASLRLSCATSTRTLDXXXXXWFRQAPGRDREFVAXXXXXRFTVSRDSAEN TVALQMNSLKPEDTAVYYCAAXXXXXWGQGTRVTVSS |
| KERE sequence no. 15 | SEQ ID NO:15 | QVQLVESGGGLVQPGGSLRLSCTVSRLTAHXXXXXWFRQAPGKEREAVSXXXXXRFTISRDYAGN TAFLQMDSLKPEDTGVYYCATXXXXXWGQGTQVTVSS |
| KERE sequence no. 16 | SEQ ID NO:16 | EVQLVESGGELVQAGGSLKLSCTASGRNFVXXXXXWFRRAPGKEREFVAXXXXXRFTVSRDNGKN TAYLRMNSLKPEDTADYYCAVXXXXXLGSGTQVTVSS |
| GLEW sequence no. 1 | SEQ ID NO:17 | AVQLVESGGGLVQPGGSLRLSCAASGFTFSXXXXXWVRQAPGKVLEWVSXXXXXRFTISRDNAKN TLYLQMNSLKPEDTAVYYCVKXXXXXXGSQGTQVTVSS |
| GLEW sequence no. 2 | SEQ ID NO:18 | EVQLVESGGGLVQPGGSLRLSCVCVSSGCTXXXXXWVRQAPGKAEEWVSXXXXXRFKISRDNAKK TLYLQMNSLGPEDTAMYYCQRXXXXXRGQGTQVTVSS |
| GLEW sequence no. 3 | SEQ ID NO:19 | EVQLVESGGGLALPGGSLTLSCVFSGSTFSXXXXXWVRHTPGKAEEWVSXXXXXRFTISRDNAKNT LYLEMNSLSPEDTAMYYCGRXXXXXRSKGIQVTVSS |
| P,R,S 103 sequence no. 1 | SEQ ID NO:20 | AVQLVESGGGLVQAGGSLRLSCAASGRTFSXXXXXWFRQAPGKEREFVAXXXXXRFTISRDNAKN TVYLQMNSLKPEDTAVYYCAAXXXXXRGQGTQVTVSS |
| P,R,S 103 sequence no. 2 | SEQ ID NO:21 | DVQLVESGGDLVQPGGSLRLSCAASGFSFDXXXXXWLRQTPGKGLEWVGXXXXXRFTISRDNAK NMLYLHLNNLKSEDTAVYYCRRXXXXXLGQGTQVTVSS |
| P,R,S 103 sequence no. 3 | SEQ ID NO:22 | EVQLVESGGGLVQPGGSLRLSCVCVSSGCTXXXXXWVRQAPGKAEEWVSXXXXXRFKISRDNAKK TLYLQMNSLGPEDTAMYYCQRXXXXXRGQGTQVTVSS |

In particular, Nanobody of the invention of the KERE group can be an amino acid sequence with the (general) structure

FR1 - CDR1 - FR2 CDR2 - FR3 - CDR3 - FR4

in which:
i) the amino acid residue at position 45 according to the Kabat numbering is a charged amino acid (as defined herein) or a cysteine residue, and position 44 is preferably an E; and in which:
ii) FR1 is an amino acid sequence that has at least 80% amino acid identity with at least one of the following amino acid sequences:

**Table A-11: Representative FW1 sequences for Nanobodies of the KERE-group.**

| | | |
|---|---|---|
| KERE FW1 sequence no. 1 | SEQ ID NO:23 | QVQRVESGGGLVQAGGSLRLSCAASGRTSS |
| KERE FW1 sequence no. 2 | SEQ ID NO:24 | QVQLVESGGGLVQTGDSLSLSCSASGRTFS |
| KERE FW1 sequence no. 3 | SEQ ID NO:25 | QVKLEESGGGLVQAGDSLRLSCAATGRAFG |
| KERE FW1 sequence no. 4 | SEQ ID NO:26 | AVQLVESGGGLVQPGESLGLSCVASGRDFV |
| KERE FW1 sequence no. 5 | SEQ ID NO:27 | EVQLVESGGGLVQAGGSLRLSCEVLGRTAG |
| KERE FW1 sequence no. 6 | SEQ ID NO:28 | QVQLVESGGGWVQPGGSLRLSCAASETILS |
| KERE FW1 sequence no. 7 | SEQ ID NO:29 | QVQLVESGGGTVQPGGSLNLSCVASGNTFN |
| KERE FW1 sequence no. 8 | SEQ ID NO:30 | EVQLVESGGGLAQPGGSLQLSCSAPGFTLD |
| KERE FW1 sequence no. 9 | SEQ ID NO:31 | AQFLEFSGGGLVQAGCSLRLSCAASGRTFN |

and in which:
iii) FR2 is an amino acid sequence that has at least 90% amino acid identity with at least one of the following amino acid sequences:

**Table A-12: Representative FW2 sequences for Nanobodies of the KERE-group.**

| | | |
|---|---|---|
| KERE FW2 sequence no. 1 | SEQ ID NO:41 | WFRQAPGKEREFVA |
| KERE FW2 sequence no. 2 | SEQ ID NO:42 | WFRQTPGREREFVA |
| KERE FW2 sequence no. 3 | SEQ ID NO:43 | WYRQAPGKQREMVA |
| KERE FW2 sequence no. 4 | SEQ ID NO:44 | WYRQGPGKQRELVA |
| KERE FW2 sequence no. 5 | SEQ ID NO:45 | WIRQAPGKEREGVS |
| KERE FW2 sequence no. 6 | SEQ ID NO:46 | WFREAPGKEREGIS |
| KERE FW2 sequence no. 7 | SEQ ID NO:47 | WYRQAPGKERDLVA |
| KERE FW2 sequence no. 8 | SEQ ID NO:48 | WFRQAPGKQREEVS |
| KERE FW2 sequence no. 9 | SEQ ID NO:49 | WFRQPPGKVREFVG |

and in which:
iv) FR3 is an amino acid sequence that has at least 80% amino acid identity with at least one of the following amino acid sequences:

**Table A-13: Representative FW3 sequences for Nanobodies of the KERE-group.**

| | | |
|---|---|---|
| KERE FW3 sequence no. 1 | SEQ ID NO:50 | RFTISRDNAKNTVYLQMNSLKPEDTAVYRCYF |
| KERE FW3 sequence no. 2 | SEQ ID NO:51 | RFAISRDNNKNTGYLQMNSLEPEDTAVYYCAA |
| KERE FW3 sequence no. 3 | SEQ ID NO:52 | RFTVARNNAKNTVNLEMNSLKPEDTAVYYCAA |
| KERE FW3 sequence no. 4 | SEQ ID NO:53 | RFTISRDIAKNTVDLLMNNLEPEDTAVYYCAA |
| KERE FW3 sequence no. 5 | SEQ ID NO:54 | RLTISRDNAVDTMYLQMNSLKPEDTAVYYCAA |
| KERE FW3 sequence no. 6 | SEQ ID NO:55 | RFTISRDNAKNTVYLQMDNVKPEDTAIYYCAA |
| KERE FW3 sequence no. 7 | SEQ ID NO:56 | RFTISKDSGKNTVYLQMTSLKPEDTAVYYCAT |
| KERE FW3 sequence no. 8 | SEQ ID NO:57 | RFTISRDSAKNMMYLQMNNLKPQDTAVYYCAA |
| KERE FW3 sequence no. 9 | SEQ ID NO:58 | RFTISRENDKSTVYLQLNSLKPEDTAVYYCAA |
| KERE FW3 sequence no. 10 | SEQ ID NO:59 | RPDSRDYAGMTAYLQMNSLKPEDTGVYYCAT |

and in which:
v) FR4 is an amino acid sequence that has at least 80% amino acid identity with at least one of the following amino acid sequences:

**Table A-14: Representative FW4 sequences for Nanobodies of the KERE-group.**

| | | |
|---|---|---|
| KERE FW4 sequence no. 1 | SEQ ID NO:60 | WGQGTQVTVSS |
| KERE FW4 sequence no. 2 | SEQ ID NO:61 | WGKGTLVTVSS |
| KERE FW4 sequence no. 3 | SEQ ID NO:62 | RGQGTRVTVSS |
| KERE FW4 sequence no. 4 | SEQ ID NO:63 | WGLGTQVTISS |

and in which:
vi) CDR1, CDR2 and CDR3 are as defined herein, and are preferably as defined according to one of the preferred aspects herein, and are more preferably as defined according to one of the more preferred aspects herein.

In the above Nanobodies, one or more of the further Hallmark residues are preferably as described herein (for example, when they are V_{HH} sequences or partially humanized Nanobodies).

Also, the above Nanobodies may for example be V_{HH} sequences or may be humanized Nanobodies. When the above Nanobody sequences are V_{HH} sequences, they may be suitably humanized, as further described herein. When the Nanobodies are partially humanized Nanobodies, they may optionally be further suitably humanized, again as described herein.

With regard to framework 1, it will be clear to the skilled person that, when an amino acid sequence as outlined above is generated by expression of a nucleotide sequence, the first four amino acid sequences (i.e. amino acid residues 1-4 according to the Kabat numbering) may often be determined by the primer(s) that have been used to generate said nucleic acid, Thus, for determining the degree of amino acid identity, the first four amino acid residues are preferably disregarded.

Also, with regard to framework 1, and although amino acid positions 27 to 30 are according to the Kabat numbering considered to be part of the framework regions (and not the CDR's), it has been found by analysis of a database of more than 1000 V_{HH} sequences that the positions 27 to 30 have a variability (expressed in terms of V_{HH} entropy and V_{HH} variability - see Tables A-6 to A-9) that is much greater than the variability on positions 1 to 26. Because of this, for determining the degree of amino acid identity, the amino acid residues at positions 27 to 30 are preferably also disregarded.

In view of this, a Nanobody of the KERE class may be an amino acid sequence that is comprised of four framework regions/sequences interrupted by three complementarity determining regions/sequences, in which:
i) the amino acid residue at position 45 according to the Kabat numbering is a charged amino acid (as defined herein) or a cysteine residue, and position 44 is preferably an E; and in which:
ii) FR1 is an amino acid sequence that, on positions 5 to 26 of the Kabat numbering, has at least 80% amino acid identity with at least one of the following amino acid sequences:

**Table A-15: Representative FW1 sequences (amino acid residues 5 to 26) for Nanobodies of the KERE-group.**

| | | |
|---|---|---|
| KERE FW1 sequence no. 10 | SEQ ID NO:32 | VESGGGLVQPGGSLRLSCAASG |
| KERE FW1 sequence no. 11 | SEQ ID NO:33 | VDSGGGLVQAGDSLKLSCALTG |
| KERE FW1 sequence no. 12 | SEQ ID NO:34 | VDSGGGLVQAGDSLRLSCAASG |
| KERE FW1 sequence no. 13 | SEQ ID NO:35 | VDSGGGLVEAGGSLRLSCQVSE |
| KERE FW1 sequence no. 14 | SEQ ID NO:36 | QDSGGGSVQAGGSLKLSCAASG |
| KERE FW1 sequence no. 15 | SEQ ID NO:37 | VQSGGRLVQAGDSLRLSCAASE |
| KERE FW1 sequence no. 16 | SEQ ID NO:38 | VESGGTLVQSG DSLKLSCASST |
| KERE FW1 sequence no. 17 | SEQ ID NO:39 | MESGGDSVQSGGSLTLSCVASG |
| KERE FW1 sequence no. 18 | SEQ ID NO:40 | QASGGGLVQAGGSLRLSCSASV |

and in which:
iii) FR2, FR3 and FR4 are as mentioned herein for FR2, FR3 and FR4 of Nanobodies of the KERE-class;
and in which:
iv) CDR1, CDR2 and CDR3 are as defined herein, and are preferably as defined according to one of the preferred aspects herein, and are more preferably as defined according to one of the more preferred aspects herein.

The above Nanobodies may for example be V_{HH} sequences or may be humanized Nanobodies. When the above Nanobody sequences are V_{HH} sequences, they may be suitably humanized, as further described herein. When the Nanobodies are partially humanized Nanobodies, they may optionally be further suitably humanized, again as described herein.

A Nanobody of the GLEW class may be an amino acid sequence that is comprised of four framework regions/sequences interrupted by three complementarity determining regions/sequences, in which
i) preferably, when the Nanobody of the GLEW-class is a non-humanized Nanobody, the amino acid residue in position 108 is Q:
ii) FR1 is an amino acid sequence that has at least 80% amino acid identity with at least one of the following amino acid sequences:

**Table A-16: Representative FW1 sequences for Nanobodies of the GLEW-group.**

| | | |
|---|---|---|
| GLEW FW1 sequence no. 1 | SEQ ID NO:64 | QVQLVESGGGLVQPGGSLRLSCAPSGFTFS |
| GLEW FW1 sequence no. 2 | SEQ ID NO:65 | EVHLVESGGGLVRPGGSLRLSCAAFGFIFK |
| GLEW FW1 sequence no. 3 | SEQ ID NO:66 | QVKLEESGGGLAQPGGSLRLSCVASGFTFS |
| GLEW FW1 sequence no. 4 | SEQ ID NO:67 | EVQLVESGGGLVQPGGSLRLSCVCVSSGCT |
| GLEW FW1 sequence no. 5 | SEQ ID NO:69 | EVQLVESGGGLALPGGSLTLSCVFSGSTFS |

and in which:
iii) FR2 is an amino acid sequence that has at least 80% amino acid identity with at least one of the following amino acid sequences:

**Table A-17: Representative FW2 sequences for Nanobodies of the GLEW-group.**

| | | |
|---|---|---|
| GLEW FW2 sequence no. 1 | SEQ ID NO:72 | WVRQAPGKVLEWVS |
| GLEW FW2 sequence no. 2 | SEQ ID NO:73 | WVRRPPGKGLEWVS |
| GLEW FW2 sequence no. 3 | SEQ ID NO:74 | WVRQAPGMGLEWVS |
| GLEW FW2 sequence no. 4 | SEQ ID NO:75 | WVRQAPGKEPEWVS |
| GLEW FW2 sequence no. 5 | SEQ ID NO:76 | WVRQAPGKDQEWVS |
| GLEW FW2 sequence no. 6 | SEQ ID NO:77 | WVRQAPGKAEEWVS |
| GLEW FW2 sequence no. 7 | SEQ ID NO:78 | WVRQAPGKGLEWVA |
| GLEW FW2 sequence no. 8 | SEQ ID NO:79 | WVRQAPGRATEWVS |

and in which:
iv) FR3 is an amino acid sequence that has at least 80% amino acid identity with at least one of the following amino acid sequences:

**Table A-18: Representative FW3 sequences for Nanobodies of the GLEW-group.**

| | | |
|---|---|---|
| CLEW FW3 sequence no. 1 | SEQ ID NO:80 | RFTISRDNAKNTLYLQMNSLKPEDTAVYYCVK |
| GLEW FW3 sequence no. 2 | SEQ ID NO:81 | RFTISRDNARNTLYLQMDSLlPEDTALYYCAR |
| GLEW FW3 sequence no. 3 | SEQ ID NO:82 | RFTSSRDNAKSTLYLQMNDLKPEDTALYYCAR |
| GLEW FW3 sequence no. 4 | SEQ ID NO:83 | RFIISRDNAKNTLYLQMNSLGPEDTAMYYCQR |
| GLEW FW3 sequence no. 5 | SEQ ID NO:84 | RFTASRDNAKNTLYLQMNSLKSEDTARYYCAR |
| GLEW FW3 sequence no. 6 | SEQ ID NO:85 | RFTISRDNAKNTLYLQMDDLQSEDTAMYYCGR |

and in which:
v) FR4 is an amino acid sequence that has at least 80% amino acid identity with at least one of the following amino acid sequences:

**Table A-19: Representative FW4 sequences for Nanobodies of the GLEW-group.**

| | | |
|---|---|---|
| GLEW FW4 sequence no. 1 | SEQ ID NO:86 | GSQGTQVTVSS |
| GLEW FW4 sequence no. 2 | SEQ ID NO:87 | LRGGTQVTVSS |
| GLEW FW4 sequence no. 3 | SEQ ID NO:88 | RGQGTLVTVSS |
| GLEW FW4 sequence no. 4 | SEQ ID NO:89 | RSRGIQVTVSS |
| GLEW FW4 sequence no. 5 | SEQ ID NO:90 | WGKGTQVTVSS |
| GLEW FW4 sequence no. 6 | SEQ ID NO:91 | WGQGTQVTVSS |

and in which:
vi) CDR1, CDR2 and CDR3 are as defined herein, and are preferably as defined according to one of the preferred aspects herein, and are more preferably as defined according to one of the more preferred aspects herein.

In the above Nanobodies, one or more of the further Hallmark residues are preferably as described herein (for example, when they are V_{HH} sequences or partially humanized Nanobodies).

With regard to framework 1, it will again be clear to the skilled person that, for determining the degree of amino acid identity, the amino acid residues on positions 1 to 4 and 27 to 30 are preferably disregarded.

In view of this, a Nanobody of the GLEW class may be an amino acid sequence that is comprised of four framework regions/sequences interrupted by three complementarity determining regions/sequences, in which:
i) preferably, when the Nanobody of the GLEW-class is a non-humanized Nanobody, the amino acid residue in position 108 is Q;
and in which:
ii) FR1 is an amino acid sequence that, on positions 5 to 26 of the Kabat numbering, has at least 80% amino acid identity with at least one of the following amino acid sequences:

**Table A-20: Representative FW1 sequences (amino acid residues 5 to 26) for Nanobodies of the KERE-group.**

| | | |
|---|---|---|
| GLEW FW1 sequence no. 6 | SEQ ID NO:69 | VESGGGLVQPGGS.RLSCAASG |
| GLEW FW1 sequence no. 7 | SEQ ID NO:70 | EESGGGLAQPGGSLRLSCVASG |
| GLEW FW1 sequence no. 8 | SEQ ID NO:71 | VESGGGLALPGGSLTLSCVFSG |

and in which:
iii) FR2, FR3 and FR4 are as mentioned herein for FR2, FR3 and FR4 of Nanobodies of the GLEW-class,
and in which:
iv) CDR1, CDR2 and CDR3 are as defined herein, and are preferably as defined according to one of the preferred aspects herein, and are more preferably as defined according to one of the more preferred aspects herein.

The above Nanobodies may for example be V_{HH} sequences or may be humanized Nanobodies. When the above Nanobody sequences are V_{HH} sequences, they may be suitably humanized, as further described herein. When the Nanobodies are partially humanized Nanobodies, they may optionally be further suitably humanized, again as described herein. In the above Nanobodies, one or more of the further Hallmark residues are preferably as described herein (for example, when they are V_{HH} sequences or partially humanized Nanobodies).

A Nanobody of the P, R, S 103 class may be an amino acid sequence that is comprised of four framework regions/sequences interrupted by three complementarity determining regions/sequences, in which
i) the amino acid residue at position 103 according to the Kabat numbering is different from W;
and in which:
ii) preferably the amino acid residue at position 103 according to the Kabat numbering is P, R or S, and more preferably R;
and in which:
iii) FR1 is an amino acid sequence that has at least 80% amino acid identity with at least one of the following amino acid sequences:

**Table A-21: Representative FW1 sequences for Nanobodies of the P,R,S 103-group.**

| | | |
|---|---|---|
| P,R,S 103 FW1 sequence no. 1 | SEQ ID NO:92 | AVQLVESGGGLVQAGGSLRLSCAASGRTFS |
| P,R,S 103 FW1 sequence no. 2 | SEQ ID NO:93 | QVQLQESGGGMVQPGGSLRLSCAASGFDFG |
| P,R,S 103 FW1 sequence no. 3 | SEQ ID NO:94 | EVHLVESGGGLVRPGGSLRLSCAAFGFIFK |
| P,R,S 103 FW1 sequence no. 4 | SEQ ID NO:95 | QVQLAESGGGLVQPGGSLKLSCAASRTIVS |
| P,R,S 103 FW1 sequence no. 5 | SEQ ID NO:96 | QEHLVESGGGLVDIGGSLRLSCAASERIFS |
| P,R,S 103 FW1 sequence no. 6 | SEQ ID NO:97 | QVKLEESGGGLAQPGGSLRLSCVASGFTFS |
| P,R,S 103 FW1 sequence no. 7 | SEQ ID NO:98 | EVQLVESGGGLVQPGGSLRLSCVCVSSGCT |
| P,R,S 103 FW1 sequence no. 8 | SEQ ID NO:99 | EVQLVESGGGLALPGGSLTLSCVFSGSTFS |

and in which
iv) FR2 is an amino acid sequence that has at least 80% amino acid identity with at least one of the following amino acid sequences:

**Table A-22: Representative FW2 sequences for Nanobodies of the P,R,S 103-group.**

| | | |
|---|---|---|
| P,R,S 103 FW2 sequence no. 1 | SEQ ID NO:102 | WFRQAPGKEREFVA |
| P,R,S 103 FW2 sequence no. 2 | SEQ ID NO:103 | WVRQAPGKVLEWVS |
| P,R,S 103 FW2 sequence no. 3 | SEQ ID NO:104 | WVRRPPGKGLEWVS |
| P,R,S 103 FW2 sequence no. 4 | SEQ ID NO:105 | WIRQAPGKEREGVS |
| P,R,S 103 FW2 sequence no. 5 | SEQ ID NO:106 | WVRQYPGKEPEWVS |
| P,R,S 103 FW2 sequence no. 6 | SEQ ID NO:107 | WFRQPPGKEHEFVA |
| P,R,S 103 FW2 sequence no. 7 | SEQ ID NO:108 | WYRQAPGKRTELVA |
| P,R,S 103 FW2 sequence no. 8 | SEQ ID NO:109 | WLRQAPGQGLEWVS |
| P,R,S 103 FW2 sequence no. 9 | SEQ ID NO:110 | WLRQTPGKGLEWVG |
| P,R,S 103 FW2 sequence no. 10 | SEQ ID NO:111 | WVRQAPGKAEEFVS |

and in which:
v) FR3 is an amino acid sequence that has at least 80% amino acid identity with at least one of the following amino acid sequences:

**Table A-23: Representative FW3 sequences for Nanobodies of the P,R,S 103-group.**

| | | |
|---|---|---|
| P,R,S 103 FW3 sequence no. 1 | SEQ ID NO:112 | RFTISRDNAKNTVYLQMNSLKPEDTAVYYCAA |
| P,R,S 103 FW3 sequence no. 2 | SEQ ID NO:113 | RFTISRDNARNTLYLQMDSLIPEDTALYYCAR |
| P,R,S 103 FW3 sequence no. 3 | SEQ ID NO:114 | RFTISRDNAKNEMYLQMNNLKTEDTGVYWCGA |
| P,R,S 103 FW3 sequence no. 4 | SEQ ID NO:115 | RFTISSDSNRNMIYLQMNNLKPEDTAVYYCAA |
| P,R,S 103 FW3 sequence no. 5 | SEQ ID NO:116 | RFTISRDNAKNMLYLHLNNLKSEDTAVYYCRR |
| P,R,S 103 FW3 sequence no. 6 | SEQ ID NO:117 | RFTISRDNAKKTVYLRLNSLNPEDTAVYSCNL |
| P,R,S 103 FW3 sequence no. 7 | SEQ ID NO:118 | RFKISRDNAKKTLYLQMNSLGPEDTAMYYCQR |
| P,R,S 103 FW3 sequence no. 8 | SEQ ID NO:119 | RFTVSRDNGKNTAYLRMNSLKPEDTADYYCAV |

and in which:
vi) FR4 is an amino acid sequence that has at least 80% amino acid identity with at least one of the following amino acid sequences:

**Table A-24: Representative FW4 sequences for Nanobodies of the P,R,S 103-group.**

| | | |
|---|---|---|
| P,R,S 103 FW4 sequence no. 1 | SEQ ID NO:120 | RGQGTQVTVSS |
| P,R,S 103 FW4 sequence no. 2 | SEQ ID NO:121 | LRGGTQVTVSS |
| P,R,S 103 FW4 sequence no. 3 | SEO ID NO:122 | GNKGTLVTVSS |
| P,R,S 103 FW4 sequence no. 4 | SEQ ID NO:123 | SSPGTQVTVSS |
| P,R,S 103 FW4 sequence no. 5 | SEQ ID NO:124 | SSQGTLVTVSS |
| P,R,S 103 FW4 sequence no. 6 | SEQ ID NO:125 | RSRGIQVTVSS |

and in which:
vii) CDR1, CDR2 and CDR3 are as defined herein, and are preferably as defined according to one of the preferred aspects herein, and are more preferably as defined according to one of the more preferred aspects herein.

In the above Nanobodies, one or more of the further Hallmark residues are preferably as described herein (for example, when they are V_{HH} sequences or partially humanized Nanobodies).

With regard to framework 1, it will again be clear to the skilled person that, for determining the degree of amino acid identity, the amino acid residues on positions 1 to 4 and 27 to 30 are preferably disregarded.

In view of this, a Nanobody of the P.R,S 103 class may be an amino acid sequence that is comprised of four framework regions/sequences interrupted by three complementarity determining regions/sequences, in which:
i) the amino acid residue at position 103 according to the Kabat numbering is different from W;
and in which:
ii) preferably the amino acid residue at position 103 according to the Kabat numbering is P, R or S, and more preferably R;
and in which:
iii) FR1 is an amino acid sequence that, on positions 5 to 26 of the Kabat numbering, has at least 80% amino acid identity with at least one of the following amino acid sequences:

**Table A-25: Representative FW1 sequences (amino acid residues 5 to 26) for Nanobodies of the P,R,S 103-group.**

| | | |
|---|---|---|
| P,R,S 103 FW1 sequence no. 9 | SEQ ID NO:100 | VESGGGLVQAGGSLRLSCAASG |
| P,R,S 1.03 FW1 sequence no. 10 | SEQ ID NO:101 | AESGGGLVQPGGSLKLSCAASR |

and in which:
iv) FR2, FR3 and FR4 are as mentioned herein for FR2, FR3 and FR4 of Nanobodies of the P,R,S 103 class;
and in which:
v) CDR1, CDR2 and CDR3 are as defined herein, and are preferably as defined according to one of the preferred aspects herein, and are more preferably as defined according to one of the more preferred aspects herein.

The above Nanobodies may for example be V_{HH} sequences or may be humanized Nanobodies. When the above Nanobody sequences are V_{HH} sequences, they may be suitably humanized, as further described herein. When the Nanobodies are partially humanized Nanobodies, they may optionally be further suitably humanized, again as described herein.

In the above Nanobodies, one or more of the further Hallmark residues are preferably as described herein (for example, when they are V_{HH} sequences or partially humanized Nanobodies).

Preferably, the CDR sequences and FR sequences in the (single) domain antibodies and/or Nanobodies of the invention are such that the Nanobodies of the invention (and polypeptides of the invention comprising the same):
- bind to heterodimeric cytokines and/or their receptors with a dissociation constant (K_{D}) of 10⁻⁵ to 10⁻¹² moles/liter or less, and preferably 10⁻⁷ to 10⁻¹² moles/liter or less and more preferably 10⁻⁸ to 10⁻¹² moles/liter (i.e. with an association constant (R_{A}) of 10⁵ to 10¹² liter/ moles or more, and preferably 10⁷ to 10¹² liter/moles or more and more preferably 10⁸ to 10¹² liter/moles);
   and/or such that they:
- bind to "heterodimeric cytokines and/or their receptors with a kₒₙ-rate of between 10² M⁻¹s⁻¹ to about 10⁷ M⁻¹s⁻¹, preferably between 10³ M⁻¹s⁻¹ and 10⁷ M⁻¹s⁻¹, more preferably between 10⁴ M⁻¹s⁻¹ and 10⁷ M⁻¹s⁻¹, such as between 10⁵ M⁻¹s⁻¹ and 10⁷ M⁻¹s⁻¹;
   and/or such that they:
- bind to heterodimeric cytokines and/or their receptors with a k_{off} rate between 1s⁻¹ (t_{1/2}=0.69 s) and 10⁻⁶s⁻¹ (providing a near irreversible complex with a t_{1/2} of multiple days), preferably between 10⁻² s⁻¹ and 10⁻⁶ s⁻¹, more preferably between. 10⁻³ s⁻¹ and 10⁻⁶ s⁻¹, such as between 10⁻⁴ s⁻¹ and 10⁻⁶ s⁻¹.

Preferably, CDR sequences and FR sequences present in the Nanobodies of the invention are such that the Nanobodies of the invention will bind to heterodimeric cytokines and/or their receptors with an affinity less than 500 nM, preferably less than 200 nM, more preferably less than 10 µM, such as less than 500 pM.

According to one non-limiting aspect of the invention, a Nanobody may be as defined herein, but with the proviso that it has at least "one amino acid difference" (as defined herein) in at least one of the framework regions compared to the corresponding framework region of a naturally occurring human V_{H} domain, and in particular compared to the corresponding framework region of DP-47. More specifically, according to one non-limiting aspect of the invention, a Nanobody may be as defined herein, but with the proviso that it has at least "one amino acid difference" (as defined herein) at at least one of the Hallmark residues (including those at positions 108, 103 and/or 45) compared to the corresponding framework region of a naturally occurring human V_{H} domain, and in particular compared to the corresponding framework region of DP-47. Usually, a Nanobody will have at least one such amino acid difference with a naturally occurring V_{H} domain in at least one of FR2 and/or FR4, and in particular at at least one of the Hallmark residues in FR2 and/or FR4 (again, including those at positions 108, 103 and/or 45).

Also, a humanized Nanobody of the invention may be as defined herein, but with the proviso that it has at least "one amino acid difference" (as defined herein) in at least one of the framework regions compared to the corresponding framework region of a naturally occurring V_{HH} domain. More specifically, according to one non-limiting aspect of the invention, a humanized Nanobody may be as defined herein, but with the proviso that it has at least "one amino acid difference" (as defined herein) at at least one of the Hallmark residues (including those at positions 108, 103 and/or 45) compared to the corresponding framework region of a naturally occurring V_{HH} domain. Usually, a humanized Nanobody will have at least one such amino acid difference with a naturally occurring V_{HH} domain in at least one of FR2 and/or FR4, and in particular at at least one of the Hallmark residues in FR2 and/or FR4 (again, including those at positions 108, 103 and/or 45).

As will be clear from the disclosure herein, it is also within the scope of the invention to use natural or synthetic analogs, mutants, variants, alleles, homologs and orthologs (herein collectively referred to as *"analogs"*) of the Nanobodies of the invention as defined herein. Thus, according to one aspect of the invention, the term "Nanobody of the invention" in its broadest sense also covers such analogs.

Generally, in such analogs, one or more amino acid residues may have been replaced, deleted and/or added, compared to the Nanobodies of the invention as defined herein. Such substitutions, insertions or deletions may be made in one or more of the framework regions and/or in one or more of the CDR's. When such substitutions, insertions or deletions are made in one or more of the framework regions, they may be made at one or more of the Hallmark residues and/or at one or more of the other positions in the framework residues, although substitutions, insertions or deletions at the Hallmark residues are generally less preferred (unless these are suitable humanizing substitutions as described herein).

By means of non-limiting examples, a substitution may for example be a conservative substitution (as described herein) and/or an amino acid residue may be replaced by another amino acid residue that naturally occurs at the same position in another V_{HH} domain (see Tables A-5 to A-8 for some non-limiting examples of such substitutions), although the invention is generally not limited thereto. Thus, any one or more substitutions, deletions or insertions, or any combination thereof, that either improve the properties of the Nanobody of the invention or that at least do not detract too much from the desired properties or from the balance or combination of desired properties of the Nanobody of the invention (i.e. to the extent that the Nanobody is no longer suited for its intended use) are included within the scope of the invention. A skilled person will generally be able to determine and select suitable substitutions, deletions or insertions, or suitable combinations of thereof, based on the disclosure herein and optionally after a limited degree of routine experimentation, which may for example involve introducing a limited number of possible substitutions and determining their influence on the properties of the Nanobodies thus obtained.

For example, and depending on the host organism used to express the Nanobody or polypeptide of the invention, such deletions and/or substitutions may be designed in such a way that one or more sites for post-translational modification (such as one or more glycosylation sites) are removed, as will be within the ability of the person skilled in the art. Alternatively, substitutions or insertions may be designed so as to introduce one or more sites for attachment of functional groups (as described herein), for example to allow site-specific pegylation (again as described herein).

As can be seen from the data on the V_{HH} entropy and V_{HH} variability given in Tables A-5 to A-8 above, some amino acid residues in the framework regions are more conserved than others. Generally, although the invention in its broadest sense is not limited thereto, any substitutions, deletions or insertions are preferably made at positions that are less conserved. Also, generally, amino acid substitutions are preferred over amino acid deletions or insertions.

The analogs are preferably such that they can bind to heterodimeric cytokines and/or their receptors with an affinity (suitably measured and/or expressed as a K_{D}-value (actual or apparent), a K_{A}-value (actual or apparent), a kₒₙ-rate and/or a k_{off}-rate, or alternatively as an IC₅₀ value, as further described herein) that is as defined herein for the Nanobodies of the invention.

The analogs are preferably also such that they retain the favourable properties the Nanobodies, as described herein.

Also, according to one preferred aspect, the analogs have a degree of sequence identity of at least 70%, preferably at least 80%, more preferably at least 90%, such as at least 95% or 99% or more; and/or preferably have at most 20, preferably at most 10, even more preferably at most 5, such as 4, 3, 2 or only 1 amino acid difference (as defined herein), with one of the p19+ sequences, p19- sequences, p40- sequences, p40+ sequences, p35 sequences, IL-27 sequences, IL-12Rb1 sequences, IL-12Rb2 sequences or IL-23R sequences, respectively,that are mentioned in Table A-2.

Also, the framework sequences and CDR's of the analogs are preferably such that they are in accordance with the preferred aspects defined herein. More generally, as described herein, the analogs will have (a) a Q at position 108; and/or (b) a charged amino acid or a cysteine residue at position 45 and preferably an E at position 44, and more preferably E at position 44 and R at position 45; and/or (c) P, R or S at position 103.

One preferred class of analogs of the Nanobodies of the invention comprise Nanobodies that have been humanized (i.e. compared to the sequence of a naturally occurring Nanobody of the invention). As mentioned in the background art cited herein, such humanization generally involves replacing one or more amino acid residues in the sequence of a naturally occurring V_{HH} with the amino acid residues that occur at the same position in a human V_{H} domain, such as a human V_{H}3 domain. Examples of possible humanizing substitutions or combinations of humanizing substitutions will be clear to the skilled person, for example from the Tables herein, from the possible humanizing substitutions mentioned in the background art cited herein, and/or from a comparision between the sequence of a Nanobody and the sequence of a naturally occurring human V_{H} domain.

The humanizing substitutions should be chosen such that the resulting humanized Nanobodies still retain the favourable properties of Nanobodies as defined herein, and more preferably such that they are as described for analogs in the preceding paragraphs. A skilled person will generally be able to determine and select suitable humanizing substitutions or suitable combinations of humanizing substitutions, based on the disclosure herein and optionally after a limited degree of routine experimentation, which may for example involve introducing a limited number of possible humanizing substitutions and determining their influence on the properties of the Nanobodies thus obtained.

Generally, as a result of humanization, the Nanobodies of the invention may become more "human-like", while still retaining the favorable properties of the Nanobodies of the invention as described herein. As a result, such humanized Nanobodies may have several advantages, such as a reduced immunogenicity, compared to the corresponding naturally occurring V_{HH} domains. Again, based on the disclosure herein and optionally after a limited degree of routine experimentation, the skilled person will be able to select humanizing substitutions or suitable combinations of humanizing substitutions which optimize or achieve a desired or suitable balance between the favourable properties provided by the humanizing substitutions on the one hand and the favourable properties of naturally occurring V_{HH} domains on the other hand.

The Nanobodies of the invention may be suitably humanized at any framework residue(s), such as at one or more Hallmark residues (as defined herein) or at one or more other framework residues (i.e. non-Hallmark residues) or any suitable combination thereof. One preferred humanizing substitution for Nanobodies of the "P,R,S-103 group" or the "KERE group" is Q108 into L108. Nanobodies of the "GLEW class" may also be humanized by a Q108 into L108 substitution, provided at least one of the other Hallmark residues contains a camelid (camelizing) substitution (as defined herein). For example, as mentioned above, one particularly preferred class of humanized Nanobodies has GLEW or a GLEW-like sequence at positions 44-47; P, R or S (and in particular R) at position 103, and an L at position 108.

The humanized and other analogs, and nucleic acid sequences encoding the same, can be provided in any manner known per se. For example, the analogs can be obtained by providing a nucleic acid that encodes a naturally occurring V_{HH} domain, changing the codons for the one or more amino acid residues that are to be substituted into the codons for the corresponding desired amino acid residues (e.g. by site-directed mutagenesis or by PCR using suitable mismatch primers), expressing the nucleic acid/nucleotide sequence thus obtained in a suitable host or expression system; and optionally isolating and/or purifying the analog thus obtained to provide said analog in essentially isolated form (e.g. as further described herein). This can generally be performed using methods and techniques known per se, which will be clear to the skilled person, for example from the handbooks and references cited herein, the background art cited herein and/or from the further description herein. Alternatively, a nucleic acid encoding the desired analog can be synthesized in a manner known per se (for example using an automated apparatus for synthesizing nucleic acid sequences with a predefined amino acid sequence) and can then be expressed as described herein. Yet another technique may involve combining one or more naturally occurring and/or synthetic nucleic acid sequences each encoding a part of the desired analog, and then expressing the combined nucleic acid sequence as described herein. Also, the analogs can be provided using chemical synthesis of the pertinent amino acid sequence using techniques for peptide synthesis known per se, such as those mentioned herein.

In this respect, it: will be also be clear to the skilled person that the Nanobodies of the invention (including their analogs) can be designed and/or prepared starting from human V_{H} sequences (i.e. amino acid sequences or the corresponding nucleotide sequences), such as for example from human V_{H}3 sequences such as DP-47, DP-51 or DP-29, i.e. by introducing one or more camelizing substitutions (i.e. changing one or more amino acid residues in the amino acid sequence of said human V_{H} domain into the amino acid residues that occur at the corresponding position in a V_{HH} domain), so as to provide the sequence of a Nanobody of the invention and/or so as to confer the favourable properties of a Nanobody to the sequence thus obtained. Again, this can generally be performed using the various methods and techniques referred to in the previous paragraph, using an amino acid sequence and/or nucleotide sequence for a human V_{H} domain as a starting point.

Some preferred, but non-limiting camelizing substitutions can be derived from Tables A-5 - A-8. It will also be clear that camelizing substitutions at one or more of the Hallmark residues will generally have a greater influence on the desired properties than substitutions at one or more of the other amino acid positions, although both and any suitable combination thereof are included within the scope of the invention. For example, it is possible to introduce one or more camelizing substitutions that already confer at least some the desired properties, and then to introduce further camelizing substitutions that either further improve said properties and/or confer additional favourable properties. Again, the skilled person will generally be able to determine and select suitable camelizing substitutions or suitable combinations of camelizing substitutions, based on the disclosure herein and optionally after a limited degree of routine experimentation, which may for example involve introducing a limited number of possible camelizing substitutions and determining whether the favourable properties of Nanobodies are obtained or improved (i.e. compared to the original V_{H} domain).

Generally, however, such camelizing substitutions are preferably such that the resulting an amino acid sequence at least contains (a) a Q at position 108; and/or (b) a charged amino acid or a cysteine residue at position 45 and preferably also an E at position 44, and more preferably E at position 44 and R at position 45; and/or (c) P, R or S at position 103; and optionally one or more further camelizing substitutions. More preferably, the camelizing substitutions are such that they result in a Nanobody of the invention and/or in an analog thereof (as defined herein), such as in a humanized analog and/or preferably in an analog that is as defined in the preceding paragraphs.

As will also be clear from the disclosure herein, it is also within the scope of the invention to use parts or fragments, or combinations of two or more parts or fragments, of the Nanobodies of the invention as defined herein, and in particular parts or fragments of the p19+, p19-, p40+, p40-, anti p35, anti- IL-27, anti TL-12Rb1, anti TL-12Rb2 and anti IL-23R Nanobodies, respectively, as further described herein and/or as listed in Table A-2; or of humanized variants thereof. Thus, according to one aspect of the invention, the term "Nanobody of the invention" in its broadest sense also covers such parts or fragments.

Generally, such parts or fragments of the Nanobodies of the invention (including analogs thereof) have amino acid sequences in which, compared to the amino acid sequence of the corresponding full length Nanobody of the invention (or analog thereof), one or more of the amino acid residues at the N-terminal end, one or more amino acid residues at the C-terminal end, one or more contiguous internal amino acid residues, or any combination thereof, have been deleted and/or removed.

The parts or fragments are preferably such that they can bind to heterodimeric cytokines and/or their receptors with an affinity (suitably measured and/or expressed as a K_{D}-value (actual or apparent), a K_{A}-value (actual or apparent), a k_{OD}-rate and/or a k_{off}-rate, or alternatively as an IC₅₀ value, as further described herein) that is as defined herein for the Nanobodies of the invention.

Any part or fragment is preferably such that it comprises at least 10 contiguous amino acid residues, preferably at least 20 contiguous amino acid residues, more preferably at least 30 contiguous amino acid residues, such as at: least 40 contiguous amino acid residues, of the amino acid sequence of the corresponding full length Nanobody of the invention.

Also, any part or fragment is such preferably that it comprises at least one of CDR1, CDR2 and/or CDR3 or at least part thereof (and in particular at least CDR3 or at least part thereof). More preferably, any part or fragment is such that it comprises at least one of the CDR's (and preferably at least CDR3 or part thereof) and at least one other CDR (i.e. CDR1 or CDR2) or at least part thereof, preferably connected by suitable framework sequence(s) or at least part thereof. More preferably, any part or fragment is such that it comprises at least one of the CDR's (and preferably at least CDR3 or part thereof) and at least part of the two remaining CDR's, again preferably connected by suitable framework sequence(s) or at least part thereof.

According to another particularly preferred, but non-limiting aspect, such a part or fragment comprises at least CDR3, such as FR3, CDR3 and FR4 of the corresponding full length Nanobody of the invention, i.e. as for example described in the International application WO 03/050531 (Lasters et al.).

As already mentioned above, it is also possible to combine two or more of such parts or fragments (i.e. from the same or different Nanobodies of the invention), i.e. to provide an analog (as defined herein) and/or to provide further parts or fragments (as defined herein) of a Nanobody of the invention. It is for example also possible to combine one or more parts or fragments of a Nanobody of the invention with one or more parts or fragments of a human V_{H} domain.

According to one preferred aspect, the parts or fragments have a degree of sequence identity of at least 50%, preferably at least 60%, more preferably at least 70%, even more preferably at least 80%, such as at least 90%, 95% or 99% or more with one of the p19+, p19-, p40+, p40-, anti p35, anti- IL-27, anti IL-12Rb1, anti IL-12Rb2 and anti IL-23R Nanobodies, respectively, as further described herein and/or as listed in Table A-2; and/or with of humanized variants thereof.

The parts and fragments, and nucleic acid sequences encoding the same, can be provided and optionally combined in any manner known per se. For example, such parts or fragments can be obtained by inserting a stop codon in a nucleic acid that encodes a full-sized Nanobody of the invention, and then expressing the nucleic acid thus obtained in a manner known per se (e.g. as described herein). Alternatively, nucleic acids encoding such parts or fragments can be obtained by suitably restricting a nucleic acid that encodes a full-sized Nanobody of the invention or by synthesizing such a nucleic acid in a manner known per se. Parts or fragments may also be provided using techniques for peptide synthesis known per se.

The invention in its broadest sense also comprises derivatives of the Nanobodies of the invention. Such derivatives can generally be obtained by modification, and in particular by chemical and/or biological (e.g enzymatical) modification, of the Nanobodies of the invention and/or of one or more of the amino acid residues that form the Nanobodies of the invention.

Examples of such modifications, as well as examples of amino acid residues within the Nanobody sequence that can be modified in such a manner (i.e. either on the protein backbone but preferably on a side chain), methods and techniques that can be used to introduce such modifications and the potential uses and advantages of such modifications will be clear to the skilled person.

For example, such a modification may involve the introduction (e.g. by covalent linking or in an other suitable manner) of one or more functional groups, residues or moieties into or onto the Nanobody of the invention, and in particular of one or more functional groups, residues or moieties that confer one or more desired properties or functionalities to the Nanobody of the invention. Example of such functional groups will be clear to the skilled person.

For example, such modification may comprise the introduction (e.g. by covalent binding or in any other suitable manner) of one or more functional groups that increase the half-life, the solubility and/or the absorption of the Nanobody of the invention, that reduce the immunogenicity and/or the toxicity of the Nanobody of the invention, that eliminate or attenuate any undesirable side effects of the Nanobody of the invention, and/or that confer other advantageous properties to and/or reduce the undesired properties of the Nanobodies and/or polypeptides of the invention; or any combination of two or more of the foregoing. Examples of such functional groups and of techniques for introducing them will be clear to the skilled person, and can generally comprise all functional groups and techniques mentioned in the general background art cited hereinabove as well as the functional groups and techniques known per se for the modification of pharmaceutical proteins, and in particular for the modifications of antibodies or antibody fragments (including ScFv's and single domain antibodies), for which reference is for example made to Remington's Pharmaceutical Sciences, 16th ed., Mack Publishing Co., Easton, PA (1980). Such functional groups may for example be linked directly (for example covalently) to a Nanobody of the invention, or optionally via a suitable linker or spacer, as will again be clear to the skilled person.

One of the most widely used techniques for increasing the half-life and/or reducing the immunogenicity of pharmaceutical proteins comprises attachment of a suitable pharmacologically acceptable polymer, such as poly(ethyleneglycol) (PEG) or derivatives thereof (such as methoxypoly(ethyleneglycol) or mPEG). Generally, any suitable form of pegylation can be used, such as the pegylation used in the art for antibodies and antibody fragments (including but not limited to (single) domain antibodies and ScFv's); reference is made to for example Chapman, Nat. Biotechnol., 54, 531-545 (2002); by Veronese and Harris, Adv. Drug Deliv. Rev. 54, 453-456 (2003), by Harris and Chess, Nat. Rev. Drug. Discov., 2, (2003) and in WO 04/060965. Various reagents for pegylation of proteins are also commercially available, for example from Nektar Therapeutics, USA.

Preferably, site-directed pegylation is used, in particular via a cysteine-residue (see for example Y ang et al., Protein Engineering, 16, 10, 761-770 (2003). For example, for this purpose, PEG may be attached to a cysteine residue that naturally occurs in a Nanobody of the invention, a Nanobody of the invention may be modified so as to suitably introduce one or more cysteine residues for attachment of PEG, or an amino acid sequence comprising one or more cysteine residues for attachment of PEG may be fused to the N- and/or C-terminus of a Nanobody of the invention, all using techniques of protein engineering known per se to the skilled person.

Preferably, for the Nanobodies and proteins of the invention, a PEG is used with a molecular weight of more than 5000, such as more than 10,000 and less than 200,000, such as less than 100,000; for example in the range of 20,000-80,000.

Another, usually less preferred modification comprises N-linked or O-linked glycosylation, usually as part of co-translational and/or post-translational modification, depending on the host cell used for expressing the Nanobody or polypeptide of the invention.

Yet another modification may comprise the introduction of one or more detectable labels or other signal-generating groups or moieties, depending on the intended use of the labelled Nanobody Suitable labels and techniques for attaching, using and detecting them will be clear to the skilled person, and for example include, but are not limited to, fluorescent labels (such as fluorescein, isothiocyaiate, rhodamine, phycoerythrin, phycocyanin, allophycocyanin, o-phthaldehyde, and fluorescamine and fluorescent metals such as ¹⁵²Eu or others metals from the lanthanide series), phosphorescent labels, chemiluminescent labels or bioluminescent labels (such as luminal, isoluminol, theromatic acridinium ester, imidazole, acridinium salts, oxalate ester, dioxetane or GFP and its analogs), radio-isotopes (such as ³H, ¹²⁵I, ³²P, ³⁵S, ¹⁴C, ⁵¹Cr, ³⁶Cl, ⁵⁷Co, ⁵⁸Co, ⁵⁹Fe, and ⁷⁵Se), metals, metal chelates or metallic cations (for example metallic cations such as ^{99m}Tc, ¹²³I, ¹¹¹In, ¹³¹I, ⁹⁷Ru, ⁶⁷Cu, ⁶⁷Ga, and ⁶⁸Ga or other metals or metallic cations that are particularly suited for use in *in vivo, in vitro* or *in situ* diagnosis and imaging, such as (¹⁵⁷Gd, ⁵⁵Mn, ¹⁶²Dy, ⁵²Cr, and ⁵⁶Fe), as well as chromophores and enzymes (such as malate dehydrogenase, staphylococcal nuclease, delta-V-steroid isomerase, yeast alcohol dehydrogenase, alpha-glycerophosphate dehydrogenase, triose phosphate isomerase, biotinavidin peroxidase, horseradish peroxidase, alkaline phosphatase, asparaginase, glucose oxidase, beta-galactosidase, ribonuclease, urease, catalase, glucose-VI-phosphate dehydrogenase, glucoamylase and acetylcholine esterase). Other suitable labels will be clear to the skilled person, and for example include moieties that can be detected using NMR or ESR spectroscopy.

Such labelled Nanobodies and polypeptides of the invention may for example be used for in vitro, in vivo or in situ assays (including immunoassays known per se such as ELISA, RIA, EIA and other "sandwich assays", etc.) as well as in vivo diagnostic and imaging purposes, depending on the choice of the specific label.

As will be clear to the skilled person, another modification may involve the introduction of a chelating group, for example to chelate one of the metals or metallic cations referred to above. Suitable chelating groups for example include, without limitation, diethylenetriaminepentaacetic acid (DTPA) or ethylenediaminetetraacetic acid (EDTA).

Yet another modification may comprise the introduction of a functional group that is one part of a specific binding pair, such as the biotin-(strept)avidin binding pair. Such a functional group may be used to link the Nanobody of the invention to another protein, polypeptide or chemical compound that is bound to the other half of the binding pair, i.e. through formation of the binding pair. For example, a Nanobody of the invention may be conjugated to biotin, and linked to another protein, polypeptide, compound or carrier conjugated to avidin or streptavidin. For example, such a conjugated Nanobody may be used as a reporter, for example in a diagnostic system where a detectable signal-producing agent is conjugated to avidin or streptavidin. Such binding pairs may for example also be used to bind the Nanobody of the invention to a carrier, including carriers suitable for pharmaceutical purposes. One non-limiting example are the liposomal formulations described by Cao and Suresh, Journal of Drug Targetting, 8, 4, 257 (2000). Such binding pairs may also be used to link a therapeutically active agent to the Nanobody of the invention.

For some applications, in particular for those applications in which it is intended to kill a cell that expresses the target against which the Nanobodies of the invention are directed (e.g. in the treatment of cancer), or to reduce or slow the growth and/or proliferation such a cell, the Nanobodies of the invention may also be linked to a toxin or to a toxic residue or moiety. Examples of toxic moieties, compounds or residues which can be linked to a Nanobody of the invention to provide - for example - a cytotoxic compound will be clear to the skilled person and can for example be found in the prior art cited above and/or in the further description herein. One example is the so-called ADEPT™ technology described in WO 03/055527.

Other potential chemical and enzymatical modifications will be clear to the skilled person. Such modifications may also be introduced for research purposes (e.g. to study function-activity relationships). Reference is for example made to Lundblad and Bradshaw, Biotechnol. Appl. Biochem., 26, 143-151 (1997).

Preferably, the derivatives are such that they bind to heterodimeric cytokines and/or their receptors with an affinity (suitably measured and/or expressed as a K_{D}-value (actual or apparent), a K_{A}-value (actual or apparent), a kₒₙ-rate and/or a k_{off}-rate, or alternatively as an IC₅₀ value, as further described herein) that is as defined herein for the Nanobodies of the invention.

As mentioned above, the invention also relates to proteins or polypeptides that essentially consist of or comprise at least one Nanobody of the invention. By "essentially consist of" is meant that the amino acid sequence of the polypeptide of the invention either is exactly the same as the amino acid sequence of a Nanobody of the invention or corresponds to the amino acid sequence of a Nanobody of the invention which has a limited number of amino acid residues, such as 1-20 amino acid residues, for example 1-10 amino acid residues and preferably 1-6 amino acid residues, such as 1, 2, 3, 4, 5 or 6 amino acid residues, added at the amino terminal end, at the carboxy terminal end, or at both the amino terminal end and the carboxy terminal end of the amino acid sequence of the Nanobody.

Said amino acid residues may or may not change, alter or otherwise influence the (biological) properties of the Nanobody and may or may not add further functionality to the Nanobody. For example, such amino acid residues:
- can comprise an N-terminal Met residue, for example as result of expression in a heterologous host cell or host organism.
- may form a signal sequence or leader sequence that directs secretion of the Nanobody from a host cell upon synthesis. Suitable secretory leader peptides will be clear to the skilled person, and may be as further described herein. Usually, such a leader sequence will be linked to the N-terminus of the Nanobody, although the invention in its broadest sense is not limited thereto;
- may form a sequence or signal that allows the Nanobody to be directed towards and/or to penetrate or enter into specific organs, tissues, cells, or parts or compartments of cells, and/or that allows the Nanobody to penetrate or cross a biological barrier such as a cell membrane, a cell layer such as a layer of epithelial cells, a tumor including solid tumors, or the blood-brain-barrier. Examples of such amino acid sequences will be clear to the skilled person. Some non-limiting examples are the small peptide vectors ("Peptrans vectors") described in WO 03/026700 and in Temsamani et al., Expert Opin. Biol. Ther., 1, 773 (2001); Temsamani and Vidal, Drug Discov. Today, 9, 1012 (004) and Rousselle, J. Pharmacol. Exp. Ther., 296, 124-131 (2001), and the membrane translocator sequence described by Zhao et al., Apoptosis, 8, 631-637 (2003). C-terminal and N-terminal amino acid sequences for intracellular targeting of antibody fragments are for example described by Cardinale et al., Methods, 34, 171 (2004). Other suitable techniques for intracellular targeting involve the expression and/or use of so-called "intrabodies" comprising a Nanobody of the invention, as mentioned below;
- may form a "tag", for example an amino acid sequence or residue that allows or facilitates the purification of the Nanobody, for example using affinity techniques directed against said sequence or residue. Thereafter, said sequence or residue may be removed (e.g. by chemical or enzymatical cleavage) to provide the Nanobody sequence (for this purpose, the tag may optionally be linked to the Nanobody sequence via a cleavable linker sequence or contain a cleavable motif). Some preferred, but non-limiting examples of such residues are multiple histidine residues, glutatione residues and a myc-tag (see for example SEQ ID NO:31 of WO 06/12282).
- may be one or more amino acid residues that have been functionalized and/or that can serve as a site for attachment of functional groups. Suitable amino acid residues and functional groups will be clear to the skilled person and include, but are not limited to, the amino acid residues and functional groups mentioned herein for the derivatives of the Nanobodies of the invention.

According to another aspect, a polypeptide of the invention comprises a Nanobody of the invention, which is fused at its amino terminal end, at its carboxy terminal end, or both at its amino terminal end and at its carboxy terminal end to at least one further amino acid sequence, i.e. so as to provide a fusion protein comprising said Nanobody of the invention and the one or more further amino acid sequences. Such a fusion will also be referred to herein as a "Nanobody fusion".

The one or more further amino acid sequence may be any suitable and/or desired amino acid sequences. The further amino acid sequences may or may not change, alter or otherwise influence the (biological) properties of the Nanobody, and may or may not add further functionality to the Nanobody or the polypeptide of the invention. Preferably, the further amino acid sequence is such that it confers one or more desired properties or functionalities to the Nanobody or the polypeptide of the invention.

For example, the further amino acid sequence may also provide a second binding site, which binding site may be directed against any desired protein, polypeptide, antigen, antigenic determinant or epitope (including but not limited to the same protein, polypeptide, antigen, antigenic determinant or epitope against which the Nanobody of the invention is directed, or a different protein, polypeptide, antigen, antigen determinant or epitope).

Example of such amino acid sequences will be clear to the skilled person, and may generally comprise all amino acid sequences that are used in peptide fusions based on conventional antibodies and fragments thereof (including but not limited to ScFv's and single domain antibodies). Reference is for example made to the review by Holliger and Hudson, Nature Biotechnology, 23, 9, 1126-1136 (2005).

For example, such an amino acid sequence may be an amino acid sequence that increases the half-life, the solubility, or the absorption, reduces the immunogenicity or the toxicity, eliminates or attenuates undesirable side effects, and/or confers other advantageous properties to and/or reduces the undesired properties of the polypeptides of the invention, compared to the Nanobody of the invention per se. Some non-limiting examples of such amino acid sequences are serum proteins, such as human serum albumin (see for example WO 00/27435) or haptenic molecules (for example haptens that are recognized by circulating antibodies, see for example WO 98/22141).

In particular, it has been described in the art that linking fragments of immunoglobulins (such as V_{H} domains) to serum albumin or to fragments thereof can be used to increase the half-life. Reference is for made to WO 00/27435 and WO 01/077137). According to the invention, the Nanobody of the invention is preferably either directly linked to serum albumin (or to a suitable fragment thereof) or via a suitable linker, and in particular via a suitable peptide linked so that the polypeptide of the invention can be expressed as a genetic fusion (protein). According to one specific aspect, the Nanobody of the invention may be linked to a fragment of serum albumin that at least comprises the domain III of serum albumin or part thereof. Reference is for example made to the US provisional application 60/788,256 of Ablynx N.V. entitled *"Albumin derived amino acid sequence, use thereof for increasing the half-life of therapeutic proteins and of other therapeutic proteins and entities, and constructs comprising the same"* filed on March 31, 2006 (see also PCT/EP2007/002817).

Alternatively, the further amino acid sequence may provide a second binding site or binding unit that is directed against a serum protein (such as, for example, human serum albumin or another serum protein such as IgG), so as to provide increased half-life in serum. Such amino acid sequences for example include the Nanobodies described below, as well as the small peptides and binding proteins described in WO 91/01743, WO 01/45746 and WO 02/076489 and the dAb's described in WO 03/002609 and WO 04/003019. Reference is also made to Harmsen et al., Vaccine, 23 (41); 4926-42, 2005, as well as to EP 0 368 684, as well as to the following the US provisional applications 60/843,349 (see also PCT/EP2007/059475), 60/850,774 (see also PCT/EP2007/060849), 60/850,775 (see also PCT/EP2007/060850) by Ablynx N.V. mentioned herein and US provisional application of Ablynx N.V. entitled *"Peptides capable of binding to serum proteins"* filed on December 5, 2006 (see also WO 068280) as well as the US provisional applications 61/050,385 and 61/045,690 of Ablynx N.V. both entitled "Improved peptides capable of binding to serum proteins". Such amino acid sequences may in particular be directed against serum albumin (and more in particular human serum albumin) and/or against IgG (and more in particular human IgG). For example, such amino acid sequences may be amino acid sequences that are directed against (human) serum albumin and amino acid sequences that can bind to amino acid residues on (human) serum albumin that are not involved in binding of serum albumin to FcRn (see for example WO 06/0122787) and/or amino acid sequences that are capable of binding to amino acid residues on serum albumin that do not form part of domain III of serum albumin (see again for example WO 06/0122787); amino acid sequences that have or can provide an increased half-life (see for example the US provisional application 60/843,349 by Ablynx N.V. entitled *"Serum albumin binding proteins with long half-lives"* filed on September 8, 2006; see also PCT/EP2007/059475); amino acid sequences against human serum albumin that are cross-reactive with serum albumin from at least one species of mammal, and in particular with at least one species of primate (such as, without limitation, monkeys from the genus *Macaca* (such as, and in particular, cynomologus monkeys (*Macaca fascicularis*) and/or rhesus monkeys (*Macaca mulatta*)) and baboon (*Papio ursinus*), reference is again made to the US provisional application 60/843,349 and PCT/EP2007/059475); amino acid sequences that can bind to serum albumin in a pH independent manner (see for example the US provisional application 60/850,774 by Ablynx N.V. entitled *"Amino acid sequences that bind to serum proteins in a manner that is essentially independent of the pH, compounds comprising the same, and uses thereof",* filed on October 11, 2006, see also WO 08/043821) and/or amino acid sequences that are conditional binders (see for example the US provisional application 60/850,775 by Ablynx N.V. entitled "*Amino acid sequences that bind to a desired molecule in a conditional manner",* filed on October 11, 2006); see also PCT/EP2007/060850).

According to another aspect, the one or more further amino acid sequences may comprise one or more parts, fragments or domains of conventional 4-chain antibodies (and in particular human antibodies) and/or of heavy chain antibodies. For example, although usually less preferred, Nanobody of the invention may be linked to a conventional (preferably human) V_{H} or V_{L} domain or to a natural or synthetic analog of a V_{H} or V_{L} domain, again optionally via a linker sequence (including but not limited to other (single) domain antibodies, such as the dAb's described by Ward et al.).

The at least one Nanobody may also be linked to one or more (preferably human) C_{H}1, C_{H}2 and/or C_{H}3 domains, optionally via a linker sequence. For instance, a Nanobody linked to a suitable C_{H}1 domain could for example be used - together with suitable light chains - to generate antibody fragments/structures analogous to conventional Fab fragments or F(ab')₂ fragments, but in which one or (in case of an F(ab')₂ fragment) one or both of the conventional V_{H} domains have been replaced by Nanobody of the invention. Also, two Nanobodies could be linked to a C_{H}3 domain (optionally via a linker) to provide a construct with increased half-life in vivo.

According to one specific aspect of a polypeptide of the invention, one or more Nanobodies of the invention may be linked (optionally via a suitable linker or hinge region) to one or more constant domains (for example, 2 or 3 constant domains that can be used as part of/to form an Fc portion), to an Fc portion and/or to one or more antibody parts, fragments or domains that confer one or more effector functions to the polypeptide of the invention and/or may confer the ability to bind to one or more Fc receptors. For example, for this purpose, and without being limited thereto, the one or more further amino acid sequences may comprise one or more C_{H}2 and/or C_{H}3 domains of an antibody, such as from a heavy chain antibody (as described herein) and more preferably from a conventional human 4-chain antibody; and/or may form (part of) and Fc region, for example from IgG (e.g. from IgG1, IgG2, IgG3 or IgG4), from IgE or from another human Ig such as IgA, IgD or IgM. For example, WO 94/04678 describes heavy chain antibodies comprising a Camelid V_{HH} domain or a humanized derivative thereof (i.e. a Nanobody), in which the Camelidae C_{H}2 and/or C_{H}3 domain have been replaced by human C_{H}2 and C_{H}3 domains, so as to provide an immunoglobulin that consists of 2 heavy chains each comprising a Nanobody and human C_{H}2 and C_{H}3 domains (but no C_{H}1 domain), which immunoglobulin has the effector function provided by the C_{H}2 and C_{H}3 domains and which immunoglobulin can function without the presence of any light chains. Other amino acid sequences that can be suitably linked to the Nanobodies of the invention so as to provide an effector function will be clear to the skilled person, and may be chosen on the basis of the desired effector function(s). Reference is for example made to WO 04/058820, WO 99/42077; WO 02/056910 and WO 05/017148, as well as the review by Holliger and Hudson, supra; and to the non-prepublished US provisional application by Ablynx N.V. entitled *"Constructs comprising single variable domains and an Fc portion derived from IgE"* which has a filing date of December 4, 2007 and the corresponding PCT application based thereon (also invoked as priority for this application), which has the same filing date as the present application. Coupling of a Nanobody of the invention to an Fc portion may also lead to an increased half-life, compared to the corresponding Nanobody of the invention. For some applications, the use of an Fc portion and/or of constant domains (i.e. C_{H}2 and/or C_{H}3 domains) that confer increased half-life without any biologically significant effector function may also be suitable or even preferred. Other suitable constructs comprising one or more Nanobodies and one or more constant domains with increased half-life in vivo will be clear to the skilled person, and may for example comprise two Nanobodies linked to a C_{H}3 domain, optionally via a linker sequence. Generally, any fusion protein or derivatives with increased half-life will preferably have a molecular weight of more than 50 kD, the cut-off value for renal absorption.

In another one specific, but non-limiting, aspect, in order to form a polypeptide of the invention, one or more amino acid sequences of the invention may be linked (optionally via a suitable linker or hinge region) to naturally occurring, synthetic or semisynthetic constant domains (or analogs, variants, mutants, parts or fragments thereof) that have a reduced (or essentially no) tendency to self-associate into dimers (i.e. compared to constant domains that naturally occur in conventional 4-chain antibodies). Such monomeric (i.e. not self-associating) Fc chain variants, or fragments thereof, will be clear to the skilled person. For example, Helm et al., J Biol Chem 1996 271 7494, describe monomeric Fcε chain variants that can be used in the polypeptide chains of the invention.

Also, such monomeric Fc chain variants are preferably such that they are still capable of binding to the complement or the relevant Fc receptor(s) (depending on the Fc portion from which they are derived), and/or such that they still have some or all of the effector functions of the Fc portion from which they are derived (or at a reduced level still suitable for the intended use). Alternatively, in such a polypeptide chain of the invention, the monomeric Fc chain may be used to confer increased half-life upon the polypeptide chain, in which case the monomeric Fc chain may also have no or essentially no effector functions.

Bivalent/multivalent, bispecific/multi specific or biparatopic/multiparatopic polypeptides of the invention may also be linked to Fc portions, in order to provide polypeptide constructs of the type that is described in the non-prepublished US provisional application entitled *"immunoglobulin constructs"* filed on December 4, 2007 (also invoked as priority for this application) and the corresponding PCT application based thereon, which has the same filing date as the present application.

The further amino acid sequences may also form a signal sequence or leader sequence that directs secretion of the Nanobody or the polypeptide of the invention from a host cell upon synthesis (for example to provide a pre-, pro- or prepro- form of the polypeptide of the invention, depending on the host cell used to express the polypeptide of the invention).

The further amino acid sequence may also form a sequence or signal that allows the Nanobody or polypeptide of the invention to be directed towards and/or to penetrate or enter into specific organs, tissues, cells, or parts or compartments of cells, and/or that allows the Nanobody or polypeptide of the invention to penetrate or cross a biological barrier such as a cell membrane, a cell layer such as a layer of epithelial cells, a tumor including solid tumors, or the blood-brain-barrier. Suitable examples of such amino acid sequences will be clear to the skilled person, and for example include, but are not limited to, the "Peptrans" vectors mentioned above, the sequences described by Cardinale et al. and the amino acid sequences and antibody fragments known per se that can be used to express or produce the Nanobodies and polypeptides of the invention as so-called "intrabodies", for example as described in WO 94/02610, WO 95/22618, US-A-7004940, WO 03/014960, WO 99/07414; WO 05/01690; EP 1 512 696; and in Cattaneo, A. & Biocca, S. (1997) Intracellular Antibodies: Development and Applications. Landes and Springer-Verlag; and in Kontermann, Methods 34, (2004), 163-170, and the further references described therein.

For some applications, in particular for those applications in which it is intended to kill a cell that expresses the target against which the Nanobodies of the invention are directed (e.g. in the treatment of cancer), or to reduce or slow the growth and/or proliferation of such a cell, the Nanobodies of the invention may also be linked to a (cyto)toxic protein or polypeptide. Examples of such toxic proteins and polypeptides which can be linked to a Nanobody of the invention to provide - for example - a cytotoxic polypeptide of the invention will be clear to the skilled person and can for example be found in the prior art cited above and/or in the further description wherein. One example is the so-called ADEPT™ technology described in WO 03/055527.

According to one preferred, but non-limiting aspect, said one or more further amino acid sequences comprise at least one further Nanobody, so as to provide a polypeptide of the invention that comprises at least two, such as three, four, five or more Nanobodies, in which said Nanobodies may optionally be linked via one or more linker sequences (as defined herein). Polypeptides of the invention that comprise two or more Nanobodies, of which at least one is a Nanobody of the invention, will also be referred to herein as "multivalent" polypeptides of the invention, and the Nanobodies present in such polypeptides will also be referred to herein as being in a "multivalent format". For example a "bivalent" polypeptide of the invention comprises two Nanobodies, optionally linked via a linker sequence, whereas a "trivalent" polypeptide of the invention comprises three Nanobodies, optionally linked via two linker sequences; etc.; in which at least one of the Nanobodies present in the polypeptide, and up to all of the Nanobodies present in the polypeptide, is/are a Nanobody of the invention.

In a multivalent polypeptide of the invention, the two or more Nanobodies may be the same or different, and may be directed against the same antigen or antigenic determinant (for example against the same part(s) or epitope(s) or against different parts or epitopes) or may alternatively be directed against different antigens or antigenic determinants; or any suitable combination thereof. For example, a bivalent polypeptide of the invention may comprise (a) two identical Nanobodies; (b) a first Nanobody directed against a first antigenic determinant of a protein or antigen and a second Nanobody directed against the same antigenic determinant of said protein or antigen which is different from the first Nanobody; (c) a first Nanobody directed against a first antigenic determinant of a protein or antigen and a second Nanobody directed against another antigenic determinant of said protein or antigen; or (d) a first Nanobody directed against a first protein or antigen and a second Nanobody directed against a second protein or antigen (i.e. different from said first antigen). Similarly, a trivalent polypeptide of the invention may, for example and without being limited thereto, comprise (a) three identical Nanobodies; (b) two identical Nanobody against a first antigenic determinant of an antigen and a third Nanobody directed against a different antigenic determinant of the same antigen; (c) two identical Nanobody against a first antigenic determinant of an antigen and a third Nanobody directed against a second antigen different from said first antigen; (d) a first Nanobody directed against a first antigenic determinant of a first antigen, a second Nanobody directed against a second antigenic determinant of said first antigen and a third Nanobody directed against a second antigen different from said first antigen; or (e) a first Nanobody directed against a first antigen, a second Nanobody directed against a second antigen different from said first antigen, and a third Nanobody directed against a third antigen different from said first and second antigen.

Polypeptides of the invention that contain at least two Nanobodies, in which at least one Nanobody is directed against a first antigen (i.e. against heterodimeric cytokines and/or their receptors,) and at least one Nanobody is directed against a second antigen (i.e. different from heterodimeric cytokines and/or their receptors,), will also be referred to as "multispecific" polypeptides of the invention, and the Nanobodies present in such polypeptides will also be referred to herein as being in a "multispecific format". Thus, for example, a "bispecific" polypeptide of the invention is a polypeptide that comprises at least one Nanobody directed against a first antigen (i.e. heterodimeric cytokines and/or their receptors,) and at least one further Nanobody directed against a second antigen (i.e. different from "heterodimeric cytokines and/or their receptors,), whereas a "trispecific" polypeptide of the invention is a polypeptide that comprises at least one Nanobody directed against a first antigen (i.e. heterodimeric cytokines and/or their receptors,), at least one further Nanobody directed against a second antigen (i.e. different from heterodimeric cytokines and/or their receptors,) and at least one further Nanobody directed against a third antigen (i.e. different from both heterodimeric cytokines and/or their receptors, and the second antigen); etc.

Accordingly, in its simplest form, a bispecific polypeptide of the invention is a bivalent polypeptide of the invention (as defined herein), comprising a first Nanobody directed against heterodimeric cytokines and/or their receptors, and a second Nanobody directed against a second antigen, in which said first and second Nanobody may optionally be linked via a linker sequence (as defined herein); whereas a trispecific polypeptide of the invention in its simplest form is a trivalent polypeptide of the invention (as defined herein), comprising a first Nanobody directed against heterodimeric cytokines and/or their receptors, a second Nanobody directed against a second antigen and a third Nanobody directed against a third antigen, in which said first, second and third Nanobody may optionally be linked via one or more, and in particular one and more, in particular two, linker sequences.

However, as will be clear from the description hereinabove, the invention is not limited thereto, in the sense that a multispecific polypeptide of the invention may comprise at least one Nanobody against heterodimeric cytokines and/or their receptors, and any number of Nanobodies directed against one or more antigens different from heterodimeric cytokines and/or their receptors.

Furthermore, although it is encompassed within the scope of the invention that the specific order or arrangement of the various Nanobodies in the polypeptides of the invention may have some influence on the properties of the final polypeptide of the invention (including but not limited to the affinity, specificity or avidity for heterodimeric cytokines and/or their receptors, or against the one or more other antigens), said order or arrangement is usually not critical and may be suitably chosen by the skilled person, optionally after some limited routine experiments based on the disclosure herein. Thus, when reference is made to a specific multivalent or multispecific polypeptide of the invention, it should be noted that this encompasses any order or arrangements of the relevant Nanobodies, unless explicitly indicated otherwise.

Finally, it is also within the scope of the invention that the polypeptides of the invention contain two or more Nanobodies and one or more further amino acid sequences (as mentioned herein).

For multivalent and multispecific polypeptides containing one or more V_{HH} domains and their preparation, reference is also made to Conrath et al., J. Biol. Chem., Vol. 276, 10. 7346-7350, 2001; Muyldermans, Reviews in Molecular Biotechnology 74 (2001), 277-302; as well as to for example WO 96/34103 and WO 99/23221. Some other examples of some specific multispecific and/or multivalent polypeptide of the invention can be found in the applications by Ablynx N.V. referred to herein.

One preferred, but non-limiting example of a multispecific polypeptide of the invention comprises at least one Nanobody of the invention and at least one Nanobody that provides for an increased half-life. Such Nanobodies may for example be Nanobodies that are directed against a serum protein, and in particular a human serum protein, such as human serum albumin, thyroxine-binding protein, (human) transferrin, fibrinogen, an immunoglobulin such as IgG, IgE or IgM, or against one of the serum proteins listed in WO 04/003019. Of these, Nanobodies that can bind to serum albumin (and in particular human serum albumin) or to IgG (and in particular human IgG, see for example Nanobody VH-1 described in the review by Muyldermans, *supra*) are particularly preferred (although for example, for experiments in mice or primates, Nanobodies against or cross-reactive with mouse serum albumin (MSA) or serum albumin from said primate, respectively, can be used. However, for pharmaceutical use, Nanobodies against human serum albumin or human IgG will usually be preferred). Nanobodies that provide for increased half-life and that can be used in the polypeptides of the invention include the Nanobodies directed against serum albumin that are described in WO 04/041865, in WO 06/122787 and in the further patent applications by Ablynx N.V., such as those mentioned above.

For example, the some preferred Nanobodies that provide for increased half-life for use in the present invention include Nanobodies that can bind to amino acid residues on (human) serum albumin that are not involved in binding of serum albumin to FcRn (see for example WO 06/0122787); Nanobodies that are capable of binding to amino acid residues on serum albumin that do not form part of domain III of serum albumin (see for example WO 06/0122787); Nanobodies that have or can provide an increased half-life (see for example the US provisional application 60/843,349 by Ablynx N.V mentioned herein; see also PCT/EP2007/059475); Nanobodies against human serum albumin that are cross-reactive with serum albumin from at least one species of mammal, and in particular with at least one species of primate (such as, without limitation, monkeys from the genus *Macaca* (such as, and in particular, cynomologus monkeys (*Macaca fascicularis*) and/or rhesus monkeys (*Macaca mulatta*)) and baboon (*Papio ursinus*)) (see for example the US provisional application 60/843,349 by Ablynx N.V see also PCT/EP2007/059475); Nanobodies that can bind to serum albumin in a pH independent manner (see for example the US provisional application 60/850,774 by Ablynx N.V. mentioned herein, see also WO 08/043821) and/or Nanobodies that are conditional binders (see for example the US provisional application 60/850,775 by Ablynx N.V.; see also PCT/EP2007/060850).

Some particularly preferred Nanobodies that provide for increased half-life and that can be used in the polypeptides of the invention include the Nanobodies ALB-1 to ALB-10 disclosed in WO 06/122787 (see Tables II and III) of which ALB-8 (SEQ ID NO: 62 in WO 06/122787) is particularly preferred.

According to a specific, but non-limiting aspect of the invention, the polypeptides of the invention contain, besides the one or more Nanobodies of the invention, at least one Nanobody against human serum albumin.

Generally, any polypeptides of the invention with increased half-life that contain one or more Nanobodies of the invention, and any derivatives of Nanobodies of the invention or of such polypeptides that have an increased half-life, preferably have a half-life that is at least 1.5 times, preferably at least 2 times, such as at least 5 times, for example at least 10 times or more than 20 times, greater than the half-life of the corresponding Nanobody of the invention per se. For example, such a derivative or polypeptides with increased half-life may have a half-life that is increased with more than 1 hours, preferably more than 2 hours, more preferably more than 6 hours, such as more than 12 hours, or even more than 24, 48 or 72 hours, compared to the corresponding Nanobody of the invention per se.

In a preferred, but non-limiting aspect of the invention, such derivatives or polypeptides may exhibit a serum half-life in human of at least about 12 hours, preferably at least 24 hours, more preferably at least 48 hours, even more preferably at least 72 hours or more. For example, such derivatives or polypeptides may have a half-life of at least 5 days (such as about 5 to 10 days), preferably at least 9 days (such as about 9 to 14 days), more preferably at least about 10 days (such as about 10 to 15 days), or at least about 11 days (such as about 11 to 16 days), more preferably at least about 12 days (such as about 12 to 18 days or more), or more than 14 days (such as about 14 to 19 days).

According to one aspect of the invention the polypeptides are capable of binding to one or more molecules which can increase the half-life of the polypeptide in vivo.

The polypeptides of the invention are stabilised in vivo and their hall-life increased by binding to molecules which resist degradation and/or clearance or sequestration. Typically, such molecules are naturally occurring proteins which themselves have a long half-life in vivo.

Another preferred, but non-limiting example of a multispecific polypeptide of the invention comprises at least one Nanobody of the invention and at least one Nanobody that directs the polypeptide of the invention towards, and/or that allows the polypeptide of the invention to penetrate or to enter into specific organs, tissues, cells, or parts or compartments of cells, and/or that allows the Nanobody to penetrate or cross a biological barrier such as a cell membrane, a cell layer such as a layer of epithelial cells, a tumor including solid tumors, or the blood-brain-barrier. Examples of such Nanobodies include Nanobodies that are directed towards specific cell-surface proteins, markers or epitopes of the desired organ, tissue or cell (for example cell-surface markers associated with tumor cells), and the single-domain brain targeting antibody fragments described in WO 02/057445 and WO 06/040153, of which FC44 (SEQ ID NO: 189 of WO 06/040153) and FC5 (SEQ ID NO: 190 of WO 06/040154) are preferred examples.

In the polypeptides of the invention, the one or more Nanobodies and the one or more polypeptides may be directly linked to each other (as for example described in WO 99/23221) and/or may be linked to each other via one or more suitable spacers or linkers, or any combination thereof.

Suitable spacers or linkers for use in multivalent and multispecific polypeptides will be clear to the skilled person, and may generally be any linker or spacer used in the art to link amino acid sequences. Preferably, said linker or spacer is suitable for use in constructing proteins or polypeptides that are intended for pharmaceutical use.

Some particularly preferred spacers include the spacers and linkers that are used in the art to link antibody fragments or antibody domains. These include the linkers mentioned in the general background art cited above, as well as for example linkers that are used in the art to construct diabodies or ScFv fragments (in this respect, however, its should be noted that, whereas in diabodies and in ScFv fragments, the linker sequence used should have a length, a degree of flexibility and other properties that allows the pertinent V_{H} and V_{L} domains to come together to form the complete antigen-binding site, there is no particular limitation on the length or the flexibility of the linker used in the polypeptide of the invention, since each Nanobody by itself forms a complete antigen-binding site).

For example, a linker may be a suitable amino acid sequence, and in particular amino acid sequences of between 1 and 50, preferably between 1 and 30, such as between 1 and 10 amino acid residues. Some preferred examples of such amino acid sequences include gly-ser linkers, for example of the type (glyₓser_{y})_{z}, such as (for example (gly₄ser)₃ or (gly₃ser₂)₃, as described in WO 99/42077 and the GS30, GS15, GS9 and GS7 linkers described in the applications by Ablynx mentioned herein (see for example WO 06/040153 and WO 06/122825), as well as hinge-like regions, such as the hinge regions of naturally occurring heavy chain antibodies or similar sequences (such as described in WO 94/04678).

Some other particularly preferred linkers are poly-alanine (such as AAA), as well as the linkers GS30 (SEQ ID NO: 85 in WO 06/122825) and GS9 (SEQ ID NO: 84 in WO 06/122825).

Other suitable linkers generally comprise organic compounds or polymers, in particular those suitable for use in proteins for pharmaceutical use. For instance, poly(ethyleneglycol) moieties have been used to link antibody domains, see for example WO 04/081026.

It is encompassed within the scope of the invention that the length, the degree of flexibility and/or other properties of the linker(s) used (although not critical, as it usually is for linkers used in ScFv fragments) may have some influence on the properties of the final polypeptide of the invention, including but not limited to the affinity, specificity or avidity for heterodimeric cytokines and/or their receptors, or for one or more of the other antigens. Based on the disclosure herein, the skilled person will be able to determine the optimal linker(s) for use in a specific polypeptide of the invention, optionally after some limited routine experiments.

For example, in multivalent polypeptides of the invention that comprise Nanobodies directed against a multimeric antigen (such as a multimeric receptor or other proteins), the length and flexibility of the linker are preferably such that it allows each Nanobody of the invention present in the polypeptide to bind to the antigenic determinant on each of the subunits of the multimer. Similarly, in a multispecific polypeptide of the invention that comprises Nanobodies directed against two or more different antigenic determinants on the same antigen (for example against different epitopes of an antigen and/or against different subunits of a multimeric receptor, channel or protein), the length and flexibility of the linker are preferably such that it allows each Nanobody to bind to its intended antigenic determinant. Again, based on the disclosure herein, the skilled person will be able to determine the optimal linker(s) for use in a specific polypeptide of the invention, optionally after some limited routine experiments.

It is also within the scope of the invention that the linker(s) used confer one or more other favourable properties or functionality to the polypeptides of the invention, and/or provide one or more sites for the formation of derivatives and/or for the attachment of functional groups (e.g. as described herein for the derivatives of the Nanobodies of the invention). For example, linkers containing one or more charged amino acid residues (see Table A-3 above) can provide improved hydrophilic properties, whereas linkers that form or contain small epitopes or tags can be used for the purposes of detection, identification and/or purification. Again, based on the disclosure herein, the skilled person will be able to determine the optimal linkers for use in a specific polypeptide of the invention, optionally after some limited routine experiments.

Finally, when two or more linkers are used in the polypeptides of the invention, these linkers may be the same or different. Again, based on the disclosure herein, the skilled person will be able to determine the optimal linkers for use in a specific polypeptide of the invention, optionally after some limited routine experiments.

Usually, for easy of expression and production, a polypeptide of the invention will be a linear polypeptide. However, the invention in its broadest sense is not limited thererto. For example, when a polypeptide of the invention comprises three of more Nanobodies, it is possible to link them by use of a linker with three or more "arms", which each "arm" being linked to a Nanobody, so as to provide a "star-shaped" construct. It is also possible, although usually less preferred, to use circular constructs.

The invention also comprises derivatives of the polypeptides of the invention, which may be essentially analogous to the derivatives of the Nanobodies of the invention, i.e. as described herein.

The invention also comprises proteins or polypeptides that "essentially consist" of a polypeptide of the invention (in which the wording "essentially consist of" has essentially the same meaning as indicated hereinabove).

According to one aspect of the invention, the polypeptide of the invention is in essentially isolated from, as defined herein.

The amino acid sequences, Nanobodies, polypeptides and nucleic a.cids of the invention can be prepared in a manner known per se, as will be clear to the skilled person from the further description herein. For example, the Nanobodies and polypetides of the invention can be prepared in any manner known per se for the preparation of antibodies and in particular for the preparation of antibody fragments (including but not limited to (single) domain antibodies and ScFv fragments). Some preferred, but non-limiting methods for preparing the amino acid sequences, Nanobodies, polypeptides and nucleic acids include the methods and techniques described herein.

As will be clear to the skilled person, one particularly useful method for preparing an amino acid sequence, Nanobody and/or a polypeptide of the invention generally comprises the steps of:
i) the expression, in a suitable host cell or host organism (also referred to herein as a "host of the invention") or in another suitable expression system of a nucleic acid that encodes said amino acid sequence, Nanobody or polypeptide of the invention (also referred to herein as a "*nucleic acid of the invention*"), optionally followed by:
ii) isolating and/or purifying the amino acid sequence, Nanobody or polypeptide of the invention thus obtained.

In particular, such a method may comprise the steps of:
i) cultivating and/or maintaining a host of the invention under conditions that are such that said host of the invention expresses and/or produces at least one amino acid sequence, Nanobody and/or polypeptide of the invention; optionally followed by:
ii) isolating and/or purifying the amino acid sequence, Nanobody or polypeptide of the invention thus obtained.

A nucleic acid of the invention can be in the form of single or double stranded DNA or RNA, and is preferably in the form of double stranded DNA. For example, the nucleotide sequences of the invention may be genomic DNA, cDNA or synthetic DNA (such as DNA with a codon usage that has been specifically adapted for expression in the intended host cell or host organism).

According to one aspect of the invention, the nucleic acid of the invention is in essentially isolated from, as defined herein.

The nucleic acid of the invention may also be in the form of, be present in and/or be part of a vector, such as for example a plasmid, cosmid or YAC, which again may be in essentially isolated form.

The nucleic acids of the invention can be prepared or obtained in a manner known per se, based on the information on the amino acid sequences for the polypeptides of the invention given herein, and/or can be isolated from a suitable natural source. To provide analogs, nucleotide sequences encoding naturally occurring V_{HH} domains can for example be subjected to site-directed mutagenesis, so at to provide a nucleic acid of the invention encoding said analog. Also, as will be clear to the skilled person, to prepare a nucleic acid of the invention, also several nucleotide sequences, such as at least one nucleotide sequence encoding a Nanobody and for example nucleic acids encoding one or more linkers can be linked together in a suitable manner.

Techniques for generating the nucleic acids of the invention will be clear to the skilled person and may for instance include, but are not limited to, automated DNA synthesis; site-directed mutagenesis; combining two or more naturally occurring and/or synthetic sequences (or two or more parts thereof), introduction of mutations that lead to the expression of a truncated expression product; introduction of one or more restriction sites (e.g. to create cassettes and/or regions that may easily be digested and/or ligated using suitable restriction enzymes), and/or the introduction of mutations by means of a PCR reaction using one or more "mismatched" primers, using for example a sequence of a naturally occurring form of heterodimeric cytokines and/or their receptors as a template. These and other techniques will be clear to the skilled person, and reference is again made to the standard handbooks, such as Sambrook et al. and Ausubel et al., mentioned above, as well as the Examples below.

The nucleic acid of the invention may also be in the form of, be present in and/or be part of a genetic construct, as will be clear to the person skilled in the art. Such genetic constructs generally comprise at least one nucleic acid of the invention that is optionally linked to one or more elements of genetic constructs known per se, such as for example one or more suitable regulatory elements (such as a suitable promoter(s), enhancer(s), terminator(s), etc.) and the further elements of genetic constructs referred to herein. Such genetic constructs comprising at least one nucleic acid of the invention will also be referred to herein as "genetic constructs of the invention".

The genetic constructs of the invention may be DNA or RNA, and are preferably double-stranded DNA. The genetic constructs of the invention may also be in a form suitable for transformation of the intended host cell or host organism, in a form suitable for integration into the genomic DNA of the intended host cell or in a form suitable for independent replication, maintenance and/or inheritance in the intended host organism. For instance, the genetic constructs of the invention may be in the form of a vector, such as for example a plasmid, cosmid, YAC, a viral vector or transposon. In particular, the vector may be an expression vector, i.e. a vector that can provide for expression in vitro and/or in vivo (e.g. in a suitable host cell, host organism and/or expression system).

In a preferred but non-limiting aspect, a genetic construct of the invention comprises
i) at least one nucleic acid of the invention; operably connected to
ii) one or more regulatory elements, such as a promoter and optionally a suitable terminator;
   and optionally also
iii) one or more further elements of genetic constructs known per se;
in which the terms "regulatory element", "promoter", "terminator" and "operably connected" have their usual meaning in the art (as further described herein); and in which said "further elements" present in the genetic constructs may for example be 3'- or 5'-UTR sequences, leader sequences, selection markers, expression markers/reporter genes, and/or elements that may facilitate or increase (the efficiency of) transformation or integration. These and other suitable elements for such genetic constructs will be clear to the skilled person, and may for instance depend upon the type of construct used, the intended host cell or host organism; the manner in which the nucleotide sequences of the invention of interest are to be expressed (e.g. via constitutive, transient or inducible expression); and/or the transformation technique to be used. For example, regulatory requences, promoters and terminators know per se for the expression and production of antibodies and antibody fragments (including but not limited to (single) domain antibodies and ScFv fragments) may be used in an essentially analogous manner.

Preferably, in the genetic constructs of the invention, said at least one nucleic acid of the invention and said regulatory elements, and optionally said one or more further elements, are "operably linked" to each other, by which is generally meant that they are in a functional relationship with each other. For instance, a promoter is considered "operably linked" to a coding sequence if said promoter is able to initiate or otherwise control/regulate the transcription and/or the expression of a coding sequence (in which said coding sequence should be understood as being "under the control of" said promotor). Generally, when two nucleotide sequences are operably linked, they will be in the same orientation and usually also in the same reading frame. They will usually also be essentially contiguous, although this may also not be required.

Preferably, the regulatory and further elements of the genetic constructs of the invention are such that they are capable of providing their intended biological function in the intended host cell or host organism.

For instance, a promoter, enhancer or terminator should be "operable" in the intended host cell or host organism, by which is meant that (for example) said promoter should be capable of initiating or otherwise controlling/regulating the transcription and/or the expression of a nucleotide sequence - e.g. a coding sequence - to which it is operably linked (as defined herein).

Some particularly preferred promoters include, but are not limited to, promoters known per se for the expression in the host cells mentioned herein; and in particular promoters for the expression in the bacterial cells, such as those mentioned herein and/or those used in the Examples.

A selection marker should be such that it allows - i.e. under appropriate selection conditions - host cells and/or host organisms that have been (successfully) transformed with the nucleotide sequence of the invention to be distinguished from host cells/organisms that have not been (successfully) transformed. Some preferred, but non-limiting examples of such markers are genes that provide resistance against antibiotics (such as kanamycin or ampicillin), genes that provide for temperature resistance, or genes that allow the host cell or host organism to be maintained in the absence of certain factors, compounds and/or (food) components in the medium that are essential for survival of the non-transformed cells or organisms.

A leader sequence should be such that - in the intended host cell or host organism - it allows for the desired post-translational modifications and/or such that it directs the transcribed mRNA to a desired part or organelle of a cell. A leader sequence may also allow for secretion of the expression product from said cell. As such, the leader sequence may be any pro-, pre-, or prepro-sequence operable in the host cell or host organism. Leader sequences may not be required for expression in a bacterial cell. For example, leader sequences known per se for the expression and production of antibodies and antibody fragments (including but not limited to single domain antibodies and ScFv fragments) may be used in an essentially analogous manner.

An expression marker or reporter gene should be such that - in the host cell or host organism - it allows for detection of the expression of (a gene or nucleotide sequence present on) the genetic construct. An expression marker may optionally also allow for the localisation of the expressed product, e.g. in a specific part or organelle of a cell and/or in (a) specific cell(s), tissue(s), organ(s) or part(s) of a multicellular organism. Such reporter genes may also be expressed as a protein fusion with the amino acid sequence of the invention. Some preferred, but non-limiting examples include fluorescent proteins such as GFP.

Some preferred, but non-limiting examples of suitable promoters, terminator and further elements include those that can be used for the expression in the host cells mentioned herein; and in particular those that are suitable for expression in bacterial cells, such as those mentioned herein and/or those used in the Examples below. For some (further) non-limiting examples of the promoters, selection markers, leader sequences, expression markers and further elements that may be present/used in the genetic constructs of the invention - such as terminators, transcriptional and/or translational enhancers and/or integration factors - reference is made to the general handbooks such as Sambrook et al. and Ausubel et al. mentioned above, as well as to the examples that are given in WO 95/07463, WO 96/23810, WO 95/07463, WO 95/21191, WO 97/11094, WO 97/42320, WO 98/06737, WO 98/21355, US-A-7,207,410, US-A- 5,693,492 and EP 1 085 089. Other examples will be clear to the skilled person. Reference is also made to the general background art cited above and the further references cited herein.

The genetic constructs of the invention may generally be provided by suitably linking the nucleotide sequence(s) of the invention to the one or more further elements described above, for example using the techniques described in the general handbooks such as Sambrook et al. and Ausubel et al., mentioned above.

Often, the genetic constructs of the invention will be obtained by inserting a nucleotide sequence of the invention in a suitable (expression) vector known per se. Some preferred, but non-limiting examples of suitable expression vectors are those used in the Examples below, as well as those mentioned herein.

The nucleic acids of the invention and/or the genetic constructs of the invention may be used to transform a host cell or host organism, i.e. for expression and/or production of the amino acid sequence, Nanobody or polypeptide of the invention. Suitable hosts or host cells will be clear to the skilled person, and may for example be any suitable fungal, prokaryotic or eukaryotic cell or cell line or any suitable fungal, prokaryotic or eukaryotic organism, for example:
- a bacterial strain, including but not limited to gram-negative strains such as strains of *Escherichia coli*; of *Proteus,* for example of *Proteus mirabilis*; of *Pseudomonas,* for example of *Pseudomonas fluorescens;* and gram-positive strains such as strains of *Bacillus,* for example of *Bacillus subtilis* or of *Bacillus brevis;* of *Streptomyces,* for example of *Streptomyces lividans; of Staphylococcus,* for example of *Staphylococcus carnosus;* and *of Lactococcus,* for example of *Lactococcus lactis;*
- a fungal cell, including but not limited to cells from species of *Trichoderma,* for example from *Trichoderma reesei;* of *Neurospora,* for example from *Neurospora crassa;* of *Sordaria,* for example from *Sordaria macrospora; of Aspergillus,* for example from *Aspergillus niger* or from *Aspergillus sojae*; or from other filamentous fungi;
- a yeast cell, including but not limited to cells from species of *Saccharomyces,* for example of *Saccharomyces cerevisiae*; of *Schizosaccharomyces,* for example of *Schizosaccharomyces pombe*; of *Pichia,* for example of *Pichia pastoris* or of *Pichia methanolica;* of *Hansenula,* for example of *Hansenula polymorpha*; of *Kluyveromyces,* for example of *Kluyveromyces lactis*; of *Arxula,* for example *of Arxula adeninivorans*; of *Yarrowia,* for examples of *Yarrowia lipolytica;*
- an amphibian cell or cell line, such as *Xenopus oocytes*;
- an insect-derived cell or cell line, such as cells/cell lines derived from lepidoptera, including but not limited to *Spodoptera* SF9 and Sf21 cells or cells/cell lines derived from *Drosophila,* such as Schneider and Kc cells;
- a plant or plant cell, for example in tobacco plants; and/or
- a mammalian cell or cell line, for example a cell or cell line derived from a human, a cell or a cell line from mammals including but not limited to CHO-cells, BHK-cells (for example BHK-21 cells) and human cells or cell lines such as HeLa, COS (for example COS-7) and PER.C6 cells;
as well as all other hosts or host cells known per se for the expression and production of antibodies and antibody fragments (including but not limited to (single) domain antibodies and ScFv fragments), which will be clear to the skilled person. Reference is also made to the general background art cited hereinabove, as well as to for example WO 94/29457; WO 96/34103; WO 99/42077; Frenken et al., (1998), supra; Riechmann and Muyldermans, (1999), supra; van der Linden, (2000), supra; Thomassen et. al., (2002), supra; Joosten et al., (2003), supra; Joosten et al., (2005), supra; and the further references cited herein.

The Amino acid sequences, Nanobodies and polypeptides of the invention can also be introduced and expressed in one or more cells, tissues or organs of a multicellular organism, for example for prophylactic and/or therapeutic purposes (e.g. as a gene therapy). For this purpose, the nucleotide sequences of the invention may be introduced into the cells or tissues in any suitable way, for example as such (e.g. using liposomes) or after they have been inserted into a suitable gene therapy vector (for example derived from retroviruses such as adenovirus, or parvoviruses such as adeno-associated virus). As will also be clear to the skilled person, such gene therapy may be performed in vivo and/or in situ in the body of a patient by administering a nucleic acid of the invention or a suitable gene therapy vector encoding the same to the patient or to specific cells or a specific tissue or organ of the patient; or suitable cells (often taken from the body of the patient to be treated, such as explanted lymphocytes, bone marrow aspirates or tissue biopsies) may be treated in vitro with a nucleotide sequence of the invention and then be suitably (re-)introduced into the body of the patient. All this can be performed using gene therapy vectors, techniques and delivery systems which are well known to the skilled person, and for example described in Culver, K. W., "Gene Therapy", 1994, p. xii, Mary Ann Liebert, Inc., Publishers, New York, N.Y); Giordano, Nature F Medicine 2 (1996), 534-539; Schaper, Circ. Res. 79 (1996), 911-919; Anderson, Science 256 (1992),808-813; Verma, Nature 389 (1994),239; Isner, Lancet 348 (1996),370-374; Muhlhauser, Circ. Res. 77 (1995),1077-1086; Onodera, Blood 91; (1998),30-36; Verma, Gene Ther. 5 (1998),692-699; Nabel, Ann. N.Y. Acad. Sci. : 811 (1997), 289-292); Verzeletti, Hum. Gene Ther. 9 (1998), 2243-51; Wang, Nature Medicine 2 (1996),714-716; WO 94/29469; WO 97/00957, US 5,580,859; US 5,5895466; or Schaper, Current Opinion in Biotechnology 7 (1996), 635-640. For example, in situ expression of ScFv fragments (Afanasieva et al., Gene Ther., 10, 1850-1859 (2003)) and of diabodies (Blanco et al., J. Immunol, 171, 1070-1077 (2003)) has been described in the art.

For expression of the Nanobodies in a cell, they may also be expressed as so-called "intrabodies", as for example described in WO 94/02610, WO 95/22618 and US-A-7004940; WO 03/014960; in Cattaneo, A. & Biocca, S. (1997) Intracellular Antibodies: Development and Applications. Landes and Springer-Verlag; and in Kontermann, Methods 34, (2004), 163-170.

The amino acid sequences, Nanobodies and polypeptides of the invention can for example also be produced in the milk of transgenic mammals, for example in the milk of rabbits, cows, goats or sheep (see for example US-A-6,741,957, US-A-6,304,489 and US-A-6,849,992 for general techniques for introducing transgenes into mammals), in plants or parts of plants including but not limited to their leaves, flowers, fruits, seed, roots or turbers (for example in tobacco, maize, soybean or alfalfa) or in for example pupae of the silkworm *Bombix mori.*

Furthermore, the amino acid sequences, Nanobodies and polypeptides of the invention can also be expressed and/or produced in cell-free expression systems, and suitable examples of such systems will be clear to the skilled person. Some preferred, but non-limiting examples include expression in the wheat germ system; in rabbit reticulocyte lysates; or in the *E. coli* Zubay system.

As mentioned above, one of the advantages of the use of Nanobodies is that the polypeptides based thereon can be prepared through expression in a suitable bacterial system, and suitable bacterial expression systems, vectors, host cells, regulatory elements, etc., will be clear to the skilled person, for example from the references cited above. It should however be noted that the invention in its broadest sense is not limited to expression in bacterial systems.

Preferably, in the invention, an (in vivo or in vitro) expression system, such as a bacterial expression system, is used that provides the polypeptides of the invention in a form that is suitable for pharmaceutical use, and such expression systems will again be clear to the skilled person. As also will be clear to the skilled person, polypeptides of the invention suitable for pharmaceutical use can be prepared using techniques for peptide synthesis.

For production on industrial scale, preferred heterologous hosts for the (industrial) production of Nanobodies or Nanobady-containing protein therapeutics include strains of E. *coli, Pichia pastoris, S. cerevisiae* that are suitable for large scale expression/production/fermentation, and in particular for large scale pharmaceutical (i.e. GMP grade) expression/production/fermentation. Suitable examples of such strains will be clear to the skilled person. Such strains and production/expression systems are also made available by companies such as Biovitrum (Uppsala, Sweden),

Alternatively, mammalian cell lines, in particular Chinese hamster ovary (CHO) cells, can be used for large scale expression/production/fermentation, and in particular for large scale pharmaceutical expression/production/fermentation. Again, such expression/production systems are also made available by some of the companies mentioned above.

The choice of the specific expression system would depend in part on the requirement for certain post-translational modifications, more specifically glycosylation. The production of a Nanobody-containing recombinant protein for which glycosylation is desired or required would necessitate the use of mammalian expression hosts that have the ability to glycosylate the expressed protein. In this respect, it will be clear to the skilled person that the glycosylation pattern obtained (i.e. the kind, number and position of residues attached) will depend on the cell or cell line that is used for the expression. Preferably, either a human cell or cell line is used (i.e. leading to a protein that essentially has a human glycosylation pattern) or another mammalian cell line is used that can provide a glycosylation pattern that is essentially and/or functionally the same as human glycosylation or at least mimics human glycosylation. Generally, prokaryotic hosts such as *E. coli* do not have the ability to glycosylate proteins, and the use of lower eukaryotes such as yeast usually leads to a glycosylation pattern that differs from human glycosylation. Nevertheless, it should be understood that all the foregoing host cells and expression systems can be used in the invention, depending on the desired amino acid sequence, Nanobody or polypeptide to be obtained.

Thus, according to one non-limiting aspect of the invention, the amino acid sequence, Nanobody or polypeptide of the invention is glycosylated. According to another non-limiting aspect of the invention, the amino acid sequence, Nanobody or polypeptide of the invention is non-glycosylated.

According to one preferred, but non-limiting aspect of the invention, the amino acid sequence, Nanobody or polypeptide of the invention is produced in a bacterial cell, in particular a bacterial cell suitable for large scale pharmaceutical production, such as cells of the strains mentioned above.

According to another preferred, but non-limiting aspect of the invention, the amino acid sequence, Nanobody or polypeptide of the invention is produced in a yeast cell, in particular a yeast cell suitable for large scale pharmaceutical production, such as cells of the species mentioned above.

According to yet another preferred, but non-limiting aspect of the invention, the amino acid sequence, Nanobody or polypeptide of the invention is produced in a mammalian cell, in particular in a human cell or in a cell of a human cell line, and more in particular in a human cell or in a cell of a human cell line that is suitable for large scale pharmaceutical production, such as the cell lines mentioned hereinabove.

When expression in a host cell is used to produce the amino acid sequences, Nanobodies and the polypeptides of the invention, the amino acid sequences, Nanobodies and polypeptides of the invention can be produced either intracellullarly (e.g. in the cytosol, in the periplasma or in inclusion bodies) and then isolated from the host cells and optionally further purified; or can be produced extracellularly (e.g. in the medium in which the host cells are cultured) and then isolated from the culture medium and optionally further purified. When eukaryotic host cells are used, extracellular production is usually preferred since this considerably facilitates the further isolation and downstream processing of the Nanobodies, and proteins obtained. Bacterial cells such as the strains of *E. coli* mentioned above normally do not secrete proteins extracellularly, except for a few classes of proteins such as toxins and hemolysin, and secretory production in *E. coli* refers to the translocation of proteins across the inner membrane to the periplasmic space. Periplasmic production provides several advantages over cytosolic production. For example, the N-terminal amino acid sequence of the secreted product can be identical to the natural gene product after cleavage of the secretion signal sequence by a specific signal peptidase. Also, there appears to be much less protease activity in the periplasm than in the cytoplasm. In addition, protein purification is simpler due to fewer contaminating proteins in the periplasm. Another advantage is that correct disulfide bonds may form because the periplasm provides a more oxidative environment than the cytoplasm. Proteins overexpressed in *E. coli* are often found in insoluble aggregates, so-called inclusion bodies. These inclusion bodies may be located in the cytosol or in the periplasm; the recovery of biologically active proteins from these inclusion bodies requires a denaturation/refolding process. Many recombinant proteins, including therapeutic proteins, are recovered from inclusion bodies. Alternatively, as will be clear to the skilled person, recombinant strains of bacteria that have been genetically modified so as to secrete a desired protein, and in particular an amino acid sequence, Nanobody or a polypeptide of the invention, can be used.

Thus, according to one non-limiting aspect of the invention, the amino acid sequence, Nanobody or polypeptide of the invention is an amino acid sequence, Nanobody or polypeptide that has been produced intracellularly and that has been isolated from the host cell, and in particular from a bacterial cell or from an inclusion body in a bacterial cell. According to another non-limiting aspect of the invention, the amino acid sequence, Nanobody or polypeptide of the invention is an amino acid sequence, Nanobody or polypeptide that has been produced extracellularly, and that has been isolated from the medium in which the host cell is cultivated.

Some preferred, but non-limiting promoters for use with these host cells include,
- for expression in *E*. *coli:* lac promoter (and derivatives thereof such as the lacUV5 promoter); arabinose promoter; left- (PL) and rightward (PR) promoter of phage lambda; promoter of the trp operon; hybrid lac/trp promoters (tac and trc); T7-promoter (more specifically that of T7-phage gene 10) and other T-phage promoters; promoter of the Tn10 tetracycline resistance gene; engineered variants of the above promoters that include one or more copies of an extraneous regulatory operator sequence;
- for expression in *S*. *cerevisiae:* constitutive: ADH1 (alcohol dehydrogenase 1), ENO (enolase), CYC1 (cytochrome c iso-1), GAPDH (glyceraldehydes-3-phosphate dehydrogenase), PGK1 (phosphoglycerate kinase), PYK1 (pyruvate kinase); regulated: GAL1,10,7 (galactose metabolic enzymes), ADH2 (alcohol dehydrogenase 2), PHO5 (acid phosphatase), CUP1 (copper metallothionein); heterologous: CaMV (cauliflower mosaic virus 35S promoter);
- for expression in *Pichia pastoris:* the AOX1 promoter (alcohol oxidase I);
- for expression in mammalian cells: human cytomegalovirus (heterodimeric cytokinesMV) immediate early enhancer/promoter; human cytomegalovirus (heterodimeric cytokinesMV) immediate early promoter variant that contains two tetracycline operator sequences such that the promoter can be regulated by the Tet repressor; Herpes Simplex Virus thymidine kinase (TK) promoter; Rous Sarcoma Virus long terminal repeat (RSV LTR) enhancer/promoter; elongation factor 1α (hFF-1α) promoter from human, chimpanzee, mouse or rat; the SV40 early promoter; HIV-1 long terminal repeat promoter; β-actin promoter;

Some preferred, but non-limiting vectors for use with these host cells include:
- vectors for expression in mammalian cells: pMAMneo (Clontech), pcDNA3 (Invitrogen), pMC1neo (Stratagene), pSG5 (Stratagene), EBO-pSV2-neo (ATCC 37593), pBPV-1 (8-2) (ATCC 37110), pdBPV-MMTneo (342-12) (ATCC 37224), pRSVgpt (ATCC37199), pRSVneo (ATCC37198), pSV2-dhfr (ATCC 37146), pUCTag (ATCC 37460) and 1ZD35 (ATCC 37565), as well as viral-based expression systems, such as those based on adenovirus;
- vectors for expression in bacterial cells: pET vectors (Novagen) and pQE vectors (Qiagen);
- vectors for expression in yeast or other fungal cells: pYES2 (Invitrogen) and *Pichia* expression vectors (lnvitrogen);
- vectors for expression in insect cells: pBlueBacII (Invitrogen) and other baculovirus vectors
- vectors for expression in plants or plant cells: for example vectors based on cauliflower mosaic virus or tobacco mosaic virus, suitable strains of *Agrobacterium,* or Ti-plasmid based vectors.

Some preferred, but non-limiting secretory sequences for use with these host cells include:
- for use in bacterial cells such as *E. coli:* PeIB, Bla, OmpA, OmpC, OmpF, OmpT, StII, PhoA, PhoE, MalE, Lpp, LamB, and the like; TAT signal peptide, hemolysin C-terminal secretion signal;
- for use in yeast: α-mating factor prepro-sequence, phosphatase (phol), invertase (Suc), etc.;
- for use in mammalian cells: indigenous signal in case the target protein is of eukaryotic origin; murine Ig κ-chain V-J2-C signal peptide; etc.

Suitable techniques for transforming a host or host cell of the invention will be clear to the skilled person and may depend on the intended host cell/host organism and the genetic construct to be used. Reference is again made to the handbooks and patent applications mentioned above.

After transformation, a step for detecting and selecting those host cells or host organisms that have been succesfullly transformed with the nucleotide sequence/genetic construct of the invention may be performed. This may for instance be a selection step based on a selectable marker present in the genetic construct of the invention or a step involving the detection of the amino acid sequence of the invention, e.g. using specific antibodies.

The transformed host cell (which may be in the form or a stable cell line) or host organisms (which may be in the form of a stable mutant line or strain) form further aspects of the present invention.

Preferably, these host cells or host organisms are such that they express, or are (at least) capable of expressing (e.g. under suitable conditions), an amino acid sequence, Nanobody or polypeptide of the invention (and in case of a host organism: in at least one cell, part, tissue or organ thereof). The invention also includes further generations, progeny and/or offspring of the host cell or host organism of the invention, that may for instance be obtained by cell division or by sexual or asexual reproduction.

To produce/obtain expression of the amino acid sequences of the invention, the transformed host cell or transformed host organism may generally be kept, maintained and/or cultured under conditions such that the (desired) amino acid sequence, Nanobody or polypeptide of the invention is expressed/produced. Suitable conditions will be clear to the skilled person and will usually depend upon the host cell/host organism used, as well as on the regulatory elements that control the expression of the (relevant) nucleotide sequence of the invention. Again, reference is made to the handbooks and patent applications mentioned above in the paragraphs on the genetic constructs of the invention.

Generally, suitable conditions may include the use of a suitable medium, the presence of a suitable source of food and/or suitable nutrients, the use of a suitable temperature, and optionally the presence of a suitable inducing factor or compound (e.g. when the nucleotide sequences of the invention are under the control of an inducible promoter); all of which may be selected by the skilled person. Again, under such conditions, the amino acid sequences of the invention may be expressed in a constitutive manner, in a transient manner, or only when suitably induced.

It will also be clear to the skilled person that the amino acid sequence, Nanobody or polypeptide of the invention may (first) be generated in an immature form (as mentioned above), which may then be subjected to post-translational modification, depending on the host cell/host organism used. Also, the amino acid sequence, Nanobody or polypeptide of the invention may be glycosylated, again depending on the host cell/host organism used.

The amino acid sequence, Nanobody or polypeptide of the invention may then be isolated from the host cell/host organism and/or from the medium in which said host cell or host organism was cultivated, using protein isolation and/or purification techniques known per se, such as (preparative) chromatography and/or electrophoresis techniques, differential precipitation techniques, affinity techniques (e.g. using a specific, cleavable amino acid sequence fused with the amino acid sequence, Nanobody or polypeptide of the invention) and/or preparative immunological techniques (i.e. using antibodies against the amino acid sequence to be isolated).

Generally, for pharmaceutical use, the polypeptides of the invention may be formulated as a Pharmaceutical preparation or compositions comprising at least one polypeptide of the invention and at least one pharmaceutically acceptable carrier, diluent or excipient and/or adjuvant, and optionally one or more further pharmaceutically active polypeptides and/or compounds. By means of non-limiting examples, such a formulation may be in a form suitable for oral administration, for parenteral administration (such as by intravenous, intramuscular or subcutaneous injection or intravenous infusion), for topical administration (for example, as a cream for the prevention and/or treatment of superficial inflammation, such as psoriasis), for administration by inhalatian, by a skin patch, by an implant, by a suppository, etc. Such suitable administration forms - which may be solid, semi-solid or liquid, depending on the manner of administration - as well as methods and carriers for use in the preparation thereof, will be clear to the skilled person, and are further described herein.

Thus, in a further aspect, the invention relates to a pharmaceutical composition that contains at least one amino acid of the invention, at least one Nanobody of the invention or at least one polypeptide of the invention and at least one suitable carrier, diluent or excipient (i.e. suitable for pharmaceutical use), and optionally one or more further active substances.

Generally, the amino acid sequences, Nanobodies and polypeptides of the invention can be formulated and administered in any suitable manner known per se, for which reference is for example made to the general background art cited above (and in particular to WO 04/041862, WO 04/041863, WO 04/041865 and WO 04/041867) as well as to the standard handbooks, such as Remington's Pharmaceutical Sciences, 18th Ed., Mack Publishing Company, USA (1990) or Remington, the Science and Practice of Pharmacy, 21th Edition, Lippincott Williams and Wilkins (2005).

For example, the amino acid sequences, Nanobodies and polypeptides of the invention may be formulated and administered in any manner known per se for conventional antibodies and antibody fragments (including ScFv's and diabodies) and other pharmaceutically active proteins. Such formulations and methods for preparing the same will be clear to the skilled person, and for example include preparations suitable for parenteral administration (for example intravenous, intraperitoneal, subcutaneous, intramuscular, intraluminal, intra-arterial or intrathecal administration) or for topical (i.e. transdermal or intradermal) administration.

Preparations for parenteral administration may for example be sterile solutions, suspensions, dispersions or emulsions that are suitable for infusion or injection. Suitable carriers or diluents for such preparations for example include, without limitation, sterile water and aqueous buffers and solutions such as physiological phosphate-buffered saline, Ringer's solutions, dextrose solution, and Hank's solution; water oils; glycerol; ethanol; glycols such as propylene glycol or as well as mineral oils, animal oils and vegetable oils, for example peanut oil, soybean oil, as well as suitable mixtures thereof. Usually, aqueous solutions or suspensions will be preferred.

The amino acid sequences, Nanobodies and polypeptides of the invention can also be administered using gene therapy methods of delivery. See, e.g., U.S. Patent No. 5,399,346, which is incorporated by reference in its entirety. Using a gene therapy method of delivery, primary cells transfected with the gene encoding an amino acid sequence, Nanobody or polypeptide of the invention can additionally be transfected with tissue specific promoters to target specific organs, tissue, grafts, tumors, or cells and can additionally be transfected with signal and stabilization sequences for subcellularly localized expression.

Thus, the amino acid sequences, Nanobodies and polypeptides of the invention may be systemically administered, e.g., orally, in combination with a pharmaceutically acceptable vehicle such as an inert diluent or an assimilable edible carrier. They may be enclosed in hard or soft shell gelatin capsules, may be compressed into tablets, or may be incorporated directly with the food of the patient's diet. For oral therapeutic administration, the amino acid sequences, Nanobodies and polypeptides of the invention may be combined with one or more excipients and used in the form of ingestible tablets, buccal tablets, troches, capsules, elixirs, suspensions, syrups, wafers, and the like. Such compositions and preparations should contain at least 0.1% of the amino acid sequence, Nanobody or polypeptide of the invention. Their percentage in the compositions and preparations may, of course, be varied and may conveniently be between about 2 to about 60% of the weight of a given unit dosage form. The amount of the amino acid sequence, Nanobody or polypeptide of the invention in such therapeutically useful compositions is such that an effective dosage level will be obtained.

The tablets, troches, pills, capsules, and the like may also contain the following: binders such as gum tragacanth, acacia, corn starch or gelatin; excipients such as dicalcium phosphate; a disintegrating agent such as corn starch, potato starch, alginic acid and the like; a lubricant such as magnesium stearate; and a sweetening agent such as sucrose, fructose, lactose or aspartame or a flavoring agent such as peppermint, oil of wintergreen, or cherry flavoring may be added. When the unit dosage form is a capsule, it may contain, in addition to materials of the above type, a liquid carrier, such as a vegetable oil or a polyethylene glycol. Various other materials may be present as coatings or to otherwise modify the physical form of the solid unit dosage form. For instance, tablets, pills, or capsules may be coated with gelatin, wax, shellac or sugar and the like. A syrup or elixir may contain the amino acid sequences, Nanobodies and polypeptides of the invention, sucrose or fructose as a sweetening agent, methyl and propylparabens as preservatives, a dye and flavoring such as cherry or orange flavor. Of course, any material used in preparing any unit dosage form should be pharmaceutically accepTable And substantially non-toxic in the amounts employed. In addition, the amino acid sequences, Nanobodies and polypeptides of the invention may be incorporated into sustained-release preparations and devices.

Preparations and formulations for oral administration may also be provided with an enteric coating that will allow the constructs of the invention to resist the gastric environment and pass into the intestines. More generally, preparations and formulations for oral administration may be suitably formulated for delivery into any desired part of the gastrointestinal tract. In addition, suitable suppositories may be used for delivery into the gastrointestinal tract.

The amino acid sequences, Nanobodies and polypeptides of the invention may also be administered intravenously or intraperitoneally by infusion or injection. Solutions of the amino acid sequences, Nanobodies and polypeptides of the invention or their salts can be prepared in water, optionally mixed with a nontoxic surfactant. Dispersions can also be prepared in glycerol, liquid polyethylene glycols, triacetin, and mixtures thereof and in oils. Under ordinary conditions of storage and use, these preparations contain a preservative to prevent the growth of microorganisms.

The pharmaceutical dosage forms suitable for injection or infusion can include sterile aqueous solutions or dispersions or sterile powders comprising the active ingredient which are adapted for the extemporaneous preparation of sterile injectable or infusible solutions or dispersions, optionally encapsulated in liposomes. In all cases, the ultimate dosage form must be sterile, fluid and stable under the conditions of manufacture and storage. The liquid carrier or vehicle can be a solvent or liquid dispersion medium comprising, for example, water, ethanol, a polyol (for example, glycerol, propylene glycol, liquid polyethylene glycols, and the like), vegetable oils, nontoxic glyceryl esters, and suitable mixtures thereof. The proper fluidity can be maintained, for example, by the formation of liposomes, by the maintenance of the required particle size in the case of dispersions or by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars, buffers or sodium chloride. Prolonged absorption of the injectable compositions can be brought about by the use in the compositions of agents delaying absorption, for example, aluminum monostearate and gelatin.

Sterile injectable solutions are prepared by incorporating the amino acid sequences, Nanobodies and polypeptides of the invention in the required amount in the appropriate solvent with various of the other ingredients enumerated above, as required, followed by filter sterilization. In the case of sterile powders for the preparation of sterile injectable solutions, the preferred methods of preparation are vacuum drying and the freeze drying techniques, which yield a powder of the active ingredient plus any additional desired ingredient present in the previously sterile-filtered solutions.

For topical administration, the amino acid sequences, Nanobodies and polypeptides of the invention may be applied in pure form, i.e., when they are liquids. However, it will generally be desirable to administer them to the skin as compositions or formulations, in combination with a dermatologically acceptable carrier, which may be a solid or a liquid.

Useful solid carriers include finely divided solids such as talc, clay, microcrystalline cellulose, silica, alumina and the like. Useful liquid carriers include water, hydroxyalkyls or glycols or water-alcohol/glycol blends, in which the amino acid sequences, Nanobodies and polypeptides of the invention can be dissolved or dispersed at effective levels, optionally with the aid of non-toxic surfactants. Adjuvants such as fragrances and additional antimicrobial agents can be added to optimize the properties for a given use. The resultant liquid compositions can be applied from absorbent pads, used to impregnate bandages and other dressings, or sprayed onto the affected area using pump-type or aerosol sprayers.

Thickeners such as synthetic polymers, fatty acids, fatty acid salts and esters, fatty alcohols, modified celluloses or modified mineral materials can also be employed with liquid carriers to form spreadable pastes, gels, ointments, soaps, and the like, for application directly to the skin of the user.

Examples of useful dermatological compositions which can be used to deliver the amino acid sequences, Nanabodies and polypeptides of the invention to the skin are known to the art; for example, see Jacquet et al. (U.S. Pat. No. 4,608,392), Geria (U.S. Pat. No. 4,992,478), Smith et al. (U.S. Pat. No. 4,559,157) and Wortzman (U.S. Pat. No. 4,820,508).

Useful dosages of the amino acid sequences, Nanobodies and polypeptides of the invention can be determined by comparing their *in vitro* activity, and *in vivo* activity in animal models. Methods for the extrapolation of effective dosages in mice, and other animals, to humans are known to the art; for example, see U.S. Pat. No. 4,938,949.

Generally, the concentration of the amino acid sequences, Nanobodies and polypeptides of the invention in a liquid composition, such as a lotion, will be from about 0.1-25 wt-%, preferably from about 0.5-10 wt-%. The concentration in a semi-solid or solid composition such as a gel or a powder will be about 0.1-5 wt-%, preferably about 0.5-2.5 wt-%.

The amount of the amino acid sequences, Nanobodies and polypeptides of the invention required for use in treatment will vary not only with the particular amino acid sequence, Nanobody or polypeptide selected but also with the route of administration, the nature of the condition being treated and the age and condition of the patient and will be ultimately at the discretion of the attendant physician or clinician. Also the dosage of the amino acid sequences, Nanobodies and polypeptides of the invention varies depending on the target cell, tumor, tissue, graft, or organ.

The desired dose may conveniently be presented in a single dose or as divided doses administered at appropriate intervals, for example, as two, three, four or more sub-doses per day. The sub-dose itself may be further divided, e.g., into a number of discrete loosely spaced administrations; such as multiple inhalations from an insufflator or by application of a plurality of drops into the eye.

An administration regimen could include long-term, daily treatment. By "long-term" is meant at least two weeks and preferably, several weeks, months, or years of duration. Necessary modifications in this dosage range may be determined by one of ordinary skill in the art using only routine experimentation given the teachings herein. See Remington's Pharmaceutical Sciences (Martin, E.W., ed. 4), Mack Publishing Co., Easton, PA. The dosage can also be adjusted by the individual physician in the event of any complication.

In another a.spect, the invention relates to a method for the prevention and/or treatment of at least one diseases and disorders associated with heterodimeric cytokines and their receptors, said method comprising administering, to a subject in need thereof, a pharmaceutically active amount of an amino acid sequence of the invention, of a Nanobody of the invention, of a polypeptide of the invention, and/or of a pharmaceutical composition comprising the same.

In the context of the present invention, the term "prevention and/or treatment" not only comprises preventing and/or treating the disease, but also generally comprises preventing the onset of the disease, slowing or reversing the progress of disease, preventing or slowing the onset of one or more symptoms associated with the disease, reducing and/or alleviating one or more symptoms associated with the disease, reducing the severity and/or the duration of the disease and/or of any symptoms associated therewith and/or preventing a further increase in the severity of the disease and/or of any symptoms associated therewith, preventing, reducing or reversing any physiological damage caused by the disease, and generally any pharmacological action that is beneficial to the patient being treated.

The subject to be treated may be any warm-blooded animal, but is in particular a mammal, and more in particular a human being. As will be clear to the skilled person, the subject to be treated will in particular be a person suffering from, or at risk of, the diseases and disorders mentioned herein.

The invention relates to a method for the prevention and/or treatment of at least one disease or disorder that is associated with heterodimeric cytokines and/or their receptors, with its biological or pharmacological activity, and/or with the biological pathways or signalling in which heterodimeric cytokines and/or their receptors is involved, said method comprising administering, to a subject in need thereof, a pharmaceutically active amount of an amino acid sequence of the invention, of a Nanobody of the invention, of a polypeptide of the invention, and/or of a pharmaceutical composition comprising the same. In particular, the invention relates to a method for the prevention and/or treatment of at least one disease or disorder that can be treated by modulating heterodimeric cytokines and/or their receptors, its biological or pharmacological activity, and/or the biological pathways or signalling in which heterodimeric cytokines and/or their receptors is involved, said method comprising administering, to a subject in need thereof, a pharmaceutically active amount of an amino acid sequence of the invention, of a Nanobody of the invention, of a polypeptide of the invention, and/or of a pharmaceutical composition comprising the same. In particular, said pharmaceutically effective amount may be an amount that is sufficient to modulate heterodimeric cytokines and/or their receptors, its biological or pharmacological activity, and/or the biological pathways or signalling in which heterodimeric cytokines and/or their receptors is involved; and/or an amount that provides a level of the amino acid sequence of the invention, of a Nanobody of the invention, of a polypeptide of the invention in the circulation that is sufficient to modulate heterodimeric cytokines and/or their receptors, its biological or pharmacological activity, and/or the biological pathways or signalling in which heterodimeric cytokines and/or their receptors is involved.

The invention furthermore relates to a method for the prevention and/or treatment of at least one disease or disorder that can be prevented and/or treated by administering an amino acid sequence of the invention, a Nanobody of the invention or a polypeptide of the invention to a patient, said method comprising administering, to a subject in need thereof, a pharmaceutically active amount of an amino acid sequence of the invention, of a Nanobody of the invention, of a polypeptide of the invention, and/or of a pharmaceutical composition comprising the same.

More in particular, the invention relates to a method for the prevention and/or treatment of at least one disease or disorder chosen from the group consisting of the diseases and disorders listed herein, said method comprising administering, to a subject in need thereof, a pharmaceutically active amount of an amino acid sequence of the invention, of a Nanobody of the invention, of a polypeptide of the invention, and/or of a pharmaceutical composition comprising the same.

In another aspect, the invention relates to a method for immunotherapy, and in particular for passive immunotherapy, which method comprises administering, to a subject suffering from or at risk of the diseases and disorders mentioned herein, a pharmaceutically active amount of an amino acid sequence of the invention, of a Nanobody of the invention, of a polypeptide of the invention, and/or of a pharmaceutical composition comprising the same.

In the above methods, the amino acid sequences, Nanobodies and/or polypeptides of the invention and/or the compositions comprising the same can be administered in any suitable manner, depending on the specific pharmaceutical formulation or composition to be used. Thus, the amino acid sequences, Nanobodies and/or polypeptides of the invention and/or the compositions comprising the same can for example be administered orally, intraperitoneally (e.g. intravenously, subcutaneously, intramuscularly, or via any other route of administration that circumvents the gastrointestinal tract), intranasally, transdermally, topically, by means of a suppository, by inhalation, again depending on the specific pharmaceutical formulation or composition to be used. The clinician will be able to select a suitable route of administration and a suitable pharmaceutical formulation or composition to be used in such administration, depending on the disease or disorder to be prevented or treated and other factors well known to the clinician.

The amino acid sequences, Nanobodies and/or polypeptides of the invention and/or the compositions comprising the same are administered according to a regime of treatment that is suitable for preventing and/or treating the disease or disorder to be prevented or treated. The clinician will generally be able to determine a suitable treatment regimen, depending on factors such as the disease or disorder to be prevented or treated, the severity of the disease to be treated and/or the severity of the symptoms thereof, the specific amino acid sequence, Nanobody or polypeptide of the invention to be used, the specific route of administration and pharmaceutical formulation or composition to be used, the age, gender, weight, diet, general condition of the patient, and similar factors well known to the clinician.

Generally, the treatment regimen will comprise the administration of one or more amino acid sequences, Nanobodies and/or polypeptides of the invention, or of one or more compositions comprising the same, in one or more pharmaceutically effective amounts or doses. The specific amount(s) or doses to administered can be determined by the clinician, again based on the factors cited above.

Generally, for the prevention and/or treatment of the diseases and disorders mentioned herein and depending on the specific disease or disorder to be treated, the potency of the specific amino acid sequence, Nanobody and polypeptide of the invention to be used, the specific route of administration and the specific pharmaceutical formulation or composition used, the amino acid sequences, Nanobodies and polypeptides of the invention will generally be administered in an amount between 1 gram and 0.01 microgram per kg body weight per day, preferably between 0.1 gram and 0.1 microgram per kg body weight per day, such as about 1, 10, 100 or 1000 microgram per kg body weight per day, either continuously (e.g. by infusion), as a single daily dose or as multiple divided doses during the day. The clinician will generally be able to determine a suitable daily dose, depending on the factors mentioned herein. It will also be clear that in specific cases, the clinician may choose to deviate from these amounts, for example on the basis of the factors cited above and his expert judgment. Generally, some guidance on the amounts to be administered can be obtained from the amounts usually administered for comparable conventional antibodies or antibody fragments against the same target administered via essentially the same route, taking into account however differences in affinity/avidity, efficacy, biodistribution, half-life and similar factors well known to the skilled person.

Usually, in the above method, a single amino acid sequence, Nanobody or polypeptide of the invention will be used. It is however within the scope of the invention to use two or more amino acid sequences, Nanobodies and/or polypeptides of the invention in combination.

The Nanobodies, amino acid sequences and polypeptides of the invention may also be used in combination with one or more further pharmaceutically active compounds or principles, i.e. as a combined treatment regimen, which may or may not lead to a synergistic effect. Again, the clinician will be able to select such further compounds or principles, as well as a suitable combined treatment regimen, based on the factors cited above and his expert judgement.

In particular, the amino acid sequences, Nanobodies and polypeptides of the invention may be used in combination with other pharmaceutically active compounds or principles that are or can be used for the prevention and/or treatment of the diseases and disorders cited herein, as a result of which a synergistic effect may or may not be obtained. Examples of such compounds and principles, as well as routes, methods and pharmaceutical formulations or compositions for administering them will be clear to the clinician.

When two or more substances or principles are to be used as part of a combined treatment regimen, they can be administered via the same route of administration or via different routes of administration, at essentially the same time or at different times (e.g. essentially simultaneously, consecutively, or according to an alternating regime). When the substances or principles are to be administered simultaneously via the same route of administration, they may be administered as different pharmaceutical formulations or compositions or part of a combined pharmaceutical formulation or composition, as will be clear to the skilled person.

Also, when two or more active substances or principles are to be used as part of a combined treatment regimen, each of the substances or principles may be administered in the same amount and according to the same regimen as used when the compound or principle is used on its own, and such combined use may or may not lead to a synergistic effect. However, when the combined use of the two or more active substances or principles leads to a synergistic effect, it may also be possible to reduce the amount of one, more or all of the substances or principles to be administered, while still achieving the desired therapeutic action. This may for example be useful for avoiding, limiting or reducing any unwanted side-effects that are associated with the use of one or more of the substances or principles when they are used in their usual amounts, while still obtaining the desired pharmaceutical or therapeutic effect.

The effectiveness of the treatment regimen used according to the invention may be determined and/or followed in any manner known per se for the disease or disorder involved, as will be clear to the clinician. The clinician will also be able, where appropriate and on a case-by-case basis, to change or modify a particular treatment regimen, so as to achieve the desired therapeutic effect, to avoid, limit or reduce unwanted side-effects, and/or to achieve an appropriate balance between achieving the desired therapeutic effect on the one hand and avoiding, limiting or reducing undesired side effects on the other hand.

Generally, the treatment regimen will be followed until the desired therapeutic effect is achieved and/or for as long as the desired therapeutic effect is to be maintained. Again, this can be determined by the clinician.

In another aspect, the invention relates to the use of an amino acid sequence, Nanobody or polypeptide of the invention in the preparation of a pharmaceutical composition for prevention and/or treatment of at least one diseases and disorders associated with heterodimeric cytokines and their receptors; and/or for use in one or more of the methods of treatment mentioned herein.

The subject to be treated may be any warm-blooded animal, but is in particular a mammal, and more in particular a human being. As will be clear to the skilled person, the subject to be treated will in particular be a person suffering from, or at risk of, the diseases and disorders mentioned herein.

The invention also relates to the use of an amino acid sequence, Nanobody or polypeptide of the invention in the preparation of a pharmaceutical composition for the prevention and/or treatment of at least one disease or disorder that can be prevented and/or treated by administering an amino acid sequence, Nanobody or polypeptide of the invention to a patient.

More in particular, the invention relates to the use of an amino acid sequence, Nanobody or polypeptide of the invention in the preparation of a pharmaceutical composition for the prevention and/or treatment of diseases and disorders associated with heterodimeric cytokines and their receptors, and in particular for the prevention and treatment of one or more of the diseases and disorders listed herein.

Again, in such a pharmaceutical composition, the one or more amino acid sequences, Nanobodies or polypeptides of the invention may also be suitably combined with one or more other active principles, such as those mentioned herein.

Finally, although the use of the Nanobodies of the invention (as defined herein) and of the polypeptides of the invention is much preferred, it will be clear that on the basis of the description herein, the skilled person will also be able to design and/or generate, in an analogous manner, other amino acid sequences and in particular (single) domain antibodies against heterodimeric cytokines and/or their receptors, as well as polypeptides comprising such (single) domain antibodies.

For example, it will also be clear to the skilled person that it may be possible to "graft" one or more of the CDR's mentioned above for the Nanobodies of the invention onto such (single) domain antibodies or other protein scaffolds, including but not limited to human scaffolds or non-immunoglobulin scaffolds. Suitable scaffolds and techniques for such CDR grafting will be clear to the skilled person and are well known in the art, see for example US-A-7,180,370, WO 01/27160, EP 0 605 522, EP 0 460 167, US-A-7,054,297, Nicaise et al., Protein Science (2004), 13:1882-1891; Ewert et al., Methods, 2004 Oct; 34(2):184-199; Kettleborough et al., Protein Eng. 1991 Oct; 4(7): 773-783; O'Brien and Jones, Methods Mol. Biol. 2003: 207: 81-100; Skerra, J. Mol. Recognit. 2000: 13: 167-187, and Saerens et al., J. Mol. Biol. 2005 Sep 23;352(3):597-607, and the further references cited therein. For example, techniques known per se for grafting mouse or rat CDR's onto human frameworks and scaffolds can be used in an analogous manner to provide chimeric proteins comprising one or more of the CDR's of the Nanobodies of the invention and one or more human framework regions or sequences.

It should also be noted that, when the Nanobodies of the inventions contain one or more other CDR sequences than the preferred CDR sequences mentioned above, these CDR sequences can be obtained in any manner known per se, for example from Nanobodies (preferred), V_{H} domains from conventional antibodies (and in particular from human antibodies), heavy chain antibodies, conventional 4-chain antibodies (such as conventional human 4-chain antibodies) or other immunoglobulin sequences directed against heterodimeric cytokines and/or their receptors. Such immunoglobulin sequences directed against heterodimeric cytokines and/or their receptors can be generated in any manner known per se, as will be clear to the skilled person, i.e. by immunization with heterodimeric cytokines and/or their receptors or by screening a suitable library of immunoglobulin sequences with heterodimeric cytokines and/or their receptors, or any suitable combination thereof. Optionally, this may be followed by techniques such as random or site-directed mutagenesis and/or other techniques for affinity maturation known per se. Suitable techniques for generating such immunoglobulin sequences will be clear to the skilled person, and for example include the screening techniques reviewed by Hoogenboom, Nature Biotechnology, 23, 9, 1105-1116 (2005) Other techniques for generating immunoglobulins against a specified target include for example the Nanoclone technology (as for example described in the published US patent application 2006-0211088), so-called SLAM technology (as for example described in the European patent application 0 542 810), the use of transgenic mice expressing human immunoglobulins or the well-known hybridoma techniques (see for example Larrick et al, Biotechnology, Vol.7, 1989, p. 934). All these techniques can be used to generate immunoglobulins against heterodimeric cytokines and/or their receptors, and the CDR's of such immunoglobulins can be used in the Nanobodies of the invention, i.e. as outlined above. For example, the sequence of such a CDR can be determined, synthesized and/or isolated, and inserted into the sequence of Nanobody of the invention (e.g. so as to replace the corresponding native CDR), all using techniques known per se such as those described herein, or Nanobodies of the invention containing such CDR's (or nucleic acids encoding the same) can be synthesized de novo, again using the techniques mentioned herein.

Further uses of the amino acid sequences, Nanobodies, polypeptides, nucleic acids, genetic constructs and hosts and host cells of the invention will be clear to the skilled person based on the disclosure herein. For example, and without limitation, the amino acid sequences of the invention can be linked to a suitable carrier or solid support so as to provide a medium than can be used in a manner known per se to purify heterodimeric cytokines and/or their receptors from compositions and preparations comprising the same. Derivatives of the amino acid sequences of the invention that comprise a suitable detectable label can also be used as markers to determine (qualitatively or quantitatively) the presence of heterodimeric cytokines and/or their receptors in a composition or preparation or as a marker to selectively detect the presence of heterodimeric cytokines and/or their receptors on the surface of a cell or tissue (for example, in combination with suitable cell sorting techniques).

The invention will now be further described by means of the following non-limiting Examples and Figures, in which the Figures show:

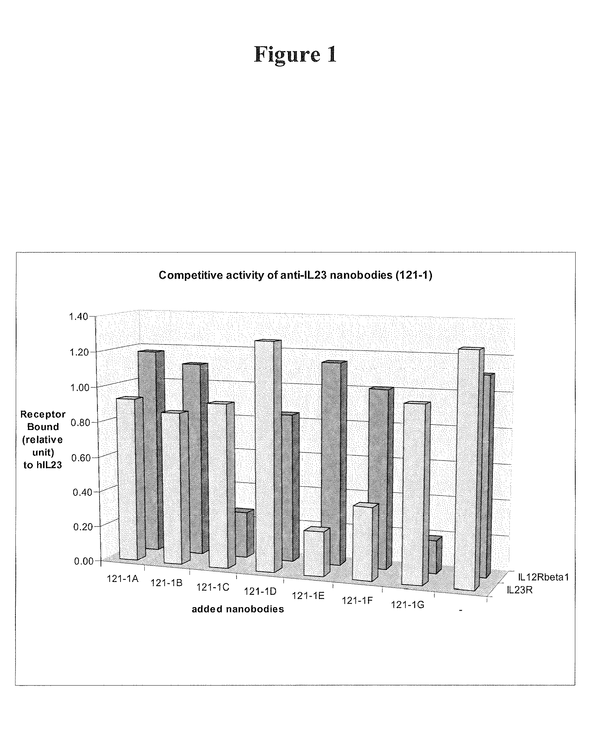

- Figure 1 : Representative result of a competitive binding ELISA showing the IL23 neutralizing activity of nanobodies (in periplasm fraction) for IL12Rbeta1 chain or IL23R chain binding as described in Example 5. The average bound receptor is set to 1. Nanobodies selected as in Example 3b.
- Figure 2: Results of the cell-based proliferation assay described in Example 6.

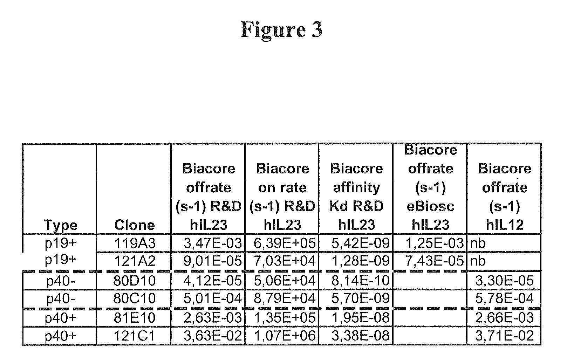

- Figure 3: Full Biacore analysis for p19+ Nbs 121A2, 119A3; for the p40- Nbs 80D10, 80C10, for the p40+ Nbs, 81E10 and 121C1

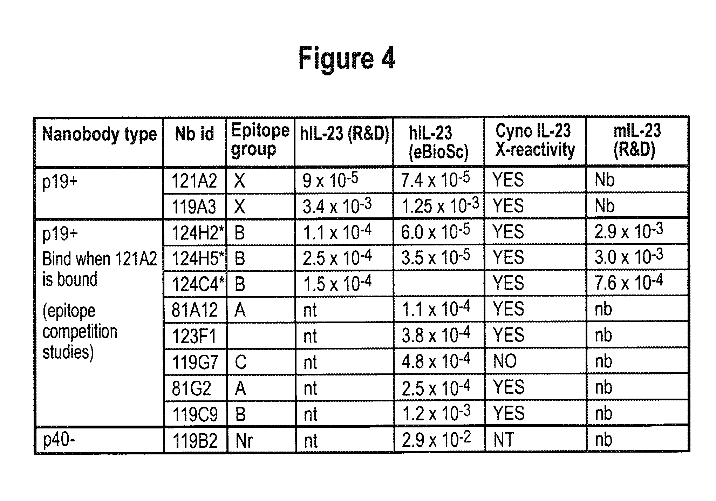

- Figure 4: Biacore off-rates for selected monovalent Nbs plus cross-reactivity and epitope "grouping" data. Biacore off-rates on hIL-23 from R&D systems, or from eBiosciences were determined for a panel of p19+ and p19- Naziobadies as well as one p40- Nb and data are indicated in table 2.

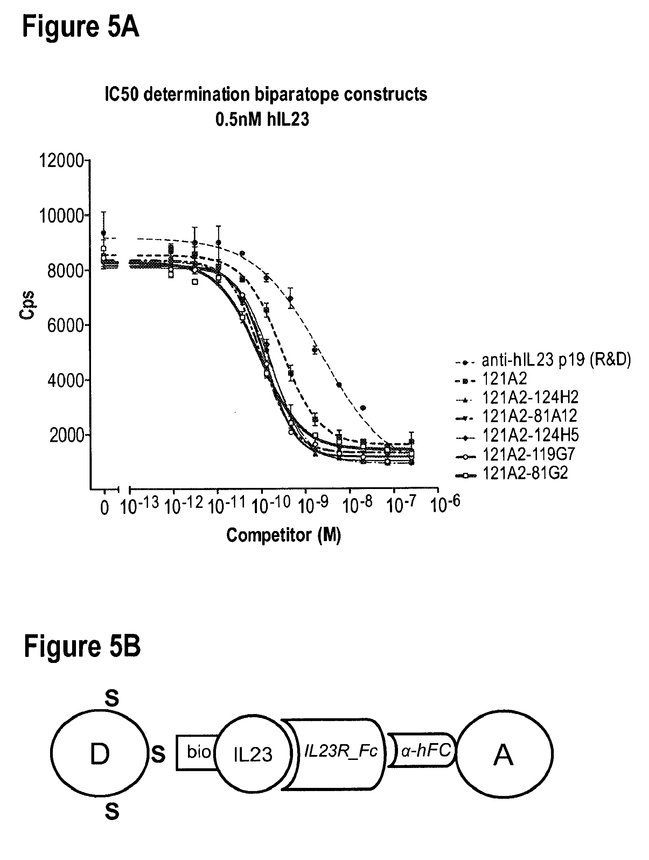

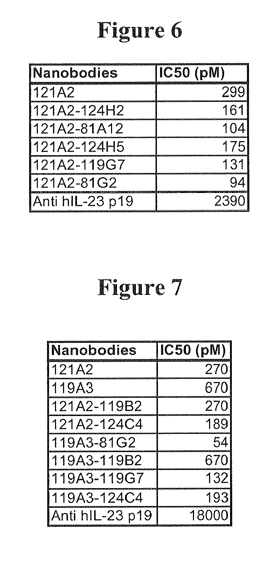

- Figure 5: Results of an alpha-screen measuring hIL-23 binding to ML23R-Fc (set up shown in Figure 5B) for determining the potency of biparatopic Nanobodies compared to the monovalent p19+ nanobodies. Figure 5A is a graphic representation of the results. See also Figures 6 and 7.
- Figure 6: potencies of monovalent and biparatopic Nanobodies in the hIL-23-hIL-23R alpha-screen.
- Figure 7: potencies of monovalent and biparatopic Nanobodies in the hIL-23-hIL-23R alpha-screen

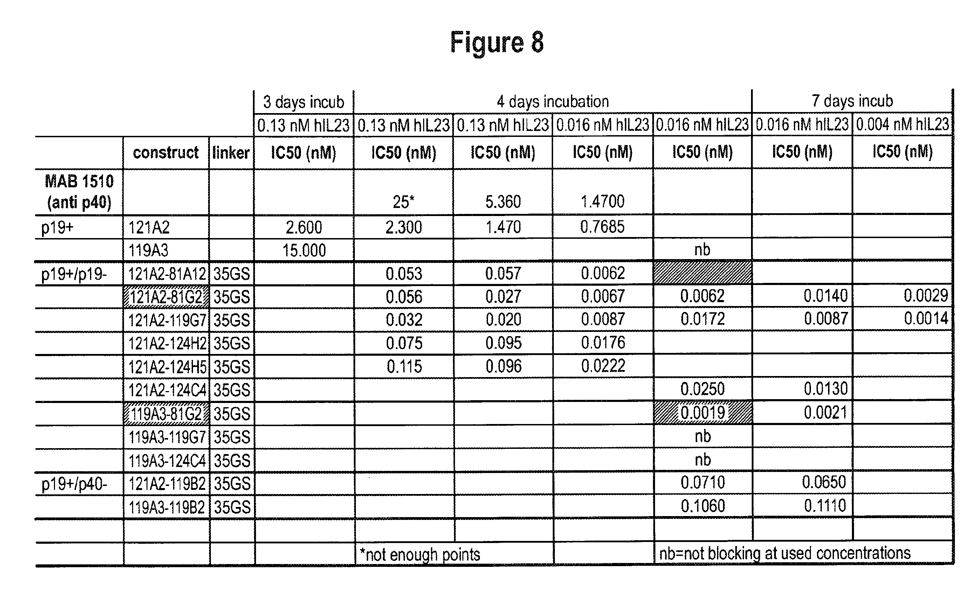

- Figure 8: Splenocyte bioassay results (measuring mIL-17 levels) for a selection of monovalent and biparatopic Nanobodies (p19+-35GS-p19-; and p19+-35GS-p40-)

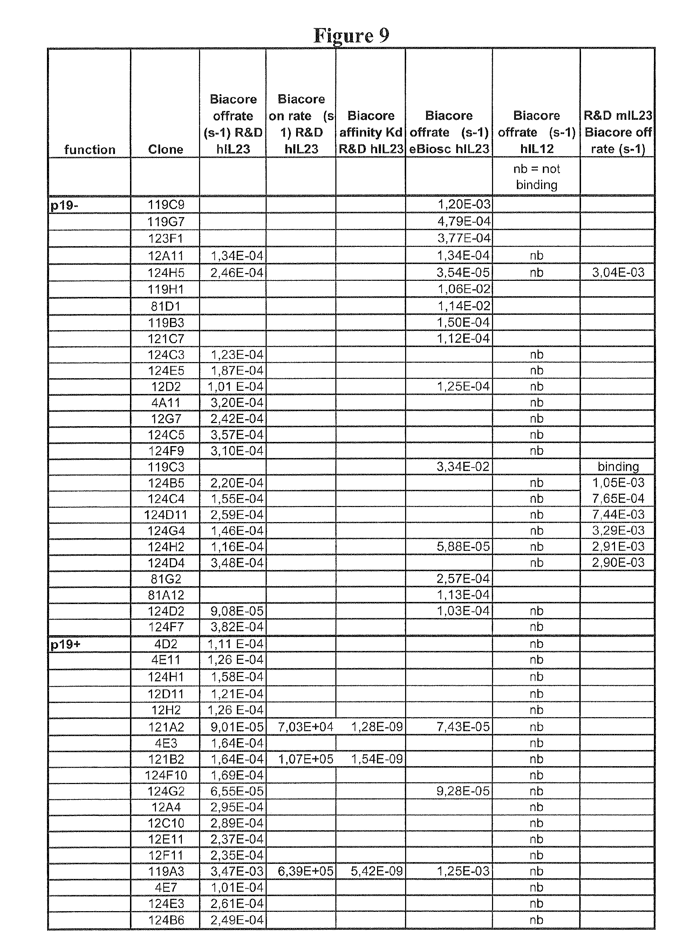

- Figure 9: Biacore data with selected p19- (non-neutralizing) and p19+ (neutralizing) Nanobodies

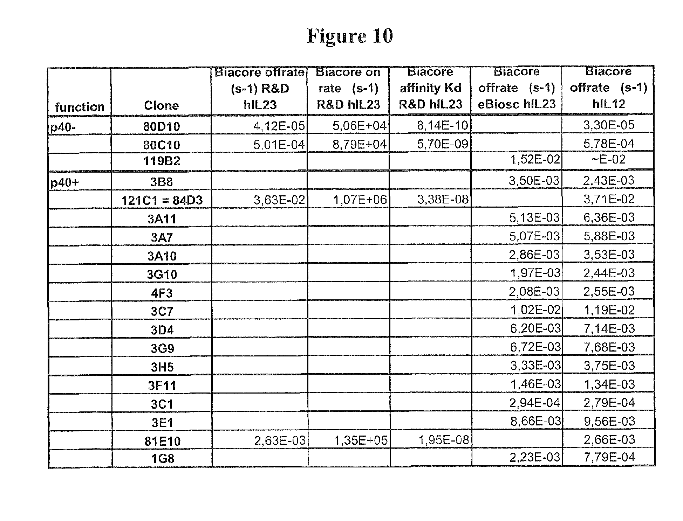

- Figure 10: Biacore data with selected p40- (non-neutralizing) and p40+ (neutralizing) Nanobodies
- Figure II: table showing the framework regions and CDR's of some preferred, but non-limiting "p19+ sequences" (in casu, p19+ Nanobodies)
- Figure 12: table showing the Framework regions and CDR's of some preferred, but non-limiting "p19- sequences" (in casu, p19- Nanobodies)
- Figure 13: table showing the framework regions and CDR's of some preferred, but non-limiting "p40- sequences" (in casu, p40- Nanobodies) (*)
- Figure 14: table showing the framework regions and CDR's of some preferred, but non-limiting "p40+ sequences" (in casu, p40+ Nanobodies) (*)
- Figure 15: table showing the framework regions and CDR's of some preferred, but non-limiting "p35 sequences" (in casu, anti p35 Nanobodies)
- Figure 16: table showing the framework regions and CDR's of some preferred, but non-limiting "IL-27 sequences" (in casu, anti IL-27 Nanobodies)
- Figure 17: table showing the framework regions and CDR's of some preferred, but non-limiting "IL-12Rb1 sequences" (in casu, anti IL-12Rb1 Nanobodies)
- Figure 18: table showing the framework regions and CDR's of some preferred, but non-limiting "IL-12Rb2 sequences" (in casu, anti IL-12Rb2 Nanobodies)
- Figure 19: table showing the framework regions and CDR's of some preferred, but non-limiting "IL-23R sequences" (in casu, anti IL-23R Nanobodies)
- Figure 20: table giving the amino acid sequences of of some preferred, but non-limiting "p19+ sequences" (in casu, p19+Nanobodies)
- Figure 21: table giving the amino acid sequences of of some preferred, but non-limiting "p19- sequences" (in casu, p19- Nanobodies)
- Figure 22: table giving the amino acid sequences of of some preferred, but non-limiting "p40- sequences" (in casu, p40- Nanobodies).(*)
- Figure 23: table giving the amino acid sequences of of some preferred, but non-limiting "p40+ sequences" (in casu, p40+ Nanobodies)(*)
- Figure 24: table giving the amino acid sequences of of some preferred, but non-limiting "p35 sequences" (in casu, anti p35 Nanobodies)

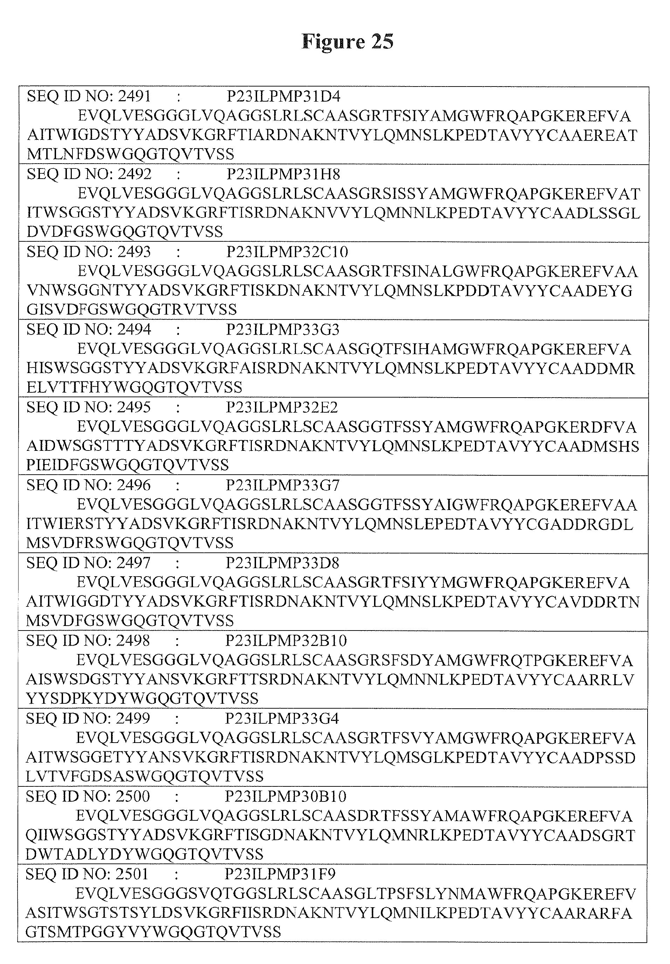

- Figure 25: table giving the amino acid sequences of of some "p19+ sequences" (in casu, p19+ Nanobodies) against mouse p 19
- Figure 26: table giving the amino acid sequences of of some preferred, but non-limiting "IL-27 sequences" (in casu, anti IL-27 Nanobodies)
- Figure 27: table giving the amino acid sequences of of some preferred, but non-limiting "II-12Rb1 sequences" (in casu, anti IL-12Rb1 Nanobodies)
- Figure 28: table giving the amino acid sequences of of some preferred, but non-limiting "IL-12Rb2 sequences" (in casu, anti IL-12Rb2 Nanobodies)
- Figure 29: table giving the amino acid sequences of of some preferred, but non-limiting "IL-23R sequences" (in casu, anti IL-23R Nanobodies)
- Figure 30: table giving the amino acid sequences of some preferred, but non-limiting examples of multivalent, multispecific and/or biparatopic, contructs comprising at least one anti p19 sequence (i.e. a p19+ or p19- sequence, or both)
- Figure 31: table giving the amino acid sequences of some preferred, but non-limiting examples of humanized and/or mutated anti p19 sequences
- Figure 32: table giving the amino acid sequences of some preferred, but non-limiting examples of multivalent, multispecific and biparatopic contructs comprising at least one humanized anti p19 sequences (i.e. a p19+ or p19- sequence, or both)
- Figure 33: table giving the amino acid sequences of some preferred, but non-limiting examples of multispecific "p19-p40" contructs (i.e. comprising at least one anti p19 sequence and at least one anti p40 sequence)
- Figure 34: table giving the amino acid sequences of some preferred, but non-limiting multivalent, multispecific and/or biparatopic p40 constructs
- Figure 35: table giving the amino acid sequences of some preferred, but non-limiting multivalent, multi specific and/or biparatopic p35 constructs
- Figure 36: table giving the amino acid sequences of some preferred, but non-limiting muitispecific "p35-p40" constructs (i.e. comprising at least one anti p35 sequence and at least one anti p40 sequence)

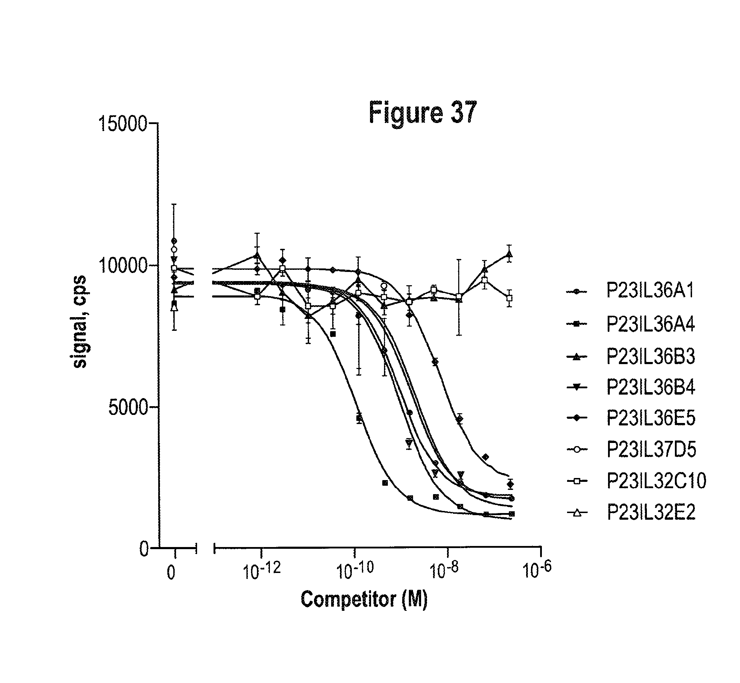

- Figure 37: graph showing the results obtained in Example 22 for the measurement of the potency of some anti p19 and anti p40 sequences in alpha-screen using hIL-23

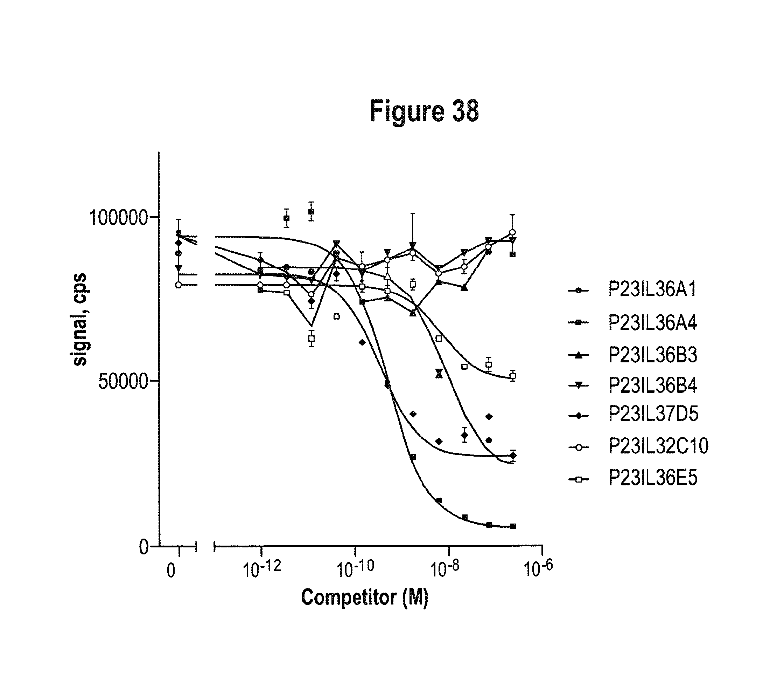

- Figure 38: graph showing the results obtained in Example 22 for the measurement of the potency of some anti p19 and anti p40 sequences in Alphascreen using cyno IL-23

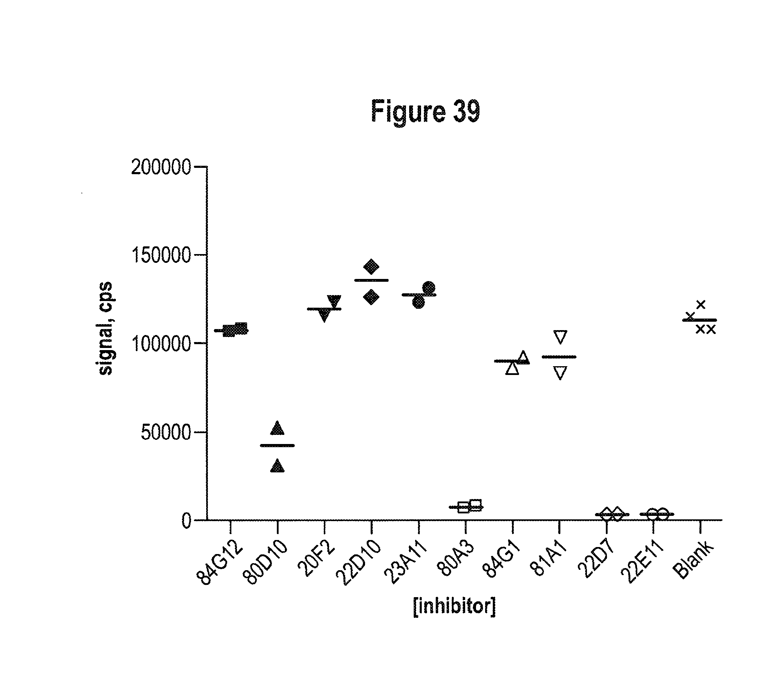

- Figure 39: graph showing the results of epitope mapping as obtained in Example 24

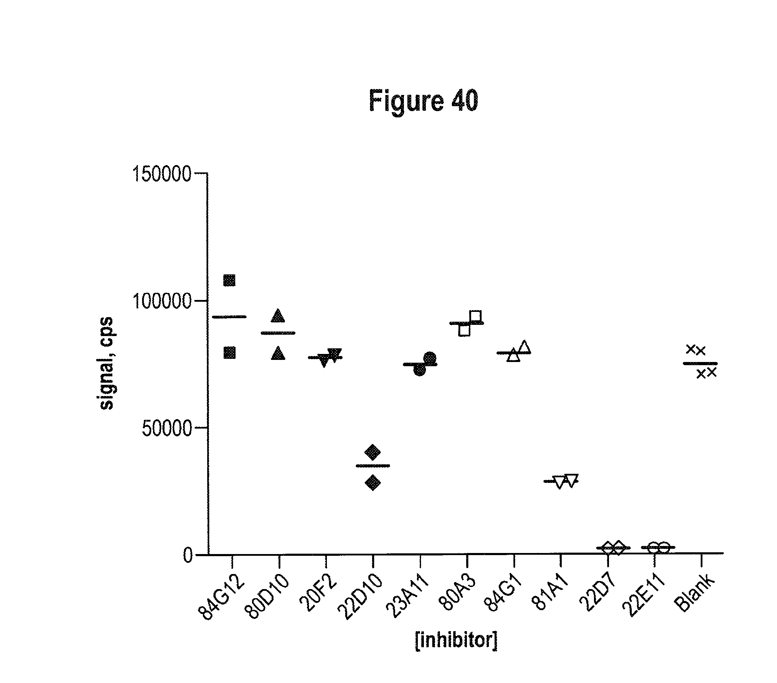

- Figure 40: graph showing the results of epitope mapping as obtained in Example 24

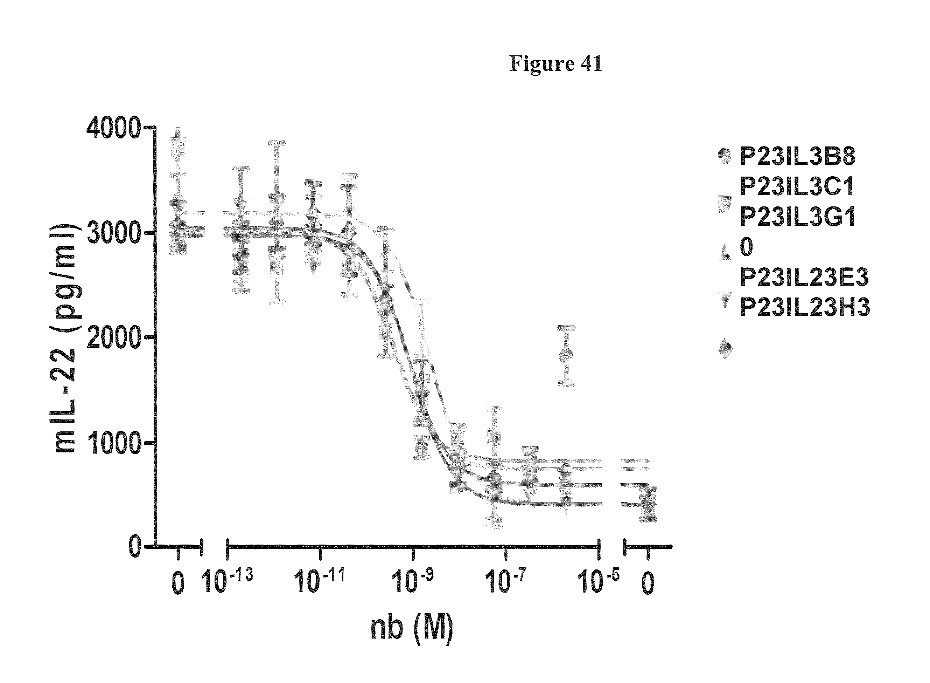

- Figure 41: graph showing the results obtained in Example 25 for the measurement of the mIL-22 synthesis in a mouse splenocyte assay after stimulation with hIL-23, which can be blocked by monovalent anti p40 nanobodies

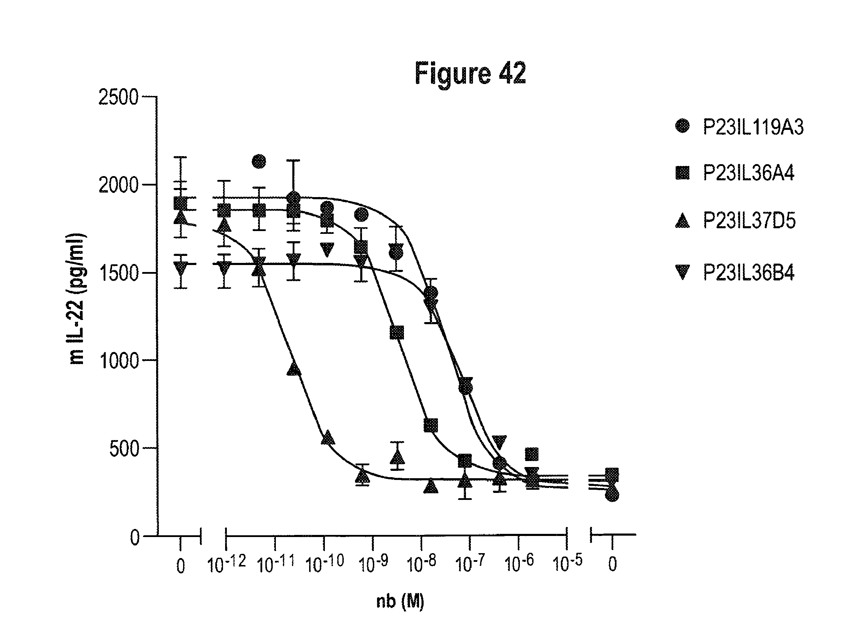

- Figure 42: graph showing the results obtained in Example 25 for the measurement of the mIL-22 synthesis in a mouse splenocyte assay after stimulation cynomolgus IL-23, which can be blocked by monovalent anti p19 nanobodies.

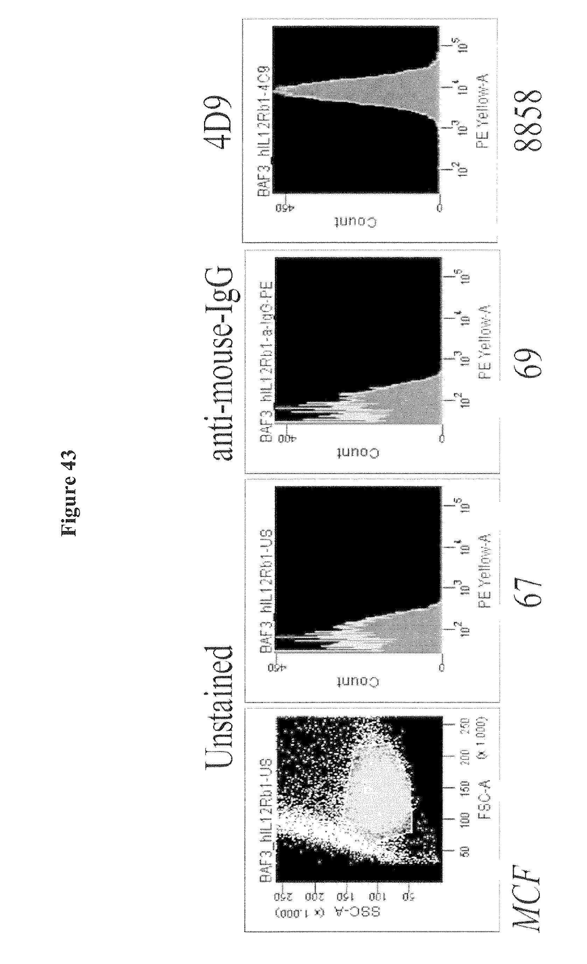

- Figure 43 shows the results of flow cytometric analyis of h-IL12Rβ1 expressing Ba/F3 subclone 4D9, as obtained in Example 26. Detection was performed by a mouse-anti-hIL-12Rβ1 antibody followed by a PE-labeled polyclonal anti-mouse Ig antibody. The presence of h-IL12β1 on the cells was measured by an increase in fluorescence intensity as compared to cells that were incubated with FACS buffer (PBS + 10% FBS) followed by PE-labeled polyclonal anti-mouse Ig antibody. Fluorescence intensity is plotted on the X-axis, the number of events on the Y-axis. The FACS profile of unstained cells is also shown.

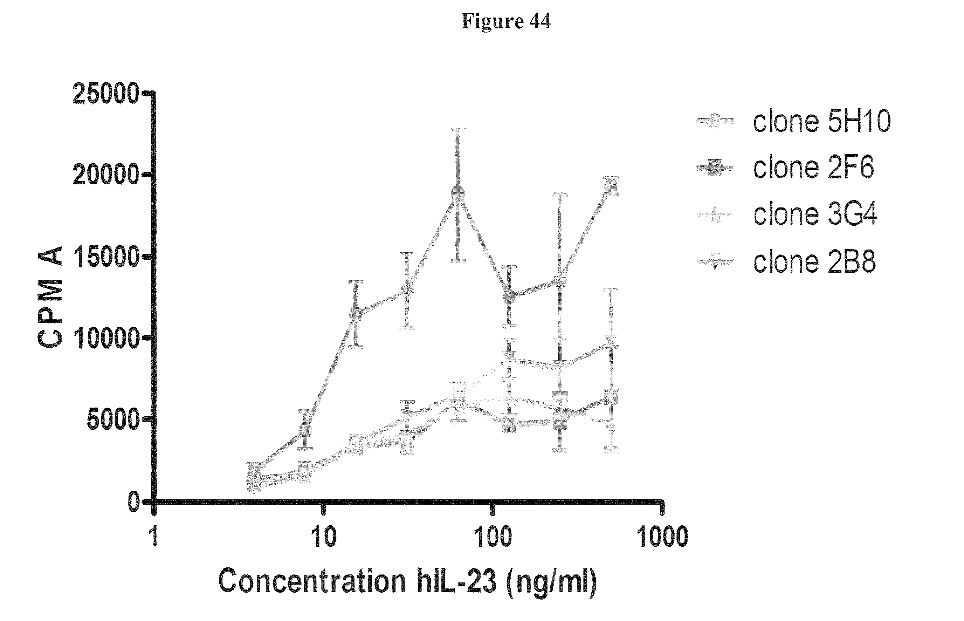

- Figure 44: graph showing the results obtained in Example 26 for hIL-23 responsiveness of several Ba/F3-hIL12Rβ1-hIL23R single cell clones, in a cell proliferation assay after a 72 hour incubation with several concentrations hIL-23, as a readout using [methyl-3H]-thymidine incorporation [ON pulse]

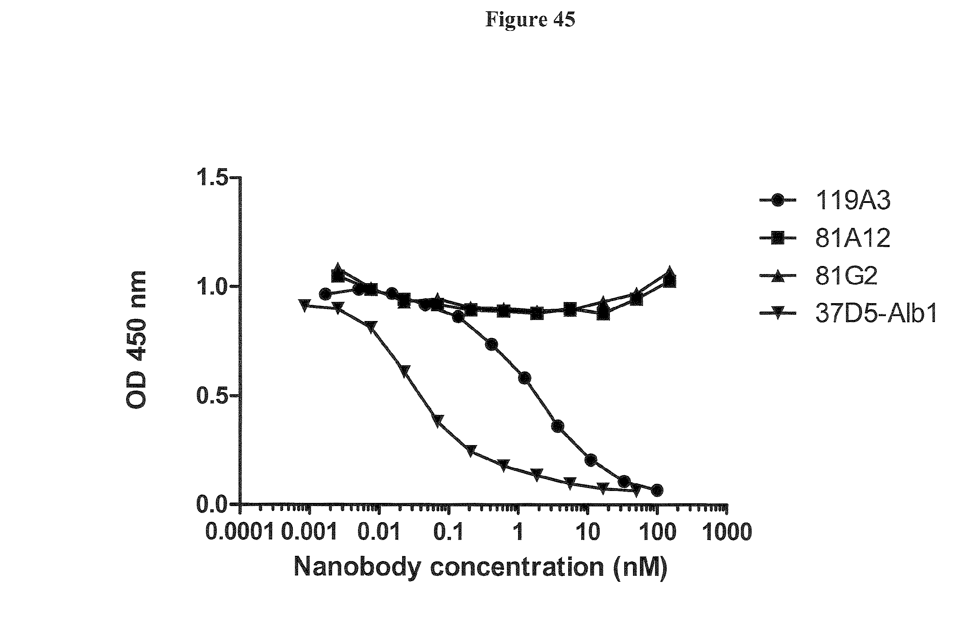

- Figure 45: graph showing the results obtained in Example 34 for the inhibition of the hIL-23 interaction with hIL-23R by monovalent (p19+: 119A3, 37D5-ALB1; p19-: 81A12, 81G2) Nanobodies.

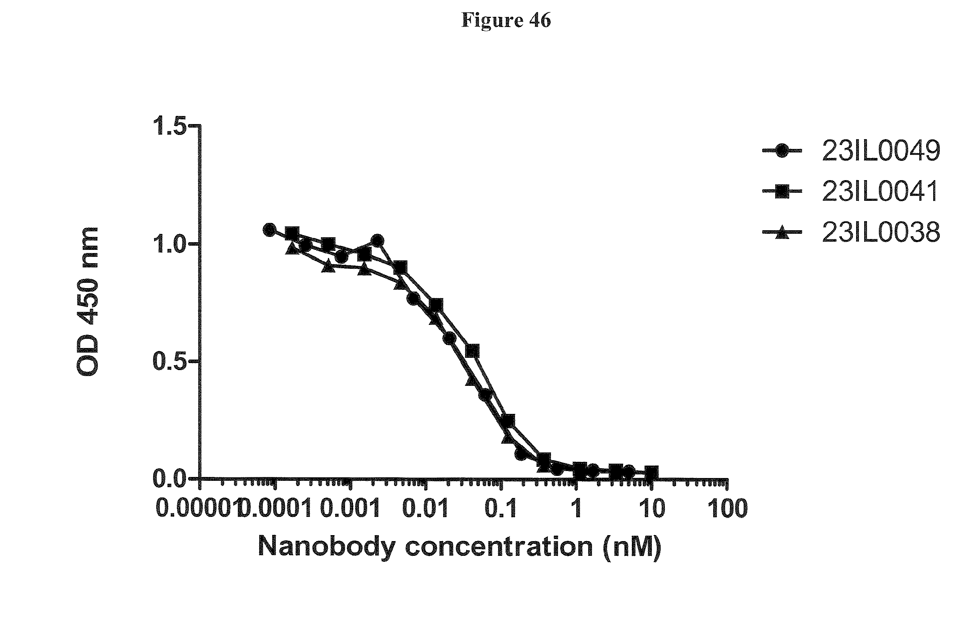

- Figure 46: graph showing the results obtained in Example 34 for the inhibition of the IL-23 interaction with IL-23R by biparatopic (230049, 23IL0041, 23IL0038) Nanobodies

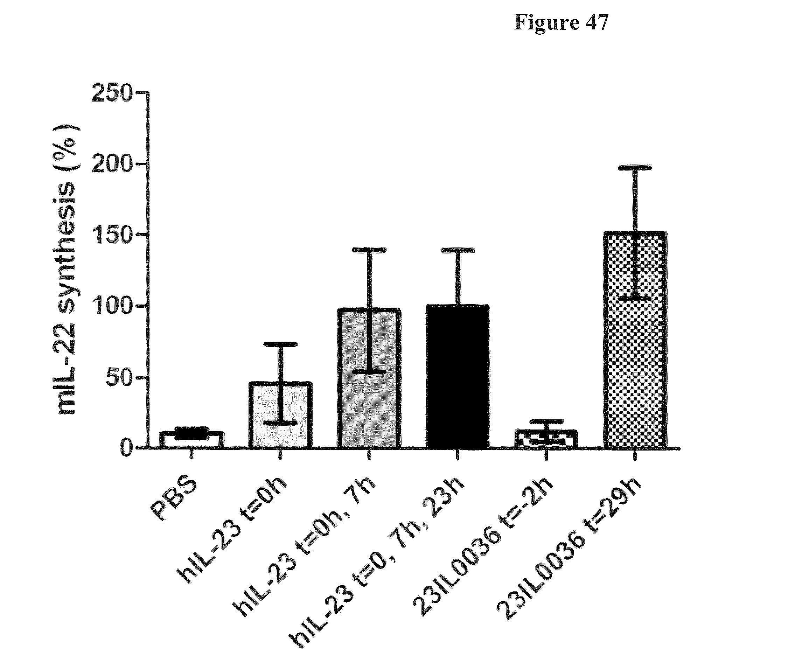

- Figure 47: graph showing the results obtained in Example 35 for determination of the optimal frequency of injections of hIL-23 to obtain optimal mIL-22 synthesis. mIL22 was measured in a mouse splenocyte assay upon administration of 1 x 3 microgram, 2 x 3 microgram or x 3 microgram hIL-23 (first part of the graph). In the second part of the graph the inhibition of mIL22 production is monitored when P23IL0036 is injected 2hours before or 29 hours after the first hIL-23 injections. Average mIL-22 synthesis is expressed as % change compared to group 4.

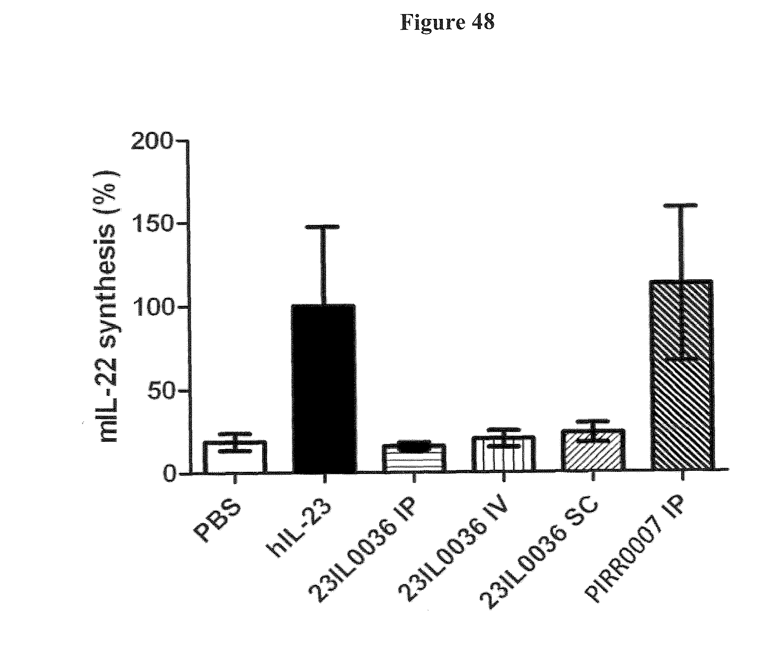

- Figure 48: graph showing the results obtained in Example 35 for the inhibition of the mIL-22 synthesis in a mouse splenocyte assay upon administration of 23IL0036 or IRR007 via different routes of administration. Average mIL-22 synthesis is expressed as % change compared to the group which received hIL-23 only.

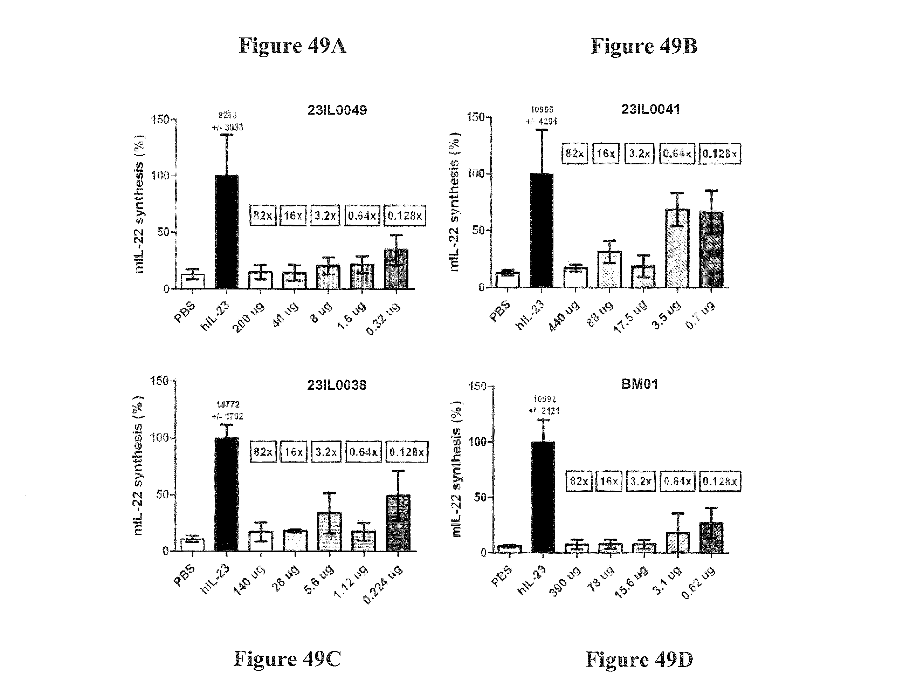

- Figure 49 A to D: graphs showing the results obtained in Example 36 for the inhibition of the mIL-22 synthesis in a mouse splenocyte assay upon administration of Nanobodies (Fig. 49A: 23IL0049; Fig.49B: 23IL0041; Fig.49C: 231L0038) or positive control antibody (Fig. 49D: BM01). Average mIL-22 synthesis is expressed as % change compared to the group which received hIL-23 only.

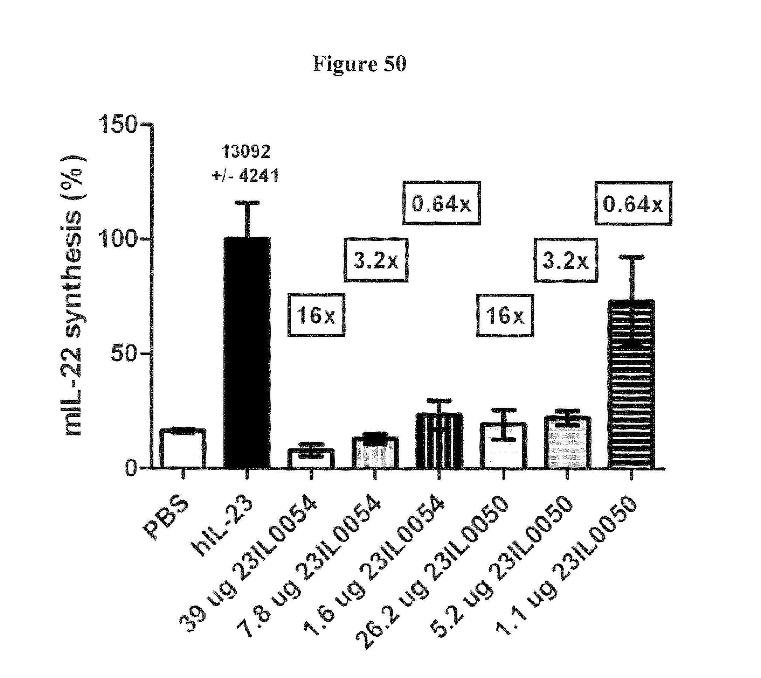

- Figure 50: graph showing the results obtained in Example 37 for the inhibition of the mIL-22 synthesis in a mouse splenocyte assay upon administration of 23IL0054 and 23IL0050. Average mIL-22 synthesis is expressed as % change compared to the group which received hIL-23 only.

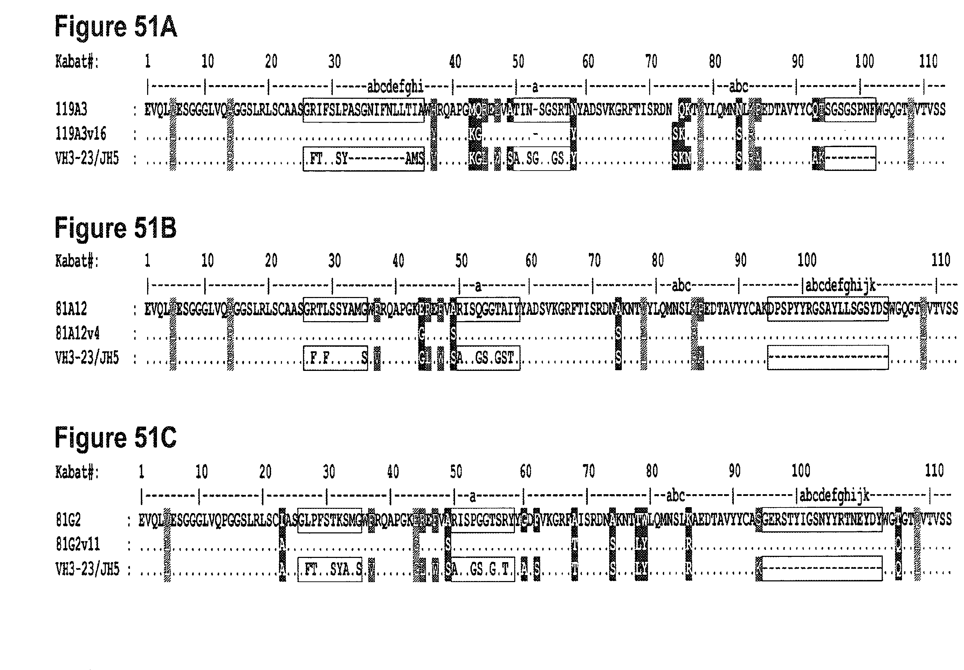

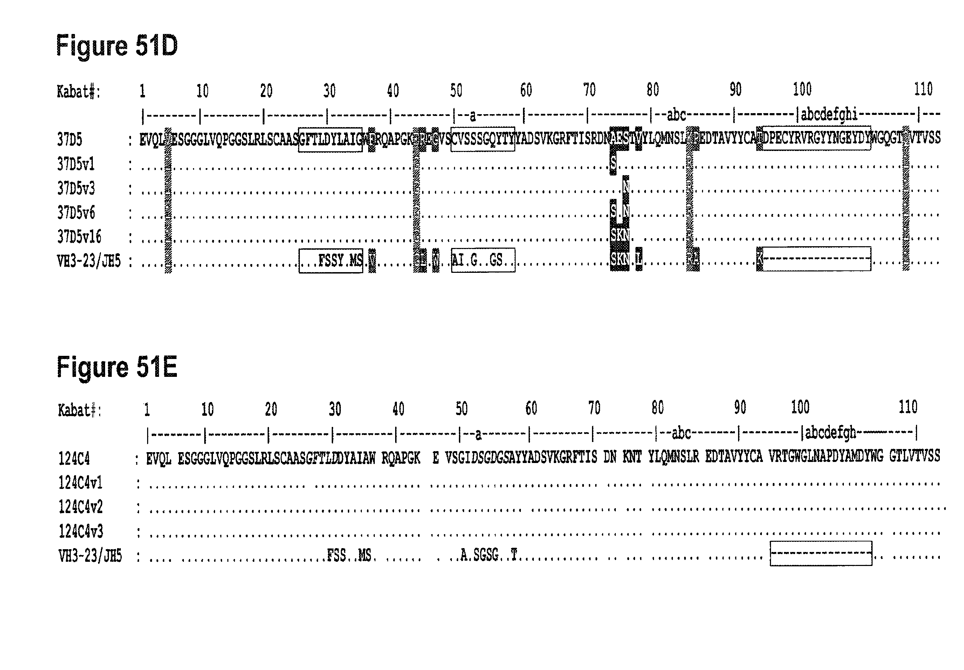

- Figure 51A to E: sequence alignment of humanized amino acid sequences of the invention and the corresponding wild-type sequence and the VH3-23/JH5 germline sequences. Amino acid differences in the framework regions between the wild-type nanobody and the human germline sequence are indicated in black boxes, the CDR regions are highlighter in grey. The black boxes in the humanized sequences indicate the residues that were changed compared to the wild-type sequence. Fig. 51 A: Alignment of P23IL119A3 and the final humanization variant with VH3-23/ JH5 germline sequences. Fig. 51B: Alignment of P23IL81A12 and the final humanization variant with VH3-23/JH5 germline sequences. Fig. 51C: Alignment of P23IL81G2 and the final humanization variant with VH3-23/JH5 germime sequences. Fig. 51D: Alignment of P23IL37D5 and humanization variants with VH3-23/JH5 germline sequences. Fig. 51E: Alignment of P23IL124C4 and humanization variants with VH3-23/JH5 germline sequences.

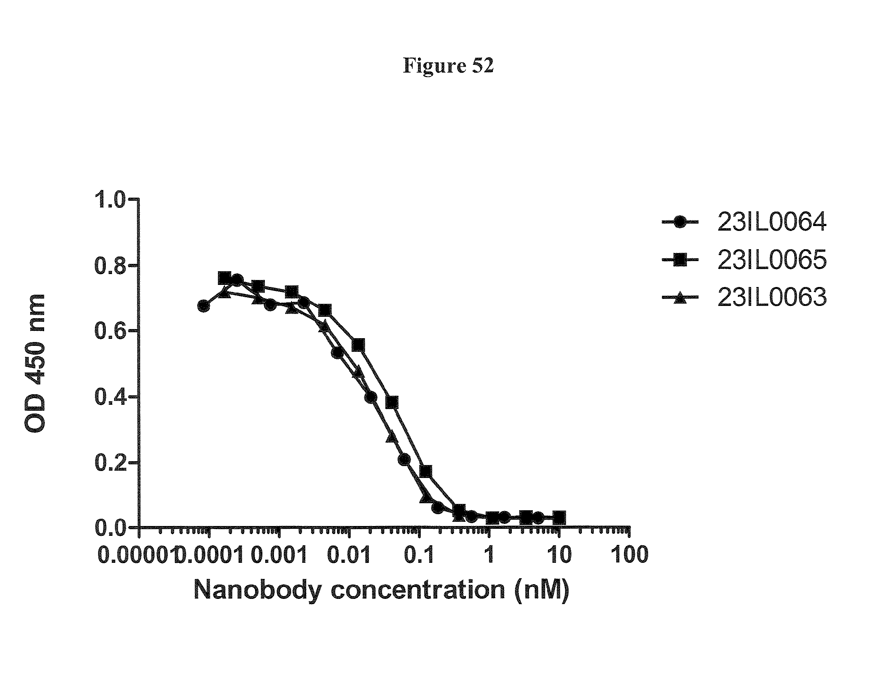

- Figure 52: graph showing the results obtained in Example 40 for the inhibition of the IL-23 interaction with IL-23R by formatted humanized Nanobodies.

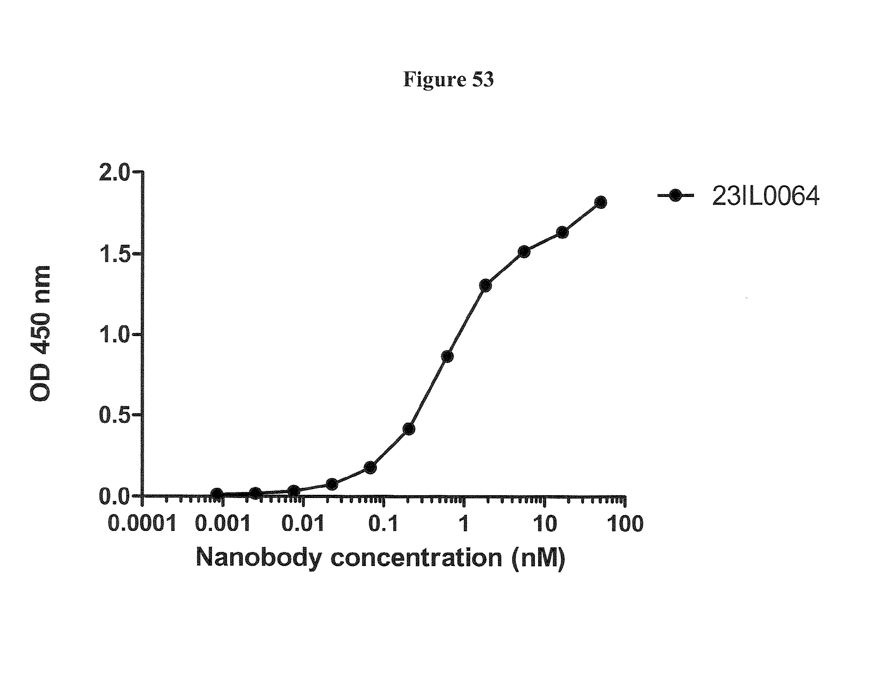

- Figure 53: graph showing the results obtained in Example 40 for the binding of 23IL0064 to human serum albumin.

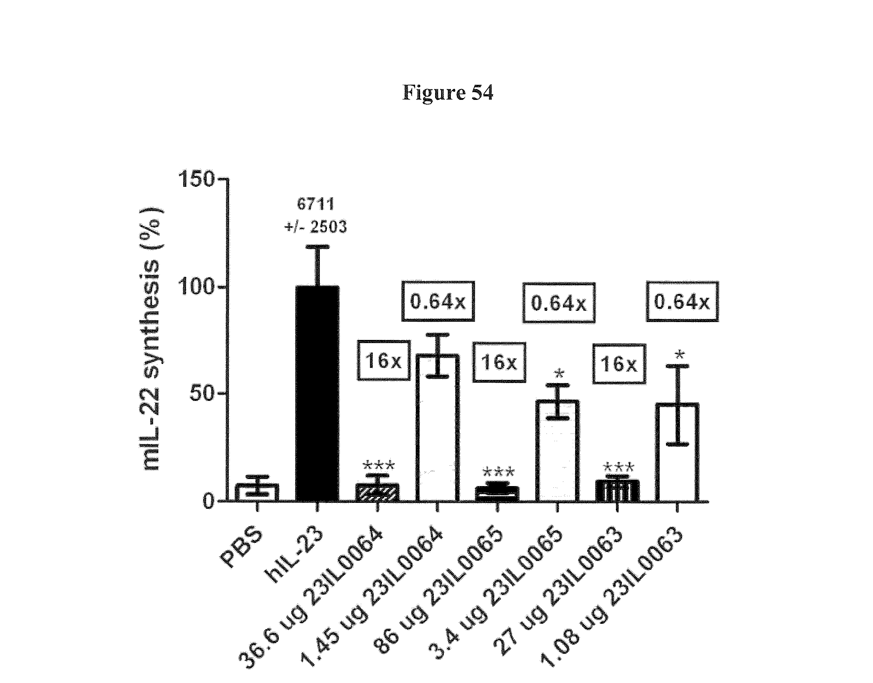

- Figure 54: graph showing the results obtained in Example 41 for the inhibition of mIL-22 synthesis upon administration of Nanobodies or positive control antibody. Average mIL-22 synthesis is expressed as % change compared to the group which received hIL-23 only.

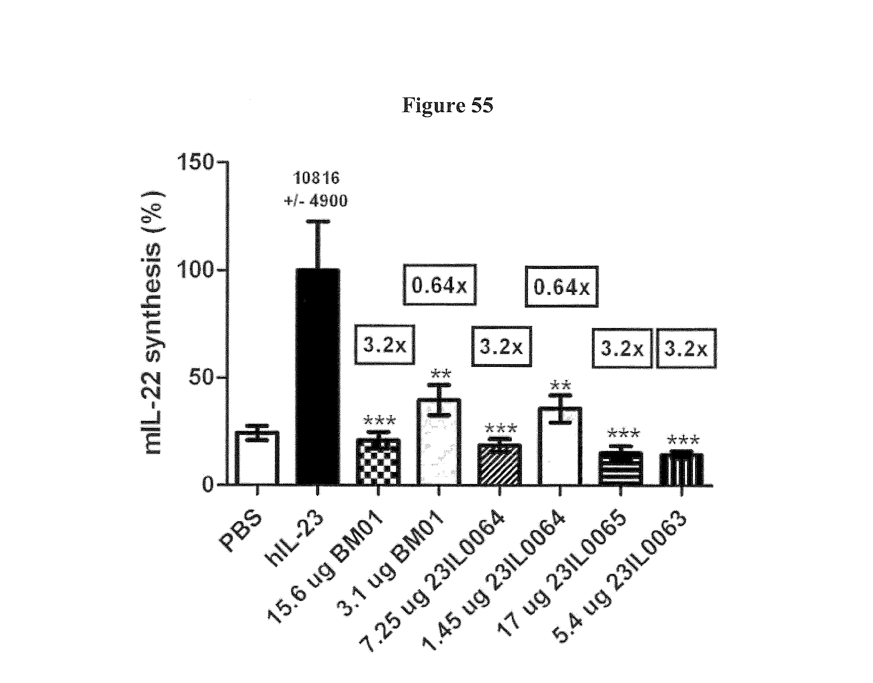

- Figure 55: graph showing the results obtained in Example 41 for the inhibition of mIL-22 synthesis upon administration of Nanobodies or positive control antibody. Average mIL-22 synthesis is expressed as % change compared to the group which received hIL-23 only.

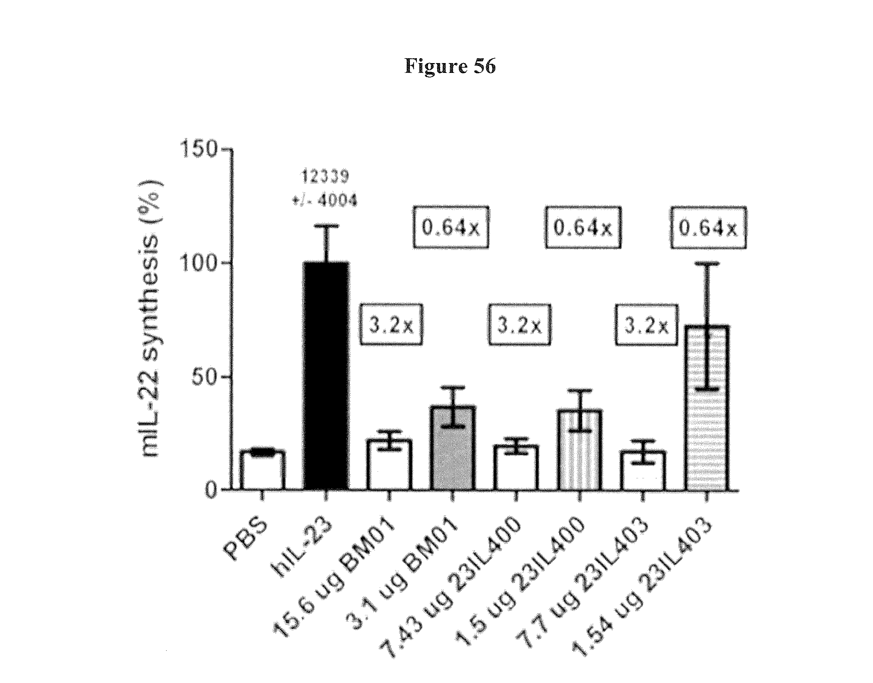

- Figure 56: graph showing the results obtained in Example 47 for the inhibition of the mIL-22 synthesis in a mouse splenocyte assay upon administration of BM01, 23IL400 and 23IL403 at two different dose levels. Average mIL-22 synthesis is expressed as % change compared to the group which received hIL-23 only.
(*) Note: P23ILp40-PMP84G12 is a partial blocker of p40 and is therefore mentioned in both Figure 13 and 14, as well as Figure 22 and 23.

### EXPERIMENTAL PART:

### Example 1: Immunizations

Two llamas were immunized (according to standard protocols with 6 boosts of a cytokine cocktail (2x40ug + 4x20ug, containing equal amount of human IL12, IL23 and IL27). Blood and lymph nodes were collected from these animals after 7 days after boost 6 and 10 days after boost 6. All 3 cytokines were recombinant proteins purchased from R&D systems: hIL-12 (Cat. No 219-IL/CF); hIL-23 (Cat. No 1290-IL/CF) and hIL-27 (Cat. No 2526-IL/CF)

### Example 2: Library construction

Peripheral blood mononuclear cells were prepared from blood samples using Ficoll-Hypaque according to the manufacturer's instructions. Next, total RNA extracted was extracted from these cells as well as from the lymph node bow cells and used as starting material for RT-PCR to amplify Nanobody encoding gene fragments. These fragments were cloned into phagemid vector pAX50. Phage was prepared according to standard methods (see for example the prior art and applications filed by applicant cited herein) and stored at 4°C for further use, resulting in two phage libraries ("A" and "B").

### Example 3a: Selections

To identify nanobodies recognizing the cytokines, the phage libraries from Example 2 were used for selections on the immunized cytokines and biotinylated cytokines. The (biotinylated) proteins were immobilized independently at 5 microg/ml, 0.5 ug/ml or 0ug/ml (control) on Nunc Maxisorp ELISA plates previously coated with Neutravidine (5ug/ml) when biotinylated proteins were used for selection. Bound phages were eluted from the IL12, IL23 or IL27 (and the biotinylated form) using trypsin (1mg/ml) as in standard protocols.

Outputs of both R1 selections were analyzed for enrichment factor (phage present in eluate relative to controls). Based on these parameters the best selections were chosen for further analysis. The polyclonal output was then recloned in PAX51 and individual TG1 colonies were picked and grown in 96 deep well plates (1ml volume) and induced by adding IPTG for Nanobody expression. Periplasmic extracts (volume: ∼ 100 ul) were prepared according to standard methods (see for example the prior art and applications filed by applicant cited herein).

### Example 3b: Selections of hIL12 or hIL23 specific nanobodies.

To identify nanobodies specifically recognizing hIL12 or hIL23 and therefore recognizing IL12p35 or IL23p19 respectively, 2 rounds of selections with relevant counterselection were performed.

For IL23 specific nanobodies, 2.5ug/ml IL23 was coated on Nunc Maxisorp ELISA plates. Phages from libraries 146 and 147 were added to the coated wells in the presence of 10ug IL12 (1.00ug/ml). The presence of IL12 prevents the phages recognizing non specific IL23 epitops (on IL12p40) to binds IL23. To identify IL12 specific nanobodies, the same was done with 2.5ug/ml IL12 coated and 10ug 1L23 (100ug/ml) added with the phages. In all cases, bound phages were eluted (R1) using trypsin (1mg/ml) using standard protocols.

The phages eluted in the first round were used to do a second round using the same condition as in the first round, giving R2.

Output of R2 selections were rescued, cloned in PAX51 using standard polyclonal recloning. Individual colonies containing PAX51-expressing nanobodies were picked and grown in 96 deep well plates (1 ml volume) and induced by adding IPTG for nanobody expression. Periplasmic extracts (volume: ~ 100 ul) were prepared according to standard methods (see for example the prior art and applications filed by applicant cited herein).

### Example 3c: Setections of mouse IL12 or mouse IL23 specific nanobodies.

In order to complete animal studies, it is desirable to have nanobodies recognizing the mouse cytokines isoforms. To identify nanobodies crossreacting with the mouse IL12 or mouse IL23, one approach is to test the nanobodies identified earlier against human IL12/IL23 for cross-reactivity with mouse IL12 or IL23. Several cross reacting nanobodies were identified that way.

A second approach is to select phages directly from the library of Example 2 directed against human IL12 2 and I123. Therefore, the selection was done as in Example 3a except that mouse IL12 (5ug/ml in 100ul; cat. 419-ML/CF, R&Dsystem) or mouse IL23 (5ug/ml in 100ul; cat. 1887-MF/CF, R&Dsystem) was coated. In addition, the same selection was done in the presenece of mouse IL12 (when IL23 was coated) and humanIL23 (when IL12 was coated) as counterselection.

A third approach is to select phages directly from the phage eluted during the R1 as in Example 3b (the approach is then 1 round against humanILs with counterseletion and a second round against mouseILs). Therefore, the second round of selection was done as in Example 3b except mouse IL12 (5ug/ml in 100ul; cat. 419-ML/CF, R&Dsystem) or mouse IL23 (5ug/ml in 100ul; cat. 1887-MF/CF, R&Dsystem) was coated. In parallel, to identify specific mouse IL12 and mouse IL23, the same was done with the presence of mouse IL12 (when IL23 was coated) and human IL23 (when IL12 was coated) to act as counterselective protein.

In all selections, bound phages were eluted using trypsin and resued in TG1. Individual TG1 colonies were picked and grown in 96 deep well plates (1 ml volume) and induced by adding IPTG for Nanobody expression. Periplasmic extracts (volume: ~ 100 ul) were prepared according to standard methods (see for example the prior art and applications filed by applicant cited herein).

### Example 4: Screening for binding

In order to determine binding specificity to the cytokines, the clones were tested in an ELISA binding assay setup.

In short, 1ug/ml of cyokines (IL12, IL23 or IL27) was immobilized directly on polysorp microtiter plates (Nunc). Free binding sites were blocked using 4% Marvel in PBS. Next, 5 ul of periplasmic extract containing nanobody of the different clones in 100ul 2% Marvel PBST were allowed to bind to the immobilized antigen. After incubation and washing, nanobody binding was revealed using a mouse-anti-myc secondary antibody, which was after a wash step detected with a HRP-conjugated goat-anti-mouse antibody. Binding specificity was determined based on OD values compared to controls having received no nanobody (as exemplified in Table B-1). Nanobodies binding to p40 were identified as nanobodies recognizing IL12 and IL23.

**Table B-1: Percentage clones positive in ELISA (example for result of selection as described in Example 3a). Total of 22 or 24 clones per target and per library.**

| **Target** | **IL12** | | **IL23** | | **IL27** | | **Biot-IL12** | **Biot-IL23** |
|---|---|---|---|---|---|---|---|---|
| LIB | A | B | A | B | A | B | B | B147 |
| % | **63** | **77** | **79** | **95** | **96** | **82** | **71** | **100** |

### Example 5: Screening for neutralizing activity

In order to determine neutralizing efficiency of the nanobodies, the clones were tested in a receptor binding assay (Competitive ELISA). The cytokine were coated in 96 wells (Maxisorp, Nunc). After washing and blocking as usual, the coated cytokine was incubated with 15ul periplasmic fraction prepared from above. Finally, specific receptor chain fused to human Fc (recombinant protein purchased from R&D system) was added.

After washing, the presence of bound receptor was detected using anti-humanIgG-HRP antibody. In the case where a nanobody present in the periplasm neutralizes the cytokine (ie, compete for receptor binding), no bound receptor was detected. In all cases, the concentration of protein tested was used to get sub-optimal response (Table B-2 shows an example and Figure 1 shows a representative result).. For IL12, IL12Rbeta1 or I112Rbeta2 was used. For IL23, IL12Rbetal or IL23R was used.

For IL27, IL27R was used.

**Table B-2: Percentage of positive clones in ELISA and percent of clones competiting for IL23R binding in a competitive ELISA (example of result from selection as described in Example 3b). Total of 46-47 clones per library.**

| Target | **IL23** (1.5ug/ml) | |
|---|---|---|
| LIB | A | B |
| % binding (ELISA) | **44** | **94** |
| % competing IL23R binding (competitive ELISA) | **25** | **40** |
| % competing IL12Rbeta1 binding (competitive ELISA) | **2** | **11** |

### Example 6: Neutralizing activity in cell based assays.

Based on the results from Example 4 and/or based on their sequence (families of Nanobodies present only in IL12 selections and not found in IL23 selection, for example, suggested that this families were specific for IL12), some nanobodies were selected to test their neutralizing activity on cells.

Those selected nanobodies (encoded in a PAX51 plasmid) were produced in a bacterial culture (50ml) using standard IPTG induction and purified using TALON beads as recommended by the manufacturer (eluted from the beads using imidazole and dialysed against sterile PBS).

To test the nanobodies against IL12, PBMC activated for 3 days with PHA + 1 day with TL-2 were used. Then we added IL-12 (in the presence of a range of anti-IL-12 nanobodies). After 2 days, the proliferation was measured with 3H-thymidine incorporation.

Using this setup, the maximum of proliferation was obtained with 60pg/well (=300pg/ml) of rhIL-12. So the purified nanobodies (concentration varied from 1 µg to 10-6 µg/well) were tested against 60pg/well of IL-12. The antibodies LG120C7 and 120F8 were tested against commercially available antibody (anti-p35 and anti-p40, MAB219 and MAB1510 respectively were from R&D system; B-T21 was from Diaclone). The results are shown in Figure 2.

### Example 7: immunization:

2 Llamas were immunized with a cocktail of (2x40ug each +4x20ug each): human IL12Rbeta1-hFc (Cat. No 839-B1/CF); human IL12Rbeta2-hFc (Cat. No 1959-B2-b1/CF) and human IL23R (Cat. No 1400-IR/CF). Immunizations were performed essentially as described in Example 1.

### Example 8: Library construction

Construction of libraries ("C" and "D") was performed essentially as described in Example 2.

### Example 9a: Selections of nanobodies against IL12-family receptors

To identify nanobodies recognizing the receptor of the IL12-family cytokines, phages from two libraries ("C" and "D") were used for selections on the immunized recombinant proteins. The proteins were immobilized independently at 5 microg/ml, 0.5 microg/ml or 0 microg/ml (control) on Nunc Maxisorp ELISA plates.

To get rid of phages binding to the Fc fusion domain fused to the recombinant protein, total human IgG (250microg/ml from sigma) and non-relevant hFc fusion protein (hRAGE-Fc, at 25microg/ml from R&D System) was added to the phages during the time of selection.

Bound phages were eluted from the coated proteins using trypsine (1mg/ml) as in standard protocols.

### Example 9b: Selections of Nanobodies neutralizing, IL12-family receptors

This was performed as described in Example 9a, except that only 0.5ug/ml of receptor was coated and that a competitive elution was performed instead of trypsin elution. For this, after binding of the phages and extensive washing, the ligands of the respective receptor chains were was used to elute the phages. In short, 5ug of IL12 (for IL12Rbetal and IL12Rbeta2) or 5ug of IL23 (for IL12Rbetal or IL23R) was added to the phages still bound to the coated receptors. After 2h shacking, the eluted phages were recovered and rescued in TG1.

Outputs of both R1 selections were analyzed for enrichment factor (phage present in eluate relative to controls). Based on these parameters the best selections were chosen for further analysis. The polyclonal output was then recloned in PAX51 (for trypsin elution only) and individual TG1 colonies were picked and grown in 96 deep well plates (1 ml volume) and induced by adding IPTG for nanobody expression. Periplasmic extracts (volume: ~ 100 ul) were prepared according to standard methods (see for example the prior art and applications filed by applicant cited herein).

### Example 10: Screening for binding

In order to determine binding specificity to the receptor chains, the clones were tested in an ELISA binding assay setup.

2ug/ml of protein (IL12Rb1, IL12Rb2 and IL23R) or total hIgG (15ug/ml) was immobilized directly on maxisorp microtiter plates (Nunc). Free binding sites were blocked using 4% Marvel in PBS. Next, 7.5 ul of periplasmic extract containing nanobody of the different clones in 100ul 2% Marvel PBST were allowed to bind to the immobilized antigen. After incubation and washing, nanobody binding was revealed using a mouse-anti-myc secondary antibody, which was after a wash step detected with an AP-conjugated goat-anti-mouse antibody. Binding specificity was determined based on OD values compared to controls having received no nanobody (as exemplified in Figure 1) and compared to the hIgG coated wells (nanobodies binding to hIgG are binding to the Fc and not to the receptor chain). The results are shown on Table B-3:

**Table B-3: Percentage of clones positive and specific for the receptors in ELISA (example for result of selection as described in 3d). Total of 46 clones per target and per Library tested.**

| Target | **IL12Rbetal** | | **IL12Rbeta2** | | **IL23R** | |
|---|---|---|---|---|---|---|
| LIB | A | B | A | B | A | B |
| % | **83** | **41** | **70** | **15** | **83** | **89** |

### Example 11: Screening for neutralizing activity

In order to determine neutralizing efficiency of the nanobodies, the clones were tested in a receptor binding assay (Competitive ELISA) the same way as previously done for IL12/IL23 and IL27. Shortly, the cytokine was coated in 96 wells (Maxisorp, Nunc). After washing and blocking as usual, the coated cytokine was incubated with 15ul periplasmic fraction prepared from above. Finally, specific receptor chain fused to human Fc (recombinant protein purchased from R&D system) was added. After washing, the presence of bound receptor was detected using anti-human IgG-HRP antibody. In the case where a nanobody present in the periplasm neutralizes the cytokine (ie, compete for receptor binding), no receptor bound was detected. In all cases, the concentration of protein tested was use to get sub-optimal response. (See Table B-4 for data given as an example). For IL12, IL12Rbetal or I112Rbeta2 was used. For IL23, IL12Rbetal or 1L23R was used. For IL27, IL27R was used.

**Table B-4: Number of nanobodies (in periplasm) blocking IL23 binding to IL12Rbetal in a competitive ELISA (example of result from selection as described in 3d and 3e). Total of 46-47 clones per library.**

| Target | **IL12Rbeta1** | | | | **IL23R** | | | |
|---|---|---|---|---|---|---|---|---|
| Elution method | **trypsine** | | **IL23** | | **trypsine** | | **IL23** | |
| LIB | A | B | A | B | A | B | A | B |
| Nub clone | **2** | **1** | **15** | **8** | **3** | **4** | **12** | **8** |

### Example 12: Biacore analysis of IL-23 monovalent p19+, p19-, and IL-23/IL-12 p40+, and p40- nanobodies.

hIL-23 (obtained from R&D systems) or hIL-12 (also obtained from R&D systems) was attached to Biacore chips and the binding characteristics of a selection of p19+, p40+ and p40- Nanobodies were determined. Results of the Biacore analysis for p19+ Nanobodies 121A2, 119A3, for the p40- Nanobodies 80D10, 80C10 and for the p40+ Nanobodies, 81E10 and 121C1 are shown in Figure 3, which gives the results

### Biacore

Cross-reactivity profiles (see Figure 4) of the nonovalent Nanobodies were studied in Biacore experiments (mIL-23) and in ELISAs using cynomolgus (cyno) IL-23 (supernantents from transfected cells) in which the Nanobodies to be tested were attached to plates, the cyno IL-23 was added and binding revealed using anti-p40 mAb (mAB 1510 R&D)

Epitope mapping experiments were performed using Bbicore where monovalent nanobodies were attached to the chip and used to capture ML-23. Subsequently the other monovalent nanobodies were tested for their capacity to bind. Nanobodies were considered to bind a separate epitope group when they could still bind hIL-23 already attached to the Nanobody fixed to the Biacore chip. Nanobodies that failed to bind were considered to belong to the same epitope group as the fixed Nanobody (results are also indicated in Figure 4).

Figure 4 shows Biacore off-rates for selected monovalent Nanobodies plus cross-reactivity and epitope "grouping" data. Biacore off-rates were determined using on ML-23 from R&D systems (or from eBiosciences) for a panel of p19+ and p19- Nanobodies as well as one p40- Nanobody.

### Example 13: Construction, production and purification of bispecific/biparatopic IL23 nanobody constructs.

Biparatopic constructs were made in the pAX55 vector, with a p19+ nanobody (121A2 or 119A3) in the N-terminal position and a p19- nanobody (81G2, 81A12, 123F1, 119G7, 124C4 or 124H5) or a p40- nanobody (119B2) in the C-terminal position fused by a 35GS linker sequence. Additionally contructs were made with a p19- nanobody (81G2, 81A12, 123F1, 119G7, 124C4 or 124H5) in the N-terminal position and the p19+ nanobody 121A2 in the C-terminal position fused by a 35 GS linker. Similarly, the monovalent nanobodies were also cloned into pAX55 for use as controls.

The constructs were transformed in TG1 cells which were induced with 1 mM IPTG. Nanobodies were purified from the periplasmic extract using histidine trap affinity chromatography followed by desalting. LPS removal was performed by anion exhange chromatography at neutral pH, or by gel filtration in the presence of 50 mM octyl-glycosyl pyranoside, when additional purification was required

### Example 14: Alpha-screen blockade of IL-23-IL23R binding

An alpha-screen was performed in which the blocking of hIL-23 binding to hIL23R-Fc (see Figure 5b) was monitored to determine the potency of biparatopic nanobodies compared to the monovalent p19+ nanobodies. Results are indicated in Figures 5A, 6 and 7.

Figure 5 shows the results of an Alpha-screen measuring hIL-23 binding to hIL23R-Fc (see Figure 5B) for determining the potency of biparatopic nanobodies compared to the monovalent p19+ nanobodies. Figure 5A is a graphic representations of the results. Figures 6 and 7 show potencies of monovalent and biparatopic Nanobodies in the hIL-23-hIL-23R alpha-screen.

### Example 15: Neutralization of IL-23 biological function by IL-23 Nanobodies,

Freshly isolated mouse splenocytes were treated with hIL-23 (eBiosciences) preincubated with titrated hIL-23 nanobodies or control nanobodies or antibodies. After 3-7 days in culture, cell supernatants were removed and assayed by ELISA using IL-17 ELISA duo set (R&D systems). Figure 8 shows results of a Splenocyte bioassay (measuring mIL-17 levels) for a selection of monovalent and biparatopic Nbs (p19+-35GS-p19-; and p19+-35GS-p40-). Reference is made to Aggarwal, Journal of Biological Chemistry, 278, 3, 2003, 1910-1914.

As shown in Figure 8, monovalent p19+ Nbs inhibit hIL-23 mediated IL-17 7 production and most biparatopics show a considerable enhancement of potency.

### Example 16 : Immunizations

Two llamas were immunized according to standard protocols with 6 intramuscular injections at weekly intervals of recombinant human IL-23 (R&D Systems, Minneapolis, MN, US) (two first injections at 40 µg/dose, 4 following injections at 20 µg/dose). Two weeks after the last injection a boost of 20µg was given. The antigen was formulated in Stimune (Cedi-Diagnostics B.V., Lelystad, The Netherlands). At week 3, sera were collected to define antibody titters against human IL-23 by ELISA. 96-well Maxisorp plates (Nunc, Wiesbaden, Germany) were coated with human IL-23. After blocking and adding diluted sera samples, the presence of anti-IL-23 antibodies was demonstrated by using HRP (horseradish peroxidase) conjugated goat anti-llama immunoglobulin (Bethyl Laboratories Inc., Montgomery, Texas USA) and a subsequent enzymatic reaction in the presence of the substrate TMB (3,3',5,5'-tetramentylbenzidine) (Pierce, Rockford, IL, USA). OD₄₅₀ₙₘ exceeded 1 in both animals. Blood and lymph nodes were collected from these animals, blood 4 and 8 days after the last injection and lymph nodes 13 days after the boost.

### Example 17 : Library construction

Peripheral blood mononuclear cells were prepared from blood samples using Ficoll-Hypaque according to the manufacturer's instructions. Next, total RNA was extracted from these cells as well as from the lymph node bow cells and used as starting material for RT-PCR to amplify Nanobody encoding gene fragments. These fragments were cloned into phagemid vector pAX50. Phage was prepared according to standard methods (see for example the prior art and applications filed by applicant cited herein) and stored at 4°C for further use, resulting in two phage libraries ("E" and "F").

### Example 18 : New selections of anti IL-23 p19 and p40 Nanobodies

To identify Nanobodies recognizing specifically the p19 subunit of human IL-23, 5 or 0.5 nM human IL-23 (R&D Systems, Minneapolis, MN, US) was coated on Nunc Maxisorp ELISA plates. Phages from libraries E and F were predepleted 3 times by binding to wells coated with 5 µg/ml human IL-12 (to remove p40 binding phages) and further preincubated with 1 µM hIL-12 in solution before adding to the IL-23 coated wells. Phages were either specifically eluted with either 500 nM of a biparatopic anti-p19 Nanobody or 500 nM of recombinant IL-23R (R&D Systems, Minneapolis, MN, US), or were eluted with trypsin as described in standard protocols. Outputs of these round 1 selections were analyzed for enrichment factor (number of phage present in eluate relative to controls). Based on this parameter the best outputs were chosen for a second selection round using the same conditions as round 1. Enriched outputs of these round 2 selections were screened in an ELISA for binding to hIL-23 versus hIL-12. Mostly specific binding to hIL-23 was observed. Individual TG1 colonies were picked and grown in 96 deep well plates (1 ml volume) and induced by adding IPTG for Nanobody expression. Periplasmic extracts (volume: ~ 100 ul) were prepared according to standard methods (see for example the prior art and applications filed by applicant cited herein).

The p40 binding Nanobodies were obtained as described above, however bound phages were eluted using trypsin (1 mg/ml). Individual TC1 colonies from first round outputs were picked, grown and induced as described above. Clones were screened in an ELISA for binding to hIL-23 versus hIL-12. Clones binding to both hIL-23 and hIL-12 were selected for further characterization as p40 binders.

### Example 19 : Screening for p19 and p40 binding Nanobodies and identification of p19 and p40 Nanobodies blocking receptor interaction

In order to determine binding specificity to the p19 or the p40 subunit of IL-23, the periplasmic extracts were tested in an ELISA binding assay setup. In this screen also periplasmic extracts derived from selections on libraries derived from Ilama's 146 and 147 were included.

1µg/ml of hIL-12 or hIL-23 was immobilized directly on polysorp microtiter plates (Nunc). Free binding sites were blocked using 1% casein in PBS. Next, periplasmic extracts containing Nanobody of the different clones wered diluted 1/50 in 100 µl PBS + 0.1% casein = 0.05% tween and were allowed to bind to the immobilized antigen. After incubation and washing, Nanobody binding was revealed using a biotinylated anti-His antibody, which, after a wash step, was detected with HRP-conjugated extravidin and TMB substrate. Binding specificity was determined based on OD values compared to controls having received no Nanobody. Nanobodies binding to p40 were identified as Nanobodies recognizing hIL-12 and hIL-23, Nanobodies binding to p19 as the ones only recognizing hIL-23.

Cross-reactivity towards cynomolgus IL-23 was tested in an ELISA, where cynomolgus IL-23 was bound on the plate via an immobilized anti-IL-23 antibody (AF1716 R&D systems). Binding of the Nanobodies present in the extracts was revealed using HRP conjugated anti-myc antibodies and TMB substrate.

In order to determine neutralizing efficiency of the expressed Nanobodies, the periplasmic extracts were screened in a hIL-23 or hIL-12 alpha-screen assay. This assay relies on the use of Donor and Acceptor beads which can be conjugated to biological molecules. When a biological interaction between molecules brings the beads into proximity, excited singlet oxygen molecules that are produced upon laser excitation at 680 nm by a photosensitizer in the Donor bead, diffuse across to react with a chemiluminiscer in the acceptor bead that further activates fluorophores which subsequently emit light at 520-620 nm. If the Nanobody inhibits binding of hIL-23 to hIL-23R, or hIL-12 to hIL12Rbeta1 fluorescent output will decrease, and the amount of Nanobody present will be inversely related to the amount of fluorescence.

ML-12 (R&D Systems) and hIL-23 (eBioscience) were biotinylated using Sulfo-NHS-LC-Biotin (Pierce). Human IL23R-Fc chimera and hIL12Rbetal-Fc chimera (R&D Systems) were coupled to acceptor beads according to manufacturer instructions (Perkin Elmer, Waltham, MA, US). To evaluate the neutralizing capacity of anti-p19 Nanobodies, dilution series of the periplasmic extracts were pre-incubated with biotinylated hIL-23. To this mixture, the IL-23R-Fc coupled acceptor beads and the streptavidin donor beads were added and further incubated for 1 hour at room temperature. Fluorescence was measured by reading plates on the EnVision Multilabel Plate Reader (Perkin Elmer) using an excitation wavelength of 680 nm and an emission wavelength of 520 nm. Decrease in fluorescence signal indicates that the binding of biotinylated hIL-23 to the IL-23 receptor is blocked by the Nanobody expressed in the periplasmic extract, the Nanobodies are designated 19+ when neutralizing and p19- when not neutralizing. The neutralizing capacity of anti-p40 Nanobodies was evaluated in the same way, but using biotinylated hIL-12 and h1L12Rbeta1-Fc coupled acceptor beads, with p40+ neutralizing Nanobodies and p40- non neutralizing Nanobodies.

### Example 20 : Biacore off-rate screening of Periplasmic extracts containing monovalent anti-p19 and anti p40 monovalent Nanobodies.

Human IL-23 (eBioscience) was covalently bound to CM5 sensor chips surface via amine coupling using EDC/NHS for activation and HCl for deactivation. Periplasmic extracts containing p19+, p19-, p40+ or p40- Nanobodies were injected for 4 minutes at a flow rate of 45 µl/min to allow binding to chip-bound antigen. Next, binding buffer without periplasmic extracts was sent over the chip at the same flow rate to allow spontaneous dissociation of bound Nanobody. From the sensorgrams obtained for the different periplasmic extract k_{off}-values (k_{d}) were calculated. (Figure 9 and 10), As a control hIL-12 (R&D systems) was immobilized on the Biacore chips, p19 Nanobodies did not bind to his chip, whereas the p40 Nanobodies bound and showed similar off rates as for hIL-23.

Based on this Bbiacore analysis, p19 and p40 Nanobodies with the best off rates were selected and sequenced. This provided, after sequencing analysis, 6 unique p19+ Nanobodies belonging to 4 families, 2 unique p19- Nanobodies belonging to 2 families, 6 unique p40+ sequences belonging to 5 families and 6 unique p40- sequences belonging to 6 families. Together with the sequences obtained in Examples 1 to 6, this makes a total of 7 families of p19+ Nanobodies, 15 families of p19- Nanobodies, 14 families of p40+ Nanobodies and 11 families of p40- Nanobodies. The amino acid sequences of these Nanobodies are listed in Figures 20 to 23, respectively.

The periplasmic extracts were also screened for cross-reactivity towards murine IL-23, by determining off rates on immobilized mIL-23. Two p19- families were cross-reactive. towards mIL-23, no p19+, p40+ nor p40- mouse cross-reactive Nanobodies were retrieved.

### Example 21: anti IL-23 Nanobody expression and purification

Six p19+ Nanobodies from Example 20, one of the p19- Nanobodies from Example 20 and 12 of the p40+ Naolobodies from Example 20 and 8 of the p40- Nanobodies from Example 20 were expressed, purified and further characterised. Expression occurred in *E. coli* TG1 cells as c-myc, His6-tagged proteins in a culture volume of 250 mL. Expression was induced by addition of 1 mM IPTG and further incubation for 3h at 37°C. After spinning down the cell cultures, periplasmic extracts were prepared by freeze-thawing the pellets and resuspension in dPBS. These extracts were used as starting material for immobilized metal affinity chromatography (IMAC) using Histrap FF crude columns (GE Healthcare). Nanobodies were eluted from the column with 250 mM imidazole and subsequently desalted towards dPBS. For the splenocyte assays described below, endotoxins should be removed from the Nanobodies and this was obtained by either gelfiltration in the presence of 50 mM Octylβ-D-glucopyranoside (OGP, Sigma) or by anion exchange (Mono Q HR5/50, GE healthcare) in dPBS, where the Nanobody is recovered in the flow through, whereas LPS is retained on the column. LPS levels are determined using a LAL-assay.

### Example 22 : Potency of neutralizing anti p19 and anti p40 Nanobodies determined in alpha-screen

hIL-12 (R&D Systems), hIL-23 (eBioscience) and cynomolgus IL-23 were biotinylated using Sulfo-NHS-LC-Biotin (Pierce). Human IL-23R-Fc chimera and hIL12Rbeta1-Fc chimera (R&D Systems, Minneapolis, MN, US) were coupled to acceptor beads according to manufacturer instructions (Perkin Elmer, Waltham, MA, US).

To determine potencies of the p19+ Nanobodies, a dilution series of anti-p19 Nanobodies starting from 250 nM up to 1 pM was pre-incubated with 500 pM biotinylated hIL-23 or cyno IL-23 during 15 minutes at room temperature (RT). To this mixture, the IL-23R acceptor beads and the streptavidin donor beads were added and further incubated for 1 hour at RT. Preincubation of all Nanobodies with biotinylated IL-23 reduced Fluorescence intensity at 520 nm, demonstrating that the Nanobodies can effectively inhibit hIL-23 binding to IL-23R in a dose-dependent manner. The results are shown in Figure 37, which is a graph showing the results obtained in this Example 22 for the measurement of the potency of some anti p19 and anti p40 Nanobodies in alpha-screen using hIL-23; and in Figure 38, which is a graph showing the results obtained in this Example 22 for the measurement of the potency of some anti p19 and anti p40 Nanobodies in alpha-screen using cyno IL-23. The calculated IC50 values are shown in Table B-5, and vary from 7.3 nM for P23IL36A1 to 110 pM for P23IL37D5. However, cyno IL-23 binding is only effectively inhibited by P23IL36A4.

To determine potencies of the p40+ Nanobodies, a dilution series of anti-p40 Nanobodies starting from 250 nM up to 1 pM was pre-incubated with 3 nM biotinylated hIL-12 during 15 minutes at room temperature (RT). To this mixture, the IL12Rbetal acceptor beads and the streptavidin donor beads were added and further incubated for 1 hour at RT. Preincubation of most tested Nanabodies with biotinylated IL-12 reduced fluorescence intensity at 520 nm, demonstrating that the Nanobodies can effectively inhibit hIL-12 binding to IL12Rbeta1 in a dose-dependent manner. The calculated IC50 values are shown in Table B-6, and vary from 23 nM for P23IL81E10 up to 350 pM for P23IL3B8.

**Table B-5: IC50 values for p19+ Nanobodies in alpha-screen for blocking hIL-23 or cynomolgus IL-23 (cIL-23) receptor interaction**

| **P19+ Nanobody** | **IC50 (nM) hIL-23** | **IC50 (nM) cIL-23** |
|---|---|---|
| P23IL 36A1 | 7,3 | 7,80 (partial blocking) |
| P23IL 36B3 | 2,2 | Not blocking |
| P23IL 36B4 | 1,8 | Not blocking |
| P23IL 36E5 | 0,96 | Partial blocking |
| P23IL 36A4 (= 36G2) | 1,00 | 0,47 |
| P23IL 37D5 | 0,11 | 0,32 (partial blocking) |

**Table B-6: IC50 values for p40+ Nanobodies in alpha-screen**

| **P40+ Nanobody** | **IC50 (nM) hIL-12** |
|---|---|
| P23IL 3B8 | 0,35 |
| P23IL 3G10 | 0,90 |
| P23IL 84A12 | 0,96 |
| P23IL 3F11 | not tested |
| P23IL 2 1 C4 | 1,63 |
| P23IL 3C1 | 1,0 |
| P23IL 84G12 | partial blocking |
| P23IL 81E10 | 23 |
| P23IL 22D7 | 1,9 |
| P23IL 22E11 | 1,3 |
| P23IL 23H3 | 1,2 |
| P23IL 23E3 | 1,2 |

### Example 23: Binding kinetics of IL-23 monovalent p19+, p19-, and IL-23/IL-12 p40+, and p40- Nanobodies.

Binding kinetics of of a selection of p19+, p19-, p40+ and p40- Nanobodies were analysed by Surface Plasmon Resonance (Biacore T100). Human IL-23 was covalently bound to CM5 sensor chips surface via amine coupling using EDC/NHS for activation and HCl for deactivation. Nanobody binding was assessed at one concentration (100 nM). Each Nanobody was injected for 4 minutes at a flow rate of 45 µl/min to allow binding to chip-bound antigen. Next, binding buffer without Nanobody was sent over the chip at the same flow rate to allow spontaneous dissociation of bound Nanobody. From the sensorgrams obtained for the different Nanobodies k_{off}-values (k_{d}) were calculated and are indicated in Tables B-7 to B-11. For P23IL37D5, binding was assessed at various concentration, the association and dissociation rate constant (kₒₙ or k_{off}), and hence the binding constants (K_{D}) was determined and are shown in Table B-9.

Cross-reactivity profiles of the monovalent Nanobodies were studied in Biacore experiments using immobilized cynomolgus IL-23). Results are shown in Tables B-7 to B-11.

### Table B-7 to B-11: Off-rates of the anti p19 and p40 for human and cynomolgus IL-23 determined on Biacore.

**Table B-7:**

| **P19+ Nanobody** | **koff(s-1) hIL-23** | **koff (s-1) cyno IL-23** |
|---|---|---|
| P23IL 36A1 | 4,40E-02 | 7,70E,-02 |
| P23IL 36E5 | 3,40E-04 | 4,00E-03 |
| P23IL 36A4 (= 36G2) | 9,00 E-04 | 1,20E-03 |
| P23IL 37D5 | 1,90E-04 | 5,10E-04 |

**Table B-8:**

| **P19- Nanobody** | **koff(s-1) hIL-23** | **koff (s-1) cyno IL-23** |
|---|---|---|
| P23IL 20B11 | 1,3 E-03 / 3 E-04 | 1,6E-03 / 2,9 E-04 |

**Table B-9:**

| **P19+ Nanobody** | **Koff (s-1)** | **Kon (s-1)** | **Kd (M)** |
|---|---|---|---|
| P23IL 37D5 | 1,80E-04 | 3,20E+05 | 5,70E-10 |

**Table B-10:**

| **P40+ Nanobody** | **koff (s-1) hIL-23** | **koff (s-1) cyno IL-23** |
|---|---|---|
| P23IL 3B8 | 3,50E-03 | >1,0E-02 à 7,7 E-04 |
| P23IL 3G10 | 1,97E-03 | 2 à 4 E-03 |
| P23IL 84A12 | 1,60E-03 | |
| P23IL 3F11 | 1,46E-03 | |
| P23IL 21CA | 5,10E-04 | |
| P23IL 3C1 | 2,94E-04 | 5,9 E-05 |
| P23IL 84G12 | 2,90E-04 | 7 E-05 |
| P23IL 81E10 | 2,63E-03 | 1,32E-03/1,2E-03 |
| P23IL 22D7 | 1,70E-04 | 2,30E-04 |
| P23IL 22E11 | 2,30E-04 | 2,30E-04 |
| P23IL 23H3 | 1,10E-03 | 6,7 E-04 |
| P23IL 23E3 | 1,80E-04 | 1,2 à 2,5 E-03 |

**Table B-11:**

| **P40- Nanobody** | **koff (s-1) hIL-23** | **koff (s-1) cyno IL-23** |
|---|---|---|
| P23IL 81A1 | 5,90E-04 | 1,20E-02 |
| P23IL 80D10 | 9,70E-05 | 1,05E-04 7,9E-05 |
| P23IL 84G 1 | 2,00E-04 | 2,40E-03 |
| P23IL 119B2 | 1,52E-02 | |
| P23IL 22D10 | 1,00E-03 | 1,0 E-04 |
| P23IL 20F2 | 5.4E-04 | |
| P23IL 23A11 | 1,10E-03 | |

### Example 24: Epitope grouping of anti-p19 and anti-p40 Nanobodies

In order to determine compatibility of Nanobodies to build into biparatopic or bispecific constructs, some relative epitope mapping experiments were conducted.

To determined relative epitopes of p19 Nanobodies, a given anti-p19 Nanobody was biotinylated using Sulfo-NHS-LC-Biotin (Pierce) and an anti human IL-23 antibody (R&D Mab6091) was coupled to acceptor beads according to manufacturer instructions (Perkin Elmer, Waltham, MA, US). A dilution series of a second anti-p19 Nanobody starting from 200 nM up to 1 pM was pre-incubated with 500 pM hIL-23 during 15 minutes at RT. To this mixture, the anti IL-23 antibody coupled acceptor beads and the biotinylated p19 Nanobody is added and further incubated for I hour at RT, and finally the mixture is incubated with the streptavidin donor beads for 1 hour at RT. If preincubation of a given Nanobody with hIL-23 reduced fluorescence intensity at 520 nm, this Nanobody recognizes the same or an overlapping epitope on hIL-23 as the biotinylated Nanobody. The relative epitopes determined as such are shown in Table B-12.

Accordingly, further aspects of the invention are:
- p19+ sequences (as defined herein, and in particular, (single) domain antibodies and/or Nanobodies binding to the same epitope on p19 as P23IL-121A2 (SEQ ID NO:1890), P231L119A3 (SEQ ID NO:1898) and/or P23IL36A4 (SEQ ID NO:2486), and/or capable of cross-blocking the binding of the same to p19, and/or capable of competing with the same for binding to p19;
- p19- sequences (as defined herein, and in particular, (single) domain antibodies and/or Nanobodies binding to the same epitope on p19 as P23IL81A12 (SEQ ID NO: 1936) and/or P23IL81G2 (SEQ ID NO:1930), and/or capable of cross-blocking the binding of the same to p19, and/or capable of competing with the same for binding to p19;
- p19- sequences (as defined herein, and in particular, (single) domain antibodies and/or Nanobodies) binding to the same epitope on p19 as P23IL-124C4 (SEQ ID NO: and/or P23IL20B11 (SEQ ID NO:2502), or capable of cross-blocking the binding of the same to p19, and/or capable of competing with the same for binding to p19;
- p19- sequences (as defined herein, and in particular, (single) domain antibodies and/or Nanobodies binding to the same epitope on p19 as P23IL119G7 (SEQ ID NO:1902), and/or capable of cross-blocking the binding of the same to p19, and/or capable of competing with the same for binding to p19.

**Table B-12 : relative epitope grouping of p19 Nanobodies, X, A, B, C correspond to separate epitopes; X/A is a separate epitope where the Nanobody, prevents binding of Nanobodies recognizing epitope X or epitope A**

| **Nanobody type** | **ID** | **Epitope group** |
|---|---|---|
| p19+ | P23IL-121A2 | X |
| | P23IL119A3 | x |
| | P23IL37D5 | X/A |
| | P23IL36A4 | X |
| p19- | P23IL81A12 | A |
| | P23IL81G2 | A |
| | P23IL-124C4 | B |
| | P23IL20B11 | B |
| | P23IL119G7 | C |

A similar experiment was conducted to determine relative epitopes of p40 Nanobodies. Acceptor beads were coupled to the p19+ Nanobody P23IL-121A2. In a first experiment the p40+Nanabodies P23IL3B8, 3C1, 81E10, 23E3, 23H3 and 3G10 and the p40- Nanobody P23IL80D10 were biotinylated. Not biotinylated Nanobodies P23IL3B8, 3C1, 23E3, 23H3, 3G10, 81E10, 80D10, 20F2, 22D10, 23A11, 80A3, 84G1, 81A1, 21C4, 22D7, 22E11, 84A12, 84G12 and 3F1 were preincubated at a concentration of 100 nM with 400 pM hIL-23 during 15 minutes at RT. To the Nanobody-hIL-23 mixture, the P23IL-121A2 coupled acceptor beads and a given biotinylated Nanobody is added and further incubated for 1 hour at RT, and finally the mixture is incubated with the streptavidin donor beads for 1 hour at RT. If preincubation of a given Nanobody with hIL-23 reduced fluorescence intensity at 520 nm, this Nanobody recognizes the same or an overlapping epitope on hIL-23 as the biotinylated Nanobody/mAB.

As expected all p40+ Nanobodies bind to different epitopes as the p40- Nanobodies. For the p40+ Nanobodies, the relative mapping is more complicated, but the results show that:
- Of the Nanobodies shown in Figure 23, Nanobodies P23IL23E3 (SEQ ID NO: 2514), 23H3 (SEQ ID NO:2515), 3G10 (SEQ ID NO: 2516), 21C4 (SEQ ID NO:2510), 22D7 (SEQ ID NO: 2511), 22E1 (SEQ, ID NO:2512), 84A12 (SEQ ID NO: 1970) and 3F11 (SEQ ID NO: 2525) recognize at least part of the same epitope as P23IL3B8 (SEQ ID NO:2527). Accordingly, p40+ sequences (as defined herein, and in particular, (single) domain antibodies and/or Nanobodies) binding to the same epitope on p40 as P23IL3B8 (SEQ ID NO:2527), and/or capable of cross-blocking the binding of the same to p40, and/or capable of competing with the same for binding to p40, form a further aspect of the invention.
- Nanobodies P23IL23E3 (SEQ ID NO: 2514), 23H3 (SEQ ID NO:2515), 81E10 (SEQ ID NO:1946), 80D10 (SEQ ID NO:1940), 20F2 (SEQ ID NO:2504), 22D10 (SEQ ID NO:2505), 80A3 (SEQ ID NO:1952), 84G1 (SEQ ID NO:1992), 81A1(SEQ ID NO:1962), 22D7 (SEQ ID NO:2511) and 84G12 (SEQ ID NO:1942) recognize at least part of the same epitope as P23IL3C1 (SEQ ID NO:2033). Accordingly, p40+ or p40-sequences (as defined herein, and in particular, (single) domain antibodies and/or Nanobodies) binding to the same epitope on p40 as P23IL3C1 (SEQ ID NO:2033), and/or capable of cross-blocking the binding of the same to p40, and/or capable of competing with the same for binding to p40, form a further aspect of the invention;
- Nanobodies P23IL3B8 (SEQ ID NO:2527), 3C1 (SEQ ID NO:2033), 23H3 (SEQ ID NO: 2515), 3G10 (SEQ ID NO: 2516), 81E10 (SEQ ID NO: 1946), 80D10 (SEQ ID NO:1940), 80A3 (SEQ ID NO:1952), 21C4 (SEQ ID NO:2510), 22D7 (SEQ ID NO:2511), 22E11 (SEQ ID NO:2512), 84A12 (SEQ ID NO: 1970), 3F11 (SEQ ID NO_{:} 2525) recognize at least part of the same epitope as P23IL-23E3 (SEQ ID NO: 2514). Accordingly, p40+ or p40- sequences (as defined herein, and in particular, (single) domain antibodies and/or Nanobodies) binding to the same epitope on p40 as P23IL-23E3 (SEQ ID NO: 2514), and/or capable of cross-blocking the binding of the same to p40, and/or capable of competing with the same for binding to p40, form further aspects of the invention;
- Nanobodies P23IL 3B8 (SEQ ID NO:2527), 3C1 (SEQ ID NO:2033), 23E3 (SEQ ID NO: 2514), 3G10 (SEQ ID NO: 2516), 81E10 (SEQ ID NO:1946), 80A3 (SEQ ID NO:1952), 84G1 (SEQ ID NO:1992), 81A1 (SEQ ID NO:1962), 21C4 (SEQ ID NO:2510), 22D7 (SEQ ID NO:2511), 22E11 (SEQ ID NO:2512), 84A12 (SEQ ID NO: 1970), 84G12 (SEQ ID NO:1942), 3F11 (SEQ ID NO: 2525) recognize at least part of the same epitope as P23IL-23H3 (SEQ ID NO:2515). Accordingly, p40+ or p40-sequences (as defined herein, and in particular, (single) domain antibodies and/or Nanobodies) binding to the same epitope on p40 as P23IL-23H3 (SEQ ID NO:2515), and/or capable of cross-blocking the binding of the same to p40, and/or capable of competing with the same for binding to p40, form further aspects of the invention.
- Nanobodies P23IL 3B8 (SEQ ID NO:2527), 23E3 (SEQ ID NO: 2514), 23H3 (SEQ ID NO: 2515), 81A1 (SEQ ID NO:1962), 21C4 (SEQ ID NO:2510), 22D7 (SEQ ID NO:2511), 22E11 (SEQ ID NO:2512), 84A12 (SEQ ID NO: 1970) and 3F11 (SEQ ID NO: 2525) recognize the same epitope as 23IL3G10 (SEQ ID NO: 2516).
- Nanobodies P23IL 3C1 (SEQ ID NO:2033), 23E3 (SEQ ID NO:2514), 23H3 (SEQ ID NO: 2515), 80D10 (SEQ ID NO:1940), 22D10 (SEQ ID NO: 2505), 80A3 (SEQ ID NO:1952), 8401 (SEQ ID NO:1992), 81A1 (SEQ ID NO: 1962) and 84G12 (SEQ ID NO: 1942) recognize the same epitope as P23IL81E10 (SEQ ID NO: 1946). Accordingly, p40+ or p40- sequences (as defined herein, and in particular, (single) domain antibodies and/or Nanobodies) binding to the same epitope on p40 as P23IL81E10 (SEQ ID NO: 1946), and/or capable of cross-blocking the binding of the same to p40, and/or capable of competing with the same for binding to p40, form further aspects of the invention.

In a second experiment the p40+ Nanobodies P23IL-22D7 (SEQ ID NO:2511) and 22E11 (SEQ ID NO:2512) were biotinylated. Non-biotinylated Nanobodies P23IL84G12, 80D10, 20F2, 22D10, 23A11, 80A3, 84G1, 81A1, 22D7 and 22E11 were preincubated at a concentration of 250 nM or 71 nM with 400 pM hIL-23 during 15 minutes at RT. To the Nanobody-hIL-23 mixture, the P23IL-121 A2 coupled acceptor beads and biotinylated P23IL-22D7 or 22E11 is added and further incubated for 1 hour at RT, and finally the mixture is incubated with the streptavidin donor beads for 1 hour at RT. If preincubation of a given Nanobody with hIL-23 reduced fluorescence intensity at 520 nm, this Nanobody recognizes the same or an overlapping epitope on hIL-23 as the biotinylated Nanobody.. The results are shown in Figures 39 (epitope mapping against 22D7) and 40 (epitope mapping against 22E11). Accordingly, p40+ or p40- sequences (as defined herein, and in particular, (single) domain antibodies and/or Nanobodies) binding to the same epitope on p40 as P23IL-22D7 (SEQ ID NO:2511), and/or capable of cross-blocking the binding of the same to p40, and/or capable of competing with the same for binding to p40, form further aspects of the invention. Similarly, Accordingly, p40+ or p40- sequences (as defined herein, and in particular, (single) domain antibodies and/or Nanobodies) binding to the same epitope on p40 as P23IL-22E11 (SEQ ID NO:2512), and/or capable of cross-blocking the binding of the same to p40, and/or capable of competing with the same for binding to p40, form further aspects of the invention.

Relative epitope mapping of the p19+ Nanobody P23IL119A3 versus the three p40+ Nanobodies P23IL3B8, P23IL3C1 and P23IL-23E3 was performed using biacore where P23IL119A3 was attached to the chip and used to capture hIL-23. Subsequently the p40+ monovalent Nanobodies were tested for their capacity to bind. As expected the p40+ Nanobodies bind to a separate epitope group since they could still bind hIL-23 already attached to P23IL119A3 fixed to the Biacore chip.

### Example 25 : Neutralizing activity of p19+ and p40+ Nanobodies in cell based assays

A mouse splenocyte assay (modified from Example 15) was used to determine inhibition of IL-23 activity based on the ability of IL-23 to stimulate IL-22 secretion from mouse spleen cells. The basic protocol is as follows: the spleens of 5 C57BL/6 mice are removed and the splenocytes are harvested and a single cell suspension is prepared. The splenocytes are washed 3 times in RPMI (Invitrogen, Cat 72400-021) supplemented with 10% FBS (Invitrogen, Cat 10270-106), 1% Pen/Strep (Invitrogen, Cat 15140-122), 80µM β-mercapto-ethanol (Invitrogen, Cat 31350-010) and 1 mM SodiumPyruvate (Gibco, Cat 1136). This medium is further referred to as complete RPMI. The splenocyte suspension is treated with 1x erythxocyte lysis buffer (10x stock: 82.9g NH4Cl, 10.9g KHCO3 and 370 mg EDTA in 1 liter MilliQ) to remove all residual erythrocytes. After 3 wash steps in complete RPMI, the cells are filtered over a 100µM cell strainer (BD, Cat 35260) and resuspended in complete RPMI containing 20ng/ml recombinant mIL-2 (R&D systems, Cat 402-ML). Cells are seeded at 400 000 cells/well in 96-well flat bottom plates (Falcon, Cat 353072).

Nanobodies (50 µl) were pre-incubated with recombinant hIL-23 in complete RPMI for 30 minutes at room temperature and then incubated for another 5 days with the splenocytes, at a pre-determined final concentration of hIL-23 or cyno IL-23 (ranging from 3.7-19 µM). Supernatants were collected and levels of mIL-22 measured using ELISA (mouse IL-22 ELISA construction kit, Antigenix America, Cat RMF222CK).

As shown in Figures 41 and 42, monovalent p40+ (Figure 41) and p19+ (Figure 42) Nanobodies inhibit hIL-23 mediated IL-22 production. As control an anti-p40 mAB was used (referred to herein as "*BM01*"), which was a human mAB according to WO 00/56772 (with the variable chain sequences of SEQ ID NO's; 31 and 32 of WO 00/56772), which was prepared according to standard techniques.

Table B-13 shows the potencies for p19+ Nanobodies for blocking 19 pM hIL-23 and 3.7 pM cynomolgus IL-23 (eIL-23). Table B-14 shows the potencies for p40+ Nanobodies for blocking 19 pM hIL-23 and 3.7 pM cyno IL-23 (nc = blocking not complete).

**Table B-13: potencies for p19+ Nanobodies for blocking 19 pM hIL-23 and 3.7 pM cynomolgus IL-23 (cIL-23).**

| **Namobody** | **hIL-23 IC50 (pM)** | **cIL-23 IC50 (pM)** |
|---|---|---|
| P23IL119A3 | 25510 | 37430 |
| BM01 | 8 | 25 |
| P23IL36A4 | 3120 | 10955 |
| P23IL37D5 | 17 | 288 |

**Table B-14: potencies for p40+ Nanobodies for blocking 19 pM hIL-23 and 3.7 pM cyno IL-23 (nc = blocking not complete).**

| **Nanobody** | **hIL-23 IC50 (pM)** | **cIL-23 IC50 (pM)** |
|---|---|---|
| BM01 | 15 | 40 |
| P23IL3B8 | 408 | 1787 |
| P23IL3C1 | nc 510 | nc 517 |
| P23IL3G10 | 2325 | 9451 |
| P23IL-23E3 | 935 | 5461 |
| P23IL-23H3 | 790 | 32540 |
| P23IL-22D7 | 116 | 705 |
| P23IL-22E11 | 186 | 1269 |

### Example 26 : Generation of a Ba/F3 cell line stably expressing hIL23R and hIL12Rβ1

Ba/F3, a murine IL-3 dependent pro-B cell line, was cultured in RPMI (Invitrogen, Cat 72400-021) supplemented with 10% FBS (Invitrogen, Cat 10270-106), 1% Pen/Strep (Invitrogen, Cat 15140-122) and 10% conditioned medium of WEHI-3B, a mouse myelornonocytic cell line, constitutively producing IL-3. At first, the BA/F3 cell line was stably transfected with an expression vector encoding hIL-12Rβ1. Transfection was performed using an Amaxa nucleofector II (Cat. No. AAD-1001) and the Amaxa nucleofection kit V (Cat. No. VCA-1003). Immediately after transfection, 500µl pre-warmed culture medium is added to the cuvette and the entire content is transferred into a T25 containing 4ml culture medium as described above. To select the transfected cells, 1.1mg/ml Hygromycin B (Invitrogen, Cat 10687-010) was added 48h after transfection.

The pool of hIL12Rβ1 expressing Ba/F3 cells was single-cell sorted using a FACSaria. Hereto, cells were resuspended in a 15ml Falcon tube at 20E6/ml in diluted mouse-anti-hIL-12Rβ1 (0.5 mg/ml, R&D systems, Cat MAB839). The cells were counted using Trypan Blue and haematocytometer. All dilutions and wash steps are done with PBS 1x (Invitrogen: 141190-094) supplemented with 10% FBS. After 30 minutes incubation at 4°C, the cells are washed three times with PBS. A second staining is done with phycoerythrin-labeled anti-mouse IgG (Jackson ImmunoResearch Laboratories, Cat 115-115-164). The cells are incubated for 30 minutes at 4°C and washed three times. A final stain with TOPRO3 (Molecular Probes T3605) is included to exclude dead cells. H-IL12Rβ1 expressing Ba/F3 cells were single-cell sorted into 96-well culture plates and further cultured.

After several screenings on FACSarray, h-IL12Rβ1 expressing Ba./F3 subclone 4D9 was selected for transfection with a hIL-23 encoding plasmid. The results of flow cytometric analyis of h-IL12Rβ1 expressing Ba/F3 subclone 4D9, as obtained in this Example 26 arc shown in Figure 43. Detection was performed by a mouse-anti-hIL,- 12Rβ1antibody followed by a PE-labeled polyclonal anti-mouse Ig antibody. The presence of h-IL12Rβ1 on the cells was measured by an increase in fluorescence intensity as compared to cells that were incubated with FACS buffer (PBS + 10% FBS) followed by PE-labeled polyclonal anti-mouse Ig antibody. Fluorescence intensity is plotted on the X-axis, the number of events on the Y-axis. The FACS profile of unstained cells is also shown.

For the stable transfection of the hIL12Rβ1 expressing Ba/F3 subclone 4D9 with hIL-23R, the same protocol as described above is used, with the only difference that 2µg pcDNA3.1Neo-hIL23R instead of 2µg pcDNA3.1Hygro- h-IL12Rβ1 is used. To select for stable transfected cells, 1mg/ml G418 (Invitrogen, Cat 10131-07) was added 48h after transfection. After culturing a few passages on G418 selection, the pool of Ba/F3-hIL12Rβ1-hIL23R cells was depleted of WEHI-3B culture supernatant (containing the mIL-3 growth factor) and supplemented with 100ng/ml recombinant hIL-23 (e-Bioscience, Cat 34-8239-85). The surviving cells were expanded and seeded out at 3 cells/well, 1 cell/well or 0.3 cells/well in RPMI supplemented with 10% FBS, 1% P/S and 100ng/ml recombinant hIL-23.

Obtained single cell clones are expanded and screened for their hIL-23 responsiveness in a cell proliferation assay. Hereto, selected cell clones are washed three times in RPMI supplemented with 10% FBS and 1% Pen/Strep and subsequently incubated for 3 hours in hIL-23-free medium at a cell density of 3E06 - 5E06 cells/ml. After starvation, the cells are seeded at 7500 cells/well in 96-well flat bottom plates (Greiner bio-one, Cat 655180) in a volume of 100µl hIL-23-free medium. Next, 100µl of a hIL-23 dilution curve is added to the cells in such a manner that a 1/2 serial dilution starting from 500ng/ml is obtained. After 72 hours of incubation, the cells are pulsed with 1 µCi [³H]-thymidine and incubated for an additional 24h prior to freezing at -80°C. Cells were subsequently thawed and embedded on glass fiber membranes using a cell harvester (Perkin Elmer Life Sciences, Wellesley, MA, USA). After several washings with water, filters were air-dried and counted using a γ-counter (Perkin Elmer Life Sciences). The results are shown in Figure 44, which is a graph showing the results obtained in this Example 26 for hIL-23 responsiveness of several Ba/F3-hIL12Rβ1-hIL23R single cell clones, in a cell proliferation assay after a 72 hour incubation with several concentrations hIL-23, as a readout using [methyl-³H]-thymidine incorporation [ON pulse]. Clone 5H10 was chosen for further characterization of IL-23 neutralizing Nanobody formats.

### Example 27 : IL-12 dependent PHA blast Proliferation assay

PHA blasts were derived by culturing PBMC in PHA (50µg/ml) during 3 days and IL-2 (50 µ/ml) during one day. These PHA blasts were stimulated with 1 pM hIL-12 pre-incubated with a dilution series of a p40+ Nanobody starting starting from 2000 nM up to 1 pM or control Nanobodies or antibodies. Cells were stimulated for 2 days and pulsed with 1 µCi/well ³H-thymidine for the 6 last hours, hence proliferation was determined by measuring ³H-thymidine incorporation by scintillation counting.

As shown in Table B-15, most tested monovalent p40+ Nanobodies inhibit hIL-12 mediated proliferation.

**Table B-15: potency of p40+ Nanobodies to block the hIL-12 mediated proliferation using 1 pM hIL-12 (nc = no compete blocking).**

| **Nanobody** | **Input hIL-12 (pM)** | **IC 50 (pM)** |
|---|---|---|
| **BM01** | 1 | 0,67 |
| 3B8 **(SEQ ID NO: 2527)** | 1 | nc 1505 |
| 3C1 **(SEQ ID NO: 2526)** | 1 | hardly blocking |
| 3G10 **(SEQ ID NO: 2516)** | 1 | 194 |
| 23E3 **(SEQ ID NO: 2514)** | 1 | nc 1734 |
| 23H3 **(SEQ ID NO: 2515)** | 1 | 174 |
| 22D7 **(SEQ ID NO: 2511)** | 1 | 116 |
| 22E11 **(SEQ ID NO: 2513)** | 1 | 159 |

### Example 28 : Construction, production and purification of additional bispecific/biparatopic IL-23 p19 Nanobodies in order to investigate the effect of linker length on potency

In addition to the biparatopic constructs described in Example 13, some biparatopic constructs were made with shorter linkers to determine the optimal linker length. The individual building blocks were fused by a 9 Gly/Ser (GGGGSGGGS; SEQ ID NO: 2649) or a 20 Gly/Ser linker (GGGGSCGGSGGGGSGGGGSGGGGSGGGGS; SEQ ID NO: 2650) and cloned in the the pAX100 vector. The sequences of these biparatopic anti-p19 Nanobodies are shown in Figure 30. The constructs were transformed in TG1 cells which were induced with 1 mM IPTG. Nanobodies were purified from the Periplasmic extract using histidine trap affinity chromatography followed by desalting. Further purification and LPS removal was performed by gel filtration in the presence of 50mM Octylβ-D-glucopyranoside (Sigma). Potency was evaluated in the splenocyte assay (see Example 27) and IC50's are listed in Tables B-16 and B-17. Results clearly show that shortening the linker length worsens the IC50, so the use of a linker with more than 20 amino acid residues in total (such as between 20 amino acid residues and 45 amino acid residues, for example a 35 Gly/Ser linker (GGGGSGGGGSGGGGSGGGGSGGGGSGGGGSGGGGS, SEQ ID NO: 2651) may be preferred.

**Table B-16: potencies in the splenocyte assay using 19 pM hIL-23**

| **Nanobody** | **hIL-13 IC50 (pM)** |
|---|---|
| P23IL119A3-35GS-81G2 | 14 |
| P23IL119A3-9GS-81G2 | 1988 |
| P23IL-121A2-35GS-81G2 | 94 |
| P23IL-121A2-9GS-81G2 | 740 |

**Table B-17: potencies in the splenocyte assay using 37 pM hIL-23**

| **Nanobody** | **hIL-23 IC50 (pM)** |
|---|---|
| P23IL-121A2-35GS-124C4 | 57 |
| P23IL-121A2-20GS-124C4 | 1360 |
| P23IL-121A2-9GS-124C4 | 1715 |
| P23IL-121A2-35GS-119G7 | 42 |
| P23IL-121A2-9GS-119G7 | 4755 |

### Example 29 : Construction, production and characterization of HLE p19 biparatopic constructs with extended half-life

### A) HLE1 : Anti Human Serum Albumin Nanobody, ALB-1:

Trivalent biparatopic anti-p19 Nanobodies were constructed (e.g. P23IL-121A2-9GS-ALB1-9GS-124C4) which comprise of two building blocks corresponding to anti-p19 Nanobodies with in the middle a third building block corresponding to an anti Human Serum Albumin Nanobody building block (ALB-1; SEQ ID NO: 790). The individual building blocks are fused by a 9 Gly/Ser linker (GGGGSGGGS; SEQ ID NO: 792). To test if the positioning of the ALB1 Nanobody influences the potency, we also made a construct with ALB-1 at the C-terminus (e.g. P23IL119A3-35GS-81G2-9GS-ALB1), where the p19 Nanobodies are fused by a 35 Gly/Ser linker and the ALB1 is fused to this by a 9 Gly/Ser linker. The sequences of these trivalent bispecific anti-p19 Nanobodies are also shown in Figure 30.

These constructs were cloned in pAX100, transformed to TG1 cells, expressed as c-myc, His6-tagged proteins and subsequently purified from the periplasmic extract by immobilized metal affinity chromatography (IMAC) and size exclusion chromotagraphy (SEC) in the presenc of OGP to remove endotoxins. Two of such formatted Nanobodies were produced at larger scale to test in the acute in vivo mouse splenocyte assay (see example 36).

P23IL0036=119A3-9GS-ALB1-9GS-81 G2 was produced in *E. coli* and captured on MabCapture A (Poros), eluted using 100mM Glycine pH 2.5 and immediately neutralized with 150mM Tris pH8.8. The Nanobody was further polished on Poros 50HS (Poros), incubated, overnight at 4°C, with 50mM OGP (Octylβ-D-glucopyranoside, Sigma) for LPS-removal, followed by SEC (Superdex75pg, GE Healtheare).P23IL0044 = 119A3(H37Y-M43K)-9GS-ALB1-9GS-81A12 was first recloned in pAX98 (which results in P23IL0049) and transformed *Pichia pastoris.* It was then purified starting from the clarified fermentation broth of the recombinant *Pichia postoris,* expressing the Nanobody®. The fermentation broth was made particle free and concentrated in D-PBS via tangential flow filtration using respectively 0.22µm and 10KMWCO HydroSart cassettes (Sartorius). The TFF retentate was further processed on MabCaptureA (Poros), after elution using 100mM Glycine pH 2.5, fractions were neutralized with 150mM Tris pH7.5 and dialysed to 1/10 PBS (SnakeSkin Pleated Dialysis Tubing 10,000 MWCO (Pierce, ref 68100)). The Nanobody was polished via cation exhange chromatography ((Poros 50HS, Poros) followed by Size exclusion chromatography (Superdex75 pg, GE Healthcare).

### B) HLE2 : HSA

A HSA fusion of the biparapic anti-p19 nanbody P23IL119A3-35GS-81G2 was made by fusing HSA directly to the C-terminus of this construct. This occurred by ligating the coding sequence for P23IL119A3-35GS-81G2 into the pAX99 vector (which is pPICZalphaA vector containing the coding sequence for full length human serum albumin) resulting in P23IL0041 =P23IL119A3-35GS-81G2-HSA. The plasmid was transformed into *Pichia pastoris* strain X33. It was then purified starting from the clarified fermentation broth of the recombinant *Pichia pastoris,* expressing the Nanobody. The clarified fermentation broth was made particle free via TFF (Hydrosart 0.22 µm cassette, Sartorius), followed by a concentration and diafiltration step to PBS using TFF (PESU-AL cassette Sartorius). The protein of interest was purified via a capture step on MabCaptureA (Poros) and further polishing steps using Poros50HQ and Superdex200 XK26160.

### C) HLE3 : PEG

In order to pegylate biparatopic Nanobodies, constructs were generated whereby the C-termninal tag was replaced by a GGGC-sequence. Therefore the coding sequences of P23IL119A3-35GS-81G2 and of P23TL-121A2-35GS-124C4 were cloned into the pAX 93 vector. Constructs were expressed in TG1. Purification and pegylation was carried out as follows: Nanobody P23IL0038=P23IL119A3-35GS-81G2-GGGC was captured on MabSelect Xtra^{™} (GE Healthcare) and eluted using 100mM Glycine pH 2.5. The collected sample was immediately neutralized using 150mM Tris pH7.5, dialyzed against 1/10 PBS (SnakeSkin Pleated Dialysis Tubing 10,000 MWCO (Pierce, ref 688100)) and further polished via CEX (Poros 50HS, Poros) in the presence of 5 mM DTT. The fraction containing the Nanobody was concentrated via Vivaspin (5 kDa), DTT was added to a final concentration of 10 mM and incubated overnight. After removal of the excess of free DTT by SEC (Superdex 75pg XK 16/60, GE healthcare), the reduced Nanobody was incubated overnight with a 5 molar excess of branched-mPEG40 (NOF, SunBright GL2-400MA). The PEGylated Nanobody was further purified on MacroCap SP (GE Healthcare) to remove the excess of non-reacting PEG40. Finally, the PEGylated Nanobody was treated overnight with 50nM Octylβ-D-glucopyranoside (Sigma) for LPS-removal, and sized using Superdex75pg (GE Healthcare).

For the generation of a second batch of pegylated P23IL0038, the Nanobody was captured on MabCapture A (POROS), eluted using 100mM Glycine pH 2.5 and immediately neutralized using 150mM Tris pH7.5. After overnight dialysis at 4°C (SnakeSkin Pleated Dialysis Tubing 10,000 MWCO (Pierce, ref 68100)) against 5L 1/10 PBS, the sample was further polished on Poros 50HS (PoroS), followed by a reduction with 10mM DTT. The excess of DTT was removed by SEC (Superdex75pg, GE healthcare) and the seduced Nanobody was coupled to linear-mPEG40 (40 kDa methoxy poly (ethylene glycol) maleimido - propionamice, Dow Pharma). The PEGylated Nanobody was separated from the excess of mPEG40 and non-reacting Nanobody on MacroCap SP (GE healthcare). Finally, the PEGylated Nanobody was treated with 50mM Octylβ-D-gluvopyranoside (Sigma) for LPS-removal, followed by a final a SEC (Superdex75pg, GE Healthcare).

Nanobody P231L0039 = P23IL-121A2-35GS-124C4-GGGC was captured on MabSelect Xtra™ (GE Healthcare) and eluted using 100mM Glycine pH 2.5. The collected sample was immediately neutralized using 150mM Tris pH7.5, and further polished via Anion exchange chromatography (Poros 50HQ, Poros). The fraction containing the Nanobody was reduced in the presence of 10 mM DTT. After an overnight incubation, the excess of free DTT was removed by size exclusion chromatography (Superdex 200pg XK26/60, GE healthcare). The reduced Nanobody was incubated overnight with a 5 molar excess of branched-mPEG40 (NOF, SunBright GL2-400MA). The PEGylated Nanobody was further purified on MacroCap SP (GE Healthcare) to remove the excess of non-reacting PEG40. Finally, the PEGylated Nanobody was sized using Superdex 200pg XK26/60 (GE Healthcare).

### Example 30: Sptenocyte assay of HLE Nanobodies and cross-reactivity on cynomolgus IL-23

The HLE nanobodies of Example 29 were tested and compared in the splenacyte assay as described in Example 25, results are shown in Table B-18. All three type of HLE are suitable since no HLE type leads to loss of potency, on the contrary, potency slightly improves. Also the position of the ALB1 Nanobody does not influence the potency. As the 9GS-ALB1-9GS moiety can efficiently replace the 35Gly/Ser-linker, anti-p19 biparatopic Nanobodies containing the P23IL37D5 building block, were immeadiately constructed, expressed and tested as HLE Nanobodies with ALB1 in the middle.

In addition, cross reactivity towards cynomolgus IL-23 was checked. All anti-p19 biparatopic Nanobodies containing the P23IL119A3 and the P23IL37D5 building block are cross-reactive, the ones containing the P23IL-121A2 building block are not.

**Table B-18: Comparison of potencies of anti-p19 biparatopic Nanobodies with different HLE in splenocyte assay with 19 µM ML-23 or 3,8 pM cynomolgus IL-23 (cIL-23), nb = not blocking; nt= not tested**

| **Nanobody/mAB** | **hIL-23 IC50 (pM)** | **cIL-23 IC50 (pM)** |
|---|---|---|
| P23IL119A3-35GS-81A121 | 7.0 | 5.0 |
| P23IL119A3-9GS-ALB1-9GS-81A12 | 2.5 | 3.3 |
| P23IL119A3(H37Y)- 9GS-ALB1-9GS-81A12 | 2.1 | 2.8 |
| P23IL119A3(H37Y-M43K)-9GS-ALB1-9GS-81A12 | 3.1 | 4.3 |
| P23IL119A3-35GS-81G2 | 12.0 | 13.0 |
| P23IL119A3-9GS-ALB1-9GS-81G2 | 4.0 | 3.0 |
| P23IL119A3-35GS-81G2-9GS-ALB1 | 4.4 | 6.2 |
| P23IL119A3-35GS-81G2-HSA | 5.4 | 8.1 |
| P23IL119A3-35GS-81G2-GGGC-PEG40 branched | 5.6 | 5.3 |
| P231L119A3-35GS-81G2-GGGC-PEG40 linear | 7.1 | 4.7 |
| P23IL-121A2-35GS-119G7 | 26.0 | nb |
| P23IL-121A2-9GS-ALB1-9GS-119G7 | 39.0 | nb |
| P23IL-121A2-35CS-81G2 | 154,0 | nb |
| P23IL-121A2-9GS-ALB1-9GS-81G2 | 8.0 | nb |
| P23L-121A2-9GS-ALB1-9GS-124C4 | 22.0 | nb |
| P23IL-121A2-35GS-124C4-GGGC-PEG40 branched | 66.0 | nb |
| BM01 | 14.2 | 28.0 |
| P23IL37D5 | 24 | 212 |
| P23IL37D5-9GS-ALB1 | 29,8 | 419 |
| P23IL37D5-9GS-ALB1-9GS-124C4 | 8,6 - 17 | 130-258 |
| P23IL37D5-9GS-ALB1-15GS-124C4 | 1,57 - 3,1 | 129 - 257 |
| P23IL37D5-9GS-ALB1-9GS-20B1 | 0,55 - 1,1 | 3,4 - 6,9 |
| P23IL-124C4-15GS-ALB1-9GS-37D5 | 4,2 | 3,2 |
| P23IL37D5-15GS-ALB1-15GS-20B11 | 5,77 | 5,75 |
| P23IL37D5-15GS-ALB1-15GS-124C4 | 3,83 | 18,6 |
| P23IL20B11-9GS-ALBI-9GS-37D5 | 4,47 | 8,59 |
| P23IL-124C4-15GS-ALB1-15GS-37D5 | 2,25 | nt |

### Example 31: Effect of biparatopic/bispecific anti-ML-23 Nanobodies on hIL-23 induced Ba/F3- hIL12Rβ1-hIL23R cell proliferation.

The Ba/F3-ML12Rβ1-ML23R subclone 5H10, generated as described in Example 26, is cultured in RPMI supplemented with 10% FBS, 1% Pen/Strep, 10% conditioned medium of WEHI-3B cell line, 1mg/ml G418 and 1.1mg/ml Hygromycin B. The cells are washed three times in RPMI supplemented with 10% FBS and 1% Pen/Strep. The cells are incubated for 1 hour in this mIL-3-free medium at a cell density of 3E06 - 5E06 cells/ml to remove all residual growth factor.

To evaluate the neutralizing capacity of the different Nanobodies, 50µl of Nanobody dilution is pre-incubated for 30 minutes with 50µl hIL-23 (final concentration of 4ng/ml) in 96-well flat bottom plates (Greiner bio-one, Cat 655180). Next, 50 µl Ba/F3- ML12Rβ1-hIL23R cells are added to these pre-incubated samples to reach a final density of 50000 cells/well.

After 24 hours at 37°C in presence of 5% CO₂, cell viablility was measured using CellTiter-Glo® Luminescent Cell Viability Assay (Promega G7571) according to the manufacturer's instructions. The HLE.Nanobodies of Example 29 that were tested are able to efficiency block the proliferation of these cells as shown in Example 32.

### Example 32: IL-23 dependant BAF3 proliferation assay of HLE Nanobodies

The HLE Nanobodies of Example 29 were also tested and compared in the hIL-23 dependent BAF3 proliferation assay as described in example 27 using 76 pM hIL-23. All Nanobodies tested are able to efficiently block the proliferation of these cells as shown in Table B-19.

**Table B-19: potencies in the BAF3 proliferation assay using 76 pM hIL-23**

| **Nanobody/mAB** | **IC 50 (pM)** |
|---|---|
| P23IL119A3(H37Y-M43K)-9GS-ALB1-9GS-81A12 | 46,5 |
| P23IL119A3-35GS-81G2-HSA | 61,4 |
| P23IL119A3-35GS-81G2-GGGC-PEG40 linear | 58,5 |
| P23IL119A3-35GS-81G2-GGGC-PEG40 branched | 61,2 |
| BM01 | 59,7 |
| | |

| **Nanobody/mAB** | **IC 50 (pM)** |
|---|---|
| P23IL37D5-9GS-ALB1-9GS-20B11 | 19,5 |
| P23IL-124C4-9GS-ALB1-9GS-37D5 | 13,5 |
| BM01 | 33,2 |
| | |

| **Nanobody/mAB** | **IC 50 (pM)** |
|---|---|
| PIL2337D5 | 13 |
| BM01 | 41 |

### Example 33: Induction of native human IL- 23 and determination of the potency of the HLE nanobodies towards this native human IL-23

The human monocytic cell line THP-1 (ATCC TIB-202) was used for the production of native human IL-23. THP-1 cell were cultured in RPMI1640 medium supplemented with 10% fetal bovine serum, 100U/mL, penicillin and 100 µg/mL streptomycin (all from Invitrogen, Carlsbad, USA). Cells were maintained by subculturing every 2-3 days, keeping the cell density between 10⁴-10⁶ cells/mL. Secretion of IL-23 was induced by stimulating THP-1 cells with fixed *Staphylococcus aureus* cells (SAC; Pansorbin, Calbiochem, Merck). THP-1 cells (2.5-5x10⁶ cells/mL) were stimulated for 20-24 hours with 0.05% SAC (37°C, 5% CO₂). Subsequently, the supernatant was harvested by centrifugation (200g, 5min. room temperature), filtered (0.22 µm PVDF, Millipore, Ireland) to remove remaining cells and debris and stored at 70°C. The concentration of human IL-23 was determined using the Human IL-23 (p19/p40) Elisa Ready-SET-Go! kit (eBioscience, San Diego, USA). Typically, 20-40 ng/mL human IL-23 was induced. The IL-23 containing supernatant was then used as a source of IL-23 in the mouse splenocyte assay to determine whether the Nanbodies are able to neutralize native human IL-23. A limited panel of the biparatopic Nanobodies were tested and all of them were capable to neutralize the native hIL-23 as indicated in Table B-20.

**Table B-20: potencies in splenocyte assay with 19 pM native hIL-23**

| **Nanobody/mAB** | **hIL-23 IC50 (pM)** | **assay** |
|---|---|---|
| P23IL119A3-35GS-81G2 | 76 | Expt 1 |
| P23IL119A3-35GS-81G2 | 153 | Expt 1 |
| P23IL119A3(H37Y-M43K)-9GS-ALB1-9GS-81A12 | 9.3 | Expt 2 |
| P23IL119A3(H37Y-M43K)-9GS-ALB1-9GS-81A12 | 20 | Expt 3 |
| BM01 | 161 | Expt 2 |
| BM01 | 39 | Expt 3 |
| P23IL37D5-9GS-ALB1 | 60 | Expt 3 |
| P23IL37D5-9GS-ALB1-9GS-20B11 | 12 | Expt 3 |
| P23IL-124C4-9GS-ALB1-9GS-37D5 | 10 | Expt 3 |

### Example 34: ELISA Potency assay - Inhibition of IL-23 interaction with IL-23R by monovalent and biparatopic Nanobodies

96-well plates were coated overnight at 4°C with anti-Fc Nanobody (6 µg/ml) and subsequently blocked with Superblock T20 (PBS) blocking buffer for 30 min. Different concentrations of monovalent and Biparatopic Nanobodies were preincubated with 4 ng/ml hIL-23 for 30 min prior to a 30 min incubation with 300ng/ml hIL-23R-Fc, after which the mixtures were transferred to the coated wells. Bound hIL-23 was detected for 30 min at room temperature with a 1/750 dilution of Biotinylated anti-human interleukin-12 (IL-12) p40/70 monoclonal antibody (eBiosciences, San Diego, USA) in PBS containing 10% Superblock T20 (PBS) blocking buffer, followed by a 30 min incubation at room temperature with 1/5000 horseradish peroxidase labeled streptavidin in PBS containing 10% Superblock T20 (PBS) blocking buffer. Visualisation was performed with enhanced soluble 3,3',5,5'-tetramethylbenzidine (esTMB) colouring reagent, after which the coloring reaction was stopped with 1N HCl. The absorbance was measured at 450 nm. As shown in Figure 45, the p19+ (119A3; 37D5-ALB1) monovalent Nanobodies inhibit the interaction with IL-23R, while p19- (81A12, 81G2) monovalent Nanobodies do not. The biparatopic (230049, 23IL0041 and 23IL0038) Nanobodies have a significantly higher potency to inhibit the hIL-23 - HIL-23R interaction than the monovalent ones (Figure 46). Table B-21 presents the corresponding potencies (IC50) for the different Nanobodies,

**Table B-21: Potency of monovalent and biparatopic anti-IL23 (p19) Nanobodies in the inhibition ELISA assay (see SEQ ID NO.2490 for the amino acid sequence of 37D5)**

| **Nanobody** | **IC50 (pM)** |
|---|---|
| 119A3 (SEQ ID NO: 1898) | 1756 |
| 81A12 (SEQ ID NO: 1936) | - |
| 81G2(SEQ ID NO:1930) | - |
| 37D5-ALB1 | 37 |
| 23IL0049 (SEQ ID NO: 2544) | 28 |
| 23IL0041 (SEQ ID NO: 2541) | 40 |
| 23IL0038 (SEQ ID NO: 2539) | 32 |

### Example 35: Optimization of an acute in vivo mouse splenocyte model

An acute *in vivo* mouse splenocyte model was optimized and standardized to analyze the *in vivo* efficacy of IL-23p19-neutralizing Nanobodies

In this model, neutralization of hIL-23 occurs *in vivo* with an *ex vivo* readout of mIL-22 synthesis. In all experiments, C57BL/6 mice have been injected intraperitoneally (i.p.) with hIL-23. Exactly 31 hours after the only/first hIL-23 injection, the mice were bled and subsequently sacrificed. The spleens were removed and the splenocyte were isolated by homogenizing the spleens between frosted glass slides. The splenocytes were washed and resuspended in medium at a concentration of 106 cells/ml. The medium used was RPMI1640 supplemented with 10% FCS, 10U/ml penicillin, 100 µg/ml streptomycin, 1% non-essential amino acids, 1% sodium pyruvate, 2.5 mM HEPES and 0.00035% 2-mercapto ethanol The cells were seeded at 200,000 cells/200µL/well in a 96-well culture plate that was pre-coated with hamster anti-mouse CD3e antibody (5 µg/ml in PBS overnight at 4oC). For each spleen, 6 wells were seeded. The 96-well plates were then incubated for 24 hours in a humidified CO2 incubator. A sandwich ELISA kit for mouse IL-22 (RMF222CK, Antigenix) was used to measure the amount of mIL,-22 in each supernatant.

The results are shown in Figure 47, which is a graph showing the results obtained in this Example 35 for the inhibition of the mIL-22 synthesis in a mouse splenocyte assay upon administration of 3 microgram hIL-23 alone or in combination 23IL0036. Average mIL -22 synthesis is expressed as % change compared to group 4; and in Figure 48, which is a graph showing the results obtained in this Example 35 for the inhibition of the mIL-22 synthesis in a mouse splenocyte assay upon administration of 23IL0036 or IRR007 via different routes of administration. Average mIL-22 synthesis is expressed as % change compared to the group which received hIL-23 only.

In a first experiment, different groups of mice (n = 4) were injected i.p. with PBS at t=0h, 7h and 31h (group 1), 1x 3 µg hIL-23 at t=0h (group 2), 2x 3 µg hIL-23 at t=0h and 7h (group 3) or 3x 3 µg hIL-23 at t=0h, 7h and 23h (groups 4, 5 and 6). In groups 5 and 6, 0.2 mg of the anti-IL-23p19 Nanobody 23IL0036 was additionally administered i.p. 2 hours before and 29 hours after the first hIL-23 injection, respectively. The results showed clearly that 2 or 3 injections of hIL-23 are needed to get a considerable induction of mIL-22 synthesis (Figure 47). The results were normalized to the mean mIL-22 concentration of group 4, which was injected with 3x 3 µg hIL-23. Based on these results, we decided to induce mIL-22 synthesis in subsequent experiments with three injections of 3µg hIL-23 at t=0h, 7h and 23h. In addition, we have demonstrated with this experiment that neutralization of hIL-23 is required, during the *in vivo phase* and not *ex vivo* as 23IL0036 can inhibit mIL-22 synthesis when administered 2 hours before the first hIL-23 injection and not when administered 2 hours before the isolation of the splenocytes (Figure 47).

In a second experiment, different groups of mice (n = 4) were injected with PBS (group 1) or 3 x 3 µg hIL-23 (groups 2-6). In addition, groups 3-5 were injected with 0.2 mg 23IL0036, while group 6 was injected with the irrelevant Nanobody IRR007. The route of administration of the Nanobodies varied between the different groups. 23IL0036 and IRR007 were administered i.p. in groups 3 and 6, respectively, while 23IL0036 was injected intraveneously (i.v.) in group 4 and subcutaneously (s.c.) in group 5. The results were normalized to the mean mIL-22 concentration of group 2, which was injected with hTL-23 only. There was no difference observed in inhibition of mIL-22 synthesis between the different routes of administration (Figure 48). Due to the fact that hIL-23 is administered i.p. and we want to inhibit hIL-23 systemically and not in the intraperitoneal cavity, we decided to inject the Nanobodies s.c. in following experiments. The irrelevant Nanobody IRR007 had no effect on mIL-22 synthesis. In Figure 48, the graph shows the results obtained in this Example 35 for the inhibition of the mIL-22 synthesis in a mouse splenocyte assay upon administration of 23IL0036 or IRR007 via different routes of administration. Average mIL-22 synthesis is expressed as % change compared to the group which received hIL-23 only.

### Example 36: Dose range analysis of the wild type Nanobodies 23IL0038, 23IL0041 and 23IL0049 in an acute in vivo mouse splenocyte model

Efficacy of the wild type IL-23p19-neutralizing Nanobodies 23IL0038, 23IL004 and 23IL0049 was demonstrated in the optimized acute *in vivo* mouse splenocyte model described in Example 35.

In four separate experiments, C57BL/6 mice were assigned to seven groups, each group consisting of four individuals. All mice from groups 2-7 were 3x injected i.p. with 3 µg hIL-23 at 3 different time points (t = 0h, 7h and 23h). The animals from group 1 received PBS instead of h1L-23 at the same time points. In each of the four experiments, 5 different doses of a specific Nanobody or positive control antibody were injected s.c. 2 hours before the first hIL-23 injection. The doses were chosen such that in all experiments the molar excess to each injection of 3 µg hIL-23 was the same for each of the three Nanobodies and positive control antibody BM01 (82-fold, 16-fold, 3.2-fold, 0.64-fold and 0.128-fold excess).

For each experiment, the results were normalized to the mean mIL-22 concentration of group 2, which was injected with hIL-23 only, and the normalized results are represented in Figures 49 A to D, , which are graphs showing the results obtained in this Example 36 for the inhibition of the mIL-22 synthesis in a mouse splenocyte assay upon administration of Nanobodies (Fig. 49A: 23IL0049; Fig.49B: 23IL0041; Fig.49C: 23IL0038) or positive control antibody (Fig. 49D: BM01). Average mIL-22 synthesis is expressed as % change compared to the group which received hIL-23 only.

All three Nanobodies were capable to neutralize hIL-23 *in vivo.* The Nanobodies 23IL0049 and 23IL0038 significantly blocked the synthesis of mIL-22 at the four highest doses. The lowest dose of these Nanobodies (0.128-fold excess) still had an effect but could not completely block mIL-22 production. For 23IL0041, the highest three doses blocked the synthesis of mIL-22 significantly, while inhibition in the two lowest dose groups was not complete. The efficacy of the Nanobodies was comparable with the efficacy of BM01.

The hIL-23 concentration was measured in the final bleeding samples of the groups which were injected with hIL-23 only. The mean serum concentration of hIL-23, 8 hours after the last injection of 3 µg hIL-23, is 22.5 ng/ml.

### Example 37: Activity of Nanobodies 23IL0050 and 23IL0054 in an acute in vivo. mouse splenocyte model

The monovalent Nanobody 231L0050 and the biparatopic Nanobody 23IL0054 have been tested in the acute *in vivo* mouse splenocyte model described in Example 35. C57BL/6 mice, seven groups with each four mice, were stimulated with 3 µg hIL-23 on t = 0h, 7h and 23h. The mice from group 1 received PBS instead of hIL-23 at the same time points. Both Nanobodies were injected s.c. 24 hours before the first hIL-23 injection. Three doses were analyzed and for each dose level the same molar excess with respect to each injection of 3 µg hIL-23 was used (16-fold, 3.2-fold or 0.64-fold excess).

The results are shown in Figure 50, which is a graph showing the results obtained in this Example 37 for the inhibition of the mIL-22 synthesis in a mouse splenocyte assay upon administration of P23IL0054 and P23IL0050. The results were normalized to the mean mIL; 22 concentration of group 2, which was injected with hIL-23 only.

Both Nanobodíes blocked synthesis of mIL-22 completely at the highest two dose levels. At the lowest dose, there was a clear difference between the monovalent and the biparatopic Nanobody. While the biparatopic 23IL0054 still inhibited mIL-22 synthesis at a 0.64-fold molar excess, mIL-22 concentrations were back to basal levels at the same molar dose of 23IL0050.

### Example 38: Humanization of P23IL119A3, 81A12, 81G2, 37D5, 20B11 and 124C4

The amino acid sequence of P23IL119A3 was blasted to the human germline V_{H} sequence database using an in-house sequence query/alignment tool (see Figure 51A). The human germlines VH3-23 (also called DP-47) and JH5 showed the closest related sequence. 10 amino acid residues (indicated in black boxes in 119A3v16 in Figure 51A) were substituted for humanization purposes and 1 (H37Y) for stability purposes to make P23IL119A3v16 (SEQ ID NO: 2578).

In the humanization process of P23IL1 19A3, eighteen P23IL119A3 versions (P23IL119A3-BASIC and P23IL119A3v1 to v17) were constructed. P23IL119A3-BASIC contains 5 substitutions: A14P, H37Y, V78L, K84R and Q108L. In addition to these changes, additional substitutions have been introduced in the v1-v17 versions: V5L, M43K, Q44G, A49S, N58Y, A74S, Q75K, K76N, N82bS and Q93A. They were assembled from oligonucleotides using a PCR overlap extension method. The constructs were expressed in *E.coli* and purified by IMAC and desalting.

All variants were were evaluated for their hIL-23binding capacity by surface plasmon resonance and for their neutralizing activity in alpha-screen and in the splenocyte assay as described in Examples 27 and 35. Also thermal stability of the variants was tested in a thermal shift assay using the Lightcylcer (Roche). In this assay the Nanobodies variants are incubated at different pH values in the presence of sypro orange and a temperature gradient is applied. When the Nanobodies start denaturing, sypro orange binds and the measured fluorescence increases suddenly, as such a melting temperature can be determined for a certain pH. The analysis occurred in two rounds, in a first round single mutations upon the basic variants were evaluated, and based upond these results, new variants were made with combined mutations. Results are summarized in Table B-22.

Binding kinetics of the second round variants are in depth analysed by Surface Plasmon Resonance (Biacore 3000). Human soluble IL-23 was covalently bound to CM5 sensor chips surface via amine coupling using EDC/NHS for activation and HCl for deactivation. Nanobody binding was assessed at one concentration (100 nM). Each Nanobody was injected for 4 minutes at a flow rate of 45 µl/min to allow binding to chip-bound antigen. Next, binding buffer without Nanobody was sent over the chip at the same flow rate to allow spontaneous dissociation of bound Nanobody. From the sensorgrams obtained for the different Nanobodies k_{off}-values (k_{d}) were calculated and are indicated in Table B-23. Association rate constants (kₒₙ or kₐ), and hence also K_{D} values, were only indicative as only one concentration of Nanobody was used to fit the bindingmodel. Results are summarized in Table B-23

**Table B-22: results of analysis of round 1 humanisation for P23IL119A3**

| **Nanobody** | **Biacore offrate (s-1)kd1 125-155s** | **Biacore offrate (s-1) kd2 200-1000s** | **IC 50 (nM-1) α-screen hIL-23 hIL-23R** | **IC 50 (nM) α-screen cIL-23 hIL-23R** | **IC 50 splenocyte assay hIL-23 (nM)** | **IC50 splenocyte assay cIL-23 (nM)** | **Stability (Tm in °C at pH 7,5)** |
|---|---|---|---|---|---|---|---|
| P23IL119A3 | 4.1^{E}-03 | 1.4^{E}-03 | 1,21 | 0,50 | 16,8 | 60,5 | 70,24 |
| P23IL119A3-BASIC | 5.2^{E}-03 | 2.0^{E}-03 | 1,47 | 0,73 | 37,3 | 66,6 | 56,96 |
| P23IL119A3v1 | 5.6"-03 | 2.0^{E}-03 | 0,91 | 0,54 | 33,0 | 37,4 | 61,11 |
| P23IL119A3v2 | 5.4^{E}-03 | 2.1^{E}-03 | 1,48 | 0,88 | 42,1 | 27,4 | 56,55 |
| P23IL119A3v3 | 5.0^{E}-03 | 2.1^{E}-03 | 9,07 | 4,40 | - | - | - |
| P23IL119A3v4 | 5.2^{E}-03 | 2.0^{E}-03 | 7,71 | 0,66 | - | - | 63,60 |
| P23IL119A3v5 | 5.4^{E}-03 | 1.9^{E}-03 | 2,23 | 3,79 | 35,9 | 71,7 | 56,13 |
| P23IL119A3v6 | 5.4^{E}-03 | 1.7^{E}-03 | 1,51 | 0,90 | 33.7 | 66,9 | 60,28 |
| P23IL119A3v7 | 1.3^{E}-02 | 7.8^{E}-03* | 12,10 | 12,84 | 79,3 | 190 | 65,28 |
| P23IL119A3v8 | 5.9^{E}-03 | 2.1^{E}-03 | 2,47 | 0,55 | 44,7 | 74,3 | 59,06 |
| P23IL119A3v9 | 1.7^{E}-02 | 8.9^{E}-03* | 8,22 | 4,81 | 191,0 | 167 | 65,70 |

**Table B-23: results of analysis of round 2 humanisation for P23IL119A3 (nd= not determined)**

| **Variant** | **Biacore kon (Ms-1) hIL-23** | **Biacore koff (s-1) hIL-23** | **Biacore Kd (M) hIL-23** | **IC 50 (nM)** α**-screen hIL-23** | **IC 50 (nM)** α-**screen cIL-23** | **IC 50 splenocyte assay hIL-23 (nM)** | **IC 50 splenocyte assay cIL-23 (nM)** | **Stability (Tm in °C at pH 7,5)** |
|---|---|---|---|---|---|---|---|---|
| P231L119A3 | 3,7E+06 | 4,8E-03 | 1,4E-09 | 0,76 | 0,42 | 16,8 | 60,5 | 70,65 |
| P23IL 119A3v 10 | 3.5E+06 | 5.5E-03 | 1.6E-09 | 5,90 | 2,75 | 27,58 | 85,90 | 56,92 |
| P23IL 119A3v11 | 2.1E+06 | 1.5E-02 | 7.2E-09 | 2,47 | 3,93 | 48,60 | 220,30 | nd |
| P23IL 119A3v12 | nd | nd | nd | nd | nd | nd | nd | nd |
| P23IL H9A3v13 | 2.6E+06 | 1.5E-02 | 5.8E-09 | 3,07 | 2,09 | 110,40 | 308,80 | 62,33 |
| P23IL 119A3v14 | 1.7E+06 | 1.7E-02 | 1.0E-08 | 4,42 | 1,26 | nd | 349,30 | nd |
| P23IL 119A3v15 | - | 4.8E-03 -1.9E-03 | - | nd | nd | nd | nd | nd |
| P23IL 119A3v16 | - | 55E-03 - 1.9E-03 | - | 1,24 | 0,70 | 45,12 | 82,60 | 63,58 |
| P23IL 119A3v17 | 2.7E+06 | 5.0E-03 | 1.9E-09 | 2,17 | 0,21 | 14,2 | 56,50 | nd |

The amino acid sequence of P23IL81A12 was blasted to the human germline V_{H} sequence database using an in-house sequence query/alignment tool (Figure 51B). Human germline VH3-23 (also called DP-47) and JH5 showed the closest related sequence. 8 amino acid residues (indicated in black boxes in 81A12v4 in Figure 51B) were substituted for humanization purposes to make P23IL81A12v4 (SEQ ID NO: 2584).

In the humanization process of P23IL81A12, five T23IL81A12 versions (P23IL81A12-BASIC and P23IL8LA12v1 to v4) were constructed. P23IL81A12-BASIC contains 4 substitutions: A14P, V78L, K84R and Q108L. In addition to these changes, additional substitutions have been introduced in the v1-v4 versions: V5L, E44G, A49S and A74S. They were assembled from oligonucleotides using a PCR overlap extension method. The constructs were expressed in *E.coli* and purified by IMAC and desalting.

All variants were were evaluated for their hIL-23binding capacity by surface plasmon resonance and also thermal stability of the variants was tested. The analysis occurred in two rounds, in a first round single mutations upon the basic variants were evaluated of which results are summarized in Table B-24.

**Table B-24: results of round 1 humanization of P231L81A12**

| **Nanobody** | **Biacore offrate (s-1) hIL-23** | **Stability (Tm in °C at pH 7,5)** |
|---|---|---|
| P23IL81A12 | 2.2E-04 | 67,34 |
| P 23IL81A12-BASIC | 2.4E-04 | 66,09 |
| P 23IL81A12vl | 2.4E-04 | 65,26 |
| P 23IL81A12v2 | 2.1E-04 | 59,45 |
| P 23IL81A12v3 | 2.3E-04 | 66,09 |

A final variant with all mutations: P23IL 81A12v4 was prepared and tested. Results of the analysis of this final variant are shown in Table B-25. This molecule is 91.95% human in the framework regions (according to the Kabat numbering)

**Table B-25: results of round 2 humanisation of P23IL81A12**

| **Nanobody** | **Biacore kon (Ms-1) hIL-23** | **Biacore koff (s-1) hIL-23** | **Biacore Kd (M) hIL-23** | **Stability (Tm in °C at pH 7,5)** |
|---|---|---|---|---|
| P23IL81A12 | 5,60E+05 | 2,10E-04 | 3,80E-1,0 | 67,32 |
| P23IL81A12v4 | 6,00E+05 | 2,00E-04 | 3,30E-10 | 56,55 |

The amino acid sequence of P23IL81 G2 was blasted to the human germline V_{H} sequence database using an in-house sequence query/alignment tool (Figure 51C). Human germline VH3-23 (also called DP-47) and JH5 showed the closest related sequence. 11 amino acid residues (indicated in black boxes in 81G2v 11 in Figure 51 C) were substituted for humanization purposes to make P23IL81G2v11 (SEQ ID NO: 2597).

In the humanization process of P23IL81 G2, twelve P23IL81 G2 versions (P23IL81G2basic and P23IL81 G2v1 to v11) were constructed. P23IL81A12basic contains 2 substitutions: E44G and Q108L. In addition to these changes, additional substitutions have been introduced in the v1-v11 versions: V5L, I22A, A49S, G60A, F62S, A68T, A74S, T78L, W79Y K83R and T105Q. They were assembled from oligonucleotides using a PCR overlap extension method. The constructs were expressed in *E.coli* and purified by IMAC and desalting. All variants were were evaluated for their hIL-23 binding capacity by surface plasmon resonance and also thermal stability of the variants was tested. The analysis occurred in two rounds, in a first round single mutations upon the basic variants were evaluated of which results are summarized in Table B-26.

**Table B-26: results of round 1 humanisation of P23IL81G2**

| **Nanobody** | **Biacore koff (s-1) hIL-23** | **Stability (Tm in °C at pH 7,5)** |
|---|---|---|
| P23IL81G2 | 3.4E-04 | 64,43 |
| P23IL81G2-BASIC | 3.2E-04 | 63,60 |
| P23IL81G2v1 | 3.4E-04 | 62,36 |
| P23IL81G2v2 | 2.4E-04 | 58,62 |
| P23IL81G2v3 | 4.2E-04 | 66,51 |
| P23IL81G2v4 | 6.5E-04 | 65,26 |
| P23IL81G2v5 | 3.1E-04 | 65,68 |
| P23IL81G2v6 | 3.2E-04 | 64,02 |
| P23IL81G2v7 | 2.8E-04 | 61,13 |
| P23IL81G2v8 | 3.7E-04 | 64,45 |
| P23IL81G2v9 | 3.4E-04 | 65,28 |
| P23IL81G2v10 | 3.2E-04 | 64,04 |

A final variant with all mutations except the ones from V3 and V4: P23IL 81 G2v11 was prepared and tested. The result of the analysis of this final variant is shown in Table B-27. This molecule is 92.10 % human in the framework regions (according to the Kabat numbering)

**Table B-27: results of round 2 humanisation of P23IL81G2**

| **Nanobody** | **Biacore kon (Ms-1) hIL-23** | **Biacore koff (s-1) hIL-23** | **Biacore Kd (M) eBiosc hIL-23** | **Stability (Tm in °C at pH 7,5)** |
|---|---|---|---|---|
| P23IL81G2 | 5,20E+05 | 3,90E-04 | 7,50E-10 | 64,41 |
| P231L81G2v11 | 6,10E+05 | 3, 10E-04 | 5,10E-10 | 62,75 |

The amino acid sequence of P23IL37D5 was blasted to the human germine V_{H} sequence database using an in-house sequence query/alignment tool (Figure 51D). Human germline VH3-23 (also called DP-47) and JH5 showed the closest related sequence. 8 amino acid residues (indicated in yellow and blue in 37D5v16 in Figure 51D) can be substituted for humanization purposes to make P23IL37B5v16 (SEQ ID NO: 2601). In this case the basic variant was made, containing mutations V5L, E44G, K84R and Q108L. Then a mini-library of the basic mutant with 16 (2⁴) permutations at positions 74, 75, 76 and 78 was constructed. The library was transformed in TG1 and individual colonies were picked and grown in a 96 well plate. Periplasmic extracts were prepared and screened for off-rates and in the blocking alpha-screen. Also sequences of these clones were determined.

The variants with no loss or minor loss in potency and affinity were produced and purified by IMAC and desalting and are in depth analysed. Results are shown in Table B-28. The amino acid residues that are changed for humanisation purposes are indicated with black boxes in the variants in Figure 51D).

**Table B-28 : results of analysis of humanization for P23IL37D5**

| **Nanobody** | **IC 50 (pM) comp α-screen hIL-23** | **Stability (Tm in °C at pH 7,5)** | **IC₅₀ potency assay (pM)** |
|---|---|---|---|
| P23IL37D5 | 176 | 69,02 | 38,44 |
| P23IL37D5v1 | 128 | 66,53 | 36,36 |
| P23IL37D5v3 | 79 | 68,19 | 60,15 |
| P23IL3 71Dv6 | 66 | 68,60 | 71,95 |
| P23IL37D5v16 | 44 | 71,93 | 88,76 |
| P23IL37D5v17 | 93 | 63,62 | 185,51 20 |

The amino acid sequence of 23IL-124C4 was blasted to the human germline V_{H} sequence database using an in-house sequence query/alignment tool (Figure 51E). Human germline VH3-23 (also called DP-47) and JH5 showed the closest related sequence. Six amino acid residues can be substituted for humanization purposes. The basic variant P23IL-124C4-BASIC was made and contains mutations E44G and V78L. In addition to these changes, additional substitutions have been introduced in the v1-v3 versions: S71R, A74S and K105Q. They were assembled from oligonucleotides using a PCR overlap extension method. The constructs were expressed in *E.coli* and purified by IMAC and desalting.

### Example 39: Construction and production of different formats of the humanized Nanobodies

Biparatopic anti-p19 Nanobodies were constructed with three different types of HLE, using the building blocks P23IL119A3v16 or P23IL119A3v10, P23IL81G2v11 or P23IL81A12v4. Some preferred, but non-limiting examples of the different formatted humanized Nanobodies with corresponding Nanobody IDs are represented in Figure 32.

### A) HLE1 : Anti Human Serum Albumin Nanobody

The trivalent humanized biparatopic molecules (P23IL0057 and P23IL0058/0064) have two building blocks corresponding to humanized anti-p19 Nanobodies with in the middle a third humanized Nanobody building block corresponding to an anti-Human Serum Albumin Nanobody building block. The individual building blocks are fused by a 9 Cly/Ser (GGGGSGGGS; SEQ ID NO: 792) linker. P23IL0057 consists of P23IL119A3v10 and P231L84A12, whereas P23IL0058 consists P23IL119A3v16 and P23IL84AI2. The constructs were transformed into TG1. After expression, these Nanobodies were purified from the periplasmic extract by capturing on Mabselect Extra and elution with 100mM Glycine pH 2.5 followed by immediate neutralization with 1M TrisHCl pH7.5. The sample was polished by cation exchange using a Mono S HR5/50 column (GE healthcare) and size exclusion on Superdex 75 10/300 GL (GE healthcare).

For the production of large batches of these Nanobodies, the constructs were cloned in pAX89 (a modified pPICZalphaA vector, Invitrogen, Carlsbad, CA) and transformed in *Pichia pastoris* strain X33. Nanobody P23IL0064 was purified starting from the clarified fermentation broth of the recombinant *Pichia pastoris,* expressing the Nanobody Nanobody. The clarified fermentation broth was made particle free via TFF (Hydrosart 0.22 µm cassette, Sartorius), followed by a concentration and diafiltration step to PBS using TFF (Hydrosart 10kDa MWCO-cassette Sartorius). The Nanobody was captured on MabGapture A (Poros followed by a buffer switch to 1/10 PBS via SEC (Sephadex G25 fine, GE healthcare) and a polish step on Poros 50HS (Poros). After treatment with 50 mM Octylβ-D-glucopyranoside (Sigma) for LPS-removal, the Nanobody was sized on a Superdex 75 pg (GE Healthcacare).

### B) HLE2 : HSA:

The HSA-fused humanized bipartopic Nanobody (P23IL0065) consists of two building blocks corresponding to humanized anti-p19 Nanobodies P23IL119A3v16 and P23IL81G2v11 fused by a 35 Gly/Ser linker. HSA was fused directly to the C-terminus of this construct. This occurred by ligating the coding sequence for P23IL119A3v16-35GS-81G2v11 into the pAX99 vector (which is a pPICZalphaA vector containing the coding sequence for full length human serum albumin). The plasmid was transformed into *Pichia pastoris* strain X33. The Nanobodywas purified starting from the clarified fermentation broth of the recombinant *Pichia pastoris,* expressing the Nanobody. The clarified fermentation broth was made particle free via TFF (Hydrosart 0.22 µm cassette, Sartorius), followed by a concentration and diafiltration step to PBS using TFF (PESU-AL cassette Sartorius). The protein of interest was captured on Blue Sepharose 6FF (GE Healthcare, Uppsala, Sweden) and further purified via polishing steps using Paros50HQ and Superdex200 XK26/60.

### C) HLE3:PEG:

The pegylated humanized biparatopic Nanobody P23IL0062/0063, consists of two building blocks corresponding to humanized anti-p19 Nanobodies P23IL119A3v16 and P23IL81G2v11 fused by a 35 Gly/Ser linker. A GGGC sequence was added at the C-terminus of the construct. This occurred by ligating the coding sequence of P23IL119A3v16-35GS-81G2v11 in the pAX97 vector (which is a pPICZalphaA vector containing this GGGC sequence) resulting in P23IL0063 or in the pAX101 vector resulting in P23IL0062. The pAX97 construct was transformed into *Pichia pastoris* strain X33, and the pAX101 contstruct in TG1. Purification and pegylation was carried out as follows: The Nanobody P23IL0063 was produced in the *Pichia postoris.* The Nanobody was purified starting from the clarified fermentation broth by a capture step on MabCaptureA (Poros), and eluted using 100mM glycine pH 2.7. After a buffer switch to 1/10 PBS and the protein of interest was further polished on Poros 50HS (Poros), followed by a reduction with10mM DTT. The excess of DTT was be removed by SEC (Superdex75pg, GE Healthcare), and the reduced Nanobody was coupled to linear-mPEG40 (40 kDa methoxy poly (ethylene glycol) maleimido - propionamice, Dow Pharma). The PEGylated Nanobody was further purified on MacroCap SP (GE healtheare) to remove the excess of non-reacting PEG40. Finally, the PEGylated Nanobody was treated with 50 mM Octylβ-D-glucopyranoside (Sigma) for LPS-removal, followed by size exclusion chromatography (Superdex75pg, GE Healthcare).

### Example 40: Characterization of the formatted humanized anti-p19 Nanobodies,

### A) Potency in mouse splenocyte assays

The humanized HLE Nanobodies were tested and compared to the WT HLE Nanobodi.es in the splenocyte assay using hIL-23 and cynomolgus IL-23 as described in Example 27. Results are shown in Tables B-29 and B-30.

### B) Potency in BAF3 proliferation assay

The humanised HLE Nanobodies were tested and compared to the WT HLE Nanobodies in the BAF3 proliferation assay using 76 pM hIL-23 as described in Example 27. Results are shown in Table B-31.

**Table B-29: potency of HLE humanized biparatopic anti-p19 Nanobodies in the splenocyte assay with 19 pM hIL-23 and 3.8 pM cynomolgus IL-23 (cIL-23)**

| **Nanobody/mAB** | **hIL-23 IC50 (pM)** | **cIL-23 IC₅₀ (pM)** |
|---|---|---|
| P23IL119A3V10-9GS-ALB-9GS-81A12V4 | 5.1 | 3.5 |
| P23IL119A3V16-9GS-ALB-9GS-81A12V4 | 4.6 | 6.7 |

**Table B-30: potency of HLE humanized biparatopic anti-p19 Nanobodies compared to their non-humanized equivalents in the splenocyte assay with 19 pM hIL-23 and 3,8 pM cynomolgus IL-23 (cIL-23).**

| **Nanobody/mAB** | **hIL-23 IC₅₀ (pM)** | **cIL-23 IC₅₀ (pM)** |
|---|---|---|
| P23IL119A3V16-9GS-ALB8-9GS-81A12V4 | 3.6 | 5.4 |
| P23IL119A3(H37Y-M43K)-9GS-ALB1-9GS-81A12 | 3.1 | 4.3 |
| P23IL119A3V16-35GS-81G2V11-HSA | 4.6 | 6.7 |
| P23I119A3-35GS-81G2-HSA | 5.4 | 8.1 |
| P23IL119A3V16-35GS-81G2V11-GGGC-PEG40 linear | 2.7 | 13.1 |
| P23IL119A3-35GS-81G2-GGGC-PEG40 linear | 7.1 | 4.7 |
| BM01 | 3.2 | 20.3 |

**Table B-31: potency of HLE humanized biparatopic anti-p19 Nanobodies versus the non-humanized equivalents in the BA/F3 proliferation assay with 76 pM hIL-23**

| **Nanobody/mAB** | **IC 50 (pM)** |
|---|---|
| P23IL119A3V16-9GS-ALB8-9GS-81A12V4 | 49 |
| P23IL119A3(H37Y-M43K)-9GS-ALB1-9GS-81A12 | 43 |
| P23IL119A3V16-35GS-81G2V11-HSA | 125 |
| P23IL119A3-35GS-81G2-HSA | 66 |
| P23IL119A3V16-35GS-81G2V11-GGGC-PEG40 linear | 71 |
| P23IL119A3-35GS-81 G2-GGGC-PEG40 linear | 71 |
| BM01 | 58 |

### C) ELISA Potency assays: Inhibition of IL-23 interaction with IL-23R by formatted humanized Nanobodies

96-well plates were coated overnight at 4°C with anti-Fc Nanobody (6 µg/ml) and subsequently blocked with Superblock T20 (PBS) blocking buffer for 30 min. Different concentrations of formatted humanized Nanobodies were preincubated with 4 ng/ml hIL-23 for 30 min prior to a 30 min incubation with 300ng/ml hIL-23R-Fc, after which the mixtures were transferred to the coated wells. Bound hIL-23 was detected for 30 min at room temperature with a 1/750 dilution of Biotinylated anti-human interleukin-12 (IL-12) p40/70 monoclonal antibody (eBiosciences, San Diego, USA) in PBS containing 10% Superblock T20 (PBS) blocking buffer, followed by a 30 min incubation at room temperature with 1/5000 horseradish peroxidase labeled streptavidin in PBS containing 10% Superblock T20 (PBS) blocking buffer. Visualisation was performed with enhanced soluble 3,3',5,5'-tetramethylbenzidine (esTMB) colouring reagent, after which the coloring reaction was stopped with IN HCl. The absorbance was measured at 450 nm. Figure 52 shows the inhibition of the IL-23 interaction with IL-23R by formatted humanized Nanobodies (231L0064, 231L0065 and 23IL00063) and Table B-32 presents the corresponding IC50 values.

In this assay, also cross-reactivity of the formatted humanized Nanobodies with marmoset IL-23 was illustrated.

**Table B-32: Potency of formatted humanized anti-IL23(p19) Nanobodies in the inhibition ELISA assay**

| **Nanobody** | **IC50 (pM)** |
|---|---|
| 23IL0064 | 23 |
| 231L0065 | 38 |
| 231L0063 | 25 |

### D) Binding of 231L0064 to human serum albumin

96-well microtiter plates were coated overnight at 4°C with recombinant human serum albumin (HSA, 125 µg/ml in PBS). Following aspiration of the wells and 30 min blocking with Superblock T20 (PBS) blocking buffer, wells were incubated for 1h with a dilution series of 23TL0064. Bound 23IL0064 was detected with a bivalent, horseradish peroxidase (HRP) conjugated Nanobody. After visualization with 1/3 diluted enhanced soluble 3.3',5,5'-tetramethythenzidine (esTMB) colouring reagent, the coloring reaction was stopped with 1N HCl and absorbance was measured at 450 nm (Figure 53).

### Example 41: Activity of humanized Nanobodies 23IL0063, 23IL0064 and 23IL0065 in an acute in vivo mouse splenocyte model

Efficacy of the humanized IL-23p19-neutralizing Nanobodies 23IL0063, 23IL0064 and 23IL0065 was demonstrated in the acute *in vivo* mouse splenocyte model described in Example 35.

In two separate experiments, C57BL/6 mice were assigned to eight groups, each group consisting of four individuals. All mice from groups 2-8 were three times injected IP with 3 µg hIL-23 at three different time points (t = 0h, 7h and 23h). The animals from group 1 received PBS instead of hIL-23 at the same time points. The Nanobodies or positive control antibody were injected s.c. 24 hours before the first hIL-23 injection. Spread over the two experiments, Nanobodies 23IL0063, 23IL0064 and 23IL0065 were administered at three different doses. The doses were chosen such that the molar excess to each injection of 3 µg hIL-23 was the same for each of the three Nanobodies (16-fold, 3.2,-fold or 0.64-fold excess). As positive control, BM01 was used.

The results were normalized to the mean mIL-22 concentration of group 2, which was injected with hIL-23 only, and are represented in Figures 54 and 55. All three Nanobodies were capable of neutralizing hIL-23 and significantly blocked the synthesis of mIL-22 at the two highest doses. The lowest dose of the Nanobodies (0.64-fold excess) still had an effect but could not completely block mIL-22 production. The efficacy of the Nanobodies was comparable with the efficacy of BM01 .

### Example 42: Pharmacokineties of biparatopic wild type Nanobodies in mouse

To determine the pharmacokinetic properties of some representative biparatopic wild-type Nanobodies, seventy-two male Balb/c mice were assigned to six groups, each group consisting of twelve mice. The mice were approximately 12 weeks old with an initial body weight between 19.8 and 24.9 grams.

Nanobodies 23IL0049, 23IL0041 and 23IL0038 were tested. Animals were dosed intravenously or subcutaneously in a single administration on test day 1 as described in Table B-33. Serum samples were taken for determination of the Nanobody levels and antibody analysis on timepoints described in Table B-34.

**Table B-33: Study design of the mouse PK study with wild type Nanobodies (23RL0049,23IL0041 and 23IL0038)**

| **Study design / Group size / Dose levels** | | | | | | |
|---|---|---|---|---|---|---|
| **Group** | **Test item** | **Route of admini-stration** | **Dose [µg/animal]** | **Administriation volume [µL/animal]** | **Number and sex of animals** | **Animal no.** |
| **1** | 119A3-ALB1-81A12 (23IL0049) | i.v. | 140 | 200 | 12 m | 1-12 |
| **2** | 119A3-ALB1-81A12(23IL0049) | s.c. | 140 | 200 | 12 m | 13-24 |
| **3** | 119A3-81G2-HSA (23IL0041) | i.v. | 100 | 200 | 12m | 25-36 |
| **4** | 119A3-81G2-HSA (23IL0041) | s.c. | 100 | 200 | 12 m | 37-48 |
| **5** | 119A3-81G2-PEG40 (231L0038) | i.v. | 100 | 200 | 12 m | 49-60 |
| **6** | 119A3-81G2-PEG40 (231L0038) | s.c. | 100 | 200 | 12 m | 61-72 |

**Table B-34: Timepoints of blood sampling in the mouse PK study with wild type Nanobodies (23IL0049, 23IL0041 and 23IL0038)**

| **Test day** | **Time-point of blood sampling** | **Group** | **Serum volume [µL/animal]** |
|---|---|---|---|
| -1 | predose | 1 - 6 (12 animals/group of 12 animals) | 75 |
| 1 | 5 min after administration | 1, 3, 5 (4 animals/group of 12 animals) | 100 |
| | 30 mm after administration | 2, 4, 6 (4 animals/group of 12 animals) | |
| | 3 h after administration | 1 - 6 (4 animals/group of 12 animals) | |
| | 8 h after administration | 1 - 6 (4 animals/group of 12 animals) | |
| 2 | 24 h after administration | 1 - 6 (4 animals/group of 12 animals) | 100 |
| 3 | 48 h after administration | 1 - 6 (4 animals/group of 12 animals) | 100 |
| 5 | 4 days after administration | 1 - 6 (4 animals/group of 12 animals) | 100 |
| 7 | 6 days after administration | 1 - 6 (4 animals/group of 12 animals) | 100 |
| 9 | 8 days after administration | 1 - 6 (4 animals/group of 12 animals) | as much as possible |
| 11 | 10 days after administration | 1 - 6 (4 animals/group of 12 animals) | as much as possible |
| 13 | 12 days after administration | 1 - 6 (4 animals/group of 12 animals) | as much as possible |

Concentrations of Nanobodies 23IL0049 (119A3-Alb1-81A12; P1aWT), 23IL0041 (119A3-81G2-HSA; P1bWT) and 23IL0038 (119A3-81G2-PEG40; P1cWT) were determined in serum by three different immunoassays.

Concentrations of Nanobody 23IL0049 were determined in mouse serum as follows: 96-well microtiter plates (Maxisorp, Nunc, Wiesbaden, Germany) were coated overnight at 4°C with 100 µL/well neutravidin (2 µg/mL in PBS, Pierce, Rockford, IL). Wells were aspirated and blocked for 30 minutes at room temperature with SuperBlock®T20 PBS (Pierce, Rockford, IL) (300 µL/well). After 3 washing steps with PBS-0.05% Tween20, 100 µL/well biotinylated hIL23 (0.2 µg/mL in PBS-0.1% casein-0.05% Tween20; Carrier-Free Recombinant Human IL-23 (p19/p40) eBioScience, San Diego, USA) was captured by incubating for 1 hour at R.T while shaking at 600 rpm. Biotinylated hIL23 was prepared with EZ-Link® Sulfo-NHS-LC-Biotin (Pierce, Rockford, IL, USA) and free biotin was removed using Zeba desalt spin columns (Pierce, Rockford, IL, USA). After this incubation step, wells were washed 3 times with PBS-0.05% Tween20. All preparations for standards, QC samples and test samples were done in a non-coated (polypropylene) plate. Predilutions of standards and QC samples were prepared in 100% mouse serum. To prepare standards and QC samples, a 1/10 dilution of the predilutions was made in PBS-0.1% casein-0.05% Tween20 (final concentration of mouse serum is 10%). Test samples were diluted 1/10 in PBS-0.1% casein-0.05% Tween20 and where needed further diluted in PBS-0.1% casein-0.05% Tween20 with 10% Mouse Serum (final concentration of mouse serum is 10%). Standards, QC samples and test samples were transferred to the coated plate and incubated for 2 hours at RT while shaking at 600 rpm. After 3 washing steps with PBS-0.05% Tween20, the plates were incubated with 100 µL/well of a rabbit anti-Nanobody polyclonal antibody (1/5000 diluted in PBS-0.1% casein-0.05% Tween20) for 1 hour at RT while shaking at 600 rpm. After 3 washing steps with PBS-0.05% Tween20, the plates were incubated with 100 µL/well horseradish peroxidase (HRP) labeled polyclonal goat anti-rabbit (1/5000 diluted in PBS-0.1% casein-0.05% Tween20, DakoCytomation, Glostrup, Denmark) for 1 hour at RT while shaking at 600 rpm. Visualization was performed by incubation with 100 µL/well enhanced soluble 3,3',5,5'-tetramethylbenzidine (esTMB, SDT, Brussels, Belgium). After 10 min, the colouring reaction was stopped with 100 µL/well 1N HCl. The absorbance was determined at 450 nm after a 10 seconds shake in the Tecan ELISA reader, and corrected for background absorbance at 620 nm. Concentration in each sample was determined based on a sigmoid standard curve.

Concentrations of Nanobody 23IL0041 were determined in mouse serum as follows: 96-well microtiter plates (Maxisorp, Nunc, Wiesbaden, Germany) were coated overnight at 4°C with 100 µL/well neutravidin (2 µg/mL in PBS, Pierce, Rockford, IL). Wells were aspirated and blocked for 30 minutes at room temperature with SuperBlock®T20 PBS (Pierce, Rockford, IL) (300 µL/well). After 3 washing steps with PBS-0.05% Tween20, 100 µL/well biotinylated hIL23 (0.2 µg/mL in PBS-0.1% casein-0.05% Tween20; Carrier-Free Recombinant Human IL-23 (p19/p40) eBioScience, San Diego, USA) was captured by incubating for 1 hour at RT while shaking at 600 rpm. After this incubation step, wells were washed 3 times with PBS-0.05% Tween20. All preparations for standards, QC samples and test samples were done in a non-coated (polypropylene) plate. Predilutions of standards and QC samples were prepared in 100% mouse serum. To prepare standards and QC samples, a 1/10 dilution of the predilutions was made in PBS-0.1% casein-0.05% Tween20 (final concentration of mouse serum is 10%). Test samples were diluted 1/10 in PBS-0.1% casein-0.05% Tween20 and where needed further diluted in PBS-0.1% casein-0.05% Twesn20 with 10% Mouse Serum (final concentration of mouse serum is 10%). Standards, QC samples and test samples were transferred to the coated plate and incubated for 2 hours at RT while shaking at 600 rpm. After 3 washing steps with PBS-0.05% Tween20, the plates were incubated with 100 µL/well of a horseradish peroxidise (HRP) labeled polyclonal goat anti-human albumin (1/5000 diluted in PBS-0.1% casein-0.05% Tween20, Nordic Immunology, Tilburg, The Netherlands) for 1 hour at RT while shaking at 600 rpm. Visualization was performed by incubation with 100 µL/well enhanced soluble 3,3',5,5'-tetramethylbenzidine (esTMB, SDT, Brussels, Belgium), After 10 min, the colouring reaction was stopped with 100 µL/well 1N HCl. The absorbance was determined at 450 nm after a 10 seconds shake in the Tecan ELISA reader, and corrected for background absorbance at 620 nm. Concentration in each sample was determined based on a sigmoid standard curve.

Concentrations of Nanobody 23IL0038 were determined in mouse serum as follows: 96-well microtiter plates (Maxisorp, Nunc, Wiesbaden, Germany) were coated overnight at 4°C with 100 µL/well Human IL-12/IL-23 p40 MAb (Clone 169516) (2.5 µg/mL in PBS, R&D Systems, Minneapolis, USA). Wells were aspirated and blocked for 30 minutes at room temperature with SuperBlock®T20 PBS (Pierce, Rockford, IL) (300 µL/well). After 3 washing steps with PBS-0.05% Tween20, 100 µL/well Carrier-Free Recombinant Human IL-23 (p19/p40) (0.25 µg/mL in PBS-0.1% casein-0.05% Tween20, eBioScience, San Diego, USA) was captured by incubating for I hour at RT while shaking at 600 rpm. After this incubation step, wells were washed 3 times with PBS-0.05% Tween20. All preparations for standards, QC samples and test samples were done in a non-coated (polypropylene) plate. Predilutions of standards and QC samples were prepared in 100% mouse serum. To prepare standards and QC samples, a 1/10 dilution of the predilutions was made in PBS-0.1% casein-0.05% Tween20 (final concentration of mouse serum is 10%). Test samples were diluted 1/10 in PBS-0.1% casein-0.05% Tween20 and where needed further diluted in PBS-0.1% casein-0.05% Tween20 with 10% Mouse Serum (final concentration of mouse serum is 10%). Standards, QC samples and test samples were transferred to the coated plate and incubated for 2 hours at RT while shaking at 600 rpm. After 3 washing steps with PBS-0.05% Tween20, the plates were incubated with 100 µL/well of a rabbit anti-Nanobody polyclonal antibody (1/2000 diluted in PBS-0.1% casein-0.05% Tween20) for 1 hour at RT while shaking at 600 rpm. After 3 washing steps with PBS-0.05% Tween20, the plates were incubated with 100 µL/well horseradish peroxidase (HRP) labeled polyclonal goat anti-rabbit (1/5000 diluted in PBS-0.1% casein-0.05% Tween20, DakoCytomation, Glostrup, Denmark) for 1 hour at RT while shaking at 600 rpm. Visualization was performed by incubation with 100 µL/well enhanced soluble 3,3',5,5'-tetramethylbenzidine (esTMB, SDT, Brussels, Belgium). After 10 min, the colouring reaction was stopped with 104 µL/well 1N HCl. The absorbance was determined at 450 nm after a 10 seconds shake in the Tecan ELISA reader, and corrected for background absorbance at 620 nm. Concentration in each sample was determined based on a sigmoid standard curve.

Average plasma concentration-time profiles injected with 23IL0049, 23IL0041 and 23IL0038 were subjected to a compartmental pharmacokinetic analysis using the preprogrammed Model 1-7 and Model 3-4 for IV and SC route of administration, respectively, using WinNonlin Professional Software Version 5.1 (Pharsight Corporation, Mountain View California, USA). Model and Weighting discrimination was performed by visual inspection of the fit, and evaluation of minimum AIC (Akaike's Information Criterion), SBC (Schwarz Bayesian Criterion), WRSS (Weighted Residual Sum of Squares) and CV% (Coefficient of variation of the parameters). An overview of the calculated pharmacokinetic parameters is presented in Table B-35 and Table B-36.

Profiles after intravenous administration of 23IL0038 or 23IL0041-23IL0049 seemed to decline in a mono or biphasic manner, respectively. Profiles after subcutaneous administration of 23IL0049, 23IL0041 and 23IL0038 declined in a monophasic manner after the maximum concentrations were reached. Expected PK parameters were obtained for 231L0049 and 23IL0038 after both intravenous and subcutaneous administration, with slightly higher terminal half-lives after both intravenous and subcutaneous administration and higher bioavailability for 23IL0049. The terminal half-lives after both intravenous and subcutaneous administration and bioavailability of 23IL0041 were significantly lower.

**Table 35: Basic pharmacokinetic parameters¹ of 23IL0049, 23IL0041 and 23IL0038 after a single intravenous administration (100µg/ml) in the male Balb/c mouse.**

| **Parameter¹** | **23IL0049^{1,3}** | **23IL0041¹** | **23IL0038²** |
|---|---|---|---|
| C₍₀₎ (µg/ml) | 132 | 88.3 | 42.4 |
| Vₛₛ (mL) | 2.09 | 1.5.5 | 2.36 |
| V_{c} (ml) | 1.06 | 1.13 | 2.36 |
| V_{†}(mL) | 1.03 | 0.413 | - |
| CL (mL/day) | 1.06 | 4.99 | 1.43 |
| CL_{d} (mL/day) | 3.85 | 1.97 | - |
| t_{½α}(hr) | 2.11 | 1.97 | - |
| t_{½β}(day) | 1.46 | 0.277 | 1.14 |
| MRT (day) | 1.97 | 0.310 | 1.65 |
| AUC_{inf}(µg•day/ml) | 132 | 20.0 | 70.0 |
| AUC_{inf}/D (day/ml) | 0.940 | 0.200 | 0.700 |
| ¹ Parameters were calculated with two-compartmental modeling. | | | |
| ² Parameters were calculated with one-compartmental modeling. | | | |
| ³ Dose was 140 µg/animal. | | | |

**Table B-36: Basic pharmacokinetic parameters1 of 23IL0049, 23IL0041 and 23IL0038 after a single subcutaneous administration (100 µg/animal) in the male Balb/c mouse**

| **Parameter¹** | **23IL0049²** | **23IL0041** | **23IL0038** |
|---|---|---|---|
| V/F (mL) | 2.07 | 16.9 | 3.84 |
| CL/F (mL/day) | 0.929 | 33.9 | 2.04 |
| t_{lag}(hr) | 2.09 | - | 2.41 |
| t_{½absorption}(=0.693/K₀₁)(hr) | 6.26 | 1.98 | 2.46 |
| t_{½elimination}(=0.693/K₁₀)(day) | 1.55 | 0.346 | 1.30 |
| tₘₐₓ(day) | 0.894 | 0.218 | 0.508 |
| Cₘₐₓ(µg/ml) | 47.1 | 3.82 | 21.0 |
| AUC_{inf}(µg•day/ml) | 151 | 2.95 | 49.0 |
| AUC_{inf}/D (day/ml) | 1.08 | 0.0295 | 0.490 |
| F(%) | 114 | 14.7 | 70.0 |
| ¹ All parameters were calculated with compartmental modeling. | | | |
| ² Dose was 140 µg/animal. | | | |

No significant antibody titres to 23IL0049 and 23IL0038 Nanobodies are detected in the animals. Little amount of antibodies were seen to 23IL0041 after 6 days after intravenous administration and after 10 days after subcutaneous administration.

### Example 43: PK of formatted humanized Nanobodies (23IL0064, 23IL0065 and 23ILM63) in mouse.

Humanized IL23 Nanobodies 23IL0064, 3IL0065 and 23IL0063 are administered intravenous and subcutaneous to male Balb/c mice. The concentrations of Nanobodies are measured in serum samples. Antibodies to the Nanobodies are measured.

### Example 44: PK of formatted humanized Nanobodies (23IL0064, 23IL0065 and 23IL0063) in cynomolgus monkey.

Humanized IL23 Nanobodies 23IL0064, 23IL0065 and 23IL0063 are administered intravenous and subcutaneous to male cynomolgus monkeys. The concentrations of Nanobodies are measured in serum samples. Antibodies to the Nanobodies are measured.

### Example 45: Efficacy of 23IL0064, 23IL0065 and 23IL0064 in a humanized mouse psoriasis model

The ability of the anti-IL23p19 Nanobodies to suppress aspects of psoriasis in a humanized mouse model is evaluated. This model is based on studies described by Wrone-Smith and Nickoloff (1996). It is the only available model in which the effects of drugs on the development of a human psoriatic lesion can be monitored *in vivo*. Briefly, non-lesional skin from psoriasis patients are transplanted onto immunodeficient mice and, after acceptance of the grafts, autologous pre-activated PBMCs are intradermally injected triggering the psoriatic process. Mice are treated intraperitoneally twice weekly. After three weeks of treatment, mice are sacrificed and the human skin transplants are evaluated histologically, with regard to epidermal hyperplasia (HE-staining) as well as proliferation and differentiation of keratinocytes (e.g. Ki-67 staining).

### Example 46: anti-p40 biparatopic) Nanobodies and anti-p19-anti-p40 bispecific Nanobodies

### A) Construction of anti-p40 biparatopic Nanobodies and anti-p19 anti-p40 bispecific Nanobodies

Based on the data from the epitope grouping experiment with the p40 Nanobodies (Example 26), the following Nanobodies were combined in an anti-p40 biparatopic format: P23IL3C1 with P23IL3B8 or P23IL3G10, P23IL3B8 with P23IL80D10 or P23IL84G12, P23TL-23E3 with P23IL81A1 or P23IL84G1, P23IL-22D7 with P23IL80D10 or P23IL-22D10 and P23IL-22E11 with P23IL80D10 or P23IL84G12 or in an anti-p19 and anti-p40 bispecific format: P23IL119A3 with P23IL3C1, P23IL3B8 or P23IL-23E3 and P23IL37D5 with P23IL-22D7. The influence of the formatting, the positioning of the Nanobodies and the linker lengths on the potency was investigated. All constructs used are listed in Figures 33 and 34.

The coding sequences were cloned in pAX100, transformed in TG1 and expressed as myc-His tagged molecules. They were purified by IMAC, desalting, concentration and gelfiltration in the presence of 50 mM OGP, to remove LPS.

### B) Potency, of anti-p40 biparatopic Nanobodies and anti-p19 anti-p40 bispecific Nanobodies

### in the mouse splenocyte assay

The anti-p40 biparatopic and anti-p19 anti-p40 bispecific HLE Nanobodies were tested and compared with the mAB BM01 in the splenocyte assay using hIL-23 and cynomolgus IL-23 as described in Example 27. Results are shown in Table B-37. For the bispecific Nanobodies, all performed well on hIL-23, but P23IL0202 and 0204 performed less on cynomolgus IL-23. For the biparatopic Nanobodies P23IL0406 and 0407 have a bad potency when using cynomolgus IL-23, so their cross-reactivity is not optimal.

**Table B-37: potency of anti-p40 biparatopic Nanobodies and anti-p19 anti-p40 bispecific Nanobodies) in the mouse splenocyte assay using 19 pM HL-23 and 3.8 pM cynomolgus IL-23 (cIL-23)**

| **Bispecific Nanobody/mAB** | **hIL-23 IC50 (pM)** | **cIL-23 IC50 (pM)** |
|---|---|---|
| BM01 | 5.2 | 23.0 |
| P23IL0200 | 1.9 | 2.6 |
| P23IL0201 | 3.1 | 1.9 |
| P23IL0202 | 5.1 | 34.0 |
| P23IL0203 | 1.8 | 1.7 |
| P23IL0204 | 1.4 | 23.0 |
| P23IL0205 | 2.3 | 3.5 |

| **Biparatopic Nanobody/mAB** | **hIL-23 IC50 (pM)** | **cIL-23 IC50 (pM)** |
|---|---|---|
| BM01 | 4.7 | 29.0 |
| P23IL0400 | 3.0 | 3.3 |
| P23IL0401 | 3.6 | 5.6 |
| P23IL0402 | 11.0 | 8.5 |
| P23IL0403 | 2.7 | 4.9 |
| P23IL0404 | 3.6 | 3.7 |
| P23IL0405 | 4.2 | 6.8 |
| P23IL0406 | 11.0 | 628.0 |
| P23IL0407 | 5.6 | 883.0 |
| P23IL0408 | 4.6 | 6.7 |
| P23IL0409 | 3.0 | 6.8 |

### C Potency of anti-p40 biparatopic Nanobodies and anti-p19 anti-p40 bispecific Nanobodies in the BAF3 proliferation assay

Based on the results from the mouse splenocyte assay, a selected set of anti-p40 biparatopic and anti-p19 anti-p40 bispecific HLE Nanobodies with best potencies were tested and compared with the mAB BM0 in the BA/F3 proliferation assay using 76 pM hIL-23 as described in Example 27. Results are shown in Table B-38.

**Table B-38: potency potency of anti-p40 biparatopic Nanobodies and anti-p19 anti-p40 bispecific Nanobodies in the BA/F3 proliferation assay with 76 pM hIL-23**

| **Nanobody/mAB** | **IC 50** (**pM**) |
|---|---|
| P23IL0200 | 15.4 |
| P23IL0201 | 24.3 |
| P23IL0400 | 19.3 |
| P23IL0402 | 70.8 |
| P23IL0404 | 64.2 |
| BM01 | 33.2 |
| P23IL0405 | 60.3 |
| P23JL0408 | 55.5 |
| P23IL0409 | 33.3 |

### D) Activity on native hIL-23 in splenocyte assays:

Based on results from the mouse splenocyte assay with recombinant hIL-23, a selected set of anti-p40 biparatopic and anti-p19 anti-p40 bispecific HLE Nanobodies with best potencies, were tested and compared with the mAB BM01 in the mouse splenocyte assay using 19 pM native hIL-23, prepared as described in Example 31. Results are shown in Table B-39.

**Table B-39: potency of anti-p40 biparatopic Nanobodies and anti-p19 anti-p40 bispecific Nanobodies in the mouse splenocyte assay with 19 pM native hIL-23**

| **Nanobody/mAB** | **hIL-23 IC50** (**pM)** |
|---|---|
| BM01 | 39 |
| P23IL0200 | 11 |
| P23IL201 | 13 |
| P23IL0400 | 12 |
| P23IL0402 | 40 |
| P23IL0404 | 40 |
| P23IL0405 | 88 |
| P23IL0408 | 69 |
| P23IL0409 | 52 |

### E) Potency in IL-12 dependent PHA blast proliferation assay

The potency of the neutralization of hIL-12 is determined in the IL-12 dependent PHA blast proliferation assay. Shortly PHA blasts were derived by culturing PBMC in PHA (50µg/ml) during 3 days and IL-2 (50 U/ml) during one day. These PHA blasts are stimulated with hIL-12 pre-incubated with a dilution series of a Nanobody test items (for instance starting starting from 60 nM u to 0.15 pM or p19-p40 bispecific Nanobodies and p 40 monovalent Nanobodies starting from 2000 nM to 1 pM or control Nanobodies or antibodies. Cells are stimulated for 2 days and pulsed with 1 µCi/well ³H-thymidine for the 6 last hours, hence proliferation was determined by measuring ³H-thymidine incorporation by scintillation counting.

### Example 47: Activity of Nanobodies 23IL400 and 23IL403 in an acute in vivo mouse splenocyte model

The bivalent p40+/p40+ Nanobodies 23IL400 (3B8-ALB1-3C1, SEQ ID NO: 2630) and 23IL403 (3G10-ALB1 -3C1, SEQ ID NO: 2633) have been analyzed in the acute in vivo mouse splenocyte modeldescribed in Example 35. Seven groups with each four C57BL/6 mice were stimulated with 3 µg hIL-23 on t = 0h, 7h and 23h. The mice from group 1 received PBS instead of hIL-23 at the same time points. The positive control antibody (BM01; groups 3 and 4), 23IL400 (groups 5 and 6) and 23IL403 (groups 7 and 8) were injected s.c. 24 hours before the first hIL-23 injection. Two doses were analyzed and for both doses was the molar excess to each injection of 3 µg hIL-23 the same for all three test items (3.2-fold excess and 0.64-fold excess).

The results are shown in Figure 51, which is a graph showing the results obtained in Example 38 for the inhibition of the mIL-22 synthesis in a mouse splenocyte assay upon administration of BM01, P23IL0400 and P23IL0403 at two different dose levels.

The results were normalized to the mean mIL-22 concentration of group 2, which was injected with hIL-23 only. Both Nanobodies blocked synthesis of mIL-22 completely at the highest dose. At the lowest dose, there was a clear difference between 23IL400 and 23IL403. While 23IL400 still inhibited mIL-22 synthesis significantly at a 0.64-fold molar excess, mIL-22 concentrations were almost back to basal levels at the same molar dose of 23IL403. The efficacy of 23IL400 is comparable with BM01.

### Preferred Embodiments

1. Protein or polypeptide, comprising at least one amino acid sequence that is directed against the p19 subunit and at least one amino acid sequence that is directed against the p40 subunit, optionally linked via a suitable linker, and optionally comprising one or more further amino acid sequences, binding domains and/or binding units.
2. Protein or polypeptide according to item 1, in which the amino acid sequence that is directed against the p19 subunit is a p19+ sequence (i.e. an amino acid sequence that is capable of modulating, neutralizing, blocking and/or inhibiting the binding of a heterodimeric cytokine comprising a p19 subunit to its receptor), and in which the amino acid sequence that is directed against the p40 subunit is a p40+ sequence (i.e, an amino acid sequence that is capable of modulating, neutralizing, blocking and/or inhibiting the binding of a heterodimeric cytokine comprising a p40 subunit to its receptor).
3. Protein or polypeptide according to item 1, in which the amino acid sequence that is directed against the p19 subunit is a p19+ sequence (i.e. an amino acid sequence that is capable of modulating, neutralizing, blocking and/or inhibiting the binding of a heterodimeric cytokine comprising a p19 subunit to its receptor), and in which the amino acid sequence that is directed against the p40 subunit is a p40- sequence (i.e. an amino acid sequence that is essentially not capable of modulating, neutralizing, blocking and/or inhibiting the binding of a heterodimeric cytokine comprising a p40 subunit to its receptor).
4. Protein or polypeptide according to item 1, in which the amino acid sequence that is directed against the p19 subunit is a p19+ sequence (i.e. an amino acid sequence that is essentially not capable of modulating, neutralizing, blocking and/or inhibiting the binding of a heterodimeric cytokine comprising a p19 subunit to its receptor), and in which the amino acid sequence that is directed against the p40 subunit is a p40+ sequence (i.e. an amino acid sequence that is capable of modulating, neutralizing, blocking and/or inhibiting the binding of a heterodimeric cytokine comprising a p40 subunit to its receptor).
5. Protein or polypeptide, comprising at least one amino acid sequence that is directed against the p35 subunit and at least one amino acid sequence that is directed against the p40 subunit, optionally linked via a suitable linker, and optionally comprising one or more further amino acid sequences, binding domains and/or binding units.
6. Protein or polypeptide, comprising at least one amino acid sequence that is directed against a first epitope or antigenic determinant on the p19 subunit and at least one further amino acid sequence that is directed against a second epitope or antigenic determinant on the p19 subunit different from the first, optionally linked via a suitable linker, and optionally comprising one or more further amino acid sequences, binding domains and/or binding units.
7. Protein or polypeptide according to item 6, in which the first amino acid sequence is a p19+ sequence (i.e. an amino acid sequence that is essentially not capable of modulating, neutralizing, blocking and/or inhibiting the binding of a heterodimeric cytokine comprising a p19 subunit to its receptor), and in which the second amino acid sequence is a p19- sequence (i.e. an amino acid sequence that is essentially not capable of modulating, neutralizing, blocking and/or inhibiting the binding of a heterodimeric cytokine comprising a p 19 subunit to its receptor).
8. Protein or polypeptide, comprising at least one amino acid sequence that is directed against a first epitope or antigenic determinant on the p40 subunit and at least one further amino acid sequence that is directed against a second epitope or antigenic determinant on the p40 subunit different from the first, optionally linked via a suitable linker, and optionally comprising one or more further amino acid sequences, binding domains and/oz binding units.
9. Protein or polypeptide, in which the first amino acid sequence is a p40+ sequence (i.e. an amino acid sequence that is essentially not capable of modulating, neutralizing, blocking and/or inhibiting the binding of a heterodimeric cytokine comprising a p40 subunit to its receptor), and in which the second amino acid sequence is a p40- sequence (i.e. an amino acid sequence that is essentially not capable of modulating, neutralizing, blocking and/or inhibiting the binding of a heterodimeric cytokine comprising a p40 subunit to its receptor).
10. Protein or polypeptide according to any of items 1 to 9, in which each amino acid sequence that is comprised within said protein or polypeptide and that is directed against a subunit forms and/or essentially consist of a single (antigen) binding domain or binding unit, and/or is capable of forming and/or of functioning as a single (antigen) binding domain or binding unit (optionally after suitable folding).
11. Protein or polypeptide according to any of items 1 to 10, in which each amino acid sequence that is comprised within said protein or polypeptide and that is directed against a subunit comprises an immunoglobulin fold or is capable of, under suitable conditions, forming an immunoglobulin fold.
12. Protein or polypeptide according to any of items 1 to 11, in which each amino acid sequence that is comprised within said protein or polypeptide and that is directed against a subunit essentially consist of 4 framework regions (FR1 to FR4 respectively) and 3 complementarity determining regions.
13. Protein or polypeptide according to any of items 1 to 12, in which each amino acid sequence that is comprised within said protein or polypeptide and that is directed against a subunit is a domain antibody (or an amino acid sequence that is suitable for use as a domain antibody), a single domain antibody (or an amino acid sequence that is suitable for use as a single domain antibody), a "dAb" (or an amino acid sequence that is suitable for use as a dAb) or a Nanobody™ (including but not limited to a V_{HH} sequence) or another single variable domain, or any suitable fragment of any one thereof.
14. Protein or polypeptide that is directed against a heterodimeric protein, polypeptide, ligand or receptor that comprises:
   - at least a first subunit;
      and
   - at least a second subunit;
   wherein said protein or polypeptide at least comprises a first binding domain or binding unit that is directed against said first subunit and a second binding domain or binding unit that is directed against said second subunit.
15. Protein or polypeptide that is directed against a first heterodimeric protein, polypeptide, ligand or receptor that comprises:
   - at least a first subunit that is shared between said first heterodimeric protein, polypeptide, ligand or receptor and at least a second, different heterodimeric protein, polypeptide, ligand or receptor;
      and
   - at least a second subunit that is not shared between said first heterodimeric protein, polypeptide, ligand or receptor and said second, different heterodimeric protein, polypeptide, ligand or receptor;
   wherein, said protein or polypeptide at least comprises a first binding domain or binding unit that is directed against said first (i.e. shared) subunit and a second binding domain or binding unit that is directed against said second (i.e. not shared) subunit.
16. Protein or polypeptide that is directed against a first heterodimeric protein, polypeptide, ligand or receptor that comprises:
   - at least a first subunit;
      and
   - at least a second subunit;
   wherein said protein or polypeptide at least comprises a first binding domain or binding unit that is directed against said first subunit and a second binding domain or binding unit different from said first binding domain or binding unit that is also directed against said first subunit, but against a different epitope, antigenic determinant or binding site on said first subunit.
17. Protein or polypeptide according to item 15 or 16, which is directed against a ligand for a receptor, and which comprises at least one binding domain or binding unit that is capable of modulating, neutralizing, blocking and/or inhibiting the binding of the ligand to its (cognate) receptor and at least one binding domain or binding unit that is essentially not capable of modulating, neutralizing, blocking and/or inhibiting the binding of the ligand to its (cognate) receptor.
18. Protein or polypeptide according to item 15, which is directed against a ligand for a receptor, in which both the first binding domain or binding unit as well as the second binding domain or binding unit are capable of modulating, neutralizing, blocking and/or inhibiting the binding of the ligand to its (cognate) receptor.
19. Protein or polypeptide according to any of items 14 to 18, in which each binding domain or binding unit comprises an immunoglobulin fold or is capable of, under suitable conditions, forming an immunoglobulin fold.
20. Protein or polypeptide according to any of items 14 to 19, in which each binding domain or binding unit essentially consist of 4 framework regions (FR1 to FR4 respectively) and 3 complementarity determining regions.
21. Protein or polypeptide according to any of items 14 to 20, in which each binding domain or binding unit is a domain antibody (or an amino acid sequence that is suitable for use as a domain antibody), a single domain antibody (or an amino acid sequence that is suitable for use as a single domain antibody), a "dAb" (or an amino acid sequence that is suitable for use as a dAb) or a Nanobody™ (including but not limited to a V_{HH} sequence) or another single variable domain, or any suitable fragment of any one thereof.
22. Nucleotide sequence or nucleic acid encoding a protein or polypeptide according to any of items 1 to 21.
23. Composition comprising at least one protein or polypeptide according to any of items 1 to 21 or a nucleotide sequence or nucleic acid according to item 22.
24. Pharmaceutical composition comprising at least one protein or polypeptide according to any of items 1 to 21 and at least one pharmaceutically acceptable carrier, diluent or excipient and/or adjuvant.
25. Use of (a nucleotide sequence and/or nucleic acid that encodes) a p19+ sequence in providing, constructing, and/or as part of (a nucleotide sequence and/or nucleic acid that encodes) a multivalent, multispecific and/or multiparatopic construct, protein and/or polypeptide that comprises said p19+ sequence (one or more) and one or more further binding domains or binding units.
26. Use of (a nucleotide sequence and/or nuclei acid that encodes) a p19- sequence in providing, constructing, and/or as part of (a nucleotide sequence and/or nucleic acid that encodes) a multivalent, multispecific and/or multiparatopic construct, protein and/or polypeptide that comprises said p19- sequence (one or more) and one or more further binding domains or binding units.
27. Use of (a nucleotide sequence and/or nucleic acid that encodes) a p40- sequence in providing, constructing, and/or as part of (a nucleotide sequence and/or nucleic acid that encodes) a multivalent, multispecific and/or multiparatopic construct, protein and/or polypeptide that comprises said p40- sequence (one or more) and one or more further binding domains or binding units.
28. Use of (a nucleotide sequence and/or nucleic acid that encodes) a p40+ sequence in providing, constructing, and/or as part of (a nucleotide sequence and/or nucleic acid that encodes) a multivalent, multispecific and/or multiparatopic construct, protein and/or polypeptide that comprises said p40+ sequence (one or more) and one or more further binding domains or binding units.
29. Use of (a nucleotide sequence and/or nucleic acid that encodes) a p35 sequence in providing, constructing, and/or as part of (a nucleotide sequence and/or nucleic acid that encodes) a multivalent, multispecific and/or multiparatopic construct, protein and/or polypeptide that comprises said p35 sequence (one or more) and one or more further binding domains or binding units.
30. Use according to any of items 25 to 29, in which the multivalent, multispecific and/or multiparatopic construct, protein and/or polypeptide is directed against a heterodimeric cytokine.
31. Use according to any of items 25 to 30, in which the construct, protein and/or polypeptide is a biparatopic construct, protein and/or polypeptide that is directed against one subunit of the heterodimeric cytokine.
32. Use according to any of items 30 and 31, in which the construct, protein and/or polypeptide is a multispecific construct, protein and/or polypeptide comprising at least one binding domain or binding unit that is directed against a first subunit of said heterodimeric cytokine and at least one binding domain or binding unit that is directed against a second subunit of said heterodimeric cytokine.
33. Use according to any of items 30 to 32, in which the construct, protein and/or polypeptide comprises at least one least one binding domain or binding unit that is capable of modulating, neutralizing, blocking and/or inhibiting the binding of the heterodimeric cytokine to its (cognate) receptor.
34. Use of (a nucleotide sequence and/or nucleic acid that encodes) an amino acid sequence that comprises or essentially consists of a single binding domain or binding unit in providing, constructing, and/or as part of (a nucleotide sequence and/or nucleic acid that encodes) a multispecific construct, protein and/or polypeptide that is directed against a heterodimeric protein, polypeptide, ligand or receptor, wherein said construct, protein and/or polypeptide comprises said amino acid sequence (one or more) and at least one further binding domain or binding unit, and wherein said one or more amino acid sequences are directed against a first subunit of the heterodimeric protein, polypeptide, ligand or receptor and at least one of said further binding domains or binding units is directed against a second subunit of the heterodimeric protein, polypeptide, ligand or receptor different from the first subunit.
35. Use according to item 34, in which the construct, protein and/or polypeptide is a directed against a heterodimeric cytokine or against a heterodimeric receptor for a heterodimeric cytokine.
36. Use of (a nucleotide sequence and/or nucleic acid that encodes) an amino acid sequence that comprises or essentially consists of a single binding domain or binding unit in providing, constructing, and/or as part of (a nucleotide sequence and/or nucleic acid that encodes) a biparatopic construct, protein and/or polypeptide that is directed against a heterodimeric protein, polypeptide, ligand or receptor, wherein said construct, protein and/or polypeptide comprises said amino acid sequence (one or more) and at least one further binding domain or binding unit, and wherein said one or more amino acid sequences are directed against a first subunit of the heterodimeric protein, polypeptide, ligand or receptor and at least one of said further binding domains or binding units is also directed against said first subunit, but to a different epitope or antigenic determinant on sais subunit.
37. Use according to item 36, in which the construct, protein and/or polypeptide is directed against a ligand for a receptor and comprises at least one binding domain or binding unit that is capable of modulating, neutralizing, blocking and/or inhibiting the binding of the ligand to its (cognate) receptor and at least one binding domain or binding unit that is essentially not capable of modulating, neutralizing, blocking and/or inhibiting the binding of the ligand to its (cognate) receptor.

## Claims

1. Protein or polypeptide, comprising at least one amino acid sequence that forms and/or essentially consist of a single binding domain or binding unit, and/or that is capable of forming and/or of functioning as a single binding domain or binding unit, and that is directed against the p 19 subunit and at least one amino acid sequence that forms and/or essentially consist of a single binding domain or binding unit, and/or that is capable of forming and/or of functioning as a single binding domain or binding unit, and that is directed against the p40 subunit, optionally linked via a suitable linker, and optionally comprising one or more further amino acid sequences, binding domains and/or binding units.

2. Protein or polypeptide according to claim 1, in which the amino acid sequence that is directed against the p19 subunit is a p19+ sequence, i.e. an amino acid sequence that is capable of modulating, neutralizing, blocking and/or inhibiting the binding of a heterodimeric cytokine comprising a p 19 subunit to its receptor, and in which the amino acid sequence that is directed against the p40 subunit is a p40+ sequence, i.e. an amino acid sequence that is capable of modulating, neutralizing, blocking and/or inhibiting the binding of a heterodimeric cytokine comprising a p40 subunit to its receptor.

3. Protein or polypeptide according to claim 1, in which the amino acid sequence that is directed against the p19 subunit is a p19+ sequence, i.e. an amino acid sequence that is capable of modulating, neutralizing, blocking and/or inhibiting the binding of a heterodimeric cytokine comprising a p19 subunit to its receptor, and in which the amino acid sequence that is directed against the p40 subunit is a p40- sequence,(i.e. an amino acid sequence that is essentially not capable of modulating, neutralizing, blocking and/or inhibiting the binding of a heterodimeric cytokine comprising a p40 subunit to its receptor.

4. Protein or polypeptide according to claim 1, in which the amino acid sequence that is directed against the p19 subunit is a p19- sequence, i.e. an amino acid sequence that is essentially not capable of modulating, neutralizing, blocking and/or inhibiting the binding of a heterodimeric cytokine comprising a p19 subunit to its receptor, and in which the amino acid sequence that is directed against the p40 subunit is a p40+ sequence, i.e. an amino acid sequence that is capable of modulating, neutralizing, blocking and/or inhibiting the binding of a heterodimeric cytokine comprising a p40 subunit to its receptor.

5. Protein or polypeptide, comprising at least one amino acid sequence that forms and/or essentially consist of a single binding domain or binding unit, and/or that is capable of forming and/or of functioning as a single binding domain or binding unit, and that is directed against a first epitope or antigenic determinant on the p 19 subunit and at least one further amino acid sequence that forms and/or essentially consist of a single binding domain or binding unit, and/or that is capable of forming and/or of functioning as a single binding domain or binding unit, and that is directed against a second epitope or antigenic determinant on the p19 subunit different from the first, optionally linked via a suitable linker, and optionally comprising one or more further amino acid sequences, binding domains and/or binding units.

6. Protein or polypeptide according to claim 5, in which the first amino acid sequence is a p19+ sequence, i.e. an amino acid sequence that is essentially capable of modulating, neutralizing, blocking and/or inhibiting the binding of a heterodimeric cytokine comprising a p 19 subunit to its receptor, and in which the second amino acid sequence is a p 19- sequence, i.e. an amino acid sequence that is essentially not capable of modulating, neutralizing, blocking and/or inhibiting the binding of a heterodimeric cytokine comprising a p19 subunit to its receptor.

7. Protein or polypeptide, comprising at least one amino acid sequence that forms and/or essentially consist of a single binding domain or binding unit, and/or that is capable of forming and/or of functioning as a single binding domain or binding unit, and that is directed against a first epitope or antigenic determinant on the p40 subunit and at least one further amino acid sequence that forms and/or essentially consist of a single binding domain or binding unit, and/or that is capable of forming and/or of functioning as a single binding domain or binding unit, and that is directed against a second epitope or antigenic determinant on the p40 subunit different from the first, optionally linked via a suitable linker, and optionally comprising one or more further amino acid sequences, binding domains and/or binding units.

8. Protein or polypeptide according to claim 7, in which the first amino acid sequence is a p40+ sequence, i.e. an amino acid sequence that is essentially capable of modulating, neutralizing, blocking and/or inhibiting the binding of a heterodimeric cytokine comprising a p40 subunit to its receptor, and in which the second amino acid sequence is a p40- sequence, i.e. an amino acid sequence that is essentially not capable of modulating, neutralizing, blocking and/or inhibiting the binding of a heterodimeric cytokine comprising a p40 subunit to its receptor.

9. Protein or polypeptide according to any of claims 1 to 8, in which each amino acid sequence that is comprised within said protein or polypeptide and that is directed against a subunit comprises an immunoglobulin fold or is capable of, under suitable conditions, forming an immunoglobulin fold.

10. Protein or polypeptide according to any of claims 1 to 9, in which each amino acid sequence that is comprised within said protein or polypeptide and that is directed against a subunit is a domain antibody or an amino acid sequence that is suitable for use as a domain antibody, a single domain antibody or an amino acid sequence that is suitable for use as a single domain antibody, a "dAb" or an amino acid sequence that is suitable for use as a dAb or a Nanobody™ including but not limited to a V_{HH} sequence or another single variable domain, or any suitable fragment of any one thereof.

11. Nucleotide sequence or nucleic acid encoding a protein or polypeptide according to any of claims 1 to 10.

12. Composition comprising at least one protein or polypeptide according to any of claims 1 to 10 or a nucleotide sequence or nucleic acid according to claim 11.

13. Pharmaceutical composition comprising at least one protein or polypeptide according to any of claims 1 to 10 and at least one pharmaceutically acceptable carrier, diluent or excipient and/or adjuvant.
